# EUROPEAN PATENT APPLICATION

(11) **EP 4 559 909 A1**
(43) Date of publication of application: **28.05.2025**
(21) Application number: 23842443.6
(22) Date of filing: 21.07.2023
(51) Int. Cl.: C07D 403/14, C07D 405/14, C07D 519/00, A61K 31/4192, A61K 31/4162, A61P 35/00, A61P 37/00

(54) **COMPOUND FOR INHIBITING IRAK4 ACTIVITY AND USE THEREOF**

(30) Priority: 22.07.2022 CN 202210870938; 21.11.2022 CN 202211473224; 05.07.2023 CN 202310819823
(71) Applicant: Shenzhen Zhongge Biological Technology Co., Ltd., Shenzhen, Guangdong 518000 (CN)
(72) Inventor: DU, Xinming, Shenzhen, Guangdong 518000 (CN); XU, He, Shenzhen, Guangdong 518000 (CN); DU, Lifei, Shenzhen, Guangdong 518000 (CN); CHEN, Zhaoqiang, Shenzhen, Guangdong 518000 (CN); LI, Yuanpeng, Shenzhen, Guangdong 518000 (CN); MI, Yutao, Shenzhen, Guangdong 518000 (CN); QU, Mengyang, Shenzhen, Guangdong 518000 (CN); JIA, Jilong, Shenzhen, Guangdong 518000 (CN); MA, Junjun, Shenzhen, Guangdong 518000 (CN); ZOU, Junjie, Shenzhen, Guangdong 518000 (CN); ZOU, Rongfeng, Shenzhen, Guangdong 518000 (CN); WEN, Xiaoming, Shenzhen, Guangdong 518000 (CN); WANG, Shaohui, Shenzhen, Guangdong 518000 (CN); CHEN, Bin, Shenzhen, Guangdong 518000 (CN); ZHANG, Peiyu, Shenzhen, Guangdong 518000 (CN)
(74) Representative: Meissner Bolte Partnerschaft mbB
(86) International application number: PCT/CN2023/108727
(87) International publication number: WO 2024/017381

(57) **Abstract**

The present application relates to a compound for inhibiting IRAK4 activity and a use thereof, and specifically provides a compound as represented by formula A, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuterated substance, or a prodrug thereof,

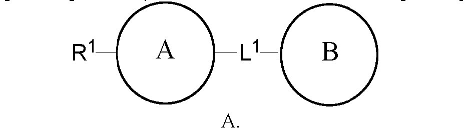

## Description

### Technical field

The present invention relates to the field of medicine, and particularly relates to compound for inhibiting IRAK4 activity and use thereof.

### Background art

IRAK4 is a serine/threonine protein kinase belonging to the Interleukin-1 receptor-associated kinase family, which includes four subtypes IRAK1, IRAK2, IRAK3 (or IRAKM) and IRAK4. Among these four subtypes, IRAK1, IRAK2 and IRAK4 promote the release of inflammatory factors, while IRAK3 is involved in the process of inflammation inhibition. Among the four subtypes, the biological function of IRAK4 has been clearly elucidated. When TLR or IL-1R senses external signal stimulation, Myddosome complex formed by IRAK4 activates MAPK and NF-kB pathways, thereby releasing various inflammatory factors.

Research proves that IRAK4 is highly expressed in various tumor cells and inflammation models, and the development of an inhibitor targeting IRAK4 increasingly becomes an important direction for the treatment of autoimmune diseases and tumors. IRAK4 has two functions: kinase activity and skeleton activity, which play an important role in downstream signal regulation.

In addition, Proteolysis Targeting Chimeria (PROTAC) technology is a new technology emerging in recent years, which has attracted much attention since its emergence in 2001, and a plurality of drug developments based on the technology are now in clinical research stage, and partially in clinical stage 2. PROTAC, as a heterogeneous bifunctional molecule, consists of a small molecule inhibitor capable of recognizing a target protein at one end, a connecting chain and a ligand capable of recognizing an E3 ubiquitin ligase at the other end. The bifunctional molecule recognizes and binds a target protein and an E3 ubiquitin ligase to form ternary complex *in vivo,* and then ubiquitination marking is carried out on the target protein, so that a degradation pathway depending on ubiquitin-proteasome is started. Compared with conventional small molecule inhibitor, the PROTAC technology realizes simultaneous inhibition of two functions of IRAK4 by degrading IRAK4 protein, thereby effectively solving the problem of insufficient activity or mutation of small molecules.

Therefore, the development of a new small molecule IRAK4 kinase inhibitor is of great importance, and a PROTAC molecule targeting IRAK4 can be obtained based on the new small molecule IRAK4 kinase inhibitor.

### Contents of the invention

Through deep research and creative discovery, the inventors obtain new small molecule IRAK4 kinase inhibitors, which can be used for the treatment of various diseases. And the new small molecule IRAK4 kinase inhibitors lay a foundation for further development of a PROTAC molecule targeting IRAK4.

Therefore, in the first aspect, the present invention provides a compound represented by formula A, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, 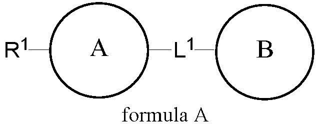 wherein:
A is selected from 5-6 membered heteroaromatic ring optionally substituted with substituent R^{k};
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl;
R^{p}, R^{q} are each independently selected from the group consisting of H, C1-C6 alkyl, C1-C6 alkoxy, and C1-C6 haloalkyl;
B is selected from the group consisting of 6-10 membered heteroaryl, and 6-10 membered aryl, and B is optionally substituted with at least one of substituents R^{a}, R^{b}; preferably, B is substituted with at least one of substituents R^{a}, R^{b};
R^{a} is selected from the group consisting of H, halogen, cyano, carboxyl, 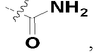 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, 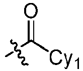 (wherein Cy₁ is 4-9 membered heterocyclyl optionally substituted with R_{cy1} and R_{cy2}), -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), and 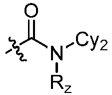 (wherein R_{z} is H or C1-C6 alkyl, Cy₂ is 5-7 membered heterocyclyl, C3-C7 cycloalkyl or C6-C10 aryl, wherein, the 5-7 membered heterocyclyl, C3-C7 cycloalkyl and C6-C10 aryl are optionally substituted with 1-3 substituents selected from the group consisting of halogen, C1-C6 alkyl, C1-C6 alkoxy, CN, and hydroxy), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl);
R_{cy1} and R_{cy2} are each independently selected from the group consisting of C1-C6 alkyl, C1-C6 alkoxy, C1-C6 haloalkyl, halogen, hydroxy, nitro, cyano, amino, -NH(C1-C6 alkyl), -N(C1-C6 alkyl)(C1-C6 alkyl), and oxo;
R^{b} is selected from the group consisting of H, halogen, amino, cyano, carboxyl, 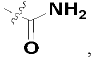 C1-C6 alkyl, C1-C6 haloalkyl (such as trifluoromethyl), and 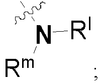 R^{l}, R^{m} are each independently selected from the group consisting of H, C1-C6 alkyl, and C3-C6 cycloalkyl;
or, R^{a}, R^{b} and the carbon atoms to which they are attached, respectively, together form a substituted or unsubstituted 5-membered heterocycle, wherein, the substituent is selected from C1-C6 alkyl (such as tert-butyl);
L¹ is selected from the group consisting of a direct bond, C1-C6 alkyl, 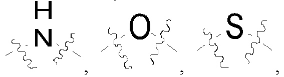 and 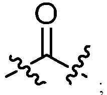
R¹ is selected from the group consisting of 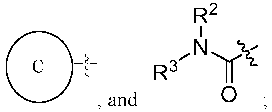
C is selected from 5-6 membered heteroaryl, and C is optionally substituted with at least one of substituents R^{d}, R^{e}, R^{f};
R^{d}, R^{e}, R^{f} are each independently selected from the group consisting of H, C1-C6 alkyl, C1-C6 haloalkyl (such as difluoromethyl), C3-C7 cycloalkyl, 6-10 membered aryl, 5-6 membered heteroaryl, 4-7 membered saturated heterocyclyl, and 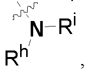 wherein, the C3-C7 cycloalkyl, 6-10 membered aryl, 5-6 membered heteroaryl, and 4-7 membered saturated heterocyclyl are each independently optionally substituted with C1-C6 alkyl, 1-2 substituents selected from the group consisting of halogen, alkoxy (such as C1-C6 alkoxy), hydroxy, amino, hydroxy C1-C6 alkyl (such as methylol), amino C1-C6 alkyl, and formyl; R^{h}, Rⁱ are each independently selected from the group consisting of H, C1-C6 alkyl, and C3-C6 cycloalkyl;
or, R^{e}, R^{f} and the carbon atoms to which they are attached, respectively, together form a substituted or unsubstituted 5-9 membered heterocycle, wherein, the substituent is selected from the group consisting of C1-C6 alkyl (such as ethyl), alkoxy (such as C1-C6 alkoxy), C3-C6 cycloalkyl, amino, and hydroxy;
R², R³ are each independently selected from the group consisting of H, C1-C6 alkyl, and C3-C6 cycloalkyl; or, R², R³ and the N atom to which they are both attached together form 5-6 membered saturated heterocyclyl, preferably, the 5-6 membered saturated heterocyclyl contains 2-3 heteroatoms selected from the group consisting of N, S, and O atoms.

In certain embodiments, A is selected from the group consisting of 5 membered heteroaromatic ring, and 6 membered heteroaromatic ring, wherein, the 6 membered heteroaromatic ring is optionally substituted with substituent R^{k}.

In certain embodiments, A is selected from the group consisting of 5 membered heteroaromatic ring, and 6 membered heteroaromatic ring, the 5 membered heteroaromatic ring containing 2-3 heteroatoms, wherein at least two heteroatoms are N atoms; the 6 membered heteroaromatic ring containing 1-2 heteroatoms, wherein at least one heteroatom is N atom, and, the 5 membered heteroaromatic ring and 6 membered heteroaromatic ring are optionally substituted with substituent R^{k}.

In certain embodiments, A is selected from the group consisting of 1,2,3-triazole ring, 1,2,4-triazole ring, pyrazole ring, imidazole ring, 1,3,4-thiadiazole ring, 1,3,4-oxadiazole ring, 2-pyridone, and pyridazine ring, wherein, the 2-pyridone is optionally substituted with substituent R^{k}.

In certain embodiments, A is selected from the group consisting of 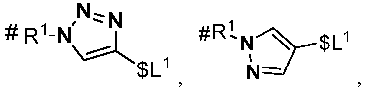 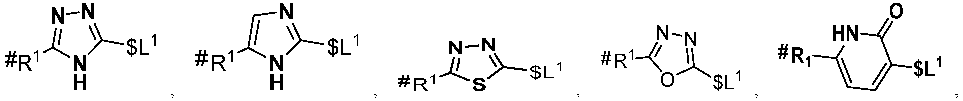 and 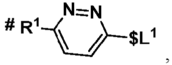 wherein, the 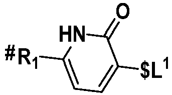 is optionally substituted with substituent R^{k}, wherein #R¹ represents the attachment point to R¹, $L¹ represents the attachment point to L¹.

In certain embodiments, A is selected from the group consisting of 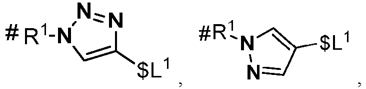 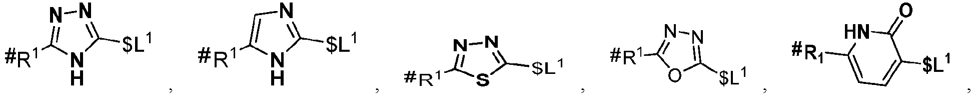 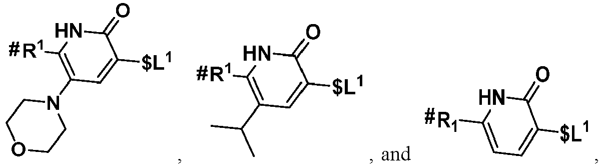 wherein #R¹ represents the attachment point to R¹, $L¹ represents the attachment point to L¹.

In certain embodiments, A is selected from the group consisting of 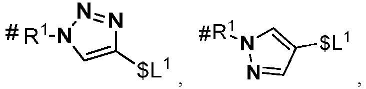 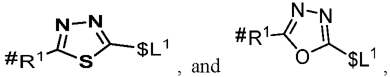 wherein #R¹ represents the attachment point to R¹, $L¹ represents the attachment point to L¹.

In certain embodiments, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl).

In certain embodiments, R^{k} is selected from the group consisting of C1-C6 alkyl, and 5-6 membered saturated heterocyclyl.

In certain embodiments, R^{k} is selected from the group consisting of C1-C6 alkyl, C3-C6 cycloalkyl, R^{p}R^{q}N-, C1-C6 haloalkyl, and morpholinyl.

In certain embodiments R^{k} is selected from the group consisting of isopropyl, cyclopropyl, dimethylamino, difluoromethyl, and

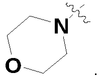

In certain embodiments, R^{p}, R^{q} are each independently selected from the group consisting of H, C1-C6 alkyl, C1-C6 alkoxy, and C1-C6 haloalkyl.

In certain embodiments, R^{p}, R^{q} are each independently selected from the group consisting of H, and C1-C6 alkyl.

In certain embodiments, B is selected from the group consisting of 6-10 membered heteroaryl, and 6-10 membered aryl, and B is substituted with substituents R^{a}, R^{b}.

In certain embodiments, B is selected from the group consisting of 9 membered heteroaryl, pyridinyl, phenyl, and pyrimidinyl, and B is substituted with at least one of substituents R^{a}, R^{b}; preferably, the 9 membered heteroaryl contains 1-4 heteroatoms selected from the group consisting of N, O, and S atoms.

In certain embodiments, B is selected from the group consisting of 9 membered heteroaryl, pyridinyl, and phenyl, and B is substituted with at least one of substituents R^{a}, R^{b}; preferably, the 9 membered heteroaryl contains 1-4 heteroatoms selected from the group consisting of N, O, and S atoms.

In certain embodiments, B is selected from the group consisting of 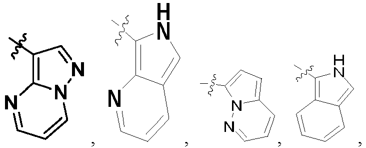 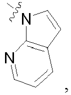 pyridinyl, phenyl, and pyrimidinyl, and B is substituted with at least one of substituents R^{a}, R^{b}.

In certain embodiments, B is selected from the group consisting of 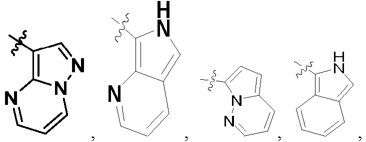 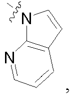 pyridinyl, and phenyl, and B is substituted with at least one of substituents R^{a}, R^{b}.

In certain embodiments, B is selected from the group consisting of 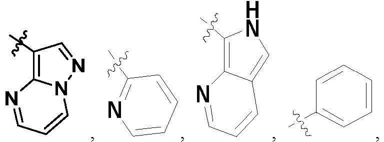 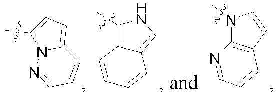 and B is substituted with substituents R^{a}, R^{b}.

In certain embodiments, B is selected from the group consisting of 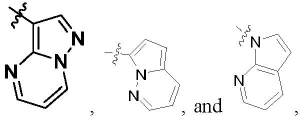 and B is substituted with substituents R^{a}, R^{b}.

In certain embodiments, R^{a} is selected from the group consisting of H, halogen, amino, cyano, carboxyl, 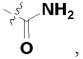 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of hydroxy, C3-C6 cycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, halogen, hydroxy, hydroxy-C1-C6 alkyl-, cyano, oxo, - NH₂, -N(C1-C6 alkyl), and -N(C1-C6 alkyl)(C1-C6 alkyl).

In certain embodiments, R^{a} is selected from the group consisting of H, cyano, carboxyl, 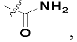 C1-C6 haloalkyl (such as trifluoromethyl), 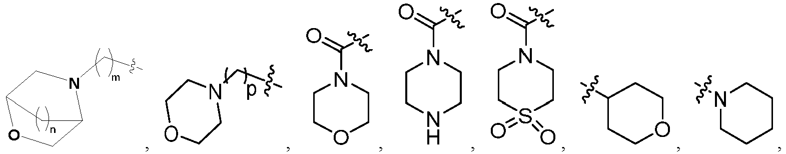 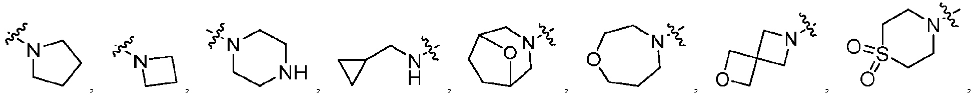 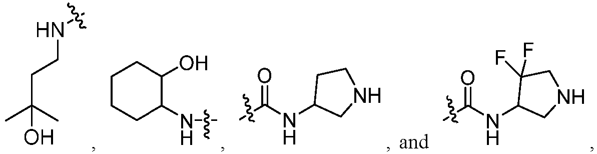 wherein, the 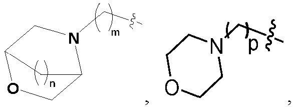 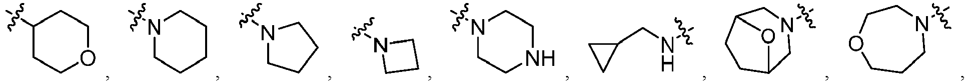 and 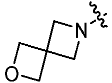 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl), wherein m is selected from the group consisting of 0, 1, 2, and 3, n is selected from the group consisting of 0, 1, 2, and 3, p is selected from the group consisting of 0, 1, 2, and 3.

In certain embodiments, R^{a} is selected from the group consisting of H, cyano, carboxyl, 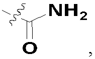 C1-C6 haloalkyl (such as trifluoromethyl), 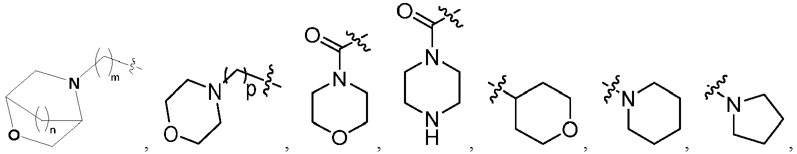 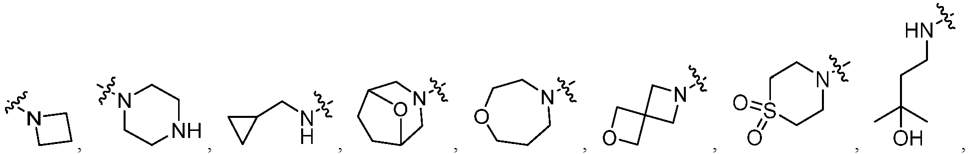 and 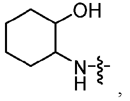 wherein, the 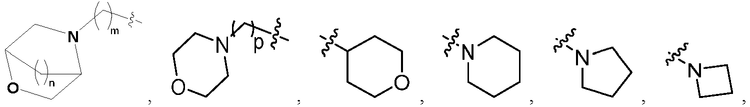 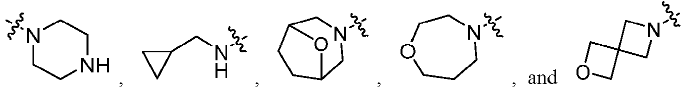 are optionally substituted with 1-3 substituents selected from the group consisting of C1-C6 alkyl, halogen, hydroxy, cyano, -NH₂, -N(C1-C6 alkyl), and - N(C1-C6 alkyl)(C1-C6 alkyl), wherein m is selected from the group consisting of 0, 1, 2, and 3, n is selected from the group consisting of 0, 1, 2, and 3, p is selected from the group consisting of 0, 1, 2, and 3.

In certain embodiments, R^{a} is selected from the group consisting of H, cyano, 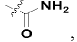 C1-C6 haloalkyl (such as trifluoromethyl), and 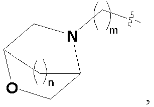 wherein, the 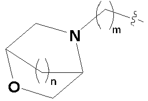 is optionally substituted with C1-C6 alkyl (preferably methyl), F, Cl, Br, wherein, wherein m is selected from the group consisting of 0, 1, 2, and 3, n is selected from the group consisting of 0, 1, 2, and 3.

In certain embodiments, R^{a} is selected from the group consisting of H, cyano, carboxyl, 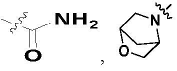 (preferably 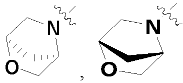 ), 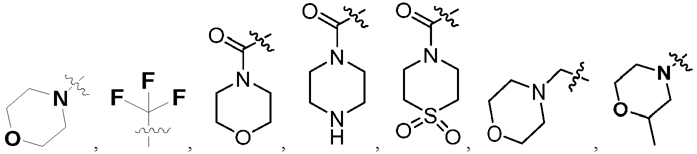 (preferably 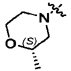 ), 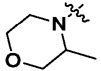 (preferably 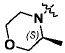 ), 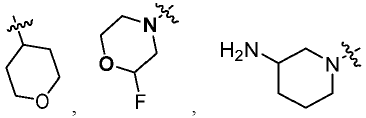 (preferably 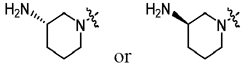 ), 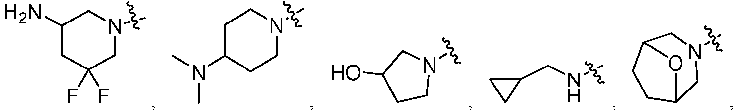 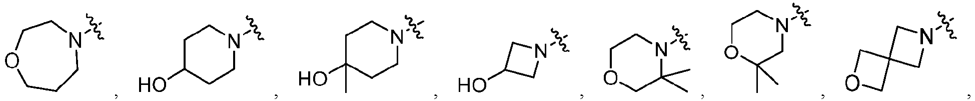 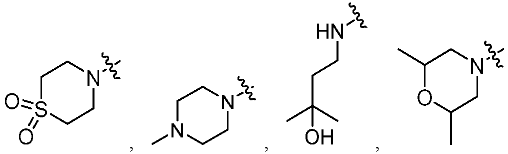 (preferably 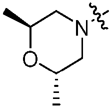 ), 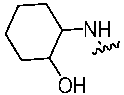 (preferably 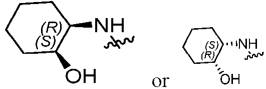 ), 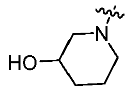 (preferably 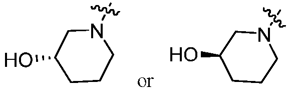 ), 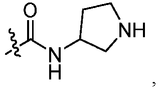 and

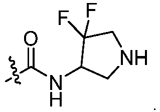

In certain embodiments, R^{a} is selected from the group consisting of H, cyano, carboxyl, 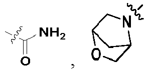 (preferably 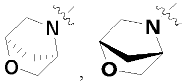 ), 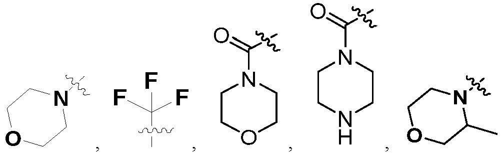 (preferably 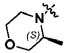 ), 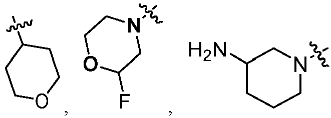 (preferably 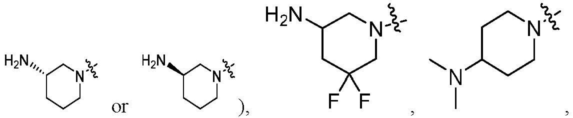 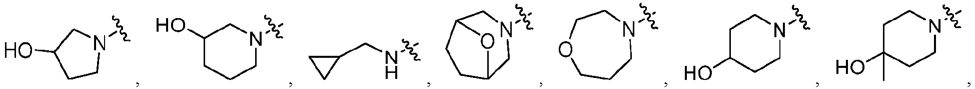 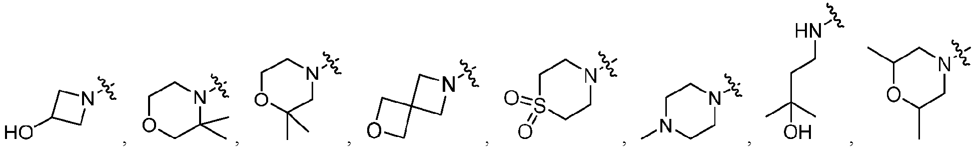 (preferably 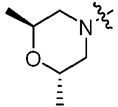 ), 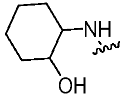 (preferably 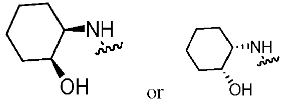 ), and 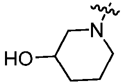 (preferably 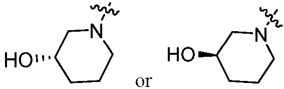 ).

In certain embodiments, R^{a} is selected from the group consisting of H, cyano,

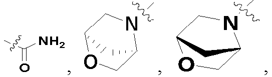

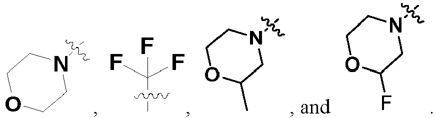

In certain embodiments, R^{a} is selected from the group consisting of H, cyano,

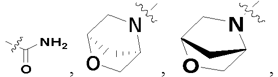

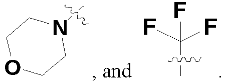

In certain embodiments, m is selected from the group consisting of 0, and 1. In certain embodiments, n is selected from the group consisting of 0, 1, 2, and 3. In certain embodiments, n is selected from the group consisting of 0, and 1. In certain embodiments, p is selected from the group consisting of 0, and 1. In certain embodiments, m is 0, and n is 0.

In certain embodiments, Cy₁ is 5-7 membered heterocyclyl optionally substituted with R_{cy1} and R_{cy2}, further preferably, Cy₁ is 6 membered heterocyclyl optionally substituted with R_{cy1} and R_{cy2}, further preferably, Cy₁ is morpholinyl, piperazinyl, or thiomorpholinyl optionally substituted with R_{cy1} and R_{cy2}.

In certain embodiments, Cy₂ is 5-7 membered heterocyclyl optionally substituted with 1-3 substituents selected from the group consisting of halogen, C1-C6 alkyl, C1-C6 alkoxy, CN, and hydroxy.

In certain embodiments, Cy₂ is selected from the group consisting of azetidinyl, pyrrolidinyl, imidazolidinyl, piperidyl, piperazinyl, and morpholinyl, wherein, the azetidinyl, pyrrolidinyl, imidazolidinyl, piperidyl, piperazinyl, and morpholinyl are optionally substituted with 1-3 substituents selected from the group consisting of halogen, and C1-C6 alkyl.

In certain embodiments, Cy₂ is selected from the group consisting of pyrrolidinyl, imidazolidinyl, and piperidyl, wherein, the pyrrolidinyl, imidazolidinyl, and piperidyl are optionally substituted with 1-3 substituents selected from the group consisting of F, Cl, Br, and methyl.

In certain embodiments, Cy₂ is pyrrolidinyl optionally substituted with 1-3 (such as 1 or 2 or 3) F.

In certain embodiments, R_{cy1} and R_{cy2} are each independently selected from the group consisting of C1-C6 alkyl, halogen, hydroxy, amino, and oxo, further preferably R_{cy1} and R_{cy2} are oxo.

In certain embodiments, R^{b} is selected from the group consisting of H, cyano,

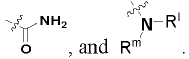

In certain embodiments, R^{l}, R^{m} are each independently selected from the group consisting of H, and C1-C6 alkyl.

In certain embodiments, R^{a}, R^{b} and the carbon atoms to which they are attached, respectively, together form 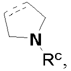 wherein R^{c} is selected from the group consisting of H, and C1-C6 alkyl.

In certain embodiments, R^{c} is selected from C1-C6 alkyl.

In certain embodiments, B, as a whole, is selected from the group consisting of: 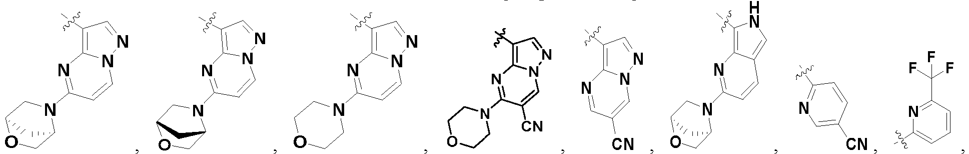 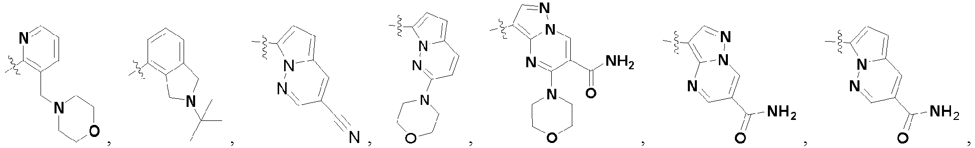 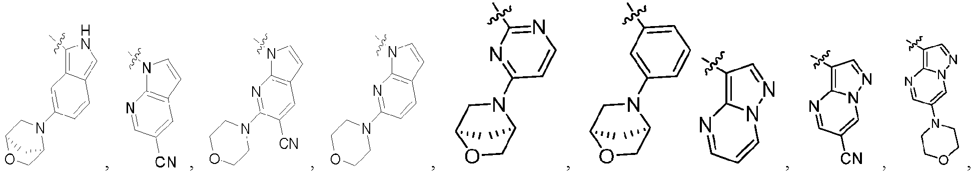 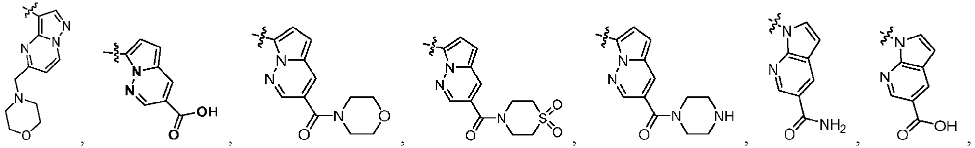 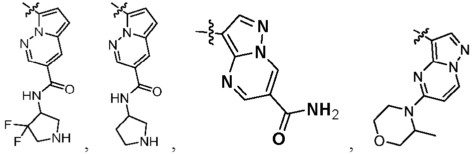 (preferably 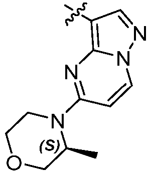 ), 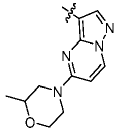 (preferably 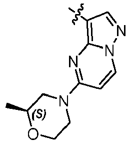 ), 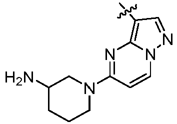 ( 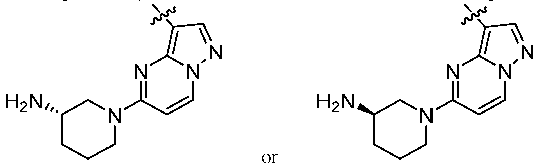 ), 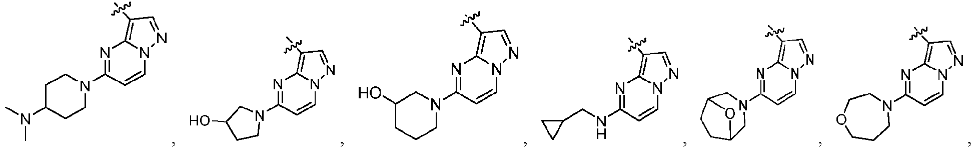 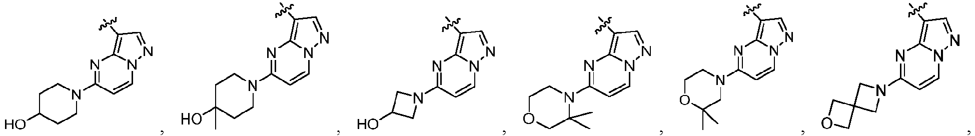 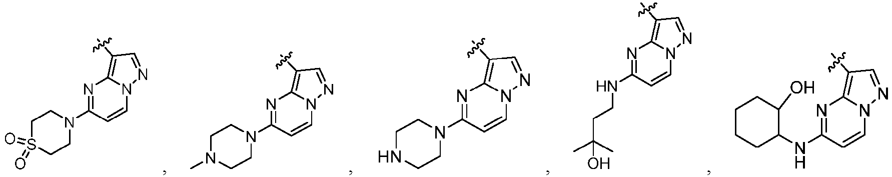 (preferably 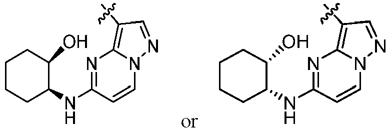 ), 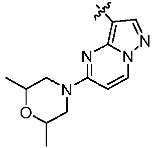 (preferably 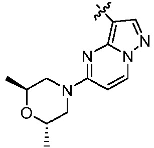 ), 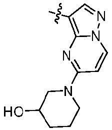 (preferably 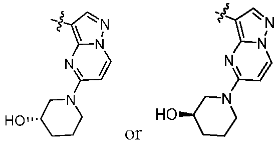 ), and

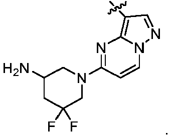

In certain embodiments, B, as a whole, is selected from the group consisting of 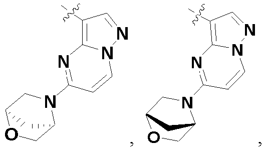 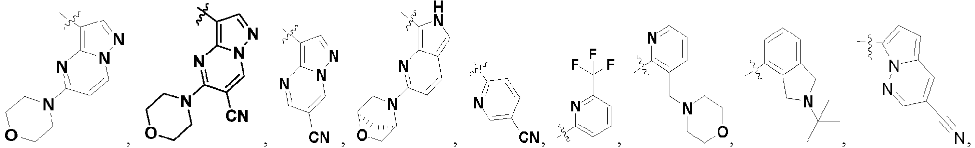 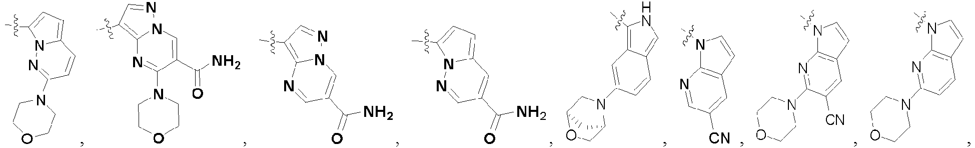 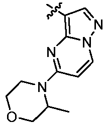 (preferably 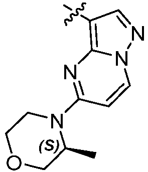 ), 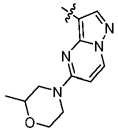 (preferably 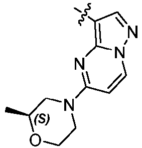 ), 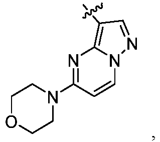 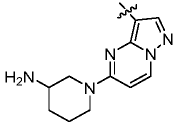 ( 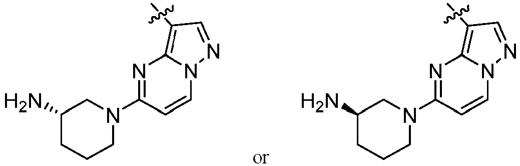 ), 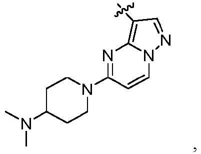 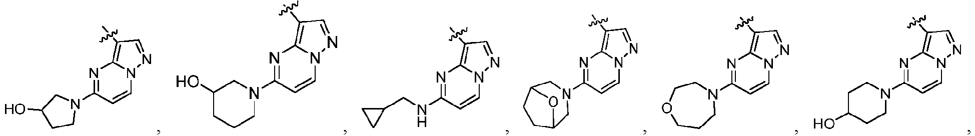 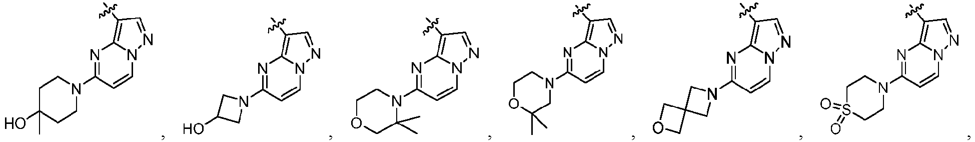 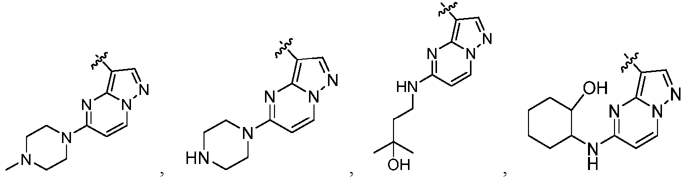 (preferably 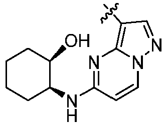 or 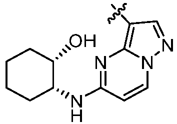 ), 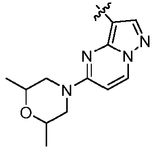 (preferably 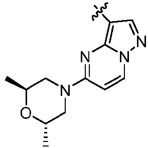 ), 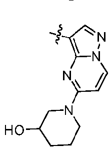 (preferably 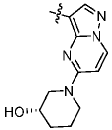 or 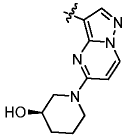 ), and

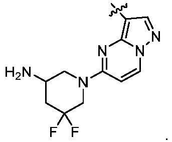

In certain embodiments, B, as a whole, is selected from the group consisting of 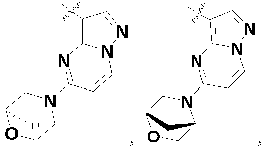 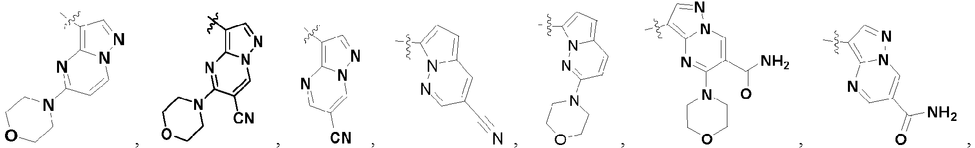 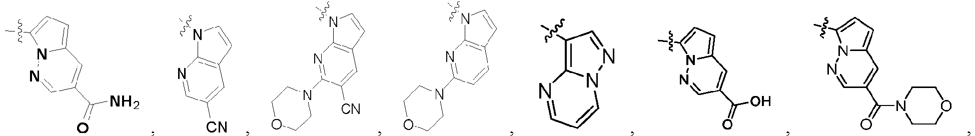 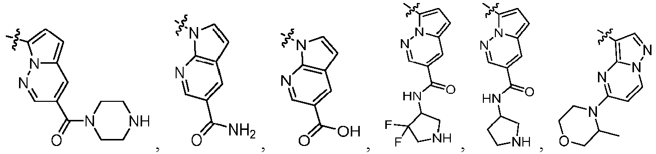 (preferably 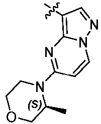 ), 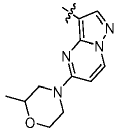 (preferably 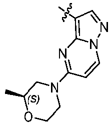 ), 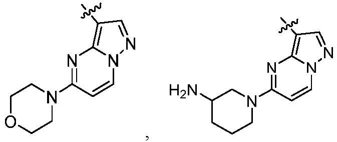 ( 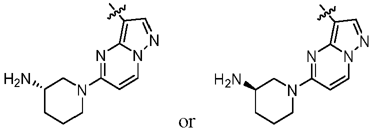 ), 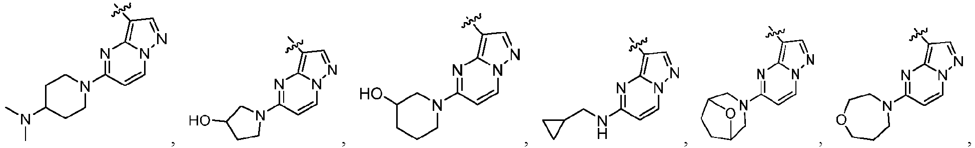 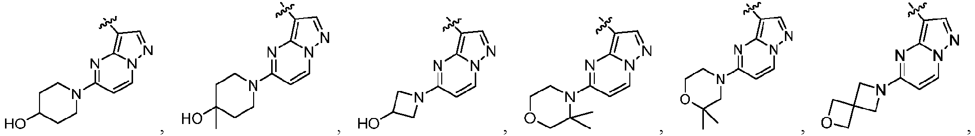 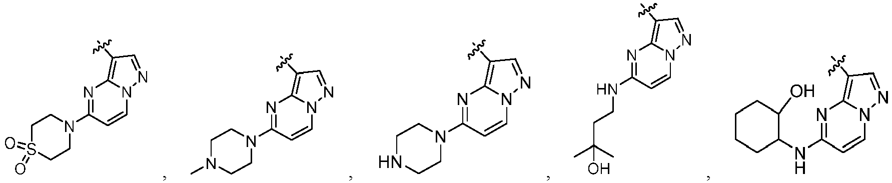 (preferably 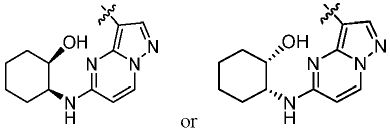 ), 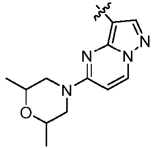 (preferably 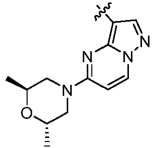 ), 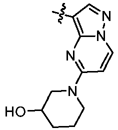 (preferably 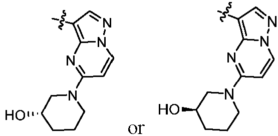 ), and

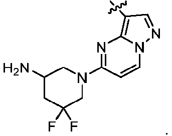

In certain embodiments, B, as a whole, is selected from the group consisting of

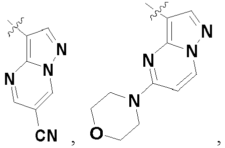

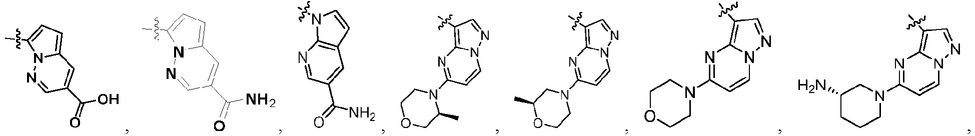

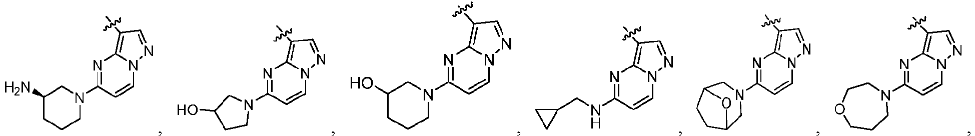

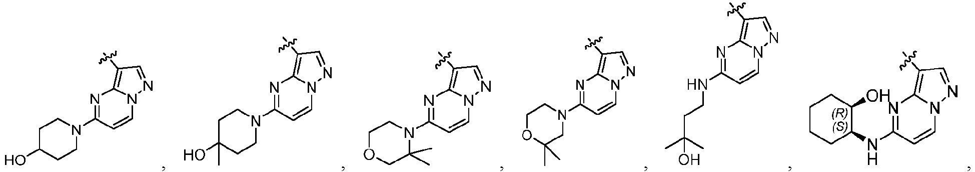

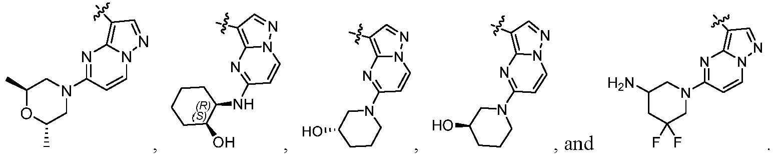

In certain embodiments, L¹ is selected from the group consisting of a direct bond, 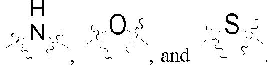 In certain embodiments, L¹ is selected from the group consisting of a direct bond, methylene, and 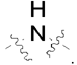 In certain embodiments, L¹ is selected from the group consisting of a direct bond, and 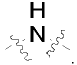 In certain embodiments, L¹ is a direct bond.

In certain embodiments, C is selected from 5-6 membered heteroaryl containing 1-3 heteroatoms, and C is optionally substituted with at least one of substituents R^{d}, R^{e}, R^{f}.

In certain embodiments, C is selected from the group consisting of pyrazolyl, imidazolyl, thiazolyl, pyridinyl, pyrrolyl, oxazolyl, furanyl, 1,2,3-triazolyl, and 1,2,4-triazolyl, and C is optionally substituted with at least one of substituents R^{d}, R^{e}, R^{f}.

In certain embodiments, C is selected from the group consisting of pyrazolyl, thiazolyl, pyridinyl, pyrrolyl, oxazolyl, furanyl, 1,2,3-triazolyl, and 1,2,4-triazolyl, and C is optionally substituted with at least one of substituents R^{d}, R^{e}, R^{f}.

In certain embodiments, R^{d}, R^{e}, R^{f} are each independently selected from the group consisting of H, C1-C6 alkyl, C1-C6 haloalkyl (such as difluoromethyl), C3-C7 cycloalkyl, 6-10 membered aryl, 5-6 membered heteroaryl, 4-7 membered saturated heterocyclyl, and 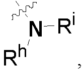 wherein, the C3-C7 cycloalkyl, 6-10 membered aryl, 5-6 membered heteroaryl, and 4-7 membered saturated heterocyclyl are each independently optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl.

In certain embodiments, R^{d} is selected from the group consisting of C1-C6 alkyl, C3-C6 cycloalkyl, phenyl, pyridinyl, morpholinyl, piperazinyl, and piperidyl, wherein, the C3-C6 cycloalkyl, phenyl, pyridinyl, morpholinyl, piperazinyl, and piperidyl are each independently optionally substituted with 1-2 substituents selected from the group consisting of C1-C6 alkyl, halogen, alkoxy (such as C1-C6 alkoxy), hydroxy, amino, hydroxy C1-C6 alkyl (such as methylol), amino C1-C6 alkyl, and formyl.

In certain embodiments, R^{d} is selected from the group consisting of C3-C6 cycloalkyl, phenyl, morpholinyl, and piperidyl, wherein, the C3-C6 cycloalkyl, phenyl, morpholinyl, and piperidyl are each independently optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl.

In certain embodiments, R^{d} is selected from the group consisting of C1-C6 alkyl, 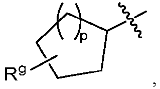 phenyl, morpholinyl, piperazinyl, and piperidyl, wherein, the 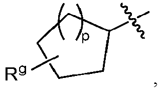 phenyl, morpholinyl, piperazinyl, and piperidyl are optionally substituted with C1-C6 alkyl (preferably methyl), wherein p is selected from the group consisting of 0, 1, and 2, R^{g} is selected from the group consisting of H, amino, hydroxy, hydroxy C1-C6 alkyl, and formyl.

In certain embodiments, R^{d} is selected from the group consisting of 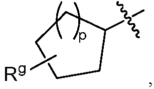 phenyl, morpholinyl, and piperidyl, wherein p is selected from the group consisting of 0, 1, and 2, R^{g} is selected from the group consisting of H, amino, hydroxy, hydroxy C1-C6 alkyl, and formyl.

In certain embodiments, R^{d} is selected from the group consisting of methyl, ethyl, isopropyl, 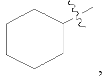 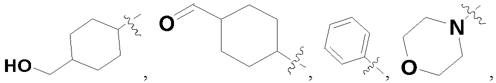 piperazinyl, piperidyl,

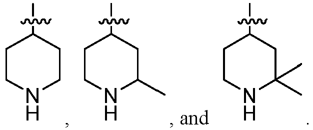

In certain embodiments, R^{d} is selected from the group consisting of 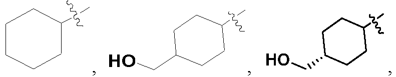 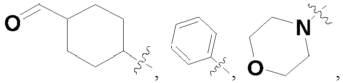 and piperazinyl.

In certain embodiments, R^{d} is selected from the group consisting of

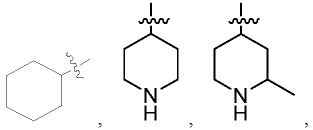

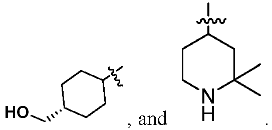

In certain embodiments, R^{h}, Rⁱ are each independently selected from the group consisting of H, methyl, ethyl, isopropyl, and cyclopropyl.

In certain embodiments, R^{e} is selected from the group consisting of H, methyl, 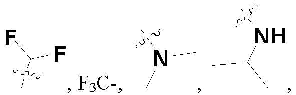 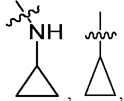 and -NHCH₃.

In certain embodiments, R^{e} is selected from the group consisting of H, methyl,

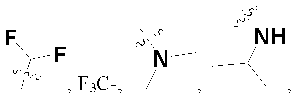

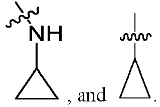

In certain embodiments, R^{e}, R^{f} and the carbon atoms to which they are attached, respectively, together form a substituted or unsubstituted 5-9 membered heterocycle, wherein, the substituent is selected from the group consisting of C1-C6 alkyl (such as ethyl), alkoxy (such as C1-C6 alkoxy), C3-C6 cycloalkyl, amino, and hydroxy.

In certain embodiments, R^{e}, R^{f} and the carbon atoms to which they are attached, respectively, together form 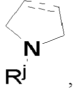 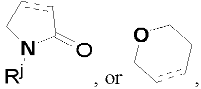 wherein each R^{j} is independently selected from the group consisting of H, C1-C6 alkyl, alkoxy (such as C1-C6 alkoxy), C3-C6 cycloalkyl, amino, and hydroxy.

In certain embodiments, R^{e}, R^{f} and the carbon atoms to which they are attached, respectively, together form

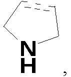

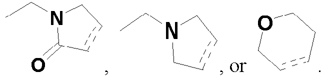

In certain embodiments, R², R³ are each independently selected from the group consisting of H, methyl, ethyl, propyl, and

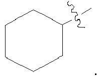

In certain embodiments, R², R³ and the N atom to which they are both attached together form

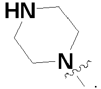

In certain embodiments, R¹, as a whole, is selected from the group consisting of:

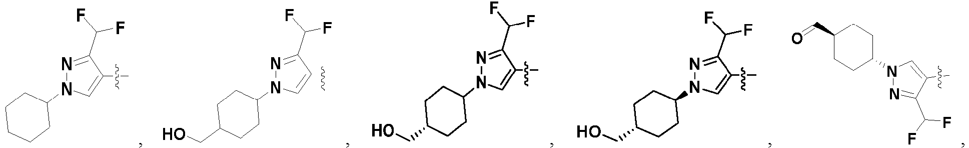

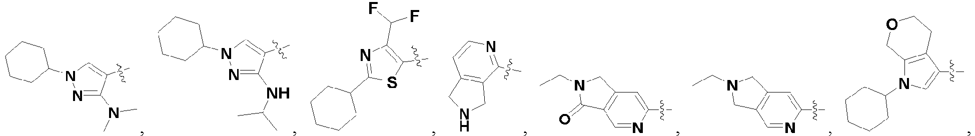

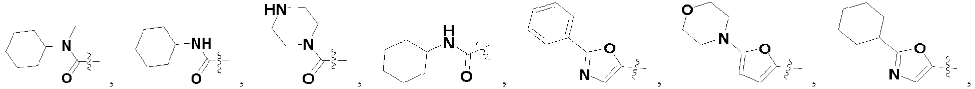

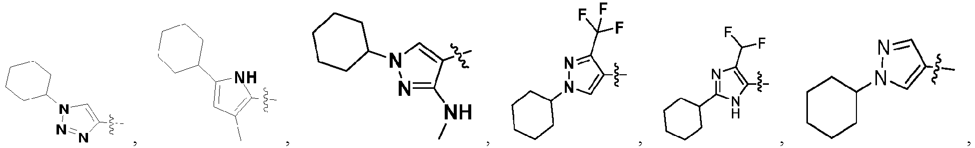

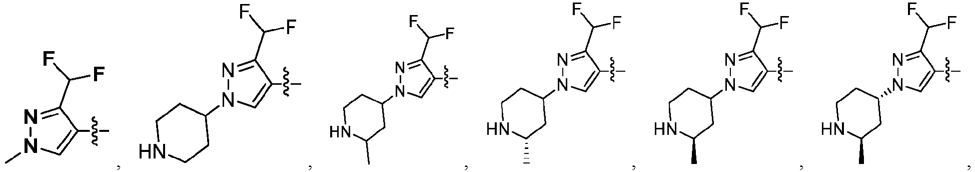

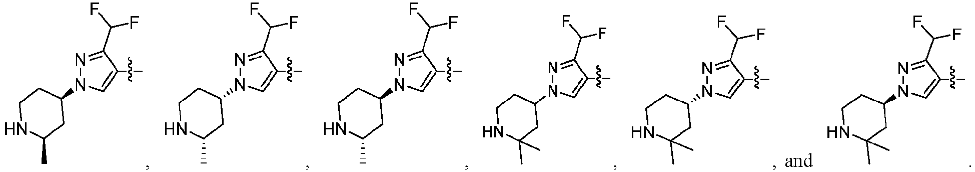

In certain embodiments, R¹, as a whole, is selected from the group consisting of:

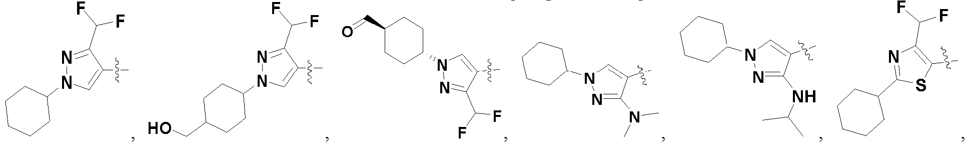

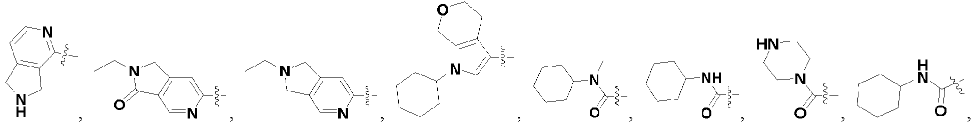

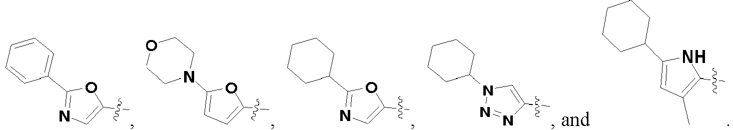

In certain embodiments, R¹, as a whole, is selected from the group consisting of:

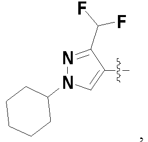

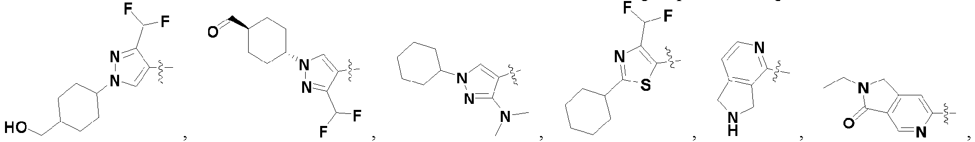

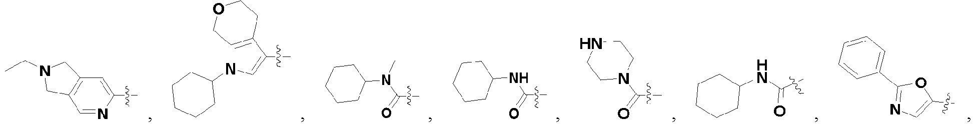

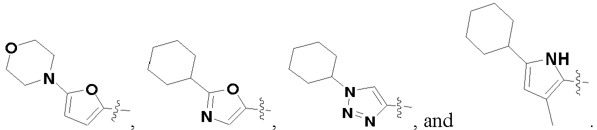

In certain embodiments, R¹, as a whole, is selected from the group consisting of

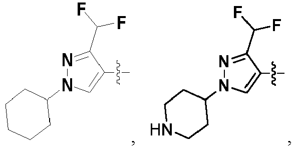

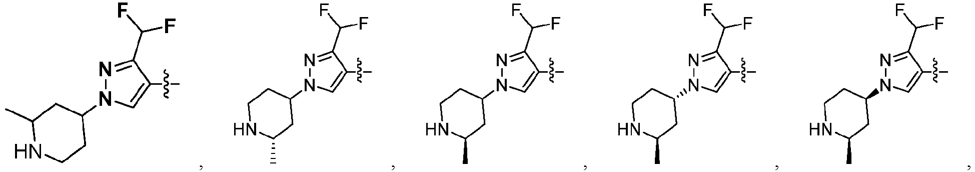

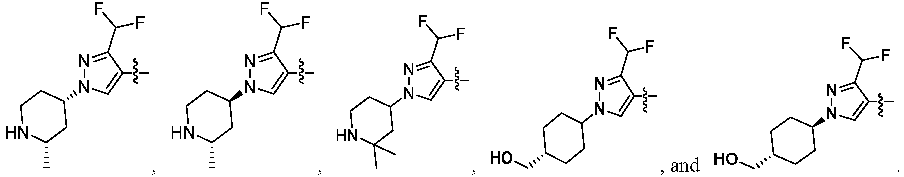

In the second aspect, the present invention provides a compound represented by formula i, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, 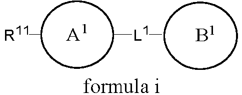 wherein:
A¹ is selected from 5 membered heteroaromatic ring optionally substituted with substituent R^{k}; preferably, A^{l} is selected from 5 membered heteroaromatic ring containing 2-3 heteroatoms, wherein at least two heteroatoms are N atoms; further preferably, A¹ is selected from the group consisting of 1,2,3-triazole ring, 1,2,4-triazole ring, pyrazole ring, imidazole ring, 1,3,4-thiadiazole ring, and 1,3,4-oxadiazole ring,
further preferably, A¹ is selected from the group consisting of 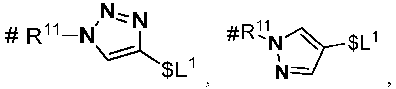 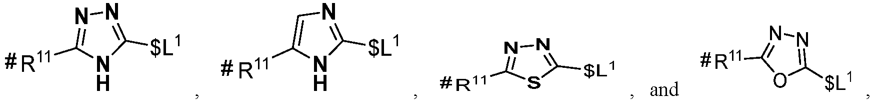 wherein #R¹¹ represents the attachment point to R¹¹, $L¹ represents the attachment point to L¹,
further preferably, A¹ is selected from the group consisting of 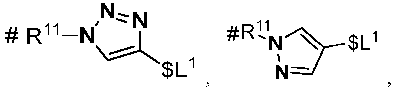 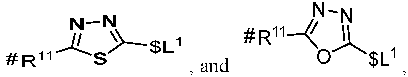 wherein #R¹¹ represents the attachment point to R¹¹, $L¹ represents the attachment point to L¹,
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl; preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl;
further preferably, R^{k} is selected from the group consisting of isopropyl, cyclopropyl, dimethylamino, difluoromethyl, and 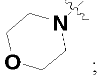
R^{p}, R^{q} are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
B¹ is selected from the group consisting of 6-10 membered heteroaryl, and 6-10 membered aryl, and B¹ is optionally substituted with at least one of substituents R^{a}, R^{b}; preferably, B¹ is substituted with at least one of substituents R^{a}, R^{b}; preferably, B¹ is selected from the group consisting of 9 membered heteroaryl, pyridinyl, phenyl, and pyrimidinyl, and B¹ is substituted with at least one of substituents R^{a}, R^{b}; preferably, the 9 membered heteroaryl contains 1-4 heteroatoms selected from the group consisting of N, O, and S atoms; preferably, B¹ is selected from the group consisting of 9 membered heteroaryl, pyridinyl, and phenyl, and B¹ is substituted with at least one of substituents R^{a}, R^{b}; preferably, the 9 membered heteroaryl contains 1-4 heteroatoms selected from the group consisting of N, O, and S atoms;
further preferably, B¹ is selected from the group consisting of 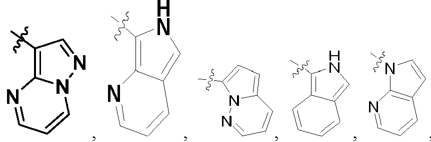 pyridinyl, phenyl, and pyrimidinyl, and B¹ is substituted with at least one of substituents R^{a}, R^{b}; further preferably, B¹ is selected from the group consisting of 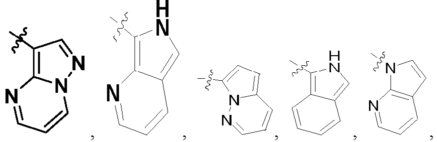 pyridinyl, and phenyl, and B¹ is substituted with at least one of substituents R^{a}, R^{b}; further preferably, B¹ is selected from the group consisting of 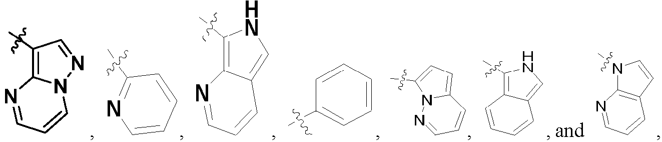 and B¹ is substituted with substituents R^{a}, R^{b};
further preferably, B¹ is selected from the group consisting of 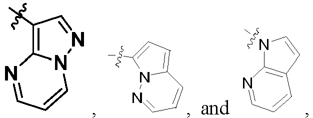 and B¹ is substituted with substituents R^{a}, R^{b}; R^{a} is selected from the group consisting of H, halogen, cyano, carboxyl, 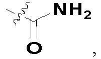 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, 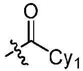 (wherein Cy₁ is 4-9 membered heterocyclyl optionally substituted with R_{cy1} and R_{cy2}), and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), and 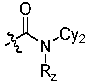 (wherein R_{z} is H or C1-C6 alkyl, Cy₂ is 5-7 membered heterocyclyl, C3-C7 cycloalkyl or C6-C10 aryl, wherein, the 5-7 membered heterocyclyl, C3-C7 cycloalkyl and C6-C10 aryl are optionally substituted with 1-3 substituents selected from the group consisting of halogen, C1-C6 alkyl, C1-C6 alkoxy, CN, and hydroxy), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl), wherein, the C4-C9 cycloalkyl, and 4-9 membered saturated heterocyclyl are optionally substituted with C1-C6 alkyl, halogen, hydroxy, cyano;
preferably, Cy₁ is 5-7 membered heterocyclyl, further preferably, Cy₁ is 6 membered heterocyclyl, further preferably, Cy₁ is morpholinyl, piperazinyl, or thiomorpholinyl;
R_{cy1} and R_{cy2} are each independently selected from the group consisting of C1-C6 alkyl, C1-C6 alkoxy, C1-C6 haloalkyl, halogen, hydroxy, nitro, cyano, amino, -NH(C1-C6 alkyl), -N(C1-C6 alkyl)(C1-C6 alkyl), and oxo; preferably, R_{cy1} and R_{cy2} are each independently selected from the group consisting of C1-C6 alkyl, halogen, hydroxy, amino, and oxo, further preferably, R_{cy1} and R_{cy2} are oxo;
preferably, Cy₂ is 5-7 membered heterocyclyl optionally substituted with 1-3 substituents selected from the group consisting of halogen, C1-C6 alkyl, C1-C6 alkoxy, CN, and hydroxy; further preferably, Cy₂ is selected from the group consisting of azetidinyl, pyrrolidinyl, imidazolidinyl, piperidyl, piperazinyl, and morpholinyl, wherein, the azetidinyl, pyrrolidinyl, imidazolidinyl, piperidyl, piperazinyl, and morpholinyl are optionally substituted with 1-3 substituents selected from the group consisting of halogen, and C1-C6 alkyl; further preferably, Cy₂ is selected from the group consisting of pyrrolidinyl, imidazolidinyl, and piperidyl, wherein, the pyrrolidinyl, imidazolidinyl, and piperidyl are optionally substituted with 1-3 substituents selected from the group consisting of F, Cl, Br, and methyl; further preferably, Cy₂ is pyrrolidinyl optionally substituted with 1-3 (such as 1 or 2 or 3) F;
preferably, R^{a} is selected from the group consisting of H, halogen, cyano, carboxyl, 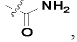 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of hydroxy, C3-C6 cycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, halogen, hydroxy, hydroxy-C1-C6 alkyl-, cyano, oxo, - NH₂, -N(C1-C6 alkyl), and -N(C1-C6 alkyl)(C1-C6 alkyl),
preferably, R^{a} is selected from the group consisting of H, cyano, carboxyl, 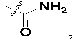 C1-C6 haloalkyl (such as trifluoromethyl), 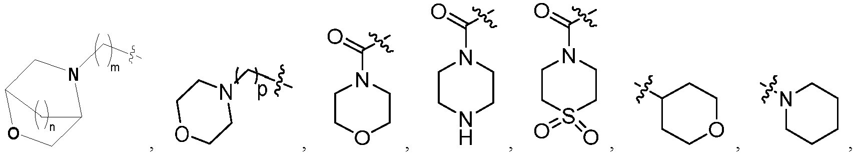 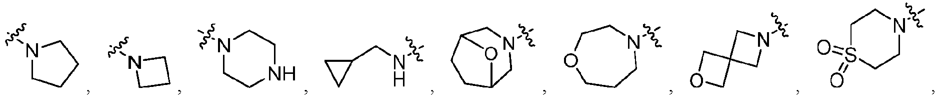 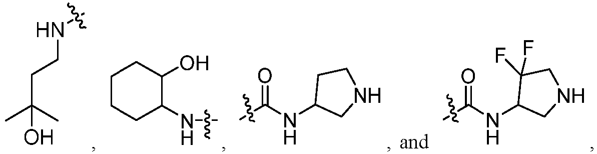 wherein, the 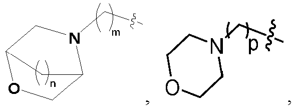 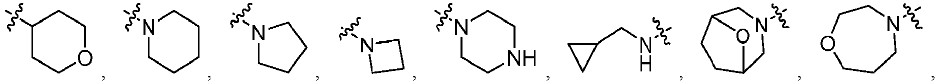 and 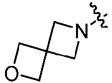 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl);
preferably, R^{a} is selected from the group consisting of H, cyano, carboxyl, 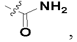 C1-C6 haloalkyl (such as trifluoromethyl), 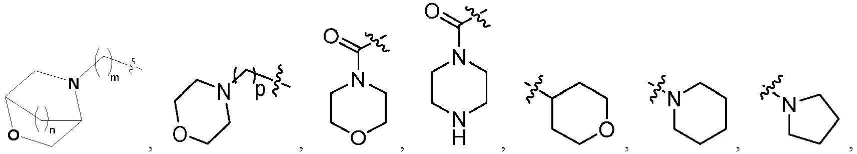 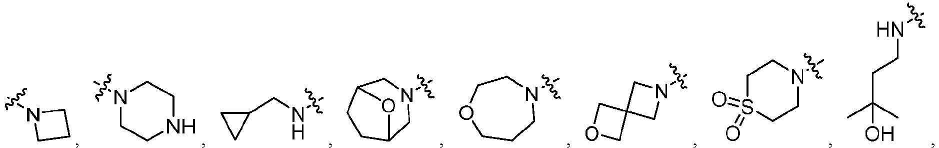 and 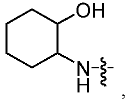 wherein, the 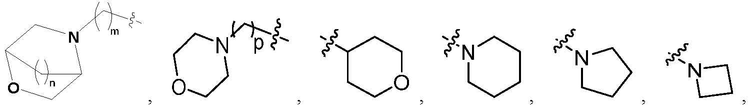 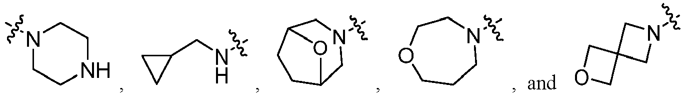 are optionally substituted with 1-3 substituents selected from the group consisting of C1-C6 alkyl, halogen, hydroxy, cyano, -NH₂, -N(C1-C6 alkyl), and - N(C1-C6 alkyl)(C1-C6 alkyl);
preferably, R^{a} is selected from the group consisting of H, cyano, 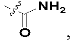 C1-C6 haloalkyl (such as trifluoromethyl), and 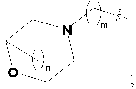 wherein, the 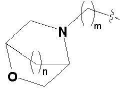 is optionally substituted with C1-C6 alkyl (preferably methyl), halogen (such as F, Cl, Br);
m is selected from the group consisting of 0, 1, 2, and 3; preferably, m is selected from the group consisting of 0, and 1; n is selected from the group consisting of 0, 1, 2, and 3; preferably, n is selected from the group consisting of 0, and 1; p is selected from the group consisting of 0, 1, 2, and 3; preferably, p is selected from the group consisting of 0, and 1;
further preferably, R^{a} is selected from the group consisting of H, cyano, carboxyl, 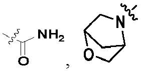 (preferably 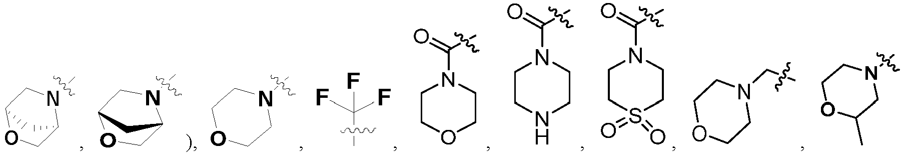 (preferably 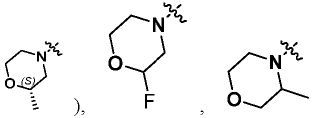 (preferably 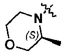 ), 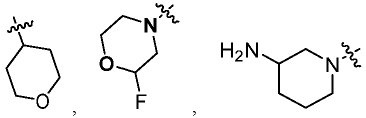 (preferably 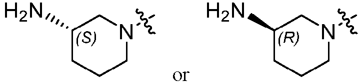 ), 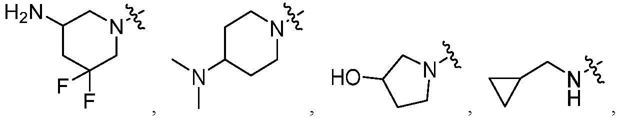 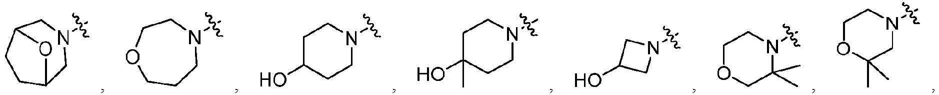 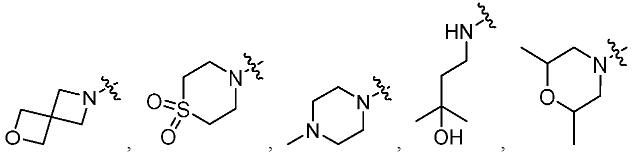 (preferably 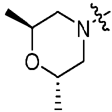 ), 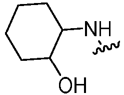 (preferably 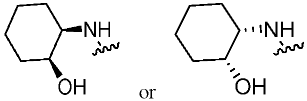 ), 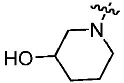 (preferably 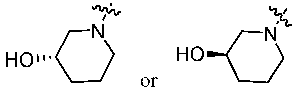 ), 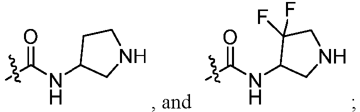
further preferably, R^{a} is selected from the group consisting of H, cyano, 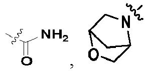 (preferably 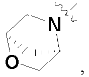 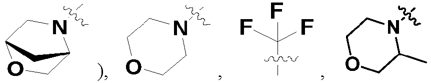 (preferably 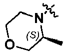 ), 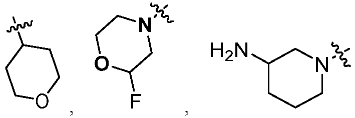 (preferably 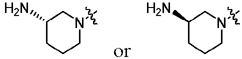 ), 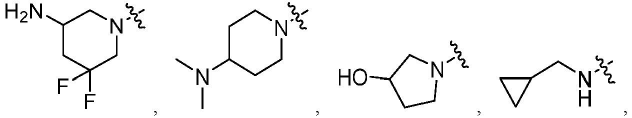 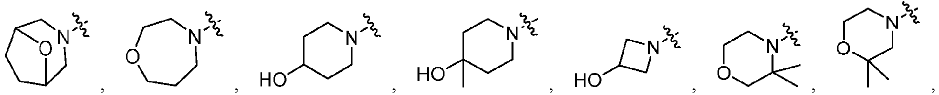 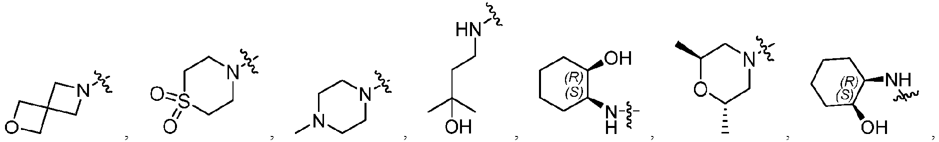 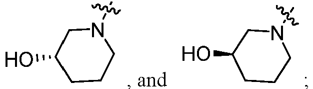
R^{b} is selected from the group consisting of H, halogen, amino, cyano, carboxyl, 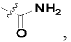 C1-C6 alkyl, C1-C6 haloalkyl (such as trifluoromethyl), and 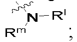
preferably, R^{b} is selected from the group consisting of H, cyano, 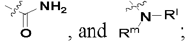
R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl,
or, R^{a}, R^{b} and the carbon atoms to which they are attached, respectively, together form a substituted or unsubstituted 5-membered heterocycle, wherein, the substituent is selected from C1-C6 alkyl (such as tert-butyl); preferably, R^{a}, R^{b} and the carbon atoms to which they are attached, respectively, together form 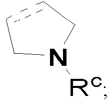
R^{c} is selected from the group consisting of H, and C1-C6 alkyl, preferably, R^{c} is selected from C1-C6 alkyl,
further preferably, B¹, as a whole, is selected from the group consisting of: 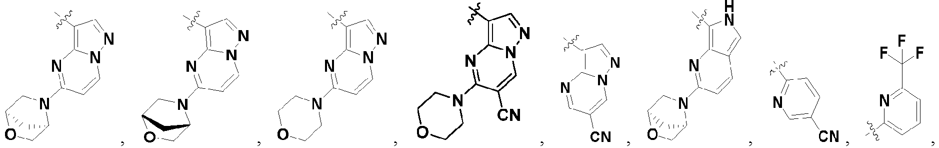 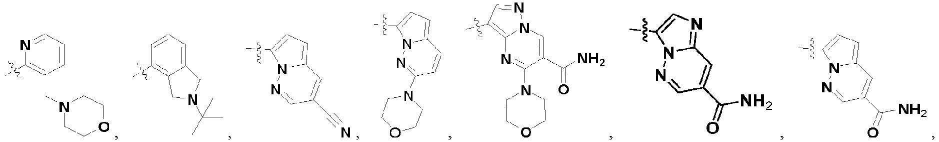 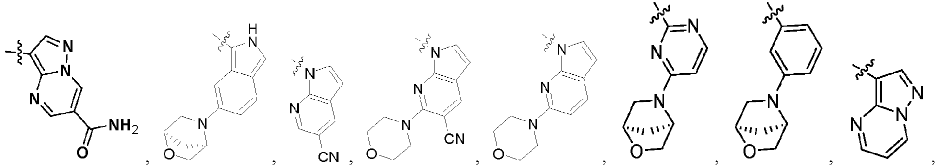 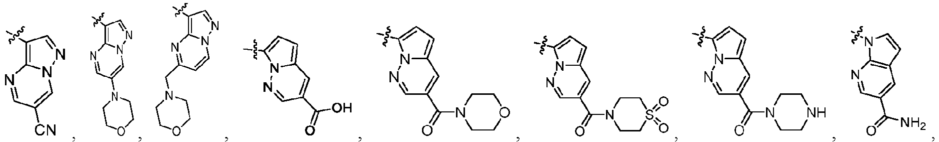 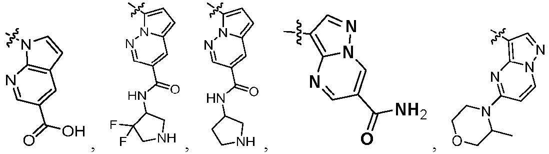 (preferably 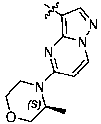 ), 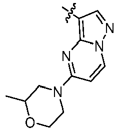 (preferably 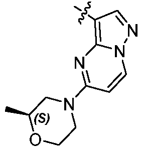 ), 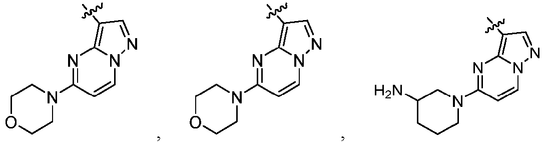 ( 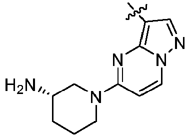 or 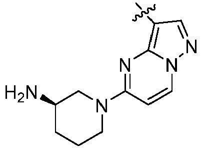 ), 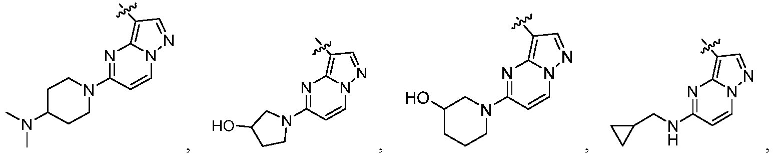 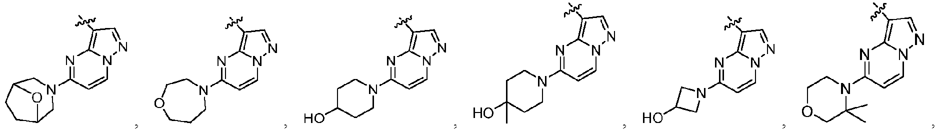 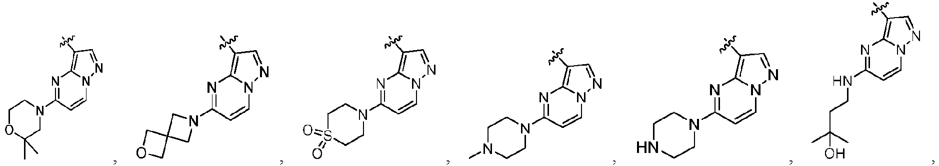 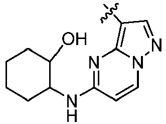 (preferably 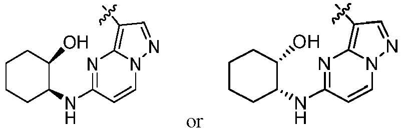 ), 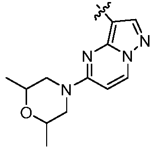 (preferably 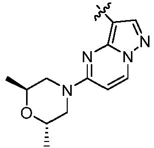 ), 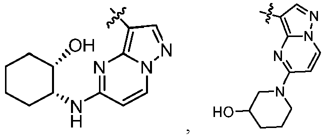 (preferably 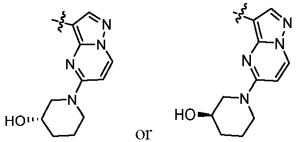 ), and 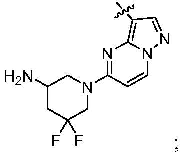
further preferably, B¹, as a whole, is selected from the group consisting of: 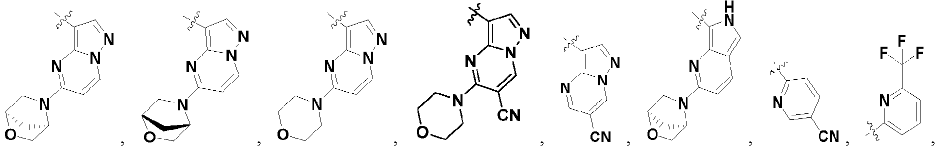 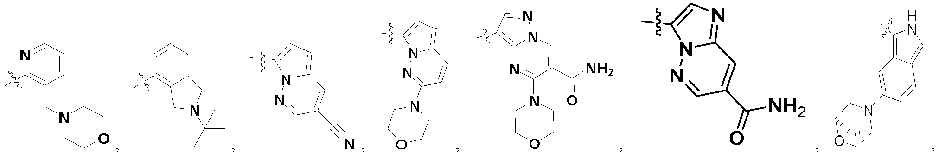 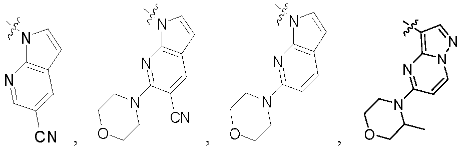 (preferably 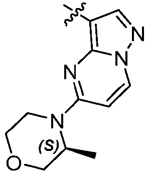 ), 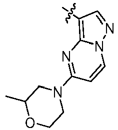 (preferably 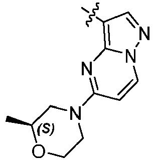 ), 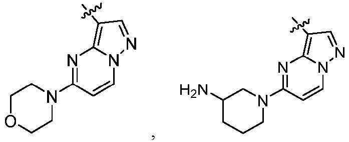 ( 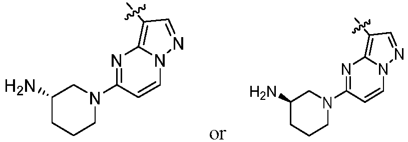 ), 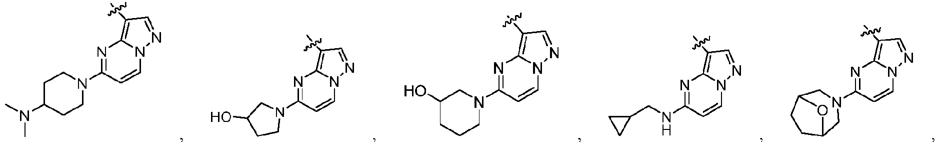 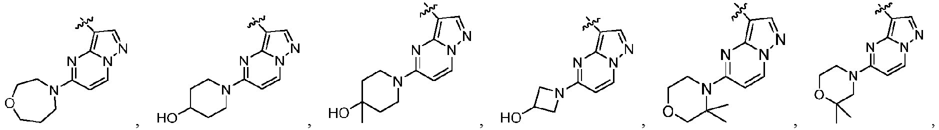 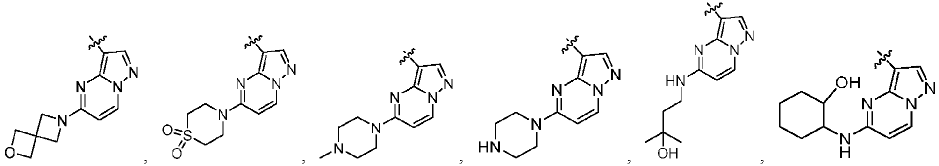 (preferably 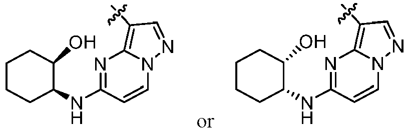 ), 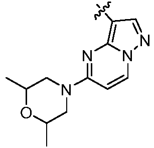 (preferably 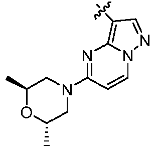 ), 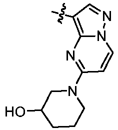 (preferably 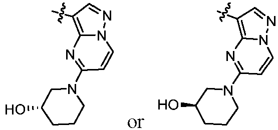 ), and 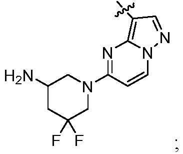
further preferably, B¹, as a whole, is selected from the group consisting of 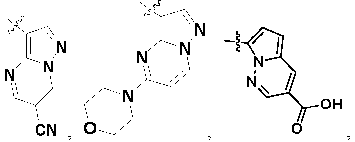 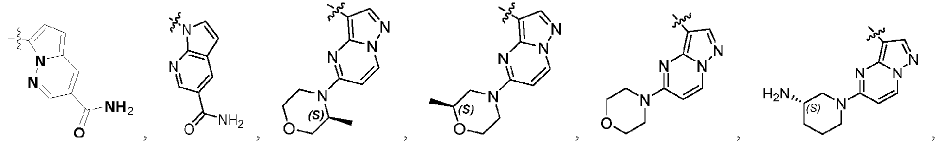 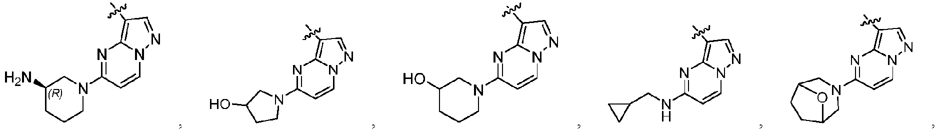 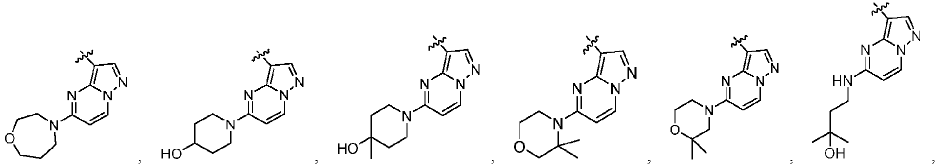 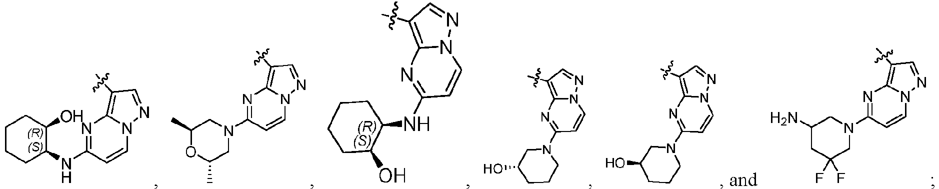
L¹ is selected from the group consisting of a direct bond, C1-C6 alkyl, 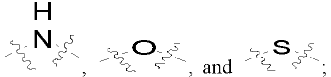 preferably, L¹ is selected from the group consisting of a direct bond, 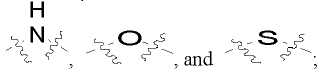 preferably, L¹ is selected from the group consisting of a direct bond, methylene, and 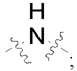 preferably, L¹ is selected from the group consisting of a direct bond, and 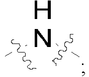
R¹¹ is selected from the group consisting of 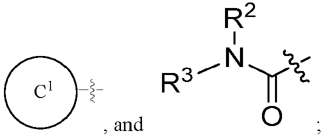
C¹ is selected from 5-6 membered heteroaryl, and C¹ is optionally substituted with at least one of substituents R^{d}, R^{e}, R^{f}; preferably, C¹ is selected from 5-6 membered heteroaryl containing 1-3 heteroatoms, and C¹ is optionally substituted with at least one of substituents R^{d}, R^{e}, R^{f}; further preferably, C¹ is selected from the group consisting of pyrazolyl, imidazolyl, thiazolyl, pyridinyl, and pyrrolyl, and C¹ is optionally substituted with at least one of substituents R^{d}, R^{e}, R^{f}; further preferably, C¹ is selected from the group consisting of pyrazolyl, thiazolyl, pyridinyl, and pyrrolyl, and C¹ is optionally substituted with at least one of substituents R^{d}, R^{e}, R^{f};
R^{d}, R^{e}, R^{f} are each independently selected from the group consisting of H, C1-C6 alkyl, C1-C6 haloalkyl (such as difluoromethyl), C3-C7 cycloalkyl, 4-7 membered saturated heterocyclyl, and 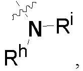 wherein, the C3-C7 cycloalkyl and 4-7 membered saturated heterocyclyl are optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
R^{d}, R^{e}, R^{f} are each independently selected from the group consisting of H, C1-C6 alkyl, C1-C6 haloalkyl (such as difluoromethyl), C3-C7 cycloalkyl, and 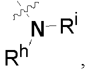 wherein, the C3-C7 cycloalkyl is optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d} is selected from C3-C6 cycloalkyl optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d} is selected from 4-7 membered saturated heterocyclyl optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
further preferably, R^{d} is selected from the group consisting of C1-C6 alkyl, and 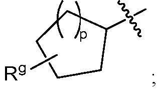 or further preferably, R^{d} is selected from the group consisting of pyrrolyl, piperidyl, imidazolidinyl, and piperazinyl, wherein, the pyrrolyl, piperidyl, imidazolidinyl, and piperazinyl are optionally substituted with 1-2 substituents selected from the group consisting of halogen, C1-C6 alkoxy, and C1-C6 alkyl (preferably methyl); further preferably, R^{d} is selected from 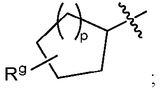
p is selected from the group consisting of 0, 1, and 2;
or further preferably, R^{d} is selected from the group consisting of pyrrolyl, and piperidyl, wherein, the pyrrolyl, and piperidyl are optionally substituted with 1-2 substituents selected from the group consisting of halogen, and C1-C6 alkyl (preferably methyl);
R^{g} is selected from the group consisting of H, amino, hydroxy, hydroxy C1-C6 alkyl, and formyl;
further preferably, R^{d} is selected from the group consisting of methyl, ethyl, isopropyl, 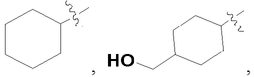 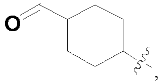 piperidyl, 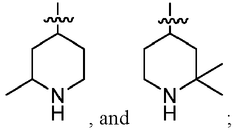
further preferably, R^{d} is selected from the group consisting of 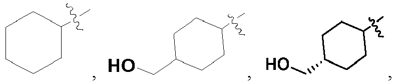 and 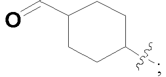
further preferably, R^{d} is selected from the group consisting of 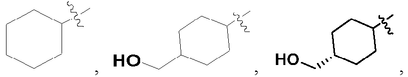 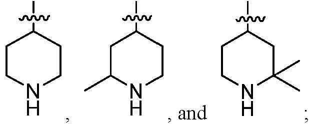
R^{h}, Rⁱ are each independently selected from the group consisting of H, C1-C6 alkyl, and C3-C6 cycloalkyl; preferably, R^{h}, Rⁱ are each independently selected from the group consisting of H, methyl, ethyl, isopropyl, and cyclopropyl;
preferably, R^{e} is selected from the group consisting of H, methyl, 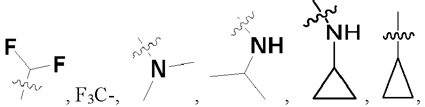 and -NHCH₃; preferably, R^{e} is selected from the group consisting of H, methyl, 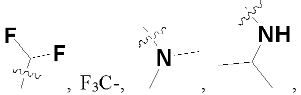 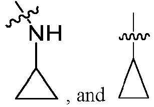
or, R^{e}, R^{f} and the carbon atoms to which they are attached, respectively, together form a substituted or unsubstituted 5-9 membered heterocycle, wherein, the substituent is selected from the group consisting of C1-C6 alkyl (such as ethyl), alkoxy (such as C1-C6 alkoxy), C3-C6 cycloalkyl, amino, and hydroxy,
preferably, R^{e}, R^{f} and the carbon atoms to which they are attached, respectively, together form 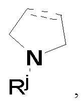 , 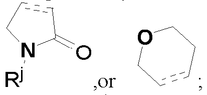
each R^{j} is independently selected from the group consisting of H, C1-C6 alkyl, alkoxy (such as C1-C6 alkoxy), C3-C6 cycloalkyl, amino, and hydroxy;
further preferably, R^{e}, R^{f} and the carbon atoms to which they are attached, respectively, together form 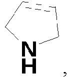 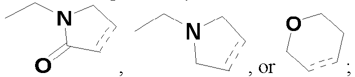
R², R³ are each independently selected from the group consisting of H, C1-C6 alkyl, and C3-C6 cycloalkyl; or, R², R³ and the N atom to which they are both attached together form 5-6 membered saturated heterocyclyl, preferably, the 5-6 membered saturated heterocyclyl contains 2-3 heteroatoms selected from the group consisting of N, S, and O atoms; preferably, R², R³ are each independently selected from the group consisting of H, methyl, ethyl, propyl, and 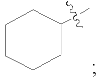
or preferably, R², R³ and the N atom to which they are both attached together form 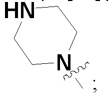
further preferably, R¹¹, as a whole, is selected from the group consisting of: 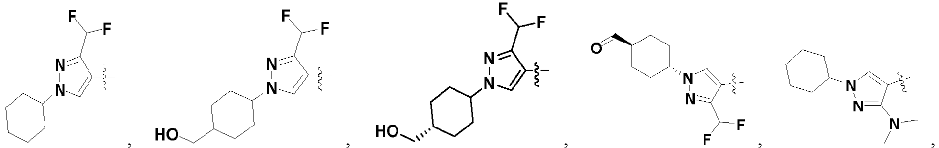 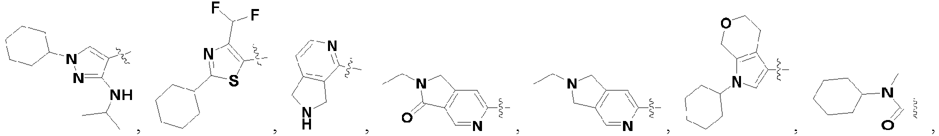 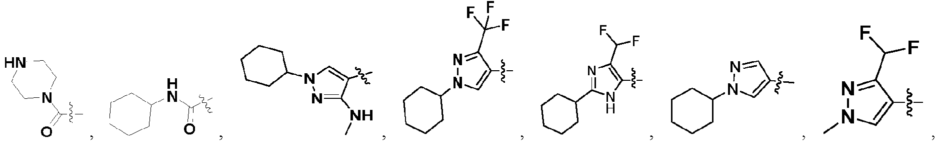 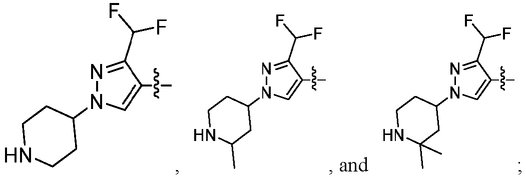
further preferably, R¹¹, as a whole, is selected from the group consisting of: 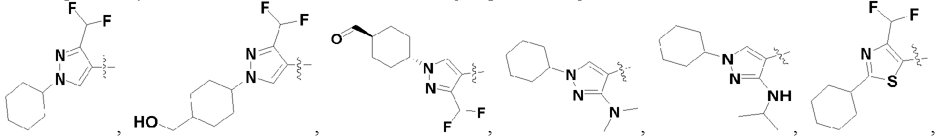 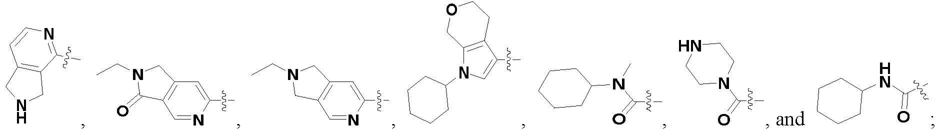
further preferably, R¹¹, as a whole, is seleted from the group consisting of

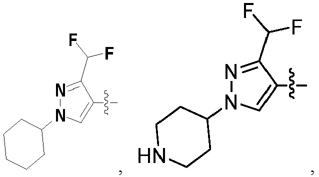

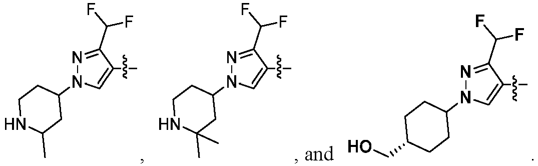

In the third aspect, the present invention provides a compound represented by formula i-1, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, 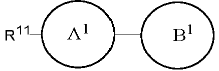 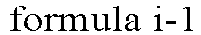 wherein:
A¹ is selected from 5 membered heteroaromatic ring optionally substituted with substituent R^{k}; preferably, A¹ is selected from 5 membered heteroaromatic ring containing 2-3 heteroatoms, wherein at least two heteroatoms are N atoms; further preferably, A¹ is selected from the group consisting of 1,2,3-triazole ring, 1,2,4-triazole ring, pyrazole ring, imidazole ring, 1,3,4-thiadiazole ring, and 1,3,4-oxadiazole ring, further preferably, A¹ is selected from the group consisting of 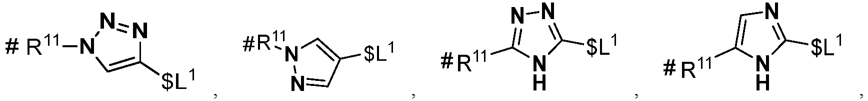 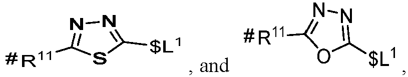 wherein #R¹¹ represents the attachment point to R¹¹, $L¹ represents the attachment point to L¹; further preferably, A¹ is selected from the group consisting of 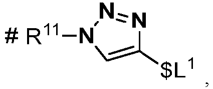 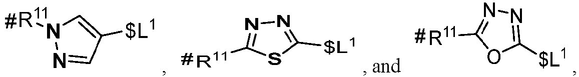 wherein #R¹¹ represents the attachment point to R¹¹, $L¹ represents the attachment point to L¹;
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl; preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl;
R^{p}, R^{q} are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
further preferably, R^{k} is selected from the group consisting of isopropyl, cyclopropyl, dimethylamino, difluoromethyl, and 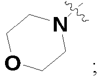
B¹ is selected from the group consisting of 6-10 membered heteroaryl, and 6-10 membered aryl, and B¹ is optionally substituted with at least one of substituents R^{a}, R^{b}; preferably, B¹ is substituted with at least one of substituents R^{a}, R^{b}; preferably, B¹ is selected from the group consisting of 9 membered heteroaryl, pyridinyl, phenyl, and pyrimidinyl, and B¹ is substituted with at least one of substituents R^{a}, R^{b}; preferably, the 9 membered heteroaryl contains 1-4 heteroatoms selected from the group consisting of N, O, and S atoms; preferably, B¹ is selected from the group consisting of 9 membered heteroaryl, pyridinyl, and phenyl, and B¹ is substituted with at least one of substituents R^{a}, R^{b}; preferably, the 9 membered heteroaryl contains 1-4 heteroatoms selected from the group consisting of N, O, and S atoms;
further preferably, B¹ is selected from the group consisting of 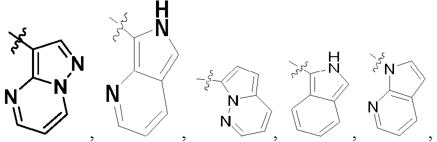 pyridinyl, phenyl, and pyrimidinyl, and B¹ is substituted with at least one of substituents R^{a}, R^{b}; further preferably, B¹ is selected from the group consisting of 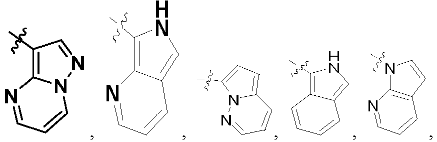 pyridinyl, and phenyl, and B¹ is substituted with at least one of substituents R^{a}, R^{b};
further preferably, B¹ is selected from the group consisting of 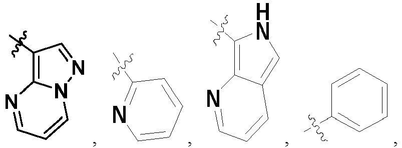 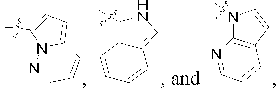 and B¹ is substituted with substituents R^{a}, R^{b}; further preferably, B¹ is selected from the group consisting of 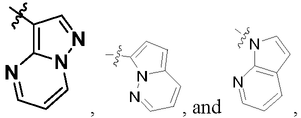 and B¹ is substituted with substituents R^{a}, R^{b}; R^{a} is selected from the group consisting of H, halogen, cyano, carboxyl, 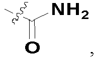 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, 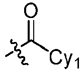 (wherein Cy₁ is 4-9 membered heterocyclyl optionally substituted with R_{cy1} and R_{cy2}), -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), and 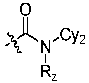 (wherein R_{z} is H or C1-C6 alkyl, Cy₂ is 5-7 membered heterocyclyl, C3-C7 cycloalkyl or C6-C10 aryl, wherein, the 5-7 membered heterocyclyl, C3-C7 cycloalkyl and C6-C10 aryl are optionally substituted with 1-3 substituents selected from the group consisting of halogen, C1-C6 alkyl, C1-C6 alkoxy, CN, and hydroxy), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂,- N(C1-C6 alkyl), and -N(C1-C6 alkyl)(C1-C6 alkyl), wherein, the C4-C9 cycloalkyl, and 4-9 membered saturated heterocyclyl are optionally substituted with C1-C6 alkyl, halogen, hydroxy, cyano;
preferably, Cy₁ is 5-7 membered heterocyclyl, further preferably, Cy₁ is 6 membered heterocyclyl, further preferably, Cy₁ is morpholinyl, piperazinyl, thiomorpholinyl;
R_{cy1} and R_{cy2} are each independently selected from the group consisting of C1-C6 alkyl, C1-C6 alkoxy, C1-C6 haloalkyl, halogen, hydroxy, nitro, cyano, amino, -NH(C1-C6 alkyl), -N(C1-C6 alkyl)(C1-C6 alkyl), and oxo;
preferably, R_{cy1} and R_{cy2} are each independently selected from the group consisting of C1-C6 alkyl, halogen, hydroxy, amino, and oxo, further preferably R_{cy1} and R_{cy2} are oxo;
preferably, Cy₂ is 5-7 membered heterocyclyl optionally substituted with 1-3 substituents selected from the group consisting of halogen, C1-C6 alkyl, C1-C6 alkoxy, CN, and hydroxy; further preferably, Cy₂ is selected from the group consisting of azetidinyl, pyrrolidinyl, imidazolidinyl, piperidyl, piperazinyl, and morpholinyl, wherein, the azetidinyl, pyrrolidinyl, imidazolidinyl, piperidyl, piperazinyl, and morpholinyl are optionally substituted with 1-3 substituents selected from the group consisting of halogen, and C1-C6 alkyl; further preferably, Cy₂ is selected from the group consisting of pyrrolidinyl, imidazolidinyl, and piperidyl, wherein, the pyrrolidinyl, imidazolidinyl, and piperidyl are optionally substituted with 1-3 substituents selected from the group consisting of F, Cl, Br, and methyl; further preferably, Cy₂ is pyrrolidinyl optionally substituted with 1-3 (such as 1 or 2 or 3) F;
preferably, R^{a} is selected from the group consisting of H, halogen, cyano, carboxyl, 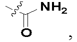 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of hydroxy, C3-C6 cycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, halogen, hydroxy, hydroxy-C1-C6 alkyl-, cyano, oxo, - NH₂, -N(C1-C6 alkyl), and -N(C1-C6 alkyl)(C1-C6 alkyl);
preferably, R^{a} is selected from the group consisting of H, cyano, carboxyl, 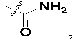 C1-C6 haloalkyl (such as trifluoromethyl), 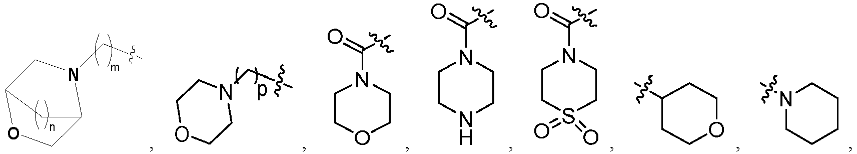 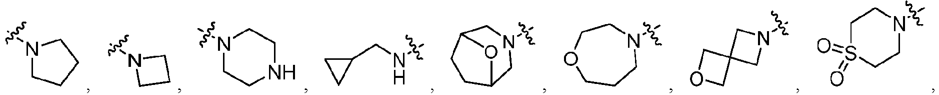 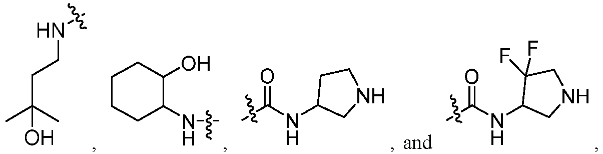 wherein, the 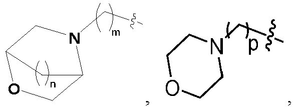 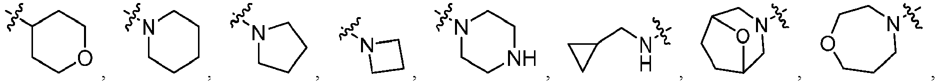 and 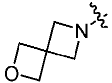 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl),
preferably, R^{a} is selected from the group consisting of H, cyano, carboxyl, 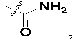 C1-C6 haloalkyl (such as trifluoromethyl), 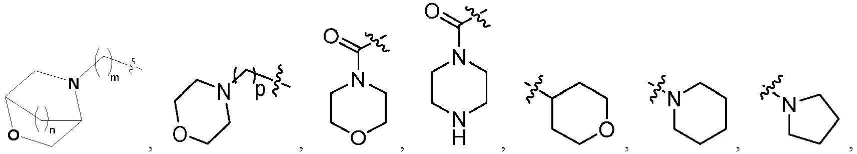 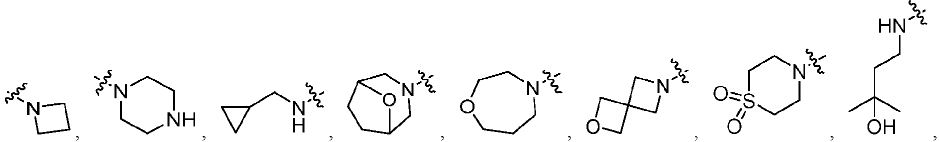 and 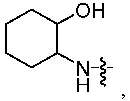 wherein, the 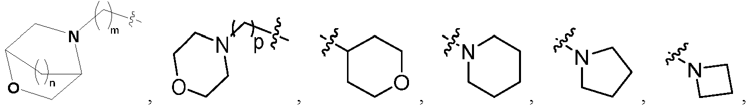 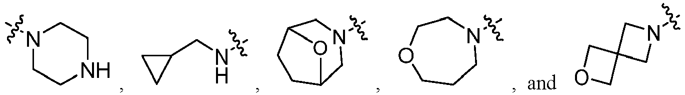 are optionally substituted with 1-3 substituents selected from the group consisting of C1-C6 alkyl, halogen, hydroxy, cyano, -NH₂, -N(C1-C6 alkyl), and - N(C1-C6 alkyl)(C1-C6 alkyl);
preferably, R^{a} is selected from the group consisting of H, cyano, 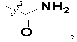 C1-C6 haloalkyl (such as trifluoromethyl), and 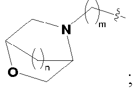 wherein, the 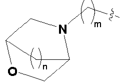 is optionally substituted with C1-C6 alkyl (preferably methyl), halogen (such as F, Cl, Br);
m is selected from the group consisting of 0, 1, 2, and 3; preferably, m is selected from the group consisting of 0, and 1; n is selected from the group consisting of 0, 1, 2, and 3; preferably, n is selected from the group consisting of 0, and 1; p is selected from the group consisting of 0, 1, 2, and 3; preferably, p is selected from the group consisting of 0, and 1;
further preferably, R^{a} is selected from the group consisting of H, cyano, carboxyl, 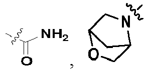 (preferably 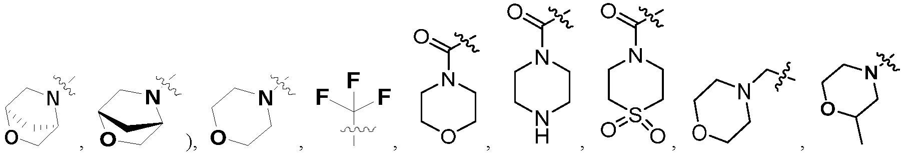 (preferably 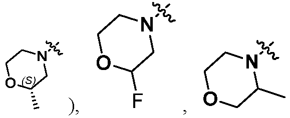 (preferably 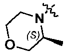 ), 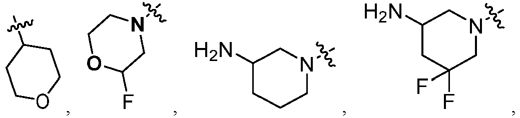 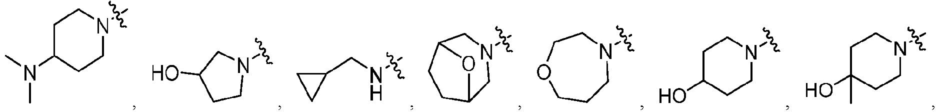 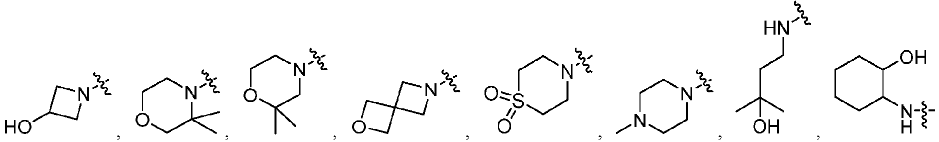 (preferably 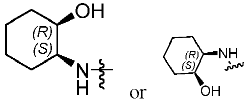 ), 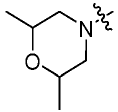 (preferably 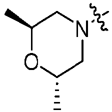 ), 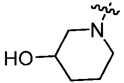 (preferably 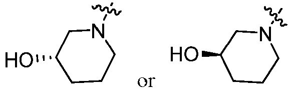 ), 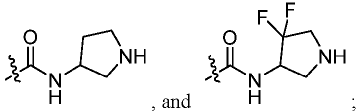
further preferably, R^{a} is selected from the group consisting of H, cyano, 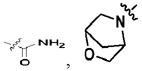 (preferably 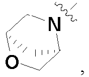 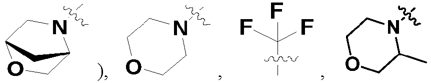 (preferably 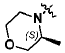 ), 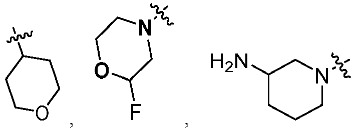 (preferably 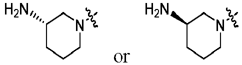 ), 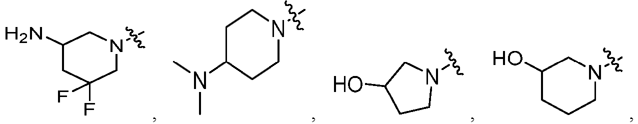 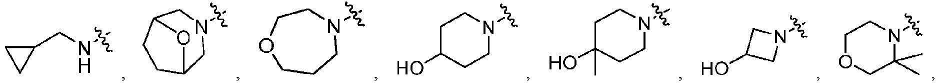 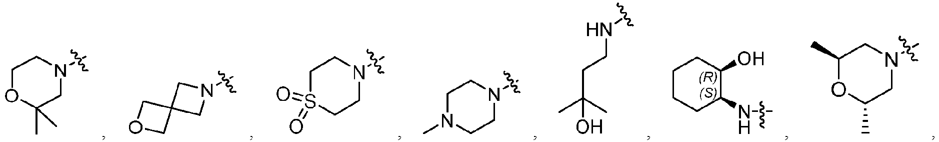 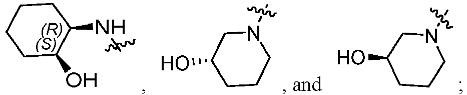
R^{b} is selected from the group consisting of H, halogen, amino, cyano, carboxyl, 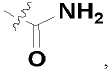 C1-C6 alkyl, C1-C6 haloalkyl (such as trifluoromethyl), and 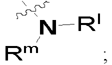
preferably, R^{b} is selected from the group consisting of H, cyano, 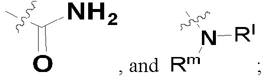
R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl;
or, R^{a}, R^{b} and the carbon atoms to which they are attached, respectively, together form a substituted or unsubstituted 5-membered heterocycle, wherein, the substituent is selected from C1-C6 alkyl (such as tert-butyl);
preferably, R^{a}, R^{b} and the carbon atoms to which they are attached, respectively, together form 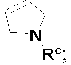
R^{c} is selected from the group consisting of H, and C1-C6 alkyl, preferably, R^{c} is selected from C1-C6 alkyl;
further preferably, B¹, as a whole, is selected from the group consisting of: 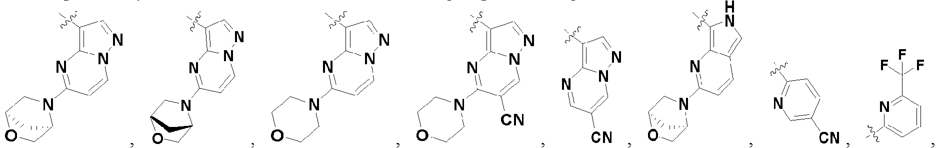 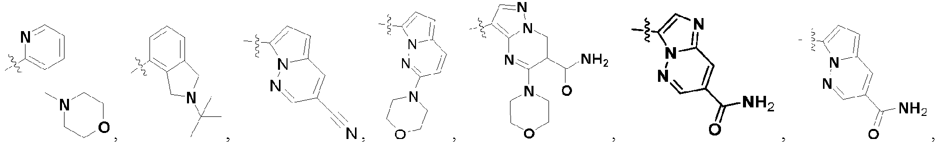 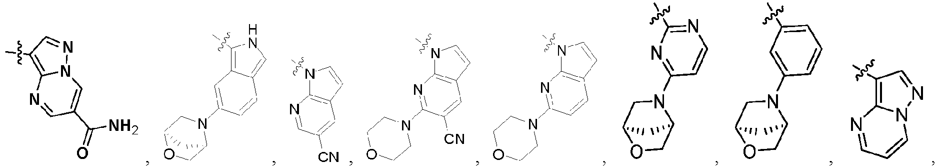 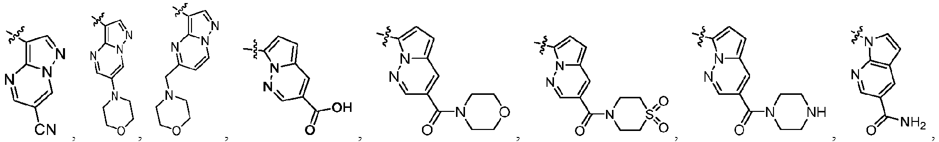 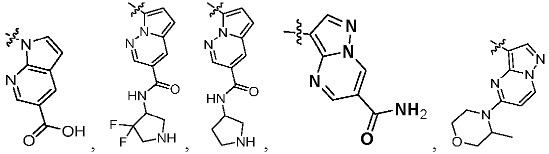 (preferably 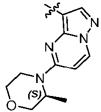 ), 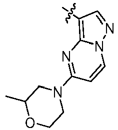 (preferably 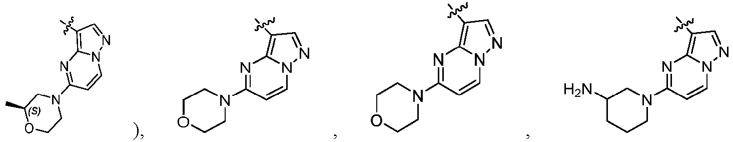 ( 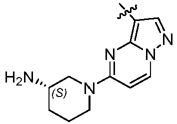 or 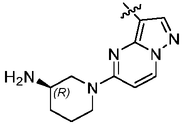 ), 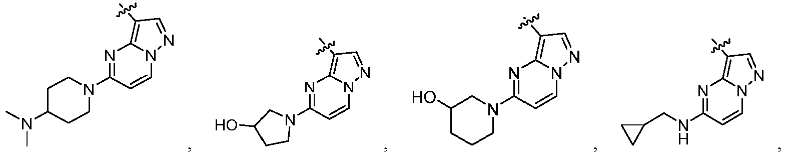 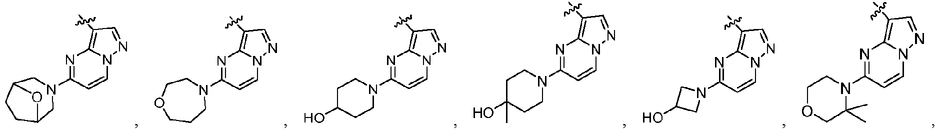 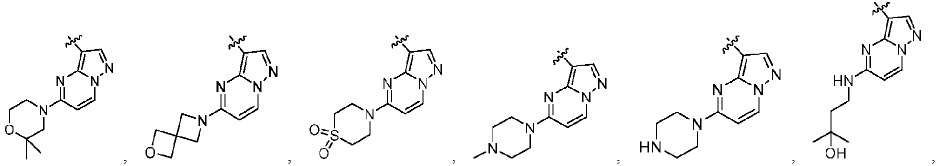 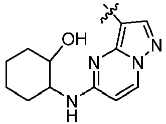 (preferably 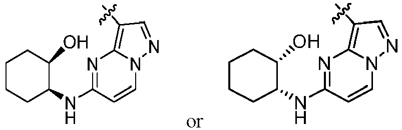 ), 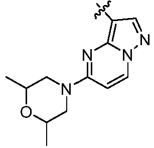 (preferably 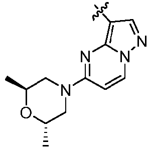 ), 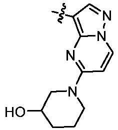 (preferably 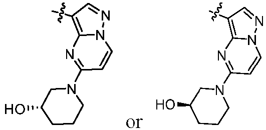 ), and 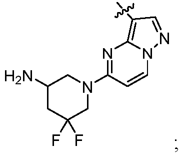
further preferably, B¹, as a whole, is selected from the group consisting of: 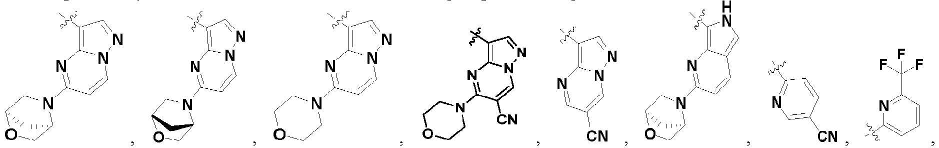 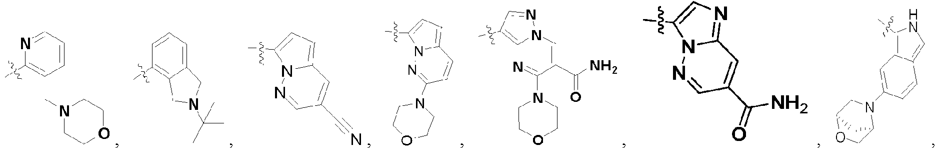 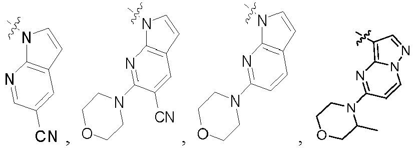 (preferably 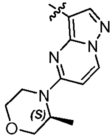 ), 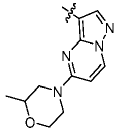 (preferably 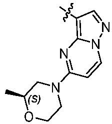 ), 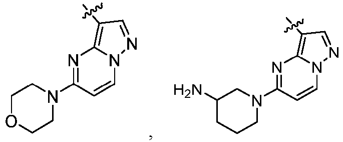 ( 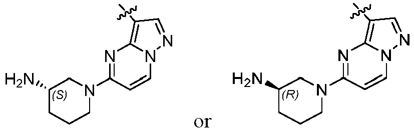 ), 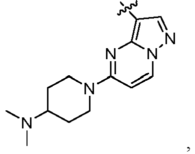 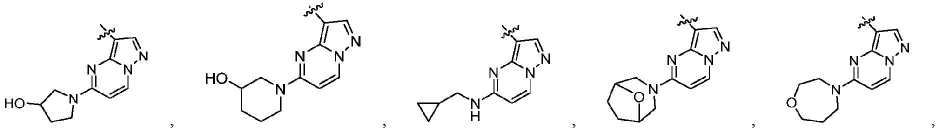 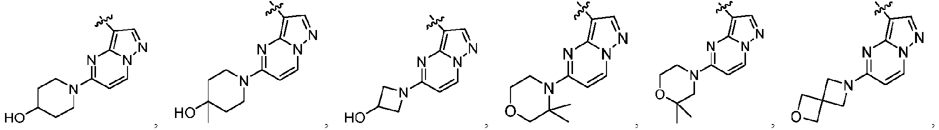 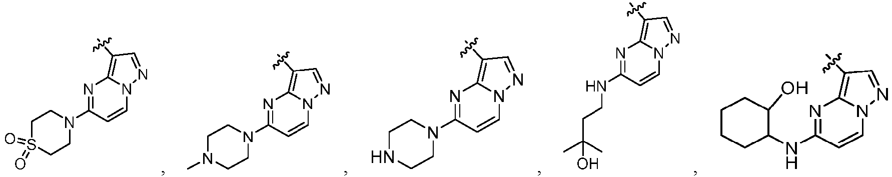 (preferably 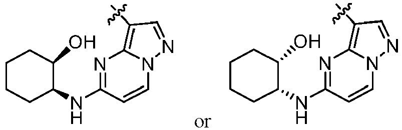 ), 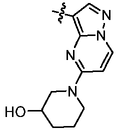 (preferably 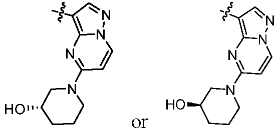 ), and 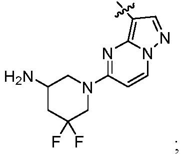
further preferably, B¹, as a whole, is selected from the group consisting of 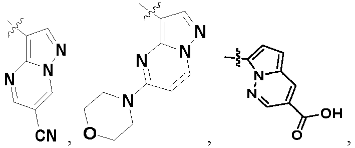 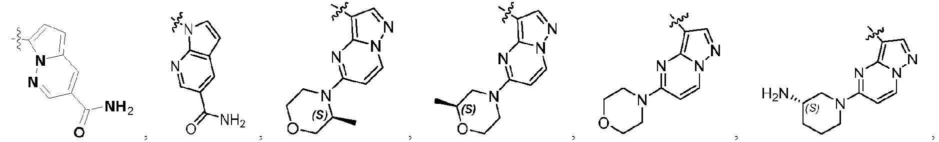 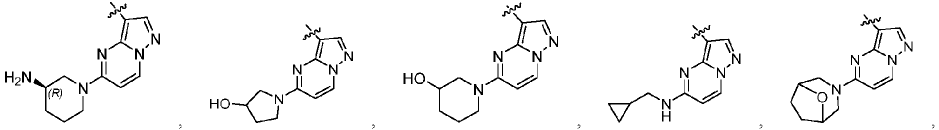 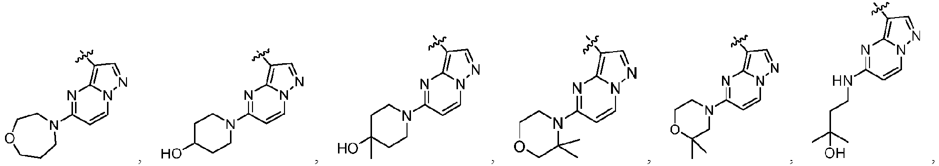 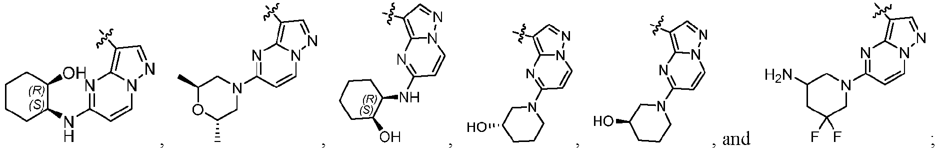
L¹ is selected from the group consisting of a direct bond, C1-C6 alkyl, 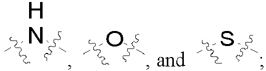 preferably, L¹ is selected from the group consisting of a direct bond, 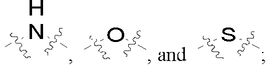 preferably, L¹ is selected from the group consisting of a direct bond, methylene, and 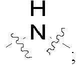 preferably, L¹ is selected from the group consisting of a direct bond, and 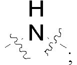
R¹¹ is selected from the group consisting of 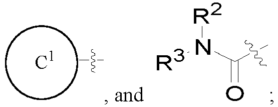
C¹ is selected from 5-6 membered heteroaryl, and C¹ is optionally substituted with at least one of substituents R^{d}, R^{e}, R^{f}; preferably, C¹ is selected from 5-6 membered heteroaryl containing 1-3 heteroatoms, and C¹ is optionally substituted with at least one of substituents R^{d}, R^{e}, R^{f}; further preferably, C¹ is selected from the group consisting of pyrazolyl, imidazolyl, thiazolyl, pyridinyl, and pyrrolyl, and C¹ is optionally substituted with at least one of substituents R^{d}, R^{e}, R^{f}; further preferably, C¹ is selected from the group consisting of pyrazolyl, thiazolyl, pyridinyl, and pyrrolyl, and C¹ is optionally substituted with at least one of substituents R^{d}, R^{e}, R^{f};
R^{d}, R^{e}, R^{f} are each independently selected from the group consisting of H, C1-C6 alkyl, C1-C6 haloalkyl (such as difluoromethyl), C3-C7 cycloalkyl, 4-7 membered saturated heterocyclyl, and 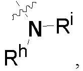 wherein, the C3-C7 cycloalkyl and 4-7 membered saturated heterocyclyl are optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
R^{d}, R^{e}, R^{f} are each independently selected from the group consisting of H, C1-C6 alkyl, C1-C6 haloalkyl (such as difluoromethyl), C3-C7 cycloalkyl, and 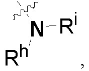 wherein, the C3-C7 cycloalkyl is optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d} is selected from C3-C6 cycloalkyl optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d} is selected from 4-7 membered saturated heterocyclyl optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
further preferably, R^{d} is selected from the group consisting of C1-C6 alkyl, and 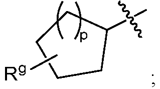
or further preferably, R^{d} is selected from the group consisting of pyrrolyl, piperidyl, imidazolidinyl, and piperazinyl, wherein, the pyrrolyl, piperidyl, imidazolidinyl, and piperazinyl are optionally substituted with 1-2 substituents selected from the group consisting of halogen, C1-C6 alkoxy, and C1-C6 alkyl (preferably methyl);
further preferably, R^{d} is selected from 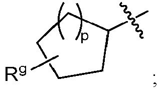 p is selected from the group consisting of 0, 1, and 2;
or further preferably, R^{d} is selected from the group consisting of pyrrolyl, and piperidyl, wherein, the pyrrolyl, and piperidyl are optionally substituted with 1-2 substituents selected from the group consisting of halogen, and C1-C6 alkyl (preferably methyl);
R^{g} is selected from the group consisting of H, amino, hydroxy, hydroxy C1-C6 alkyl, and formyl;
further preferably, R^{d} is selected from the group consisting of methyl, ethyl, isopropyl, 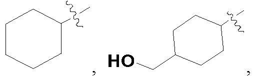 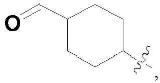 piperidyl, 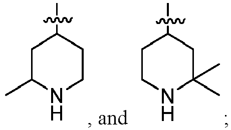 further preferably, R^{d} is selected from the group consisting of 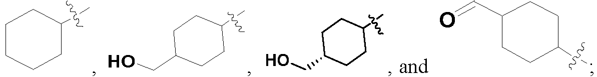 further preferably, R^{d} is selected from the group consisting of 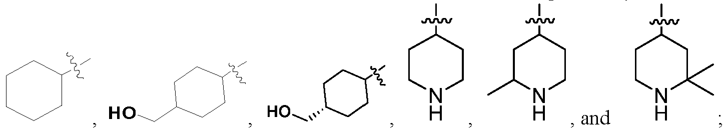
R^{h}, Rⁱ are each independently selected from the group consisting of H, C1-C6 alkyl, and C3-C6 cycloalkyl; preferably, R^{h}, Rⁱ are each independently selected from the group consisting of H, methyl, ethyl, isopropyl, and cyclopropyl;
preferably, R^{e} is selected from the group consisting of H, methyl, 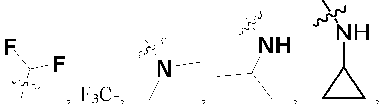 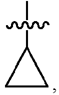 and -NHCH₃; preferably, R^{e} is selected from the group consisting of H, methyl, 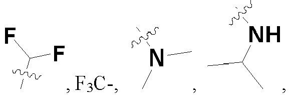 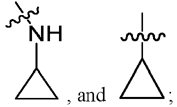
or, R^{e}, R^{f} and the carbon atoms to which they are attached, respectively, together form a substituted or unsubstituted 5-9 membered heterocycle, wherein, the substituent is selected from the group consisting of C1-C6 alkyl (such as ethyl), alkoxy (such as C1-C6 alkoxy), C3-C6 cycloalkyl, amino, and hydroxy,
preferably, R^{e}, R^{f} and the carbon atoms to which they are attached, respectively, together form 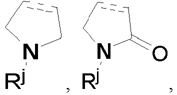 or 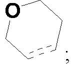 each R^{j} is independently selected from the group consisting of H, C1-C6 alkyl, alkoxy (such as C1-C6 alkoxy), C3-C6 cycloalkyl, amino, and hydroxy;
further preferably, R^{e}, R^{f} and the carbon atoms to which they are attached, respectively, together form 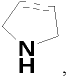 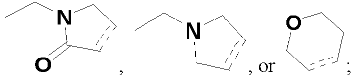
R², R³ are each independently selected from the group consisting of H, C1-C6 alkyl, and C3-C6 cycloalkyl; or, R², R³ and the N atom to which they are both attached together form 5-6 membered saturated heterocyclyl, preferably, the 5-6 membered saturated heterocyclyl contains 2-3 heteroatoms selected from the group consisting of N, O, and S atoms; preferably, R², R³ are each independently selected from the group consisting of H, methyl, ethyl, propyl, and 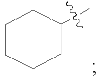
or preferably, R², R³ and the N atom to which they are both attached together form 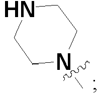
further preferably, R¹¹, as a whole, is selected from the group consisting of: 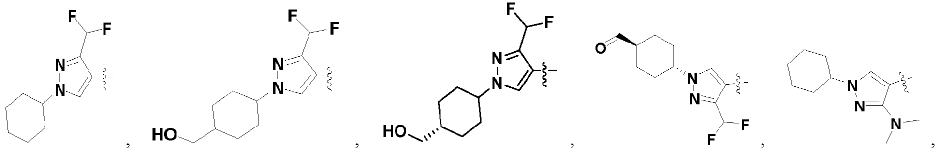 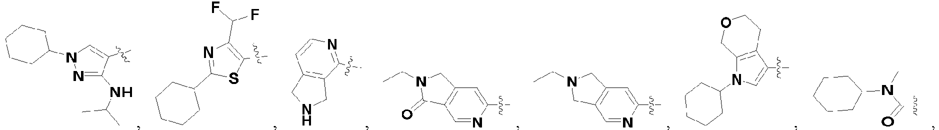 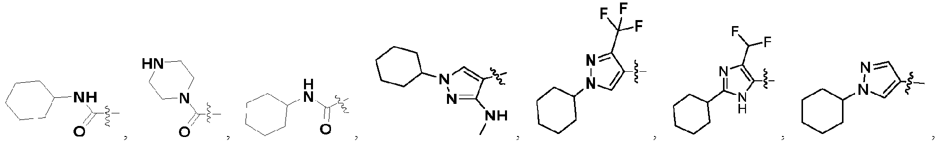 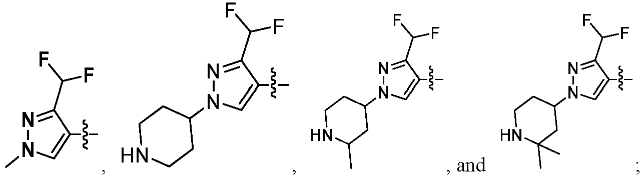
further preferably, R¹¹, as a whole, is selected from the group consisting of: 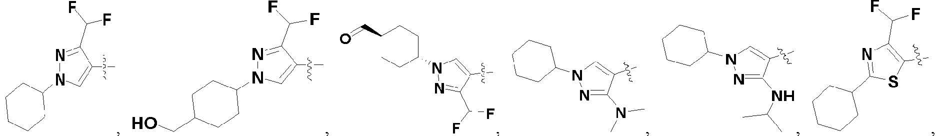 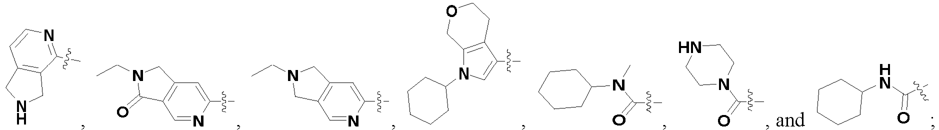
further preferably, R¹¹, as a whole, is selected from the group consisting of

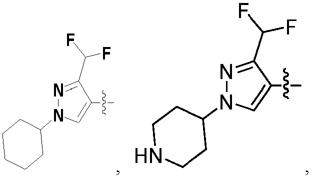

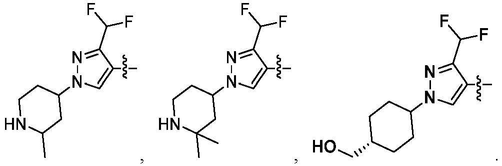

In the fourth aspect, the present invention provides a compound represented by formula i-1-1, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, 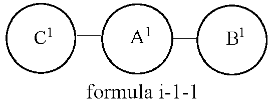 wherein:
A¹ is selected from 5 membered heteroaromatic ring optionally substituted with substituent R^{k}; preferably, A¹ is selected from 5 membered heteroaromatic ring containing 2-3 heteroatoms, wherein at least two heteroatoms are N atoms; further preferably, A¹ is selected from the group consisting of 1,2,3-triazole ring, 1,2,4-triazole ring, pyrazole ring, imidazole ring, 1,3,4-thiadiazole ring, and 1,3,4-oxadiazole ring,
further preferably, A¹ is selected from the group consisting of 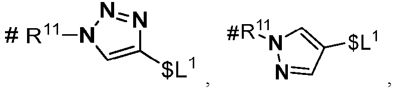 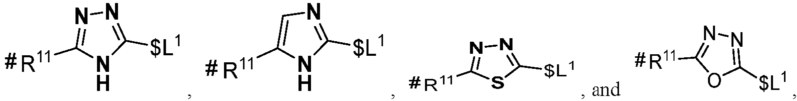 wherein #R¹¹ represents the attachment point to R¹¹, $L¹ represents the attachment point to L¹, further preferably, A¹ is selected from the group consisting of 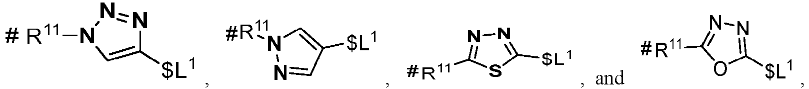 wherein #R¹¹ represents the attachment point to R¹¹, $L¹ represents the attachment point to L¹;
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl; preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl);
further preferably, R^{k} is selected from the group consisting of C1-C6 alkyl, C3-C6 cycloalkyl, R^{p}R^{q}N-, C1-C6 haloalkyl, and morpholinyl;
R^{p}, R^{q} are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
further preferably, R^{k} is selected from the group consisting of isopropyl, cyclopropyl, dimethylamino, difluoromethyl, and 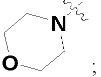
B¹ is selected from the group consisting of 6-10 membered heteroaryl, and 6-10 membered aryl, and B¹ is optionally substituted with at least one of substituents R^{a}, R^{b}; preferably, B¹ is substituted with at least one of substituents R^{a}, R^{b}; preferably, B¹ is selected from the group consisting of 9 membered heteroaryl, pyridinyl, phenyl, and pyrimidinyl, and B¹ is substituted with at least one of substituents R^{a}, R^{b}; preferably, the 9 membered heteroaryl contains 1-4 heteroatoms selected from the group consisting of N, O, and S atoms; preferably, B¹ is selected from the group consisting of 9 membered heteroaryl, pyridinyl, and phenyl, and B¹ is substituted with at least one of substituents R^{a}, R^{b}; preferably, the 9 membered heteroaryl contains 1-4 heteroatoms selected from the group consisting of N, O, and S atoms; further preferably, B¹ is selected from the group consisting of 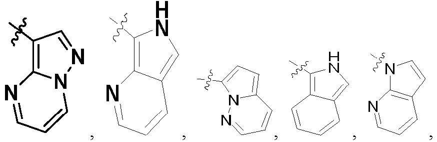 pyridinyl, phenyl, and pyrimidinyl, and B¹ is substituted with at least one of substituents R^{a}, R^{b}; further preferably, B¹ is selected from the group consisting of 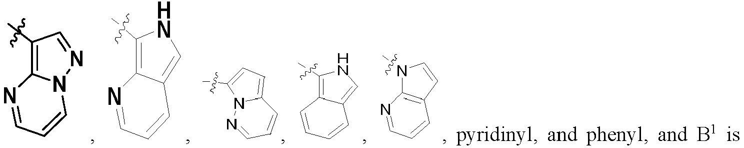 substituted with at least one of substituents R^{a}, R^{b};
further preferably, B¹ is selected from the group consisting of 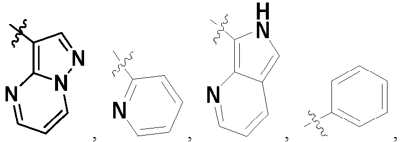 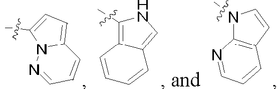 and B¹ is substituted with substituents R^{a}, R^{b};
further preferably, B¹ is selected from the group consisting of 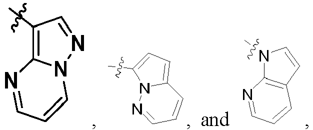 and B¹ is substituted with substituents R^{a}, R^{b}; R^{a} is selected from the group consisting of H, halogen, cyano, carboxyl, 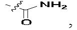 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, 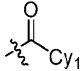 (wherein Cy₁ is 4-9 membered heterocyclyl optionally substituted with R_{cy1} and R_{cy2}), -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), and 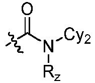 (wherein R_{z} is H or C1-C6 alkyl, Cy₂ is 5-7 membered heterocyclyl, C3-C7 cycloalkyl or C6-C10 aryl, wherein, the 5-7 membered heterocyclyl, C3-C7 cycloalkyl and C6-C10 aryl are optionally substituted with 1-3 substituents selected from the group consisting of halogen, C1-C6 alkyl, C1-C6 alkoxy, CN, and hydroxy), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl), wherein, the C4-C9 cycloalkyl, and 4-9 membered saturated heterocyclyl are optionally substituted with C1-C6 alkyl, halogen, hydroxy, cyano;
preferably, Cy₁ is 5-7 membered heterocyclyl, further preferably, Cy₁ is 6 membered heterocyclyl, further preferably, Cy₁ is morpholinyl, piperazinyl, or thiomorpholinyl ;
R_{cy1} and R_{cy2} are each independently selected from the group consisting of C1-C6 alkyl, C1-C6 alkoxy, C1-C6 haloalkyl, halogen, hydroxy, nitro, cyano, amino, -NH(C1-C6 alkyl), -N(C1-C6 alkyl)(C1-C6 alkyl), and oxo; preferably, R_{cy1} and R_{cy2} are each independently selected from the group consisting of C1-C6 alkyl, halogen, hydroxy, amino, and oxo, further preferably R_{cy1} and R_{cy2} are oxo;
preferably, Cy₂ is 5-7 membered heterocyclyl optionally substituted with 1-3 substituents selected from the group consisting of halogen, C1-C6 alkyl, C1-C6 alkoxy, CN, and hydroxy; further preferably, Cy₂ is selected from the group consisting of azetidinyl, pyrrolidinyl, imidazolidinyl, piperidyl, piperazinyl, and morpholinyl, wherein, the azetidinyl, pyrrolidinyl, imidazolidinyl, piperidyl, piperazinyl, and morpholinyl are optionally substituted with 1-3 substituents selected from the group consisting of halogen, and C1-C6 alkyl; further preferably, Cy₂ is selected from the group consisting of pyrrolidinyl, imidazolidinyl, and piperidyl, wherein, the pyrrolidinyl, imidazolidinyl, and piperidyl are optionally substituted with 1-3 substituents selected from the group consisting of F, Cl, Br, and methyl; further preferably, Cy₂ is pyrrolidinyl optionally substituted with 1-3 (such as 1 or 2 or 3) F;
preferably, R^{a} is selected from the group consisting of H, halogen, cyano, carboxyl, 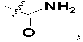 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of hydroxy, C3-C6 cycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, halogen, hydroxy, hydroxy-C1-C6 alkyl-, cyano, oxo, - NH₂, -N(C1-C6 alkyl), and -N(C1-C6 alkyl)(C1-C6 alkyl);
preferably, R^{a} is selected from the group consisting of H, cyano, carboxyl, 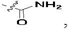 C1-C6 haloalkyl (such as trifluoromethyl), 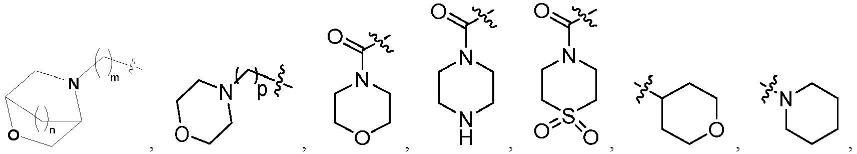 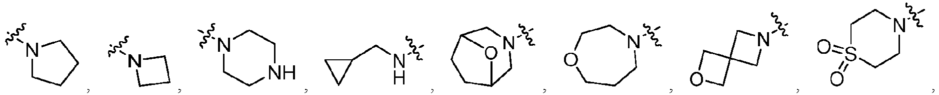 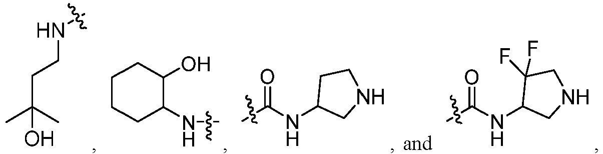 wherein, the 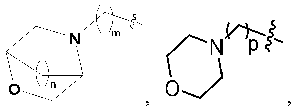 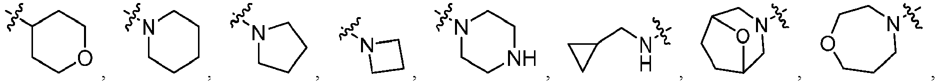 and 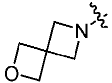 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl);
preferably, R^{a} is selected from the group consisting of H, cyano, carboxyl, 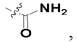 C1-C6 haloalkyl (such as trifluoromethyl), 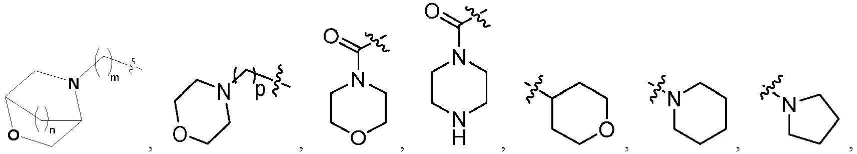 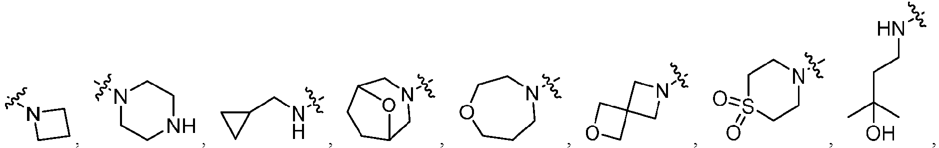 and 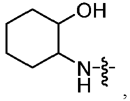 wherein, the 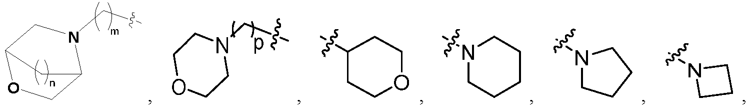 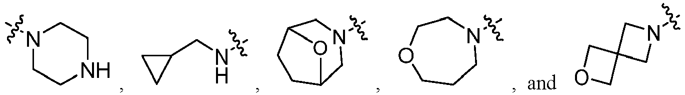 are optionally substituted with 1-3 substituents selected from the group consisting of C1-C6 alkyl, halogen, hydroxy, cyano, -NH₂, -N(C1-C6 alkyl), and - N(C1-C6 alkyl)(C1-C6 alkyl);
preferably, R^{a} is selected from the group consisting of H, cyano, 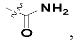 C1-C6 haloalkyl (such as trifluoromethyl), and 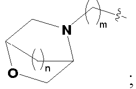 wherein, the 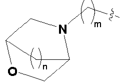 is optionally substituted with C1-C6 alkyl (preferably methyl), halogen (such as F, Cl, Br);
m is selected from the group consisting of 0, 1, 2, and 3; preferably, m is selected from the group consisting of 0, and 1; n is selected from the group consisting of 0, 1, 2, and 3; preferably, n is selected from the group consisting of 0, and 1; p is selected from the group consisting of 0, 1, 2, and 3; preferably, p is selected from the group consisting of 0, and 1;
further preferably, R^{a} is selected from the group consisting of H, cyano, carboxyl, 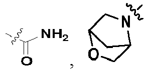 (preferably 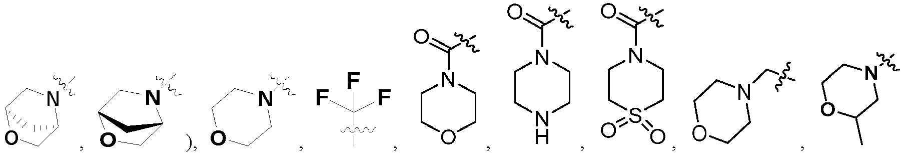 (preferably 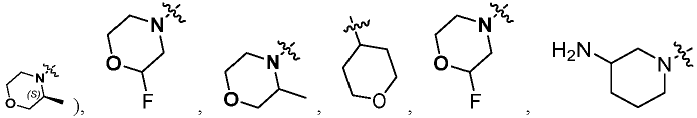 (preferably 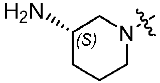 or 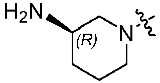 ), 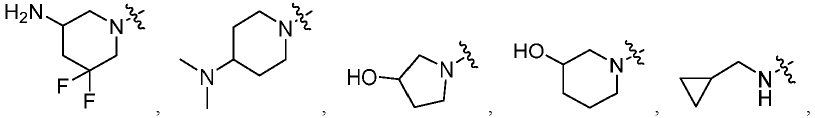 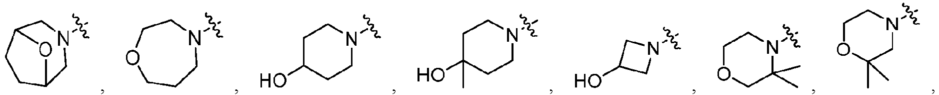 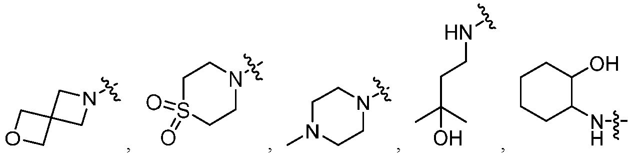 (preferably 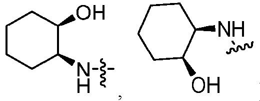 ), 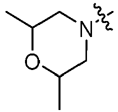 (preferably 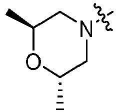 ), 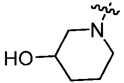 (preferably 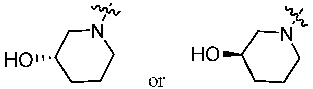 ), 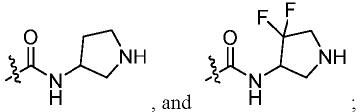
further preferably, R^{a} is selected from the group consisting of H, cyano, 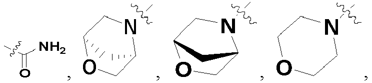 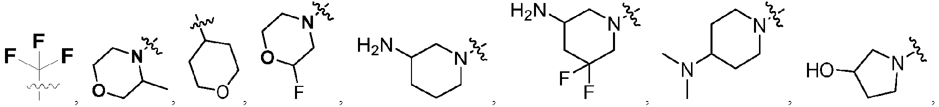 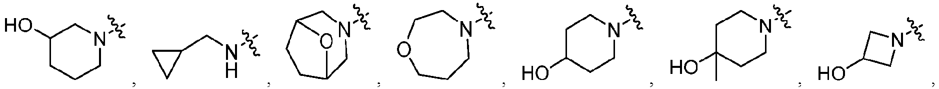 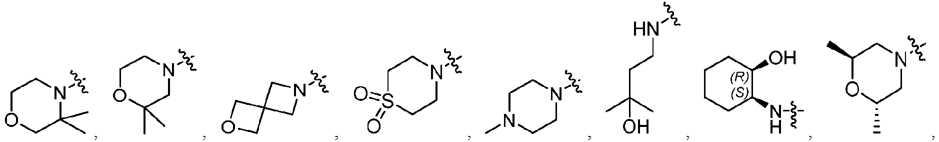 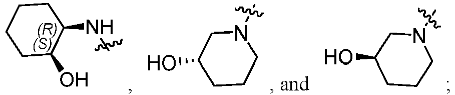
R^{b} is selected from the group consisting of H, halogen, amino, cyano, carboxyl, 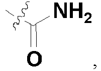 C1-C6 alkyl, C1-C6 haloalkyl (such as trifluoromethyl), 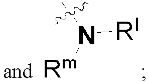 preferably, R^{b} is selected from the group consisting of H, cyano, 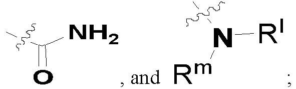
R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl,
or, R^{a}, R^{b} and the carbon atoms to which they are attached, respectively, together form a substituted or unsubstituted 5-membered heterocycle, wherein, the substituent is selected from C1-C6 alkyl (such as tert-butyl);
preferably, R^{a}, R^{b} and the carbon atoms to which they are attached, respectively, together form 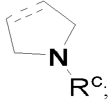 R^{c} is selected from the group consisting of H, and C1-C6 alkyl; preferably, R^{c} is selected from C1-C6 alkyl;
further preferably, B¹, as a whole, is selected from the group consisting of: 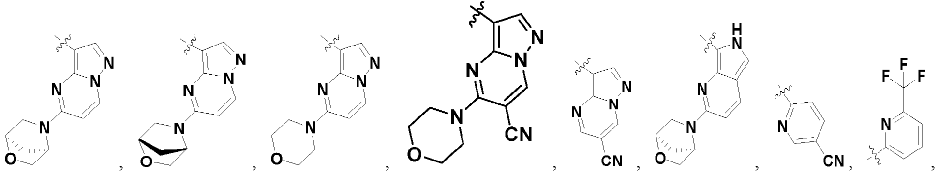 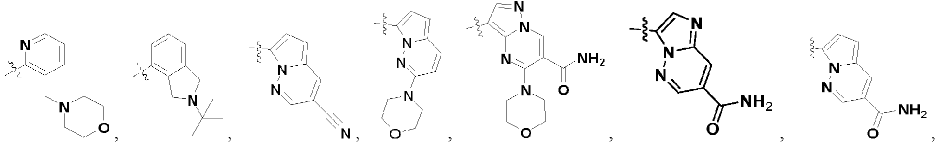 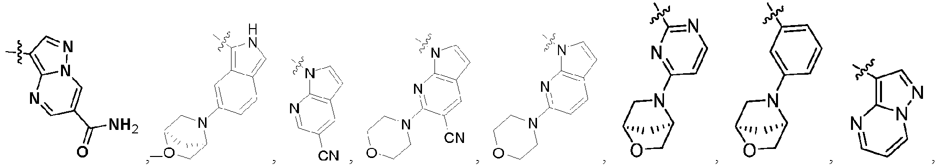 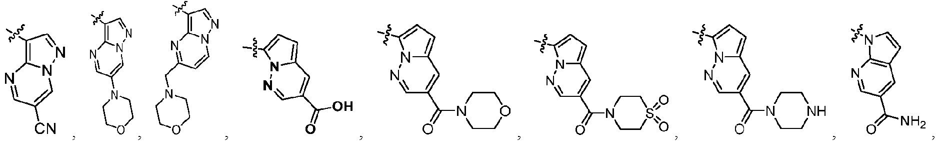 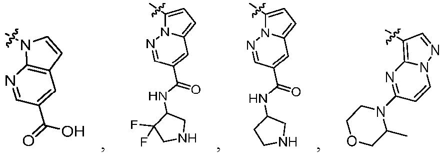 (preferably 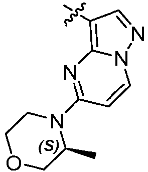 ), 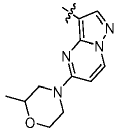 (preferably 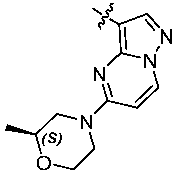 ), 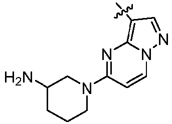 ( 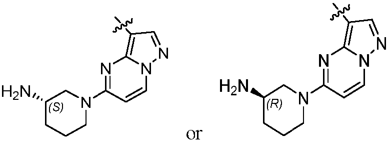 ), 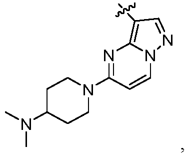 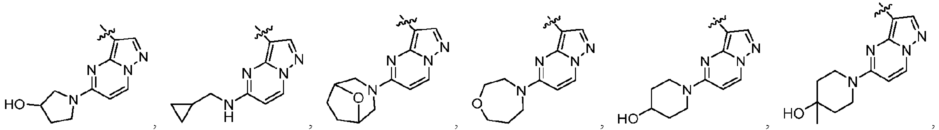 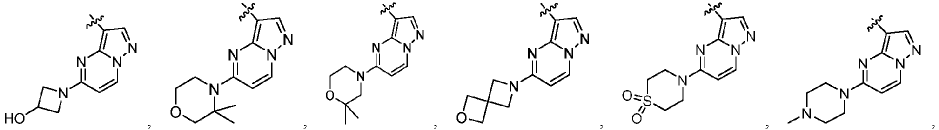 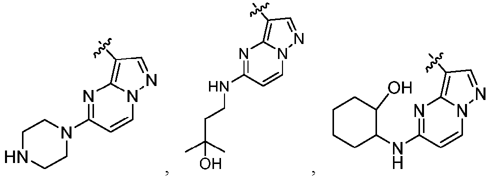 (preferably 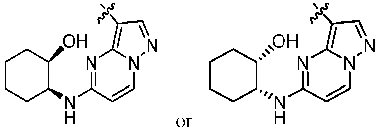 ), 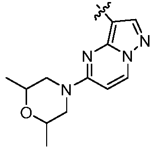 (preferably 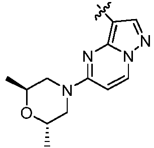 ), 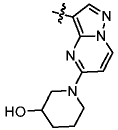 (preferably 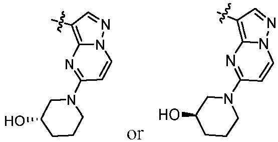 ), and 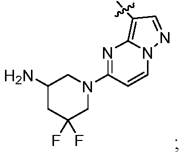
further preferably, B¹, as a whole, is selected from the group consisting of: 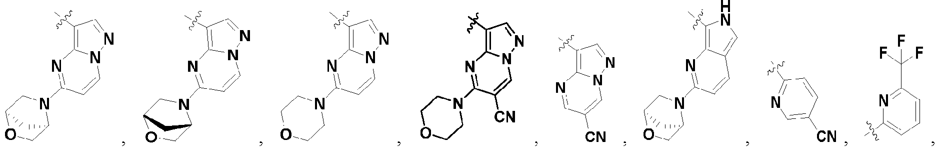 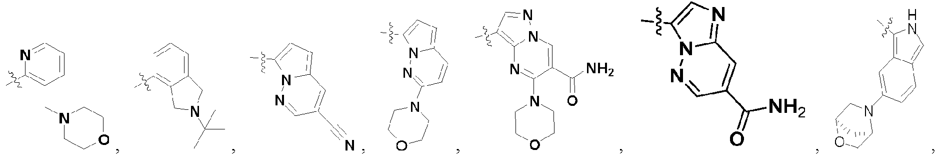 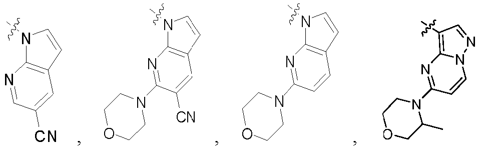 (preferably 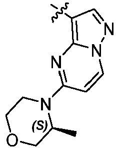 ), 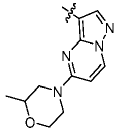 (preferably 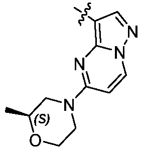 ), 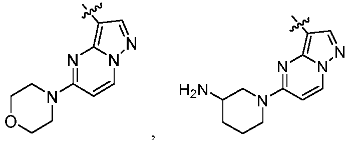 ( 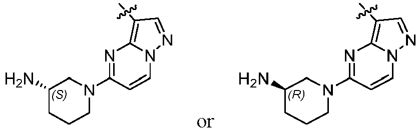 ), 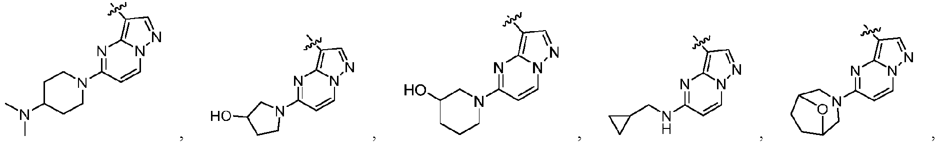 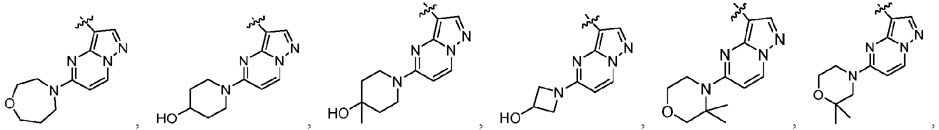 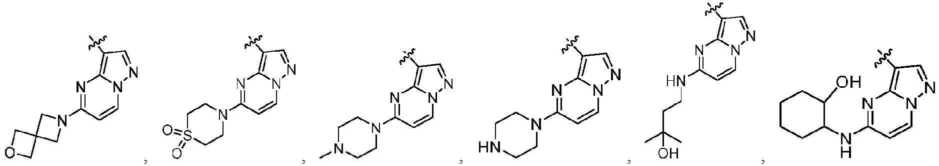 (preferably 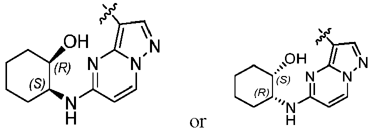 ), 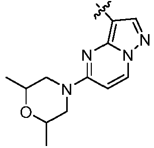 (preferably 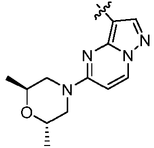 ), 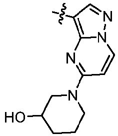 (preferably 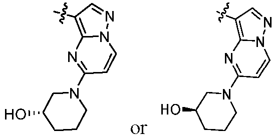 ), and 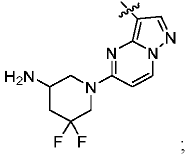
further preferably, B¹, as a whole, is selected from the group consisting of 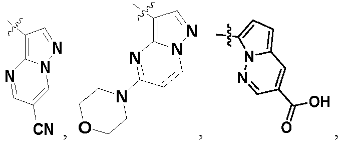 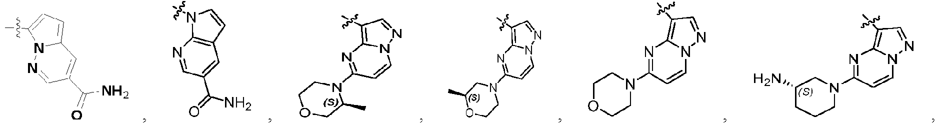 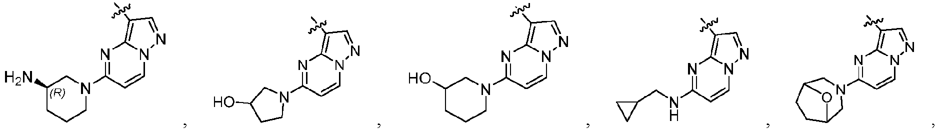 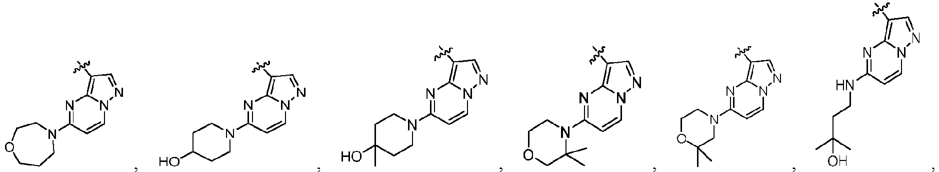 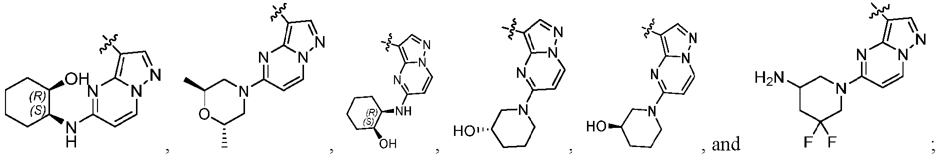
L¹ is selected from the group consisting of a direct bond, C1-C6 alkyl, 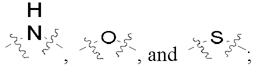 preferably, L¹ is selected from the group consisting of a direct bond, 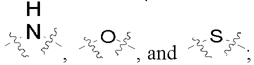 preferably, L¹ is selected from the group consisting of a direct bond, methylene, and 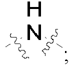 preferably, L¹ is selected from the group consisting of a direct bond, and 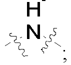
R¹¹ is selected from the group consisting of 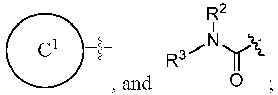
C¹ is selected from 5-6 membered heteroaryl, and C¹ is optionally substituted with at least one of substituents R^{d}, R^{e}, R^{f}; preferably, C¹ is selected from 5-6 membered heteroaryl containing 1-3 heteroatoms, and C¹ is optionally substituted with at least one of substituents R^{d}, R^{e}, R^{f}; further preferably, C¹ is selected from the group consisting of pyrazolyl, imidazolyl, thiazolyl, pyridinyl, and pyrrolyl, and C¹ is optionally substituted with at least one of substituents R^{d}, R^{e}, R^{f}; further preferably, C¹ is selected from the group consisting of pyrazolyl, thiazolyl, pyridinyl, and pyrrolyl, and C¹ is optionally substituted with at least one of substituents R^{d}, R^{e}, R^{f};
R^{d}, R^{e}, R^{f} are each independently selected from the group consisting of H, C1-C6 alkyl, C1-C6 haloalkyl (such as difluoromethyl), C3-C7 cycloalkyl, 4-7 membered saturated heterocyclyl, and 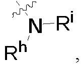 wherein, the C3-C7 cycloalkyl and 4-7 membered saturated heterocyclyl are optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
R^{d}, R^{e}, R^{f} are each independently selected from the group consisting of H, C1-C6 alkyl, C1-C6 haloalkyl (such as difluoromethyl), C3-C7 cycloalkyl, and 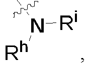 wherein, the C3-C7 cycloalkyl is optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d} is selected from C3-C6 cycloalkyl optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d} is selected from 4-7 membered saturated heterocyclyl optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
further preferably, R^{d} is selected from the group consisting of C1-C6 alkyl, and 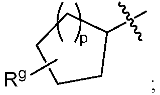
or further preferably, R^{d} is selected from the group consisting of pyrrolyl, piperidyl, imidazolidinyl, and piperazinyl, wherein, the pyrrolyl, piperidyl, imidazolidinyl, and piperazinyl are optionally substituted with 1-2 substituents selected from the group consisting of halogen, C1-C6 alkoxy, and C1-C6 alkyl (preferably methyl);
further preferably, R^{d} is selected from 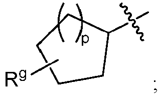 p is selected from the group consisting of 0, 1, and 2;
or further preferably, R^{d} is selected from the group consisting of pyrrolyl, and piperidyl, wherein, the pyrrolyl, and piperidyl are optionally substituted with 1-2 substituents selected from the group consisting of halogen, and C1-C6 alkyl (preferably methyl);
R^{g} is selected from the group consisting of H, amino, hydroxy, hydroxy C1-C6 alkyl, and formyl;
further preferably, R^{d} is selected from the group consisting of methyl, ethyl, isopropyl, 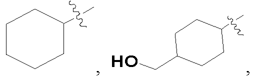 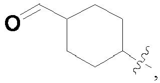 piperidyl, 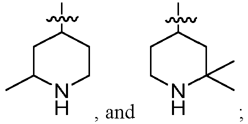
further preferably, R^{d} is selected from the group consisting of 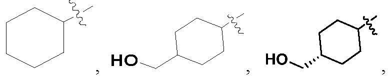 and 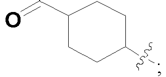
further preferably, R^{d} is selected from the group consisting of 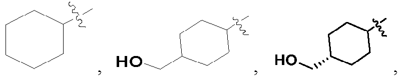 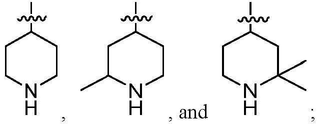
R^{h}, Rⁱ are each independently selected from the group consisting of H, C1-C6 alkyl, and C3-C6 cycloalkyl; preferably, R^{h}, Rⁱ are each independently selected from the group consisting of H, methyl, ethyl, isopropyl, and cyclopropyl;
preferably, R^{e} is selected from the group consisting of H, methyl, 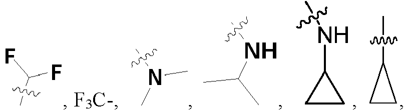 and -NHCH₃;
preferably, R^{e} is selected from the group consisting of H, methyl, 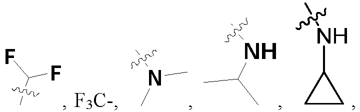 and 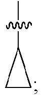
or, R^{e}, R^{f} and the carbon atoms to which they are attached, respectively, together form a substituted or unsubstituted 5-9 membered heterocycle, wherein, the substituent is selected from the group consisting of C1-C6 alkyl (such as ethyl), alkoxy (such as C1-C6 alkoxy), C3-C6 cycloalkyl, amino, and hydroxy,
preferably, R^{e}, R^{f} and the carbon atoms to which they are attached, respectively, together form 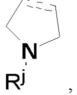 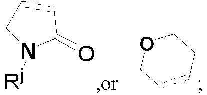
each R^{j} is independently selected from the group consisting of H, C1-C6 alkyl, alkoxy (such as C1-C6 alkoxy), C3-C6 cycloalkyl, amino, and hydroxy;
further preferably, R^{e}, R^{f} and the carbon atoms to which they are attached, respectively, together form 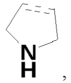 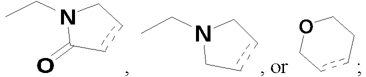
R², R³ are each independently selected from the group consisting of H, C1-C6 alkyl, and C3-C6 cycloalkyl; or, R², R³ and the N atom to which they are both attached together form 5-6 membered saturated heterocyclyl, preferably, the 5-6 membered saturated heterocyclyl contains 2-3 heteroatoms selected from the group consisting of N, S, and O atoms;
preferably, R², R³ are each independently selected from the group consisting of H, methyl, ethyl, propyl, and 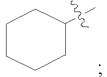
or preferably, R², R³ and the N atom to which they are both attached together form 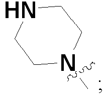
further preferably, R¹¹, as a whole, is selected from the group consisting of: 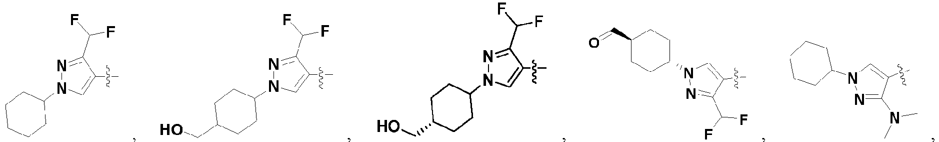 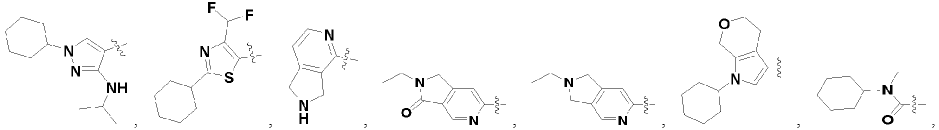 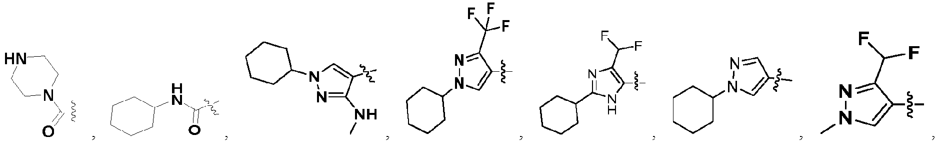 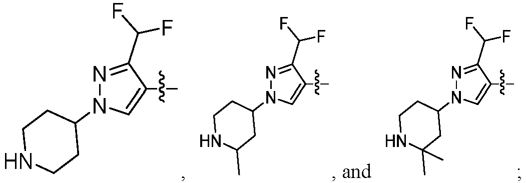
further preferably, R¹¹, as a whole, is selected from the group consisting of: 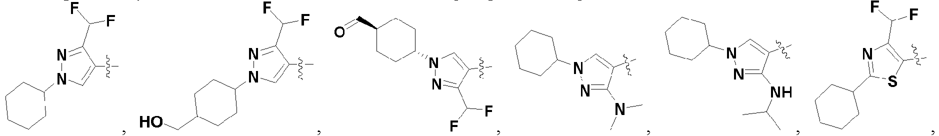 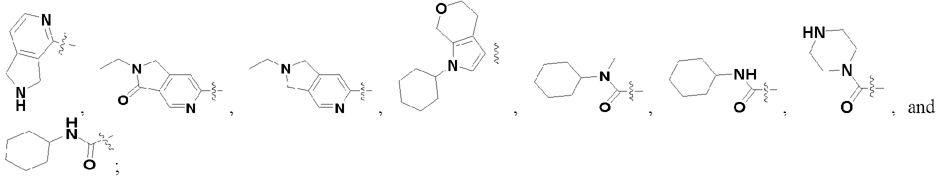
further preferably, R¹¹, as a whole, is selected from the group consisting of

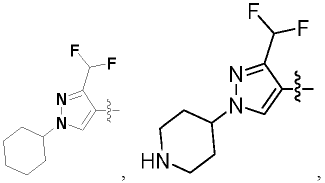

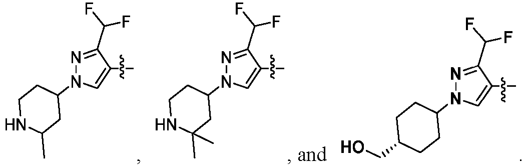

In the fifth aspect, the present invention provides a compound represented by formula ii, or a stereoisomer, a tautomer, a prodrug, a crystal form, a hydrate, an isotope-labeled compound, a metabolite, an ester, a pharmaceutically acceptable salt, or a pharmaceutically acceptable solvate thereof, 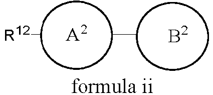 wherein:
A² is selected from 6 membered heteroaromatic ring optionally substituted with substituent R^{k}; preferably, A² is selected from 6 membered heteroaromatic ring containing 1-2 heteroatoms, wherein at least one heteroatom is N atom, and, the 6 membered heteroaromatic ring is optionally substituted with substituent R^{k}; further preferably, A² is selected from the group consisting of 2-pyridone, and pyridazine ring, wherein, the 2-pyridone is optionally substituted with substituent R^{k}; further preferably, A² is selected from the group consisting of 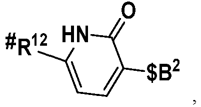 and 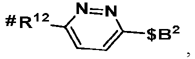 wherein, the 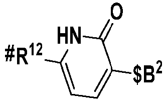 is optionally substituted with substituent R^{k}, wherein #R¹² represents the attachment point to R¹², $B² represents the attachment point to B², further preferably, A² is selected from the group consisting of 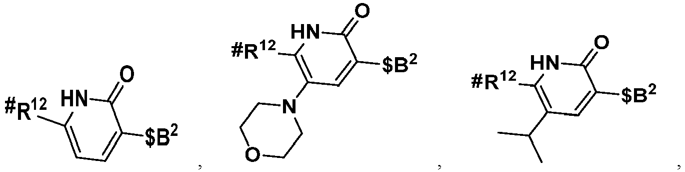 and 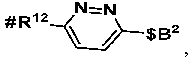 wherein #R¹² represents the attachment point to R¹², $B² represents the attachment point to B²,
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl;
preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl;
R^{p}, R⁹ are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
further preferably, R^{k} is selected from the group consisting of isopropyl, cyclopropyl, dimethylamino, difluoromethyl, and 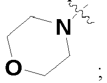
B² is selected from 6-10 membered heteroaryl, and B² is optionally substituted with at least one of substituents R^{a}, R^{b}; preferably, B² is substituted with at least one of substituents R^{a}, R^{b}; preferably, B² is selected from 9 membered heteroaryl, and B² is substituted with at least one of substituents R^{a}, R^{b}; preferably, the 9 membered heteroaryl contains 1-4 heteroatoms selected from the group consisting of N, O, and S atoms; further preferably, B² is selected from the group consisting of 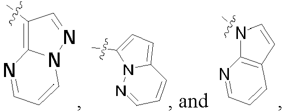 and B² is substituted with at least one of substituents R^{a}, R^{b};
R^{a} is selected from the group consisting of H, halogen, amino, cyano, carboxyl, 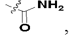 C1-C6 alkyl, and C1-C6 haloalkyl (such as trifluoromethyl); preferably, R^{a} is selected from the group consisting of H, cyano, 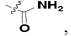 and C1-C6 haloalkyl (such as trifluoromethyl); further preferably, R^{a} is selected from the group consisting of H, cyano, 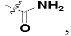 and 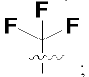
R^{b} is selected from the group consisting of H, halogen, amino, cyano, carboxyl, 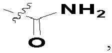 C1-C6 alkyl, C1-C6 haloalkyl (such as trifluoromethyl), and 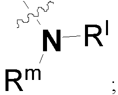 preferably, R^{b} is selected from the group consisting of H, cyano, and 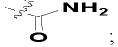
R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl;
further preferably, B², as a whole, is selected from the group consisting of 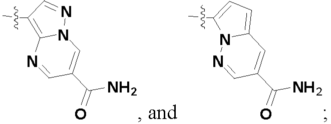
R¹² is selected from 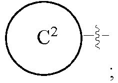
C² is selected from 5-6 membered heteroaryl, and C² is optionally substituted with at least one of substituents R^{d}, R^{e}, R^{f}; preferably, C² is selected from 5-6 membered heteroaryl containing 1-3 heteroatoms, and C² is optionally substituted with at least one of substituents R^{d}, R^{e}, R^{f}; further preferably, C² is selected from the group consisting of pyrrolyl, oxazolyl, furanyl, 1,2,3-triazolyl, and 1,2,4-triazolyl, and C² is optionally substituted with at least one of substituents R^{d}, R^{e}, R^{f};
R^{d}, R^{e}, R^{f} are each independently selected from the group consisting of H, C1-C6 alkyl, C1-C6 haloalkyl (such as difluoromethyl), C3-C7 cycloalkyl, 6-10 membered aryl, 5-6 membered heteroaryl, and 4-7 membered saturated heterocyclyl, wherein, the C3-C7 cycloalkyl, 6-10 membered aryl, 5-6 membered heteroaryl, and 4-7 membered saturated heterocyclyl are each independently optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d} is selected from the group consisting of C3-C6 cycloalkyl, phenyl, morpholinyl, and piperazinyl, wherein, the C3-C6 cycloalkyl, phenyl, morpholinyl, and piperazinyl are each independently optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl; further preferably, R^{d} is selected from the group consisting of C3-C6 cycloalkyl, phenyl, morpholinyl, and piperazinyl; further preferably, R^{d} is selected from the group consisting of 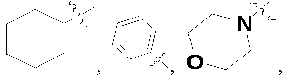 and piperazinyl;
preferably, R^{e} is selected from the group consisting of H, methyl, 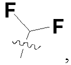 F₃C-, and 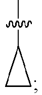
further preferably, R¹², as a whole, is selected from the group consisting of:

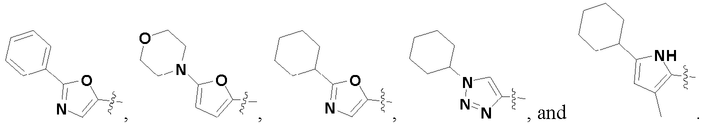

In the sixth aspect, the present invention provides a compound represented by formula I, or a stereoisomer, a tautomer, a prodrug, a crystal form, a hydrate, an isotope-labeled compound, a metabolite, an ester, a pharmaceutically acceptable salt, or a pharmaceutically acceptable solvate thereof, 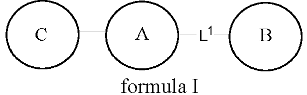 wherein:
A is selected from 5-6 membered heteroaromatic ring optionally substituted with substituent R^{k}; preferably, A is selected from the group consisting of 5 membered heteroaromatic ring, and 6 membered heteroaromatic ring, the 5 membered heteroaromatic ring containing 2-3 heteroatoms, wherein at least two heteroatoms are N atoms; the 6 membered heteroaromatic ring containing 1-2 heteroatoms, wherein at least one heteroatom is N atom, and, the 5 membered heteroaromatic ring and 6 membered heteroaromatic ring are optionally substituted with substituent R^{k}; further preferably, A is selected from the group consisting of 1,2,3-triazole ring, 1,2,4-triazole ring, pyrazole ring, imidazole ring, 1,3,4-thiadiazole ring, 1,3,4-oxadiazole ring, 2-pyridone, and pyridazine ring, wherein, the 2-pyridone is optionally substituted with substituent R^{k}; further preferably, A is selected from the group consisting of 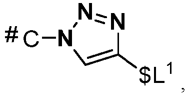 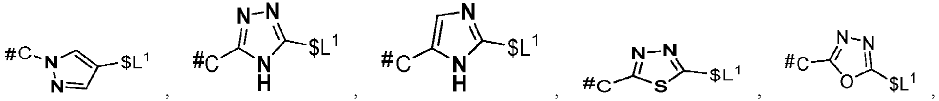 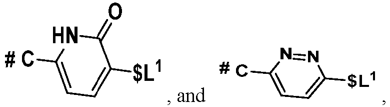 wherein, the 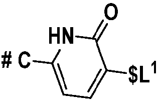 is optionally substituted with substituent R^{k}, wherein #C represents the attachment point to C, $L¹ represents the attachment point to L¹; further preferably, A is selected from the group consisting of 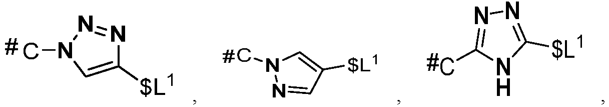 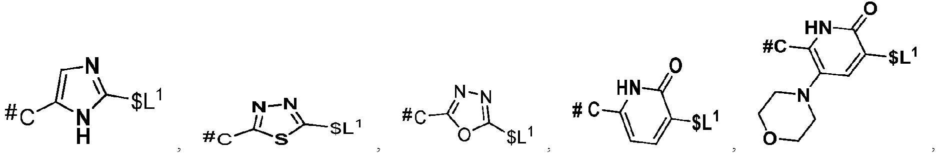 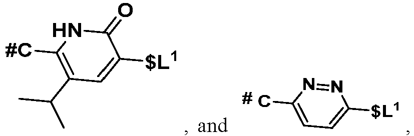 wherein #C represents the attachment point to C, $L¹ represents the attachment point to L¹;
further preferably, A is selected from the group consisting of 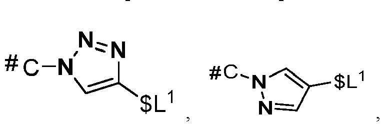 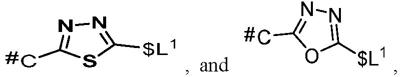 wherein #C represents the attachment point to C, $L¹ represents the attachment point to L¹;
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl; preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl;
R^{p}, R^{q} are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
further preferably, R^{k} is selected from the group consisting of isopropyl, cyclopropyl, dimethylamino, difluoromethyl, and 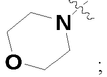
B is selected from any one of groups (1)-(7): 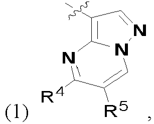 wherein:
   R⁴ is selected from the group consisting of H, C1-C6 alkyl, C4-C9 cycloalkyl, C4-C9 cycloalkyl-C1-C6 alkyl, 4-9 membered saturated heterocyclyl, 4-9 membered saturated heterocyclyl-C1-C6 alkyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl); 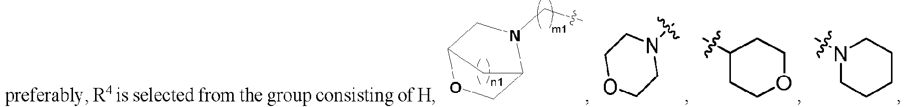 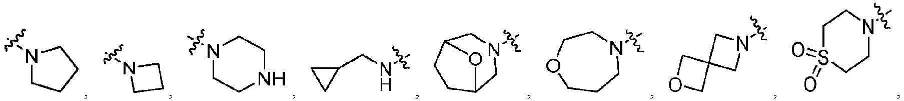 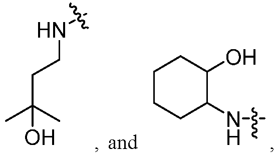 wherein, the 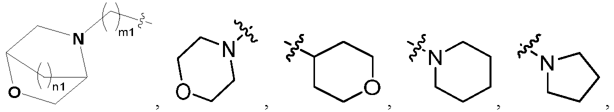 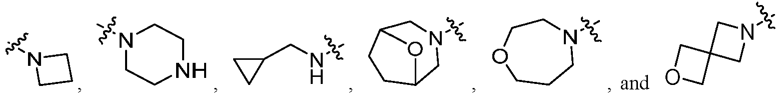 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl);
   m1 is selected from the group consisting of 0, 1, 2, and 3; m1 is preferably 0; n1 is selected from the group consisting of 0, 1, 2, and 3; n1 is preferably 0, or 1;
   further preferably, R⁴ is selected from the group consisting of H, 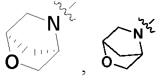 (preferably 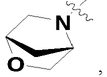 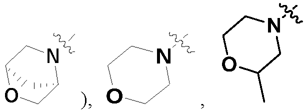 (preferably 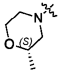 ), 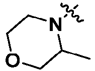 (preferably 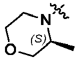 ), 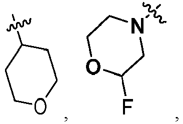 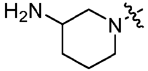 (preferably 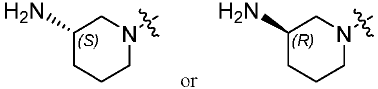 ), 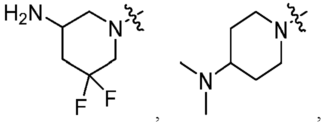 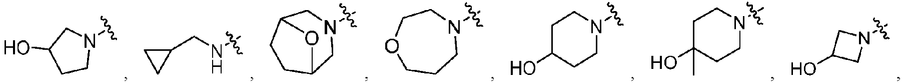 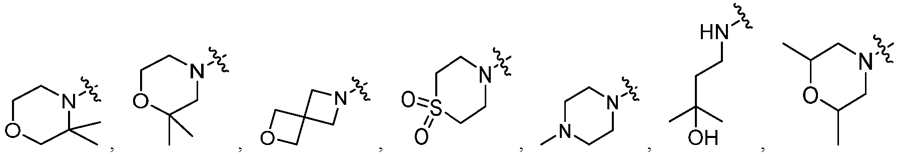 (preferably 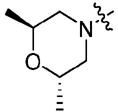 ), 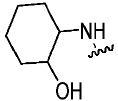 (preferably 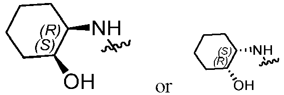 ), and 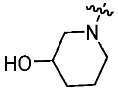 (preferably 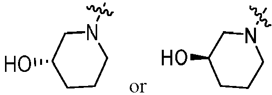 );
   R⁵ is selected from the group consisting of H, cyano, C1-C6 alkyl, 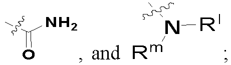 preferably, R⁵ is selected from the group consisting of H, cyano, 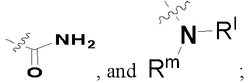
   R^{l}, R^{m} are each independently selected from the group consisting of H, C1-C6 alkyl, and C3-C6 cycloalkyl,
   preferably, R^{l}, R^{m} are each independently selected from the group consisting of H, and C1-C6 alkyl;
   preferably, R⁵ is selected from the group consisting of H, and cyano;
   further preferably, 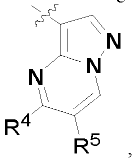 as a whole, is selected from the group consisting of 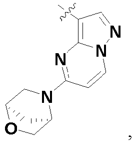 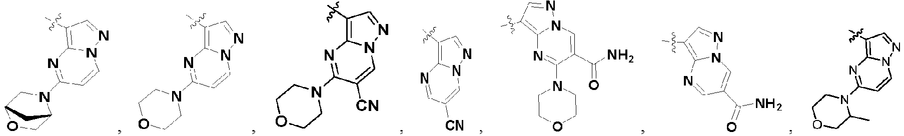 (preferably 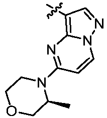 ), 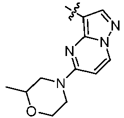 (preferably 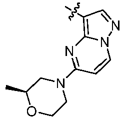 ), 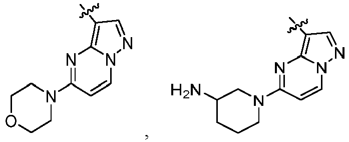 ( 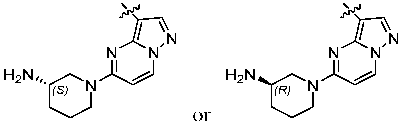 ), 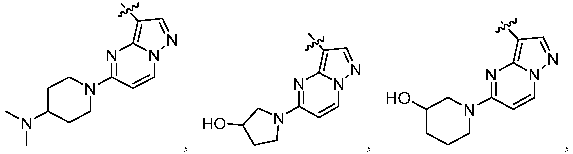 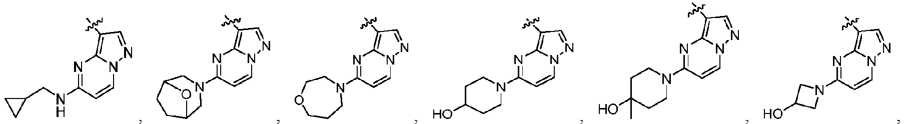 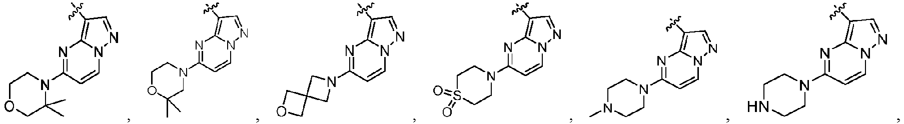 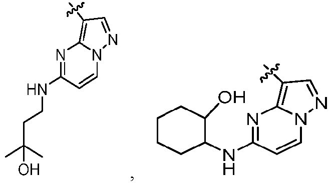 (preferably 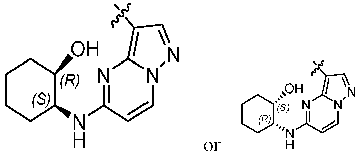 ), 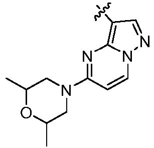 (preferably 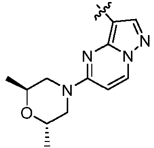 ), 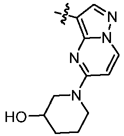 (preferably 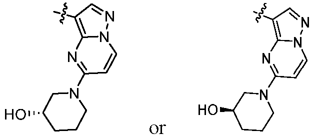 ), and 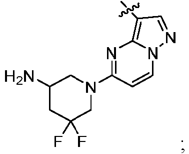
   further preferably, 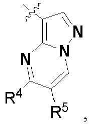 as a whole, is selected from the group consisting of 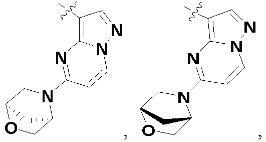 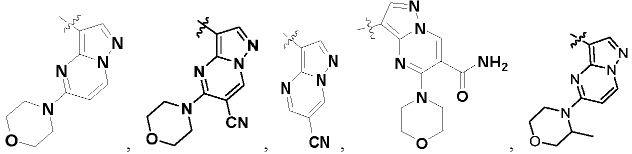 (preferably 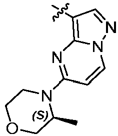 ), 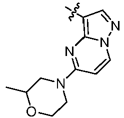 (preferably 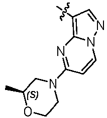 ), 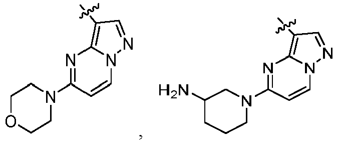 ( 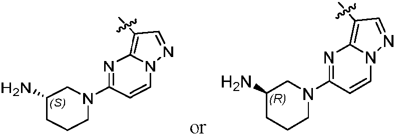 ), 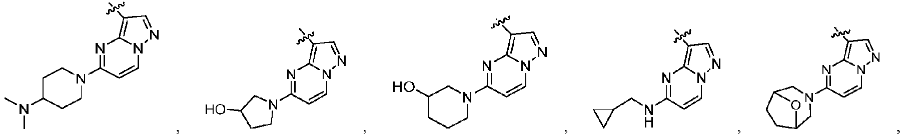 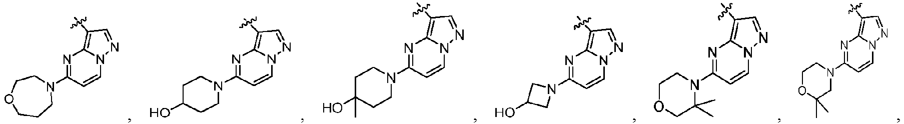 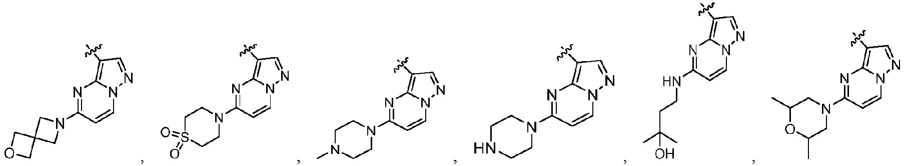 (preferably 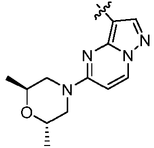 ), 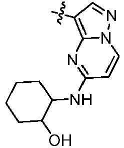 (preferably 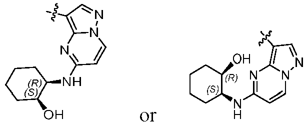 ), and 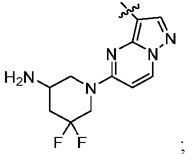 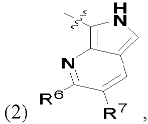 wherein:
      R⁶ is selected from the group consisting of H, C1-C6 alkyl, C4-C9 cycloalkyl, C4-C9 cycloalkyl-C1-C6 alkyl, 4-9 membered saturated heterocyclyl, and 4-9 membered saturated heterocyclyl-C1-C6 alkyl,
      preferably, R⁶ is selected from 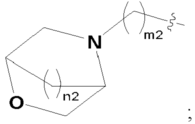 m2 is selected from the group consisting of 0, 1, 2, and 3; n2 is selected from the group consisting of 0, 1, 2, and 3;
      further preferably, R⁶ is 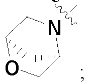
      R⁷ is selected from the group consisting of H, and C1-C6 alkyl;
      further preferably, 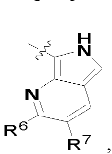 as a whole, is 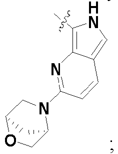 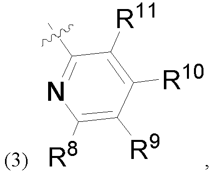 wherein:
         one of R⁸, R⁹, R¹⁰, and R¹¹ is selected from the group consisting of cyano, C1-C6 haloalkyl (such as trifluoromethyl), C4-C9 cycloalkyl, C4-C9 cycloalkyl-C1-C6 alkyl, 4-9 membered saturated heterocyclyl, and 4-9 membered saturated heterocyclyl-C1-C6 alkyl, and the rest are H;
         preferably, among R⁸, R⁹, R¹⁰, and R¹¹, R⁸, R⁹ or R¹¹ is selected from the group consisting of cyano, C1-C6 haloalkyl (such as trifluoromethyl), and 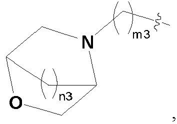 and the rest are H; m3 is selected from the group consisting of 0, 1, 2, and 3; n3 is selected from the group consisting of 0, 1, 2, and 3;
         further preferably, 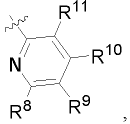 as a whole, is selected from the group consisting of 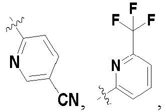 and 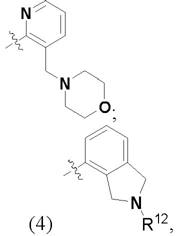 wherein:
            R¹² is selected from the group consisting of H, and C1-C6 alkyl;
            preferably, R¹² is selected from C1-C6 alkyl; 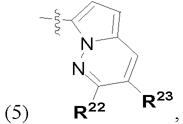 wherein:
               R²² is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²² is selected from the group consisting of H, and 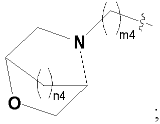 m4 is selected from the group consisting of 0, 1, 2, and 3; n4 is selected from the group consisting of 0, 1, 2, and 3;
               further preferably, R²² is selected from the group consisting of H, and 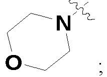
               R²³ is selected from the group consisting of H, C1-C6 alkyl, cyano, carboxyl, 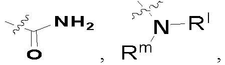 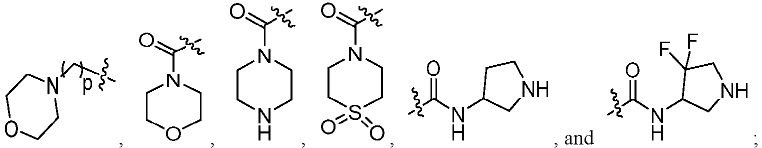
               preferably, R²³ is selected from the group consisting of H, C1-C6 alkyl, cyano, 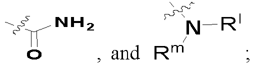 preferably, R²³ is selected from the group consisting of H, cyano, carboxyl, 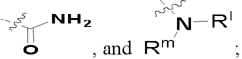 preferably, R²³ is selected from the group consisting of H, cyano, 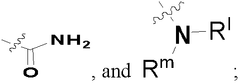
               R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl;
               further preferably, 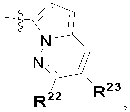 as a whole, is selected from the group consisting of 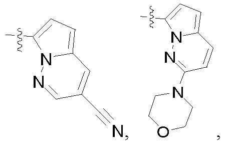 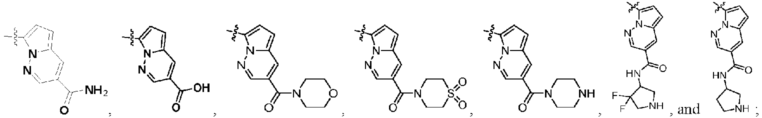
               further preferably, 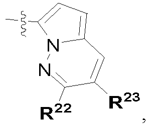 as a whole, is selected from the group consisting of 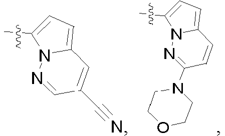 and 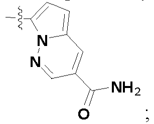 further preferably, 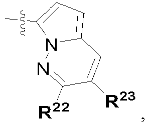 as a whole, is selected from the group consisting of 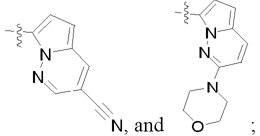 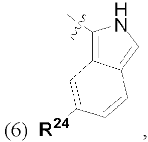 wherein:
                  R²⁴ is selected from the group consisting of H, C1-C6 alkyl, C4-C9 cycloalkyl, C4-C9 cycloalkyl-C1-C6 alkyl, 4-9 membered saturated heterocyclyl, and 4-9 membered saturated heterocyclyl-C1-C6 alkyl;
                  preferably, R²⁴ is selected from 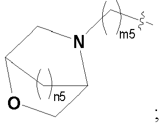 m5 is selected from the group consisting of 0, 1, 2, and 3; n5 is selected from the group consisting of 0, 1, 2, and 3;
                  further preferably, R²⁴ is 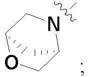 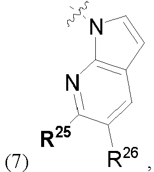 wherein:
                     R²⁵ is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl;
                     preferably, R²⁵ is selected from the group consisting of H, 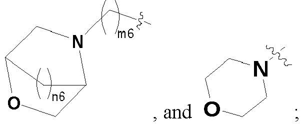 m6 is selected from the group consisting of 0, 1, 2, and 3; n6 is selected from the group consisting of 0, 1, 2, and 3;
                     further preferably, R²⁵ is selected from the group consisting of H, and 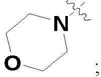 preferably, R²⁵ is H;
                     R²⁶ is selected from the group consisting of H, C1-C6 alkyl, cyano, carboxyl, and 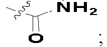 preferably, R²⁶ is selected from the group consisting of H, C1-C6 alkyl, cyano, and 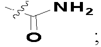 preferably, R²⁶ is selected from the group consisting of H, C1-C6 alkyl, and 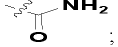
                     further preferably, 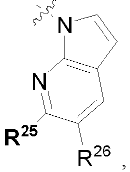 as a whole, is selected from the group consisting of 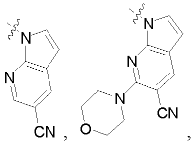 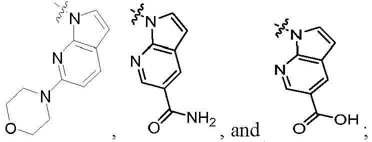
                     further preferably, 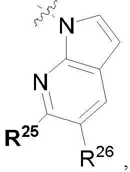 as a whole, is selected from the group consisting of 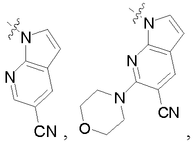 and 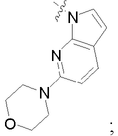
                     L¹ is selected from the group consisting of a direct bond, C1-C6 alkyl, 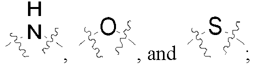
                     preferably, L¹ is selected from the group consisting of a direct bond, and 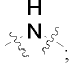 preferably, L¹ is a direct bond;
                     C is selected from any one of groups (1)-(10): 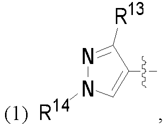 wherein:
                        R¹³ is selected from the group consisting of C1-C6 haloalkyl (such as difluoromethyl), and 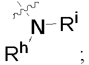
                        R^{h}, Rⁱ are each independently selected from the group consisting of H, and C1-C6 alkyl; preferably, R^{h}, Rⁱ are each independently selected from the group consisting of H, methyl, and isopropyl;
                        preferably, R¹³ is selected from the group consisting of 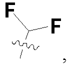 trifluoromethyl, 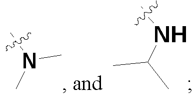 preferably, R¹³ is selected from the group consisting of 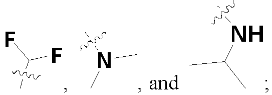 further preferably, R¹³ is selected from the group consisting of 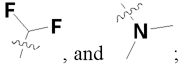
                        R¹⁴ is selected from the group consisting of C3-C6 cycloalkyl, and piperidyl, wherein, the C3-C6 cycloalkyl and piperidyl are optionally substituted with 1-2 substituents selected from the group consisting of hydroxy C1-C6 alkyl (such as methylol), formyl, and C1-C6 alkyl;
                        preferably, R¹⁴ is selected from C3-C6 cycloalkyl optionally substituted with 1-2 substituents selected from the group consisting of hydroxy C1-C6 alkyl (such as methylol), and formyl; preferably, R¹⁴ is selected from 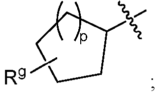 p is selected from the group consisting of 0, 1, and 2; R^{g} is selected from the group consisting of H, hydroxy C1-C6 alkyl, and formyl;
                        or, preferably, R¹⁴ is piperidyl optionally substituted with 1-2 substituents selected from C1-C6 alkyl (preferably methyl);
                        further preferably, R¹⁴ is selected from the group consisting of 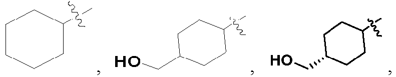 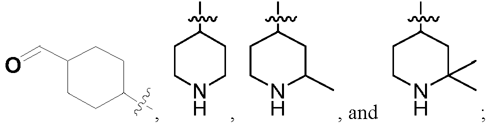
                        further preferably, 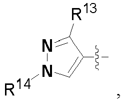 as a whole, is selected from the group consisting of 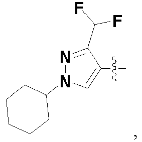 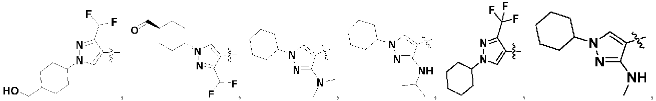 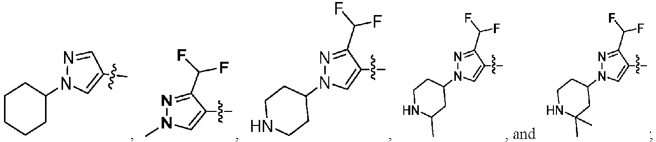
                        further preferably, 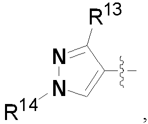 as a whole, is selected from the group consisting of 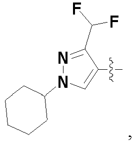 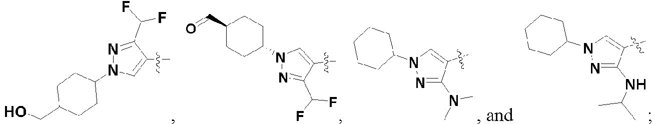
                        further preferably, 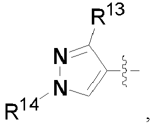 as a whole, is selected from the group consisting of 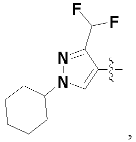 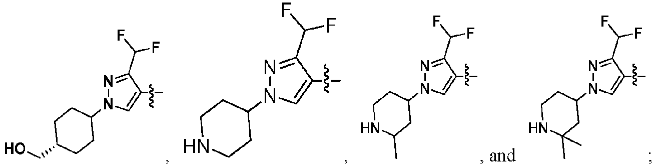 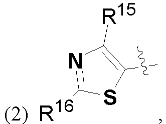
                        wherein: R¹⁵ is selected from C1-C6 haloalkyl; R¹⁶ is selected from C3-C6 cycloalkyl; further preferably, 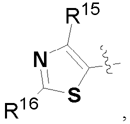 as a whole, is 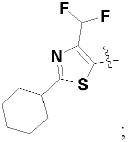 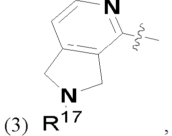
                        wherein: R¹⁷ is selected from the group consisting of H, and C1-C6 alkyl; preferably, 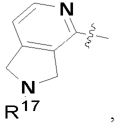 as a whole, is 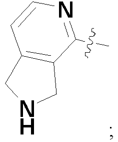 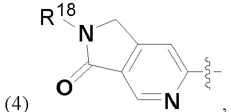
                        wherein: R¹⁸ is selected from the group consisting of H, and C1-C6 alkyl; preferably, R¹⁸ is selected from C1-C6 alkyl; further preferably, 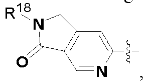 as a whole, is 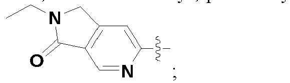 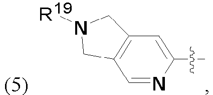
                        wherein: R¹⁹ is selected from the group consisting of H, and C1-C6 alkyl; preferably, R¹⁹
                        is selected from C1-C6 alkyl; further preferably, 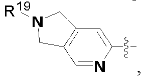 as a whole, is 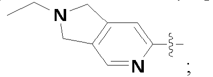 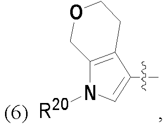
                        wherein: R²⁰ is selected from C3-C6 cycloalkyl; further preferably, 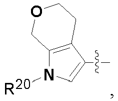 as a whole, 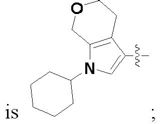 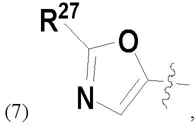
                        wherein: R²⁷ is selected from the group consisting of phenyl, and C3-C6 cycloalkyl; further preferably, 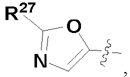 as a whole, is selected from the group consisting of 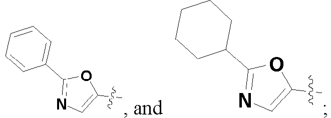 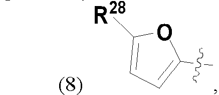 wherein: R²⁸ is selected from 6 membered saturated heterocyclyl; preferably, R²⁸ is morpholinyl; further preferably, 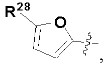 as a whole, is 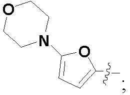 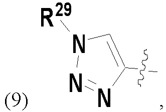
                        wherein: R²⁹ is selected from C3-C6 alkyl; further preferably, 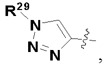 as a whole, is 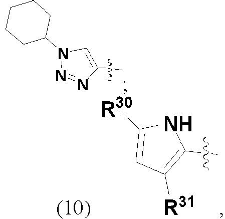 wherein: R³⁰ is selected from C3-C6 cycloalkyl; R³¹ is selected from C1-C6 alkyl; further preferably, 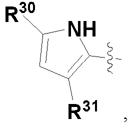 as a whole, is

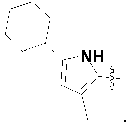

In the seventh aspect, the present invention provides a compound represented by formula I-1, or a stereoisomer, a tautomer, a prodrug, a crystal form, a hydrate, an isotope-labeled compound, a metabolite, an ester, a pharmaceutically acceptable salt, or a pharmaceutically acceptable solvate thereof, 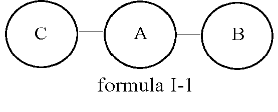 wherein:
A is selected from 5-6 membered heteroaromatic ring optionally substituted with substituent R^{k}; preferably, A is selected from the group consisting of 5 membered heteroaromatic ring, and 6 membered heteroaromatic ring, the 5 membered heteroaromatic ring containing 2-3 heteroatoms, wherein at least two heteroatoms are N atoms; the 6 membered heteroaromatic ring containing 1-2 heteroatoms, wherein at least one heteroatom is N atom, and, the 5 membered heteroaromatic ring and 6 membered heteroaromatic ring are optionally substituted with substituent R^{k}; further preferably, A is selected from the group consisting of 1,2,3-triazole ring, 1,2,4-triazole ring, pyrazole ring, imidazole ring, 1,3,4-thiadiazole ring, 1,3,4-oxadiazole ring, 2-pyridone, and pyridazine ring, wherein, the 2-pyridone is optionally substituted with substituent R^{k};
further preferably, A is selected from the group consisting of 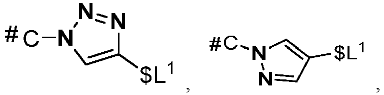 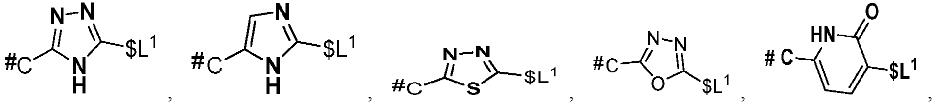 and 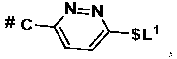 wherein, the 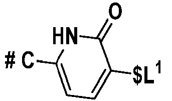 is optionally substituted with substinuent R^{k}, wherein #C represents the attachment point to C, $L¹ represents the attachment point to L¹;
further preferably, A is selected from the group consisting of 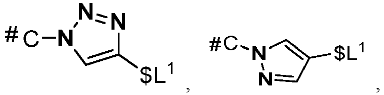 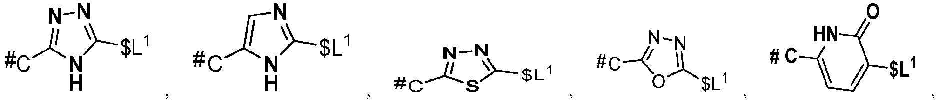 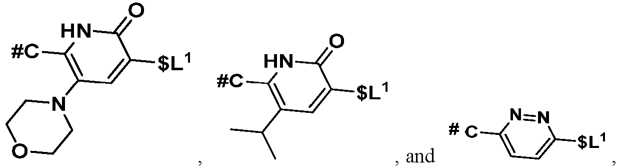 wherein #C represents the attachment point to C, $L¹ represents the attachment point to L¹;
further preferably, A is selected from the group consisting of 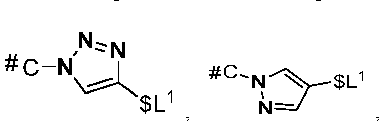 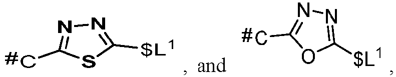 wherein #C represents the attachment point to C, $L¹ represents the attachment point to L¹;
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl; preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl;
R^{p}, R^{q} are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
further preferably, R^{k} is selected from the group consisting of isopropyl, cyclopropyl, dimethylamino, difluoromethyl, and 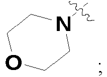
B is selected from any one of groups (1)-(7): 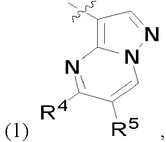 wherein:
   R⁴ is selected from the group consisting of H, C1-C6 alkyl, C4-C9 cycloalkyl, C4-C9 cycloalkyl-C1-C6 alkyl, 4-9 membered saturated heterocyclyl, 4-9 membered saturated heterocyclyl-C1-C6 alkyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl);
   preferably, R⁴ is selected from the group consisting of H, 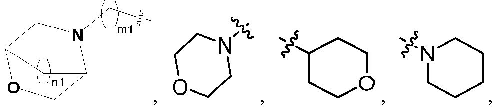 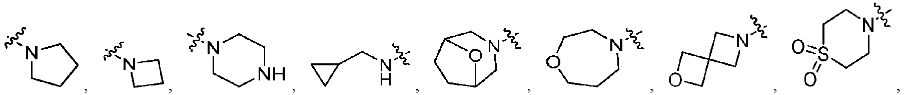 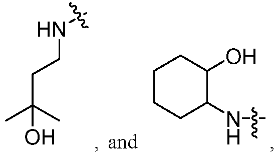 wherein, the 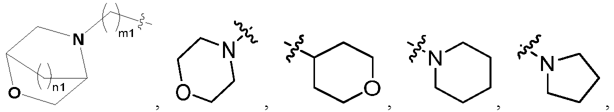 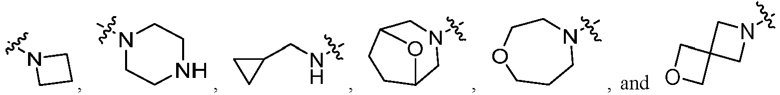 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl); m1 is selected from the group consisting of 0, 1, 2, and 3; m1 is preferably 0; n1 is selected from the group consisting of 0, 1, 2, and 3; n1 is preferably 0, or 1;
   further preferably, R⁴ is selected from the group consisting of H, 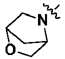 (preferably 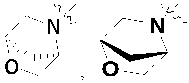 ), 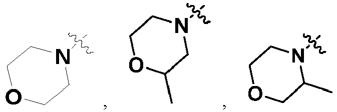 (preferably 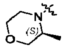 ), 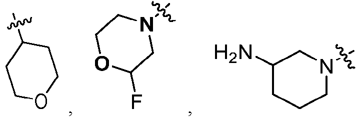 (preferably 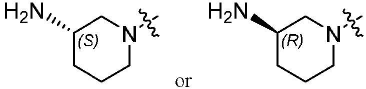 ), 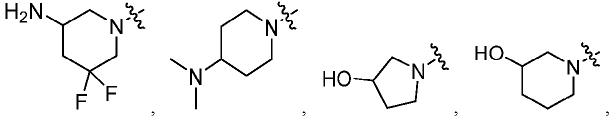 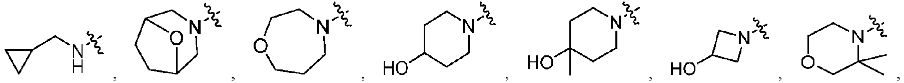 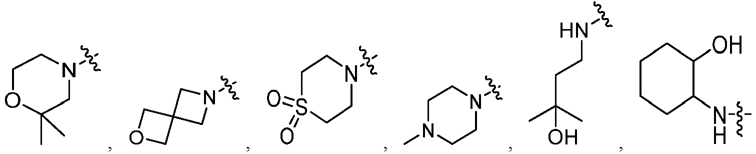 (preferably 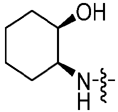 ), 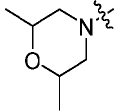 (preferably 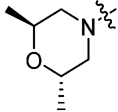 ), 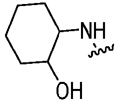 (preferably 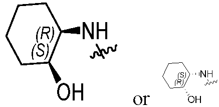 ), and 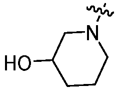 (preferably 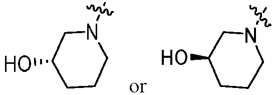 );
   R⁵ is selected from the group consisting of H, cyano, C1-C6 alkyl, 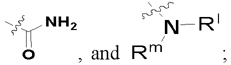 preferably, R⁵ is selected from the group consisting of H, cyano, 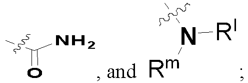
   R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl;
   preferably, R⁵ is selected from the group consisting of H, and cyano;
   further preferably, 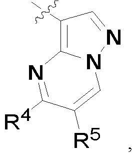 as a whole, is selected from the group consisting of 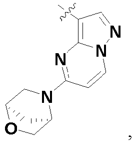 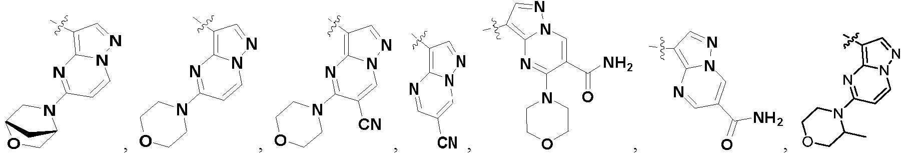 (preferably 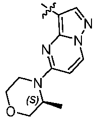 ), 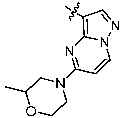 (preferably 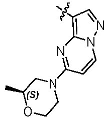 ), 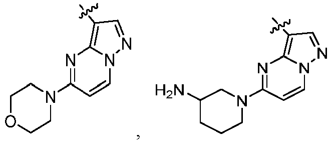 ( 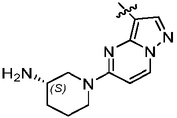 or 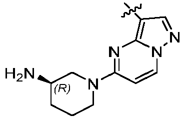 ), 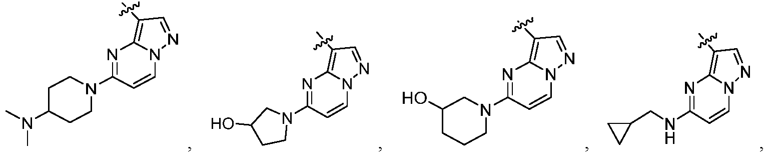 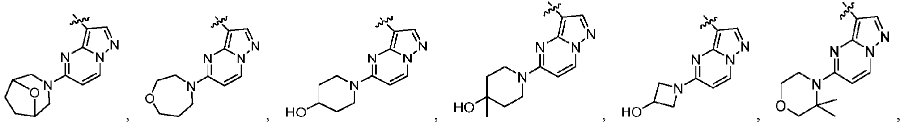 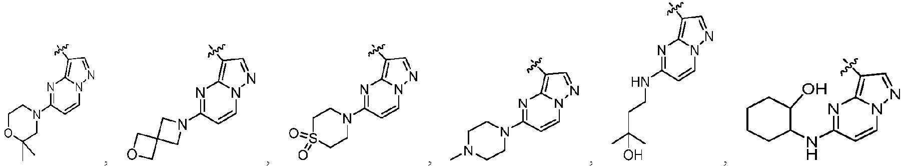 (preferably 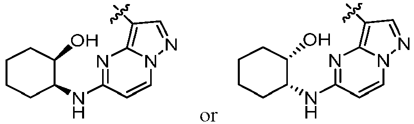 ), 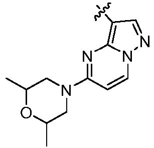 (preferably 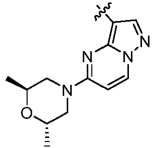 ), 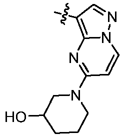 (preferably 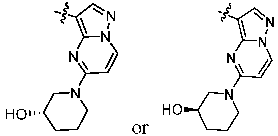 ), and 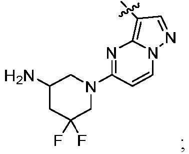
   further preferably, 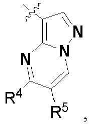 as a whole, is selected from the group consisting of 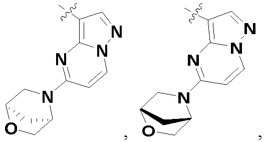 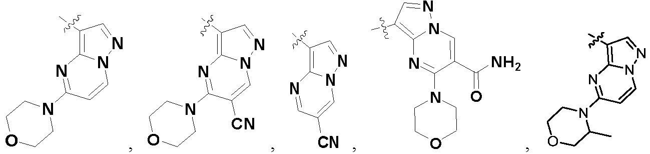 (preferably 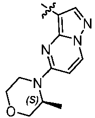 ), 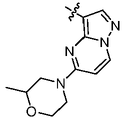 (preferably 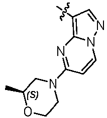 ), 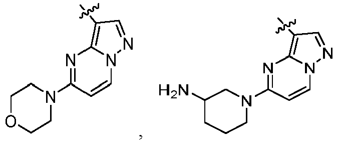 ( 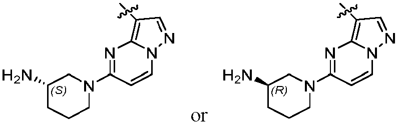 ), 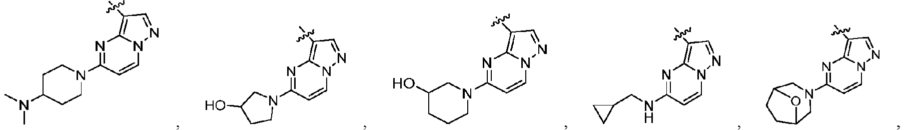 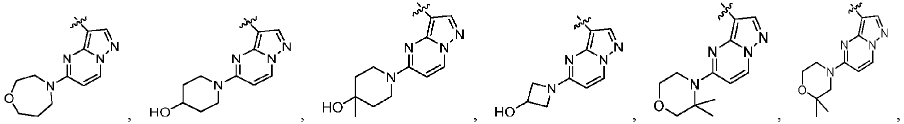 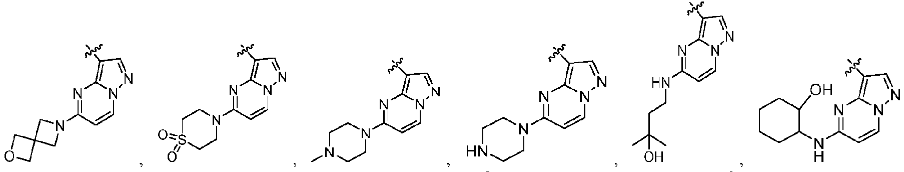 (preferably 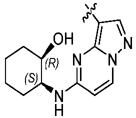 or 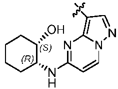 ), 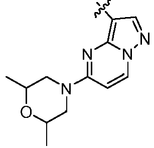 (preferably 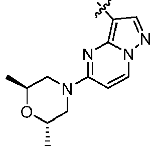 ), 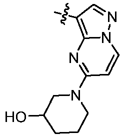 (preferably 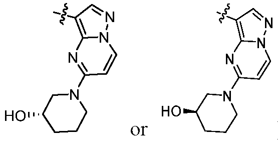 ), and 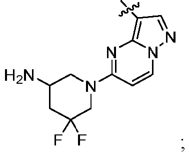 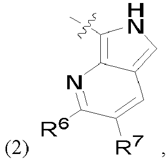
   wherein: R⁶ is selected from the group consisting of H, C1-C6 alkyl, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R⁶ is selected from 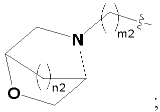 m2 is selected from the group consisting of 0, 1, 2, and 3; n2 is selected from the group consisting of 0, 1, 2, and 3; further preferably, R⁶ is 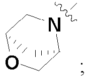
   R⁷ is selected from the group consisting of H, and C1-C6 alkyl;
   further preferably, 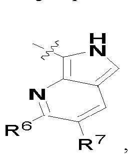 as a whole, is 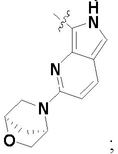 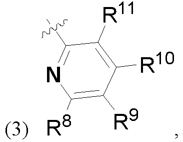
   wherein: one of R⁸, R⁹, R¹⁰, and R¹¹ is selected from the group consisting of cyano, C1-C6 haloalkyl (such as trifluoromethyl), C4-C9 cycloalkyl, C4-C9 cycloalkyl-C1-C6 alkyl, 4-9 membered saturated heterocyclyl, and 4-9 membered saturated heterocyclyl-C1-C6 alkyl, and the rest are H; preferably, among R⁸, R⁹, R¹⁰, and R¹¹, R⁸, R⁹ or R¹¹ is selected from the group consisting of cyano, C1-C6 haloalkyl (such as trifluoromethyl), and 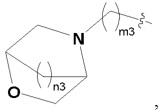 and the rest are H; m3 is selected from the group consisting of 0, 1, 2, and 3; n3 is selected from the group consisting of 0, 1, 2, and 3;
   further preferably, 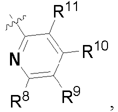 as a whole, is selected from the group consisting of 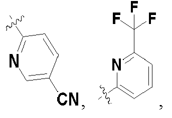 and 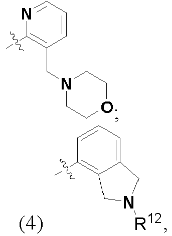 wherein: R¹² is selected from the group consisting of H, and C1-C6 alkyl; preferably, R¹² is selected from C1-C6 alkyl; 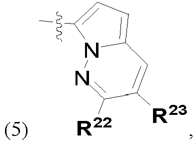
   wherein: R²² is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²² is selected from the group consisting of H, and 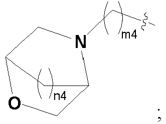 m4 is selected from the group consisting of 0, 1, 2, and 3; n4 is selected from the group consisting of 0, 1, 2, and 3;
   further preferably, R²² is selected from the group consisting of H, and 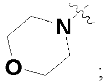
   R²³ is selected from the group consisting of H, C1-C6 alkyl, cyano, carboxyl, 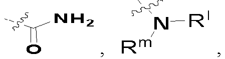 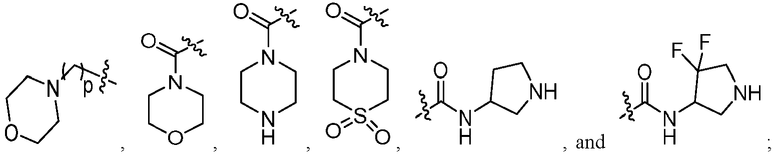 preferably, R²³ is selected from the group consisting of H, C1-C6 alkyl, cyano, 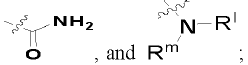 preferably, R²³ is selected from the group consisting of H, cyano, carboxyl, 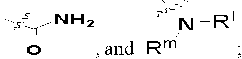 preferably, R²³ is selected from the group consisting of H, cyano, 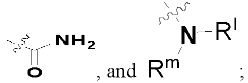
   R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl;
   further preferably, 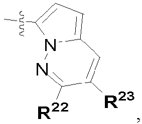 as a whole, is selected from the group consisting of 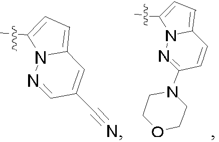 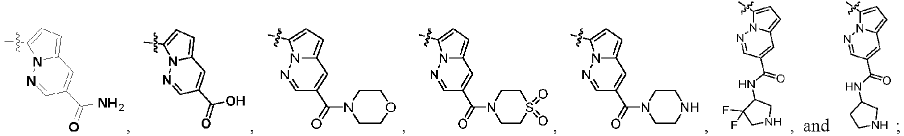 further preferably, 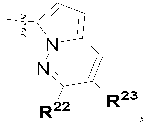 as a whole, is selected from the group consisting of 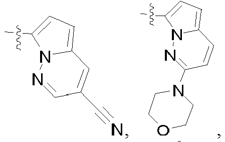 and 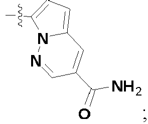 further preferably, 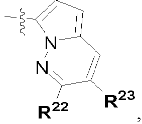 as a whole, is selected from the group consisting of 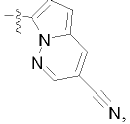 and 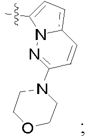 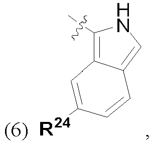
   wherein: R²⁴ is selected from the group consisting of H, C1-C6 alkyl, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²⁴ is selected from 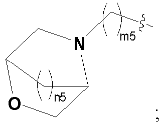 m5 is selected from the group consisting of 0, 1, 2, and 3; n5 is selected from the group consisting of 0, 1, 2, and 3; further preferably, R²⁴ is 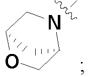 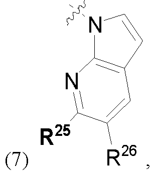
   wherein: R²⁵ is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²⁵ is selected from the group consisting of H, 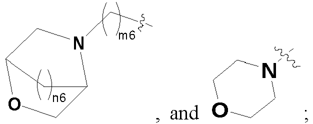 m6 is selected from the group consisting of 0, 1, 2, and 3; n6 is selected from the group consisting of 0, 1, 2, and 3; further preferably, R²⁵ is selected from the group consisting of H, and 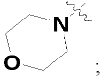 preferably, R²⁵ is H;
   R²⁶ is selected from the group consisting of H, C1-C6 alkyl, cyano, carboxyl, and 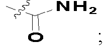 preferably, R²⁶ is selected from the group consisting of H, C1-C6 alkyl, cyano, and 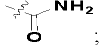 preferably, R²⁶ is selected from the group consisting of H, C1-C6 alkyl, and 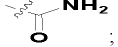
   further preferably, 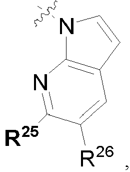 as a whole, is selected from the group consisting of 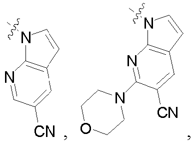 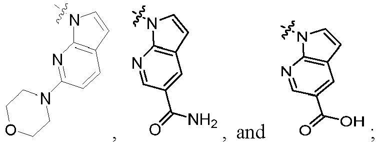 further preferably, 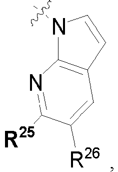 as a whole, is selected from the group consisting of 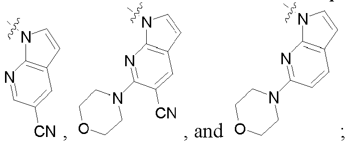
   L¹ is selected from the group consisting of a direct bond, C1-C6 alkyl, 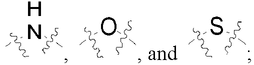 preferably, L¹ is selected from the group consisting of a direct bond, and 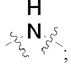 preferably, L¹ is a direct bond;
   C is selected from any one of groups (1)-(10): and 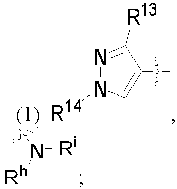 wherein: R¹³ is selected from the group consisting of C1-C6 haloalkyl (such as difluoromethyl), R^{h}, Rⁱ are each independently selected from the group consisting of H, and C1-C6 alkyl; preferably, R^{h}, Rⁱ are each independently selected from the group consisting of H, methyl, and isopropyl;
   preferably, R¹³ is selected from the group consisting of 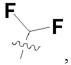 trifluoromethyl, 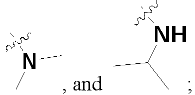 preferably, R¹³ is selected from the group consisting of 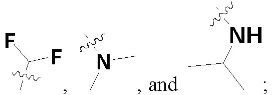 further preferably, R¹³ is selected from the group consisting of 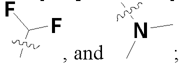
   R¹⁴ is selected from the group consisting of C3-C6 cycloalkyl, and piperidyl, wherein, the C3-C6 cycloalkyl and piperidyl are optionally substituted with 1-2 substituents selected from the group consisting of hydroxy C1-C6 alkyl (such as methylol), formyl, and C1-C6 alkyl;
   preferably, R¹⁴ is selected from C3-C6 cycloalkyl optionally substituted with 1-2 substituents selected from the group consisting of hydroxy C1-C6 alkyl (such as methylol), and formyl;
   preferably, R¹⁴ is selected from 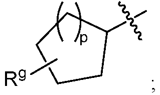 p is selected from the group consisting of 0, 1, and 2; R^{g} is selected from the group consisting of H, hydroxy C1-C6 alkyl, and formyl;
   or, preferably, R¹⁴ is piperidyl optionally substituted with 1-2 substituents selected from C1-C6 alkyl (preferably methyl);
   further preferably, R¹⁴ is selected from the group consisting of 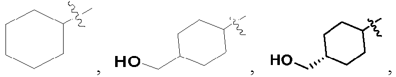 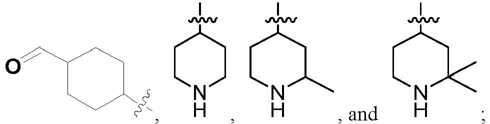
   further preferably, 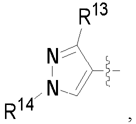 as a whole, is selected from the group consisting of 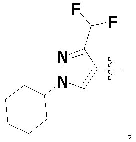 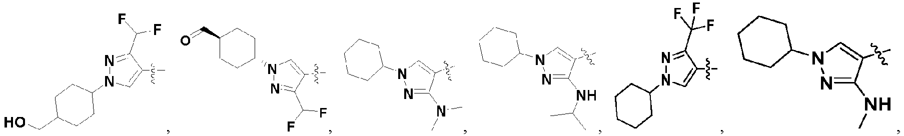 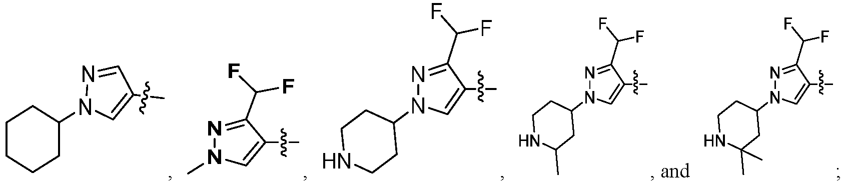
   further preferably, 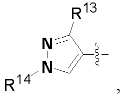 as a whole, is selected from the group consisting of 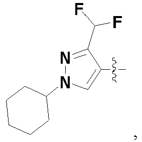 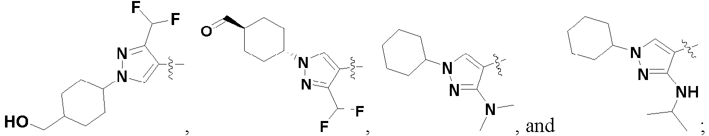
   further preferably, 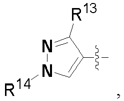 as a whole, is selected from the group consisting of 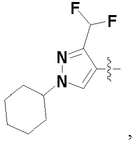 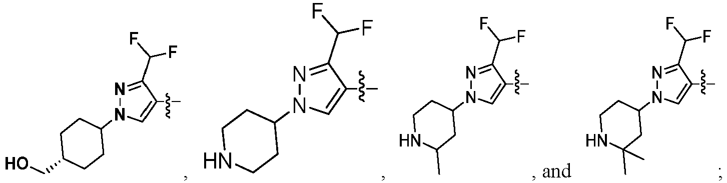 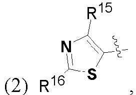
   wherein: R¹⁵ is selected from C1-C6 haloalkyl; R¹⁶ is selected from C3-C6 cycloalkyl; further preferably, 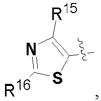 as a whole, is 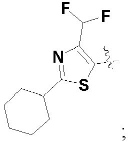 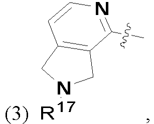
   wherein: R¹⁷ is selected from the group consisting of H, and C1-C6 alkyl; preferably, 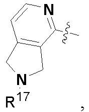 as a whole, is 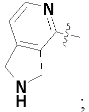 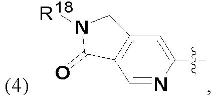
   wherein: R¹⁸ is selected from the group consisting of H, and C1-C6 alkyl; preferably, R¹⁸ is selected from C1-C6 alkyl; further preferably, 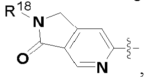 as a whole, is 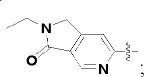 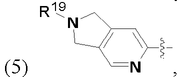
   wherein: R¹⁹ is selected from the group consisting of H, and C1-C6 alkyl; preferably, R¹⁹ is selected from C1-C6 alkyl; further preferably, 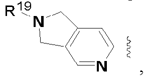 as a whole, is 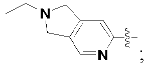 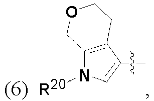
   wherein: R²⁰ is selected from C3-C6 cycloalkyl; further preferably, 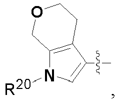 as a whole, is 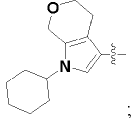 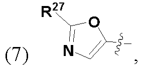
   wherein: R²⁷ is selected from the group consisting of phenyl, and C3-C6 cycloalkyl; further preferably, 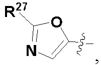 as a whole, is selected from the group consisting of 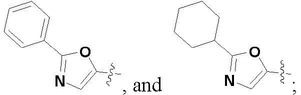 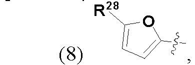 wherein: R²⁸ is selected from 6 membered saturated heterocyclyl; preferably, R²⁸ is morpholinyl; further preferably, 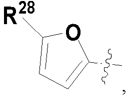 as a whole, is 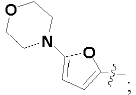 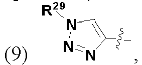
   wherein: R²⁹ is selected from C3-C6 alkyl; further preferably, 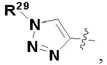 as a whole, is 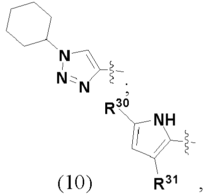 wherein: R³⁰ is selected from C3-C6 cycloalkyl; R³¹ is selected from C1-C6 alkyl; further preferably, 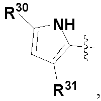 as a whole, is 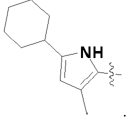
   In the eighth aspect, the present invention provides a compound represented by formula I-1-1, or a stereoisomer, a tautomer, a prodrug, a crystal form, a hydrate, an isotope-labeled compound, a metabolite, an ester, a pharmaceutically acceptable salt, or a pharmaceutically acceptable solvate thereof, 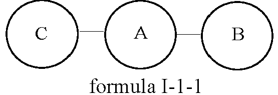
   wherein:
      A is selected from 5-6 membered heteroaromatic ring optionally substituted with substituent R^{k}; preferably, A is selected from the group consisting of 5 membered heteroaromatic ring, and 6 membered heteroaromatic ring, the 5 membered heteroaromatic ring containing 2-3 heteroatoms, wherein at least two heteroatoms are N atoms; the 6 membered heteroaromatic ring containing 1-2 heteroatoms, wherein at least one heteroatom is N atom, and, the 5 membered heteroaromatic ring and 6 membered heteroaromatic ring are optionally substituted with substituent R^{k}; further preferably, A is selected from the group consisting of 1,2,3-triazole ring, 1,2,4-triazole ring, pyrazole ring, imidazole ring, 1,3,4-thiadiazole ring, 1,3,4-oxadiazole ring, 2-pyridone, and pyridazine, wherein, the 2-pyridone is optionally substituted with substituent R^{k}; further preferably, A is selected from the group consisting of 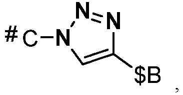 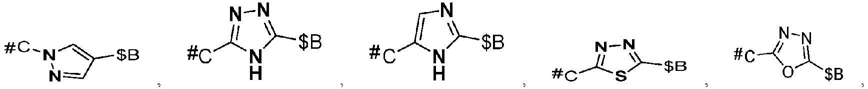 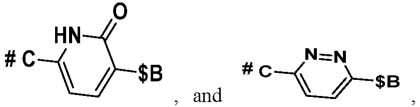 wherein, the 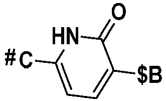 is optionally substituted with substituent R^{k}, wherein #C represents the attachment point to C, $B represents the attachment point to B; further preferably, A is selected from the group consisting of 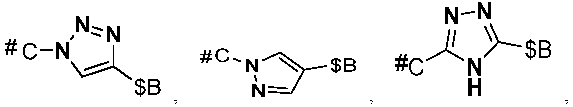 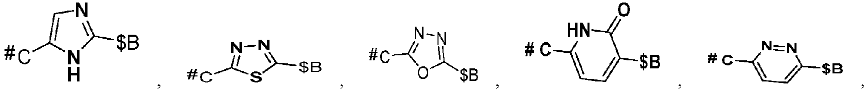 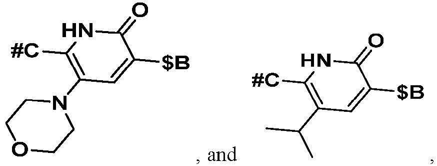 wherein #C represents the attachment point to C, $B represents the attachment point to B;
      further preferably, A is selected from the group consisting of 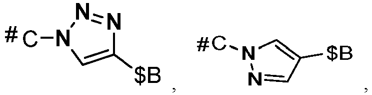 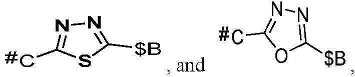 wherein #C represents the attachment point to C, $B represents the attachment point to B;
      R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl;
      preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl;
      R^{p}, R^{q} are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
      further preferably, R^{k} is selected from the group consisting of isopropyl, cyclopropyl, dimethylamino, difluoromethyl, and 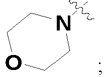
      B is selected from any one of groups (1)-(7): 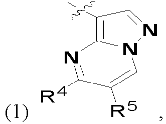
         wherein: R⁴ is selected from the group consisting of H, C1-C6 alkyl, C4-C9 cycloalkyl, 4-9 membered saturated heterocyclyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl);
         preferably, R⁴ is selected from the group consisting of H, 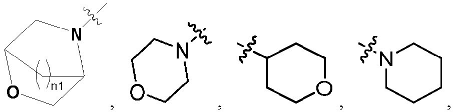 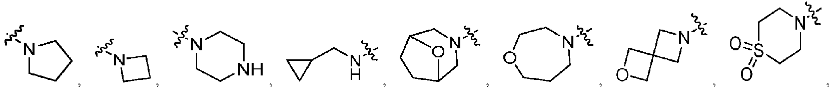 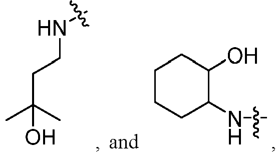 wherein, the 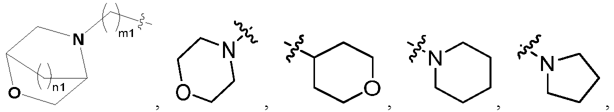 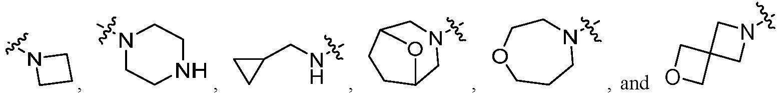 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl); n1 is selected from the group consisting of 0, 1, 2, and 3;
         further preferably, R⁴ is selected from the group consisting of H, 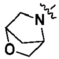 (preferably 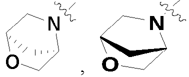 ), 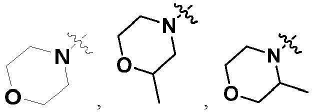 (preferably 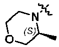 ), 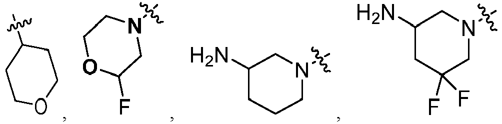 (preferably 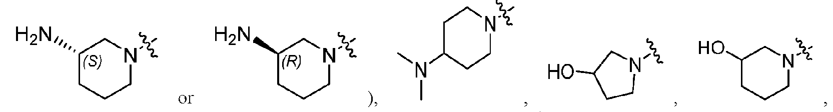 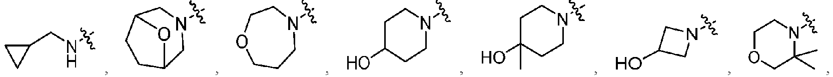 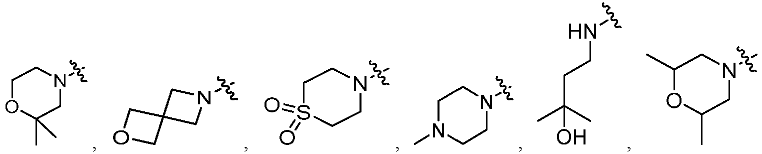 (preferably 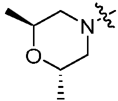 ), 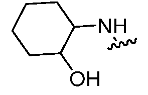 (preferably 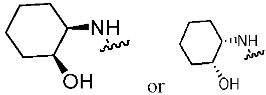 ), and 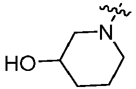 (preferably 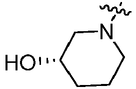 or 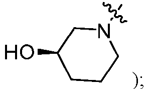
         R⁵ is selected from the group consisting of H, cyano, C1-C6 alkyl, 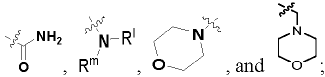 preferably, R⁵ is selected from the group consisting of H, cyano, C1-C6 alkyl, 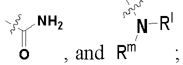 preferably, R⁵ is selected from the group consisting of H, cyano, 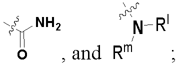 preferably, R⁵ is selected from the group consisting of H, cyano, and 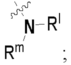
         R^{l}, R^{m} are each independently selected from the group consisting of H, C1-C6 alkyl, and C3-C6 cycloalkyl; preferably, R^{l}, R^{m} are each independently selected from the group consisting of H, and C1-C6 alkyl;
         further preferably, 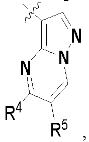 as a whole, is selected from the group consisting of 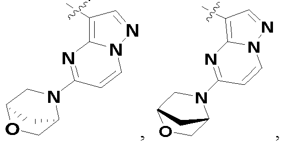 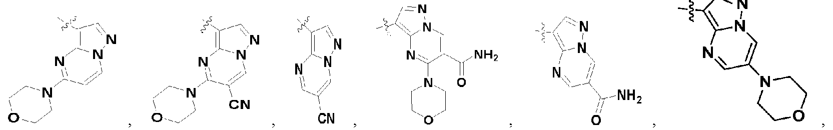 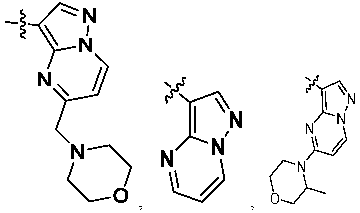 (preferably 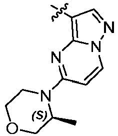 ), 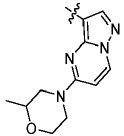 (preferably 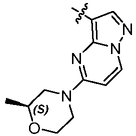 ), 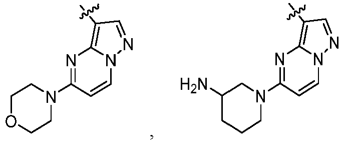 ( 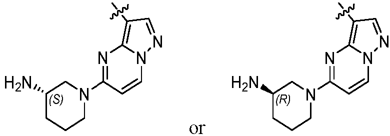 ), 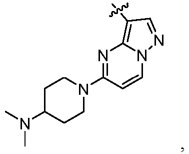 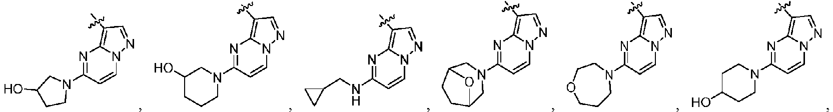 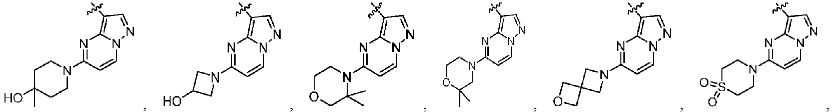 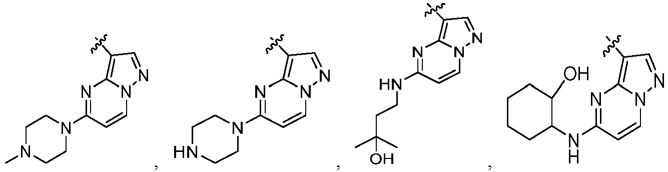 (preferably 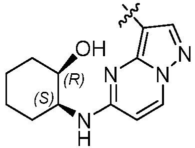 or 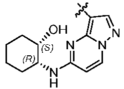 ), 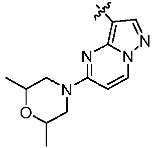 (preferably 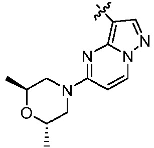 ), 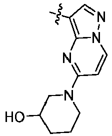 (preferably 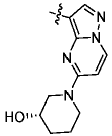 or 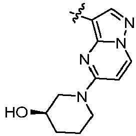 ), and 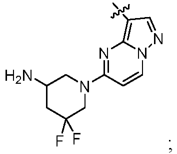
         further preferably, 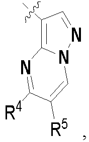 as a whole, is selected from the group consisting of 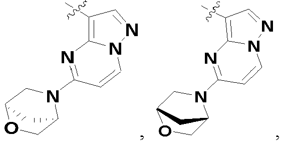 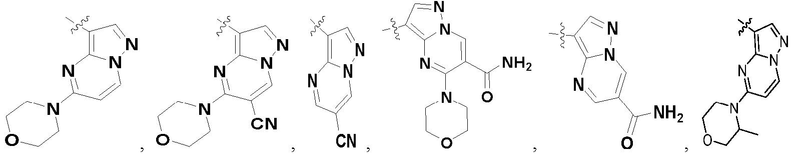 (preferably 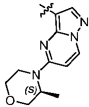 ), 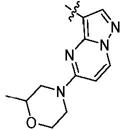 (preferably 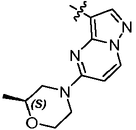 ), 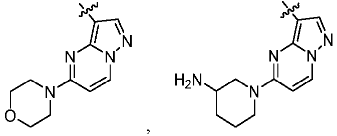 ( 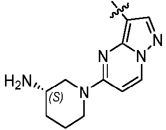 or 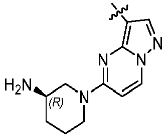 ), 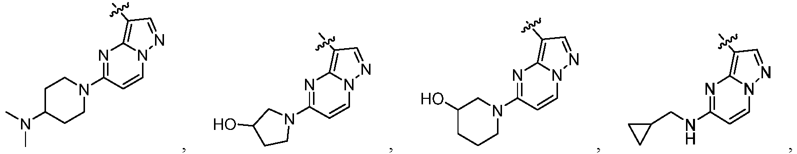 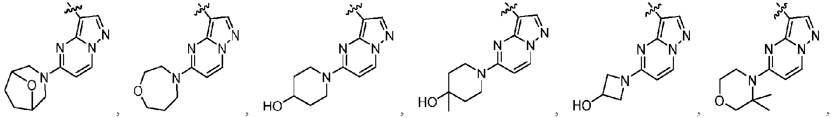 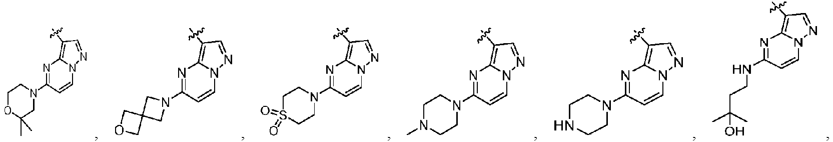 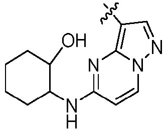 (preferably 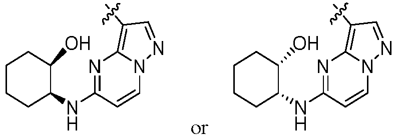 ), 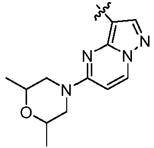 (preferably 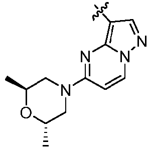 ), 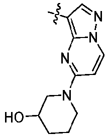 (preferably 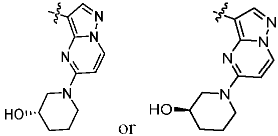 ), and 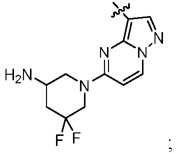
         further preferably, 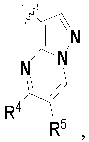 as a whole, is selected from the group consisting of 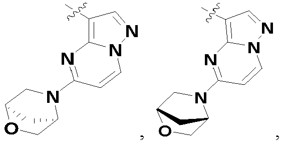 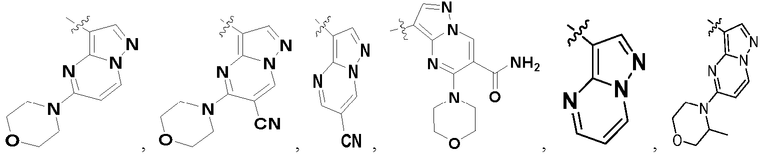 (preferably 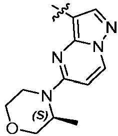 ), 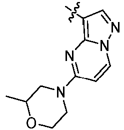 (preferably 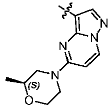 ), 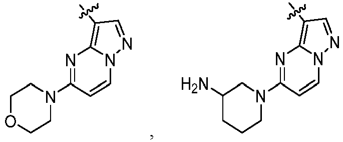 ( 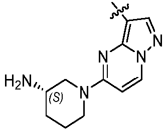 or 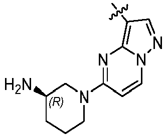 ), 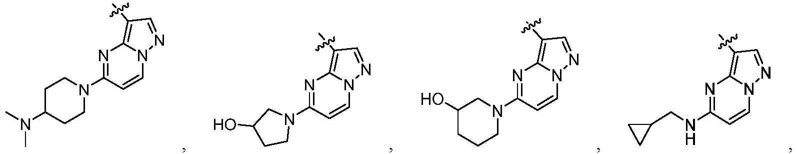 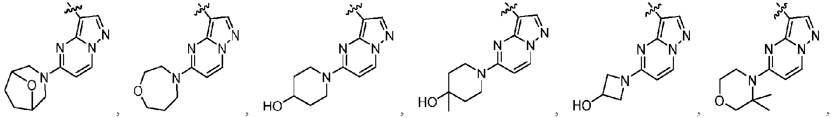 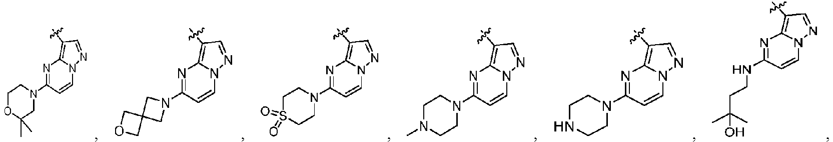 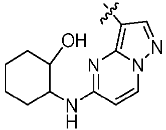 (preferably 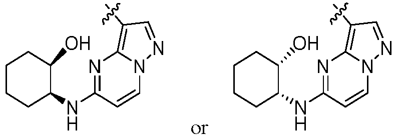 ), 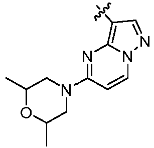 (preferably 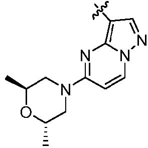 ), 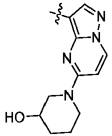 (preferably 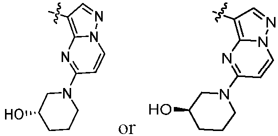 ), and 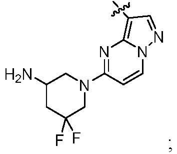
         further preferably, 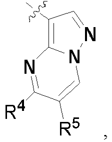 as a whole, is selected from the group consisting of 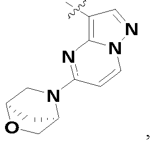 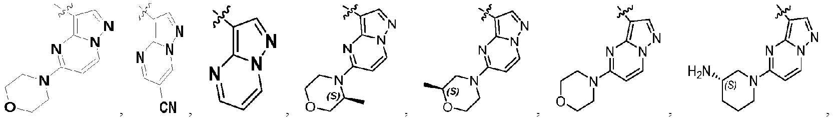 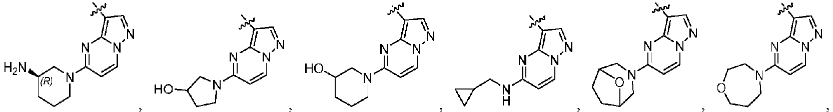 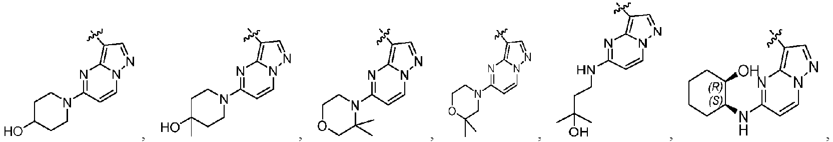 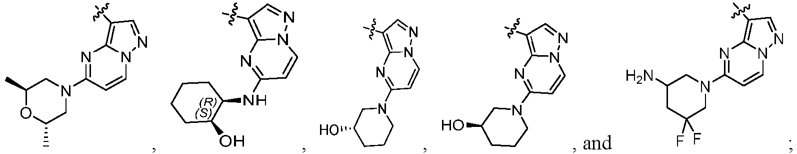 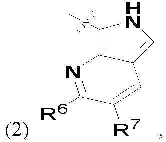
         wherein: R⁶ is selected from the group consisting of H, C1-C6 alkyl, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R⁶ is selected from 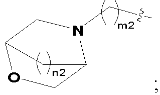 m2 is selected from the group consisting of 0, 1, 2, and 3; n2 is selected from the group consisting of 0, 1, 2, and 3; further preferably, R⁶ is 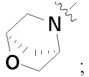
         R⁷ is selected from the group consisting of H, and C1-C6 alkyl; further preferably, 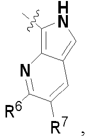 as a whole, is 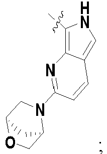 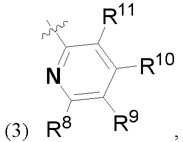
         wherein: one of R⁸, R⁹, R¹⁰, and R¹¹ is selected from the group consisting of cyano, C1-C6 haloalkyl (such as trifluoromethyl), C4-C9 cycloalkyl, C4-C9 cycloalkyl-C1-C6 alkyl, 4-9 membered saturated heterocyclyl, and 4-9 membered saturated heterocyclyl-C1-C6 alkyl, and the rest are H; preferably, among R⁸, R⁹, R¹⁰, and R¹¹, R⁸, R⁹ or R¹¹ is selected from the group consisting of cyano, C1-C6 haloalkyl (such as trifluoromethyl), and 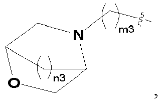 and the rest are H; m3 is selected from the group consisting of 0, 1, 2, and 3; n3 is selected from the group consisting of 0, 1, 2, and 3;
         further preferably, 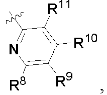 as a whole, is selected from the group consisting of 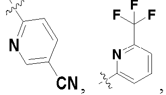 and 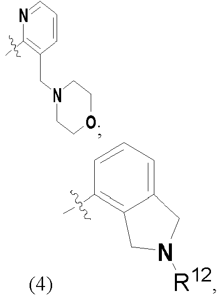 wherein: R¹² is selected from the group consisting of H, and C1-C6 alkyl; preferably, R¹² is selected from C1-C6 alkyl; 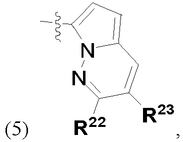
         wherein: R²² is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²² is selected from the group consisting of H, and 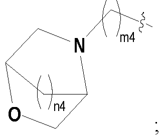 m4 is selected from the group consisting of 0, 1, 2, and 3; m4 is preferably 0; n4 is selected from the group consisting of 0, 1, 2, and 3; n4 is preferably 0, or 1; further preferably, R²² is selected from the group consisting of H, and 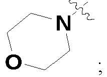
         R²³ is selected from the group consisting of H, C1-C6 alkyl, cyano, 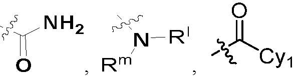 (wherein Cy₁ is 4-9 membered heterocyclyl optionally substituted with R_{cy1} and R_{cy2}), and 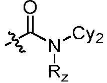 (wherein R_{z} is H or C1-C6 alkyl, Cy₂ is 5-7 membered heterocyclyl, C3-C7 cycloalkyl or C6-C10 aryl, wherein, the 5-7 membered heterocyclyl, C3-C7 cycloalkyl and C6-C10 aryl are optionally substituted with 1-3 substituents selected from the group consisting of halogen, C1-C6 alkyl, C1-C6 alkoxy, CN, and hydroxy);
         preferably, Cy₁ is 5-7 membered heterocyclyl, further preferably, Cy₁ is 6 membered heterocyclyl, further preferably, Cy₁ is morpholinyl, piperazinyl, or thiomorpholinyl;
         R_{cy1} and R_{cy2} are each independently selected from the group consisting of C1-C6 alkyl, C1-C6 alkoxy, C1-C6 haloalkyl, halogen, hydroxy, nitro, cyano, amino, -NH(C1-C6 alkyl), -N(C1-C6 alkyl)(C1-C6 alkyl), and oxo; preferably, R_{cy1} and R_{cy2} are each independently selected from the group consisting of C1-C6 alkyl, halogen, hydroxy, amino, and oxo, further preferably, R_{cy1} and R_{cy2} are oxo;
         preferably, Cy₂ is 5-7 membered heterocyclyl optionally substituted with 1-3 substituents selected from the group consisting of halogen, C1-C6 alkyl, C1-C6 alkoxy, CN, and hydroxy; further preferably, Cy₂ is selected from the group consisting of azetidinyl, pyrrolidinyl, imidazolidinyl, piperidyl, piperazinyl, and morpholinyl, wherein, the azetidinyl, pyrrolidinyl, imidazolidinyl, piperidyl, piperazinyl, and morpholinyl are optionally substituted with 1-3 substituents selected from the group consisting of halogen, and C1-C6 alkyl; further preferably, Cy₂ is selected from the group consisting of pyrrolidinyl, imidazolidinyl, and piperidyl, wherein, the pyrrolidinyl, imidazolidinyl, and piperidyl are optionally substituted with 1-3 substituents selected from the group consisting of F, Cl, Br, and methyl; preferably, R²³ is selected from the group consisting of H, C1-C6 alkyl, cyano, 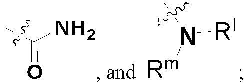
         preferably, R²³ is selected from the group consisting of H, cyano, 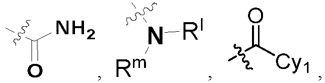 and 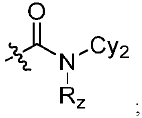 preferably, R²³ is selected from the group consisting of H, cyano, 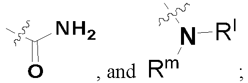
         R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl;
         further preferably, 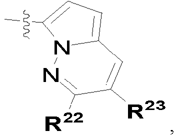 as a whole, is selected from the group consisting of 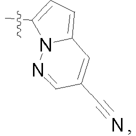 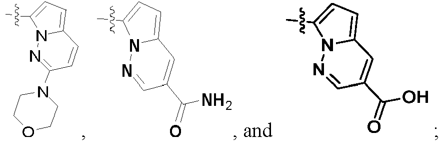
         further preferably, 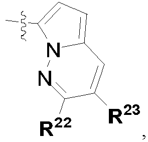 as a whole, is selected from the group consisting of 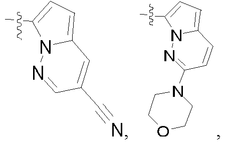 and 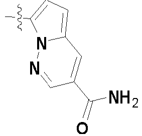
         further preferably, 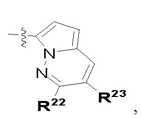 as a whole, is selected from the group consisting of 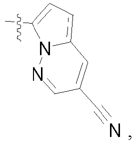 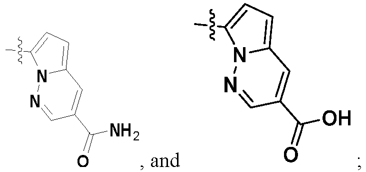 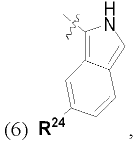
         wherein:
            R²⁴ is selected from the group consisting of H, C1-C6 alkyl, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²⁴ is selected from 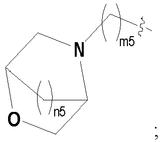 m5 is selected from the group consisting of 0, 1, 2, and 3; n5 is selected from the group consisting of 0, 1, 2, and 3; further preferably, R²⁴ is 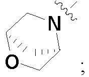 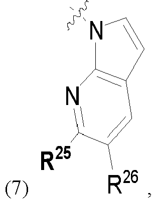
            wherein: R²⁵ is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²⁵ is selected from the group consisting of H, and 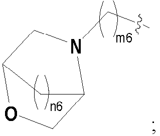 m6 is selected from the group consisting of 0, 1, 2, and 3; m6 is preferably 0; n6 is selected from the group consisting of 0, 1, 2, and 3; n6 is preferably 0, or 1; further preferably, R²⁵ is selected from the group consisting of H, and 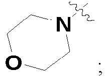
            R²⁶ is selected from the group consisting of H, C1-C6 alkyl, cyano, carboxyl, and 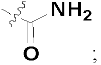 preferably, R²⁶ is selected from the group consisting of H, C1-C6 alkyl, cyano, and 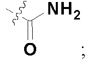 preferably, R²⁶ is selected from the group consisting of H, cyano, and 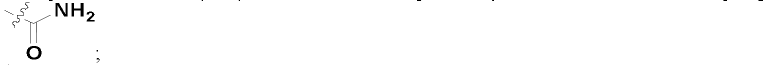
            further preferably, 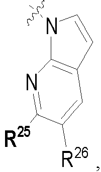 as a whole, is selected from the group consisting of 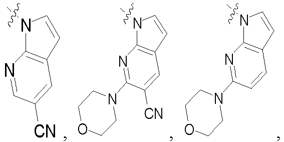 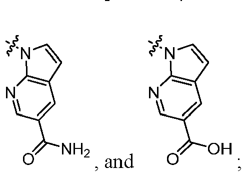 further preferably, 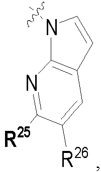 as a whole, is selected from the group consisting of 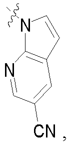 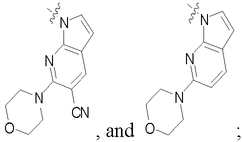 further preferably, 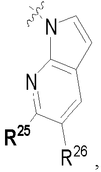 as a whole, is selected from the group consisting of 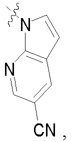 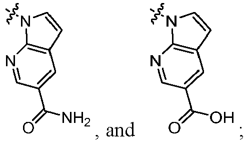
            C is selected from any one of groups (1)-(11): 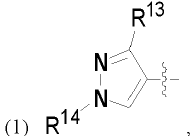 wherein: R¹³ is selected from the group consisting of C1-C6 haloalkyl (such as difluoromethyl), and 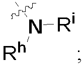 R^{h}, Rⁱ are each independently selected from the group consisting of H, and C1-C6 alkyl; preferably, R^{h}, Rⁱ are each independently selected from the group consisting of H, methyl, and isopropyl;
            preferably, R¹³ is selected from the group consisting of 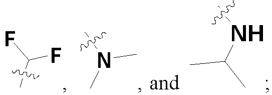 further preferably, R¹³ is selected from the group consisting of 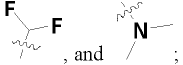
            R¹⁴ is selected from C3-C6 cycloalkyl optionally substituted with 1-2 substituents selected from the group consisting of hydroxy C1-C6 alkyl (such as methylol), and formyl; or R¹⁴ is selected from the group consisting of phenyl, morpholinyl, piperazinyl, and piperidyl, wherein, the phenyl, morpholinyl, piperazinyl, and piperidyl are optionally substituted with 1-2 substituents selected from the group consisting of C1-C6 alkyl, halogen, alkoxy (such as C1-C6 alkoxy), hydroxy, amino, hydroxy C1-C6 alkyl (such as methylol), amino C1-C6 alkyl, and formyl, preferably, R¹⁴ is selected from 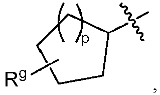 p is selected from the group consisting of 0, 1, and 2; or preferably, R¹⁴ is piperidyl optionally substituted with 1-2 substituents selected from the group consisting of C1-C6 alkyl (preferably methyl), halogen, hydroxy, and amino;
            R^{g} is selected from the group consisting of H, hydroxy C1-C6 alkyl, and formyl;
            further preferably, R¹⁴ is selected from the group consisting of 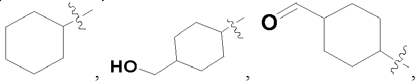 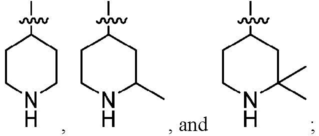
            further preferably, R¹⁴ is selected from the group consisting of 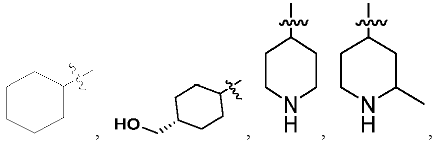 and 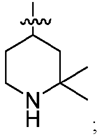
            further preferably, 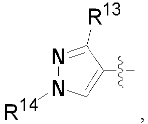 as a whole, is selected from the group consisting of 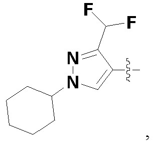 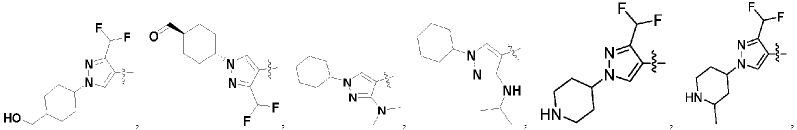 and 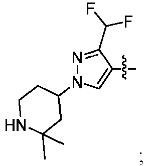
            further preferably, 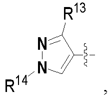 as a whole, is selected from the group consisting of 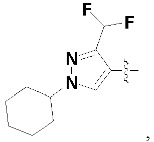 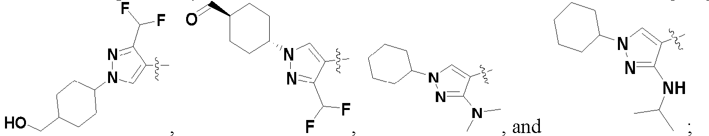
            further preferably, 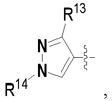 as a whole, is selected from the group consisting of 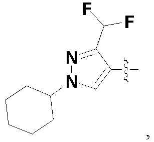 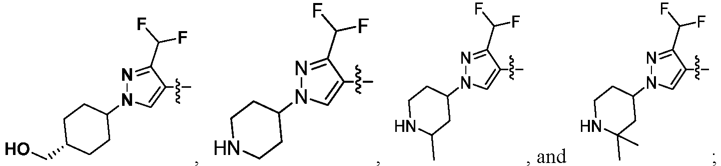 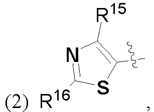
            wherein: R¹⁵ is selected from C1-C6 haloalkyl; R¹⁶ is selected from C3-C6 cycloalkyl; further preferably, 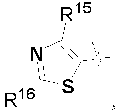 as a whole, is 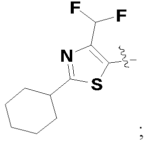 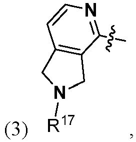
            wherein: R¹⁷ is selected from the group consisting of H, and C1-C6 alkyl; preferably, 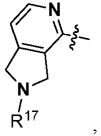 as a whole, is 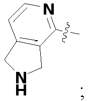 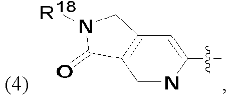
            wherein: R¹⁸ is selected from the group consisting of H, and C1-C6 alkyl; preferably, R¹⁸ is selected from C1-C6 alkyl; further preferably, 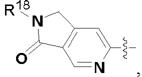 as a whole, is 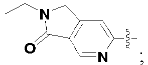 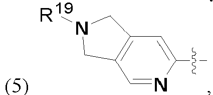
            wherein: R¹⁹ is selected from the group consisting of H, and C1-C6 alkyl; preferably, R¹⁹ is selected from C1-C6 alkyl; further preferably, 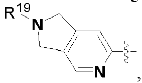 as a whole, is 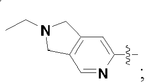 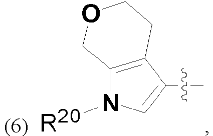
            wherein: R²⁰ is selected from C3-C6 cycloalkyl; further preferably, 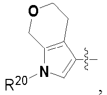 as a whole, is 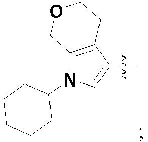 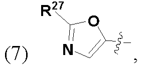
            wherein: R²⁷ is selected from the group consisting of phenyl, and C3-C6 cycloalkyl; further preferably, 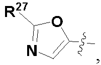 as a whole, is selected from the group consisting of 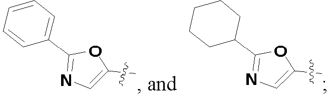 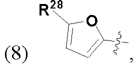
            wherein: R²⁸ is selected from 6 membered saturated heterocyclyl; preferably, R²⁸ is morpholinyl; further preferably, 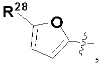 as a whole, is 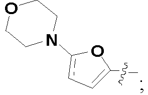 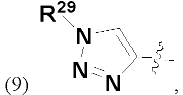
            wherein: R²⁹ is selected from C3-C6 alkyl; further preferably, 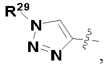 as a whole, is 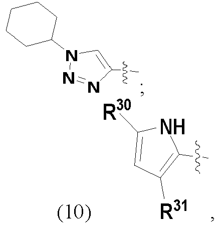 wherein: R³⁰ is selected from C3-C6 cycloalkyl; R³¹ is selected from C1-C6 alkyl; further preferably, 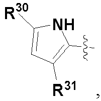 as a whole, is 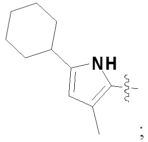 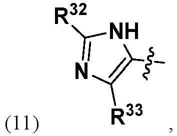 wherein: R³² is selected from the group consisting of phenyl, and C3-C6 cycloalkyl; R³³ is selected from the group consisting of C1-C6 alkyl, and C1-C6 haloalkyl (such as trifluoromethyl, difluoromethyl); further preferably, 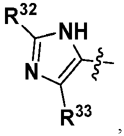 as a whole, is

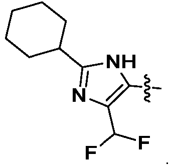

In the ninth aspect, the present invention provides a compound represented by formula II, or a stereoisomer, a tautomer, a prodrug, a crystal form, a hydrate, an isotope-labeled compound, a metabolite, an ester, a pharmaceutically acceptable salt, or a pharmaceutically acceptable solvate thereof, 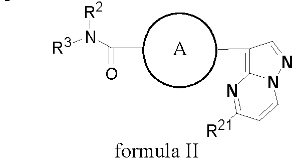 wherein:
A is selected from 5 membered heteroaromatic ring, preferably, A is selected from 5 membered heteroaromatic ring containing 2-3 heteroatoms, wherein at least two heteroatoms are N atoms; further preferably, A is selected from the group consisting of 1,2,3-triazole ring, and imidazole ring; further preferably, A is selected from the group consisting of 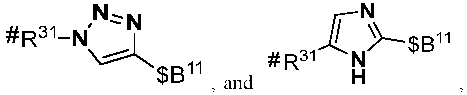 wherein #R³¹ represents the attachment point to carbonyl, $B¹¹ represents the attachment point to fused bicyclic ring;
R², R³ are each independently selected from the group consisting of H, C1-C6 alkyl, and C3-C6 cycloalkyl;
or, R², R³ and the N atom to which they are both attached together form 6-membered saturated heterocyclyl, preferably, the 6 membered saturated heterocyclyl contains 2 heteroatoms, and the heteroatoms are N atom; preferably, R², R³ are each independently selected from the group consisting of H, methyl, ethyl, isopropyl, and 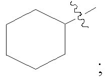
or preferably, R², R³ and the N atom to which they are both attached together form 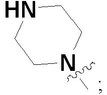
further preferably, 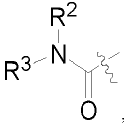 as a whole, is selected from the group consisting of 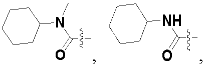 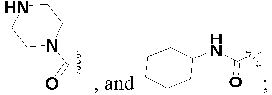
R²¹ is selected from the group consisting of C4-C9 cycloalkyl, C4-C9 cycloalkyl-C1-C6 alkyl, 4-9 membered saturated heterocyclyl, and 4-9 membered saturated heterocyclyl-C1-C6 alkyl;
preferably, R²¹ is selected from 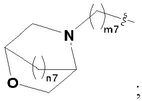 m7 is selected from the group consisting of 0, 1, 2, and 3; n7 is selected from the group consisting of 0, 1, 2, and 3;
further preferably, R²¹ is selected from the group consisting of

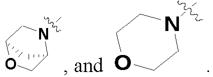

In the tenth aspect, the present invention provides a compound represented by formula III, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula III, 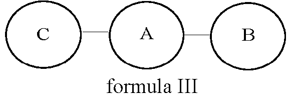 wherein:
A is selected from the group consisting of 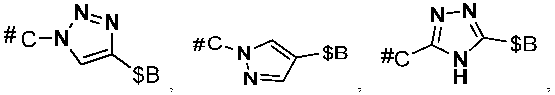 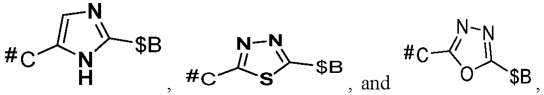 wherein #C represents the attachment point to C, $B represents the attachment point to B; further preferably, A is selected from the group consisting of 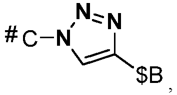 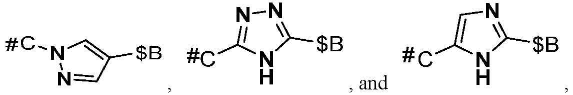 wherein #C represents the attachment point to C, $B represents the attachment point to B; further preferably, A is point to C, $B represents the attachment point to B; 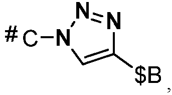 wherein #C represents the attachment
B is 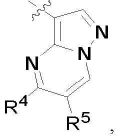 wherein: R⁴ is selected from the group consisting of H, C1-C6 alkyl, C4-C9 cycloalkyl, 4-9 membered saturated heterocyclyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl);
preferably, R⁴ is selected from the group consisting of H, 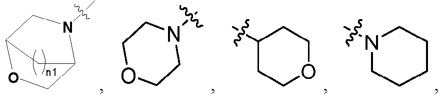 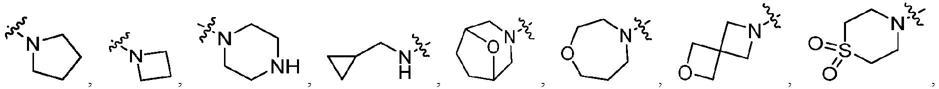 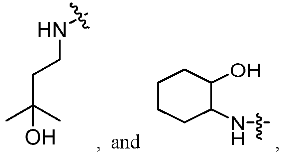 wherein, the 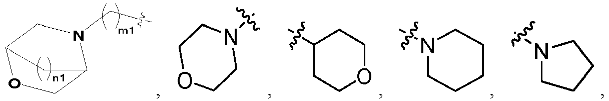 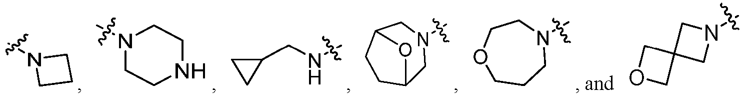 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl); m1 is selected from the group consisting of 0, 1, 2, and 3; n1 is selected from the group consisting of 0, 1, 2, 3; preferably n1 is 0;
further preferably, R⁴ is selected from the group consisting of H, 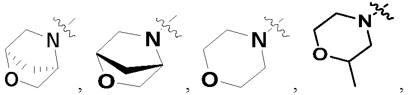 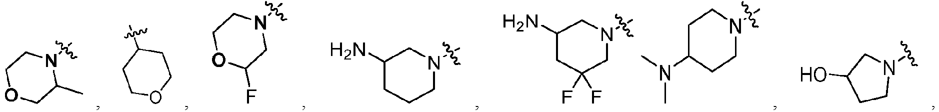 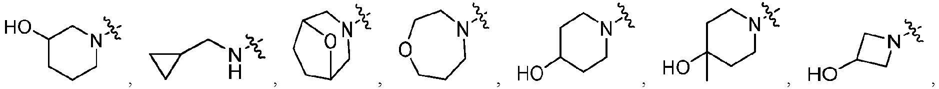 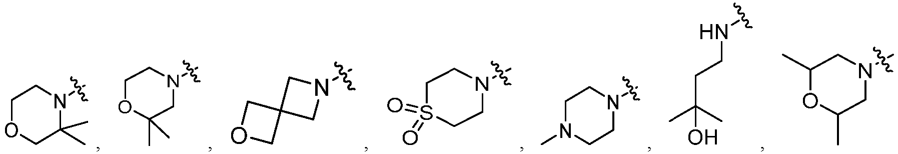 (preferably 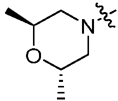 ), 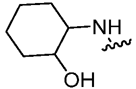 (preferably 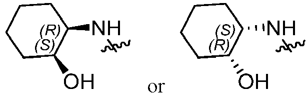 ), and 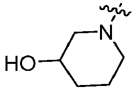 (preferably 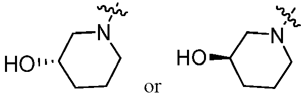 );
further preferably, R⁴ is selected from the group consisting of H, 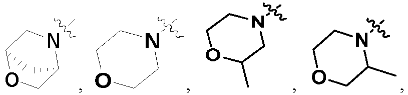 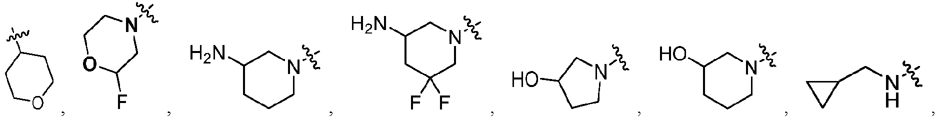 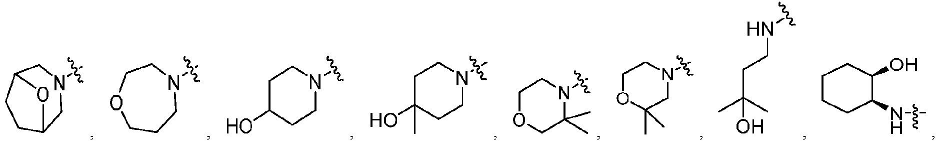 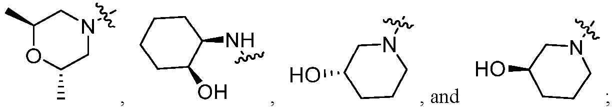
R⁵ is selected from the group consisting of H, cyano, C1-C6 alkyl, 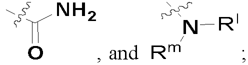 R^{m}, R^{I} are each independently selected from the group consisting of H, and C1-C6 alkyl; preferably, R^{m}, R^{I} are each independently selected from the group consisting of H, methyl, and isopropyl; preferably, R⁵ is selected from the group consisting of H, and cyano;
preferably, 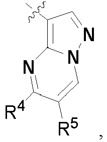 as a whole, is selected from the group consisting of 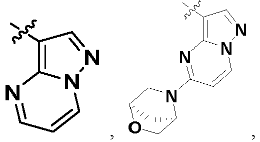 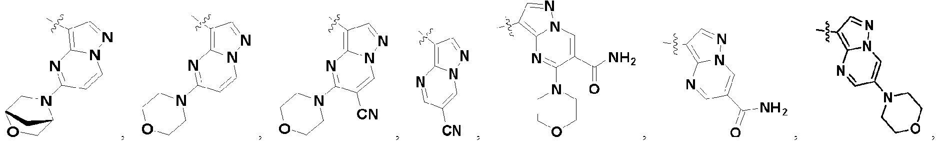 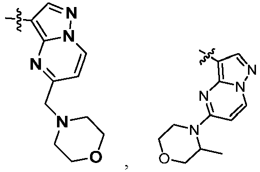 (preferably 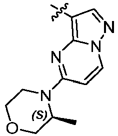 ), 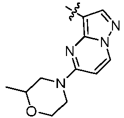 (preferably 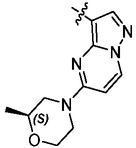 ), 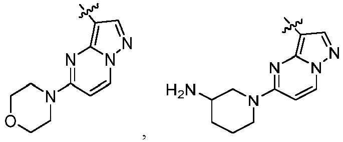 ( 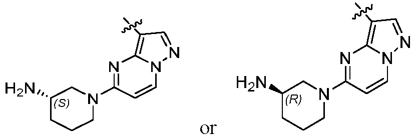 ), 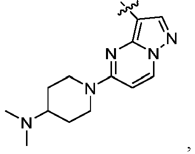 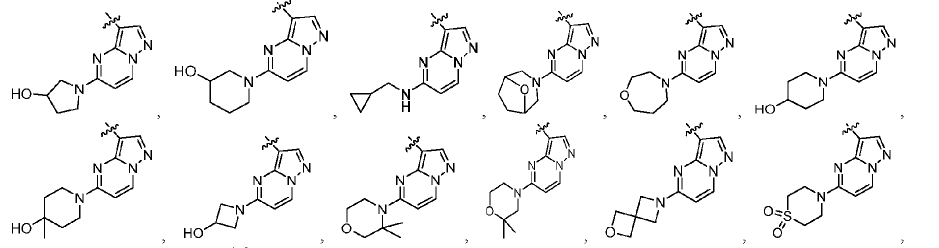 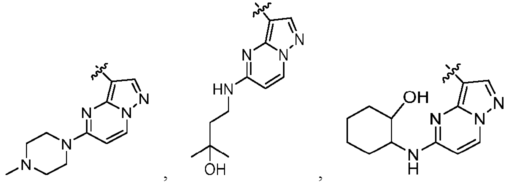 (preferably 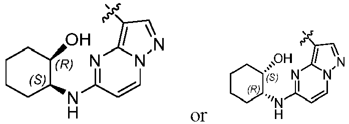 ), 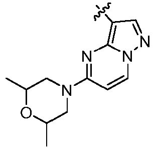 (preferably 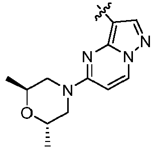 ), 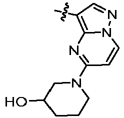 (preferably 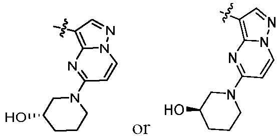 ), and 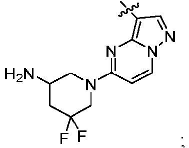
further preferably, 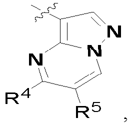 as a whole, is selected from the group consisting of 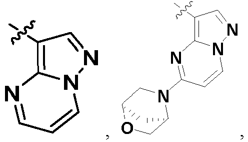 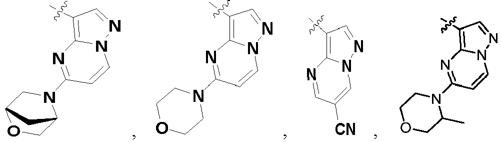 (preferably 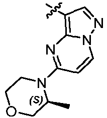 ), 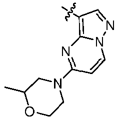 (preferably 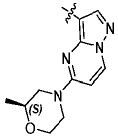 ), 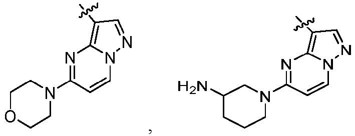 ( 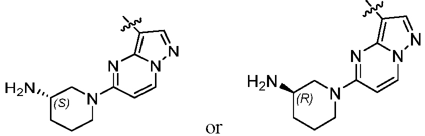 ), 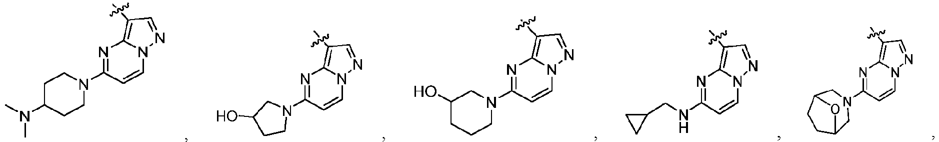 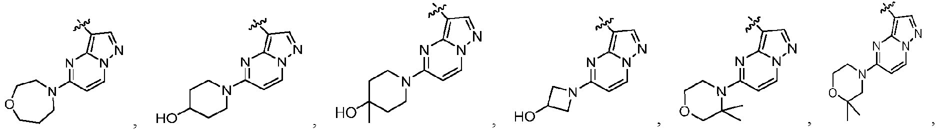 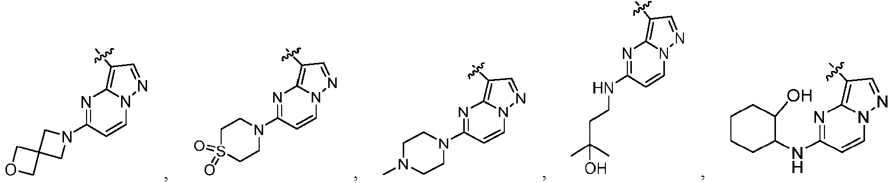 (preferably 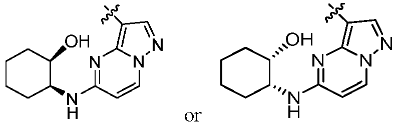 ), 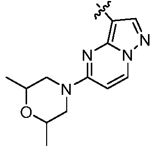 (preferably 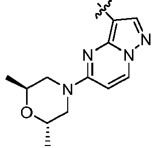 ), 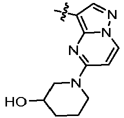 (preferably 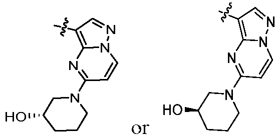 ), and 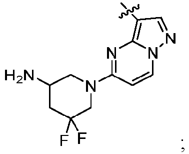
further preferably, 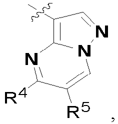 as a whole, is selected from the group consisting of 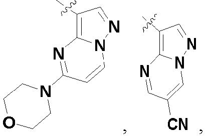 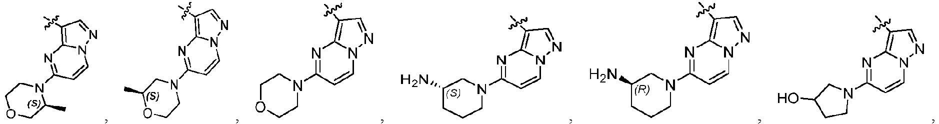 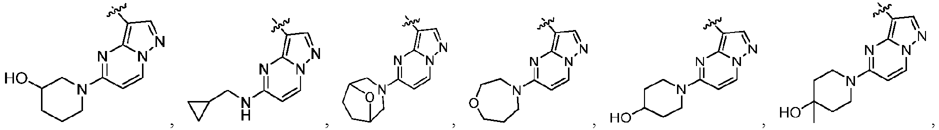 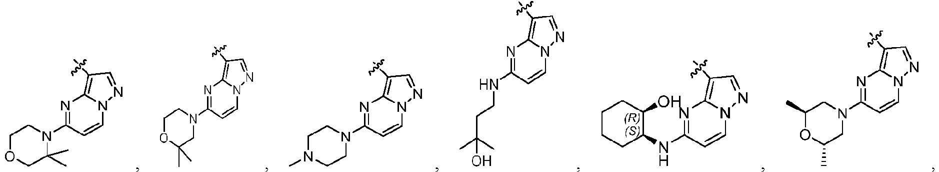 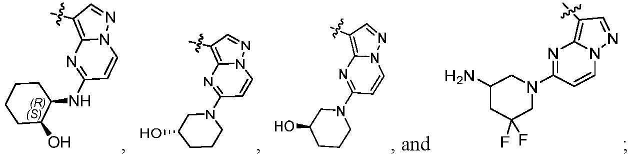 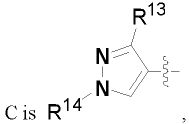 wherein: R¹³ is selected from the group consisting of C1-C6 haloalkyl (such as difluoromethyl or trifluoromethyl), and 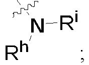
R^{h}, Rⁱ are each independently selected from the group consisting of H, and C1-C6 alkyl; preferably, R^{h}, Rⁱ are each independently selected from the group consisting of H, methyl, and isopropyl; preferably, R¹³ is selected from C1-C6 haloalkyl (such as difluoromethyl);
preferably, R¹³ is selected from the group consisting of 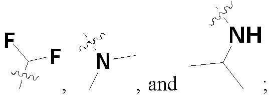 further preferably, R¹³ is selected from the group consisting of 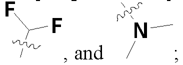
R¹⁴ is selected from the group consisting of C3-C6 cycloalkyl, piperidyl, piperazinyl, and morpholinyl, wherein, the C3-C6 cycloalkyl, piperidyl, piperazinyl, and morpholinyl are optionally substituted with 1-2 substituents selected from the group consisting of hydroxy C1-C6 alkyl (such as methylol), formyl, and C1-C6 alkyl (preferably methyl);
preferably, R¹⁴ is selected from the group consisting of 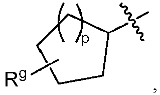 and piperidyl, wherein, the piperidyl is optionally substituted with 1-2 substituents selected from C1-C6 alkyl (preferably methyl); p is selected from the group consisting of 0, 1, and 2; preferably, p is 2;
R^{g} is selected from the group consisting of H, hydroxy C1-C6 alkyl, and formyl; preferably, R^{g} is H;
further preferably, R¹⁴ is selected from the group consisting of 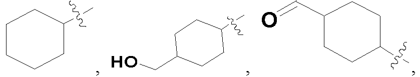 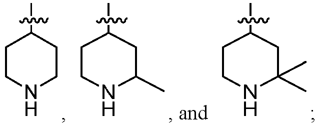
further preferably, R¹⁴ is selected from the group consisting of 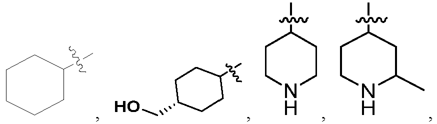 and 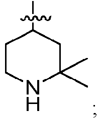
further preferably, 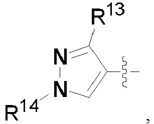 as a whole, is selected from the group consisting of 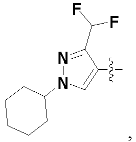 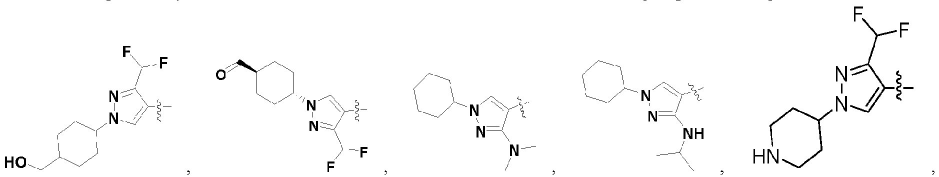 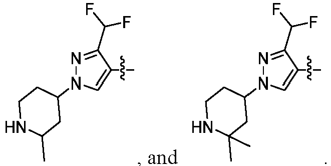
further preferably, 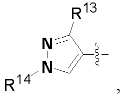 as a whole, is selected from the group consisting of

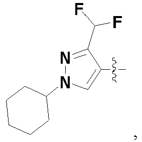

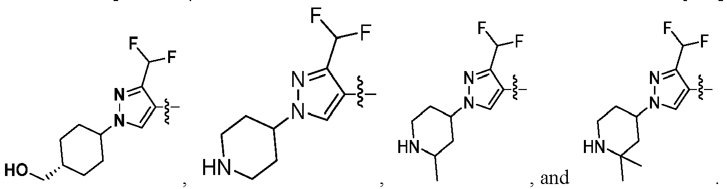

In the eleventh aspect, the present invention provides a compound represented by formula IV, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula IV, 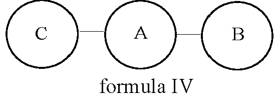 wherein:
A is selected from the group consisting of 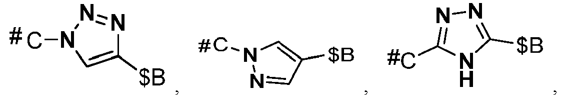 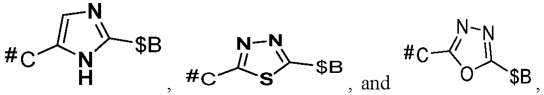 wherein #C represents the attachment point to C, $B represents the attachment point to B;
further preferably, A is selected from the group consisting of 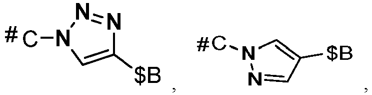 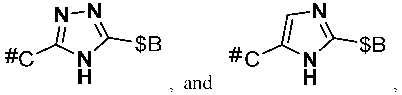 wherein #C represents the attachment point to C, $B represents the attachment point to B; further preferably, A is 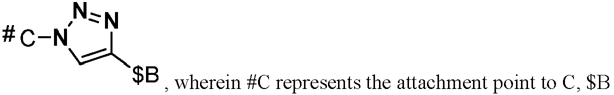 represents the attachment point to B;
B is 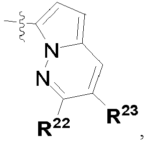 wherein: R²² is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²² is selected from the group consisting of H, and 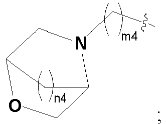
m4 is selected from the group consisting of 0, 1, 2, and 3; preferably, m4 is 0; n4 is selected from the group consisting of 0, 1, 2, and 3; preferably, n4 is selected from the group consisting of 0, and 1;
further preferably, R²² is selected from the group consisting of H, and 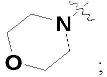
R²³ is selected from the group consisting of H, C1-C6 alkyl, cyano, carboxyl, 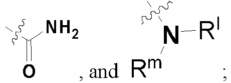 preferably, R²³ is selected from the group consisting of H, carboxyl, and 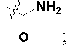
R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl;
further preferably, 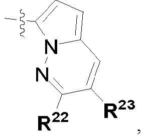 as a whole, is selected from the group consisting of 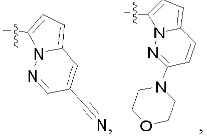 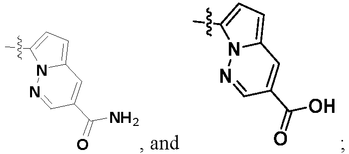
C is 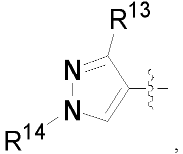 wherein: R¹³ is selected from the group consisting of C1-C6 haloalkyl (such as difluoromethyl or trifluoromethyl), and 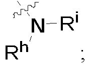
R^{h}, Rⁱ are each independently selected from the group consisting of H, and C1-C6 alkyl; preferably, R^{h}, Rⁱ are each independently selected from the group consisting of H, methyl, and isopropyl;
preferably, R¹³ is selected from C1-C6 haloalkyl (such as difluoromethyl); preferably, R¹³ is selected from the group consisting of 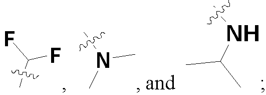 further preferably, R¹³ is selected from the group consisting of 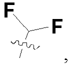 and 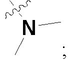
R¹⁴ is selected from the group consisting of C3-C6 cycloalkyl, piperidyl, piperazinyl, and morpholinyl, wherein, the C3-C6 cycloalkyl, piperidyl, piperazinyl, and morpholinyl are optionally substituted with 1-2 substituents selected from the group consisting of hydroxy C1-C6 alkyl (such as methylol), formyl, and C1-C6 alkyl (preferably methyl);
preferably, R¹⁴ is selected from the group consisting of 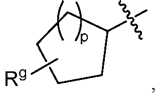 and piperidyl, wherein, the piperidyl is optionally substituted with 1-2 substituents selected from C1-C6 alkyl (preferably methyl); p is selected from the group consisting of 0, 1, and 2; preferably, p is 2; R^{g} is selected from the group consisting of H, hydroxy C1-C6 alkyl, and formyl; preferably, R^{g} is H;
further preferably, R¹⁴ is selected from the group consisting of 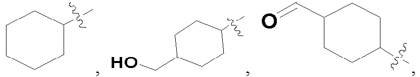 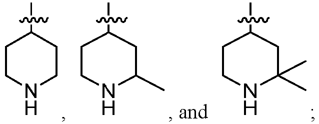
further preferably, R¹⁴ is selected from the group consisting of 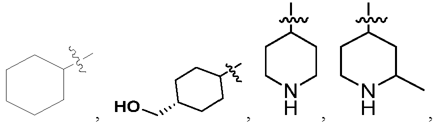 and 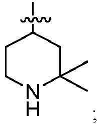
further preferably, 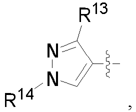 as a whole, is selected from the group consisting of 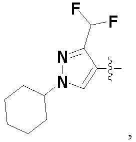 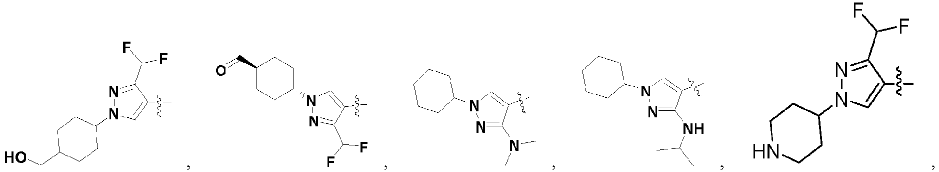 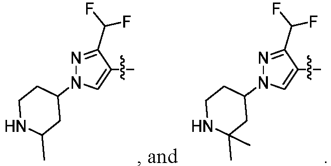
further preferably, 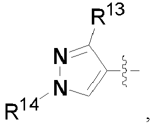 as a whole, is selected from the group consisting of

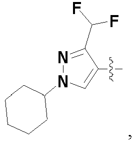

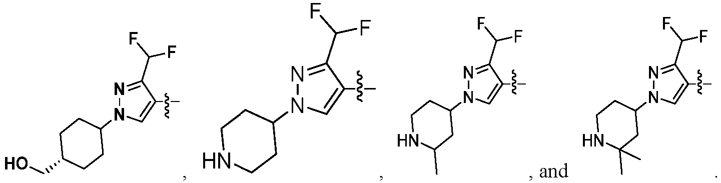

In the twelfth aspect, the present invention provides a compound represented by formula V, or a stereoisomer, a tautomer, a prodrug, a crystal form, a hydrate, an isotope-labeled compound (a deuteride), a metabolite, an ester, a pharmaceutically acceptable salt, or a pharmaceutically acceptable solvate thereof, wherein, the compound is represented by formula V, 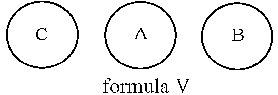 wherein:
A is selected from the group consisting of 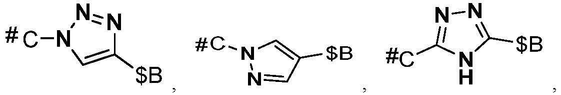 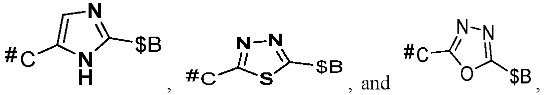 wherein #C represents the attachment point to C, $B represents the attachment point to B;
further preferably, A is selected from the group consisting of 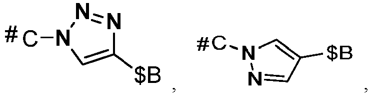 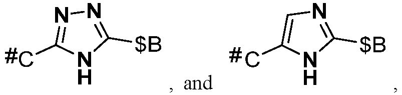 wherein #C represents the attachment point to C, $B represents the attachment point to B;
further preferably, A is 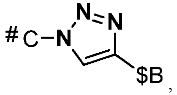 attachment point to B; wherein #C represents the attachment point to C, $B represents the
B is 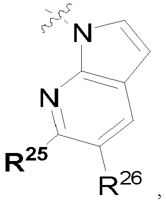 wherein: R²⁵ is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²⁵ is selected from the group consisting of H, and 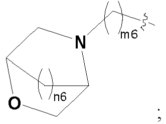
m6 is selected from the group consisting of 0, 1, 2, and 3; preferably, m6 is 0; n6 is selected from the group consisting of 0, 1, 2, and 3; preferably, n6 is 0, or 1;
further preferably, R²⁵ is selected from the group consisting of H, and 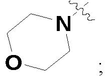
R²⁶ is selected from the group consisting of H, C1-C6 alkyl, cyano, carboxyl, and 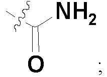 preferably, R²⁶ is selected from the group consisting of H, cyano, carboxyl, and 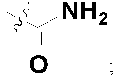
further preferably, 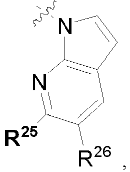 as a whole, is selected from the group consisting of 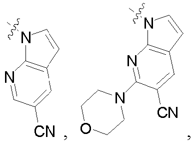 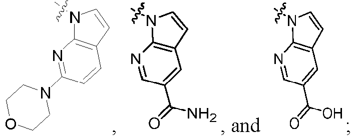
further preferably, 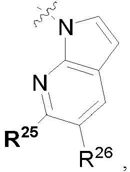 as a whole, is selected from the group consisting of 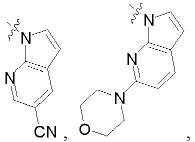 and 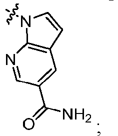
C is 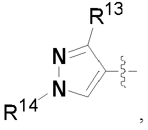 wherein: R¹³ is selected from the group consisting of C1-C6 haloalkyl (such as difluoromethyl or trifluoromethyl), and 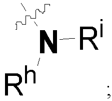 R^{h}, Rⁱ are each independently selected from the group consisting of H, and C1-C6 alkyl, preferably, R^{h}, Rⁱ are each independently selected from the group consisting of H, methyl, and isopropyl;
preferably, R¹³ is selected from C1-C6 haloalkyl (such as difluoromethyl); preferably, R¹³ is selected from the group consisting of 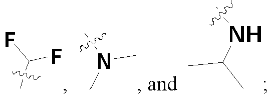
further preferably, R¹³ is selected from the group consisting of 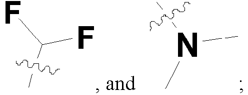
R¹⁴ is selected from the group consisting of C3-C6 cycloalkyl, piperidyl, piperazinyl, and morpholinyl, wherein, the C3-C6 cycloalkyl, piperidyl, piperazinyl, and morpholinyl are optionally substituted with 1-2 substituents selected from the group consisting of hydroxy C1-C6 alkyl (such as methylol), formyl, and C1-C6 alkyl (preferably methyl);
preferably, R¹⁴ is selected from the group consisting of 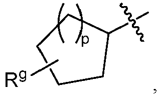 and piperidyl, wherein, the piperidyl is optionally substituted with 1-2 substituents selected from C1-C6 alkyl (preferably methyl);
p is selected from the group consisting of 0, 1, and 2; preferably, p is 2;
R^{g} is selected from the group consisting of H, hydroxy C1-C6 alkyl, and formyl; preferably, R^{g} is H;
further preferably, R¹⁴ is selected from the group consisting of 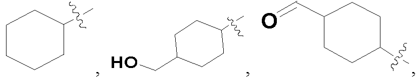 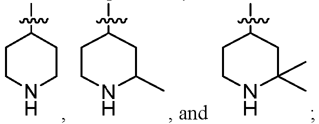
further preferably, R¹⁴ is selected from the group consisting of 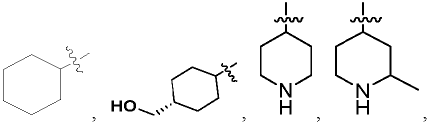 and 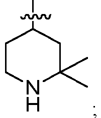
further preferably, 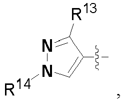 as a whole, is selected from the group consisting of 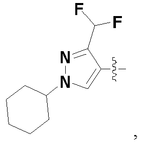 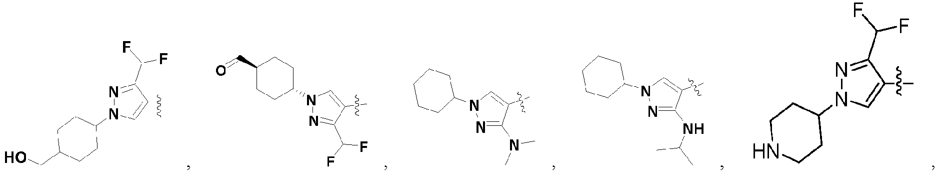 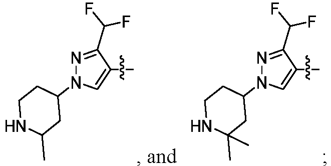
further preferably, 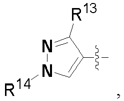 as a whole, is selected from the group consisting of

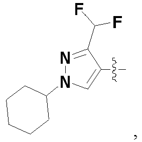

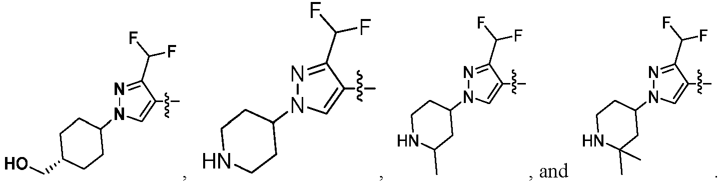

In the thirteenth aspect, the present invention provides a compound represented by formula VI-1, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VI-1, 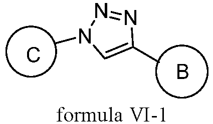 wherein B and C are as defined in anyone of the first to twelfth aspects of the present invention.

In the fourteenth aspect, the present invention provides a compound represented by formula VI-2 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VI-2, 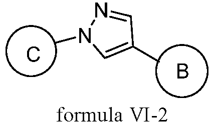 wherein B and C are as defined in anyone of the first to twelfth aspects of the present invention.

In the fifteenth aspect, the present invention provides a compound represented by formula VII-1 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VII-1, 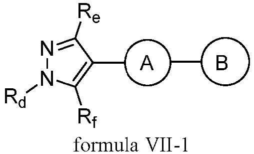 wherein A, B, R_{d}, Rₑ, R_{f} are as defined in anyone of the first to twelfth aspects of the present invention.

In the sixteenth aspect, the present invention provides a compound represented by formula VII-1-1 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VII-1-1, 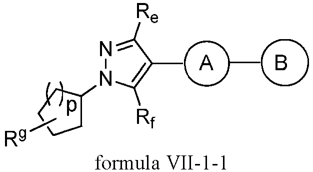 wherein A, B, Rₑ, R_{f}, R^{g}, p are as defined in the fifteenth aspect of the present invention.

In the seventeenth aspect, the present invention provides a compound represented by formula VII-1-2 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VII-1-2, 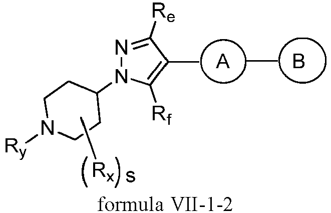 wherein each Rₓ is independently selected from the group consisting of halogen, C1-C6 alkoxy, hydroxy, amino, C1-C6 alkyl, and cyano, s is selected from the group consisting of 0, 1, 2, and 3, or two Rₓ together with the C atom to which they are attached may form C1-C6 cycloalkyl, R_{y} is selected from the group consisting of H, and C1-C6 alkyl, A, B, Rₑ, R_{f} are as defined in the fifteenth aspect of the present invention.

In the eighteenth aspect, the present invention provides a compound represented by formula VII-2-1 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VII-2-1, 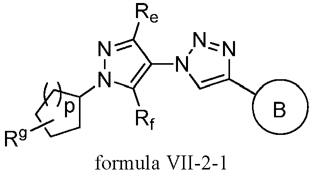 wherein B, Rₑ, R_{f}, R^{g}, p are as defined in the sixteenth aspect of the present invention.

In the nineteenth aspect, the present invention provides a compound represented by formula VII-2-2 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VII-2-2, 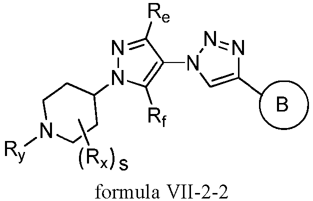 wherein B, Rₑ, R_{f}, Rₓ, R_{y}, s are as defined in the seventeenth aspect of the present invention.

In the twentieth aspect, the present invention provides a compound represented by formula VII-3-1 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VII-3-1, 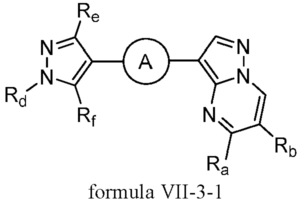 wherein A, R_{d}, Rₑ, R_{f}, Rₐ, R_{b} are as defined in the fifteenth aspect of the present invention.

In the twenty-first aspect, the present invention provides a compound represented by formula VII-3-2 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VII-3-2, 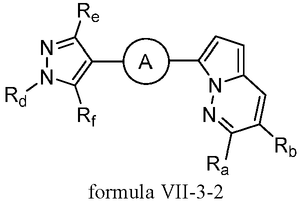 wherein A, R_{d}, Rₑ, R_{f}, Rₐ, R_{b} are as defined in the fifteenth aspect of the present invention.

In the twenty-second aspect, the present invention provides a compound represented by formula VII-3-3 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VII-3-3, 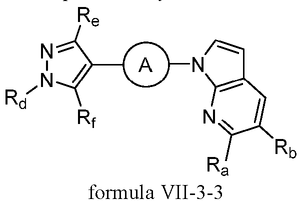 whereinA, R_{d}, Rₑ, R_{f}, Rₐ, R_{b} are as defined in the fifteenth aspect of the present invention.

In the twenty-third aspect, the present invention provides a compound represented by formula VIII-1 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VIII-1, 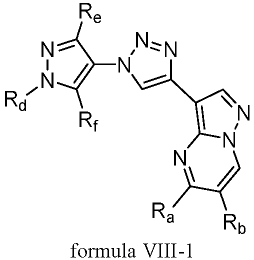 wherein R_{d}, Rₑ, R_{f}, Rₐ, R_{b} are as defined in anyone of the first to twelfth aspects of the present invention.

In the twenty-fourth aspect, the present invention provides a compound represented by formula VIII-2 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VIII-2, 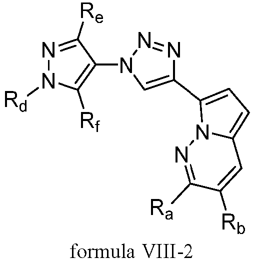 wherein R_{d}, Rₑ, R_{f}, Rₐ, R_{b} are as defined in anyone of the first to twelfth aspects of the present invention.

In the twenty-fifth aspect, the present invention provides a compound represented by formula VIII-3 or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, wherein, the compound is represented by formula VIII-3, 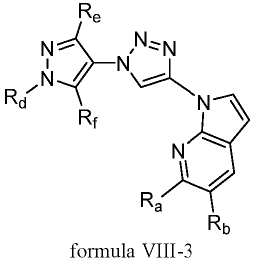 wherein R_{d}, Rₑ, R_{f}, Rₐ, R_{b} are as defined in anyone of the first to twelfth aspects of the present invention.

In certain embodiments, the compound is selected from the group consisting of:

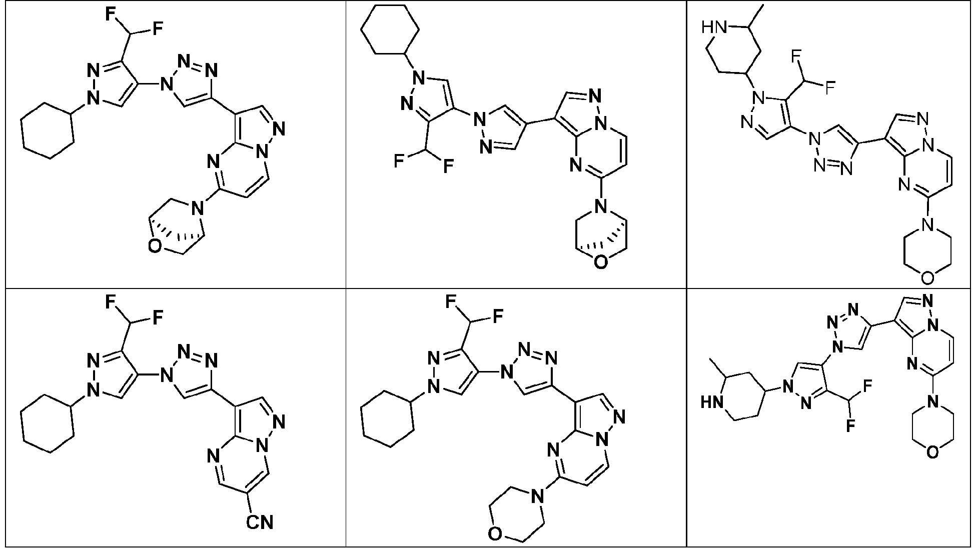

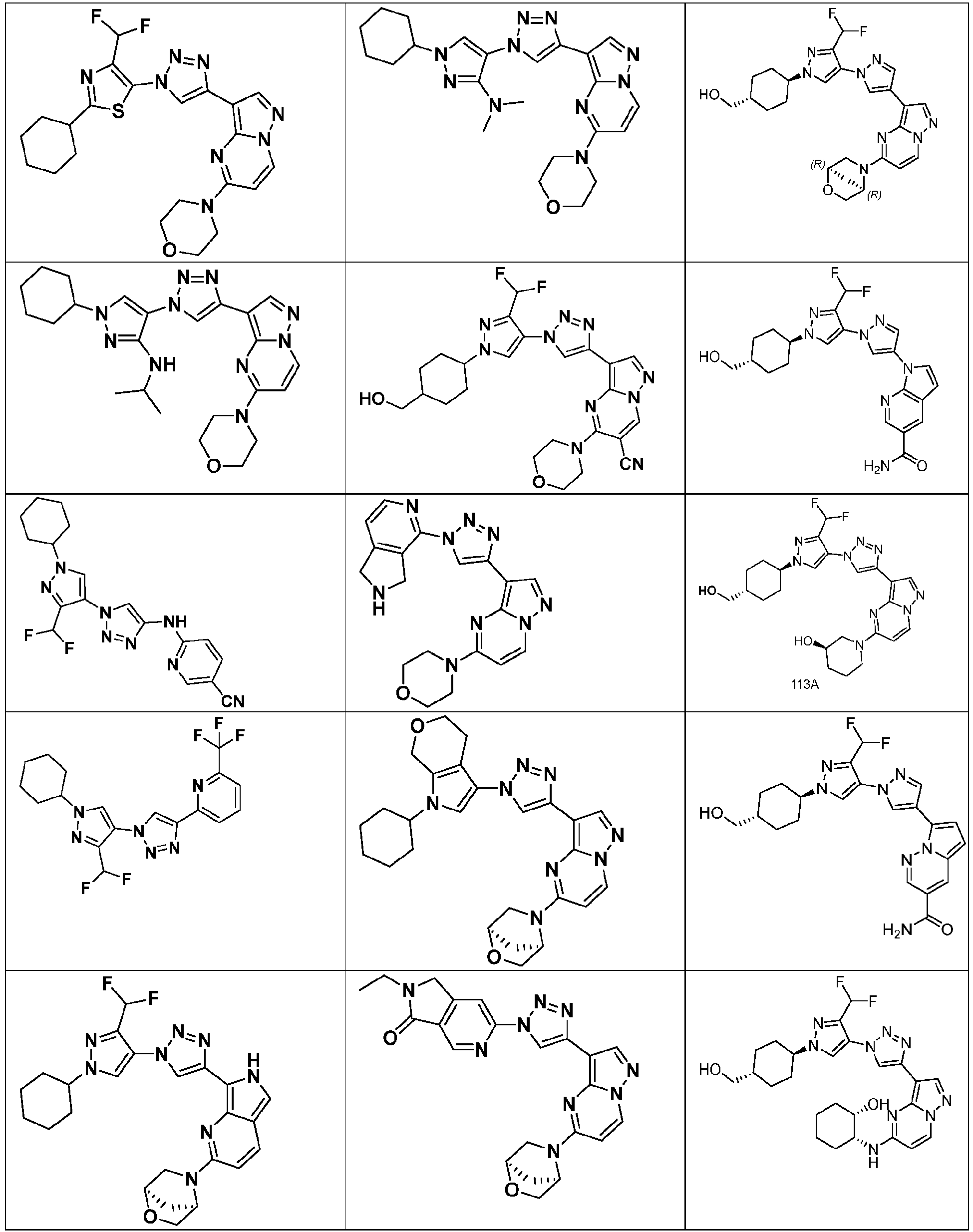

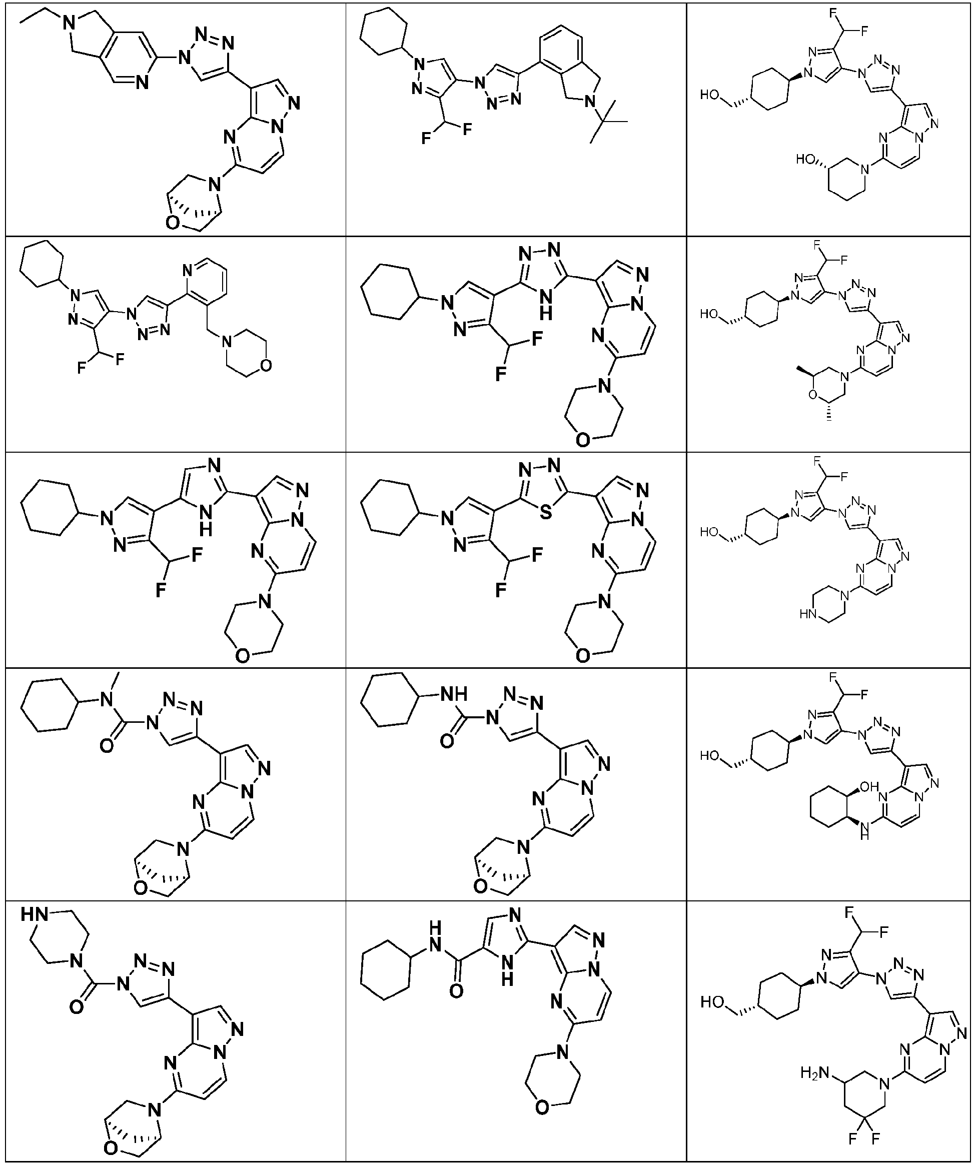

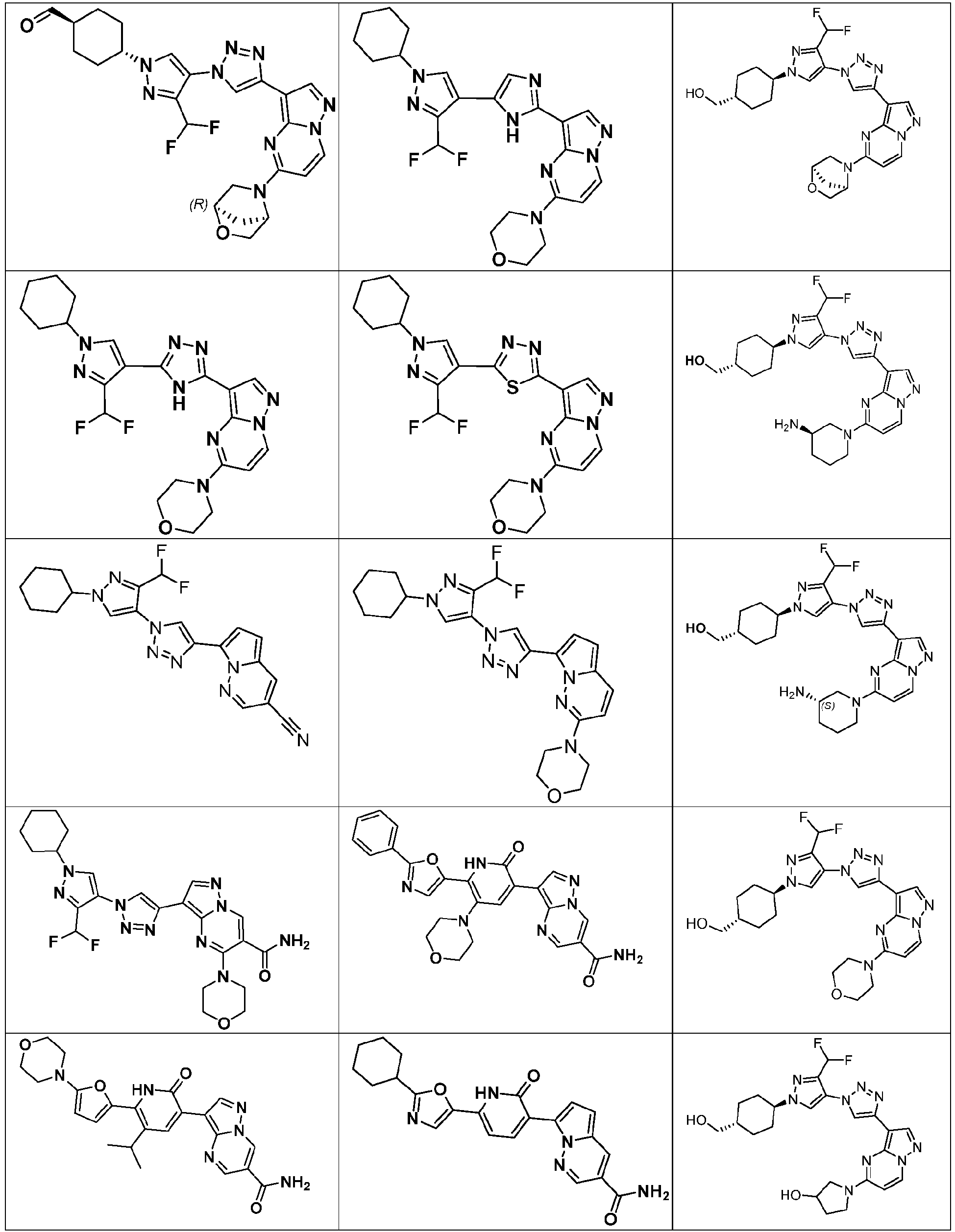

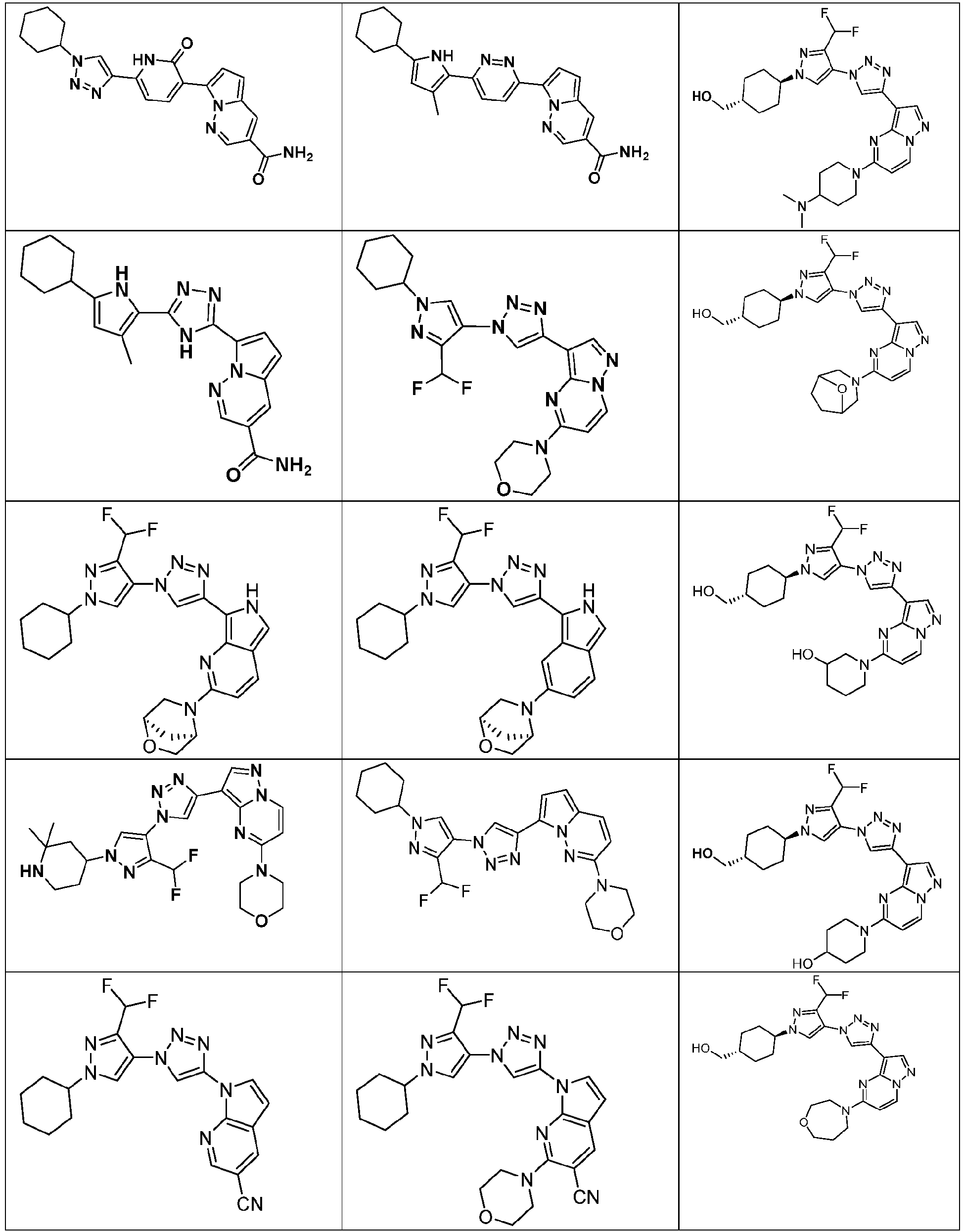

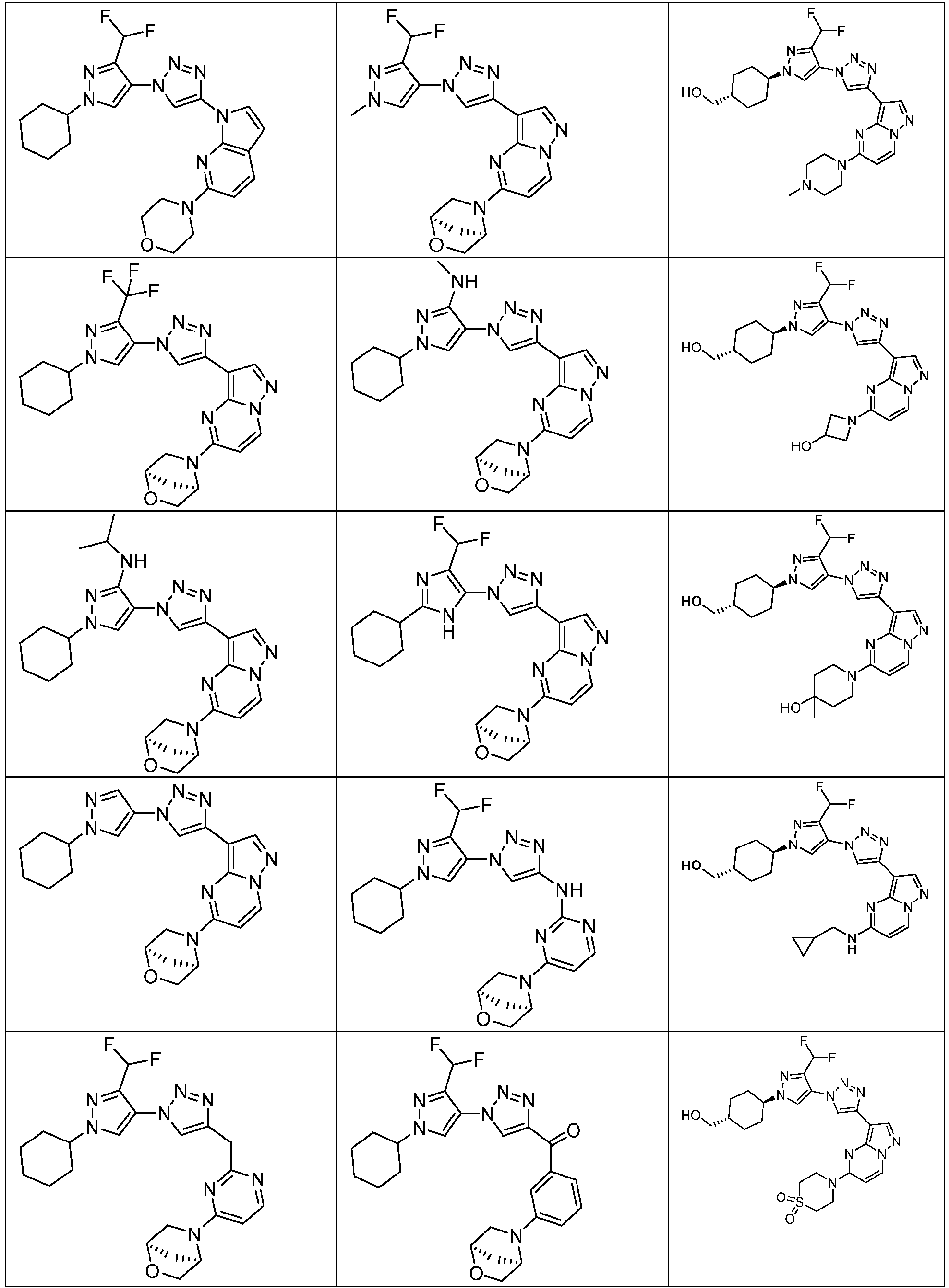

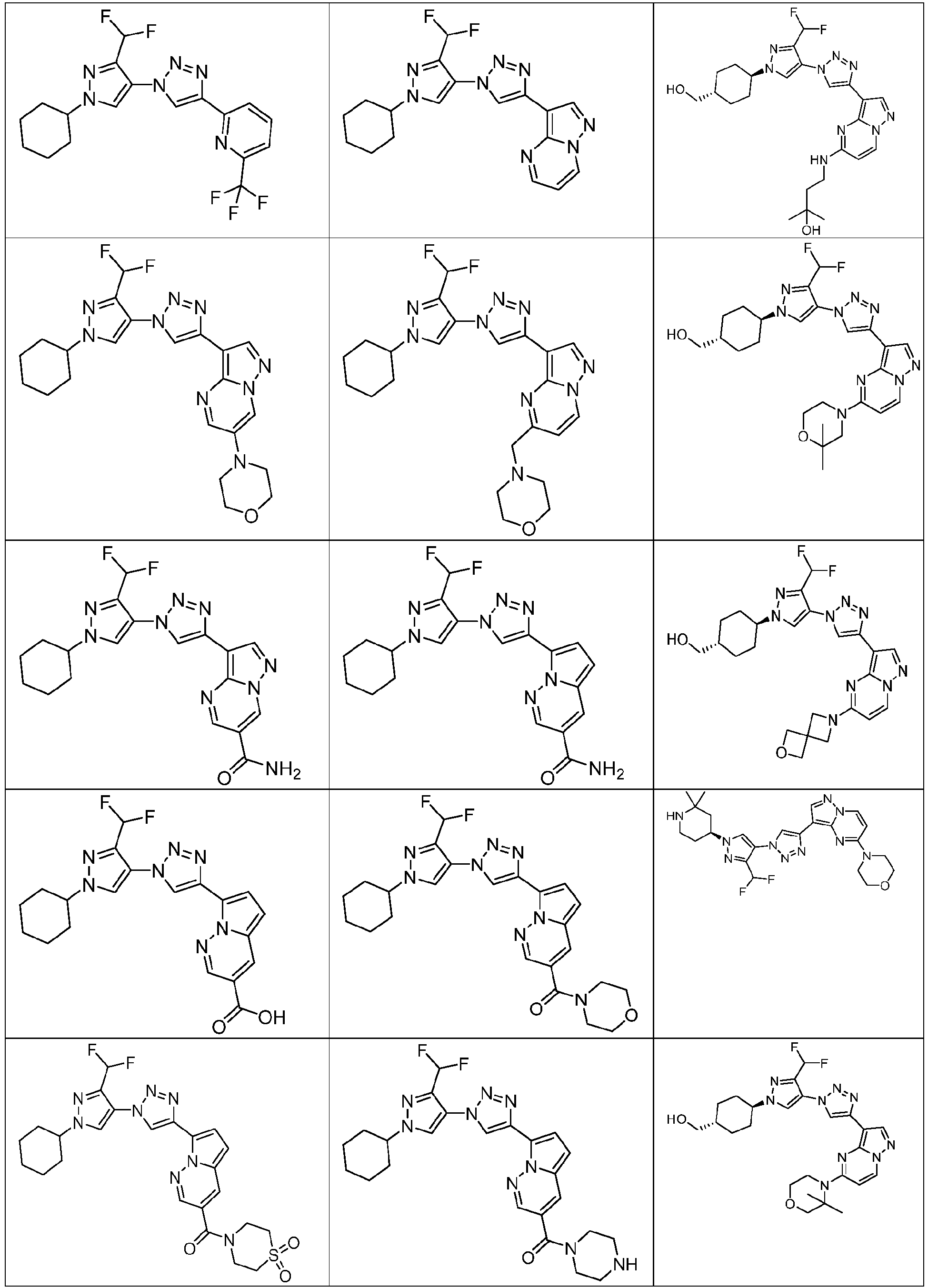

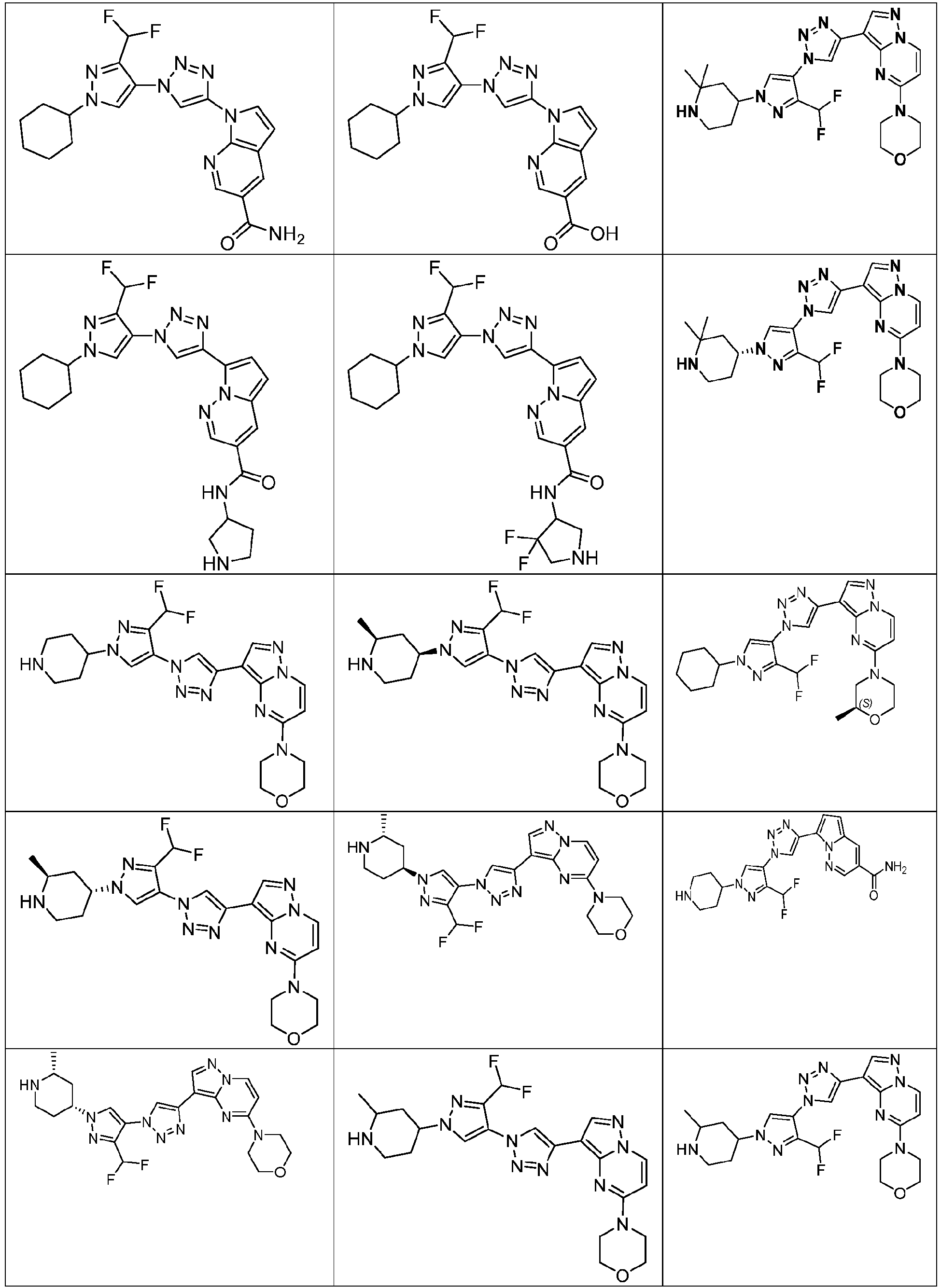

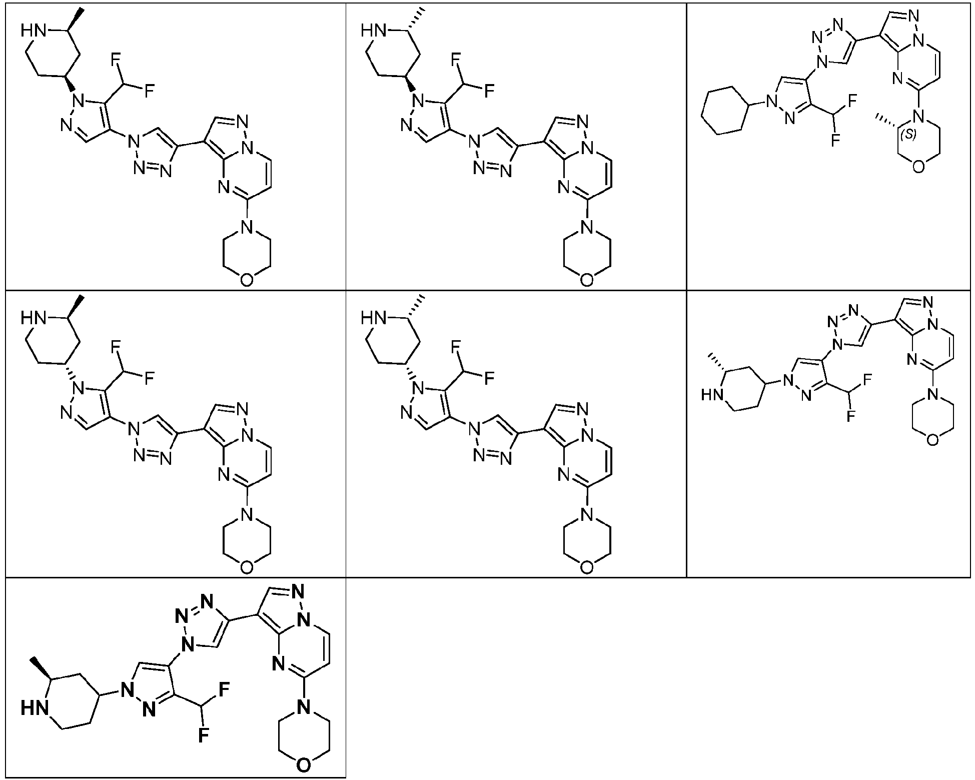

In certain embodiments, the compound is selected from the group consisting of:

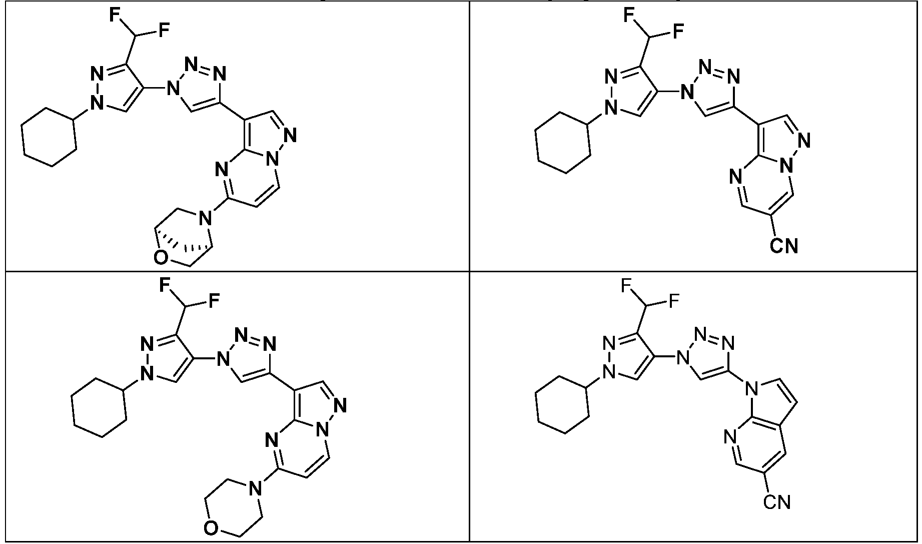

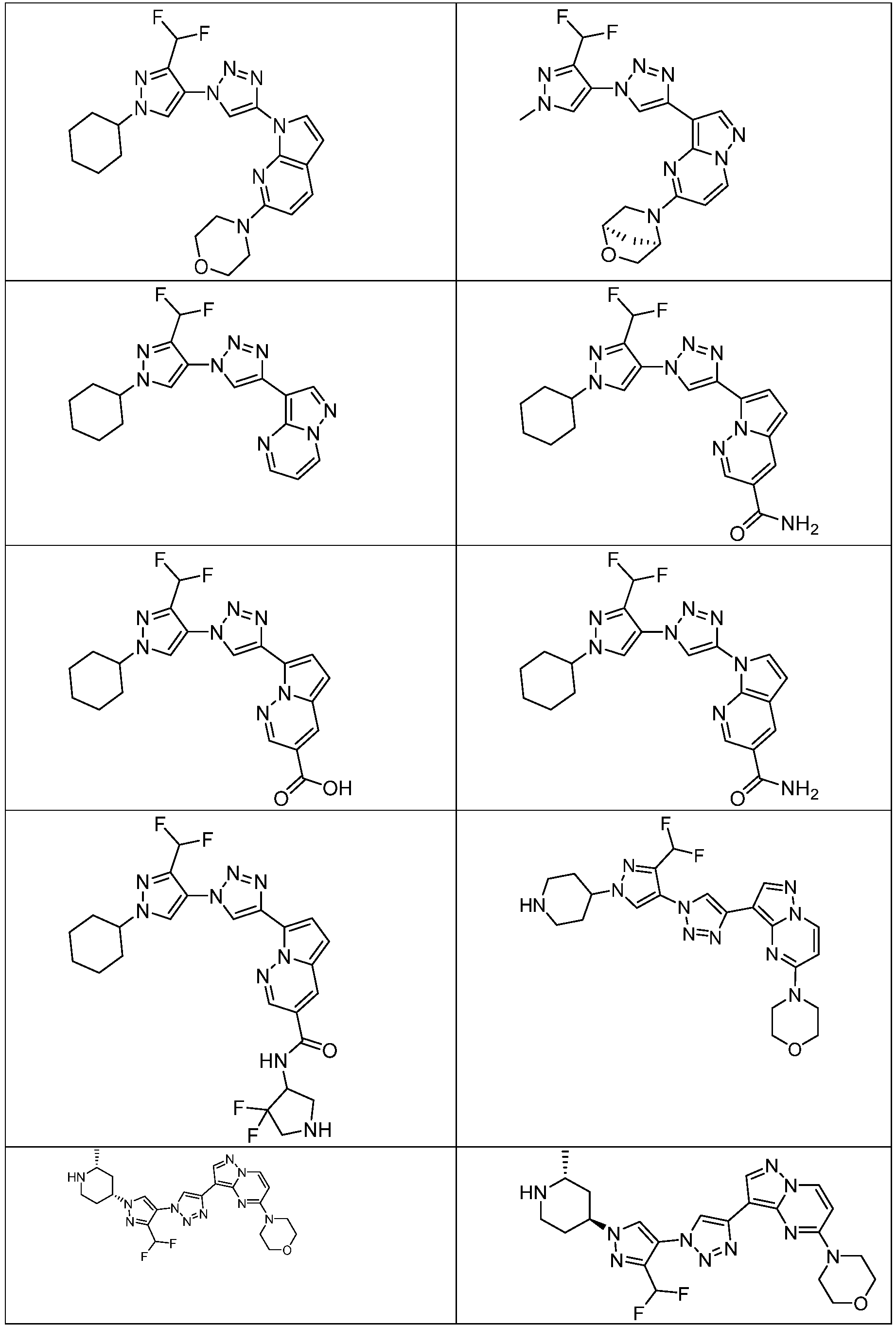

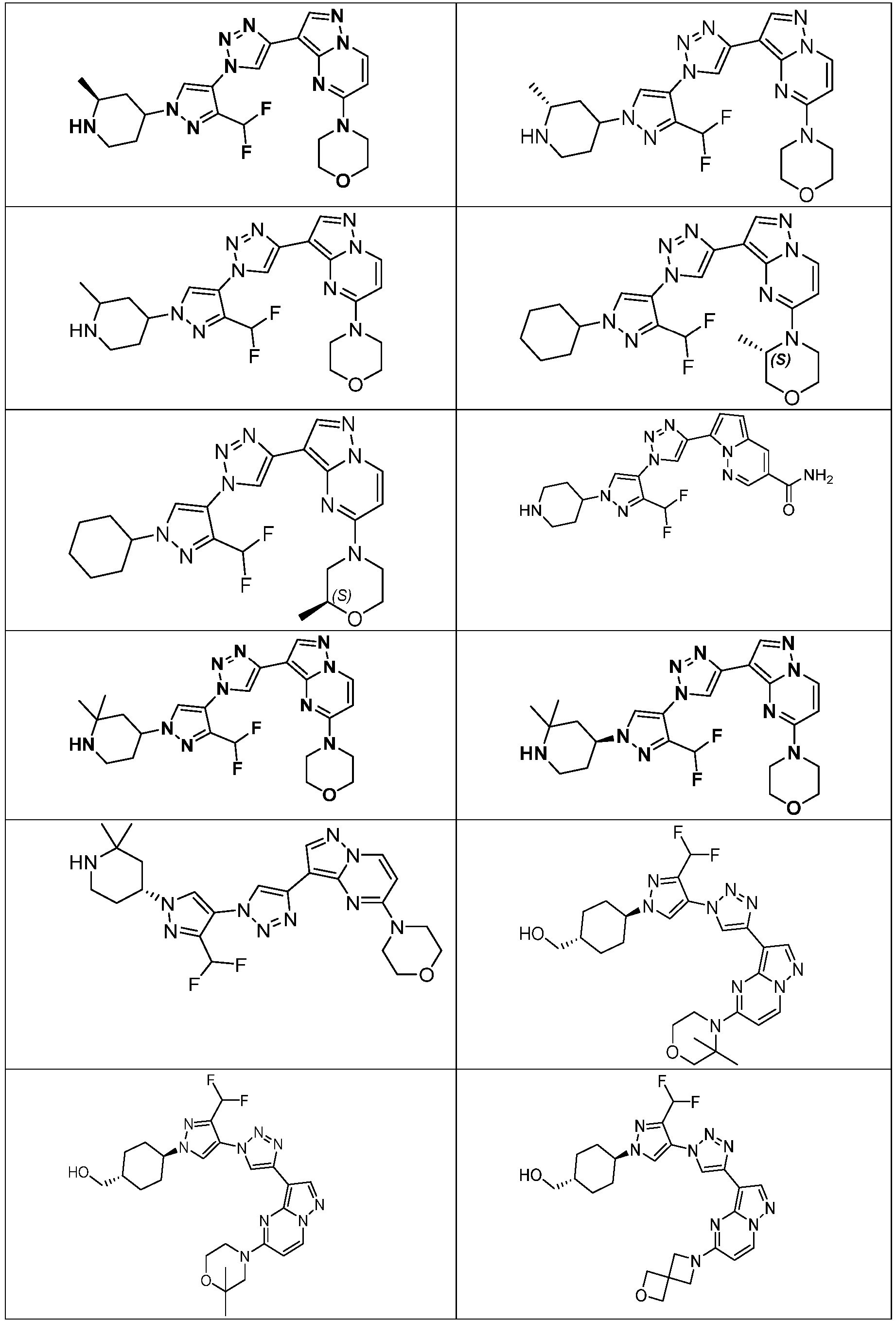

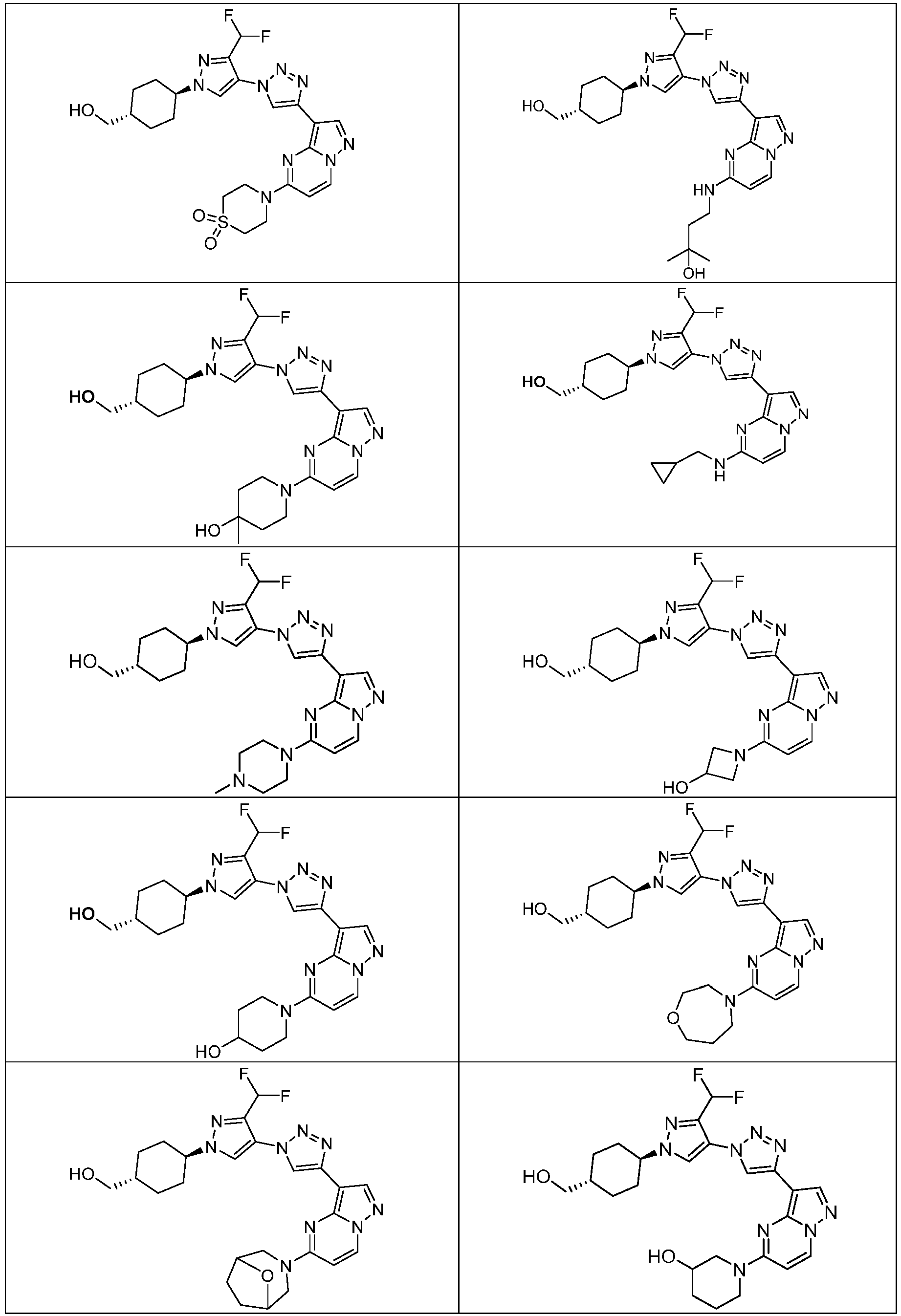

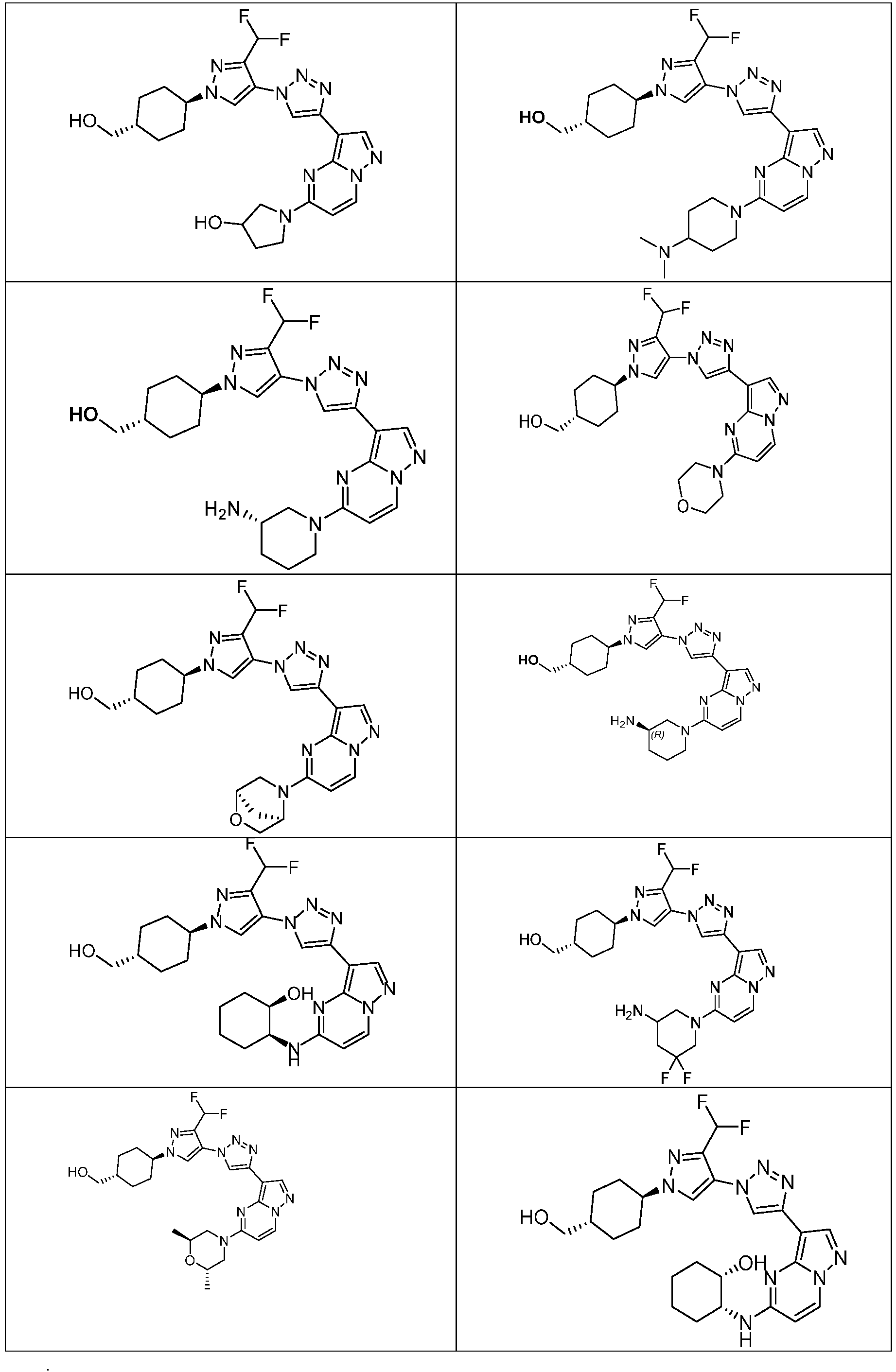

In the twenty-sixth aspect, the present invention provides a method for preparing a compound of formula A as described in anyone of the above aspects of the present invention, wherein, in formula A, A is 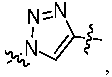 the method comprises: 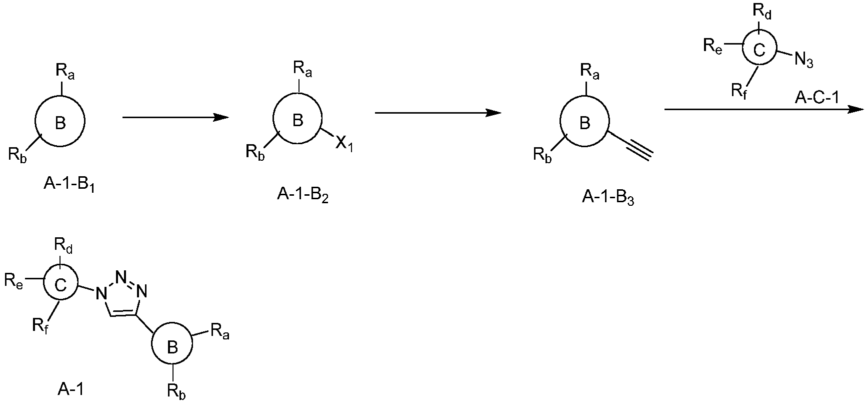
allowing compound A-1-B₁ to undergo a halogenation reaction to obtain compound A-1-B₂, allowing the compound A-1-B₂ to undergo a substitution reaction to obtain compound A-1-B₃, and allowing the compound A-1-B₃ to undergo a cyclization reaction with compound A-C-1 to obtain compound A-1;
wherein X₁ is halogen (such as Cl, Br, I), Rₐ, R_{b}, R_{d}, Rₑ, R_{f}, B, and C are as defined in anyone of the first to twenty-fifth aspects of the present invention.

In the twenty-seventh aspect, the present invention provides a method for preparing compound A-C-1, comprising: 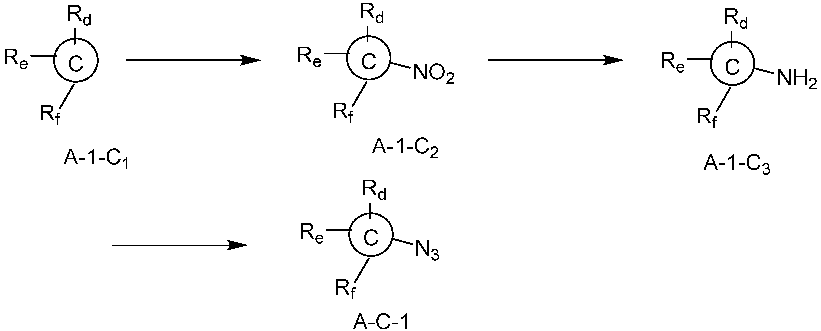 allowing compound A-1-C₁ to undergo a nitration reaction to obtain compound A-1-C₂, allowing the compound A-1-C₂ to undergo a reduction reaction to obtain compound A-1-C₃, and allowing the compound A-1-C₃ to undergo an azidation reaction to obtain the compound A-C-1, wherein R_{d}, Rₑ, R_{f} and C are as defined in anyone of the first to twenty-fifth aspects of the present invention.

In the twenty-eighth aspect, the present invention provides a method for preparing a compound of formula A as described in anyone of the above aspects of the present invention, wherein, A is 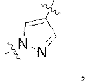 the method comprises: 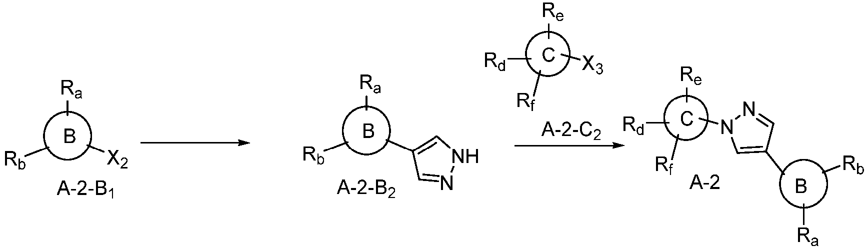 allowing compound A-2-B₁ to undergo a coupling reaction to obtain compound A-2-B₂, and allowing the compound A-2-B₂ and compound A-2-C₂ to undergo a coupling reaction to obtain compound A-2, wherein X₂ and X₃ are each independently halogen (such as Cl, Br, I), Rₐ, R_{b}, R_{d}, Rₑ, R_{f}, B, and C are as defined in anyone of the first to twenty-fifth aspects of the present invention.

In the twenty-ninth aspect, the present invention provides a method for preparing compound A-2-C₂, comprising: 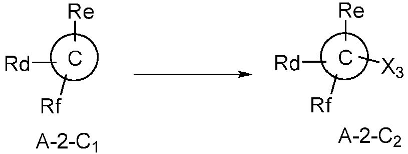 allowing the compound A-2-C₁ to undergo a halogenation reaction to obtain the compound A-2-C₂, wherein X₃ is halogen (such as Cl, Br, I), R_{d}, Rₑ, R_{f}, and C are as defined in anyone of the first to twenty-fifth aspects of the present invention.

In the thirtieth aspect, the present invention provides a method for preparing a compound of formula A as described in anyone of the first to twenty-fifth aspects of the present invention, characterized in that: in formula A, A is 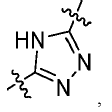 the method comprises: 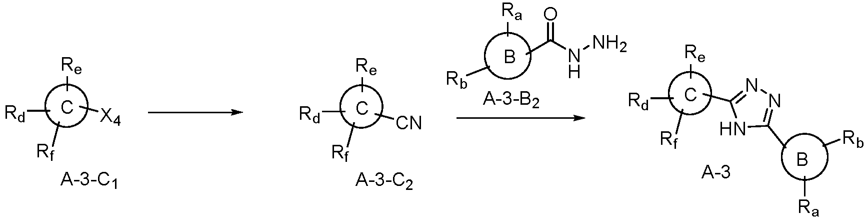
allowing compound A-3-C₁ to undergo a cyanation reaction to obtain compound A-3-C₂, and allowing the compound A-3-C₂ to undergo a cyclization reaction with A-3-B₂ to obtain A-3,
wherein X₄ is halogen (such as Cl, Br, I), Rₐ, R_{b}, R_{d}, Rₑ, R_{f}, B, and C are as defined in anyone of the first to twenty-fifth aspects of the present invention.

In the thirty-first aspect, the present invention provides a method for preparing compound A-3-B₂, comprising: 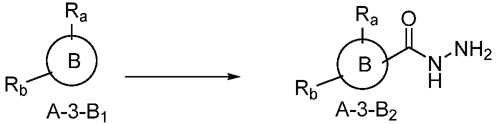 allowing compound A-3-B₁ to undergo an acylation reaction to obtain the compound A-3-B₂, wherein Rₐ, R_{b}, and B are as defined in claims 1-27.

In the thirty-second aspect, the present invention provides a pharmaceutical composition, which comprises a compound, or a stereoisomer, a tautomer, a prodrug, a crystal form, a hydrate, an isotope-labeled compound, a metabolite, an ester, a pharmaceutically acceptable salt, or a pharmaceutically acceptable solvate thereof described above, and optionally a pharmaceutically acceptable excipient.

In the thirty-third aspect, the present invention provides a PROTAC molecule, comprising: a warhead moiety, a ligase binding moiety and a connecting chain, wherein, the warhead moiety is a compound, or a stereoisomer, a tautomer, a prodrug, a crystal form, a hydrate, an isotope-labeled compound, a metabolite, an ester, a pharmaceutically acceptable salt, or a pharmaceutically acceptable solvate thereof described above, the connecting chain is covalently linked to the warhead moiety and the ligase binding moiety respectively.

In certain embodiments, the ligase binding moiety is an E3 ubiquitin ligase binding moiety.

In the thirty-fourth aspect, the present invention provides use of a compound, or a stereoisomer, a tautomer, a prodrug, a crystal form, a hydrate, an isotope-labeled compound, a metabolite, an ester, a pharmaceutically acceptable salt, or a pharmaceutically acceptable solvate thereof described above or a pharmaceutical composition described above, or a PROTAC molecule described above in the preparation of a medicament for the treatment and/or prevention of IRAK4 kinase-related diseases.

In certain embodiments, the IRAK4 kinase-related disease is cancer or autoimmune disease.

In certain embodiments, the autoimmune disease is selected from rheumatoid arthritis, osteoarthritis, chronic obstructive pulmonary disease, systemic lupus erythematosus, psoriasis, ulcerative colitis, irritable bowel syndrome, or any combination thereof.

In certain embodiments, the cancer is selected from B-cell chronic lymphocytic leukemia, acute lymphocytic leukemia, non Hodgkin lymphoma, Hodgkin lymphoma, acute myeloid leukemia, diffuse large B-cell lymphoma, multiple myeloma, Fahrenheit macroglobulinemia, or any combination thereof.

In the thirty-fifth aspect, the present invention provides a compound, or a stereoisomer, a tautomer, a prodrug, a crystal form, a hydrate, an isotope-labeled compound, a metabolite, an ester, a pharmaceutically acceptable salt, or a pharmaceutically acceptable solvate thereof described above, or a pharmaceutical composition described above, or a PROTAC molecule described above, for use in the treatment and/or prevention of IRAK4 kinase-related diseases.

In certain embodiments, the IRAK4 kinase-related disease is cancer or autoimmune disease.

In certain embodiments, the autoimmune disease is selected from rheumatoid arthritis, osteoarthritis, chronic obstructive pulmonary disease, systemic lupus erythematosus, psoriasis, ulcerative colitis, irritable bowel syndrome, or any combination thereof.

In certain embodiments, the cancer is selected from B-cell chronic lymphocytic leukemia, acute lymphocytic leukemia, non Hodgkin lymphoma, Hodgkin lymphoma, acute myeloid leukemia, diffuse large B-cell lymphoma, multiple myeloma, Fahrenheit macroglobulinemia, or any combination thereof.

In the thirty-sixth aspect, the present invention provides a method for the treatment and/or prevention of IRAK4 kinase-related diseases, comprising administering to a subject an effective amount of a compound, or a stereoisomer, a tautomer, a prodrug, a crystal form, a hydrate, an isotope-labeled compound, a metabolite, an ester, a pharmaceutically acceptable salt, or a pharmaceutically acceptable solvate thereof described above or a pharmaceutical composition described above, or a PROTAC molecule described above.

In certain embodiments, the IRAK4 kinase-related disease is cancer or autoimmune disease.

In certain embodiments, the autoimmune disease is selected from rheumatoid arthritis, osteoarthritis, chronic obstructive pulmonary disease, systemic lupus erythematosus, psoriasis, ulcerative colitis, irritable bowel syndrome, or any combination thereof.

In certain embodiments, the cancer is selected from B-cell chronic lymphocytic leukemia, acute lymphocytic leukemia, non Hodgkin lymphoma, Hodgkin lymphoma, acute myeloid leukemia, diffuse large B-cell lymphoma, multiple myeloma, Fahrenheit macroglobulinemia, or any combination thereof.

### Mode of carrying out the invention

It is to be understood that the terms used herein is for the purpose of describing particular embodiments, and is not intended to be limiting. In addition, although any methods, devices and materials similar or equivalent to those described herein can be used in the practice or testing of the present invention, the preferred methods, devices and materials are now described.

In the present invention, unless otherwise explicitly indicated, the expression "... are each independently selected from" used throughout this text means not only that specific options expressed by the same or different symbols for different groups do not affect each other, but also that specific options expressed by the same or different symbols for the same group do not affect each other.

The substituents of the compounds of the present invention are disclosed according to the type or range of groups. Specifically, the present invention includes each independent secondary combination of the various members of these group types and ranges. For example, the term "C1-C6 alkyl" specifically refers to independently disclosed methyl, ethyl, C3 alkyl, C4 alkyl, C5 alkyl, and C6 alkyl.

The term "alkyl" is meant to include both branched and straight chain saturated aliphatic hydrocarbon groups having the indicated number of carbon atoms. For example, "C1-C6 alkyl" refers to alkyl groups having 1 to 6 carbon atoms, preferably "C1-C4 alkyl", further preferably "C1-C3 alkyl". Examples of "C1-C6 alkyl" include, but are not limited to, methyl, ethyl, propyl (e.g., n-propyl, isopropyl), butyl (e.g., n-butyl, isobutyl, tert-butyl), pentyl (e.g., n-pentyl, isopentyl, neopentyl) and the like.

The term "cycloalkyl" refers to a saturated hydrocarbon monovalent ring containing from 3 to 15 ring carbon atoms or from 3 to 10 ring carbon atoms and may be, for example, "C3-C7 cycloalkyl", "C4-C9 cycloalkyl", "C3-C6 cycloalkyl". Cycloalkyl may be in the form of a single ring, fused ring, bridged ring, spiro ring, or the like. Exemplary cycloalkyl groups include, but are not limited to, the following examples: 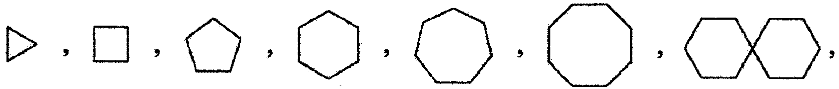 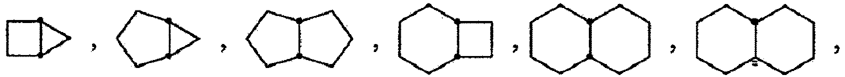 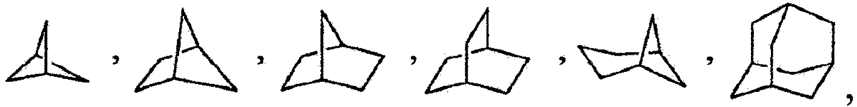 and the like. Examples of "C3-C6 cycloalkyl" include

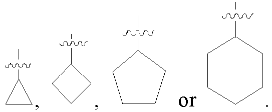

Halogen means fluorine, chlorine, bromine or iodine.

The term "heteroalkyl" refers to a group formed by replacing one or more backbone carbon atoms in any alkyl (such as C1-C6 alkyl, C1-C4 alkyl, C1-C3 alkyl and the like) described above with heteroatom(s) (N, O or S).

The term "alkoxy" refers to a moiety that any alkyl (such as C1-C6 alkyl, C1-C4 alkyl, C1-C3 alkyl and the like) described above is attached to the rest of the molecule through an O atom, examples thereof including C1-C6 alkoxy, C1-C4 alkoxy, C1-C3 alkoxy, specifically such as methoxy, ethoxy and the like.

The term "C1-C6 haloalkyl" refers to a group formed by replacing one or more hydrogen atoms in any alkyl (such as C1-C6 alkyl, C1-C4 alkyl, C1-C3 alkyl and the like) described above with halogen (preferably fluorine, chlorine), for example, monofluoromethyl, difluoromethyl, difluoroethyl, trifluoromethyl and the like.

The terms "6-10 membered aryl" and "6-10 membered aromatic ring" may be used interchangeably and refer to an aromatic 6-10 membered monocyclic or bicyclic group. Specific examples are phenyl, naphthyl, preferably phenyl.

Heteroatom refers to N, O or S.

The terms "heteroaryl" and "heteroaromatic ring" may be used interchangeably and refer to substituted and unsubstituted aromatic 5-or 6-membered monocyclic groups, 8-, 9-or 10-membered bicyclic groups and 11-to 14-membered tricyclic groups having at least one heteroatom (N, O or S) in at least one ring, the heteroatom-containing ring optionally further having 1, 2 or 3 heteroatoms selected from N, O or S. Wherein the substituted and unsubstituted aromatic 8-, 9-, or 10-membered bicyclic groups and 11-to 14-membered tricyclic groups having at least one heteroatom (N, O or S) in at least one ring are "fused heteroaryl" or "fused heteroaromatic ring". As regards a heteroaryl or heteroaromatic ring that is bicyclic or tricyclic, it requires a bicyclic or tricyclic overall structure to form an aromatic system. A heteroaryl or heteroaromatic ring may be attached at any available nitrogen or carbon atom of any ring. And those skilled in the art will understand that two adjacent atoms (preferably carbon atoms) are shared between each two rings in the fused ring.

Examples of monocyclic heteroaryl or monocyclic heteroaromatic rings include, but are not limited to: pyrrolyl/pyrrole ring, pyrazolyl/pyrazole ring, imidazolyl/imidazole ring, oxazolyl/oxazole ring, isoxazolyl/isoxazole ring, thiazolyl/thiazole ring, thiadiazolyl/thiadiazole ring, isothiazolyl/isothiazole ring, furanyl/furane ring, thienyl/thiophene ring, oxadiazolyl/oxadiazole ring, pyridinyl/pyridine ring, pyrazinyl/pyrazine ring, pyrimidinyl/pyrimidine ring, pyridazinyl/pyridazine ring, triazinyl/triazine ring, triazolyl/triazole ring, pyridazinyl/pyridazine ring, 2-pyridone and the like.

Examples of bicyclic heteroaryl include, but are not limited to: indolyl, 5-azaindolyl, pyrrolo [2,3-d] pyrimidinyl, 5, 6-diazaindolyl, 6-azaindolyl, 7-azaindolyl, pyrazolo [3,4-b] pyridinyl, pyrrolo [2, 3-c] pyridazinyl, thieno [2,3-d] imidazolyl, pyrazolo [3,4-c] pyridinyl, benzothiazolyl, benzimidazolyl, benzoxazolyl, benzothienyl, quinolinyl, isoquinolinyl, benzofuranyl, indolizinyl, quinoxalinyl, indazolyl, pyrrolopyrimidinyl, furopyridinyl, isoindolyl and the like, preferably such as

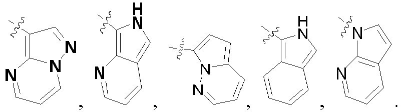

Examples of "9-membered heteroaryl" include, but are not limited to, 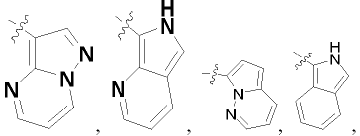 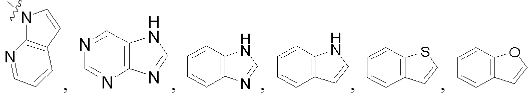 and the like.

The term "heterocycle", "heterocyclic" or "heterocyclyl" may be used interchangeably and refers to substituted and unsubstituted 3-to 7-membered (preferably 4-7 membered, further preferably 5-6 membered) monocyclic groups, 7-to 11-membered bicyclic groups and 10-to 15-membered tricyclic groups, which may contain one or more double bonds but do not constitute aromatic rings; wherein at least one ring has at least one heteroatom (N, O or S). Fused, bridged or spiro rings completing bicyclic and tricyclic groups may contain only carbon atoms and may be saturated or partially saturated, but do not constitute aromatic rings. The heterocyclyl may be attached at any available nitrogen or carbon atom. Exemplary heterocycles include, but are not limited to, the following examples:

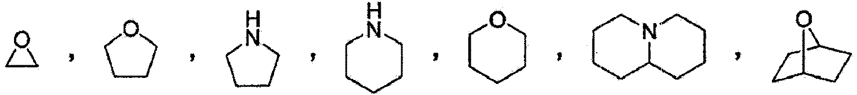

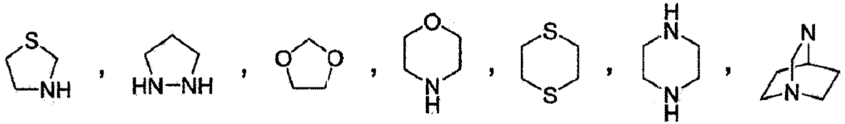

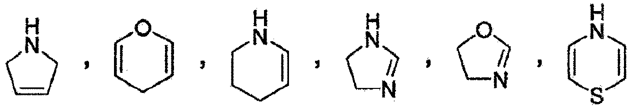

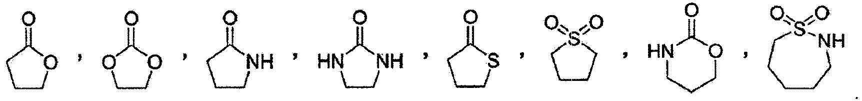

Exemplary monocyclic heterocyclyl includes azetidinyl, oxetanyl, pyrrolidinyl, imidazolinyl, oxazolidinyl, isoxazolinyl, thiazolidinyl, tetrahydrofuranyl, piperidinyl, piperazinyl, 2-oxopiperazinyl, 2-oxopiperidinyl, 2-oxopyrrolidinyl, 2-oxoazepinyl, 1-pyridonyl, 4-piperidinonyl, tetrahydropyranyl, morpholinyl, 1, 3-dioxolanyl and the like, preferably such as

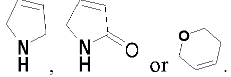

Among them, "saturated heterocyclyl" refers to the heterocycle defined above that does not contain an unsaturated bond, such as a double bond, for example, examples of "4-to 9-membered saturated heterocycle" include, but are not limited to, hexahydropyrimidine, hexahydropyrazine,

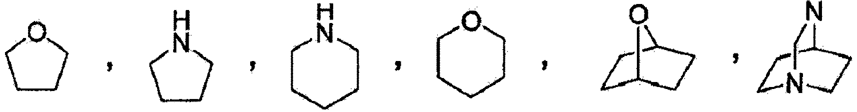

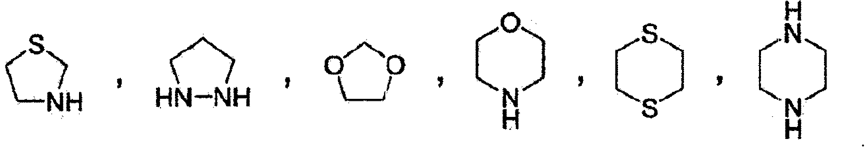

For example, examples of "6 membered saturated heterocyclyl" include, but are not limited to, morpholinyl, piperazinyl, piperidyl, tetrahydropyranyl, hexahydropyrimidinyl, hexahydropyrazinyl, specifically such as 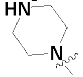 or

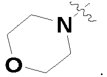

From all the above descriptions, it is obvious to those skilled in the art that any group whose name is a complex name, such as "hydroxy C1-C6 alkyl", shall mean a group that is constructed conventionally from the left to the right from a moiety where it is derived, for example, from "C1-C6 alkyl" substituted by hydroxy, wherein "C1-C6 alkyl" is as defined above. Specifically, "hydroxy C1-C6 alkyl" may be, for example, methylol. The remaining similar complex groups can be understood with reference to the foregoing.

The term "direct bond" refers to the direct connection of groups on both sides, for example, in the structural formula represented by formula A 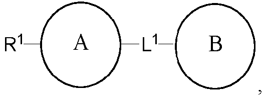 if L¹ is a direct bond, then the structural formula represented by formula A is

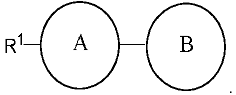

The term "substituted" or "substituted with substituent ... " refers to the selective replacement of any one or more hydrogens on a designated atom or group with designated group(s), provided that the designated atom's normal valence is not exceeded.

"B is selected from the group consisting of 6-10 membered heteroaryl, and 6-10 membered aryl, and B is substituted with substituents R^{a}, R^{b}" means that B may be 6-10 membered heteroaryl that is substituted with 2 substituents, i.e., substituents R^{a}, R^{b}, B may also be 6-10 membered aryl that is substituted with 2 substituents, i.e., substituents R^{a}, R^{b}, moreover, the substituents of the 6-10 membered heteroaryl and the 6-10 membered aryl are independent and not affected by each other, i.e., the substituents of the 6-10 membered heteroaryl and the 6-10 membered aryl may be the same or different. In addition, if both R^{a} and R^{b} are hydrogen, it will be understood by those skilled in the art that the 6-10 membered heteroaryl or the 6-10 membered aryl is not actually substituted. The remaining similar definitions may be understood with reference to the foregoing.

"R^{d} is selected from C3-C6 cycloalkyl optionally substituted by 1 substituent selected from the group consisting of hydroxy C1-C6 alkyl (such as methylol) and formyl", means that the C3-C6 cycloalkyl may be unsubstituted or substituted by 1 substituent selected from the group consisting of hydroxy C1-C6 alkyl (such as methylol) and formyl. The remaining similar definitions may be understood with reference to the foregoing.

When "B is phenyl, and B is substituted with substituents R^{a}, R^{b}", and "R^{a}, R^{b} and the carbon atoms to which they are attached, respectively, together form a substituted or unsubstituted 5-membered heterocycle, wherein, the substituent is selected from C1-C6 alkyl (such as tert-butyl)", it means that R^{a}, R^{b} and the adjacent two carbon atoms on the benzene ring to which R^{a}, R^{b} are attached, respectively, form 5-membered heterocycle, and the 5-membered heterocycle may be unsubstituted or substituted at any position with 1 substituent selected from C1-C6 alkyl (such as tert-butyl). For example, if B is phenyl, B is substituted with substituents R^{a}, R^{b}, and R^{a}, R^{b} and the carbon atoms to which they are attached, respectively, together form substituted or unsubstituted 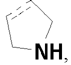 wherein, the substituent is tert-butyl, it means that B, as a whole, may be 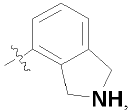 or may also be 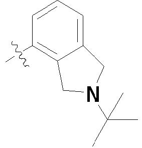 and the like, wherein, the dotted double bond in 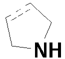 represents the site at which the 5-membered heterocycle is fused to the benzene ring. The remaining similar definitions may be understood with reference to the foregoing.

"C is selected from 5-6 membered heteroaryl, and C is optionally substituted with at least one of substituents R^{d}, R^{e}, R^{f}" refers to that C may be unsubstituted or substituted, and when it is substituted, the substituent may be only R^{d}, R^{e} or R^{f}, or may be a combination of any two of R^{d}, R^{e}, and R^{f}, or may be R^{d}, R^{e} and R^{f}. The remaining similar definitions may be understood with reference to the foregoing.

"R¹ is 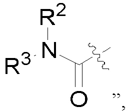 and, "R², R³ and the N atom to which they are both attached together form 6-membered saturated heterocyclyl" refer to that R², R³ and the N atom to which R², R³ are both attached together form 6-membered saturated heterocyclyl, for example, if the 6-membered saturated heterocyclyl is 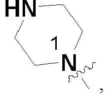 then the N atom No. 1 is just the N atom to which R², R³ are both attached.

In the present invention, "treatment" generally means obtaining a desired pharmacological and/or physiological effect. The effect may be prophylactic in terms of completely or partially preventing a disease or symptoms thereof; and/or may be therapeutic in terms of partially or completely stabilizing or curing a disease and/or side effects due to the disease. As used herein, "treatment" encompasses any treatment of a disease in a patient, including: (a) preventing a disease or symptom that occurs in a patient susceptible to the disease or symptom but has not yet been diagnosed as having the disease; (b) inhibiting symptoms of a disease, i.e., arresting its development; or (c) alleviating symptoms of a disease, i.e., causing regression of the disease or symptoms.

In the present invention, "subject" refers to a vertebrate. In certain embodiments, the vertebrate is a mammal. Mammals include, but are not limited to, livestock (such as cattle), pets (such as cats, dogs, and horses), primates, mice and rats. In certain embodiments, the mammal refers to a human.

In the present invention, "an effective amount" refers to an amount effective in achieving the desired therapeutic or prophylactic effect at the necessary dosage and time. "A therapeutically effective amount" of a substance/molecule of the present invention may vary according to factors such as the disease state, age, gender and weight of the individual, and the ability of the substance/molecule to elicit a desired response in the individual and the like. A therapeutically effective amount also encompasses an amount at which the therapeutic benefits of the substance/molecule outweigh any toxic or harmful consequences. "A prophylactically effective amount" refers to an amount effective in achieving the desired prophylactic effect at the necessary dosage and time. Typically, but not necessarily, a prophylactically effective amount may be lower than a therapeutically effective amount, since the prophylactic dose is administered to a subject prior to the onset of the disease or at an early stage of the disease. In the case of cancer, a therapeutically effective amount of a drug may reduce the number of cancer cells; reduce the volume of tumor; inhibit (i.e., slow to a certain extent, preferably stop) infiltration of cancer cells into peripheral organs; inhibit (i.e., slow to a certain extent, preferably stop) tumor metastasis; inhibiting the growth of tumor to a certain extent; and/or alleviate one or more symptoms associated with cancer to a certain extent.

The pharmaceutical composition of the present invention may comprise pharmaceutically acceptable excipients, including but not limited to: ion exchangers, alumina, aluminum stearate, lecithin, serum proteins such as human serum albumin, buffers such as phosphates, glycerol, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinylpyrrolidone, cellulosic materials, polyethylene glycol, sodium carboxymethylcellulose, polyacrylates, beeswax, lanolin and the like.

The pharmaceutical composition of the present invention may be prepared in various forms according to different administration routes. For example, the pharmaceutical composition may be administered in any of the following ways: oral, aerosol inhalation, rectal, nasal, buccal, vaginal, topical, parenteral such as subcutaneous, intravenous, intramuscular, intraperitoneal, intrathecal, intraventricular, intrasternal and intracranial injection or infusion, or via an external reservoir. Among them, oral or intravenous administration is preferred.

The compound of the present invention may optionally also be used in combination with one or more other active ingredients, the respective amounts and ratios of which may be adjusted by those skilled in the art according to specific diseases, specific conditions of the patient, clinical needs and the like.

As used herein, unless otherwise indicated, the term "prodrug" refers to derivatives that can hydrolyze, oxidize, or otherwise react under biological conditions (*in vitro* or *in vivo*) to provide compounds of the invention. Prodrugs only become active compounds through this reaction under biological conditions, or they do not have or only have low activity in their non reactive form. Prodrugs can generally be prepared using well known methods, such as those described in Burger's Medicinal Chemistry and Drug Discovery (1995) 172-178, 949-982 (Manfred E. Wolff, 5^{th} Edition).

Stereoisomers of the compounds described herein, when specifically specified by chemical name as (R)-or (S)-isomers, should be understood to have the predominant configuration as (R)-isomer or (S)-isomer, respectively. Any asymmetric carbon atom may be present in the (R)-, (S)-or (R, S)-configuration, preferably in the (R)-or (S)-configuration.

The compounds described herein include all possible tautomers of all compounds described herein. The term "tautomer" refers to a functional group isomer formed due to the rapid movement of an atom in two positions within a molecule, such as the very typical enol-keto tautomer.

The compounds described herein include all possible isotope-labeled compounds of all compounds described herein. The term "isotope-labeled compound" refers to a compound obtained by replacing any one atom of the compound with an isotopic atom thereof.

"Solvated compound" or "solvate" may be used interchangeably and refers to a compound that is present in combination with a certain solvent molecule. The combination may include a stoichiometric amount of a certain solvent, for example, when the solvent is water, a "hydrate", such as a monohydrate or dihydrate, is formed, or it may include any amount of water; as another example, when the solvent is an alcohol, such as methanol or ethanol, an "alcoholate" may be formed, which may also be stoichiometric or non-stoichiometric. The term "solvate" as used herein refers to a solid form, i.e. although a compound in a solution of a solvent may be solvated, it is not the solvate which the term used herein refers to.

As used herein, the term "metabolite" refers to a derivative of a compound that is formed when the compound is metabolized. The term "metabolism" refers to the sum of processes (including, but not limited to, hydrolysis reaction and enzyme-catalyzed reaction) by which a particular substance is altered by an organism.

As used herein, the term "ester" refers to an ester formed by-COOH present in a compound provided herein with a suitable alcohol, or an ester formed by-OH present in a compound provided herein with a suitable acid (such as carboxylic acid or oxygen-containing inorganic acid). Suitable ester groups include, but are not limited to, formate, acetate, propionate, butyrate, acrylate, ethylsuccinate, stearate or palmitate. Ester can undergo hydrolysis reaction in the presence of an acid or a base to produce the corresponding acid or alcohol.

As used herein, the term "pharmaceutically acceptable salt" refers to (i) salts formed by an acidic functional group (such as-COOH) present in a compound provided herein with a suitable inorganic or organic cation (base), including, but not limited to, alkali metal salts such as sodium salt, potassium salt, lithium salt and the like; alkaline earth metal salts such as calcium salt, magnesium salt and the like; other metal salts such as aluminum salt, iron salt, zinc salt, copper salt, nickel salt, cobalt salt and the like; inorganic base salts such as ammonium salt; organic base salts such as tert-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylamine salt, tris(hydroxymethyl) aminomethane salt. And, (ii) salts formed by a basic functional group (such as-NH₂) present in a compound provided herein with a suitable inorganic or organic anion (acid), including, but not limited to, hydrohalic salts such as hydrofluoride, hydrochloride, hydrobromide, hydroiodide and the like; inorganic acid salts such as nitrate, perchlorate, sulfate, phosphate and the like; lower alkanesulfonates such as methane sulfonate, trifluoromethanesulfonate, ethanesulfonate and the like; aryl sulfonates such as benzenesulfonate, p-benzenesulfonate and the like; organic acid salts such as acetate, malate, fumarate, succinate, citrate, tartrate, oxalate, maleate and the like; amino acid salts such as glycinate, trimethylglycinate, arginate, ornithine, glutamate, aspartate and the like.

As used herein, the term "crystal form" refers to the crystal structure of a substance. When a substance is crystallized, the intramolecular or intermolecular bonding mode takes change due to the influence of various factors, resulting in different arrangement of molecules or atoms in the lattice space, thereby forming different crystal structures. The compound of the present invention may exist in one crystal structure or in multiple crystal structures, i.e., as "polymorphs". The compound of the invention may exist in different crystal forms.

### Examples

The present invention also provides methods for preparing the corresponding compounds. The compounds described herein may be prepared using a variety of synthetic methods, including the methods described below. The compounds or pharmaceutically acceptable salts, isomers or hydrates thereof described herein may be synthesized using the methods described below and synthetic methods known in the field of organic chemical synthesis, or by variations of these methods as understood by those skilled in the art. The preferred methods include, but are not limited to, the methods described below.

In order to make the objects, technical solutions and advantages of the present invention more apparent, the present invention is described in further detail below with reference to specific embodiments. It should be understood that the specific embodiments described herein are merely illustrative of the present invention, and are not intended to limit the present invention. Where particular techniques or conditions are not specified in the examples, they are performed according to techniques or conditions described in literature in the art or according to the product instructions. The reagents or instruments used, for which manufacturers are not indicated, are all conventional products that are commercially available. As used herein, the term "and/or" includes any and all combinations of one or more related listed items. The examples provided below are intended to better illustrate the present invention, all temperatures being in °C unless otherwise indicated. The names of partial compounds of the present invention are translated according to the Chemdraw nomenclature.

### Example 1:

### Preparation of (1R, 4R)-5-(3-(1-(1-cyclohexyl-3-(difluoromethyl)-1H-pyrazol-4-yl)-1H-1,2,3-triazol-4-yl]pyrazolo[1,5-a]pyrimidin-5-yl)-2-oxa-5-azabicyclo[2.2.1]heptane (1A)

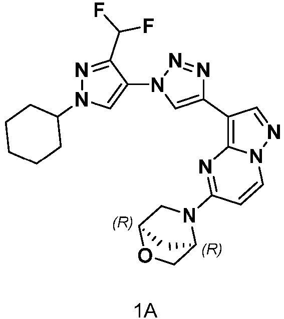

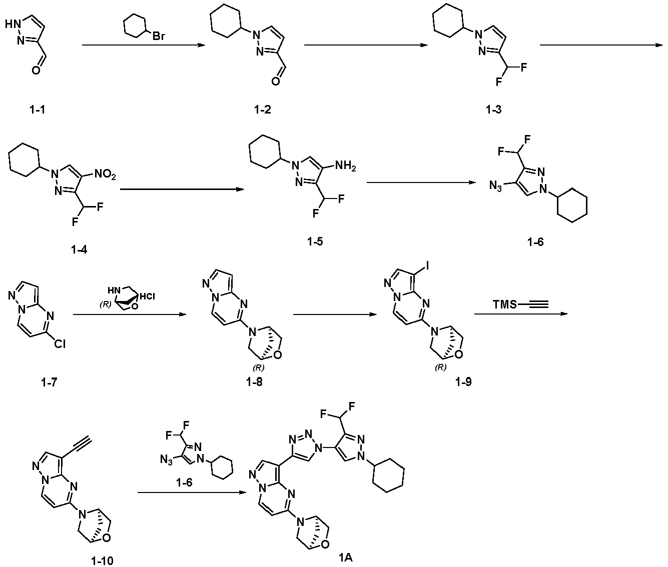

### 1) Step 1 Preparation of compound 1-2

At room temperature, to a three-necked bottle, compound 1-1 (5.00 g, 52.04 mmol), N,N-dimethylformamide (100 mL) were added and stirred to dissolve, then bromocyclohexane (12.7 g, 78.1 mmol) and potassium carbonate (14.4 g, 104 mmol) were added in sequence. After stirring at 100°C for 16 h, the reaction solution, with the addition of water, was extracted with ethyl acetate (300 mL×3). The organic phase was washed with saturated brine (100 mL×1), dried over anhydrous sodium sulfate, and concentrated under reduced pressure. The residue was purified by column chromatography (0-20% ethyl acetate/petroleum ether) to obtain compound 1-2 (1.3 g). LC-MS: m/z: 179.0 (M+H) ⁺

### 2) Step 2 Preparation of compound 1-3

At room temperature, to a three-necked bottle, compound 1-2 (1.30 g, 7.29 mmol), dichloromethane (20 mL) were added, and, after fully stirring, under ice-bath, diethylamino sulfur trifluoride (10.58 g, 65.65 mmol) was added dropwise. After stirring for 16 h, the reaction solution was slowly recovered to room temperature. The reaction solution was slowly added to ice-water, adjusted to neutral pH with sodium bicarbonate, and extracted with ethyl acetate (50 mL×3). The organic phase was washed with saturated brine (20 mL×1), dried over anhydrous sodium sulfate, and concentrated under reduced pressure. The residue was purified by column chromatography (0-30% dichloromethane/petroleum ether) to obtain compound 1-3 (1 g). LC-MS: m/z: 201.0 (M+H) ⁺

### 3) Step 3 Preparation of compound 1-4

Under ice-bath cooling, to a single-necked bottle, compound 1-3 (1.00 g, 4.99 mmol), then concentrated sulfuric acid (5 mL) were added, after uniformly mixing, concentrated nitric acid was added to the reaction solution. After stirring at 0°C for 5 min, the reaction solution was heated to 120°C to carry out reaction for 2 h. The reaction solution was slowly added to ice-water, adjusted to neutral pH with sodium bicarbonate, and extracted with ethyl acetate (50 mL×3). The organic phase was washed with saturated brine (10 mL×1), dried over anhydrous sodium sulfate, and concentrated under reduced pressure. The residue was purified by column chromatography (0-25% ethyl acetate/petroleum ether) to obtain compound 1-4 (700 mg). LC-MS: m/z: 246.0 (M+H) ⁺.

### 4) Step 4 Preparation of compound 1-5

At room temperature, to a single-necked bottle, compound 1-4 (700 mg, 2.85 mmol), then ethanol (10 mL) were added and stirred to dissolve, to which 100 mg of Pd/C (10%) was added. After replacement of the atmosphere with hydrogen three times, the reaction solution was stirred at room temperature for 2 h. The reaction solution was filtered with diatomite, the filter cake was rinsed with ethyl acetate (10 mL × 3), and the filtrate was concentrated under reduced pressure to obtain product. The product 1-5 (600 mg) was directly used in the next reaction. LC-MS: m/z: 216.0 (M+H) ⁺.

### 5) Step 5 Preparation of compound 1-6

Under ice-bath, to a three-necked bottle, compound 1-5 (600 mg, 2.79 mmol), and acetonitrile (5 mL) were added, and uniformly mixed. After replacement of the atmosphere with nitrogen three times, tert-butyl nitrite (431.18 mg, 4.18 mmol) was added, and the reaction solution was stirred under ice-bath for 0.5 h, to which azidotrimethylsilane (642.30 mg, 5.58 mmol) was added. After stirring for 16 h, the reaction solution was slowly recovered to room temperature. With the addition of water, the reaction solution was extracted with dichloromethane (20 mL×3). The organic phase was dried over anhydrous sodium sulfate, and concentrated under reduced pressure. The residue was purified by column chromatography (0-5% ethyl acetate/petroleum ether) to obtain compound 1-6 (400 mg). LC-MS: m/z: 241.9 (M+H) ⁺.

### 6) Step 6 Preparation of compound 1-8

At room temperature, to a single-necked bottle, compound 1-7 (3.02 g,19.54 mmol), then acetonitrile (30 mL) were added and stirred to dissolve. At room temperature, N,N-diisopropylethylamine (6.89 g, 53.28 mmol) and compound (1R,4R)-2-oxa-5-azabicyclo[2.2.1]heptane hydrochloride (2.41 g, 17.76 mmol) were added in sequence. After stirring at 60°C for 4 h, the reaction solution, with the addition of water, was extracted with ethyl acetate (100 mL) twice. The organic phase was washed with saturated brine (100 mL), dried over anhydrous sodium sulfate, and concentrated under reduced pressure. The crude product was purified by column chromatography (30-60% ethyl acetate/petroleum ether) to obtain compound 1-8 (3.84 g).

LC-MS: m/z: 217.1 (M+H) ⁺.

### 7) Step 7 Preparation of compound 1-9

At room temperature, to a single-necked bottle, compound 1-8 (1.02 g, 4.62 mmol) and acetonitrile (10 mL) were added, and stirred to dissolve, then N-iodosuccinimide (1.56 g, 6.94 mmol) was added. After stirring at room temperature for 18 h, the reaction was quenched with saturated sodium thiosulfate aqueous solution (20 ml), and the reaction solution was extracted with ethyl acetate (50 mL) twice. The organic phase was washed with saturated brine (50 mL), dried over anhydrous sodium sulfate, and concentrated under reduced pressure. The crude product was purified by column chromatography (30-80% ethyl acetate/petroleum ether) to obtain compound 1-9 (1.4 g). LC-MS: m/z: 343.0 (M+H) ⁺.

### 8) Step 8 Preparation of compound 1-10

At room temperature, to a single-necked bottle, compound 1-9 (1.00 g, 2.93 mmol), then tetrahydrofuran (10 mL) were added and stirred to dissolve, to which ethynyltrimethylsilane (285 mg, 2.93 mmol), triethylamine (1.25 mL, 8.77 mmol), cuprous iodide (55.1 mg, 0.29 mmol), bis(triphenylphosphine) palladium dichloride (205 mg, 0.29 mmol) were added. After replacement of the atmosphere with nitrogen three times, the reaction solution was stirred at room temperature for 12 h. With the addition of water, the reaction solution was extracted with ethyl acetate (200 mL) twice. The organic phase was washed with saturated brine (100 mL), dried over anhydrous sodium sulfate, and concentrated under reduced pressure. The crude product was dissolved with methanol (10 ml), to which potassium carbonate (810 mg, 5.85 mmol) was added. After stirring at room temperature for 2 h, the reaction solution was concentrated under reduced pressure. The crude product was purified by column chromatography (0-40% ethyl acetate/petroleum ether) to obtain compound 1-10 (800 mg). LC-MS: m/z: 241.1 (M+H) ⁺.

### 9) Step 9 Preparation of compound 1A

At room temperature, to a single-necked bottle, compound 1-10 (100 mg, 0.41 mmol), compound 1-6 (100.41 mg, 0.41 mmol), ethanol (5 mL) and water (2 ml) were added and stirred to dissolve, to which sodium ascorbate (8.21 mg, 0.04 mmol) and blue vitriol (10.35 mg, 0.04 mmol) were added. After stirring at room temperature for 18 h, the reaction solution, with the addition of water, was extracted with ethyl acetate (100 mL) twice. The organic phase was washed with saturated brine (100 mL), dried over anhydrous sodium sulfate, and concentrated under reduced pressure. The crude product was purified by HPLC (Gilson_306_1741, chromatographic column: Waters-Xbridge-C18-10µm-19*250mm; mobile phase: water (containing 10mmol/L ammonium bicarbonate) and acetonitrile, gradient ratio: acetonitrile 19%-95%, flow rate: 25mL/min), to obtain compound 1A (7 mg). ¹H NMR (400 MHz, DMSO-d6) δ 8.72 (d, J = 7.7 Hz, 1H), 8.66 (s, 1H), 8.35 (s, 1H), 7.21 (t, J = 53.2 Hz, 1H), 6.57 (d, J = 152.0 Hz, 1H), 5.13 (d, J = 102.1 Hz, 1H), 4.74 (s, 1H), 4.39 - 4.22 (m, 1H), 3.74 (dd, J = 68.0, 26.5 Hz, 5H), 2.10 (d, J = 14.7 Hz, 2H), 1.96 (d, J = 13.7 Hz, 2H), 1.89 - 1.63 (m, 5H), 1.43 (q, J = 12.8 Hz, 2H), 1.33 - 1.16 (m, 1H). LC-MS: m/z: 482.2 (M+H) ⁺.

### Example 2

### Preparation of (1R,4R)-5-(3-(1'-cyclohexyl-3'-(difluoromethyl)-1'-[1,4'-bispyrazol]-4-yl) pyrazolo[1,5-a] pyrimidin-5-yl)-2-oxa-5-azabicyclo[2.2.1]heptane (2A)

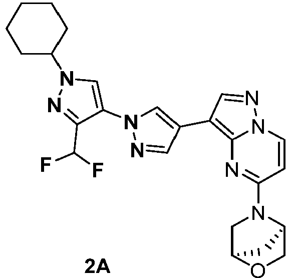

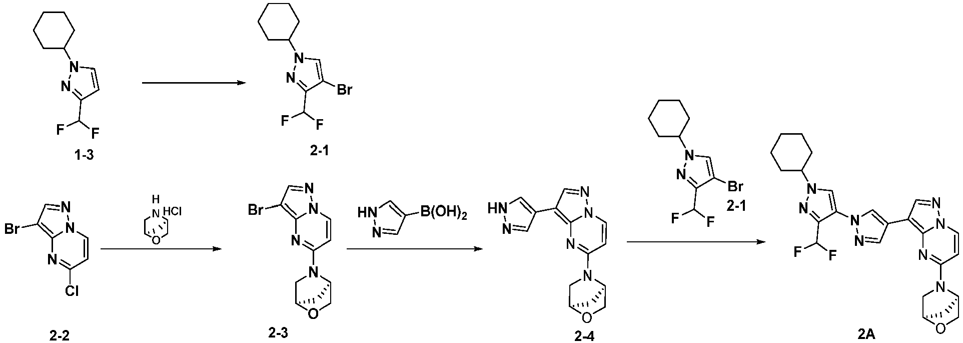

### 1) Step 1 Preparation of compound 2-1

Under ice-bath, to a single-necked bottle, compound 1-3 (700 mg, 3.50 mmol), then dichloromethane (15 mL) were added and stirred to dissolve, and, at 0°C, N-bromosuccinimide (933 mg, 5.24 mmol) was added. After stirring at room temperature for 24 h, the reaction solution, with the addition of water, was extracted with dichloromethane (20 mL×2). The organic phase was washed with saturated brine (10 mL×1), dried over anhydrous sodium sulfate, and concentrated under reduced pressure. The residue was purified by column chromatography (0-2% dichloromethane/petroleum ether) to obtain compound 2-1 (400 mg). LC-MS: m/z: 279.0 (M+H) ⁺

### 2) Step 2 Preparation of compound 2-3

At room temperature, to a solution of compound 2-2 (1.00 g, 4.30 mmol) in N,N-dimethylformamide (15 mL), N,N-diisopropylethylamine (3.56 mL, 21.51 mmol) and (1R,4R)-2-oxa-5-azabicyclo[2.2.1]heptane hydrochloride (0.58 g, 4.30 mmol) were added, and the obtained mixture was stirred at 100°C for 4 h. The reaction solution was concentrated under reduced pressure, and the residue was purified by rapid column chromatography (ethyl acetate/petroleum ether=1/3), to obtain compound 2-3 (960 mg). LC-MS: m/z: 296.7 (M+H) ⁺.

### 3) Step 3 Preparation of compound 2-4

At room temperature, to a microwave tube, compound 2-3 (250 mg, 0.85 mmol), N,N-dimethylformamide (5 mL) and water (1 mL), 1H-pyrazole-4-boric acid (94.78 mg, 0.85 mmol), sodium carbonate (270.27 mg, 2.55mmol) and tetrakis(triphenylphosphine)palladium (98.23mg, 0.09mmol) were added in sequence. After purging with nitrogen for 3 min, the microwave tube was sealed and put into a microwave instrument. After stirring at 120°C for 1 h, the reaction solution was cooled to room temperature. The reaction solution was concentrated under reduced pressure. The residue was purified by column chromatography (0-10% ethyl acetate/petroleum ether) to obtain compound 2-4 (100 mg). LC-MS: m/z: 282.8 (M+H) ⁺.

### 4) Step 4 Preparation of compound 2A

At room temperature, to a microwave tube, compound 2-4 (100 mg, 0.35 mmol), compound 2-1 (118.65 mg, 0.43 mmol), N,N-dimethylformamide (5 mL), trans-(1R,2R)-N,N'-dimethyl 1,2-cyclohexanediamine (10.08 mg, 0.07 mmol), cuprous iodide (10.12 mg, 0.05 mmol) and potassium phosphate (225.56 mg, 1.06 mmol) were added in sequence. After purging with nitrogen for 3 min, the microwave tube was sealed and put into a microwave instrument. After stirring at 120°C for 4 h, the reaction solution was cooled to room temperature. The reaction solution was concentrated under reduced pressure, and the residue was purified by HPLC (Gilson_306_1741, chromatographic column: Waters-Xbridge-C18-10µm-19*250mm; mobile phase: water (containing 10mmol/L ammonium bicarbonate) and acetonitrile, gradient ratio: acetonitrile 19%-95%, flow rate: 25mL/min) to obtain compound 2A (29.5 mg). ¹H NMR (400 MHz, DMSO-d6) δ 8.66 (s, 1H), 8.64(s, 1H), 8.44 (s, 1H), 8.39 (s, 1H), 8.21 (s, 1H), 8.15 (s, 1H), 7.23 (t, J = 53.5 Hz, 1H), 6.41 (s, 1H), 4.73 (d, J = 2.4 Hz, 1H), 4.24 (t, J = 11.5 Hz, 1H), 3.83 (d, J = 7.2 Hz, 1H), 3.74 (s, 1H), 3.59 (d, J = 9.3 Hz, 1H), 3.30 (s, 1H), 2.07 (d, J = 11.8 Hz, 2H), 2.03 - 1.91 (m, 2H), 1.83 (d, J = 14.2 Hz, 2H), 1.75 (dd, J = 12.1, 3.7 Hz, 2H), 1.73 - 1.61 (m, 2H), 1.41 (q, J = 12.9 Hz, 2H). LC-MS: m/z: 481.3 (M+H) ⁺

### Example 3

### Preparation of 3-(1-(1-cyclohexyl-3-(difluoromethyl)-1H-pyrazol-4-yl)-1H-1,2,3-triazol-4-yl) pyrazolo[1,5-a] pyrimidine-6-carbonitrile (3A)

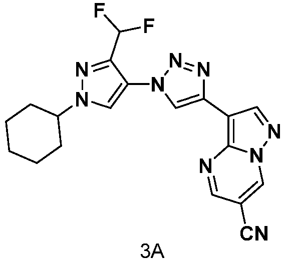

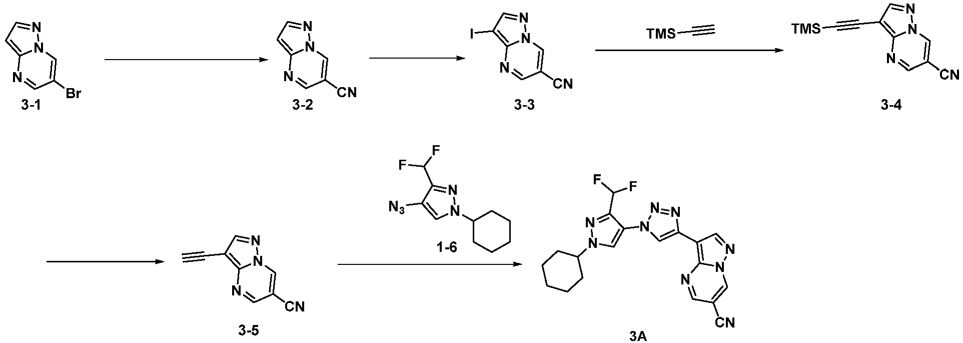

### 1) Step 1 Preparation of compound 3-2

At room temperature, to a microwave tube, compound 3-1 (1.00 g, 5.05 mmol), N,N-dimethylformamide (10 mL), zinc cyanide (0.47 g, 4.04 mmol), tris(dibenzylideneacetone)dipalladium (0.46 g, 0.50 mmol), 4,5-bis(diphenylphosphino)-9,9-dimethylxanthene (0.15 g, 0.25 mmol) were added. After replacement of the atmosphere with nitrogen three times, the reaction solution was reacted at 130°C in a microwave reactor for 30 min. The reaction solution, with the addition of water (20 ml), was extracted with ethyl acetate (20 mL ×2). The organic phase was dried over anhydrous sodium sulfate and concentrated under reduced pressure. The crude product was purified by column chromatography (0-50%, petroleum ether/ethyl acetate), to obtain compound 3-2 (350 mg). LC-MS: m/z: 144.8 (M+H) ⁺

### 2) Step 2 Preparation of compound 3-3

At room temperature, to a 50 mL single-necked bottle, compound 3-2 (350 mg, 2.43 mmol), N,N-dimethylformamide (5 mL), N-iodosuccinimide (600 mg, 2.67 mmol) were added. After stirring at 25°C for 3 h, the reaction solution was poured into 10% sodium thiosulfate solution (30 mL), and extracted with ethyl acetate (30 mL×2). The organic phase was dried over anhydrous sodium sulfate, and concentrated under reduced pressure. The crude product was purified by column chromatography (0-50%, petroleum ether/ethyl acetate), to obtain compound 3-3 (210 mg). LC-MS: m/z: 270.8 (M+H) ⁺

### 3) Step 3 Preparation of compound 3-4

At room temperature, to a single-necked bottle, compound 3-3 (200 mg, 0.740 mmol), tetrahydrofuran (5 mL), cuprous iodide (14.1 mg, 0.07 mmol), triethylamine (0.31 mL, 2.22 mmol), bis(triphenylphosphine) palladium dichloride (51.9 mg, 0.07 mmol), ethynyltrimethylsilane (0.21 mL, 1.48 mmol) were added, and stirred to dissolve. After replacement of the atmosphere with nitrogen three times, the reaction solution was stirred at 25°C for 16 h. The reaction solution was diluted with water (20 mL), and extracted with ethyl acetate (20 mL×2). The organic phase was dried over anhydrous sodium sulfate, and concentrated under reduced pressure. The crude product was purified by column chromatography (0-20%, petroleum ether/ethyl acetate), to obtain compound 3-4 (150 mg). LC-MS: m/z: 240.9 (M+H) ⁺

### 4) Step 4 Preparation of compound 3-5

At room temperature, to a single-necked bottle, compound 3-4 (110 mg, 0.46 mmol), methanol (2 mL), potassium carbonate (127 mg, 0.92 mmol) were added. The reaction solution was stirred at 25°C for 30 min, and then evaporated to dryness under reduced pressure. The crude product was directly used for the next reaction.

### LC-MS: m/z: 168.8 (M+H) ⁺

### 5) Step 5 Preparation of compound 3A

At room temperature, to a single-necked bottle, compound 3-5 (50.0 mg, 0.300 mmol), tert-butanol (1 mL), water (1 mL), compound 1-6 (86.0 mg, 0.360 mmol), blue vitriol (7.42 mg, 0.30 mmol), sodium ascorbate (5.89 mg, 0.30 mmol) were added. After stirring at 25°C for 3 h, the reaction solution was diluted with water (5 mL), and extracted with ethyl acetate (20 mL×2). The organic phase was dried over anhydrous sodium sulfate, and concentrated under reduced pressure. The crude product was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min) to obtain compound 3A (70 mg). LC-MS: m/z: 410.2 (M+H) ⁺. ¹H NMR (400 MHz, DMSO-d6) δ 10.10 (d, J = 2.0 Hz, 1H),8.98 (s, 1H), 8.95 (d, J = 2.0 Hz,1H), 8.76 (s, 1H), 8.73 (s, 1H), 7.24 (t, J = 53.2 Hz, 1H), 4.26-4.24 (m, 1H), 2.12-2.09 (m, 2H), 1.86-1.67 (m, 4H), 1.48-1.38 (m, 2H), 1.25-1.21 (m,2H).

### Example 4

### Preparation of 4-(3-(1-(1-cyclohexyl-3-(difluoromethyl)-1H-pyrazol-4-yl)-1H-1,2,3-triazol-4-yl) pyrazolo[1,5-a] pyrimidin-5-yl) morpholine (4A)

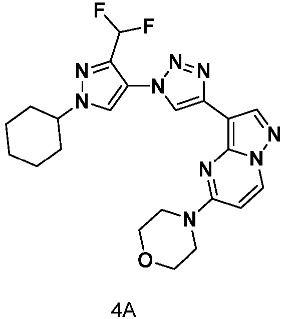

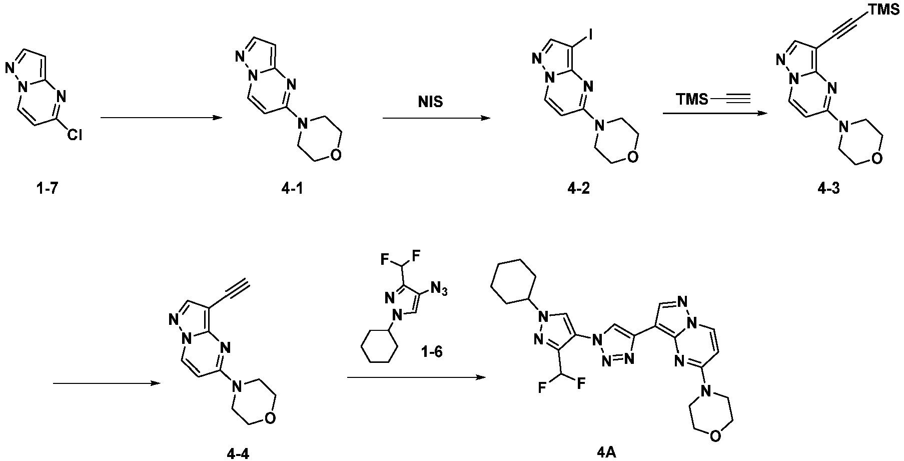

### 1) Step 1 Preparation of compound 4-1

At room temperature, to a single-necked bottle, compound 1-7 (5.00 g, 32.56 mmol) and acetonitrile (50 mL) were added in sequence, and stirred to dissolve, and then morpholine (4.25 g, 48.84 mmol) and N,N-diisopropylethylamine (12.63 g, 97.68 mmol) were added in sequence. After replacement of the atmosphere with nitrogen three times, the reaction solution was stirred at 80°C for 18 h, and then concentrated under reduced pressure. The residue was purified by column chromatography (0-1% methanol/dichloromethane) to obtain compound 4-1 (6.00 g). LC-MS: m/z: 205.4 (M+H) ⁺.

### 2) Step 2 Preparation of compound 4-2

At room temperature, to a single-necked bottle, compound 4-1 (2 g, 9.79 mmol) and acetonitrile (25 mL) were added, and stirred to dissolve, and then N-iodosuccinimide (3.30 g, 14.69 mmol) was added. After replacement of the atmosphere with nitrogen three times, the reaction solution was stirred at 25°C for 18 h, and then concentrated under reduced pressure. The residue was purified by column chromatography (0-1% methanol/dichloromethane) to obtain compound 4-2 (2.0g). LC-MS: m/z: 330.9 (M+H) ⁺.

### 3) Step 3 Preparation of compound 4-3

At room temperature, to a single-necked bottle, compound 4-2 (500 mg, 1.51 mmol) and tetrahydrofuran (10 mL) were added in sequence, and stirred to dissolve, and then ethynyltrimethylsilane (743.80 mg, 7.57 mmol), cuprous iodide (28.84 mg, 0.15 mmol), triethylamine (459.77 mg, 4.54 mmol) and bis(triphenylphosphine) palladium dichloride (106.31 mg, 0.15 mmol) were added in sequence. After replacement of the atmosphere with nitrogen three times, the reaction solution was stirred at 30°C for 18 h, and then concentrated under reduced pressure. The residue was purified by column chromatography (0-2% dichloromethane/petroleum ether) to obtain compound 4-3 ((340 mg). LC-MS: m/z: 300.8 (M+H) ⁺.

### 4) Step 4 Preparation of compound 4-4

At room temperature, to a single-necked bottle, compound 4-3 (300 mg, 1.00 mmol) and anhydrous methanol (10 mL) were added in sequence, and stirred to dissolve, and then potassium carbonate (276.00 mg, 2.00 mmol) was added. After stirring at 25°C for 1 h, the reaction solution was concentrated under reduced pressure, to obtain compound 4-4 (250 mg, 0.88 mmol, 87.75%). Without purification, the compound was directly used in the next reaction. LC-MS: m/z: 228.9 (M+H) ⁺.

### 5) Step 5 Preparation of compound 4A

At room temperature, to a 50 mL single-necked bottle, compound 4-5 (200 mg, 0.83 mmol), tert-butanol (8 mL) and water (5 mL) were added in sequence, and stirred to dissolve, and then blue vitriol (62.2 mg, 0.25 mmol), compound 1-6 (284.17 mg, 1.24 mmol) and sodium ascorbate (49.3 mg, 0.25 mmol) were added in sequence. After replacement of the atmosphere with nitrogen three times, the reaction solution was stirred at 25°C for 3 h, and then concentrated under reduced pressure. The residue was purified by HPLC (Gilson_306_1741, chromatographic column: Waters-Xbridge-C18-10µm-19*250mm; mobile phase: water (containing 10mmol/L ammonium bicarbonate) and acetonitrile, gradient ratio: acetonitrile 19%-95%, flow rate: 25mL/min) to obtain compound 4A (82 mg).

¹H NMR (400 MHz, DMSO-d6) δ 8.75 (d, J = 7.8 Hz, 1H), 8.65 (s, 1H), 8.53 (s, 1H), 8.38 (s, 1H), 7.19 (t, J = 53.2 Hz, 1H), 6.80 (d, J = 7.8 Hz, 1H), 4.33-4.32 (m, 1H), 3.72 (s, 8H), 2.09 (dd, J = 12.9, 3.6 Hz, 2H), 1.88-1.64 (m, 5H), 1.49-1.34 (m, 2H), 1.23 (dd, J = 12.5, 3.6 Hz, 1H). LC-MS: m/z: 470.2 (M+H) ⁺.

### Example 5

### Preparation of 2-(1-(1-cyclohexyl-3-(difluoromethyl)-1H-pyrazol-4-yl)-1H-1,2,3-triazol-4-yl)-6-(trifluoromethyl) pyridine (11A)

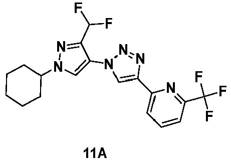

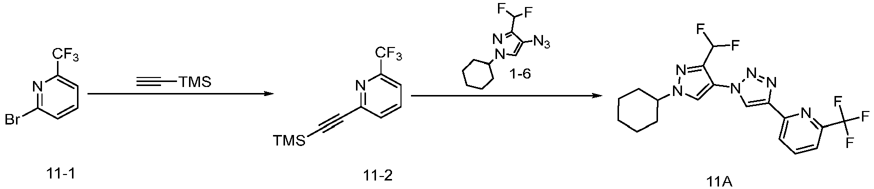

### 1) Step 1 Preparation of compound 11-2

At room temperature, to a single-necked bottle, compound 11-1 (1.00 g, 4.42 mmol) and triethylamine (10 mL) were added in sequence, and stirred to dissolve, and then cuprous iodide (80.1 mg, 0.44 mmol), ethynyltrimethylsilane (800 mg, 8.85 mmol) and bis(triphenylphosphine) palladium dichloride (310 mg, 0.44 mmol) were added in sequence. After replacement of the atmosphere with nitrogen three times, the reaction solution was stirred at 30°C for 18 h, and then concentrated under reduced pressure. The residue was purified by column chromatography (petroleum ether/ethyl acetate =10/1 to 3/1) to obtain compound 11-2 (450 mg). LC-MS: m/z: 244.0 (M+H) ⁺

### 2) Step 2 Preparation of compound 11A

At room temperature, to a single-necked bottle, compound 11-2 (430 mg, 1.77 mmol), 1-(1-cyclohexyl-3-(difluoromethyl)-1H-pyrazol-4-yl)-1H-1,2,3-triazole (554 mg, 2.30 mmol), potassium carbonate (489 mg, 3.53 mmol), blue vitriol and sodium ascorbate (140 mg, 0.710 mmol) were dissolved with a mixed solvent system of water (4 mL) and methanol (4 mL), and stirred to dissolve. After stirring at 30°C for 4 h, the reaction solution was concentrated under reduced pressure. The residue was purified by HPLC (Gilson_306_1741, chromatographic column: Waters-Xbridge-C18-10µm-19*250mm; mobile phase: water (containing 10mmol/L ammonium bicarbonate) and acetonitrile, gradient ratio: acetonitrile 19%-95%, flow rate: 25mL/min) to obtain compound 11A (640 mg).

¹H NMR (400 MHz, CDCl3) δ 8.57 (s, 1H), 8.39 (d, J = 8.0 Hz, 1H), 7.99-7.94 (m, 2H), 7.62 (d, J = 7.6 Hz, 1H), 6.86 (t, J = 53.6 Hz, 1H), 4.22-4.15 (m, 1H), 2.25-2.21 (m, 2H), 1.97-1.92 (m, 2H), 1.78-1.975 (m, 3H), 1.47-1.44 (m, 2H), 1.30-1.24 (m, 1H).LC-MS: m/z: 413.5 (M+H) ⁺

### Example 6

### Preparation of (1R,4r)-4-(4-(4-(5-(((1R,4r)-2-oxa-5-azabicyclo[2.2.1]heptan-5-yl)pyrazolo[1,5-a]pyrimidin-3-yl)-1H-1,2,3-triazol-1-yl)-3-(difluoromethyl)-1H-pyrazol-1-yl)cyclohexane-1-carbaldehyde (25A)

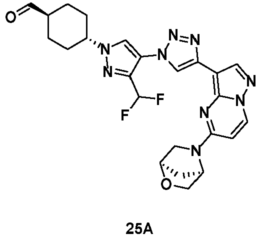

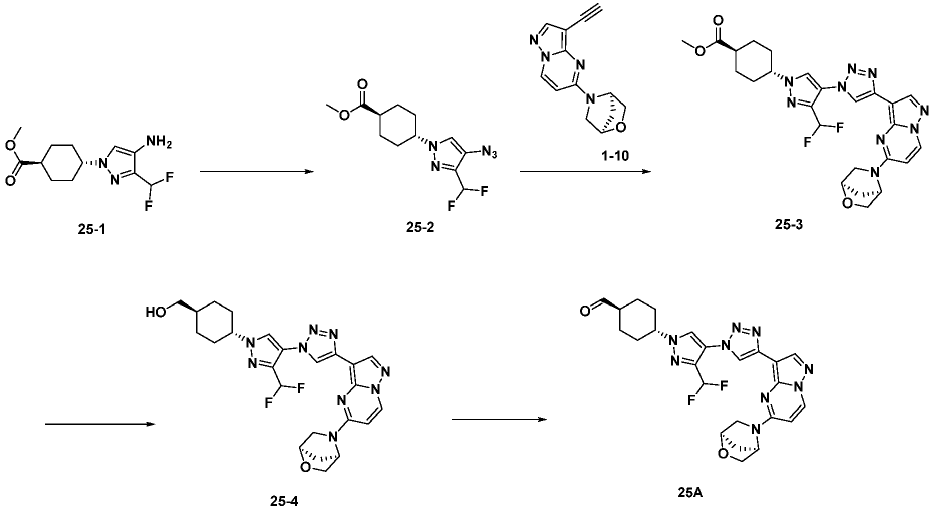

### 1) Step 1 Preparation of compound 25-2

Under ice-bath, to a solution of compound 25-1 (300 mg, 1.09 mmol) in acetonitrile (8 mL), isopentyl nitrite (153 mg, 1.31 mmol) was added. After stirring for 1 h, azidotrimethylsilane (188 mg, 1.64 mmol) was added to the reaction solution. The reaction solution was stirred at room temperature for 16 h, and then poured into water (30 mL), and extracted with ethyl acetate (30 mL×3). The organic phase was washed with saturated brine (50 mL), dried over anhydrous sodium sulfate, and concentrated under reduced pressure, to obtain compound 25-2 (325 mg). Without purification, the compound was directly used in the next reaction. LC-MS: m/z: 300.1 (M+H)⁺

### 2) Step 2 Preparation of compound 25-3

To a mixed solution of compound 25-2 (325 mg, 1.09 mmol) in ethanol (8 mL) and water (5 mL), sodium ascorbate (40 mg, 0.22 mmol), blue vitriol (50 mg, 0.22 mmol) and compound 1-10 (240 mg, 1.09 mmol) were added in sequence. After stirring at room temperature for 2 h, the reaction solution was poured into water (30 mL), and extracted with ethyl acetate (30 mL×3). The organic phase was washed with saturated brine (50 mL), dried over anhydrous sodium sulfate, and concentrated under reduced pressure. The residue was purified by column chromatography (0-8% methanol/dichloromethane) to obtain compound 25-3 (320 mg). LC-MS: m/z: 540.2 (M+H)⁺

### 3) Step 3 Preparation of compound 25-4

To a three-necked bottle, compound 25-3 (320 mg, 0.59 mmol), then tetrahydrofuran (5 mL) were added and stirred to dissolve. After replacement of the atmosphere with nitrogen three times, the mixture was cooled to-50°C, to which a solution of lithium aluminum hydride in tetrahydrofuran (0.60 mL, 0.60 mmol, 1.0 M) was added dropwise. After stirring at-50°C for 1 h, the reaction solution, with the addition of water (20 mL), was extracted with ethyl acetate (20 mL×3). The organic phase was washed with saturated brine (50 mL×1), dried over anhydrous sodium sulfate, and concentrated under reduced pressure. The residue was purified by column chromatography (0-10% methanol/dichloromethane) to obtain compound 25-4 (200 mg). LC-MS: m/z: 512.2 (M+H)⁺

### 4) Step 4 Preparation of compound 25A

To a solution of compound 25-4 (200 mg, 0.39 mmol) in dichloromethane (8 mL), Dess-Martin periodinane (250 mg, 0.59 mmol) was added. After stirring at room temperature for 30 min, the reaction solution, with the addition of water (15 mL), was extracted with dichloromethane (15 mL×3). The organic phase was washed with saturated brine (50 mL), dried over anhydrous sodium sulfate, and concentrated under reduced pressure, to obtain compound 25A (180 mg). LC-MS: m/z: 510.1 (M+H)⁺

### Example 7

### Preparation of 4-(3-(5-(1-cyclohexyl-3-(difluoromethyl)-1H-pyrazol-4-yl)-4H-1,2,4-triazol-3-yl) pyrazolo[1,5-a]pyrimidin-5-yl) morpholine (27A)

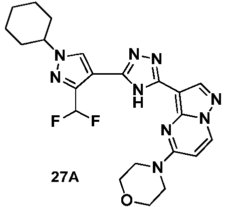

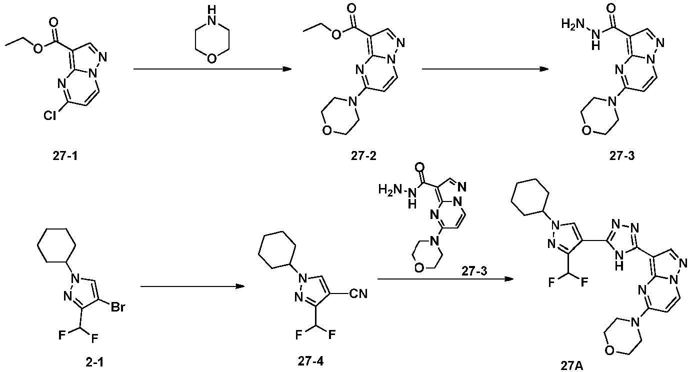

### 1) Step 1 Preparation of compound 27-2

To a single-necked bottle, compound 27-1 (10.0 g, 44.3 mmol), then acetonitrile (100 mL) were added and stirred to dissolve, and at room temperature, N,N-diisopropylethylamine (22.0 mL, 133 mmol) and morpholine (7.72 g, 88.6 mmol) were added in sequence. After stirring under reflux for 2 h, the reaction solution was cooled to room temperature, diluted with water (300 mL), and extracted with ethyl acetate (100 mL×3). The organic phase was washed with saturated brine (300 mL), dried over anhydrous sodium sulfate, and concentrated under reduced pressure. The residue was purified by silica gel column chromatography with an elution system (0~40% ethyl acetate/dichloromethane) to obtain compound 27-2 (9.54 g). LC-MS: m/z: 277 (M+H) ⁺.

¹H NMR (400 MHz, DMSO-d6) δ 8.69 (d, J = 7.9 Hz, 1H), 8.16 (s, 1H), 6.79 (d, J = 7.9 Hz, 1H), 4.13 (q, J = 7.1 Hz, 2H), 3.78 - 3.54 (m, 8H), 1.21 (t, J = 7.1 Hz, 3H).

### 2) Step 2 Preparation of compound 27-3

To a single-necked bottle, compound 27-2 (2.00 g, 7.24 mmol), hydrazine hydrate (7.25 mL, 145 mmol, 98%) and ethanol (20 mL) were added in sequence. After stirring at 45°C for 30 h, the reaction solution was cooled to room temperature, and concentrated under reduced pressure. The residue was recrystallized (petroleum ether/ethyl acetate =1/1, 10 mL) to obtain compound 27-3 (1.5 g). LC-MS: m/z: 263 (M+H) ⁺

¹H NMR (400 MHz, DMSO-d6) δ 8.78 (d, J = 7.9 Hz, 1H), 8.53 (s, 1H), 8.19 (s, 1H), 6.85 (d, J = 7.9 Hz, 1H), 4.42 (s, 1H), 3.77 - 3.70 (m, 8H), 3.33 (s, 2H).

### 3) Step 3 Preparation of compound 27-4

At room temperature, to a single-necked bottle, compound 2-1 (1.30 g, 4.66 mmol) and N,N-dimethylformamide (20 mL) were added and stirred to dissolve, to which zinc powder (0.30 g, 4.66 mmol), zinc cyanide (0.34 g, 4.66 mmol) and 1,1-bis(diphenylphosphino)ferrocene palladium dichloride (0. 34g, 0.46 mmol) were then added. After stirring at 120°C for 18 h, the reaction solution was diluted with water (30 mL), and extracted with ethyl acetate (50 mL) three times. The organic phases were combined, washed with brine (50 mL) once, dried over anhydrous sodium sulfate, and concentrated under reduced pressure. The crude product was purified by column chromatography (0-70% ethyl acetate/petroleum ether) to obtain compound 27-4 (600 mg). LC-MS: m/z: 225.9 (M+H) ⁺

### 4) Step 4 Preparation of compound 27A

To a microwave tube, compound 27-3 (100 mg, 0.38 mmol), compound 27-4 (85.9 mg, 0.38 mmol), potassium carbonate (158 mg, 1.14 mmol) and n-butanol (2 mL) were added in sequence. After heating at 150°C under microwave for 30 h, the reaction solution was filtered, and the filtrate was concentrated under reduced pressure. The residue was separated by silica gel plate (petroleum ether/ethyl acetate =1/2) and then purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min) to obtain 27A (2 mg).

¹H NMR (400 MHz, DMSO-d6) δ 13.34 (s, 1H), 8.79 (d, J = 7.9 Hz, 1H), 8.34 (s, 2H), 7.54 (t, J = 54.1 Hz, 1H), 6.85 (d, J = 8.0 Hz, 1H), 4.34-4.24 (m, 1H), 3.91-3.80 (m, 4H), 3.75-3.70 (m, 4H), 2.10-2.03 (m, 2H), 1.89-1.80 (m, 3H), 1.46-1.38 (m, 2H), 1.28-1.22 (m, 3H). LC-MS: m/z: 470.2 (M+H) ⁺

### Example 8

### Preparation of 4-(3-(5-(1-cyclohexyl-3-(difluoromethyl)-1H-pyrazol-4-yl)-1,3,4-thiadiazol-2-yl) pyrazolo[1,5-a]pyrimidin-5-yl) morpholine (28A)

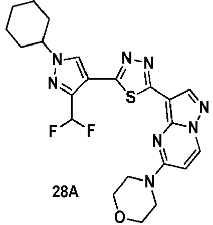

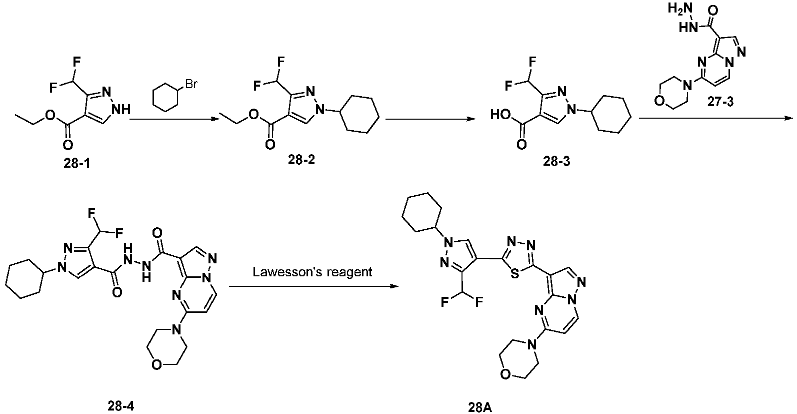

### 1) Step 1 Preparation of compound 28-2

At room temperature, to a single-necked bottle, compound 28-1 (3.75 g, 19.71 mmol, Bide) and N,N-dimethylformamide (15 mL) were added and stirred to dissolve, and then bromocyclohexane (4.93 mL, 39.41 mmol) and potassium carbonate (4.09 g, 29.51 mmol) were added in sequence. After stirring at 80°C for 18 h, the reaction solution was diluted with water (100 mL), and extracted with ethyl acetate (100 mL) three times. The organic phase was washed with saturated brine (150 mL) once, dried over anhydrous sodium sulfate, and concentrated under reduced pressure. The crude product was purified by column chromatography (0-25% ethyl acetate/petroleum ether) to obtain compound 28-2 (1.61 g).

LC-MS: m/z: 273.2 (M+H) ⁺

### 2) Step 2 Preparation of compound 28-3

At room temperature, to a single-necked bottle, compound 28-2 (1.79 g, 19.71 mmol), tetrahydrofuran (10 mL), water (10 mL) and methanol (5 mL) were added and stirred to dissolve, then lithium hydroxide (0.83g, 19.7 mmol) was added. After stirring at 40°C for 2 h, the reaction solution was concentrated under reduced pressure. The aqueous phase was adjusted to pH=2-3 with 2.0M dilute hydrochloric acid. The reaction solution was diluted with water (100 mL), and extracted with ethyl acetate (100 mL) three times. The organic phase was washed with brine (100 mL) once, dried over anhydrous sodium sulfate, and concentrated under reduced pressure to obtain compound 28-3 (1.51 g).

LC-MS: m/z: 245.1 (M+H) ⁺

### 3) Step 3 Preparation of compound 28-4

To a single-necked bottle, compound 28-3 (190 mg, 0.78 mmol), 2-(7-azabenzotriazol-1-yl)-N,N,N',N'-tetramethyluronium hexafluorophosphate (HATU) (444.88 mg, 1.17 mmol), then tetrahydrofuran (5 mL) were added and stirred to dissolve. After stirring at room temperature for 2 h, N,N-diisopropylethylamine (0.39 mL, 2.34 mmol) and compound 28-3 (245.48 mg, 0.94 mmol) were added in sequence. The reaction mixture was stirred at room temperature for 10 h. After completion of the reaction, the reaction was quenched with water (10 mL), and the reaction solution was extracted with ethyl acetate (5 mL×2). The organic phases were combined, washed with saturated brine (15 mL), dried over anhydrous sodium sulfate, and distilled under reduced pressure. The residue was purified by column chromatography (0~5% methanol/dichloromethane) to obtain compound 28-4 (300 mg).
LC-MS: m/z: 489 (M+H) ⁺
¹H NMR (400 MHz, DMSO-d6) δ 10.68 (d, J = 3.4 Hz, 1H), 9.75 (d, J = 3.4 Hz, 1H), 8.84 (d, J = 7.9 Hz, 1H), 8.54 (s, 1H), 8.25 (s, 1H), 7.27 (t, J = 54 Hz, 1H), 6.91 (d, J = 8.0 Hz, 1H), 4.27 (ddd, J = 15.1, 7.6, 3.8 Hz, 1H), 3.87 - 3.68 (m, 8H), 2.11 - 2.06 (m, 2H), 1.85 - 1.80 (m, 2H), 1.71 - 1.65 (m, 3H), 1.46 - 1.40 (m, 2H), 1.30 - 1.27 (m, 1H).

### 4) Step 4 Preparation of compound 28A

To a microwave tube, compound 28-4 (150 mg, 0.31 mmol), Lawesson's reagent (149 mg, 0.37 mmol) and toluene (2.5 mL) were added. The reaction mixture was stirred under reflux for 6 h. After completion of the reaction, the reaction was quenched with water (10 mL), and the reaction solution was extracted with ethyl acetate (5 mL×2). The organic phases were combined, washed with saturated brine (15 mL), dried over anhydrous sodium sulfate, and distilled under reduced pressure. The residue was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min) to obtain compound 28A (70.1 mg). LC-MS: m/z: 487 (M+H) ⁺. ¹H NMR (400 MHz, DMSO-d6) δ 8.86 (d, J = 7.9 Hz, 1H), 8.63 (s, 1H), 8.53 (s, 1H), 7.36 (t, J = 53.6 Hz, 1H), 6.92 (d, J = 7.9 Hz, 1H), 4.36 - 4.25 (m, 1H), 3.91 - 3.72 (m, 8H), 2.13 - 2.04 (m, 2H), 1.89 - 1.74 (m, 4H), 1.73 - 1.65 (m, 1H), 1.48 - 1.36 (m, 2H), 1.27 - 1.22 (m, 1H).

### Example 9

### Preparation of 1-(1-(1-cyclohexyl-3-(difluoromethyl)-1H-pyrazol-4-yl)-1H-1,2,3-triazol-4-yl]-1H-pyrrolo[2,3-b]pyridine-5-carbonitrile (43A)

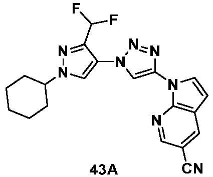

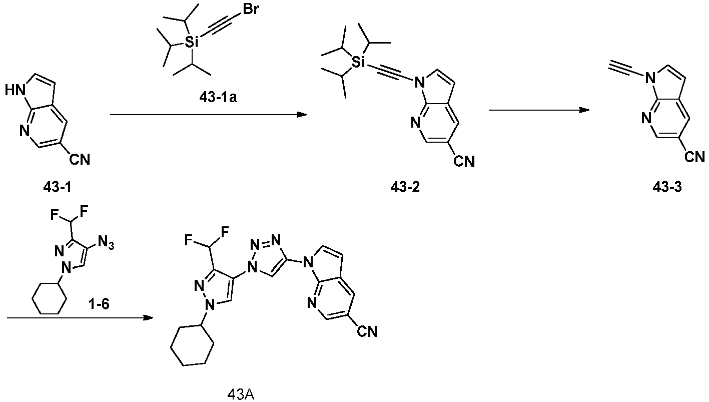

### 1) Step 1 Preparation of compound 43-1

At room temperature, to a single-necked bottle, compound 43-1(0.500 g, 3.49 mmol, Bide), toluene (10 mL) were added and stirred to dissolve, then 43-1a (1.10 g, 4.19 mmol, Bide), 1,10-phenanthroline (0.11 mL, 0.70 mmol), potassium phosphate (1.48 g, 6.98 mmol) and anhydrous cupric sulfate (0.060 g, 0.35 mmol) were added in sequence. The reaction solution was stirred at 80°C under nitrogen atmosphere for 12 h, and then cooled. With the addition of water (30mL), the reaction solution was extracted with ethyl acetate (50 mL×3). The organic phases were combined, washed with saturated brine (20 mL ×1), dried over anhydrous sodium sulfate, and concentrated under reduced pressure. The residue was purified by column chromatography (0-30% petroleum ether/ethyl acetate) to obtain compound 43-2 ((1.0 g). ¹H NMR (400 MHz, CDCl3) δ 8.68 (d, J = 1.9 Hz, 1H), 8.19 (d, J = 1.9 Hz, 1H), 7.46 (d, J = 3.8 Hz, 1H), 6.62 (d, J = 3.8 Hz, 1H), 1.30-1.12 (m, 21H).

### 2) Step 2 Preparation of compound 43-3

Under ice-bath, to a three-necked bottle, compound 43-2(100 mg, 0.31 mmol), then tetrahydrofuran (2mL) were added and stirred, and then tetrabutylammonium fluoride-tetrahydrofuran solution (0.15 mL, 0.46 mmol, 3.0 mol/L) was slowly added dropwise to the reaction solution at 0°C. After stirring at room temperature for 6 h, with the addition of saturated ammonium chloride aqueous solution (5 mL), the reaction solution was extracted with ethyl acetate (10 mL×3). The organic phase was washed with saturated brine (10 mL), dried over anhydrous sodium sulfate, and concentrated under reduced pressure. The residue was purified by column chromatography (0-15% petroleum ether/ethyl acetate) to obtain compound 43-3 (45mg). LC-MS: m/z: 168.0 (M+H) ⁺

### 3) Step 3 Preparation of compound 43A

At room temperature, to a single-necked bottle, compound 43-3 (40.0 mg, 0.17 mmol), compound 1-6 (33.26 mg, 0.20 mmol), then ethanol (2 mL) were added and stirred, to which sodium ascorbate (3.28 mg, 0.02 mmol) and blue vitriol (4.14 mg, 0.02 mmol) were added. After stirring at room temperature for 3 h, the reaction solution was concentrated under reduced pressure. The crude product was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min), to obtain compound 43A (20 mg, 0.05 mmol). ¹H NMR (400 MHz, DMSO-d6) δ 9.05 (s, 1H), 8.82 (d, J = 1.7 Hz, 1H), 8.78 (s, 1H), 8.73 (d, J = 1.8 Hz, 1H), 8.38 (d, J = 3.7 Hz, 1H), 7.25 (t, J = 53.3 Hz, 1H), 6.99 (d, J = 3.8 Hz, 1H), 4.35-4.29 (m, 1H), 2.12 (d, J = 12.6 Hz, 2H), 1.90-1.63 (m, 4H), 1.44 (q, J = 12.8 Hz, 2H), 1.32-1.17 (m, 2H).

### Example 10

### Preparation of 4-(1-(1-(1-(cyclohexyl-3-(difluoromethyl)-1H-pyrazol-4-yl)-1H-1,2,3-triazol-4-yl)-1H-pyrrolo[2,3-b]pyridin-6-yl) morpholine (45A)

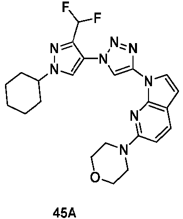

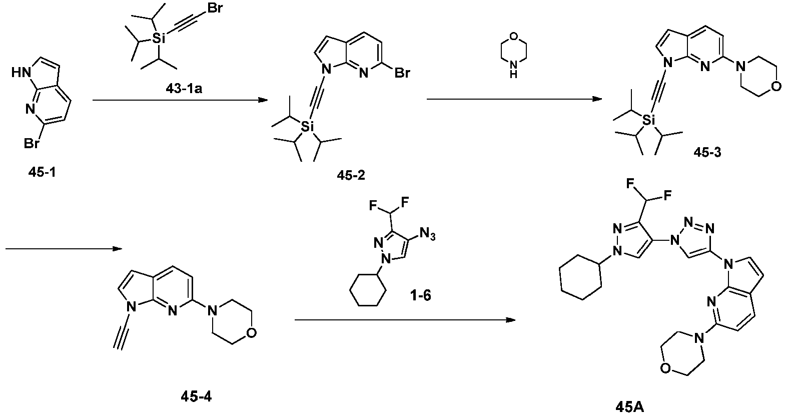

### 1) Step 1 Preparation of compound 45-2

At 25°C, to a single-necked bottle, compound 45-1 (1.00 g, 5.08 mmol, Bide), toluene (10 mL), 43-1a (1.46 g, 5.58 mmol, Bide), tripotassium phosphate (2.15 g, 10.1 mmol), 1,10-phenanthroline (0.180 g, 1.02 mmol), blue vitriol (0.080 g, 0.510 mmol) were added. After replacement of the atmosphere with nitrogen three times, the reaction solution was stirred at 80°C for 18 h. The reaction solution was dried by rotation, diluted with water (20 mL), and extracted with ethyl acetate (20 mL) three times. The organic phases were combined, dried over anhydrous sodium sulfate, and dried by rotation. The residue was purified by column chromatography (ethyl acetate/petroleum ether=1/100 to 1/1), to obtain compound 45-2 (1.40 g). ¹H NMR (400 MHz, CDCl3) δ 7.71 (d, J = 8.1 Hz, 1H), 7.30 (d, J = 8.1 Hz, 1H), 7.26 (d, J = 3.7 Hz, 1H), 6.47 (d, J = 3.7 Hz, 1H), 1.17 (d, J = 1.8 Hz, 21H).

### 2) Step 2 Preparation of compound 45-3

At 25°C, to a single-necked bottle, compound 45-2 (400 mg, 1.06 mmol), toluene (4 mL), morpholine (0.370 mL, 4.24 mmol), palladium acetate (118 mg, 0.530 mmol), tri-tert-butylphosphine (0.130 mL, 0.530 mmol), potassium tert-butoxide (356 mg, 3.18 mmol) were added. After stirring at 80°C for 1 h, the reaction solution was filtered with diatomite. The filtrate was diluted with saturated sodium chloride solution, and extracted with ethyl acetate (10 mL) three times. The organic phases were combined, dried over anhydrous sodium sulfate, and dried by rotation. The residue was purified by column chromatography (ethyl acetate: petroleum ether=1: 100 to 1: 1), to obtain compound 45-3 (250 mg). ¹H NMR (400 MHz, CDCl3) δ 7.68 (d, J = 8.6 Hz, 1H), 7.02 (d, J = 3.7 Hz, 1H), 6.57 (d, J = 8.6 Hz, 1H), 6.33 (d, J = 3.7 Hz, 1H), 3.85-3.81 (m, 4H), 3.60-3.56 (m, 4H), 1.16 (s, 18H), 1.10-1.00 (m, 3H).

### 3) Step 3 Preparation of compound 45-4

At 25°C, to a single-necked bottle, compound 45-3 (300 mg, 0.780 mmol), tetrahydrofuran (3 mL), tetrabutylammonium fluoride solution (1M, 0.940 mL, 0.940 mmol) were added. After stirring at 25°C for 30 min, the reaction was quenched with saturated ammonium chloride solution (20 mL), and the reaction solution was extracted with ethyl acetate (10 mL×2). The organic phases were combined, dried over anhydrous sodium sulfate, and concentrated under reduced pressure. The residue was purified by column chromatography (ethyl acetate: petroleum ether=1: 100 to 1: 1), to obtain compound 45-4 (150 mg). LC-MS: m/z: 228.0 (M+H) ⁺

### 4) Step 4 Preparation of compound 45A

At room temperature, to a single-necked bottle, compound 45-4 (30.0 mg, 0.130 mmol), tert-butanol (1 mL), water (1mL), compound 1-6 (3.18 mg, 0.130 mmol), blue vitriol (3.29 mg, 0.013 mmol), sodium ascorbate (2.62 mg, 0.013 mmol) were added. After stirring at room temperature for 18 h, the reaction solution was filtered. The filter cake was washed with methanol (5 mL), and dried. The residue was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min) to obtain compound 45A (17.0 mg). LC-MS: m/z: 469.2 (M+H) ⁺

¹H NMR (400 MHz, DMSO-d6) δ 8.85 (s, 1H), 8.73 (s, 1H), 7.91 (d, J = 8.7 Hz, 1H), 7.85 (d, J = 3.7 Hz, 1H), 7.24 (t, J = 53.1 Hz, 1H), 6.84 (d, J = 8.7 Hz, 1H), 6.63 (d, J = 3.7 Hz, 1H), 4.35 - 4.29 (m, 1H), 3.80 - 3.71 (m, 4H), 3.56 - 3.48 (m, 4H), 2.16 - 2.06 (m, 2H), 1.85 - 1.67 (m, 4H), 1.47 - 1.41 (m, 2H), 1.25 - 1.22 (m, 2H)

### Example 11

### Preparation of (1R, 4R)-5-(3-(1-(3-(difluoromethyl)-1-methyl-1H-pyrazol-4-yl)-1H-1,2,3-triazol-4-yl]pyrazolo[1,5-a]pyrimidin-5-yl)-2-oxa-5-azabicyclo[2.2.1]heptane (46A)

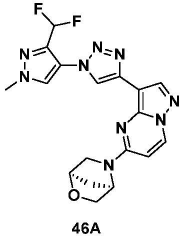

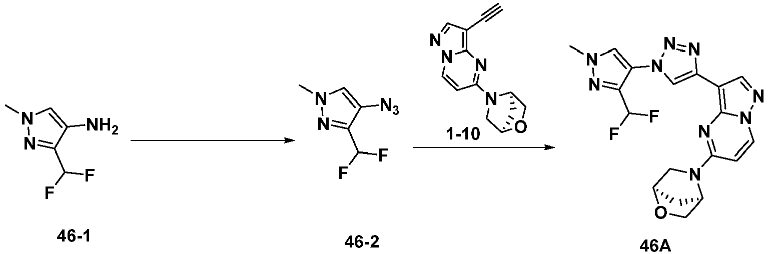

### 1) Step 1 Preparation of compound 46-2

Under ice-bath, to a three-necked bottle, compound 46-1 (50 mg, 0.340 mmol, Bide), acetonitrile (2 mL) were added and stirred to dissolve. After replacement of the atmosphere with nitrogen three times, tert-butyl nitrite (0.090 mL, 0.680 mmol) was slowly added dropwise at 0°C to the reaction solution, followed by stirring under ice-bath for 30 min. Then, azidotrimethylsilane (0.070 mL, 0.510 mmol) was slowly added dropwise at 0°C to the reaction solution, followed by stirring at room temperature for 3 h. With the addition of water, the reaction solution was extracted with ethyl acetate (10 mL×3). The organic phase was washed with saturated brine (5 mL×1), dried over anhydrous sodium sulfate, and concentrated under reduced pressure. The residue was purified by column chromatography (0-30% petroleum ether/ethyl acetate) to obtain compound 46-2 (45 mg). LC-MS: m/z: 174.0[M+1]⁺

### 2) Step 2 Preparation of compound 46A

At room temperature, to a single-necked bottle, compound 46-2 (45 mg, 0.260 mmol), then ethanol (2 mL) and water (1 mL) were added and stirred, to which compound 1-10 (74.9 mg, 0.310 mmol), blue vitriol (6.49 mg, 0.030 mmol), sodium ascorbate (5.15 mg, 0.030 mmol) were added. After stirring at room temperature for 3 h, the reaction solution, with the addition of water, was extracted with ethyl acetate (10 mL×3). The organic phase was washed with saturated brine (5 mL), dried over anhydrous sodium sulfate, and concentrated under reduced pressure. The crude product was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min), to obtain compound 46A (5 mg). ¹H NMR (400 MHz, DMSO-d6) δ 8.73 (d, J = 7.7 Hz, 1H), 8.59 (s, 1H), 8.35 (s, 1H), 7.21 (t, J = 53.2 Hz, 1H), 6.81-6.28 (m, 1H), 5.30-5.10 (m, 1H), 4.74 (s, 1H), 3.99 (s, 3H), 3.83-3.65 (m, 2H), 3.59 (s, 2H), 1.98-1.94 (m, 2H). LC-MS: m/z: 414.4[M+1]⁺.

### Example 12

### Preparation of 3-(1-(1-cyclohexyl-3-(difluoromethyl)-1H-pyrazol-4-yl)-1H-1,2,3-triazol-4-yl]pyrazolo[1,5-a]pyrimidine (56A)

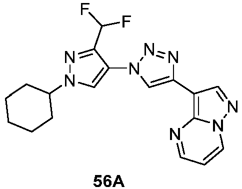

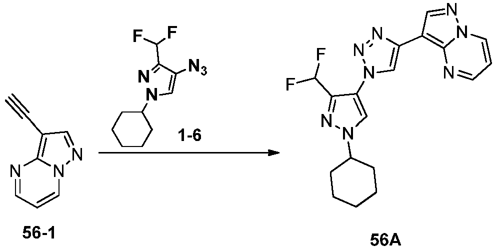

### 1) Step 1 Preparation of compound 56A

At room temperature, to a single-necked bottle, compound 56-1 (60 mg, 0.21 mmol, prepared in accordance with the method for preparation of intermediate 3-ethynylpyrazolo[1,5-a]pyrimidine disclosed on page 111 of the specification in the patent application "WO2015108490 A2"), compound 1-6 (51 mg, 0.21 mmol), and then tetrahydrofuran (1.5 mL) were added and stirred to dissolve, to which cuprous iodide (12 mg, 0.06 mmol) and triethylamine (64 mg, 0.63 mmol) were added. After stirring at room temperature under nitrogen atmosphere for 18 h, the reaction solution was concentrated under reduced pressure to obtain a crude product. The crude product was purified by HPLC (Gilson_306_1741, chromatographic column: Waters-Xbridge-C18-10µm-19*250mm; mobile phase: water (containing 10mmol/L ammonium bicarbonate) and acetonitrile, gradient ratio: acetonitrile 19%-95%, flow rate: 25mL/min), to obtain compound 56A (2.5 mg). ¹H NMR (400 MHz, DMSO-d6) δ 9.24 (d, J = 6.0 Hz, 1H), 8.72 (d, J = 10.8 Hz, 4H), 7.45-7.05 (m, 2H), 4.32-4.26 (m, 1H), 2.13-2.10 (m, 2H), 1.92-1.60 (m, 5H), 1.51-1.35 (m, 2H), 1.30-1.14 (m, 1H). MS m/z(ESI): 384.8[M+1]⁺.

### Example 13

### Preparation of 1-(1-(1-cyclohexyl-3-(difluoromethyl)-1H-pyrazol-4-yl)-1H-1,2,3-triazol-4-yl)-1H-pyrrolo[2,3-b]pyridine-5-carboxylic acid (66A)

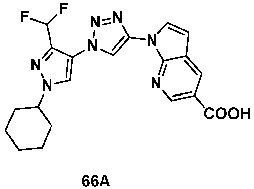

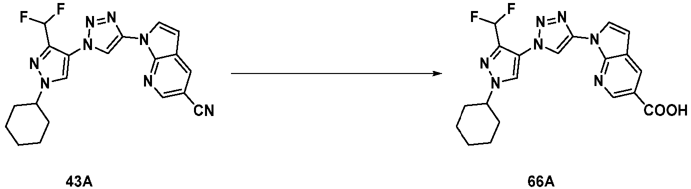

### 1) Step 1 Preparation of compound 66A

At room temperature, to a single-necked bottle, compound 43A (50.0 mg, 0.120 mmol), ethanol (1 mL), water (1 mL), sodium hydroxide (24.0 mg, 0.600 mmol) were added. After reacting at 100°C for 2 h, the reaction solution was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min), to obtain compound 66A (23.0 mg). LC-MS: m/z: 428.0 (M+H) ⁺. ¹H NMR (400 MHz, DMSO-d6) δ 9.05 (s, 1H), 8.92 (d, J = 1.9 Hz, 1H), 8.75 (s, 1H), 8.66 (d, J = 2.0 Hz, 1H), 8.27 (d, J = 3.8 Hz, 1H), 7.22 (t, J = 53.3 Hz, 1H), 6.96 (d, J = 3.8 Hz, 1H), 4.36-4.26 (m, 1H), 2.12 - 2.08 (m, 2H), 1.85-1.65 (m, 4H), 1.50-1.30(m, 2H), 1.25-1.20 (m, 2H).

### Example 14

### Preparation of 1-(1-(1-cyclohexyl-3-(difluoromethyl)-1H-pyrazol-4-yl)-1H-1,2,3-triazol-4-yl)-1H-pyrrolo[2,3-b]pyridine-5-formamide (65A)

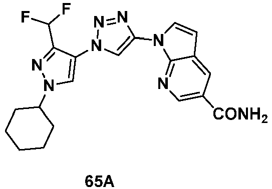

### Synthetic route:

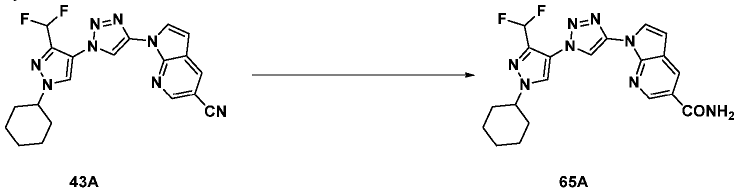

### 1) Step 1 Preparation of compound 65A

At room temperature, to a 25 mL single-necked bottle, compound 43A (50.0 mg, 0.120 mmol), dimethyl sulfoxide (1 mL), potassium carbonate (33.8 mg, 0.240 mmol) were added, and stirred to dissolve, to which hydrogen peroxide (30%, 0.05 mL, 0.61 mmol) was added. After reacting at room temperature for 5 min, the reaction solution was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min), to obtain compound 65A (20.0 mg, 0.050 mmol). LC-MS: m/z: 427.0 (M+H) ⁺. ¹H NMR (400 MHz, DMSO-d6) δ 9.02 (s, 1H), 8.88 (d, J = 1.9 Hz, 1H), 8.75 (s, 1H), 8.60 (d, J = 2.0 Hz, 1H), 8.25 (d, J = 3.7 Hz, 1H), 8.13 (s, 1H), 7.48 (s, 1H), 7.22 (t, J = 53.3 Hz, 1H), 6.92 (d, J = 3.7 Hz, 1H), 4.30 (d, J = 3.8 Hz, 1H), 2.10 (d, J = 12.4 Hz, 2H), 1.83 (d, J = 17.3 Hz, 2H), 1.78-1.74 (m, 1H), 1.70-1.60 (m, 1H), 1.50-1.30 (m, 2H), 1.25-1.20 (m, 2H).

### Example 15

### Preparation of (7-(1-(1-cyclohexyl-3-(difluoromethyl)-1H-pyrazol-4-yl)-1H-1,2,3-triazol-4-yl]pyrrolo[1, 2-b]pyridazin-3-yl) (piperazin-1-yl) methanone (64A)

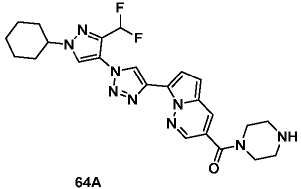

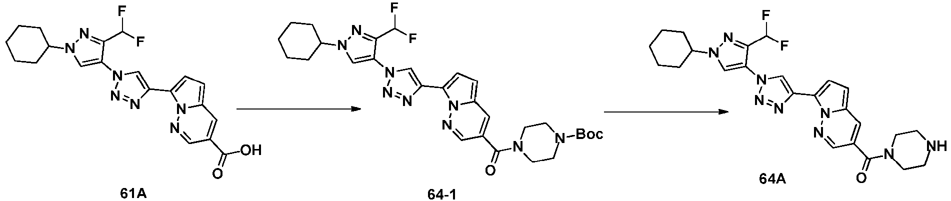

### 1) Step 1 Preparation of compound 64-1

At room temperature, to a single-necked bottle, compound 61A (80 mg, 0.19 mmol), DCM (5 mL) were added and stirred to dissolve, to which O-(7-azabenzotriazol-1-yl)-N,N,N',N'-tetramethyluronium hexafluorophosphate (HATU)

(142.34 mg, 0.37 mmol), N,N-diisopropylethylamine (0.05 mL, 0.28 mmol) and 1-tert-butoxycarbonylpiperazine (41.83 mg, 0.22 mmol) were added in sequence. After stirring at 25°C for 3 h, the reaction solution, with the addition of water (20 mL), was extracted with ethyl acetate (30 mL×3). The organic phase was washed with saturated brine (20 mL×1), dried over anhydrous sodium sulfate, and concentrated under reduced pressure. The residue was purified by column chromatography (0-15% dichloromethane/methanol) to obtain compound 64-1(90 mg). LC-MS: m/z: 596.7 (M+H) ⁺.

### 2) Step 2 Preparation of compound 64A

At room temperature, to a single-necked bottle, compound 64-1 (30 mg, 0.05 mmol), then dichloromethane (2mL) and trifluoroacetic acid (1 ml) were added and stirred. After stirring at room temperature for 0.5 h, the reaction solution was concentrated under reduced pressure. The crude product was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min), to obtain compound 64A (20 mg). ¹H NMR (400 MHz, DMSO-d6) δ 8.98 (s, 1H), 8.75 (s, 1H), 8.43 (d, J = 2.1 Hz, 1H), 8.27 (d, J = 2.1 Hz, 2H), 7.63 (d, J = 4.6 Hz, 1H), 7.24 (t, J = 53.3 Hz, 1H), 6.96 (d, J = 4.6 Hz, 1H), 4.36-4.26 (m, 1H), 3.55 (s, 4H), 2.80 (s, 4H), 2.12 (d, J = 12.3 Hz, 2H), 1.91-1.70 (m, 4H), 1.44 (q, J = 12.9 Hz, 2H), 1.33-1.21 (m, 2H). LC-MS: m/z: 496.3 (M+H) ⁺.

### Example 16

### Preparation of 7-(1-(1-cyclohexyl-3-(difluoromethyl)-1H-pyrazol-4-yl)-1H-1,2,3-triazol-4-yl]pyrrolo[1, 2-b]pyridazine-3-carbonitrile (29A)

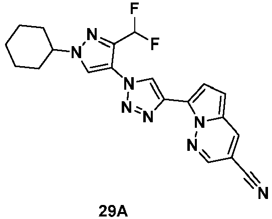

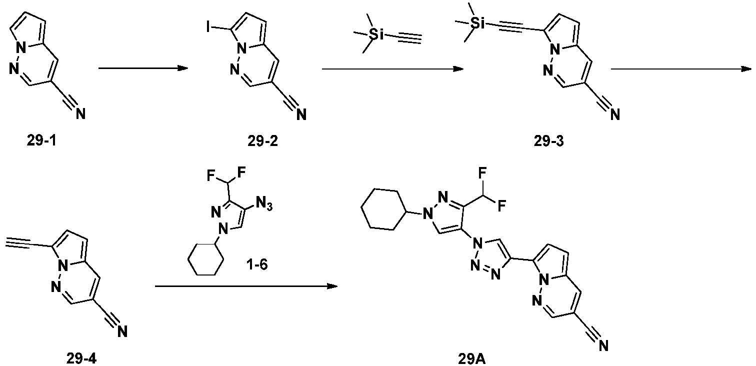

### 1) Step 1 Preparation of compound 29-2

At room temperature, to acetonitrile (10 mL), compound 29-1 (300 mg, 2.10 mmol, prepared in accordance with the method for preparation of intermediate pyrrolo[1,2-b]pyridazine-3-carbonitrile disclosed on page 75 of the specification in the patent application "WO2015117563 A1"), N-iodosuccinimide (707 mg, 3.14 mmol) were added. The reaction solution was stirred at 60°C for 1 h. After completion of the reaction as monitored by TLC, saturated sodium sulfite solution (10 mL) was carefully added to the reaction solution. Then, the mixture was extracted with ethyl acetate (100 mL), and the organic phase was washed with saturated brine (100 mL×2), dried over anhydrous sodium sulfate, filtered and concentrated under reduced pressure. The residue was purified by rapid column chromatography (petroleum ether/ethyl acetate =10/1) to obtain compound 29-2 (270 mg). ¹H NMR (400 MHz, CDCl₃): δ 8.27 (d, *J* = 2.1 Hz, 1H), 8.05 (d, *J* = 2.1 Hz, 1H), 7.24 (d, *J =* 4.7 Hz, 1H), 6.95 (d, *J* = 4.7 Hz, 1H).

### 2) Step 2 Preparation of compound 29-3

Compound 29-2 (1.16 g, 4.31 mmol) was added to tetrahydrofuran (10 mL), to which cuprous iodide (0.08 g, 0.43 mmol), bis(triphenylphosphine) palladium dichloride (0.30 g, 0.43 mmol) and triethylamine (1.20 mL, 8.62 mmol) were then added. After replacement of the atmosphere with nitrogen, ethynyltrimethylsilane (6.14 mL, 43.11 mmol) was then added to the reaction solution. After stirring at room temperature overnight, the reaction solution was added to water, and extracted with ethyl acetate (15 mL×3). The organic phases were combined, washed with saturated brine, dried over anhydrous sodium sulfate, and concentrated. The residue was purified by rapid column chromatography (petroleum ether/ethyl acetate =20/1) to obtain compound 29-3 (816 mg). ¹H NMR (400 MHz, DMSO-d6) δ 8.75 (d, J = 2.2 Hz, 1H), 8.63 (d, J = 2.2 Hz, 1H), 7.38 (d, J = 4.7 Hz, 1H), 6.96 (d, J = 4.8 Hz, 1H), 0.27 (s, 9H).

### 3) Step 3 Preparation of compound 29-4

Compound 29-3 (816 mg, 3.41 mmol) and potassium carbonate (1413.47 mg, 10.23 mmol) were added to methanol (10 mL). After stirring at room temperature for 2 h, the reaction solution, with the addition of water, was extracted with ethyl acetate (15 mL×3). The organic phases were combined, washed with saturated brine, dried over anhydrous sodium sulfate, filtered, and concentrated. The residue was purified by Flash normal phase column (EA: PE=5%) to obtain product 29-4 (175 mg, 1.05 mmol). ¹H NMR (400 MHz, CDCl3) δ 8.28 (d, J = 2.2 Hz, 1H), 8.12 (d, J = 2.2 Hz, 1H), 7.25 (s, 1H), 6.82 (d, J = 4.7 Hz, 1H), 3.78 (s, 1H).

### 4) Step 4 Preparation of compound 29A

Under the protection of nitrogen, compound 29-3 (175 mg, 1.05 mmol) and compound 1-6 (252.55 mg, 1.05 mmol) were added to a mixed solvent of ethanol (8 mL) and water (4 mL), and then blue vitriol (0.01 mL, 0.10 mmol) and sodium ascorbate (20.74 mg, 0.10 mmol) were added. After stirring at room temperature overnight, the reaction solution was distilled under reduced pressure. The residue was purified by column chromatography (MeOH/DCM=1/20), to obtain product 29A (230 mg). LC-MS: m/z: 409.0 (M+H) ⁺.

### Example 17

### Preparation of 7-(1-(1-cyclohexyl-3-(difluoromethyl)-1H-pyrazol-4-yl)-1H-1,2,3-triazol-4-yl) pyrrolo[1, 2-b]pyridazine-3-formamide (60A)

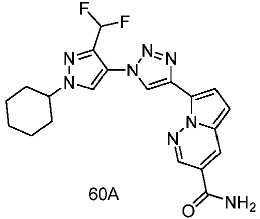

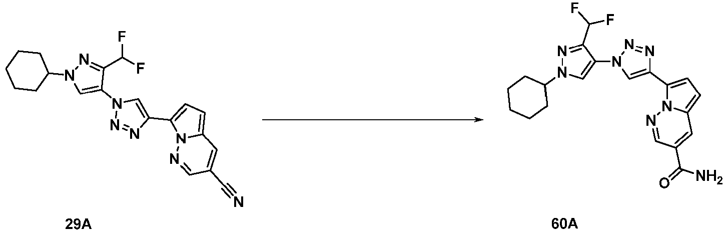

### 1) Step 1 Preparation of compound 60A

Compound 29A (100 mg, 0.24 mmol) was added to a mixed solvent of ammonia aqueous (1 mL) and ethanol (1 mL), then hydrogen peroxide (0.03 mL, 0.98 mmol, 30%) was added dropwise. After reacting at room temperature with stirring for 16h, the reaction was quenched with saturated sodium thiosulfate solution, and the reaction solution was extracted with ethyl acetate (10 mL×3). The organic phases were combined, washed with saturated brine, dried over anhydrous sodium sulfate, filtered and concentrated. The residue was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min), to obtain compound 60A (30 mg, 0.07 mmol). LC-MS: m/z: 427.0 (M+H) ⁺. ¹H NMR (400 MHz, DMSO-d6) δ 9.01 (s, 1H), 8.84-8.73 (m, 2H), 8.66 (d, J = 2.3 Hz, 1H), 8.17 (s, 1H), 7.65 (d, J = 4.6 Hz, 1H), 7.57 (s, 1H), 7.23 (t, J = 53.3 Hz, 1H), 7.05 (d, J = 4.6 Hz, 1H), 4.30 (td, J = 9.6, 7.7, 5.8 Hz, 1H), 2.18-2.06 (m, 2H), 1.94-1.62 (m, 5H), 1.55-1.37 (m, 2H), 1.24 (d, J = 11.3 Hz, 1H).

### Example 18

### Preparation of 7-(1-(1-cyclohexyl-3-(difluoromethyl)-1H-pyrazol-4-yl)-1H-1,2,3-triazol-4-yl]pyrrolo[1, 2-b]pyridazine-3-carboxylic acid (61A)

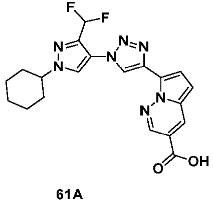

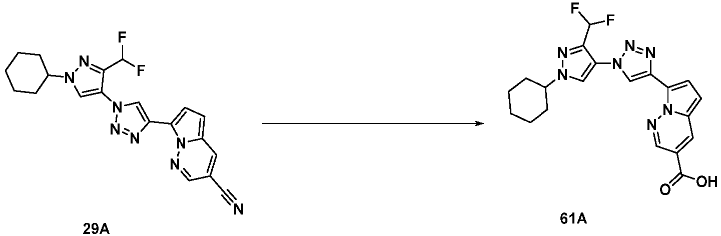

### 1) Step 1 Preparation of compound 61A

Compound 29A (50 mg, 0.12 mmol) was added to a mixed solvent of ethanol (2 mL) and water (2 mL), then sodium hydroxide (48.97 mg, 1.22 mmol) was added. After stirring under reflux for 2h, the reaction solution was concentrated to remove the organic solvent, and then adjusted to pH = 4-5 with HCl (2N), to precipitate out a large amount of solids, followed by filtering. The filter cake was dried to obtain product 61A (25 mg, 0.06 mmol). ¹H NMR (400 MHz, DMSO-d6) δ 13.20 (s, 1H), 9.05 (s, 1H), 8.75 (s, 1H), 8.73-8.69 (m, 2H), 7.69 (d, J = 4.7 Hz, 1H), 7.39-7.08 (m, 2H), 4.34-4.27 (m, 1H), 2.14 - 2.10 (m, 2H), 1.93-1.64 (m, 5H), 1.53-1.35 (m, 2H), 1.30-1.21 (m, 1H). LC-MS: m/z: 428.2 (M+H) ⁺.

### Example 19

### Preparation of 4-(3-(1-(3-(difluoromethyl)-1-(piperidin-4-yl)-1H-pyrazol-4-yl)-1,2,3-triazol-4-yl]pyrazolo[1,5-a]pyrimidin-5-yl) morpholine (69A)

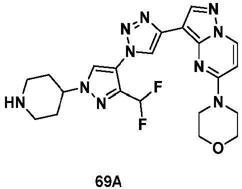

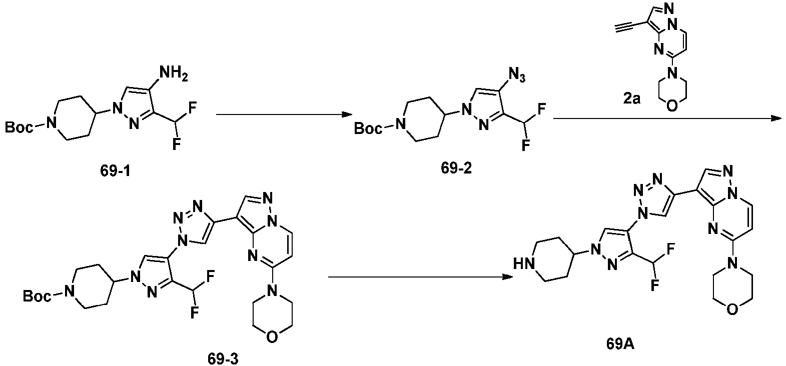

### 1) Step 1 Preparation of compound 69-2

Under ice-bath, to a three-necked bottle, compound 69-1 (1.04 g, 3.29 mmol, prepared in accordance with the method for preparation of product in step 5 disclosed on page 140 of the specification in the patent application "WO2022161414 A1"), acetonitrile (25 mL) were added and stirred to dissolve, and, at 0°C, tert-butyl nitrite (0.47 mL, 3.94 mmol) was slowly added dropwise. Under ice-bath, the reaction solution was stirred for 30 min, to which azidotrimethylsilane (0.65 mL, 4.93 mmol) was slowly added dropwise at 0°C. After stirring at room temperature for 3 h, the reaction solution, with the addition of water (40ml), was extracted with ethyl acetate (25 mL×3). The organic phase was washed with saturated brine (15 mL×1), dried over anhydrous sodium sulfate, and concentrated under reduced pressure, to obtain compound 69-2 (1.05 g, 3.07 mmol, 93.29%). Without purification, the compound was directly used in the next reaction.

LC-MS: m/z: 287.1 (M+H-55) ⁺.

### 2) Step 2 Preparation of compound 69-3

At room temperature, to a single-necked bottle, compound 69-2 (300 mg, 0.88 mmol), then ethanol (4 mL) and water (4 mL) were added and stirred, to which compound 69-2a (200 mg, 0.88 mmol), blue vitriol (21.9 mg, 0.09 mmol), sodium ascorbate (17.36 mg, 0.09 mmol) were added. After stirring at room temperature for 3 h, the reaction solution, with the addition of water (20 mL), was extracted with ethyl acetate (15 mL×3). The organic phase was washed with saturated brine (10mL×1), dried over anhydrous sodium sulfate, and concentrated under reduced pressure. The residue was purified by column chromatography (0-5% dichloromethane/methanol) to obtain compound 69-3 (195 mg, 0.34 mmol, 39.00%). LC-MS: m/z: 571.4 (M+H) ⁺.

### 3) Step 3 Preparation of compound 69A

At room temperature, to a single-necked bottle, compound 69-3 (175 mg, 0.31 mmol) and dichloromethane (3 mL) were added, then a solution of hydrogen chloride in dioxane (4.0 M, 3mL, 12.00 mmol) was added dropwise. After stirring at 25°C for 1 h, the reaction mixture was concentrated under reduced pressure. The residue was purified by HPLC (chromatographic column: Waters-SunFire-C18-10 µm-19*250 mm; mobile phase: water (containing 10 mmol/L hydrochloric acid) and acetonitrile, gradient ratio: acetonitrile 40%-50%, flow rate: 30 mL/min), to obtain compound 69A (53mg, 0.11 mmol, 36.73%). ¹H NMR (400 MHz, DMSO-d6) δ 9.30-9.13 (m, 1H), 8.94 (d, J = 10.3 Hz, 1H), 8.76 (d, J = 7.9 Hz, 1H), 8.71 (s, 1H), 8.55 (s, 1H), 8.38 (s, 1H), 7.23 (t, J = 53.1 Hz, 1H), 6.81 (d, J = 7.9 Hz, 1H), 4.75-4.57 (m, 1H), 3.73 (s, 8H), 3.42 (d, J = 12.9 Hz, 2H), 3.10 (q, J = 11.9 Hz, 2H), 2.39-2.14 (m, 4H). LC-MS: m/z: 471.0 (M+H) ⁺.

### Example 20

### Preparation of 7-(1-(1-cyclohexyl-3-(difluoromethyl)-1H-pyrazol-4-yl)-1H-1,2,3-triazol-4-yl]-N-(pyrrolidin-3-yl)pyrrolo[1, 2-b]pyridazine-3-formamide (67A)

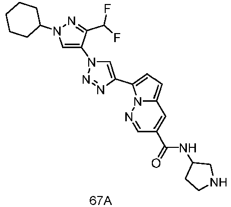

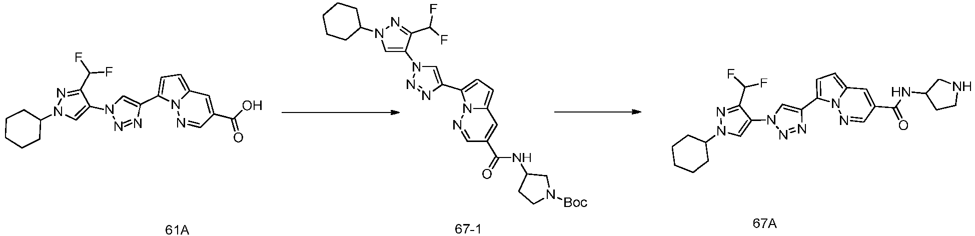

### 1) Step 1 Preparation of compound 67-1

Compound 61A (100mg, 0.23mmol), tert-butyl 3-aminopyrrolidine-1-carboxylate (52.3mg, 0.28mmol, Bide) were dissolved with dichloromethane (5mL), to which 2-(7-azabenzotriazol-1-yl)-N,N,N',N'-tetramethyluronium hexafluorophosphate (HATU) (107mg, 0.28mmol) and triethylamine (0.10mL, 0.70mmol) were added. After reacting at room temperature with stirring for 2 h, the reaction solution, with the addition of water (15ml), was extracted with dichloromethane (15mL×3). The organic phases were combined, dried over anhydrous sodium sulfate, and filtered to remove the desiccant, and then the filtrate was concentrated under reduced pressure. The residue was directly used as a raw material in the next reaction without purification, as the title compound 67-1 (145mg). MS m/z(ESI): 596.5[M+1]⁺.

### 2) Step 2 Preparation of compound 67A

Compound 67-1 (125mg, 0.21mmol) was dissolved with a solution of hydrogen chloride in dioxane (5mL, 4M). After reacting at room temperature with stirring for 1 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain the title compound 67A (30mg). MS m/z(ESI): 496.2[M+1]⁺. ¹H NMR (400 MHz, DMSO-d6) δ 9.11 - 8.95 (m, 2H), 8.90 (d, J = 6.2 Hz, 1H), 8.82 (d, J = 2.3 Hz, 1H), 8.77 - 8.70 (m, 2H), 7.68 (d, J = 4.6 Hz, 1H), 7.23 (t, J = 53.3 Hz, 1H), 7.09 (d, J = 4.6 Hz, 1H), 4.59 - 4.50 (m, 1H), 4.38 - 4.30 (m, 1H), 3.50 - 3.40 (m, 2H), 3.30 - 3.20 (m, 2H), 2.29 - 2.01 (m, 4H), 1.89 - 1.66 (m, 5H), 1.51 - 1.37 (m, 2H), 1.31 - 1.19 (m, 1H).

### Example 21

### Preparation of 7-(1-(1-cyclohexyl-3-(difluoromethyl)-1H-pyrazol-4-yl)-1H-1,2,3-triazol-4-yl]-N-(4,4-difluoropyrrolidin-3-yl)pyrrolo[1, 2-b]pyridazine-3-formamide (68A)

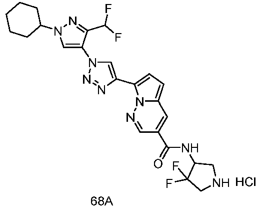

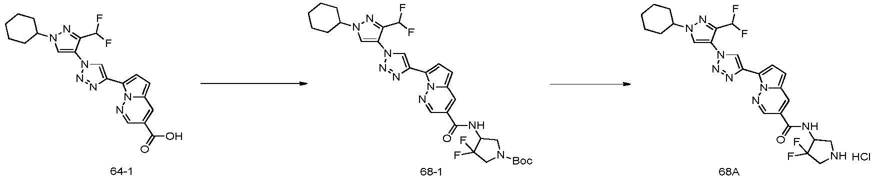

### 1) Step 1 Preparation of compound 68-1

Compound 64-1 (100mg, 0.23mmol), tert-butyl 4-amino-3,3-difluoropyrrolidine-1-carboxylate (62.4mg, 0.28mmol, Bide) were dissolved with dichloromethane (5mL), then 2-(7-azabenzotriazol-1-yl)-N,N,N',N'-tetramethyluronium hexafluorophosphate (HATU) (107mg, 0.28mmol) and triethylamine (0.10mL, 0.70mmol) were added. After reacting at room temperature with stirring for 2 h, the reaction solution, with the addition of 15mL of water, was extracted with ethyl acetate (15mL×3). The organic phases were combined, dried over anhydrous sodium sulfate, and filtered to remove the desiccant, and then the filtrate was concentrated under reduced pressure. The residue was directly used as a raw material in the next reaction without purification, as compound 68-1 (150mg). MS m/z(ESI): 632.4[M+1]+.

### 2) Step 2 Preparation of compound 68A

Compound 68-1 (130mg, 0.21mmol) was dissolved with hydrochloric acid (5mL, 4M in 1, 4-dioxane). After reacting at room temperature with stirring for 1 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 68A (50mg). MS m/z(ESI): 532.2[M+1]⁺. ¹H NMR (400 MHz, DMSO-d6) δ 9.79 (s, 2H), 9.16 (d, J = 8.4 Hz, 1H), 9.02 (s, 1H), 8.81 (dd, J = 17.8, 2.3 Hz, 2H), 8.75 (s, 1H), 7.70 (d, J = 4.6 Hz, 1H), 7.37 - 7.10 (m, 2H), 5.23 - 5.00 (m, 1H), 4.38 - 4.26 (m, 1H), 3.93 - 3.76 (m, 3H), 3.52 - 3.48 (m, 1H), 2.17 - 2.07 (m, 2H), 1.90 - 1.71 (m, 5H), 1.49 - 1.40 (m, 2H), 1.28 - 1.22 (m, 1H).

### Example 22

### Preparation of 4-(3-(1-(3-(difluoromethyl)-1-((2S)-2-methylpiperidin-4-yl)-1H-pyrazol-4-yl)-1,2,3-triazol-4-yl)pyrazolo[1,5-a]pyrimidin-5-yl) morpholine (79A)

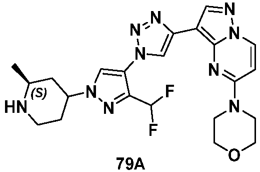

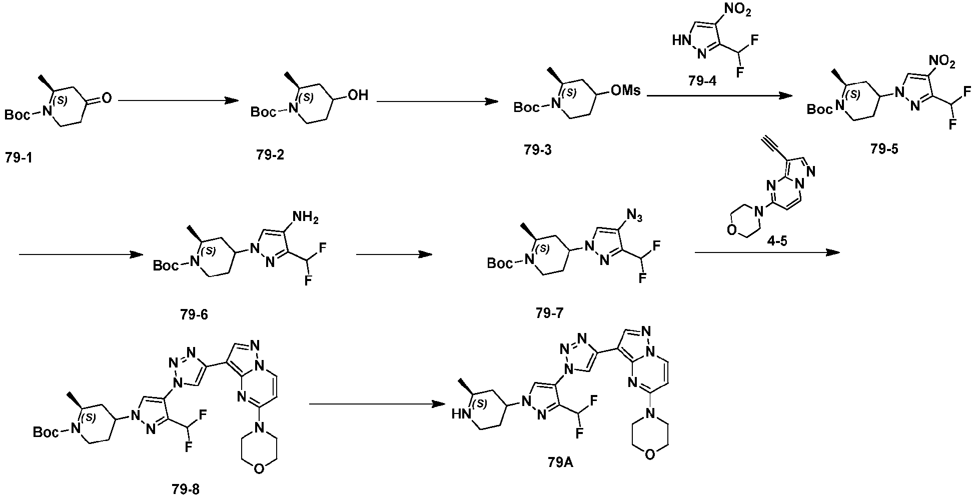

### 1) Step 1 Preparation of compound 79-2

At room temperature, to a single-necked bottle, compound 79-1 (9.09g, 42.6mmol, Leyan), then methanol (100mL) were added. The reaction system was cooled under ice-bath. Sodium borohydride (3.22 g, 85.2 mmol) was added in batches to the fast-stirring reaction solution. After completion of the addition, the reaction solution was naturally recovered to 25°C and continued to stir for 30 min. Saturated ammonium chloride solution (100 mL) was added dropwise to the reaction solution under ice-bath, followed by extraction with ethyl acetate (80 mL × 3). The ethyl acetate layer was collected, washed with saturated brine (50mL), dried over anhydrous sodium sulfate, filtered and concentrated to obtain compound 79-2 (9.54g). MS m/z(ESI): 160.2[M-55]⁺.

### 2) Step 2 Preparation of compound 79-3

At room temperature, to a single-necked bottle, compound 79-2 (9.54g, 42.1mmol), then dichloromethane (100mL) were added. The reaction system was cooled under ice-bath, and the atmosphere was replaced with nitrogen three times. Triethylamine (17.5mL, 126mmol) and methylsulfonyl chloride (3.91mL, 50.5mmol) were added in sequence. After completion of the addition, the reaction solution was naturally recovered to 25°C and continued to stir for 2 h. The reaction was quenched with saturated sodium bicarbonate solution (200mL), and the reaction solution was extracted with dichloromethane (150mL×3). The dichloromethane layer was collected, washed with saturated brine (50mL), dried over anhydrous sodium sulfate, filtered and concentrated to obtain compound 79-3 (12.5g). MS m/z(ESI): 238.0[M-55]⁺.

### 3) Step 3 Preparation of compound 79-5

At room temperature, to a single-necked bottle, compound 79-3 (5.00g, 8.52mmol), 79-4 (1.25g, 7.67mmol) and cesium carbonate (8.33g, 25.6mmol) were added, and then N,N-dimethylformamide (50mL) was added. After replacement of the atmosphere with nitrogen three times, the reaction solution was reacted at 80°C for 18 h. The reaction solution was poured into water (300mL), and extracted with ethyl acetate (100mL×3). The ethyl acetate layer was collected, washed with saturated brine (50mL), dried over anhydrous sodium sulfate, filtered and concentrated to obtain a crude product. The crude product was purified by silica gel column chromatography with an elution system (eluant: ethyl acetate/petroleum ether=0%-40%) to obtain compound 79-5 (1.30g). MS m/z(ESI): 305.2[M-55]⁺. ¹H NMR (400 MHz, DMSO-d6) δ 9.11 (d, J = 13.9 Hz, 1H), 7.46 - 7.17 (m, 1H), 4.90 - 4.14 (m, 2H), 4.02 - 3.62 (m, 2H), 2.15 - 2.06 (m, 2H), 1.49 - 1.31 (m, 11H), 1.19 - 1.17 (m, 2H), 1.00 (d, J = 6.6 Hz, 1H).

### 4) Step 4 Preparation of compound 79-6

At room temperature, to a single-necked bottle, compound 79-5 (430mg, 1.19mmol), Pd/C (100mg, 0.0900mmol) were added, then methanol (5mL) was added. After replacement of the atmosphere with hydrogen three times, the reaction solution was reacted at 25°C under hydrogen atmosphere for 18 h. The reaction solution was filtered and concentrated to obtain compound 79-6 (400mg). MS m/z(ESI): 275.2[M-55]⁺.

### 5) Step 5 Preparation of compound 79-7

At room temperature, to a single-necked bottle, compound 79-6 (400mg, 1.21mmol), then acetonitrile (5mL) were added. Under ice-bath, tert-butyl nitrite (0.220mL, 1.82mmol) was slowly added dropwise to the reaction solution, followed by stirring for 1 h. Then, azidotrimethylsilane (0.240mL, 1.82mmol) was slowly added dropwise to the reaction solution. Thereafter, the reaction solution was naturally recovered to 25°C and continued to stir for 1 h. The reaction solution was poured into water (30mL), and extracted with ethyl acetate (10mL×3). The ethyl acetate layer was collected, washed with saturated brine (10mL), dried over anhydrous sodium sulfate, filtered and concentrated to obtain a crude product as the title compound 79-7 (431mg). The crude product was directly used in the next reaction without purification. MS m/z(ESI): 301.2[M-55]⁺.

### 6) Step 6 Preparation of compound 79-8

At room temperature, to a single-necked bottle, compound 79-7 (431mg, 1.21mmol), compound 4-5 (304mg, 1.33mmol), blue vitriol (30.2mg, 0.120mmol) and sodium ascorbate (24.0mg, 0.120mmol) were added, then ethanol (6mL) and water (3mL) were added. After reacting at 25°C for 2 h, the reaction solution was poured into water (50mL), and extracted with ethyl acetate (30mL ×3). The ethyl acetate layer was collected, washed with saturated brine (30mL), dried over anhydrous sodium sulfate, filtered and concentrated to obtain a residue. The residue was purified by silica gel column chromatography with an elution system (eluant: ethyl acetate/petroleum ether=40%-100%) to obtain compound 79-8 (340mg). MS m/z(ESI): 585.4[M+1]⁺.

### 7) Step 7 Preparation of compound 79A

At room temperature, to a single-necked bottle, compound 79-8 (100mg, 0.170mmol), then dioxane (2mL) were added, and, under ice-bath, hydrochloric acid dioxane solution (2mL) at a concentration of 4.00mol/L was added dropwise. After reacting at 25°C for 1.5 h, the reaction solution was concentrated, and, with the addition of water (1mL), was neutralized with ammonia aqueous (1mL), to precipitate out a solid, followed by suction filtration to obtain filter cake as crude product. The crude product was purified by HPLC (Gilson_306_1741, chromatographic column: Waters-Xbridge-C18-10µm-19*250mm; mobile phase: water (containing 10.0mmol/L ammonium bicarbonate) and acetonitrile, gradient ratio: acetonitrile 19%-95%, flow rate: 25mL/min) to obtain compound 79A (53.1mg). MS m/z(ESI): 485.2[M+1]⁺. ¹H NMR (400 MHz, DMSO-d6) δ 8.76 (d, J = 7.9 Hz, 1H), 8.71 - 8.65 (m, 1H), 8.53 (d, J = 6.3 Hz, 1H), 8.38 (d, J = 1.2 Hz, 1H), 7.20 (td, J = 53.2, 5.5 Hz, 1H), 6.81 (d, J = 7.9 Hz, 1H), 4.68 - 4.59 (m, 1H), 4.43 - 4.31 (m, 1H), 3.72 (s, 8H), 3.09 - 2.98 (m, 1H), 2.86 - 2.63 (m, 2H), 2.24 - 1.89 (m, 3H), 1.83 - 1.65 (m, 1H), 1.08 - 1.04 (m, 3H).

### Example 23

### Preparation of 4-(3-(3-difluoromethyl)-1-(2R)-2-methylpiperidin-4-yl)-1H-pyrazol-4-yl)-1H-1,2,3-triazol-4-yl)pyrazolo[1, 5-a]pyrimidin-5-yl) morpholine hydrochloride (80A)

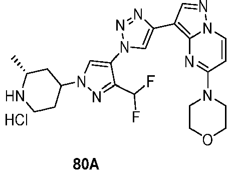

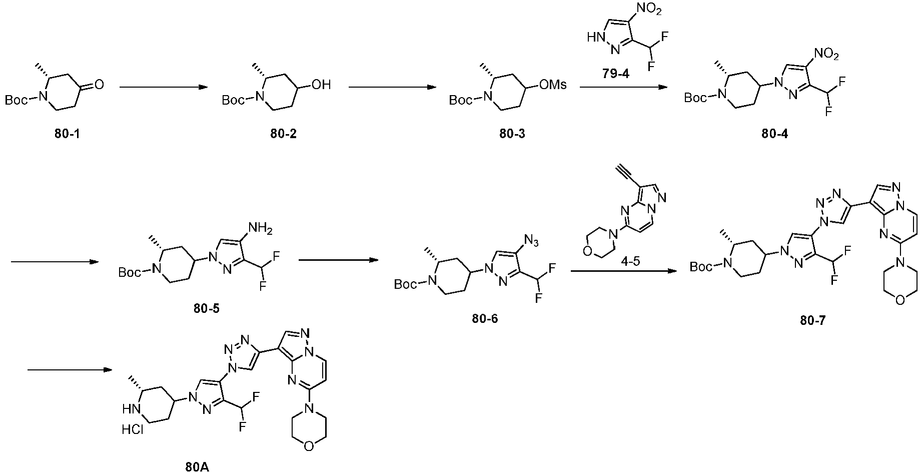

### 1) Step 1 Preparation of compound 80-2

Compound 80-1 (3.00 g, 14.1 mmol) was dissolved with methanol (30 mL), to which sodium borohydride (0.55 g, 14.5 mmol) was added. After stirring at 25°C for 2 h, the reaction solution was poured into water (30mL). The aqueous phase was extracted with ethyl acetate (30mL x 3). The combined organic phases were washed with saturated brine (50mL), dried over anhydrous sodium sulfate and filtered. The filtrate was concentrated under reduced pressure. The residue was purified by silica gel column chromatography with an elution system (dichloromethane/methanol=20/1 to 10/1) to obtain compound 80-2 (2.80 g). MS m/z: 160.0[M-55]⁺.

### 2) Step 2 Preparation of compound 80-3

Compound 80-2 (2.80 g, 13.0 mmol) was dissolved with dichloromethane (40 mL), to which triethylamine (4.00 g, 39.5 mmol) and methylsulfonyl chloride (1.80 g, 15.7 mmol) were added in sequence. After stirring at 0°C for 1 h, the reaction solution was poured into water (30 mL). The aqueous phase was extracted with dichloromethane (30 mL × 3). The combined organic phases were washed with saturated brine (80 mL), dried over anhydrous sodium sulfate and filtered. The filtrate was concentrated under reduced pressure to obtain compound 80-3 (3.20 g).

### 3) Step 3 Preparation of compound 80-4

Compound 79-4 (1.70 g, 10.4 mmol) and compound 80-3 (3.20 g, 20.2 mmol) were dissolved with N,N-dimethylformamide (80 mL), then cesium carbonate (10.5 g, 32.2 mmol) was added. After stirring at 80°C for 18 h, the reaction solution was poured into water (100 mL). The aqueous phase was extracted with ethyl acetate (80 mL×3). The combined organic phases were washed with saturated brine (80 mL), dried over anhydrous sodium sulfate and filtered. The filtrate was concentrated under reduced pressure. The residue was purified by silica gel column chromatography with an elution system (petroleum ether/ethyl acetate =5/1 to 3/1) to obtain compound 80-4 (1.10 g). MS m/z(ESI): 305.0[M-55]⁺.

### 4) Step 4 Preparation of compound 80-5

Compound 80-4 (1.10 g, 3.05 mmol) was dissolved with methanol (10 mL), then wet Pd/C (150 mg, 10%) was added. After replacement of the atmosphere with hydrogen three times, the reaction solution was stirred at 25°C for 2 h. The reaction solution was filtered, and the filtrate was concentrated under reduced pressure to obtain compound 80-5 (950 mg). MS m/z(ESI): 275. 1[M-55]⁺.

### 5) Step 5 Preparation of compound 80-6

Compound 80-5 (900 mg, 2.72 mmol) was dissolved with acetonitrile (20 mL), and, at 0°C, isopentyl nitrite (500 mg, 4.27 mmol, Bide) was added. After stirring the reaction solution for 30 min, azidotrimethylsilane (470 mg, 4.08 mmol) was added. After stirring at 25°C for 18 h, the reaction solution was poured into water (30 mL). The aqueous phase was extracted with ethyl acetate (30 mL×3). The combined organic phases were washed with saturated brine (50 mL), dried over anhydrous sodium sulfate and filtered. The filtrate was concentrated under reduced pressure to obtain compound 80-6 (900 mg). MS m/z(ESI): 301.0[M-55]⁺.

### 6) Step 6 Preparation of compound 80-7

Compound 80-6 (900 mg, 2.53 mmol) was dissolved with ethanol (20 mL) and water (5 mL), to which sodium ascorbate (50 mg, 0.25 mmol, Energy), blue vitriol (65 mg, 0.26 mmol, Energy) and compound 4-5(650 mg, 2.85 mmol) were added in sequence. After stirring at 25°C for 18 h, the reaction solution was poured into water (50 mL). The aqueous phase was extracted with ethyl acetate (30 mL × 3). The combined organic phases were washed with saturated brine (50 mL), dried over anhydrous sodium sulfate and filtered. The filtrate was concentrated under reduced pressure. The residue was purified by silica gel column chromatography with an elution system (petroleum ether/ethyl acetate =1/1 to 0/1) to obtain compound 80-7 (520 mg). MS m/z(ESI): 585.4[M+H]⁺.

### 7) Step 7 Preparation of compound 80A

Compound 80-7 (80 mg, 0.14 mmol) was dissolved with ethyl acetate (3 mL), to which hydrochloric acid ethyl acetate solution (1mL, 4M) was added. After stirring at 25°C for 2 h, the reaction solution was concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 30%-70%, flow rate: 30mL/min) to obtain the title compound 80A (20.0 mg). MS m/z(ESI): 485.2[M+H]⁺. ¹H NMR (400 MHz, DMSO-d6): δ 9.59 - 8.95 (m, 2H), 8.84 - 8.66 (m, 2H), 8.56 (d, J = 4.2 Hz, 1H), 8.39 (d, J = 1.4 Hz, 1H), 7.39 - 7.10 (m, 1H), 6.82 (d, J = 7.9 Hz, 1H), 4.86 - 4.69 (m, 1H), 3.73 (s, 8H), 3.56 - 3.05 (m, 3H), 2.46 - 1.98 (m, 4H), 1.34 (d, J = 6.5 Hz, 3H).

### Example 24

### Preparation of 4-(3-(3-difluoromethyl)-1-(2R, 4S)-2-methylpiperidin-4-yl)-1H-pyrazol-4-yl)-1H-1, 2, 3-triazol-4-yl)pyrazolo[1, 5-a]pyrimidin-5-yl) morpholine Isomer 1 (72A) and 4-(3-(3-difluoromethyl)-1-(2R, 4R)-2-methylpiperidin-4-yl)-1H-pyrazol-4-yl)-1H-1,2,3-triazol-4-yl)pyrazolo[1,5-a]pyrimidin-5-yl) morpholine Isomer 2 (73A)

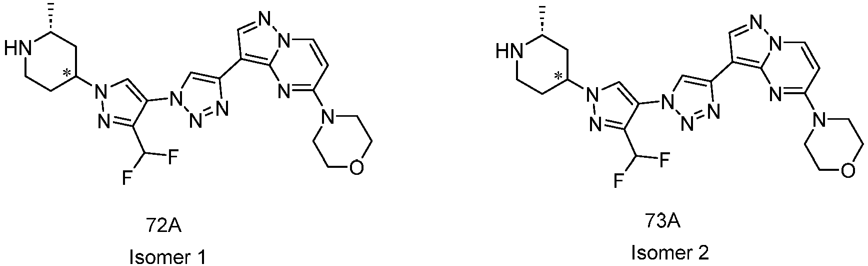

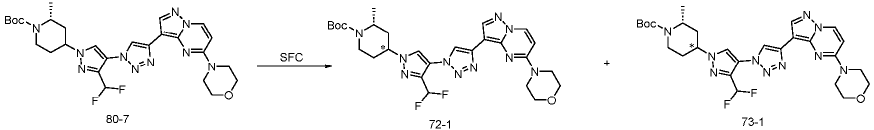

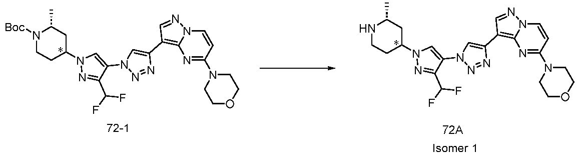

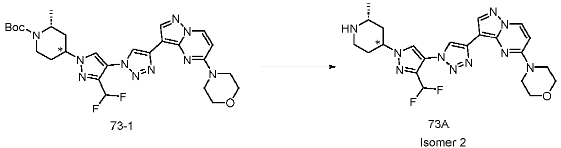

### 1) Step 1 Preparation of compounds 72-1 and 73-1

Compound 80-7 (320 mg, 0.55 mmol) was subjected to chiral resolution (WATERS 150 preparative SFC(SFC-26), chromatographic column: Chiral Cel OX, 250×30mm, ID, 10µm; mobile phase: carbon dioxide and ethanol, gradient ratio: ethanol 30-50%, flow rate: 150mL/min) to obtain compound 72-1 (120mg, retention time: 3.345 min)) and compound 73-1 (160 mg, retention time: 3.661 min). MS m/z: 585.3[M+H]⁺.

### 2) Step 2 Preparation of compound 72A

Compound 72-1 (120 mg, 0.210 mmol) was dissolved with dichloromethane (3 mL), to which trifluoroacetic acid (1 mL) was added. After stirring at 25°C for 2 h, the reaction solution was concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 30%-70%, flow rate: 30mL/min) to obtain compound 72A (45 mg). MS m/z(ESI): 485.3[M+H]+. ¹H NMR (400 MHz, DMSO-d6) δ 8.77 (d, J = 7.9 Hz, 1H), 8.66 (s, 1H), 8.54 (s, 1H), 8.38 (s, 1H), 7.20 (t, J = 53.2 Hz, 1H), 6.82 (d, J = 7.9 Hz, 1H), 4.42 - 4.32 (m, 1H), 3.73 (s, 8H), 3.10 - 3.02 (m, 1H), 2.72 - 2.61 (m, 2H), 2.10 - 1.96 (m, 3H), 1.82 - 1.71 (m, 1H), 1.49 (q, J = 11.6 Hz, 1H), 1.06 (d, J = 6.2 Hz, 3H).

### 3) Step 3 Preparation of compound 73A

Compound 73-1(140 mg, 0.241 mmol) was dissolved with dichloromethane (3 mL), to which trifluoroacetic acid (1 mL) was added. After stirring at 25°C for 2 h, the reaction solution was concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 30%-70%, flow rate: 30mL/min) to obtain compound 73A (55 mg). MS m/z(ESI): 485.3[M+H]⁺. ¹H NMR (400 MHz, DMSO-d6): δ 8.77 (d, J = 7.9 Hz, 1H), 8.72 (s, 1H), 8.55 (s, 1H), 8.39 (s, 1H), 7.21 (t, J = 53.2 Hz, 1H), 6.82 (d, J = 7.9 Hz, 1H), 4.71 - 4.60 (m, 1H), 3.73 (s, 8H), 3.04 - 2.97 (m, 1H), 2.87 - 2.74 (m, 2H), 2.25 - 2.08 (m, 2H), 1.97 - 1.89 (m, 1H), 1.75 - 1.62 (m, 1H), 1.06 (d, J = 6.5 Hz, 3H).

### Example 25

### Preparation of (S)-4-(3-(1-(1-cyclohexyl-3-(difluoromethyl)-1H-pyrazol-4-yl)-1H-1,2,3-triazol-4-yl]pyrazolo[1,5-a]pyrimidin-5-yl)-3-methylmorpholine (82A)

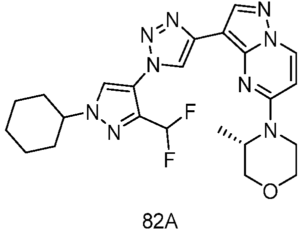

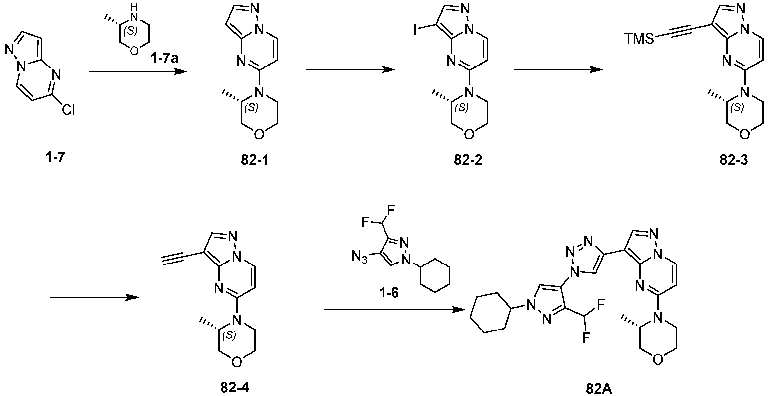

### 1) Step 1 Preparation of compound 82-1

Compound 1-7 (1.00g, 6.51mmol, Bide) was dissolved with acetonitrile (40mL), to which 1-7a (990mg, 9.77mmol, Bide), N,N-diisopropylethylamine (4.32mL, 26.1mmol) were added. After reacting at 60°C with stirring for 2 h, the reaction solution, with the addition of water (40ml), was extracted with ethyl acetate (40mL×3). The organic phases were combined, dried over anhydrous sodium sulfate, and filtered to remove the desiccant, and then the filtrate was concentrated under reduced pressure. The residue was purified by silica gel column chromatography with an elution system (dichloromethane/methanol=100/1 to 30/1) to obtain compound 82-1 (642mg). MS m/z(ESI): 219.2[M+1]⁺.

### 2) Step 2 Preparation of compound 82-2

Compound 82-1 (642mg, 2.94mmol) was dissolved with dichloromethane (30mL), and at 0°C, N-iodosuccinimide (728mg, 3.24mmol) was added. After stirring at 0°C for 2 h, the reaction was quenched with saturated sodium bicarbonate solution (40ml), and the reaction solution was extracted with dichloromethane (25mL×3). The organic phases were combined, dried over anhydrous sodium sulfate, and filtered to remove the desiccant, and then the filtrate was concentrated under reduced pressure. The residue was purified by silica gel column chromatography with an elution system (petroleum ether/ethyl acetate =100/1 to 5/1) to obtain compound 82-2 (700mg). MS m/z(ESI): 345. 1[M+1]⁺.

### 3) Step 3 Preparation of compound 82-3

Compound 82-2 (1.10g, 0.09mmol) was dissolved with tetrahydrofuran (10mL), to which cuprous iodide (600mg, 0.32mmol) and bis(triphenylphosphine) palladium dichloride (220mg, 0.32mmol) and triethylamine (0.89mL, 6.39mmol) were added in sequence. After replacement of the atmosphere with nitrogen three times, ethynyltrimethylsilane (4.55mL, 31.96mmol) was added to the reaction solution. After reacting at room temperature with stirring for 12 h, the reaction solution, with the addition of water (40ml), was extracted with ethyl acetate (30mL×3). The organic phases were combined, dried over anhydrous sodium sulfate, and filtered to remove the desiccant, and then the filtrate was concentrated under reduced pressure. The residue was purified by silica gel column chromatography with an elution system (petroleum ether/ethyl acetate =100/1 to 20/1) to obtain compound 82-3 (700mg). MS m/z(ESI): 315.6[M+1]⁺.

### 4) Step 4 Preparation of compound 82-4

Compound 82-3 (650mg, 2.07mmol) was dissolved with methanol (30mL), to which potassium carbonate (571mg, 4.13mmol) was added. After reacting at room temperature with stirring for 1 h, the reaction solution was directly concentrated under reduced pressure. The residue was purified by silica gel column chromatography with an elution system (petroleum ether/ethyl acetate =100/1 to 1/1) to obtain compound 82-4 (320mg). MS m/z(ESI): 243.1[M+1]⁺.

### 5) Step 5 Preparation of compound 82A

Compound 82-4 (200mg, 0.83mmol), 1-6 (200mg, 0.83mmol) were dissolved with a mixed solvent of water (4mL) and ethanol (4mL), to which blue vitriol (20.6mg, 0.08mmol), sodium ascorbate (16.4mg, 0.08mmol) were added. After reacting at room temperature with stirring for 12 h, the reaction solution was concentrated under reduced pressure to remove ethanol. The reaction solution, with the addition of water (15ml), was extracted with ethyl acetate (15mL×3). The organic phases were combined, dried over anhydrous sodium sulfate, and filtered to remove the desiccant, and then the filtrate was concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 82A (75mg). MS m/z(ESI): 484.5[M+1]⁺. ¹H NMR (400 MHz, CDCl3) δ 8.62 - 8.57 (m, 2H), 8.45 (d, J = 7.7 Hz, 1H), 8.11 (s, 1H), 6.82 (t, J = 53.7 Hz, 1H), 6.37 (d, J = 7.3 Hz, 1H), 4.45 (d, J = 7.3 Hz, 1H), 4.21 - 4.03 (m, 3H), 3.88 - 3.73 (m, 2H), 3.61 - 3.58 (m, 1H), 3.49 - 3.40 (m, 1H), 2.27 - 2.18 (m, 2H), 1.98 - 1.90 (m, 2H), 1.80 - 1.71 (m, 3H), 1.50 - 1.39 (m, 2H), 1.38 (d, J = 6.8 Hz, 3H), 1.36 - 1.25 (m, 1H).

### Example 26

### Preparation of (2S)-4-(3-{1-[1-cyclohexyl-3-(difluoromethyl)-1H-pyrazol-4-yl]-1H-1,2,3-triazol-4-yl}pyrazolo[1,5-a]pyrimidin-5-yl)-2-methylmorpholine (83A)

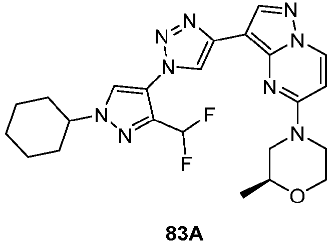

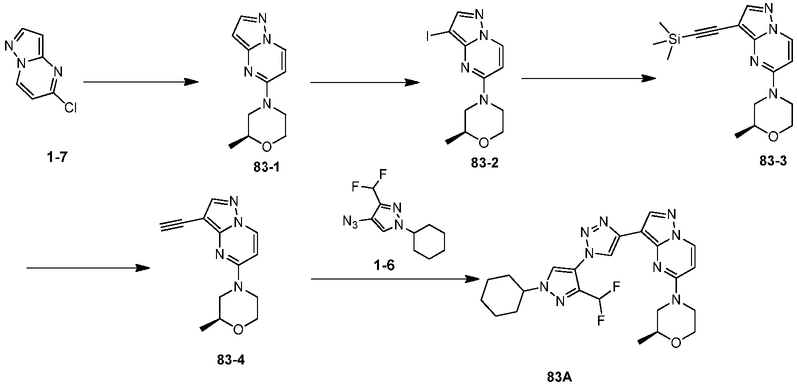

### 1) Step 1 Preparation of compound 83-1

At room temperature, to a single-necked bottle, compound 1-7 (1.00g, 6.51mmol, Shanghai Bide), N,N-dimethylformamide (8mL) were added in sequence, and, after uniformly mixing, (2S)-2-methylmorpholine hydrochloride (93.7mg, 680umol, Shanghai Haohong) and cesium carbonate (3.18g, 9.70mmol) were added. After stirring at 100°C for 16 h, the reaction solution, with the addition of 20mL of water, was extracted with 50mL of ethyl acetate twice. The organic phase was dried over anhydrous sodium sulfate, filtered and concentrated under reduced pressure. The residue was purified by silica gel column chromatography with an elution system (ethyl acetate/petroleum ether=0~3/2) to obtain compound 83-1(1.15g). ¹H NMR (400 MHz, DMSO-d6) δ 8.67 (d, J = 7.9 Hz, 1H), 7.86 (d, J = 2.0 Hz, 1H), 6.72 (d, J = 7.9 Hz, 1H), 6.04 (d, J = 1.9 Hz, 1H), 4.28 - 4.25 (m, 1H), 4.20 - 4.16 (m, 1H), 3.92 - 3.88 (m, 1H), 3.58 - 3.47 (m, 2H), 2.98 - 2.94 (m, 1H), 2.65 - 2.59 (m, 1H), 1.16 (d, J = 6.1 Hz, 3H)

### 2) Step 2 Preparation of compound 83-2

At room temperature, to a single-necked bottle, compound 83-1 (1.05g, 4.81mmol), acetonitrile (10mL) were added in sequence, and stirred to dissolve, then a solution of N-iodosuccinimide (1.62g, 7.22mmol) previously dissolved in 10mL of acetonitrile was added. After stirring at 25°C for 6 h, the reaction solution, with the addition of 20mL of saturated sodium sulfite aqueous solution, was extracted with 50mL of ethyl acetate twice. The organic phase was dried over anhydrous sodium sulfate, filtered and concentrated under reduced pressure. The residue was purified by silica gel column chromatography with an elution system (ethyl acetate/petroleum ether=0~3/2) to obtain compound 83-2 (1.58g). MS m/z(ESI): 345.2[M+1]⁺

### 3) Step 3 Preparation of compound 83-3

At room temperature, to a single-necked bottle, compound 83-2 (1.58g, 4.59mmol), tetrahydrofuran (12mL) were added in sequence, and stirred to dissolve, and then cuprous iodide (90.0mg, 460umol), bis(triphenylphosphine) palladium dichloride (320mg, 46umol) were added, followed by the addition of triethylamine (1.91mL, 13.8mmol) and ethynyltrimethylsilane(1.31mL, 9.18mmol). Nitrogen was introduced into the reaction solution for about 1 min to remove air as much as possible, then the bottle cap was quickly tightened. After stirring at 25°C for 6 h, the reaction solution, with the addition of 10mL of water, was extracted with 20mL of ethyl acetate twice. The organic phase was dried over anhydrous sodium sulfate, filtered and concentrated under reduced pressure. The residue was purified by silica gel column chromatography with an elution system (ethyl acetate/petroleum ether=1/4~4/1) to obtain compound 83-3 (830mg). MS m/z(ESI): 315.2[M+1]⁺

### 4) Step 4 Preparation of compound 83-4

At room temperature, to a single-necked bottle, compound 83-3 (770mg, 2.45mmol), methanol (10mL) were added in sequence, and stirred to dissolve, and then potassium carbonate (677mg, 4.90mmol) was added. After stirring at 25°C for 1 h, the reaction solution, with the addition of 20mL of water, was extracted with 50mL of ethyl acetate twice. The organic phase was dried over anhydrous sodium sulfate, filtered and concentrated under reduced pressure to obtain compound 83-4 (547mg).

MS m/z(ESI): 243.3[M+1]⁺

### 5) Step 5 Preparation of compound 83

At room temperature, to a single-necked bottle, compound 83-4 (150mg, 620umol), tert-butanol (1.2mL) and water (1.2mL) were added in sequence, and, after uniformly mixing, sodium ascorbate (12.3mg, 60.0umol), cuprous oxide (39.4mg, 500umol) were added, followed by the addition of compound 1-6 (149mg, 620umol). After stirring at 80°C for 2 h, the reaction solution, with the addition of 10mL of water, was extracted with 20mL of ethyl acetate twice. The organic phase was dried over anhydrous sodium sulfate, filtered and concentrated under reduced pressure. The residue was purified by HPLC (CAS-CD-SEMI-PREP-S, chromatographic column: Phenomenex luna C18 150*25mm* 10um; mobile phase: water (containing formic acid) and acetonitrile, gradient ratio: acetonitrile 50%-80%, flow rate: 25mL/min) to obtain compound 83 (88.2mg). MS m/z(ESI): 484.4[M+1]⁺. ¹H NMR (400 MHz, DMSO-d6) δ 8.75 (d, J = 7.9 Hz, 1H), 8.66 (s, 1H), 8.53 (s, 1H), 8.37 (s, 1H), 7.22 (t, J = 53.2 Hz, 1H), 6.82 (d, J = 7.9 Hz, 1H), 4.44 - 4.23 (m, 3H), 3.95 - 3.91 (m, 1H), 3.63 - 3.51 (m, 2H), 3.05 - 3.02 (m, 1H), 2.75 - 2.71 (m, 1H), 2.11 - 2.07 (m, 2H), 1.88 - 1.71 (m, 4H), 1.70 - 1.60 (m, 1H), 1.49 - 1.35 (m, 2H), 1.30 - 1.14 (m, 4H)

### Example 27

### Preparation of 7-(1-(3-(difluoromethyl)-1-(piperidin-4-yl)-1H-pyrazol-4-yl)~1H-1,2,3-triazol-4-yl)pyrrolo[1,2-b]pyridazine-3-formamide (84A)

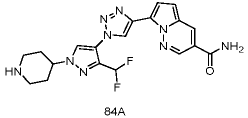

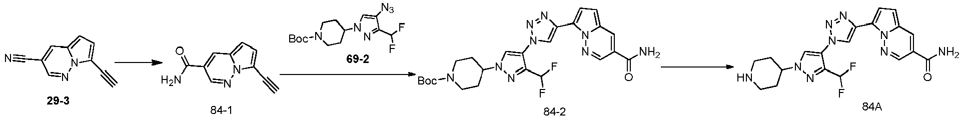

### 1) Step 1 Preparation of compound 84-1

Compound 29-3 (300 mg, 1.79 mmol) was dissolved with dimethyl sulfoxide (0.6 mL) and ethanol (3 mL), and, at 0°C, sodium hydroxide (86.0 mg, 2.15 mmol), hydrogen peroxide (0.2 mL, 30% aqueous solution) were added. After reacting at 0°C with stirring for 30 min, the reaction solution, with the addition of water (15 mL), was extracted with ethyl acetate (10 mL×3). The combined organic phases were dried over anhydrous sodium sulfate, and filtered to remove the desiccant, and then the filtrate was concentrated under reduced pressure. The residue was purified by silica gel column chromatography with an elution system (dichloromethane/methanol=100/1 to 20/1) to obtain compound 84-1 (254 mg). MS m/z(ESI): 186.1[M+1]⁺.

### 2) Step 2 Preparation of compound 84-2

Compound 84-1 (200mg, 1.08mmol), 69-2 (555mg, 1.62mmol) were dissolved with a mixed solvent of water (4mL) and ethanol (4mL), to which blue vitriol (27.0mg, 0.11mmol), sodium ascorbate (21.4mg, 0.11mmol) were added. After reacting at room temperature with stirring for 12 h, the reaction solution was concentrated under reduced pressure to remove ethanol. The reaction solution, with the addition of water (30mL), was extracted with ethyl acetate (15mL×3). The organic phases were combined, dried over anhydrous sodium sulfate, and filtered to remove the desiccant, and then the filtrate was concentrated under reduced pressure. The residue was purified by silica gel column chromatography with an elution system (dichloromethane/methanol=100/1 to 20/1) to obtain compound 84-2 (435mg). MS m/z(ESI): 528.3[M+1]⁺.

### 3) Step 5 Preparation of compound 84A

Compound 84-2 (100mg, 0.19mmol) was dissolved with dichloromethane (3mL), to which hydrochloric acid (3mL, 4M in 1, 4-dioxane) was added. After reacting at room temperature with stirring for 1 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain the title compound 84A (70mg). MS m/z(ESI): 428.2[M+1]⁺. ¹H NMR (400 MHz, DMSO-d6) δ 9.07 (s, 1H), 9.03 (s, 1H), 8.80 (s, 2H), 8.67 (d, J = 2.2 Hz, 1H), 8.17 (s, 1H), 7.66 (d, J = 4.6 Hz, 1H), 7.56 (s, 1H), 7.27 (t, J = 53.2 Hz, 1H), 7.05 (d, J = 4.6 Hz, 1H), 4.77 - 4.61 (m, 1H), 3.44 (d, J = 12.8 Hz, 2H), 3.12 (d, J = 11.8 Hz, 2H), 2.36 - 2.32 (m, 2H), 2.27 - 2.17 (m, 2H).

### Example 28

### Preparation of 4-(3-(1-(3-(difluoromethyl)-1-(2,2-dimethylpiperidin-4-yl)-1H-pyrazol-4-yl)-1,2,3-triazol-4-yl]pyrazolo[1,5-a]pyrimidin-5-yl) morpholine (85A)

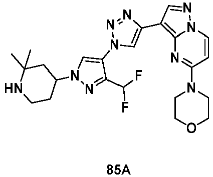

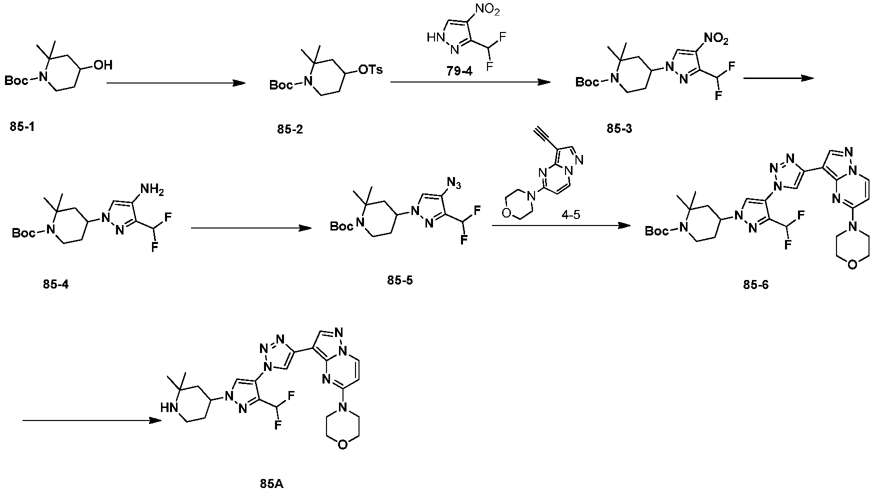

### 1) Step 1 Preparation of compound 85-2

At room temperature, to a single-necked bottle, compound 85-1 (1.88g, 8.20mmol) and 4-dimethylaminopyridine (200 mg, 1.64 mmol) were added, then dichloromethane (20mL) was added. The reaction system was cooled under ice-bath, and the atmosphere was replaced with nitrogen three times. Triethylamine (2.27mL, 16.4mmol) and p-toluene sulfonyl chloride (2.34g, 12.3mmol) were added in sequence. After completion of the addition, the reaction solution was reacted at 40°C for 18 h. Dichloromethane (200mL) was added to the reaction system, and the dichloromethane layer was washed with water (50mL×3). The dichloromethane layer was collected, washed with saturated brine (50mL), dried over anhydrous sodium sulfate, filtered and concentrated to obtain compound 85-2 (3.07g). MS m/z(ESI): 406.2[M+23]⁺.

### 2) Step 2 Preparation of compound 85-3

At room temperature, to a single-necked bottle, compound 85-2 (3.07g, 8.01mmol), 79-4 (1.31g, 8.01mmol) and cesium carbonate (7.82g, 24.0mmol) were added, then DMF (31mL) was added. After replacement of the atmosphere with nitrogen three times, the reaction solution was reacted at 80°C for 18 h. The reaction solution was poured into water (300mL), and extracted with ethyl acetate (100mL ×3). The ethyl acetate layer was collected, washed with saturated brine (50mL), dried over anhydrous sodium sulfate, filtered and concentrated to obtain a crude product. The crude product was purified by silica gel column chromatography with an elution system (eluant: ethyl acetate/petroleum ether=0%-40%) to obtain the title compound 85-3 (830mg). MS m/z(ESI): 319.2[M-55]⁺. ¹H NMR (400 MHz, DMSO-d6) δ 9.10 (s, 1H), 7.31 (t, J = 52.9 Hz, 1H), 4.75 - 4.62 (m, 1H), 3.92 - 3.80 (m, 1H), 3.26 - 3.20 (m, 1H), 2.23 - 2.12 (m, 1H), 2.05 (t, J = 12.7 Hz, 1H), 1.97 - 1.84 (m, 2H), 1.50 (s, 3H), 1.41 (s, 9H), 1.34 (s, 3H).

### 3) Step 3 Preparation of compound 85-4

At room temperature, to a single-necked bottle, compound 85-3 (830mg, 2.22mmol), Pd/C (165mg) were added, then methanol (8mL) was added. After replacement of the atmosphere with hydrogen three times, the reaction solution was reacted at 25°C under hydrogen atmosphere for 18 h. The reaction solution was filtered and concentrated to obtain the title compound 85-4 (690mg). MS m/z(ESI): 289.2[M-55] ⁺.

### 4) Step 4 Preparation of compound 85-5

At room temperature, to a single-necked bottle, compound 85-4 (200mg, 0.580mmol), then acetonitrile (2mL) were added. Under ice-bath, tert-butyl nitrite (0.100mL, 0.870mmol) was slowly added dropwise to the reaction solution, followed by stirring for 1 h. Then, azidotrimethylsilane (0.110mL, 0.870mmol) was slowly added dropwise to the reaction solution. Thereafter, the reaction solution was naturally recovered to room temperature and continued to stir for 1 h. The reaction solution was poured into water (30mL), and extracted with ethyl acetate (10mL×3). The ethyl acetate layer was collected, washed with saturated brine (10mL), dried over anhydrous sodium sulfate, filtered and concentrated to obtain crude product as compound 85-5 (215mg). The crude product was directly used in the next reaction without purification. MS m/z(ESI): 315.2[M-55] ⁺.

### 5) Step 5 Preparation of compound 85-6

At room temperature, to a single-necked bottle, compound 85-5 (215mg, 0.580mmol), 4-5 (146mg, 0.640mmol), blue vitriol (14.5mg, 0.0600mmol) and sodium ascorbate (11.5mg, 0.060 mmol) were added, then ethanol (3mL) and water (1.5mL) were added. After reacting at 25°C for 1.5 h, the reaction solution was poured into water (50mL), and extracted with ethyl acetate (30mL ×3). The ethyl acetate layer was collected, washed with saturated brine (30mL), dried over anhydrous sodium sulfate, filtered and concentrated to obtain a crude product. The crude product was purified by silica gel column chromatography with an elution system (eluant: ethyl acetate/petroleum ether=1/3-1/1) to obtain the title compound 85-6 (145mg). MS m/z(ESI): 599.4[M+1] ⁺.

### 6) Step 6 Preparation of compound 85A

At room temperature, to a single-necked bottle, compound 85-6 (40mg, 0.0700mmol), then dioxane (0.5mL) were added. Under ice-bath, hydrochloric acid dioxane solution (0.5mL) at a concentration of 4.00mol/L was added dropwise. After reacting at 25°C for 1.5 h, the reaction solution was concentrated, and, with the addition of methanol (1mL), was neutralized with ammonia aqueous (1mL), followed by purification to obtain compound 85A (12.7mg). MS m/z(ESI): 499.2[M+1]⁺. ¹H NMR (400 MHz, DMSO-d6) δ 8.76 (d, J = 7.9 Hz, 1H), 8.65 (s, 1H), 8.52 (s, 1H), 8.38 (s, 1H), 7.19 (t, J = 53.2 Hz, 1H), 6.81 (d, J = 7.9 Hz, 1H), 4.64 - 4.52 (m, 1H), 3.72 (s, 8H), 2.93 - 2.78 (m, 2H), 2.02 - 1.88 (m, 2H), 1.81 - 1.60 (m, 3H), 1.13 (s, 3H), 1.10 (s, 3H).

### Example 29

### Preparation of (R)-4-(3-(1-(3-(difluoromethyl)-1-(2,2-dimethylpiperidin-4-yl)-1H-pyrazol-4-yl)-1,2,3-triazol-4-yl]pyrazolo[1,5-a]pyrimidin-5-yl) morpholine Isomer 1 hydrochloride (86A) & (S)-4-(3-(1-(3-(difluoromethyl)-1-(2,2-dimethylpiperidin-4-yl)-1H-pyrazol-4-yl)-1,2,3-triazol-4-yl]pyrazolo[1,5-a]pyrimidin-5-yl) morpholine Isomer 2 hydrochloride (87A)

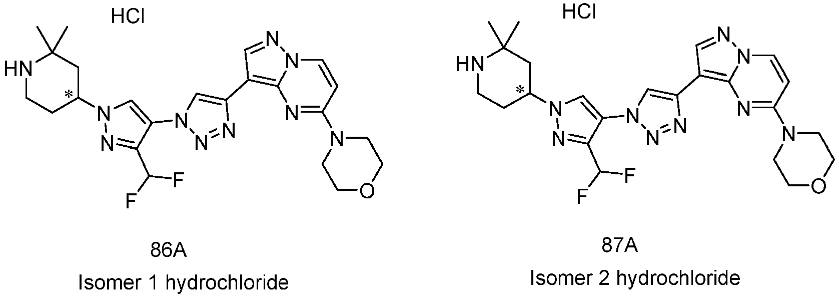

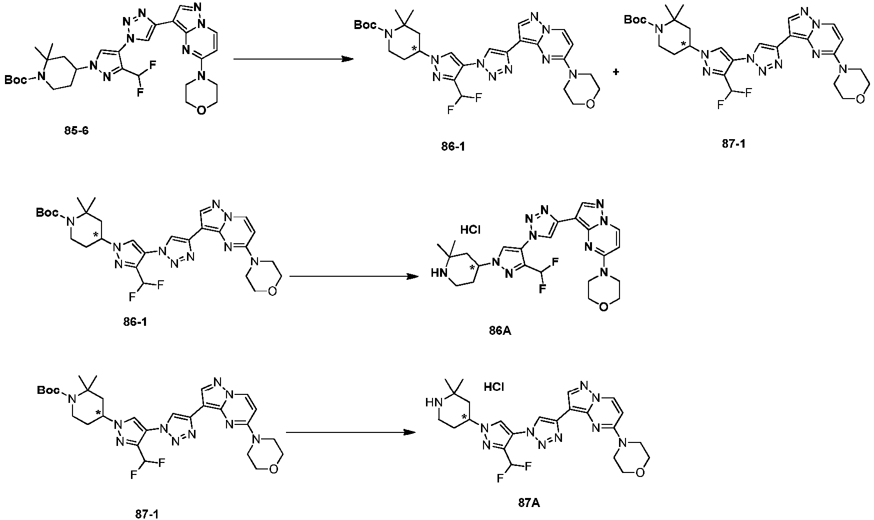

### 1) Step 1 Preparation of compounds 86-1 and 87-1

Compound 85-6 (450mg, 0.750mmol) was subjected to chiral resolution (WATERS 150 preparative SFC(SFC-26), chromatographic column: ChiralCel OX, 250×30mm I.D., 10µm; mobile phase: carbon dioxide and ethanol, gradient ratio: ethanol 40%, flow rate: 150mL/min) to obtain compound 86-1 (200mg, retention time: 3.920 min) and compound 87-1(191mg, retention time: 4.946 min). MS m/z(ESI): 599.3[M+1]⁺.

### 2) Step 2 Preparation of compound 86A

At room temperature, to a single-necked bottle, compound 86-1 (200mg, 0.330mmol), then dichloromethane (2mL) were added, to which hydrochloric acid ethyl acetate solution (4mL) at a concentration of 2.00mol/L was added. After reacting at 25°C for 3 h, the reaction solution was concentrated to obtain the title compound 86A (179mg). MS m/z(ESI): 499.2[M+1]⁺. ¹H NMR (400 MHz, DMSO-d6) δ 9.48 (s, 1H), 9.20 (s, 1H), 8.76 (d, J = 7.9 Hz, 1H), 8.69 (s, 1H), 8.55 (s, 1H), 8.38 (s, 1H), 6.82 (d, J = 7.9 Hz, 1H), 4.90 - 4.79 (m, 1H), 3.72 (s, 8H), 3.23 (d, J = 12.3 Hz, 2H), 2.35 - 2.06 (m, 4H), 1.43 (d, J = 4.3 Hz, 6H).

### 3) Step 3 Preparation of compound 87A

Compound 87-1 (191mg, 0.320mmol) was dissolved with dichloromethane (4mL), to which a solution of hydrogen chloride in dioxane (4mL, 4.0M) was slowly added dropwise. After stirring at 25°C for 2 h, the reaction solution was concentrated to obtain the title compound 87A (169mg). MS m/z(ESI): 499.4[M+1]⁺. ¹H NMR (400 MHz, DMSO-d6) δ 9.59 (d, J = 11.2 Hz, 1H), 9.32 (t, J = 10.4 Hz, 1H), 8.77 (d, J = 8.0 Hz, 1H), 8.69 (s, 1H), 8.55 (s, 1H), 8.38 (s, 1H), 7.24 (t, J = 53.2 Hz, 1H), 6.82 (d, J = 8.0 Hz, 1H), 4.92-4.80 (m, 1H), 3.72 (s, 8H), 3.37-3.13 (m, 2H), 2.30-2.06 (m, 4H), 1.44 (s, 3H), 1.43 (s, 3H).

### Example 30

### Preparation of ((1r,4r)-4-(3-(difluoromethyl)-4-(4-(5-(3,3-dimethylmorpholinyl)pyrazolo[1,5-a]pyrimidin-3-yl)-1H-1,2,3-triazol-1-yl)-1H-pyrazol-1-yl)cyclohexyl)methanol (88A)

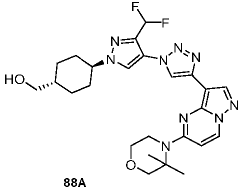

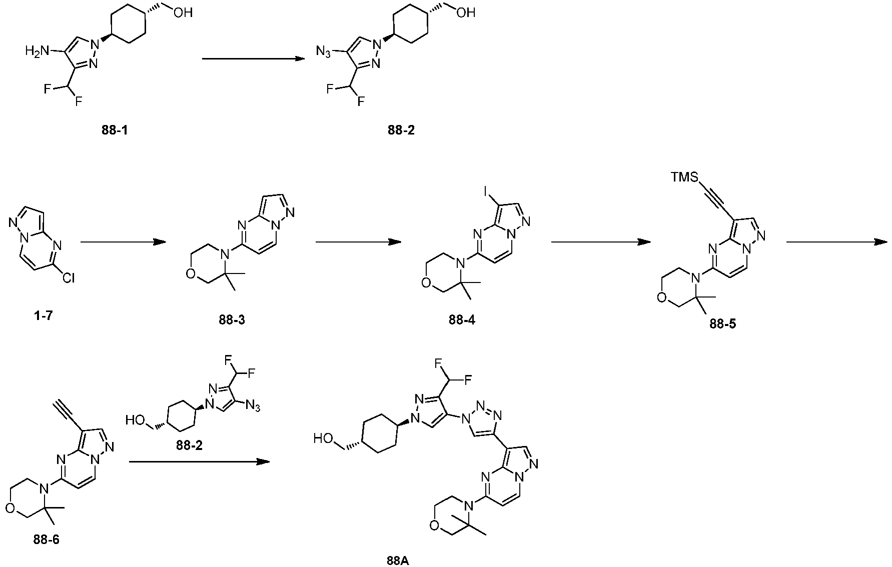

### 1) Step 1 Preparation of compound 88-2

Compound 88-1 (3.00 g, 12.2 mmol, prepared in accordance with the method for preparation of compound in step 6 for intermediate disclosed on page 137 of the specification in the patent application "WO2022161414 A1") was dissolved with acetonitrile (35 mL), and, at 0°C, isopentyl nitrite (2.10 g, 17.9 mmol, Bide) was added. After the reaction solution was stirred for 30 min, azidotrimethylsilane (2.00 g, 17.4 mmol) was added. After stirring at 25°C for 18 h, the reaction solution was poured into water (30 mL). The aqueous phase was extracted with ethyl acetate (30 mL×3). The combined organic phases were washed with saturated brine (50 mL), dried over anhydrous sodium sulfate and filtered. The filtrate was concentrated under reduced pressure to obtain compound 88-2 (3.00 g). MS m/z(ESI): 272.1[M+H]⁺.

### 2) Step 2 Preparation of compound 88-3

5-chloropyrazolo[1,5-a]pyrimidine 1-7 (2.00 g, 13.0 mmol), 3,3-dimethylmorpholine (1.80 g, 15.6 mmol), N,N-diisopropylethylamine (6.47 mL, 39.07 mmol), and N-methylpyrrolidone (15 mL) were reacted at 130°C for 18 h. Then, the reaction solution was mixed with water (100 mL), and extracted with ethyl acetate (100 mL×2). The organic phases were combined, dried over anhydrous sodium sulfate, and filtered to remove the desiccant, and then the filtrate was concentrated under reduced pressure. The residue was purified by silica gel column chromatography with an elution system (petroleum ether/ethyl acetate =100/1 to 1/1) to obtain compound 88-3 (1.0g). MS m/z(ESI): 233.1[M+1]⁺.

### 3) Step 3 Preparation of compound 88-4

Iodosuccinimide (1.94 g, 8.61 mmol) was added to a solution containing compound 88-3 (1.00 g, 4.30 mmol), and acetonitrile (15 mL). After reacting at 25°C for 18 h, the reaction was quenched with saturated sodium sulfite solution (50 mL), and the reaction solution was extracted with ethyl acetate (50 mL×2). The organic phases were combined, dried over anhydrous sodium sulfate, and filtered to remove the desiccant, and then the filtrate was concentrated under reduced pressure. The residue was purified by silica gel column chromatography with an elution system (petroleum ether/ethyl acetate =100/1 to 1/1) to obtain compound 88-4 (600mg). ¹H NMR (400 MHz, DMSO-d6): δ 8.68 (d, J = 8.0 Hz, 1H), 7.97 (s, 1H), 6.79 (d, J = 8.0 Hz, 1H), 3.81 - 3.78 (m, 2H), 3.57 - 3.54 (m, 2H), 3.41 (s, 2H), 1.54 (s, 6H).

### 4) Step 4 Preparation of compound 88-5

Under the protection of nitrogen, bis(triphenylphosphine) palladium dichloride (235 mg, 0.34 mmol) was added to a solution containing compound 88-4 (600 mg, 1.68 mmol), ethynyltrimethylsilane (2.40 mL, 16.8 mmol), triethylamine (0.70 mL, 5.03 mmol) and tetrahydrofuran (10 mL). After replacement of the atmosphere with nitrogen three times, the reaction solution was reacted at 25°C for 18 h, then the reaction was quenched with water (20 mL). The reaction solution was extracted with ethyl acetate (20 mL×2). The organic phases were combined, dried over anhydrous sodium sulfate, and filtered to remove the desiccant, and then the filtrate was concentrated under reduced pressure. The residue was purified by silica gel column chromatography with an elution system (petroleum ether/ethyl acetate =100/1 to 1/1) to obtain compound 88-5 (400mg). MS m/z(ESI): 329.2[M+1]⁺.

### 5) Step 5 Preparation of compound 88-6

Potassium carbonate (337 mg, 2.45 mmol) was added to a solution containing compound 88-5 (400 mg, 1.22 mmol) and methanol (5 mL). After stirring at 25°C for 2 h, the reaction was quenched with water (20 mL), and the reaction solution was extracted with dichloromethane (20 mL×2). The organic phases were combined, dried over anhydrous sodium sulfate, and filtered to remove the desiccant, and then the filtrate was concentrated under reduced pressure. The residue was purified by silica gel column chromatography with an elution system (petroleum ether/ethyl acetate =100/1 to 1/1) to obtain compound 88-6 (260mg). MS m/z(ESI): [M+23]⁺.

### 6) Step 6 Preparation of compound 88A

Sodium ascorbate (8.00 mg, 0.04 mmol) was added to a solution containing compound 88-2 (158.76 mg, 0.59 mmol), compound 88-6 (100 mg, 0.39 mmol), blue vitriol (9.80 mg, 0.04 mmol), ethanol (1 mL) and water (1 mL). After replacement of the atmosphere with nitrogen three times, the reaction solution was stirred at 25°C for 18 h, then the reaction was quenched with water (5 mL). The reaction solution was extracted with ethyl acetate (5 mL×2). The organic phases were combined, dried over anhydrous sodium sulfate, and filtered to remove the desiccant, and then the filtrate was concentrated under reduced pressure. The residue was purified by HPLC (A: 0.1% FA/H2O B: ACN, chromatographic column: Wetch-Ultimate-XB-C18-10µm-21.2*150mm; mobile phase: water (containing 0.1% formic acid) and acetonitrile, gradient ratio: acetonitrile 25%-45%, flow rate: 25mL/min) to obtain compound 88A (150 mg). MS m/z(ESI): 528.4 [M+1]⁺. ¹H NMR (400 MHz, DMSO-d6): δ 8.74 (d, J = 8.0 Hz, 1H), 8.65 (s, 1H), 8.45 - 8.37 (m, 2H), 7.19 (t, J = 53.2 Hz, 1H), 6.85 (d, J = 8.0 Hz, 1H), 4.58 - 4.36 (m, 1H), 4.34 - 4.23 (m, 1H), 3.88 - 3.77 (m, 2H), 3.67 - 3.59 (m, 2H), 3.43 (s, 2H), 3.29 - 3.26 (m, 2H), 2.19 - 2.10 (m, 2H), 1.93 - 1.86 (m, 2H), 1.84 - 1.74 (m, 2H), 1.53 (s, 6H), 1.49 - 1.39 (m, 1H), 1.20 - 1.05 (m, 2H).

### Example 31

### Preparation of ((1r,4r)-4-(3-(difluoromethyl)-4-(4-(5-(2,2-dimethylmorpholinyl)pyrazolo[1,5-a]pyrimidin-3-yl)-1H-1,2,3-triazol-1-yl)-1H-pyrazol-1-ylcyclohexyl)methanol (89A)

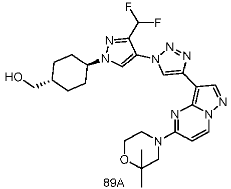

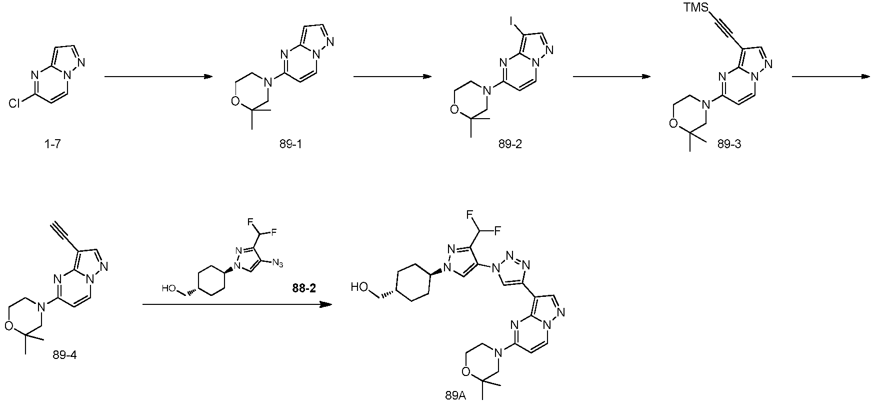

### 1) Step 1 Preparation of compound 89-1

Compound 1-7 (1.00g, 6.51mmol, Bide) was dissolved with acetonitrile (15mL), then 2,2-methylmorpholine (900mg, 7.81mmol, Bide), cesium carbonate (6.36g, 19.5mmol) were added. After reacting at 80°C with stirring for 12 h, the reaction solution, with the addition of water (40ml), was extracted with ethyl acetate (30mL×3). The organic phases were combined, dried over anhydrous sodium sulfate, and filtered to remove the desiccant, and then the filtrate was concentrated under reduced pressure. The residue was purified by silica gel column chromatography with an elution system (dichloromethane/methanol=100/1 to 20/1) to obtain compound 89-1 (1.2g). MS m/z(ESI): 233.5[M+1]⁺.

### 2) Step 2 Preparation of compound 89-2

Compound 89-1 (1.00g, 4.30mmol) was dissolved with acetonitrile (20mL), and, at 0°C, N-iodosuccinimide (970mg, 4.30mmol) was added. After reacting at room temperature with stirring for 1 h, the reaction was quenched with saturated sodium bicarbonate solution (100ml), and the reaction solution was extracted with ethyl acetate (60mL×3). The organic phases were combined, dried over anhydrous sodium sulfate, and filtered to remove the desiccant, and then the filtrate was concentrated under reduced pressure. The residue was purified by silica gel column chromatography with an elution system (petroleum ether/ethyl acetate =100/1 to 20/1) to obtain compound 89-2 (1.7g). MS m/z(ESI): 359.1[M+1]⁺.

### 3) Step 3 Preparation of compound 89-3

Compound 89-2 (1.70g, 4.75mmol) was dissolved with tetrahydrofuran (20mL), then cuprous iodide (90mg, 0.47mmol) and bis(triphenylphosphine) palladium dichloride (330mg, 0.47mmol) and triethylamine (1.97mL, 14.2mmol) were added in sequence. After replacement of the atmosphere with nitrogen three times, ethynyltrimethylsilane (6.76mL, 47.5mmol) was added to the reaction solution. After reacting at room temperature with stirring for 12 h, the reaction solution, with the addition of water (50ml), was extracted with ethyl acetate (40mL×3). The organic phases were combined, dried over anhydrous sodium sulfate, and filtered to remove the desiccant, and then the filtrate was concentrated under reduced pressure. The residue was purified by silica gel column chromatography with an elution system (petroleum ether/ethyl acetate =100/1 to 20/1) to obtain compound 89-3 (1g). MS m/z(ESI): 329.2[M+1]⁺.

### 4) Step 4 Preparation of compound 89-4

Compound 89-3 (800mg, 2.44mmol) was dissolved with methanol (12mL), then potassium carbonate (673mg, 4.87mmol) was added. After reacting at room temperature with stirring for 2 h, the reaction solution was directly concentrated under reduced pressure. The residue was purified by silica gel column chromatography with an elution system (dichloromethane/methanol=100/1 to 20/1) to obtain compound 89-4 (400mg). MS m/z(ESI): 257.2[M+1]⁺.

### 5) Step 5 Preparation of compound 89A

Compound 89-4 (100mg, 0.39mmol), 88-2 (159mg, 0.59mmol) were dissolved with a mixed solvent of water (2.5mL) and ethanol (2.5mL), then blue vitriol (9.74mg, 0.04mmol), sodium ascorbate (7.73mg, 0.04mmol) were added. After reacting at room temperature with stirring for 12 h, the reaction solution was concentrated under reduced pressure to remove ethanol. The reaction solution, with the addition of water (15mL), was extracted with ethyl acetate (15mL×3). The organic phases were combined, dried over anhydrous sodium sulfate, and filtered to remove the desiccant, and then the filtrate was concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 89A (96mg). MS m/z(ESI): 528.4[M+1]⁺. ¹H NMR (400 MHz, DMSO-d6) δ 8.75 (d, J = 7.9 Hz, 1H), 8.69 (s, 1H), 8.53 (s, 1H), 8.37 (s, 1H), 7.24 (t, J = 53.1 Hz, 1H), 6.83 (d, J = 7.9 Hz, 1H), 4.52 (t, J = 5.3 Hz, 1H), 4.30 - 4.25 (m, 1H), 3.74 (s, 4H), 3.63 (s, 2H), 3.28 (t, J = 5.7 Hz, 2H), 2.17 - 2.10 (m, 2H), 1.89 (d, J = 13.4 Hz, 2H), 1.84 - 1.74 (m, 2H), 1.49 - 1.40 (m, 1H), 1.19 (s, 6H), 1.16 - 1.06 (m, 2H).

### Example 32

### Preparation of ((1r, 4r)-4-(4-(4-(5-(2-oxa-6-azaspiro[3.3]heptan-6-yl)pyrazolo[1,5-a]pyrimidin-3-yl)-1H-1,2,3-triazol-1-yl)-3-(difluoromethyl)-1H-pyrazol-1-yl]cyclohexyl)methanol (90A)

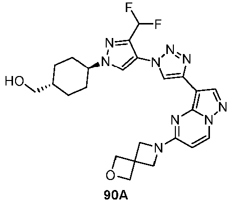

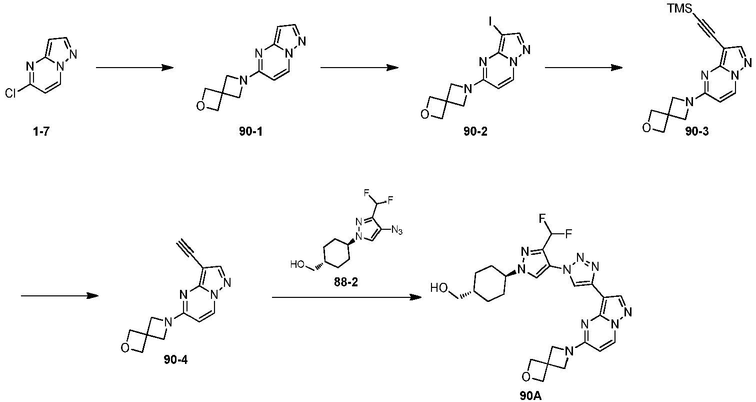

### 1) Step 1 Preparation of compound 90-1

Compound 1-7 (2.00g, 13.0mmol, Bide) and 6-aza-2-oxaspiro[3.3]heptane (1.68g, 17.0mmol, Bide) were dissolved with acetonitrile (20mL), then N,N-diisopropylethylamine (6.5mL, 39.1mmol) was added. After stirring at 80°C for 12 h, the reaction solution was concentrated under reduced pressure to obtain a residue. The residue was purified by silica gel column chromatography with an elution system (dichloromethane/methanol=100/1 to 30/1) to obtain compound 90-1 (2.8g). MS m/z(ESI): 217.1. [M+1]⁺¹H NMR (400 MHz, CDCl₃): δ 8.31 (d, J = 7.5 Hz, 1H), 7.88 (d, J = 2.2 Hz, 1H), 6.17 (d, J = 1.7 Hz, 1H), 5.96 (d, J = 7.5 Hz, 1H), 4.88 (s, 4H), 4.33 (s, 4H).

### 2) Step 2 Preparation of compound 90-2

Compound 90-1 (500mg, 2.31mmol) was dissolved with dichloromethane (10mL), then N-iodosuccinimide (624mg, 2.77mmol) was added. After stirring at 25°C for 4 h, the reaction solution was poured into saturated sodium bicarbonate aqueous solution (10mL) and saturated sodium sulfite aqueous solution (10mL). After the aqueous phase was separated, it was extracted with dichloromethane (20mL ×3). The combined organic phases were dried over anhydrous sodium sulfate, and filtered to remove the desiccant, and then the filtrate was concentrated under reduced pressure. The residue was purified by silica gel column chromatography with an elution system (dichloromethane/methanol=100/1 to 30/1) to obtain compound 90-2 (680mg). MS m/z(ESI): 343.1[M+1]⁺¹. H NMR (400 MHz, DMSO-d6): δ 8.62 (d, J = 7.6 Hz, 1H), 7.89 (s, 1H), 6.23 (d, J = 7.6 Hz, 1H), 4.73 (s, 4H), 4.29 (s, 4H).

### 3) Step 3 Preparation of compound 90-3

Compound 90-2 (580mg, 1.70mmol), triethylamine (0.7mL, 5.09mmol), ethynyltrimethylsilane (2.4mL, 17.0mmol), bis(triphenylphosphine) palladium dichloride (119mg, 0.17mmol) and cuprous iodide (32.3mg, 0.17mmol) were dissolved with tetrahydrofuran (10mL). After replacement of the atmosphere with nitrogen three times, the reaction solution was stirred at 25°C under the protection of nitrogen for 12 h, and then concentrated under reduced pressure. The residue was purified by silica gel column chromatography with an elution system (dichloromethane/methanol=100/1 to 30/1) to obtain compound 90-3 (464mg). MS m/z(ESI): 313.2[M+1]⁺. ¹H NMR (400 MHz, CDCl₃): δ 8.23 (d, J = 7.6 Hz, 1H), 7.97 (s, 1H), 5.97 (d, J = 7.5 Hz, 1H), 4.88 (s, 4H), 4.39 (s, 4H), 0.28 (s, 9H).

### 4) Step 4 Preparation of compound 90-4

Compound 90-3 (110mg, 0.35mmol) was dissolved with tetrahydrofuran (5mL), then tetrabutylammonium fluoride (0.5mL, 0.46mmol, 1.0 M tetrahydrofuran solution) was added. After stirring at 25°C for 1 h, the reaction solution was concentrated under reduced pressure. The residue was dissolved with dichloromethane (10mL). The organic phase was washed with saturated sodium bicarbonate aqueous solution (5mL×3), dried over sodium sulfate, and filtered to remove the desiccant, and then the filtrate was concentrated under reduced pressure to obtain compound 90-4 (84.6mg). MS m/z(ESI): 241.1[M+1]⁺.

### 5) Step 5 Preparation of compound 90A

Compound 90-4 (84.6mg, 0.35mmol), compound 88-2 (95.5mg, 0.35mmol) and blue vitriol (8.79mg, 0.04mmol) were dissolved with water (2mL) and ethanol (2mL), then sodium ascorbate (6.97mg, 0.04mmol) was added. After stirring at 25°C for 12 h, the reaction solution was concentrated under reduced pressure to obtain a residue. The residue was purified by HPLC (Waters-2545, chromatographic column: SharpSil-T C18,30*150mm, 5µm; mobile phase: water (containing 10mmol/L formic acid) and acetonitrile, gradient ratio: acetonitrile 38%-45%, flow rate: 30mL/min) to obtain compound 90A (70.3mg). MS m/z(ESI): 512.3[M+1]⁺. ¹H NMR (400 MHz, DMSO-d6) δ 8.71 (d, J = 7.6 Hz, 1H), 8.68 (s, 1H), 8.51 (s, 1H), 8.35 (s, 1H), 7.23 (t, J = 53.2 Hz, 1H), 6.31 (d, J = 7.6 Hz, 1H), 4.74 (s, 4H), 4.52 (t, J = 5.3 Hz, 1H), 4.33 (s, 4H), 4.30 - 4.24 (m, 1H), 3.29 - 3.26 (m, 2H), 2.17 - 2.11 (m, 2H), 1.93 - 1.77 (m, 5H), 1.16 - 1.08 (m, 2H).

### Example 33

### Preparation of 4-(3-(1-(3-(difluoromethyl)-1-((1r,4r)-4-(methylol)cyclohexyl)-1H-pyrazol-4-yl)-1H-1,2,3-triazol-4-yl)pyrazolo[1,5-a]pyrimidin-5-yl)thiomorpholine 1,1-dioxide (91A)

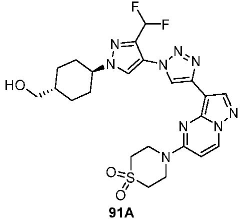

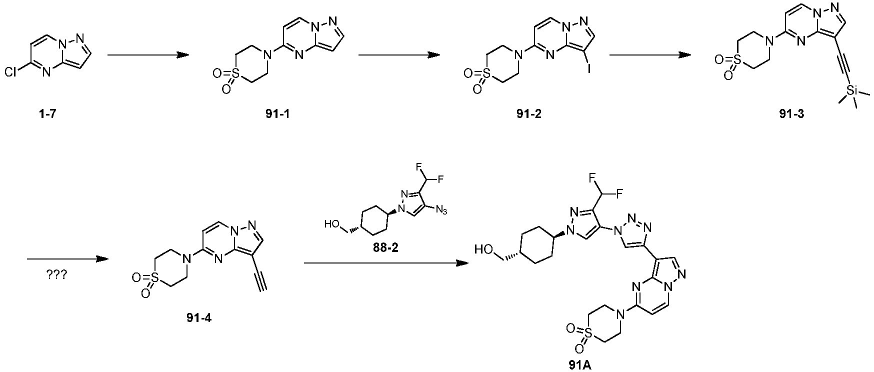

### 1) Step 1 Preparation of compound 91-1

Compound 1-7 (1.02 g, 6.51 mmol) was dissolved with N-methylpyrrolidone (10 mL), then N,N-diisopropylethylamine (3.24 mL, 19.5 mmol) and thiomorpholine-1,1-dioxide (1.32 g, 9.77mmol) were added in sequence. After stirring at 130°C for 18 h, the reaction solution was filtered to obtain compound 91-1 (3.84 g). MS m/z(ESI): 253.1[M+1]⁺.

### 2) Step 2 Preparation of compound 92-2

Compound 91-1 (900 mg, 3.57 mmol) was dissolved with acetonitrile (10 mL) and dichloromethane (10 mL), then N-iodosuccinimide (882 mg, 3.92 mmol) was added. After stirring at 25°C for 18 h, the reaction solution was filtered to obtain compound 92-2 (1.10 g). MS m/z(ESI): 379.0[M+1]⁺.

### 3) Step 3 Preparation of compound 91-3

Compound 91-2 (500mg, 1.32mmol), triethylamine (0.6mL, 3.97mmol), ethynyltrimethylsilane (1.9mL, 13.2mmol), bis(triphenylphosphine) palladium dichloride (92.8mg, 0.13mmol) and cuprous iodide (25.2mg, 0.13mmol) were dissolved with tetrahydrofuran (15mL). After replacement of the atmosphere with nitrogen three times, the reaction solution was stirred at 25°C under the protection of nitrogen for 12 h, and then concentrated under reduced pressure. The residue was purified by silica gel column chromatography with an elution system (dichloromethane/methanol=100/1 to 20/1) to obtain compound 91-3 (420mg). MS m/z(ESI): 349.0[M+1]⁺

### 4) Step 4 Preparation of compound 91-4

Compound 91-3 (390mg, 1.12mmol) was dissolved with tetrahydrofuran (15mL), then tetrabutylammonium fluoride (1.3mL, 1.34mmol, 1.0 M tetrahydrofuran solution) was added. After stirring at 25°C for 1 h, the reaction solution was concentrated under reduced pressure. The residue was dissolved with dichloromethane (10mL). The organic phase was washed with saturated sodium bicarbonate aqueous solution (5mL×2), dried over sodium sulfate, and filtered to remove the desiccant, and then the filtrate was concentrated under reduced pressure to obtain compound 91-4 (260mg). MS m/z(ESI): 277.1[M+1]⁺. ¹H NMR (400 MHz, DMSO-d6): δ 8.78 (d, J = 7.9 Hz, 1H), 8.08 (s, 1H), 6.95 (d, J = 7.9 Hz, 1H), 4.18 (s, 4H), 4.03 (s, 1H), 3.24 (s, 4H).

### 5) Step 5 Preparation of compound 91A

Compound 91-4 (50.0mg, 0.18mmol) and compound 88-2 (51.2mg, 0.19mmol) were dissolved with water (1.5mL) and ethanol (1.5mL), then blue vitriol (4.52mg, 0.02mmol) and sodium ascorbate (3.58mg, 0.02mmol) were added. After stirring at 25°C for 12 h, the reaction solution was concentrated under reduced pressure to obtain a residue. The residue was purified by HPLC (Waters-2545, chromatographic column: SharpSil-T C18,30*150mm, 5µm; mobile phase: water (containing 10mmol/L formic acid) and acetonitrile, gradient ratio: acetonitrile 38%-45%, flow rate: 30mL/min) to obtain compound 91A (25.7mg). MS m/z(ESI): 548.4[M+1]⁺. ¹H NMR (400 MHz, DMSO-d6) δ 8.84 (d, J = 7.9 Hz, 1H), 8.65 (s, 1H), 8.55 (s, 1H), 8.42 (s, 1H), 7.20 (t, J = 53.2 Hz, 1H), 6.97 (d, J = 7.9 Hz, 1H), 4.47 (t, J = 5.3 Hz, 1H), 4.32 - 4.20 (m, 5H), 3.29 - 3.21 (m, 6H), 2.17 - 2.10 (m, 2H), 1.93 - 1.86 (m, 2H), 1.85 - 1.75 (m, 2H), 1.50 - 1.41 (m, 1H), 1.19 - 1.08 (m, 2H).

### Example 34

### Preparation of 4-((3-(1-(3-(difluoromethyl)-1-((1r,4r)-4-(methylol)cyclohexyl)-1H-pyrazol-4-yl)-1H-1,2,3-triazol-4-yl]razolo[1,5-a]rimidin-5-l)amino)-2-methlbutan-2-ol (92A)

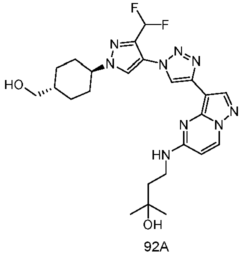

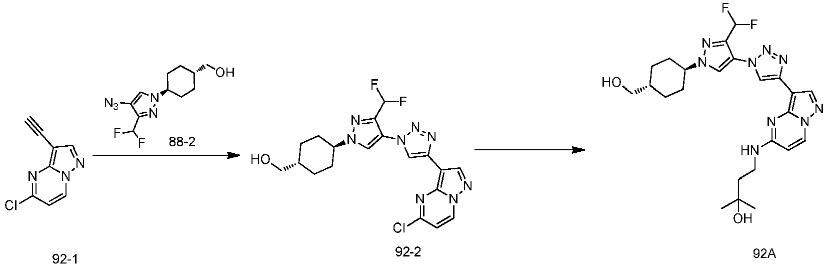

### 1) Step 1 Preparation of compound 92-2

Compound 92-1 (2.30g, 13.0mmol, prepared in accordance with the method for preparation of intermediate 5-chloro-3-ethynylpyrazolo[1,5-a]pyrimidine disclosed on page 102 of the specification in the patent application "WO2015108490 A2"), 88-2 (3.51g, 13.0mmol) were dissolved with a mixed solvent of water (40mL) and ethanol (40mL), and then blue vitriol (320mg, 1.30mmol), sodium ascorbate (260mg, 1.30mmol) were added. After reacting at room temperature with stirring for 12 h, the reaction solution was concentrated under reduced pressure to remove ethanol. Water (50mL) was added to the reaction solution, to precipitate out a large amount of solids, followed by filtering. The filter cake was washed with washing solution (petroleum ether/ethyl acetate =1/1) twice, and filtered. The filter cake was directly used as the raw material in the next reaction without purification, as compound 92-2 (2.30g). MS m/z(ESI): 449.1[M+1]⁺.

### 2) Step 2 Preparation of compound 92A

Compound 92-2 (50.0mg, 0.11mmol) was dissolved with acetonitrile (3mL), then 4-amino-2-methyl-butan-2-ol (23.0mg, 0.22mmol, Bide), N,N-diisopropylethylamine (43.2mg, 0.33mmol) were added. After reacting at 80°C with stirring for 2 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 92A (12mg). MS m/z(ESI): 516.3[M+1]⁺. ¹H NMR (400 MHz, DMSO-d6) δ 8.64 (s, 1H), 8.52 - 8.48 (m, 2H), 8.27 (s, 1H), 7.68 (s, 1H), 7.27 (t, J = 53.3 Hz, 1H), 6.30 (d, J = 7.6 Hz, 1H), 4.49 - 4.32 (m, 2H), 4.30 - 4.22 (m, 1H), 3.54 - 3.46 (m, 2H), 3.32 - 3.27 (m, 2H), 2.19 - 2.12 (m, 2H), 1.90 (d, J = 13.1 Hz, 2H), 1.85 - 1.70 (m, 4H), 1.55 - 1.47 (m, 1H), 1.20 - 1.11 (m, 8H).

### Example 35

### Preparation of 1-(3-(1-(3-difluoromethyl)-1-((1r,4r)-4-(methylol)cyclohexyl)-1H-pyrazol-4-yl)-1H-1,2,3-triazol-4-ylpyrazolo[1,5-a]pyrimidin-5-yl)-4-methylpiperidin-4-ol (93A)

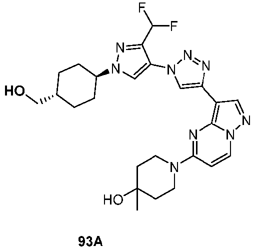

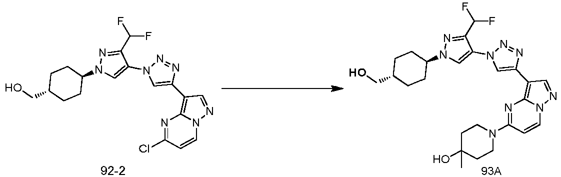

### 1) Step 1 Preparation of compound 93A

At room temperature, to a single-necked bottle, compound 92-2 (50.0mg, 0.110mmol), 4-methyl-4-hydroxypiperidine (19.3mg, 0.170mmol, Leyan) and acetonitrile (1mL) were added in sequence, and, N,N-diisopropylethylamine (27.7µL, 0.170mmol) was added with stirring. After replacement of the atmosphere with nitrogen three times, the reaction solution was reacted at 80°C for 2 h. The reaction solution was purified by HPLC (Gilson_306_1741, chromatographic column: Waters-Xbridge-C18-10µm-19*250mm; mobile phase: water (containing 10mmol/L ammonium bicarbonate) and acetonitrile, gradient ratio: acetonitrile 24%-95%, flow rate: 25mL/min) to obtain compound 93A (30.1mg). MS m/z(ESI): 528.3 [M+1]⁺. ¹H NMR (400 MHz, DMSO-d6) δ 8.67 - 8.65 (m, 2H), 8.52 (s, 1H), 8.33 (s, 1H), 7.21 (t, J = 53.2 Hz, 1H), 6.81 (d, J = 8.0 Hz, 1H), 4.48 (t, J = 5.3 Hz, 1H), 4.44 (s, 1H), 4.30 - 4.24 (m, 1H), 4.20 - 3.95 (m, 2H), 3.54 - 3.40 (m, 2H), 3.28 (t, J = 5.7 Hz, 2H), 2.16 - 2.12 (m, 2H), 1.94 - 1.73 (m, 4H), 1.61 - 1.41 (m, 5H), 1.16 (s, 3H), 1.15 - 1.05 (m, 4H).

### Example 36

### Preparation of ((1r, 4r)-4-(4-(4-(5-((cyclopropylmethyl)amino)pyrazolo[1,5-a]pyrimidin-3-yl)-1H-1,2,3-triazol-1-yl)-3-(difluoromethyl)-1H-pyrazol 1-yl)cyclohexyl)methanol (94A)

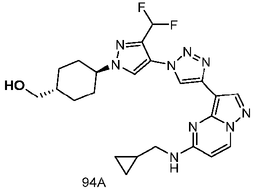

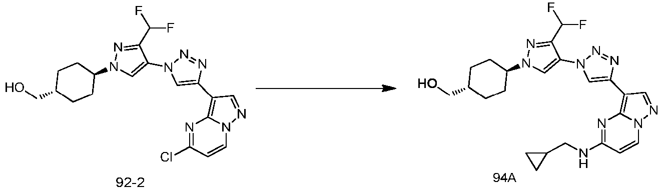

### 1) Step 1 Preparation of compound 94A

To a solution of 92-2 (50.0mg, 0.110mmol) in acetonitrile (3mL), cyclopropyl methylamine (0.030mL, 0.340 mmol) and N,N-diisopropylethylamine (58.0mg, 0.450mmol) were added. After reacting at 80°C under nitrogen atmosphere with stirring for 3 h, the reaction solution was concentrated under reduced pressure, and then purified by HPLC (Gilson_306_1741, chromatographic column: Waters-Xbridge-C18-10µm-19*250mm; mobile phase: water (containing 10mmol/L ammonium bicarbonate) and acetonitrile, gradient ratio: acetonitrile 30%-95%, flow rate: 25mL/min) to obtain compound 94A (30.0mg). MS m/z(ESI): 484.2 [M+1]⁺. ¹H NMR (400 MHz, DMSO-d6) δ 8.66 (s, 1H), 8.52 - 8.50 (m, 2H), 8.27 (s, 1H), 7.81 (s, 1H), 7.20 (t, J = 53.2 Hz, 1H), 6.35 (d, J = 7.6 Hz, 1H), 4.47 (t, J = 5.2 Hz, 1H), 4.32 - 4.24 (m, 1H), 3.29 - 3.25 (m, 4H), 2.16 - 2.13 (m, 2H), 2.02 - 1.70 (m, 4H), 1.53 -1.37 (m, 1H), 1.20 - 1.04 (m, 3H), 0.56 - 0.44 (m, 2H), 0.33 - 0.21 (m, 2H).

### Example 37

### Preparation of (1R, 4R)-4-(3-difluoromethyl)-4-(4-(5-(4-methylpiperazin-1-yl)pyrazolo[1, 5-a]pyrimidin-3-yl)-1H-1, 2, 3-triazol-1-yl)-1H-pyrazol-1-yl)cyclohexylmethanol formate (95A)

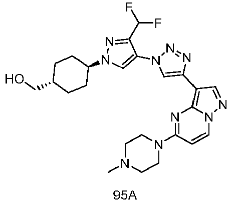

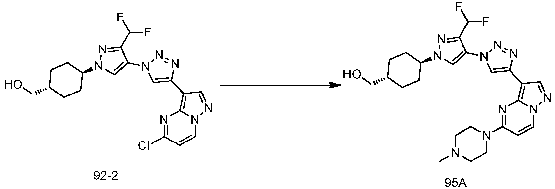

### 1) Step 1 Preparation of compound 95A

Compound 92-2 (50 mg, 0.11 mmol) was dissolved with acetonitrile (3 mL), then N-methylpiperazine (20 mg, 0.20 mmol) and N,N-diisopropylethylamine (20 mg, 0.23 mmol) were added in sequence. After stirring at 80°C for 2 h, the reaction solution was filtered, and the filtrate was concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 30%-70%, flow rate: 30mL/min) to obtain compound 95A formate (35 mg). MS m/z(ESI): 513.4[M+H]⁺. ¹H NMR (400 MHz, DMSO-d6): δ 8.72 (d, J = 7.9 Hz, 1H), 8.66 (s, 1H), 8.53 (s, 1H), 8.36 (s, 1H), 8.17 (s, 1H), 7.20 (t, J = 53.2 Hz, 1H), 6.81 (d, J = 7.9 Hz, 1H), 4.31 - 4.24 (m, 1H), 3.78 - 3.70 (m, 4H), 3.28 (d, J = 6.2 Hz, 2H), 2.47 - 2.39 (m, 4H), 2.24 (s, 3H), 2.19 - 2.11 (m, 2H), 1.94 - 1.86 (m, 2H), 1.85 - 1.74 (m, 2H), 1.50 - 1.40 (m, 1H), 1.18 - 1.06 (m, 2H).

### Example 38

### Preparation of 1-(3-(1-(3-difluoromethyl)-1-((1r,4r)-4-(methylol)cyclohexyl)-1H-pyrazol-4-yl)-1H-1,2,3-triazol-4-ylpyrazolo[1,5-a]pyrimidin-5-yl)azetidin-3-ol (96A)

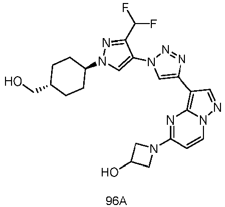

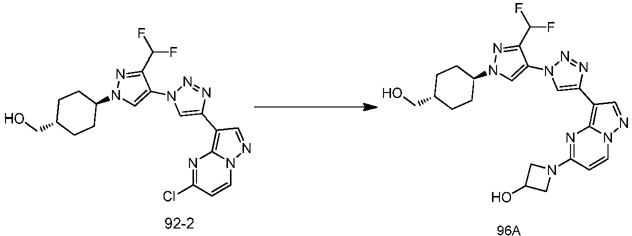

### 1) Step 1 Preparation of compound 96A

At room temperature, to a single-necked bottle, compound 92-2 (50.0mg, 0.110mmol), 3-hydroxyazetidine hydrochloride (18.3mg, 0.170mmol, Leyan) and acetonitrile (1mL) were added in sequence, and, N,N-diisopropylethylamine (27.3µL, 0.170mmol) was added with stirring. After replacement of the atmosphere with nitrogen three times, the reaction solution was reacted at 80°C for 2 h. The reaction solution was purified by HPLC (Gilson_306_1741, chromatographic column: Waters-Xbridge-C18-10µm-19*250mm; mobile phase: water (containing 10mmol/L ammonium bicarbonate) and acetonitrile, gradient ratio: acetonitrile 19%-95%, flow rate: 25mL/min) to obtain compound 96A (12.0mg). MS m/z(ESI): 486.2[M+1]⁺. ¹H NMR (400 MHz, DMSO-d6) δ 8.73 - 8.64 (m, 2H), 8.52 (s, 1H), 8.34 (s, 1H), 7.22 (t, J = 53.2 Hz, 1H), 6.31 (d, J = 7.6 Hz, 1H), 5.80 (d, J = 6.5 Hz, 1H), 4.64 - 4.62 (m, 1H), 4.47 (t, J = 5.3 Hz, 1H), 4.42 - 4.32 (m, 2H), 4.32 - 4.19 (m, 1H), 3.92 - 3.88 (m, 2H), 3.27 (d, J = 5.7 Hz, 2H), 2.14 (d, J = 11.8 Hz, 2H), 1.89 (d, J = 13.3 Hz, 2H), 1.82 - 1.79 (m, 2H), 1.53 - 1.39 (m, 1H), 1.17 - 1.07 (m, 2H).

### Example 39

### Preparation of 1-(3-(1-(3-difluoromethyl)-1-((1r,4r)-4-(methylol)cyclohexyl)-1H-pyrazol-4-yl)-1H-1,2,3-triazol-4-yl]pyrazolo[1,5-a]pyrimidin-5-yl)piperidin-4-ol (97A)

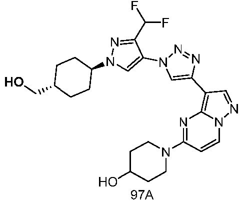

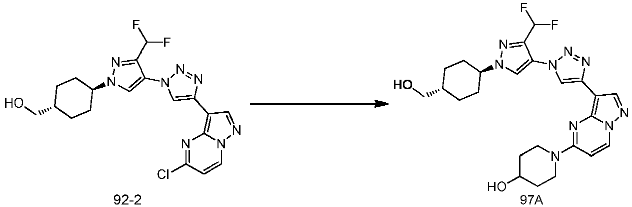

### 1) Step 1 Preparation of compound 97A

At 25°C, compound 92-2 (50.0 mg, 0.11 mmol) was dissolved with acetonitrile (1 mL), then compound 4-hydroxypiperidine (0.010 mL, 0.20 mmol), N,N-diisopropylethylamine (43.1 mg, 0.33 mmol) were added. After stirring at 60°C for 2 h, the reaction solution was concentrated under reduced pressure. The residue was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min), to obtain compound 97A (20.0 mg). MS m/z(ESI): 514.3[M+1]⁺. ¹H NMR (400 MHz, DMSO-d6): δ 8.65 - 8.62 (m, 2H), 8.48 (s, 1H), 8.29 (s, 1H), 7.17 (t, J = 53.1 Hz, 1H), 6.78 (d, J = 8.0 Hz, 1H), 4.74 (d, J = 4.2 Hz, 1H), 4.49 - 4.45 (m, 1H), 4.27 - 4.20 (m, 1H), 4.15 - 4.07 (m, 2H), 3.75 - 3.71 (m, 1H), 3.25 - 3.20 (m, 3H), 2.11 - 2.06 (m, 2H), 1.86 - 1.74 (m, 6H), 1.41 - 1.33 (m, 3H), 1.22 - 1.16 (m, 1H), 1.11 - 1.02 (m, 2H).

### Example 40

### Preparation of ((1r,4r)-4-(4-(4-(5-(1,4-oxacyclopent-4-yl)pyrazolo[1,5-a]pyrimidin-3-yl)-1H-1,2,3-triazol-1-yl)-3-(difluoromethyl)-1H-pyrazol-1-yl]cyclohexyl)methanol (98A)

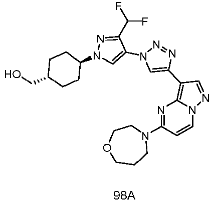

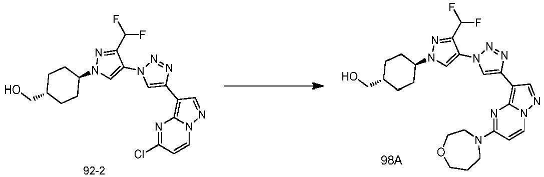

### 1) Step 1 Preparation of compound 98A

Compound 92-2 (50.0mg, 0.11mmol) was dissolved with acetonitrile (3mL), then 1,4-oxazepane (22.3mg, 0.22mmol, Bide), N,N-diisopropylethylamine (43.2mg, 0.33mmol) were added. After reacting at 80°C with stirring for 2 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 98A (22mg). MS m/z(ESI): 514.4[M+1]⁺. ¹H NMR (400 MHz, DMSO-d6) δ 8.71 (d, J = 7.9 Hz, 1H), 8.66 (s, 1H), 8.49 (s, 1H), 8.35 (s, 1H), 7.21 (t, J = 53.1 Hz, 1H), 6.74 (d, J = 7.9 Hz, 1H), 4.48 (t, J = 5.3 Hz, 1H), 4.30 - 4.21 (m, 1H), 4.05 - 3.80 (m, 4H), 3.79 - 3.75 (m, 2H), 3.64 (t, J = 5.5 Hz, 2H), 3.30 - 3.25 (m, 2H), 2.17 - 2.10 (m, 2H), 1.95 - 1.86 (m, 4H), 2.00 - 1.75 (m, 2H), 1.50 - 1.40 (m, 1H), 1.20 - 1.05 (m, 2H).

### Example 41

### Preparation of ((1r,4r)-4-(4-(4-(5-(8-oxa-3-azabicyclo[3.2.1]oct-3-yl)pyrazolo[1,5-a]pyrimidin-3-yl)-1H-1,2,3-triazol-1-yl)-3-(difluoromethyl)-1H-pyrazol 1-yl)cyclohexyl)methanol (99A)

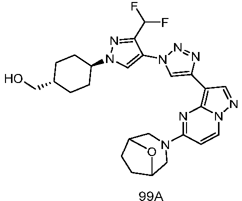

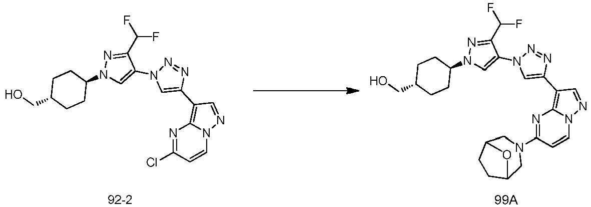

### 1) Step 1 Preparation of compound 99A

Compound 92-2 (50.0mg, 0.11mmol) was dissolved with acetonitrile (3mL), then 8-oxa-3-azabicyclo[3.2.1]octane (33.3mg, 0.22mmol, Bide), N,N-diisopropylethylamine (43.2mg, 0.33mmol) were added. After reacting at 80°C with stirring for 2 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 99A (17mg). MS m/z(ESI): 526.3[M+1]⁺. ¹H NMR (400 MHz, DMSO-d6) δ 8.73 (d, J = 7.9 Hz, 1H), 8.66 (s, 1H), 8.52 (s, 1H), 8.37 (s, 1H), 7.26 (t, J = 53.2 Hz, 1H), 6.72 (d, J = 7.9 Hz, 1H), 4.49 - 4.44 (m, 3H), 4.31 - 4.23 (m, 1H), 4.15 - 4.05 (m, 2H), 3.29 - 3.26 (m, 2H), 3.16 (d, J = 12.8 Hz, 2H), 2.17 - 2.11 (m, 2H), 1.91 - 1.78 (m, 6H), 1.75 - 1.71 (m, 2H), 1.52 - 1.40 (m, 1H), 1.17 - 1.08 (m, 2H).

### Example 42

### Preparation of 1-(3-(1-(3-difluoromethyl)-1-((1r,4r)-4-(methylol)cyclohexyl)-1H-pyrazol-4-yl)-1H-1,2,3-triazol-4-yl]pyrazolo[1,5-a]pyrimidin-5-yl)piperidin-3-ol (100A)

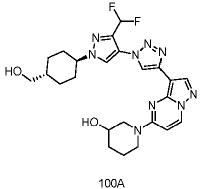

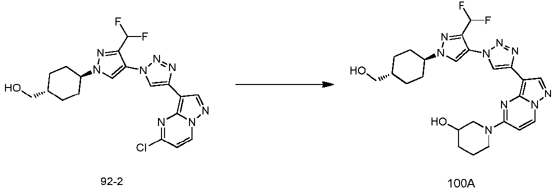

### 1) Step 1 Preparation of compound 100A

Compound 92-2 (50.0mg, 0.11mmol) was dissolved with acetonitrile (3mL), to which 3-hydroxypiperidine (23.0mg, 0.22mmol, Bide), N,N-diisopropylethylamine (43.2mg, 0.33mmol) were added. After reacting at 80°C with stirring for 2 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 100A (27mg). MS m/z(ESI): 514.3[M+1]⁺. ¹H NMR (400 MHz, DMSO-d6) δ 8.65 (d, J = 5.0 Hz, 2H), 8.49 (s, 1H), 8.33 (d, J = 2.0 Hz, 1H), 7.19 (t, J = 53.2 Hz, 1H), 6.77 (d, J = 7.9 Hz, 1H), 4.87 (d, J = 4.2 Hz, 1H), 4.48 (t, J = 5.4 Hz, 1H), 4.27 (t, J = 12.1 Hz, 1H), 4.11 - 3.97 (m, 2H), 3.59 (s, 1H), 3.49 - 3.35 (m, 1H), 3.30 - 3.25 (m, 2H), 3.25 - 3.20 (m, 1H), 2.18 - 2.12 (m, 2H), 2.00 - 1.75 (m, 6H), 1.53 - 1.43 (m, 3H), 1.18 - 1.08 (m, 2H).

### Example 43

### Preparation of 1-(3-(1-(3-difluoromethyl)-1-((1r,4r)-4-(methylol)cyclohexyl)-1H-pyrazol-4-yl)-1H-1,2,3-triazol-4-yl]pyrazolo[1,5-a]pyrimidin-5-yl)pyrrolidin-3-ol (101A)

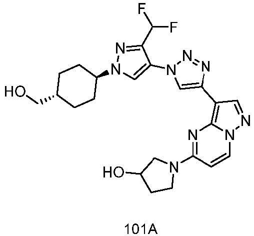

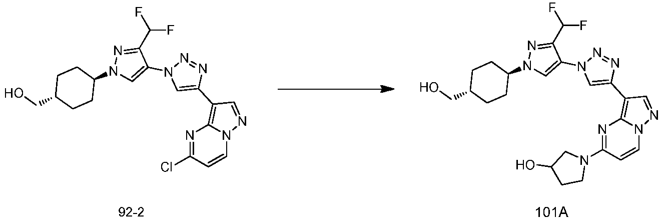

### 1) Step 1 Preparation of compound 101A

Compound 92-2 (50.0mg, 0.11mmol) was dissolved with acetonitrile (3mL), to which 3-hydroxypyrrolidine (19.2mg, 0.22mmol, Bide), N,N-diisopropylethylamine (43.2mg, 0.33mmol) were added. After reacting at 80°C with stirring for 2 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 101A (15mg). MS m/z(ESI): 500.3[M+1]⁺.

¹H NMR (400 MHz, DMSO-d6): δ 8.70 - 8.65(m, 2H), 8.52 (s, 1H), 8.33 (s, 1H), 7.20 (t, J = 53.2 Hz, 1H), 6.47 (s, 1H), 5.10 - 5.01 (m, 1H), 4.50 - 4.41 (m, 2H), 4.31 - 4.24 (m, 1H), 3.63 (s, 3H), 3.30 - 3.25 (m, 2H), 2.20 - 2.00 (m, 3H), 2.00 - 1.81 (m, 4H), 1.80 - 1.74 (m, 2H), 1.50 - 1.42 (m, 1H), 1.20 - 1.10 (m, 2H).

### Example 44

### Preparation of ((1r, 4r)-4-(3-(difluoromethyl)-4-(4-(5-(4-(dimethylamino) piperidin-1-yl) pyrazolo[1,5-a]pyrimidin-3-yl)-1H-1,2,3-triazol-1-yl)-1H-pyrazol-1-ylcyclohexyl) methanol (102A)

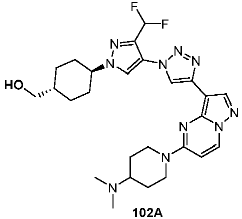

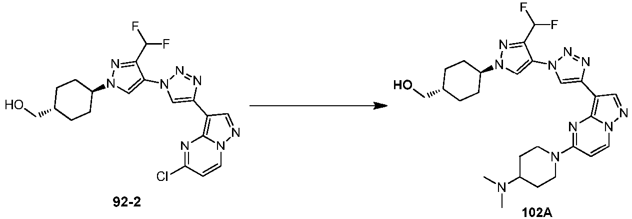

### 1) Step 1 Preparation of compound 102A

Compound 92-2 (80 mg, 0.18 mmol), compound 4-dimethylaminopiperidine (46 mg, 0.36 mmol, Bide) were dissolved with acetonitrile (2 mL), to which N,N-diisopropylethylamine (0.060 mL, 0.36 mmol) was added. After reacting at 80°C with stirring for 2 h, the reaction solution, with the addition of water (10 mL), was extracted with ethyl acetate (20 mL×3). The organic phases were combined, washed with saturated brine, dried over anhydrous sodium sulfate, and filtered to remove the desiccant, and then the filtrate was concentrated under reduced pressure. The residue was purified by HPLC (Waters-2545, chromatographic column: SharpSil-TC18,30*150mm, 5µm; mobile phase: water (containing 10 mmol/L formic acid) and acetonitrile, gradient ratio: acetonitrile 38%-45%, flow rate: 30 mL/min) to obtain compound 102A (15.0mg). MS m/z(ESI): 541.8[M+1]⁺. ¹H NMR (400 MHz, DMSO-d6): δ 8.71 (d, J = 7.9 Hz, 1H), 8.69 (s, 1H), 8.53 (s, 1H), 8.36 (s, 1H), 7.22 (t, J = 53.2 Hz, 1H), 6.84 (d, J = 8.0 Hz, 1H), 4.63 - 4.48 (m, 2H), 4.34 - 4.21 (m, 2H), 3.28 (d, J = 6.2 Hz, 2H), 3.02 (t, J = 12.3 Hz, 2H), 2.64 - 2.54 (m, 1H), 2.27 (s, 6H), 2.18 - 2.09 (m, 2H), 1.94 - 1.85 (m, 3H), 1.86 - 1.73 (m, 3H), 1.50 - 1.36 (m, 3H), 1.18 - 1.05 (m, 2H).

### Example 45

### Preparation of ((1r, 4r)-4-(4-(4-(5-((S)-3-aminopiperidin-1-yl) pyrazolo[1,5-a]pyrimidin-3-yl)-1H-1,2,3-triazol-1-yl)-3-(difluoromethyl)-1H-pyrazol-1-yl]cyclohexyl) methanol (103A)

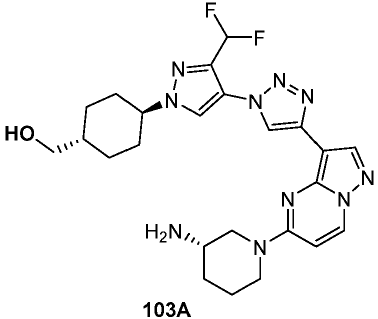

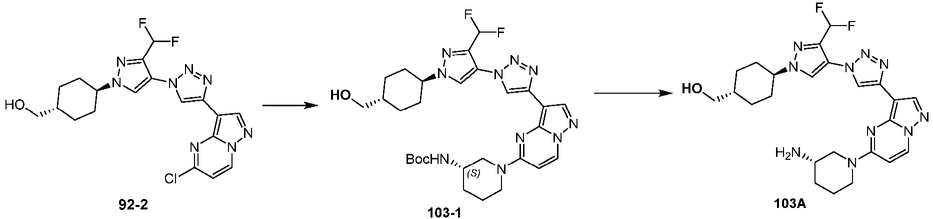

### 1) Step 1 Preparation of compound 103-1

Compound 92-2 (60 mg, 0.13 mmol), compound (S)-3-Boc-aminopiperidine (27 mg, 0.13 mmol) were dissolved with acetonitrile (2 mL), to which N,N-diisopropylethylamine (0.07 mL, 0.40 mmol) was added. After reacting at 80°C with stirring for 2 h, the reaction solution, with the addition of water (10 mL), was extracted with ethyl acetate (20 mL×3). The organic phases were combined, washed with saturated brine, dried over anhydrous sodium sulfate, and filtered to remove the desiccant, and then the filtrate was concentrated under reduced pressure. The residue was purified by silica gel column chromatography with an elution system (dichloromethane/methanol=40/1 to 15/1) to obtain the title compound 103-1 (30 mg). MS m/z(ESI): 611.5[M-1]⁻.

### 2) Preparation of compound 103A

Trifluoroacetic acid (1.0 mL) was added to a solution containing compound 103-1 (25mg, 0.040 mmol) and dichloromethane (2 mL). After stirring at 25°C for 1 h, the reaction solution was concentrated. The residue was purified by HPLC (Waters-2545, chromatographic column: SharpSil-TC18, 30*150mm, 5µm; mobile phase: water (containing 10 mmol/L formic acid) and acetonitrile, gradient ratio: acetonitrile 38%-45%, flow rate: 30 mL/min) to obtain compound 103A (10 mg). MS m/z(ESI): 513.4[M+1]⁺. ¹H NMR (400 MHz, DMSO-d6) δ 8.77 - 8.72 (m, 1H), 8.71 (s, 1H), 8.57 (s, 1H), 8.37 (s, 1H), 7.21 (t, J = 53.3 Hz, 1H), 6.80 (d, J = 7.9 Hz, 1H), 4.44 - 4.33 (m, 2H), 4.32 - 4.23 (m, 2H), 4.19 - 4.10 (m, 2H), 3.32 - 3.26 (m, 3H), 3.22 - 3.17 (m, 1H), 3.05 (s, 1H), 2.19 - 2.09 (m, 2H), 2.02 - 1.93 (m, 1H), 1.93 - 1.85 (m, 2H), 1.85 - 1.74 (m, 3H), 1.59 - 1.50 (m, 2H), 1.48 - 1.37 (m, 1H), 1.18 - 1.05 (m, 2H).

### Example 46

### Preparation of ((1r,4r)-4-(3-(difluoromethyl)-4-(4-(5-morpholinyl-pyrazolo[1,5-a]pyrimidin-3-yl)-1H-1,2,3-triazol-1-yl)-1H-pyrazol-1-yl]cyclohexyl)methanol (104A)

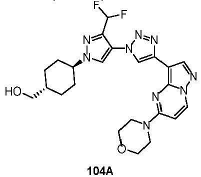

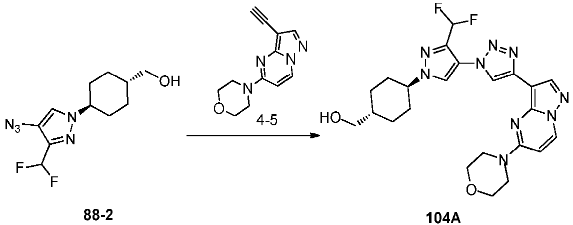

### 1) Step 1 Preparation of compound 104A

Compound 88-2 (2.20 g, 8.11 mmol) was dissolved with ethanol (30 mL) and water (10 mL), to which sodium ascorbate (0.33 g, 1.67 mmol, Energy), blue vitriol (0.41 g, 1.64 mmol, Energy) and compound 4-5 (1.86 g, 8.15 mmol) were added in sequence. After stirring at 25°C for 18 h, the reaction solution was filtered. The solids were collected, and slurried with a mixed solvent (petroleum ether/ethyl acetate =1/1, 30 mL), to obtain compound 104A (3.20 g). MS m/z(ESI): 500.4[M+1]⁺. ¹H NMR (400 MHz, DMSO-d6): δ 8.76 (d, J = 7.9 Hz, 1H), 8.66 (s, 1H), 8.53 (s, 1H), 8.38 (s, 1H), 7.20 (t, J = 53.2 Hz, 1H), 6.81 (d, J = 7.9 Hz, 1H), 4.50 (s, 1H), 4.27 (t, J = 11.9 Hz, 1H), 3.73 (s, 8H), 3.28 (d, J = 5.7 Hz, 2H), 2.19 - 2.07 (m, 2H), 1.93 - 1.76 (m, 4H), 1.54 - 1.38 (m, 1H), 1.20 - 1.07 (m, 2H).

### Example 47

### Preparation of ((1R,4r)-4-(4-(4-(5-((1R,4r)-2-oxa-5-azabicyclo[2.2.1]heptan-5-yl)pyrazolo[1,5-a]pyrimidin-3-yl)-1H-1,2,3-triazol-1-yl)-3-(difluoromethyl)-1H-pyrazol-1-yl)cyclohexyl)methanol (105A)

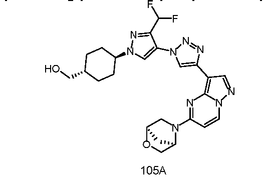

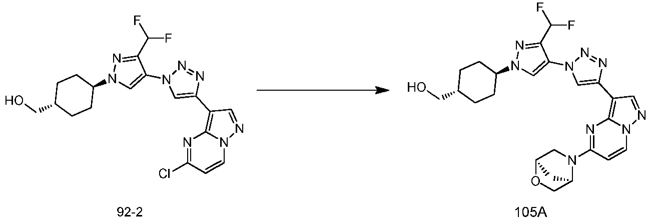

### 1) Step 1 Preparation of compound 105A

Compound 92-2 (50.0mg, 0.11mmol) was dissolved with acetonitrile (3mL), to which (1R,4R)-2-oxa-5-azabicyclo[2.2.1]heptane hydrochloride (29.8mg, 0.22mmol, Bide), N,N-diisopropylethylamine (43.2mg, 0.33mmol) were added. After reacting at 80°C with stirring for 2 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 105A (15mg). MS m/z(ESI): 512.3[M+1]⁺. ¹H NMR (400 MHz, DMSO-d6) δ: 8.73 (d, J = 7.7 Hz, 1H), 8.67 (s, 1H), 8.61 - 8.50 (m, 1H), 8.35 (s, 1H), 7.21 (t, J = 53.2 Hz, 1H), 6.80 - 6.33 (m, 1H), 5.30 - 4.98 (m, 1H), 4.74 (s, 1H), 4.55 - 4.48 (m, 1H), 4.32 - 4.24 (m, 1H), 3.84 - 3.81 (m, 1H), 3.79 - 3.68 (m, 1H), 3.60 - 3.40 (m, 2H), 3.30 - 3.26 (m, 2H), 2.17 - 2.11 (m, 2H), 1.99 - 1.87 (m, 4H), 1.84 - 1.75 (m, 2H), 1.49 - 1.41 (m, 1H), 1.17 - 1.07 (m, 2H).

### Example 48

### Preparation of ((1r,4r)-4-(4-(4-(5-(((R)-3-aminopiperidin-1-yl)pyrazolo[1,5-a]pyrimidin-3-yl)-1H-1,2,3-triazol-1-yl)-3-(difluoromethyl)-1H-pyrazol-1-yl]cyclohexyl)methanol (106A)

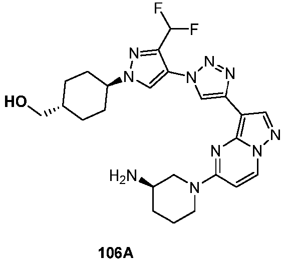

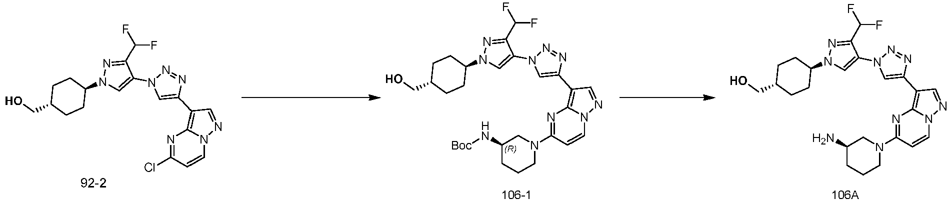

### 1) Step 1 Preparation of compound 106-1

At room temperature, to a solution of compound 92-2(50.0 mg, 0.11 mmol) in acetonitrile (1 mL), N,N-diisopropylethylamine (43.1 mg, 0.33 mmol), compound (R)-3-Boc-aminopiperidine (44.6 mg, 0.22 mmol) were added. After reacting at 80°C with stirring for 3 h, the reaction solution was concentrated under reduced pressure. The residue was purified by silica gel column chromatography with an elution system (dichloromethane/methanol=100/1 to 10/1) to obtain the title compound 106-1 (30.0 mg). MS m/z(ESI): 613.8[M+1]⁺.

### 2) Step 2 Preparation of compound 106A

Compound 106-1(30.0 mg, 0.05 mmol) was dissolved with a solution of hydrogen chloride in dioxane (1 mL, 4M). The reaction solution was stirred at 25°C for 1 h, and then evaporated to dryness under reduced pressure. The residue was purified by HPLC (Waters-2545, chromatographic column: SharpSil-T C18,30*150mm, 5µm; mobile phase: water (containing 10mmol/L formic acid) and acetonitrile, gradient ratio: acetonitrile 38%-45%, flow rate: 30mL/min) to obtain compound 106A (21.3 mg). MS m/z(ESI): 514.0[M+1]⁺.

### Example 49

### Preparation of (1R,2S)-2-((3-(1-(3-(difluoromethyl)-1-((1R,4S)-4-(methylol)cyclohexyl)-1H-pyrazol-4-yl)-1H-1,2,3-triazol-4-yl]pyrazolo[1,5-a]pyrimidin-5-yl)amino)cyclohexan-1-ol (107A)

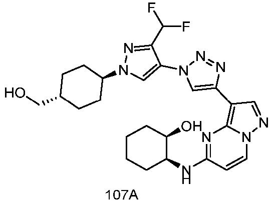

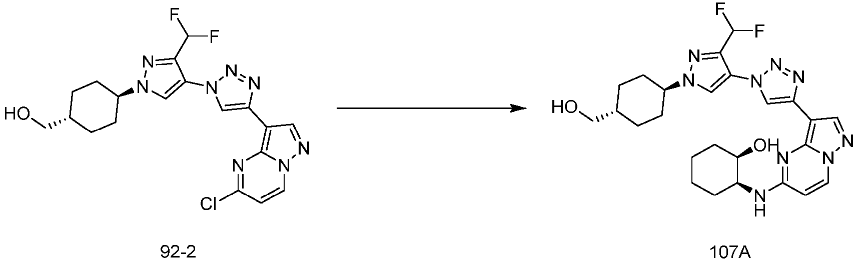

### 1) Step 1 Preparation of compound 107A

Compound 92-2 (50.0mg, 0.11mmol) was dissolved with acetonitrile (3mL), to which (1R,2S)-2-aminocyclohexanol hydrochloride (33.4mg, 0.22mmol, Bide), N,N-diisopropylethylamine (43.2mg, 0.33mmol) were added. After reacting at 80°C with stirring for 2 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 107A (7mg). MS m/z(ESI): 528.3[M+1]⁺. ¹H NMR (400 MHz, DMSO-d6) δ: 8.68 (s, 1H), 8.49 (d, J = 7.6 Hz, 1H), 8.43 (s, 1H), 8.26 (s, 1H), 7.48 (d, J = 7.6 Hz, 1H), 7.23 (t, J = 53.1 Hz, 1H), 6.50 (d, J = 7.6 Hz, 1H), 4.74 - 4.67 (m, 1H), 4.51 (t, J = 5.4 Hz, 1H), 4.32 - 4.23 (m, 1H), 4.13 - 4.05 (m, 1H), 3.95 (s, 1H), 3.27 (t, J = 5.6 Hz, 2H), 2.17 - 2.11 (m, 2H), 1.91 - 1.85 (m, 2H), 1.82 - 1.70 (m, 4H), 1.63 - 1.52 (m, 4H), 1.47 - 1.41 (m, 1H), 1.37 - 1.29 (m, 2H), 1.17 - 1.08 (m, 2H).

### Example 50

### Preparation of ((1r,4r)-4-(4-(4-(5-(5-amino-3,3-difluoropiperidin-1-yl)pyrazolo[1,5-a]pyrimidin-3-yl)-1H-1,2,3-triazol-1-yl)-3-(difluoromethyl)-1H-pyrazol-1-ylcyclohexyl)methanol (108A)

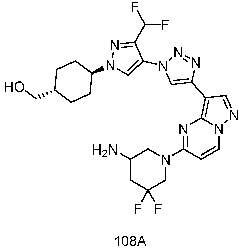

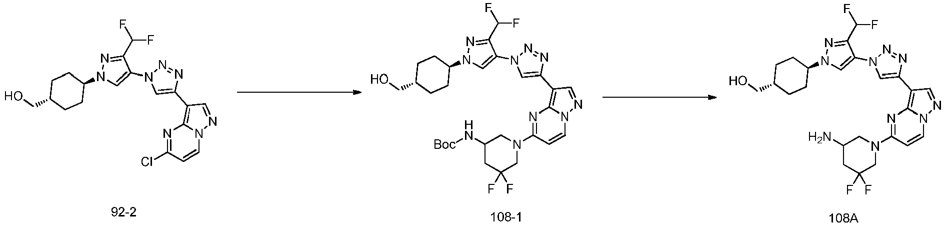

### 1) Step 1 Preparation of compound 108-1

Compound 92-2 (100mg, 0.22mmol) was dissolved with acetonitrile (5mL), to which tert-butyl N-[5,5-difluoropiperidin-3-yl]carbamate (105mg, 0.45mmol, Jiangsu Aikon), N,N-diisopropylethylamine (86.4mg, 0.67mmol) were added. After reacting at 80°C with stirring for 2 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 108-1 (100mg). MS m/z(ESI): 649.5[M+1]⁺.

### 2) Step 2 Preparation of compound 108A

Compound 108-1 (100mg, 0.15mmol) was dissolved with dichloromethane (5mL), to which a solution of hydrogen chloride in dioxane (5mL, 4M) was added. After reacting at room temperature with stirring for 1 h, the reaction solution was directly concentrated under reduced pressure. The residue was purified by HPLC (Waters-2545, chromatographic column: SharpSil-TC18, 30*150mm, 5µm; mobile phase: water (containing 10mmol/L formic acid) and acetonitrile, gradient ratio: acetonitrile 38%-45%, flow rate: 30mL/min), to obtain compound 108A (25mg). MS m/z(ESI): 549.5[M+1]⁺. ¹H NMR (400 MHz, DMSO-d6) δ: 8.82 (d, J = 7.8 Hz, 1H), 8.70 (s, 1H), 8.58 (s, 1H), 8.41 (s, 1H), 7.23 (t, J = 53.3 Hz, 1H), 6.92 (d, J = 7.9 Hz, 1H), 4.80 - 4.44 (m, 3H), 4.34 - 4.21 (m, 3H), 3.68 - 3.55 (m, 1H), 3.28 (d, J = 6.1 Hz, 2H), 3.12 - 2.99 (m, 2H), 2.45 - 2.35 (m, 1H), 2.19 - 2.11 (m, 2H), 2.02 - 1.86 (m, 3H), 1.85 - 1.74 (m, 2H), 1.51 - 1.41 (m, 1H), 1.20 - 1.06 (m, 2H).

### Example 51

### Preparation of ((1r,4r)-4-(3-(difluoromethyl)-4-(4-(5-((2S,6S)-2,6-dimethylmorpholinyl)pyrazolo[1,5-a]pyrimidin-3-yl)-1H-1,2,3-triazol-1-yl)-1H-pyrazol-1-yl cyclohexyl)methanol (109A)

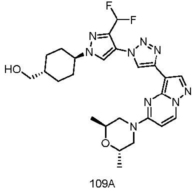

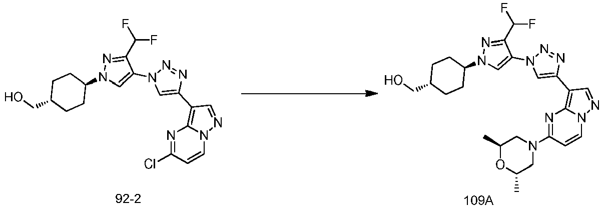

### 1) Step 1 Preparation of compound 109A

To a solution of compound 92-2 (45.0mg, 0.100mmol) in acetonitrile (2mL), (2S,6S)-2,6-dimethylmorpholine (23.0mg, 0.200mmol) and N,N-diisopropylethylamine (52.0mg, 0.400mmol) were added. After reacting at 80°C with stirring under nitrogen atmosphere for 2 h, the reaction solution was concentrated under reduced pressure, and then purified by HPLC (Gilson_306_1741, chromatographic column: Waters-SunFire-C18-10µm-19*250mm, mobile phase: water (containing 10mmol/L ammonium bicarbonate) and acetonitrile, gradient ratio: acetonitrile 30%-95%, flow rate: 25mL/min) to obtain compound 109A (35.0mg). MS m/z(ESI): 528.3 [M+1]⁺. ¹H NMR (400 MHz, DMSO-d6) δ 8.74 (d, J = 8.0 Hz, 1H), 8.68 (s, 1H), 8.52 (s, 1H), 8.36 (s, 1H), 7.23 (t, J = 53.2 Hz, 1H), 6.82 (d, J = 8.0 Hz, 1H), 4.50 (t, J = 5.2 Hz, 1H), 4.33 - 4.21 (m, 1H), 4.09 - 3.99 (m, 2H), 3.87 (d, J = 12.8 Hz, 2H), 3.55 - 3.43 (m, 2H), 3.27 (t, J = 5.6 Hz, 2H), 2.13 (d, J = 9.6 Hz, 2H), 1.96 - 1.72 (m, 4H), 1.45 (s, 1H), 1.22 - 1.07 (m, 8H).

### Example 52

### Preparation of ((1r,4r)-4-(3-(difluoromethyl)-4-(4-(5-(piperazin-1-yl)pyrazolo[1,5-a]pyrimidin-3-yl)-1H-1,2,3-triazol-1-yl)-1H-pyrazol-1-ylcyclohexyl)methanol (110A)

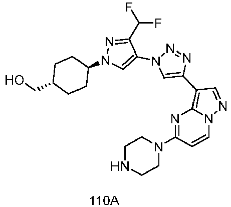

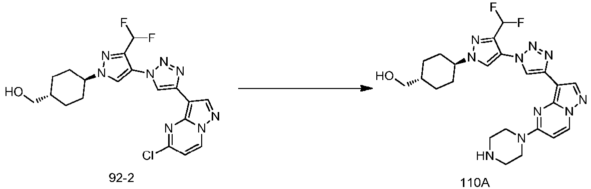

### 1) Step 1 Preparation of compound 110A

To a solution of 92-2 (50.0mg, 0.110mmol) in acetonitrile (2mL), piperazine (48.0mg, 0.56 mmol) and N,N-diisopropylethylamine (144mg, 1.11mmol) were added. After reacting at 80°C with stirring under nitrogen atmosphere for 2 h, the reaction solution was concentrated under reduced pressure, and then purified by HPLC (Gilson_306_1741, chromatographic column: Waters-SunFire-C18-10µm-19*250mm; mobile phase: water (containing 10mmol/L ammonium bicarbonate) and acetonitrile, gradient ratio: acetonitrile 20%-95%, flow rate: 25mL/min) to obtain compound 110A (35.0mg). MS m/z(ESI): 499.2 [M+1]⁺. ¹H NMR (400 MHz, DMSO-d6) δ 8.73 - 8.64 (m, 2H), 8.52 (s, 1H), 8.35 (s, 1H), 7.21 (t, J = 53.2 Hz, 1H), 6.78 (d, J = 8.0 Hz, 1H), 4.51 (t, J = 5.2 Hz, 1H), 4.35 - 4.18 (m, 1H), 3.67 (s, 4H), 3.27 (t, J = 5.4 Hz, 2H), 2.86 - 2.74 (m, 4H), 2.13 (d, J = 9.8 Hz, 2H), 1.95 - 1.72 (m, 4H), 1.53 - 1.38 (m, 1H), 1.19 - 1.03 (m, 2H).

### Example 53

### Preparation of (1S,2R)-2-((3-(1-(3-(difluoromethyl)-1-((1r,4R)-4-(methylol)cyclohexyl)-1H-pyrazol-4-yl)-1H-1,2,3-triazol-4-yl]pyrazolo[1,5-a]pyrimidin-5-yl)amino)cyclohexan-1-ol (111A)

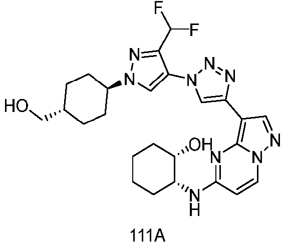

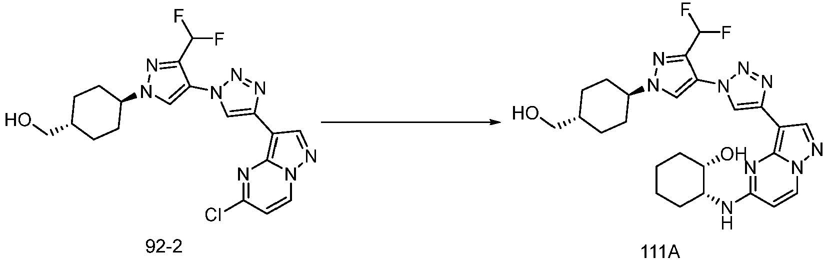

### 1) Step 1 Preparation of compound 111A

To a solution of 92-2 (100mg, 0.220mmol) in acetonitrile (2mL), (1S,2R)-2-aminocyclohexanol hydrochloride (101mg, 0.670mmol) and N,N-diisopropylethylamine (173mg, 1.34mmol) were added. After reacting at 80°C with stirring under nitrogen atmosphere for 2 h, the reaction solution was concentrated under reduced pressure, and then purified by HPLC (Gilson_306_1741, chromatographic column: Waters-SunFire-C18-10µm-19*250mm; mobile phase: water (containing 10mmol/L ammonium bicarbonate) and acetonitrile, gradient ratio: acetonitrile 42%-95%, flow rate: 20mL/min) to obtain the title compound 111A (50.0mg).
MS m/z(ESI): 528.3 [M+1]⁺. ¹H NMR (400 MHz, DMSO-d6) δ 8.67 (s, 1H), 8.49 (d, J = 7.6 Hz, 1H), 8.43 (s, 1H), 8.26 (s, 1H), 7.46 (d, J = 7.4 Hz, 1H), 7.22 (t, J = 53.2 Hz, 1H), 6.49 (d, J = 7.6 Hz, 1H), 4.70 (d, J = 4.0 Hz, 1H), 4.50 (t, J = 5.2 Hz, 1H), 4.33 - 4.21 (m, 1H), 4.15 - 4.04 (m, 1H), 3.95 (s, 1H), 3.27 (t, J = 5.6 Hz, 2H), 2.14 (d, J = 9.8 Hz, 2H), 1.89 (d, J = 12.2 Hz, 2H), 1.84 - 1.67 (m, 4H), 1.66 - 1.51 (m, 4H), 1.49 - 1.40 (m, 1H), 1.38 - 1.26 (m, 2H), 1.19 - 1.05 (m, 2H).

### Example 54

### Preparation of (S)-1-(3-(1-(3-(difluoromethyl)-1-((1r,4r)-4-(methylol)cyclohexyl)-1H-pyrazol-4-yl)-1H-1,2,3-triazol-4-yl]pyrazolo[1,5-a]pyrimidin-5-yl)piperidin-3-ol(112A)

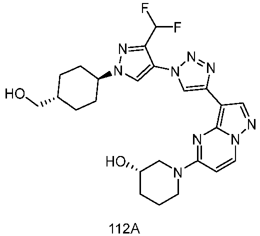

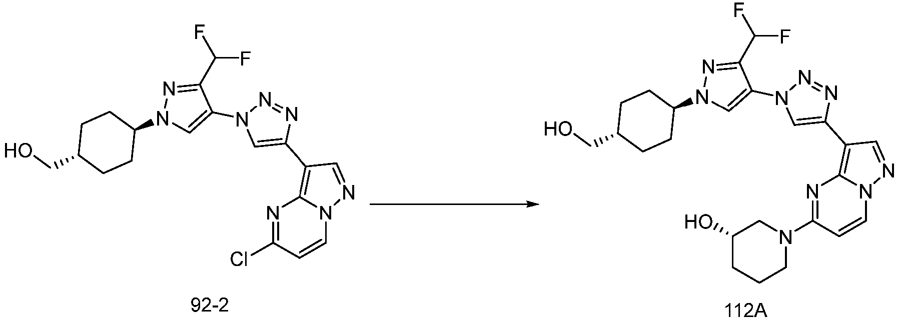

### 1) Step 1 Preparation of compound 112A

At room temperature, to a single-necked bottle, compound 92-2 (50.0mg, 0.110mmol), (S)-3-hydroxypiperidine hydrochloride (18.3mg, 0.170mmol) and acetonitrile (1mL) were added in sequence, and N,N-diisopropylethylamine (27.3µL, 0.170mmol) was added with stirring. After replacement of the atmosphere with nitrogen three times, the reaction solution was reacted at 80°C for 18 h. The reaction solution was purified by HPLC (Gilson_306_1741, chromatographic column: Waters-Xbridge-C18-10µm-19*250mm, mobile phase: water (containing 26.5mmol/L formic acid) and acetonitrile, gradient ratio: acetonitrile 21%-95%, flow rate: 25mL/min) to obtain compound 112A (10.1mg). MS m/z(ESI): 514.2[M+1]⁺. ¹H NMR (400 MHz, DMSO-d6) δ 8.67 - 8.65 (m, 2H) 8.49 (s, 1H), 8.34 (s, 1H), 7.20 (t, J = 53.2 Hz, 1H), 6.77 (d, J = 7.9 Hz, 1H), 4.89 (d, J = 4.2 Hz, 1H), 4.51 (t, J = 5.3 Hz, 1H), 4.32 - 4.22 (m, 1H), 4.15 - 4.03 (m, 1H), 4.00 (d, J = 13.3 Hz, 1H), 3.63 - 3.53 (m, 1H), 3.49 - 3.39 (m, 1H), 3.27 (t, J = 5.8 Hz, 2H), 3.24 - 3.14 (m, 1H), 2.19 - 2.08 (m, 2H), 1.93 - 1.75 (m, 6H), 1.52 - 1.40 (m, 3H), 1.18 - 1.05 (m, 2H).

### Example 55

### Preparation of (R)-1-(3-(1-(3-(difluoromethyl)-1-((1r,4r)-4-(methylol) cyclohexyl)-1H-pyrazol-4-yl)-1H-1,2,3-triazol-4-yl]pyrazolo[1,5-a]pyrimidin-5-yl) piperidin-3-ol (113A)

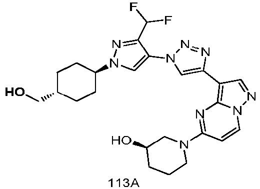

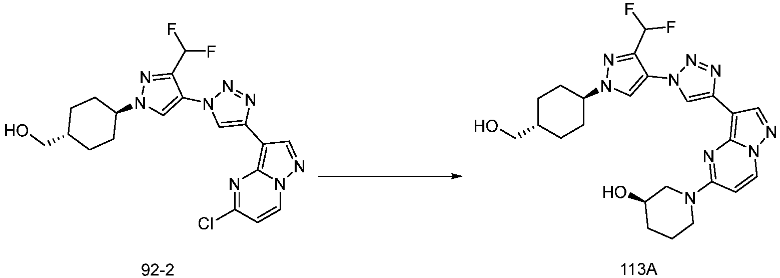

### 1) Step 1 Preparation of compound 113A

At room temperature, to a single-necked bottle, compound 92-2 (50.0mg, 0.110mmol), (R)-3-hydroxypiperidine hydrochloride (17.0mg, 0.170mmol) and acetonitrile (2mL) were added in sequence, and N,N-diisopropylethylamine (60.0 µL, 0.39 mmol) was added with stirring. After replacement of the atmosphere with nitrogen three times, the reaction solution was reacted at 80°C for 1 h, and then filtered. The filtrate was purified by HPLC (A: 0.1% FA/H2O B: ACN, chromatographic column: Wetch-Ultimate-XB-C18-10µm-21.2*150mm; mobile phase: water (containing 0.1% formic acid) and acetonitrile, gradient ratio: acetonitrile 25%-45%, flow rate: 25mL/min) to obtain compound 113A (30 mg). MS m/z(ESI): 514.2 [M+1]⁺. ¹H NMR (400 MHz, DMSO-d6): δ 8.71 - 8.63 (m, 2H), 8.50 (s, 1H), 8.34 (s, 1H), 7.20 (t, J = 52.8 Hz, 1H), 6.78 (d, J = 8.0 Hz, 1H), 4.96 - 4.85 (m, 1H), 4.58 - 4.45 (m, 1H), 4.34 - 4.22 (m, 1H), 4.15 - 3.93 (m, 2H), 3.66 - 3.54 (m, 1H), 3.47 - 3.39 (m, 1H), 3.30 - 3.25 (m, 2H), 3.23 - 3.16 (m, 1H), 2.19 - 2.09 (m, 2H), 1.94 - 1.74 (m, 6H), 1.55 - 1.40 (m, 3H), 1.19 - 1.05 (m, 2H).

### Test examples

### Test example 1. IRAK4 kinase inhibition assay:

In this assay, Cisbio HTRF KinEASE-STK SI kit (62S 1 PEB) was used, in which assay/detection buffer, STK Substrate 1-biotin, Streptavidin-XL665, STK Antibody-Cryptate were contained.

After adding compound/DMSO to a 384-well plate (PerkinElmer 6008280) using Echo 665, 5 µL of 2-fold reaction concentration of IRAK4 kinase protein (final concentration: 10 nM) dissolved with assay buffer [1 volume of 5X kinase buffer + 20mM magnesium chloride (Sigma) solution + 1mM DTT (Alfa Aesar) solution + 4 volumes deionized water] was added, followed by sealing the shallow well plate, shaking and then centrifuging at 1000rpm for 1 min, and incubating at room temperature for 5 min; then 5 µL of 2-fold reaction concentration of a 1: 1 mixture of STK Substrate 1-biotin (final concentration: 1 µM) and ATP (Sigma A1852) (final concentration: 200 µM) formulated with assay buffer was added, followed by sealing the shallow well plate, shaking and then centrifuging at 1000rpm for 1 min, and incubating at 37°C for 2 h, and then 10 µL of a 1: 1 mixture of Streptavidin-XL665 (final concentration: 62.5 nM) and STK-antibody-cryptate dissolved with detection buffer was added, followed by sealing the shallow well plate, shaking and then centrifuging at 1000rpm for 1 min, and incubating at room temperature for 1 h, and thereafter reading the value at the wavelength of 620nm/665nm using a microplate reader. The data were analyzed using Prism 9.3 software, and the IC₅₀ value of IRAK4 kinase inhibition was fitted using a four parameter Logistic equation.

The experimental results show that the compounds of the present invention have excellent inhibitory activity against IRAK4 kinase, and the IC₅₀ values of representative compounds of the present invention are shown in the following table.

**Table 1 IRAK4 kinase inhibitory activity of representative compounds of the present invention**

| Compound No. | IC50 (nM) |
|---|---|
| 1A | C |
| 3A | C |
| 4A | D |
| 43A | B |
| 45A | B |
| 46A | B |
| 56A | B |
| 60A | D |
| 61A | C |
| 64A | B |
| 65A | C |
| 66A | B |
| 67A | A |
| 68A | B |
| 69A | C |
| 72A | D |
| 73A | C |
| 79A | C |
| 80A | C |
| 82A | C |
| 83A | C |
| 84A | C |
| 85A | D |
| 86A | C |
| 87A | C |
| 88A | C |
| 89A | C |
| 90A | C |
| 91A | C |
| 92A | D |
| 93A | D |
| 94A | D |
| 95A | C |
| 96A | C |
| 97A | D |
| 98A | D |
| 99A | C |
| 100A | D |
| 101A | C |
| 102A | C |
| 103A | D |
| 104A | D |
| 105A | C |
| 106A | D |
| 107A | D |
| 108A | D |
| 109A | D |
| 111A | D |
| 112A | D |
| 113A | D |

In Table 1, A represents >5000nM; B represents 1000-5000nM; C represents 100-1000nM; and D represents <100nM.

### Test example 2. Method for detecting protein degradation by HiBit fluorescence:

Using HiBiT tag technology, HiBiT-containing tag was connected to IRAK4 plasmid, the plasmid was transfected into HEK293T cells, and the HiBiT fluorescence level was detected to characterize the degradation level of the compound for IRAK4. 1mL of fresh medium, 300000 HEK293T cells were seeded onto a 6-well plate (Corning 3516) and placed overnight in a constant temperature incubator at 37°C and 5% CO₂. 3 µL of X-tremeGENE^{™} 9 DNA Transfection Reagent (Roche # 06365787001), 1 µg of pcdna3.1-IRAK4-HiBiT were added to 200 µL of Opti-MEM (Gibico 31985062) and mixed gently, the overnight incubated 6-well plate was taken out, and left to stand at room temperature for 15 min, and then the obtained mixture was added dropwise to the 6-well plate. After 6 h of transient transfection, cells were digested with trypsin, and centrifuged at 1000rpm for 3 min, and then 5000 HEK293T cells were seeded onto a 384-well plate (Corning 3765) with 40 µL of fresh medium, and placed overnight in a constant temperature incubator at 37°C and 5% CO₂. Then, 40 nL of compound was added to the 384-well plate using an Echo 655 Liquid Handler at a maximum final concentration of 10 µM, in a 1:3 dilution series for a total of 10 concentrations, and incubated at 37°C and 5% CO₂ for 24 h. The 384-well plate was taken out and allowed to equilibrate to room temperature. 40 µL of Nano-Glo^{®} HiBiT Lytic Detection System (Promega N3030) was added to each well, followed by incubating at room temperature for 15 min, and recording the fluorescence value using EnVision Xcite Multilabel Reader (PerkinElmer 2105-0020). The data were analyzed using Prism 9.3 software, and the DC₅₀ value of IRAK4 degradation was fitted using a four parameter Logistic equation.

The experimental results show that the PROCAC molecules comprising the compounds of the present invention have excellent degradation levels for IRAK4.

## Claims

1. A compound represented by formula A, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof, 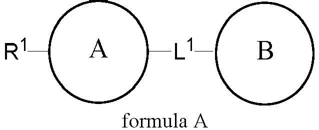 wherein:
A is selected from 5-6 membered heteroaromatic ring optionally substituted with substituent R^{k}; preferably, A is selected from the group consisting of 5 membered heteroaromatic ring, and 6 membered heteroaromatic ring, the 5 membered heteroaromatic ring containing 2-3 heteroatoms, wherein at least two heteroatoms are N atoms, the 6 membered heteroaromatic ring containing 1-2 heteroatoms, wherein at least one heteroatom is N atom, and, the 5 membered heteroaromatic ring and 6 membered heteroaromatic ring are optionally substituted with substituent R^{k}; further preferably, A is selected from the group consisting of 1,2,3-triazole ring, 1,2,4-triazole ring, pyrazole ring, imidazole ring, 1,3,4-thiadiazole ring, 1,3,4-oxadiazole ring, 2-pyridone, and pyridazine ring, wherein, the 2-pyridone is optionally substituted with substituent R^{k};
further preferably, A is selected from the group consisting of 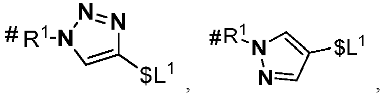 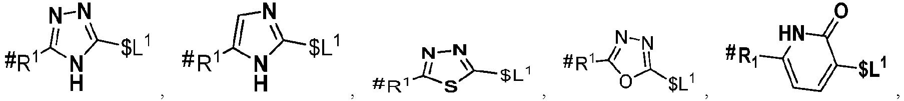 and 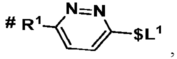 wherein, the 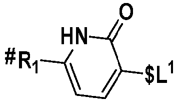 is optionally substituted with substituent R^{k}, wherein #R¹ represents the attachment point to R¹, $L¹ represents the attachment point to L¹;
further preferably, A is selected from the group consisting of 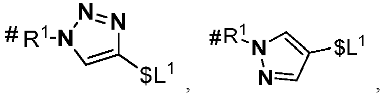 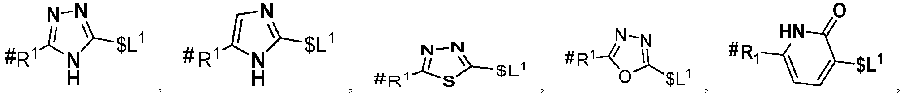 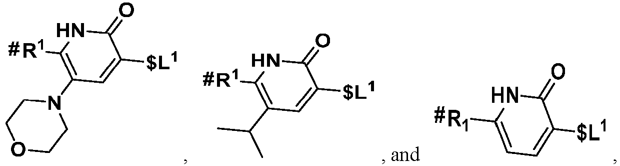 wherein #R¹ represents the attachment point to R¹, $L¹ represents the attachment point to L¹;
further preferably, A is selected from the group consisting of 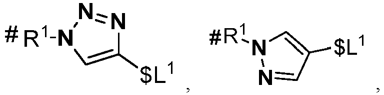 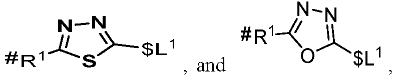 wherein #R¹ represents the attachment point to R¹, $L¹ represents the attachment point to L¹;
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl; preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl; further preferably, R^{k} is selected from the group consisting of isopropyl, cyclopropyl, dimethylamino, difluoromethyl, and 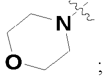
R^{p}, R^{q} are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
B is selected from the group consisting of 6-10 membered heteroaryl, and 6-10 membered aryl, and, B is optionally substituted with at least one of substituents R^{a}, R^{b}; preferably, B is substituted with at least one of substituents R^{a}, R^{b}; preferably, B is selected from the group consisting of 9 membered heteroaryl, pyridinyl, phenyl, and pyrimidinyl, and, B is substituted with at least one of substituents R^{a}, R^{b}; preferably, the 9 membered heteroaryl contains 1-4 heteroatoms selected from the group consisting of N, O, and S atoms; preferably, B is selected from the group consisting of 9 membered heteroaryl, pyridinyl, and phenyl, and, B is substituted with at least one of substituents R^{a}, R^{b}; preferably, the 9 membered heteroaryl contains 1-4 heteroatoms selected from the group consisting of N, O, and S atoms;
further preferably, B is selected from the group consisting of 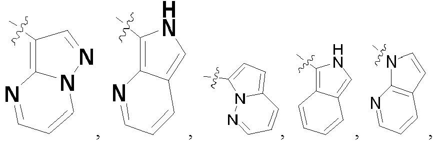 pyridinyl, phenyl, and pyrimidinyl, and, B is substituted with at least one of substituents R^{a}, R^{b};
further preferably, B is selected from the group consisting of 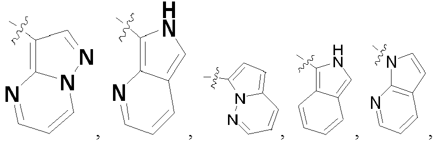 pyridinyl, and phenyl, and, B is substituted with at least one of substituents R^{a}, R^{b};
further preferably, B is selected from the group consisting of 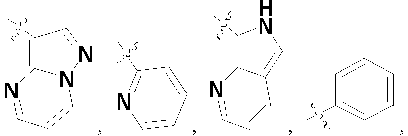 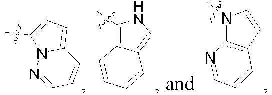 and, B is substituted with substituents R^{a}, R^{b};
further preferably, B is selected from the group consisting of 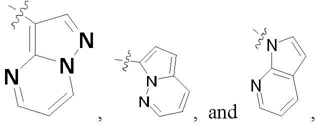 and, B is substituted with substituents R^{a}, R^{b};
R^{a} is selected from the group consisting of H, halogen, cyano, carboxyl, 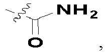 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, 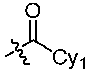 (wherein Cy₁ is 4-9 membered heterocyclyl optionally substituted with R_{cy1} and R_{cy2}), -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), and 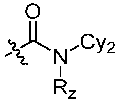 (wherein R_{z} is H or C1-C6 alkyl, Cy₂ is 5-7 membered heterocyclyl, C3-C7 cycloalkyl or C6-C10 aryl, wherein, the 5-7 membered heterocyclyl, C3-C7 cycloalkyl and C6-C10 aryl are optionally substituted with 1-3 substituents selected from the group consisting of halogen, C1-C6 alkyl, C1-C6 alkoxy, CN, and hydroxy), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl);
preferably, Cy₁ is 5-7 membered heterocyclyl optionally substituted with R_{cy1} and R_{cy2}, further preferably, Cy₁ is 6 membered heterocyclyl optionally substituted with R_{cy1} and R_{cy2}, further preferably, Cy₁ is morpholinyl, piperazinyl, or thiomorpholinyl, optionally substituted with R_{cy1} and R_{cy2};
R_{cy1} and R_{cy2} are each independently selected from the group consisting of C1-C6 alkyl, C1-C6 alkoxy, C1-C6 haloalkyl, halogen, hydroxy, nitro, cyano, amino,-NH(C1-C6 alkyl),-N(C1-C6 alkyl)(C1-C6 alkyl), and oxo;
preferably, R_{cy1} and R_{cy2} are each independently selected from the group consisting of C1-C6 alkyl, halogen, hydroxy, amino, and oxo, further preferably R_{cy1} and R_{cy2} are oxo;
preferably, Cy₂ is 5-7 membered heterocyclyl optionally substituted with 1-3 substituents selected from the group consisting of halogen, C1-C6 alkyl, C1-C6 alkoxy, CN, and hydroxy; further preferably, Cy₂ is selected from the group consisting of azetidinyl, pyrrolidinyl, imidazolidinyl, piperidyl, piperazinyl, and morpholinyl, wherein, the azetidinyl, pyrrolidinyl, imidazolidinyl, piperidyl, piperazinyl, and morpholinyl are optionally substituted with 1-3 substituents selected from the group consisting of halogen, and C1-C6 alkyl; further preferably, Cy₂ is selected from the group consisting of pyrrolidinyl, imidazolidinyl, and piperidyl, wherein, the pyrrolidinyl, imidazolidinyl, and piperidyl are optionally substituted with 1-3 substituents selected from the group consisting of F, Cl, Br, and methyl; further preferably, Cy₂ is pyrrolidinyl optionally substituted with 1-3 (such as 1 or 2 or 3) F;
preferably, R^{a} is selected from the group consisting of H, halogen, amino, cyano, carboxyl, 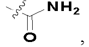 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of hydroxy, C3-C6 cycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, halogen, hydroxy, hydroxy-C1-C6 alkyl-, cyano, oxo, - NH₂, -N(C1-C6 alkyl), and -N(C1-C6 alkyl)(C1-C6 alkyl);
preferably, R^{a} is selected from the group consisting of H, cyano, carboxyl, 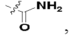 C1-C6 haloalkyl (such as trifluoromethyl), 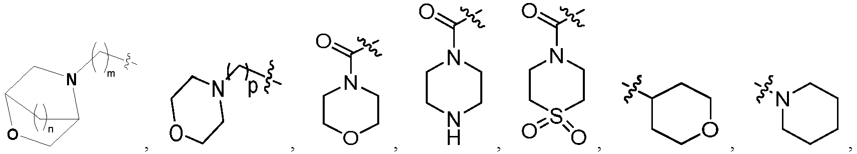 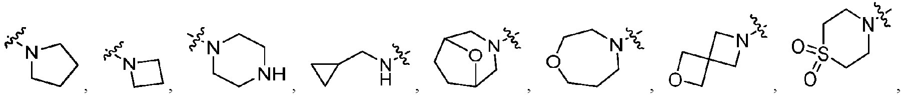 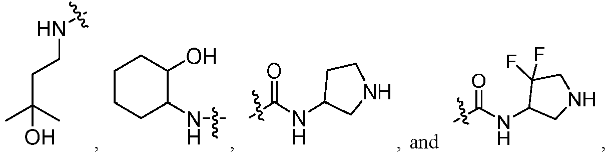 wherein, the 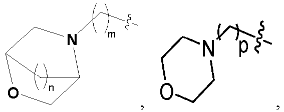 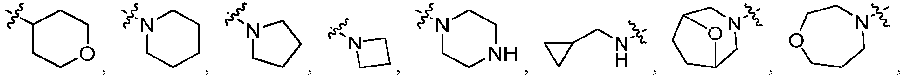 and 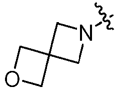 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl);
preferably, R^{a} is selected from the group consisting of H, cyano, carboxyl, 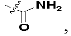 C1-C6 haloalkyl (such as trifluoromethyl), 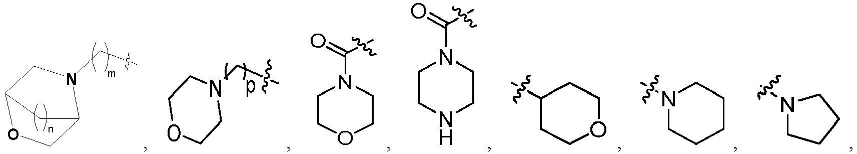 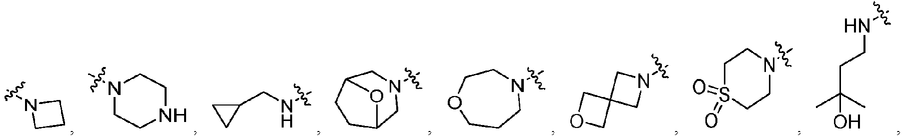 and 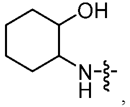 wherein, the 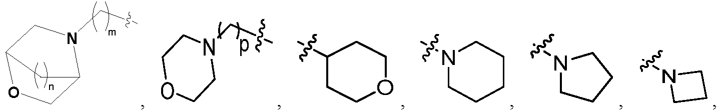 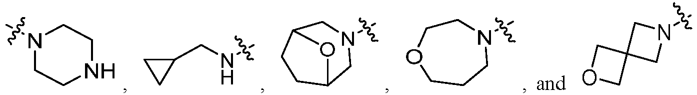 are optionally substituted with 1-3 substituents selected from the group consisting of C1-C6 alkyl, halogen, hydroxy, cyano, -NH₂, -N(C1-C6 alkyl), and - N(C1-C6 alkyl)(C1-C6 alkyl);
preferably, R^{a} is selected from the group consisting of H, cyano, 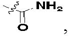 C1-C6 haloalkyl (such as trifluoromethyl), and 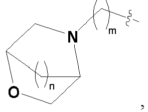 wherein, the 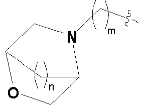 is optionally substituted with C1-C6 alkyl (preferably methyl), F, Cl, Br;
m is selected from the group consisting of 0, 1, 2, and 3; preferably, m is selected from the group consisting of 0, and 1;
n is selected from the group consisting of 0, 1, 2, and 3; preferably, n is selected from the group consisting of 0, and 1;
p is selected from the group consisting of 0, 1, 2, and 3; preferably, p is selected from the group consisting of 0, and 1;
further preferably, m is 0, and n is 0;
further preferably, R^{a} is selected from the group consisting of H, cyano, carboxyl, 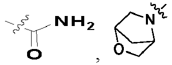 (preferably 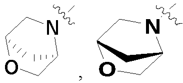 ), 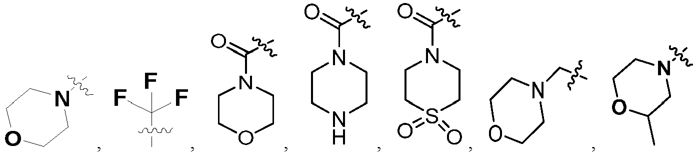 (preferably 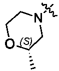 ), 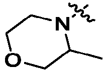 (preferably 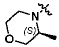 ), 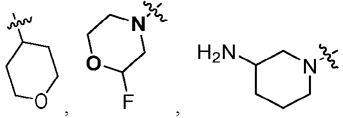 (preferably 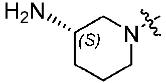 or 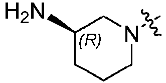 ), 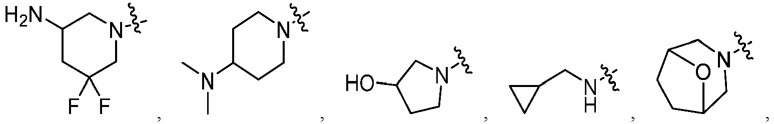 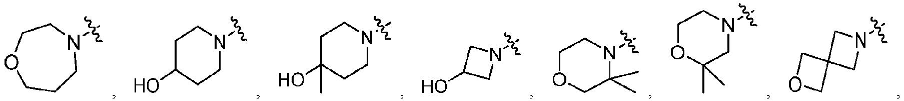 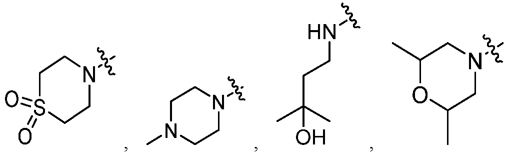 (preferably 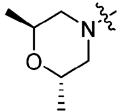 ), 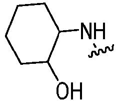 (preferably 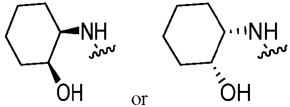 ), 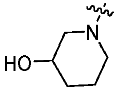 (preferably 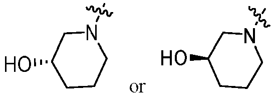 ), 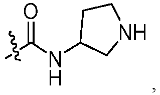 and 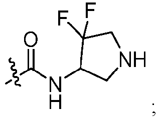
further preferably, R^{a} is selected from the group consisting of H, cyano, carboxyl, 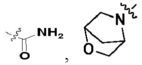 (preferably 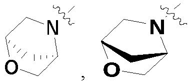 ), 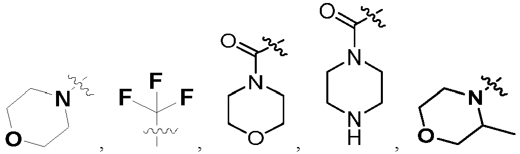 (preferably 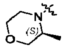 ), 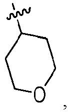 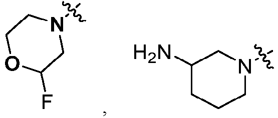 (preferably 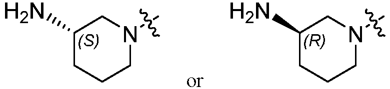 ), 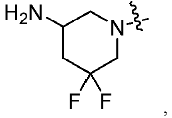 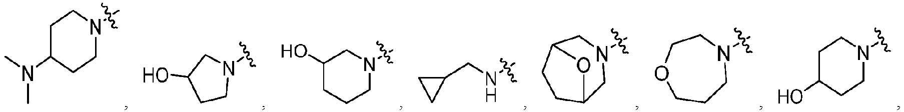 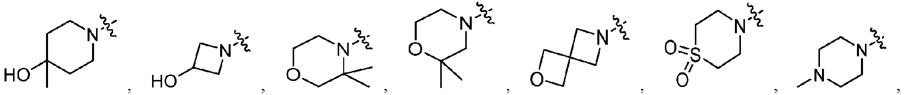 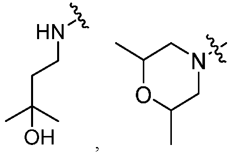 (preferably 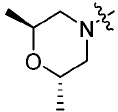 ), 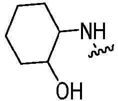 (preferably 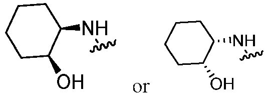 ), and 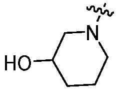 (preferably 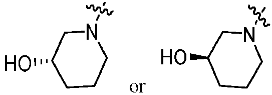 );
further preferably, R^{a} is selected from the group consisting of H, cyano, 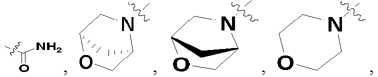 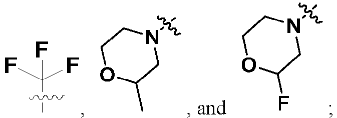
further preferably, R^{a} is selected from the group consisting of H, cyano, 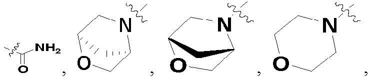 and 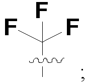
R^{b} is selected from the group consisting of H, halogen, amino, cyano, carboxyl, 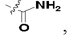 C1-C6 alkyl, C1-C6 haloalkyl (such as trifluoromethyl), and 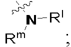 preferably, R^{b} is selected from the group consisting of H, cyano, 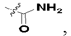 and 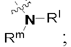
R^{l}, R^{m} are each independently selected from the group consisting of H, C1-C6 alkyl, and C3-C6 cycloalkyl; preferably, R^{l}, R^{m} are each independently selected from the group consisting of H, and C1-C6 alkyl;
or, R^{a}, R^{b} and the carbon atoms to which they are attached, respectively, together form a substituted or unsubstituted 5-membered heterocycle, wherein, the substituent is selected from C1-C6 alkyl (such as tert-butyl); preferably, R^{a}, R^{b} and the carbon atoms to which they are attached, respectively, together form 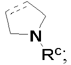
R^{c} is selected from the group consisting of H, and C1-C6 alkyl, preferably, R^{c} is selected from C1-C6 alkyl;
further preferably, B, as a whole, is selected from the group consisting of: 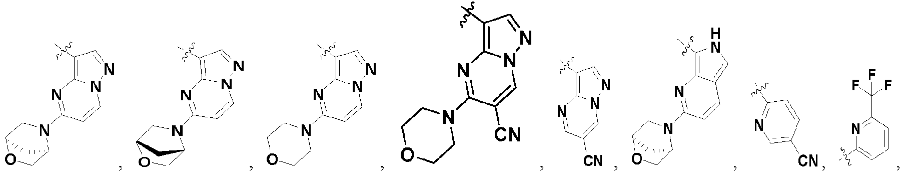 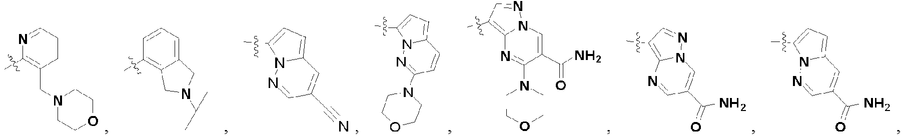 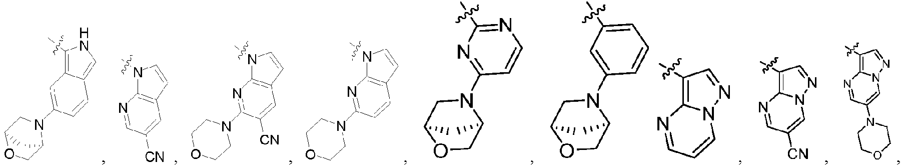 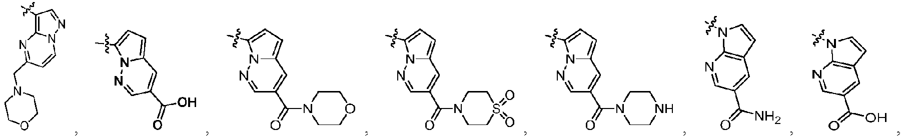 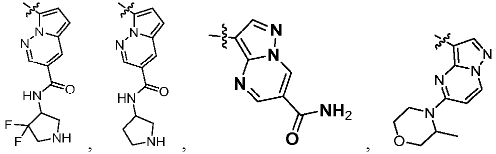 (preferably 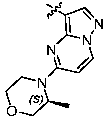 ), 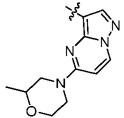 (preferably 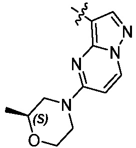 ), 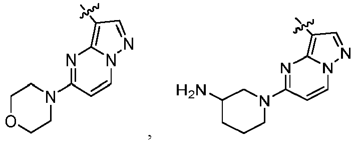 ( 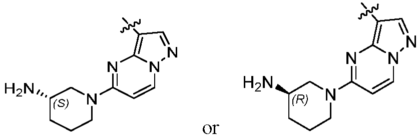 ), 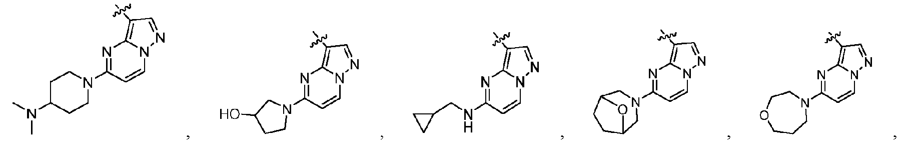 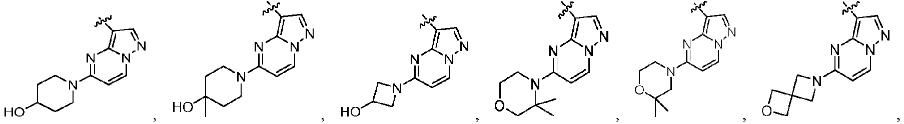 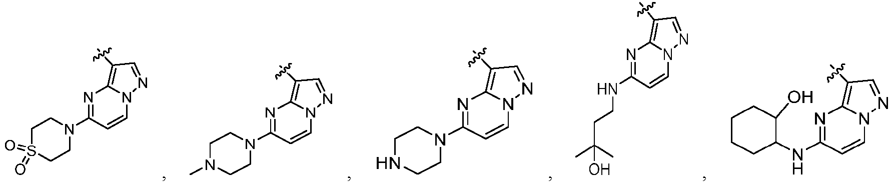 (preferably 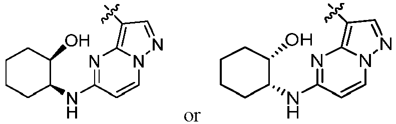 ), 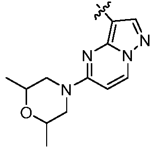 (preferably 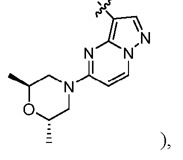 ), 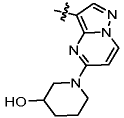 (preferably 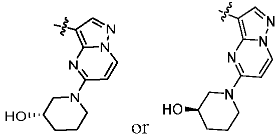 ), and 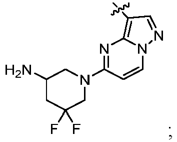
further preferably, B, as a whole, is selected from the group consisting of 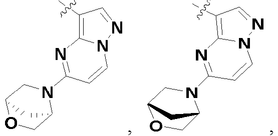 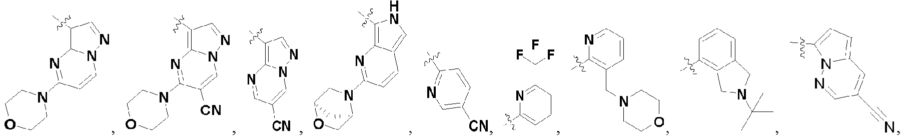 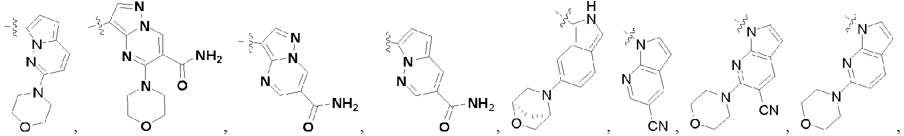 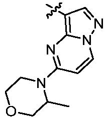 (preferably 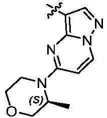 ), 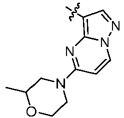 (preferably 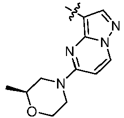 ), 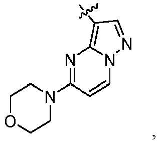 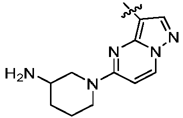 ( 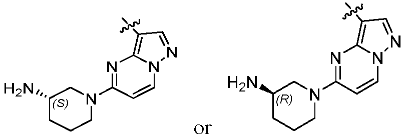 ), 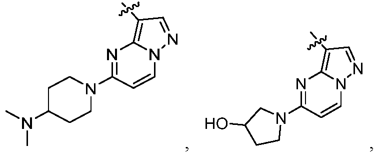 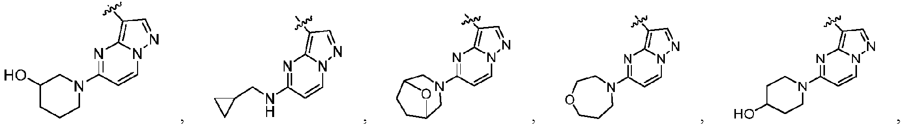 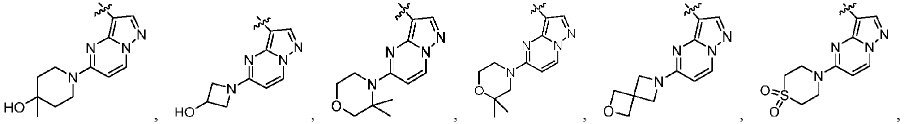 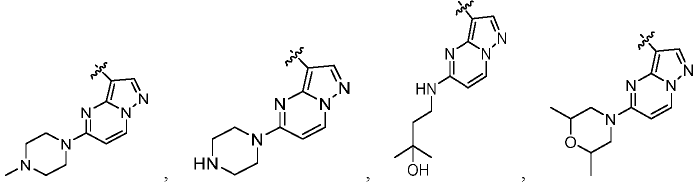 (preferably 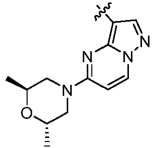 ), 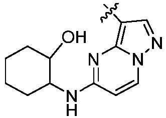 (preferably 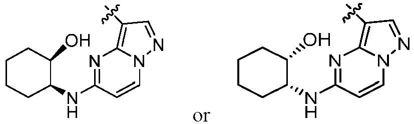 ), 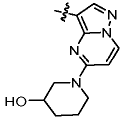 (preferably 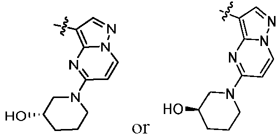 ), and 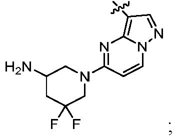
further preferably, B, as a whole, is selected from the group consisting of 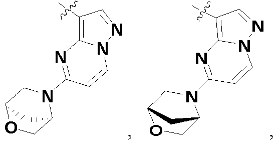 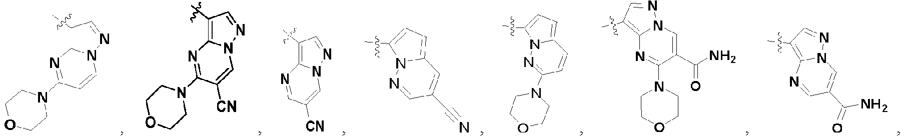 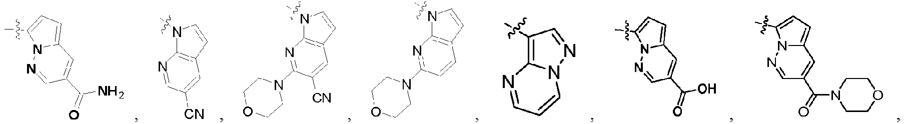 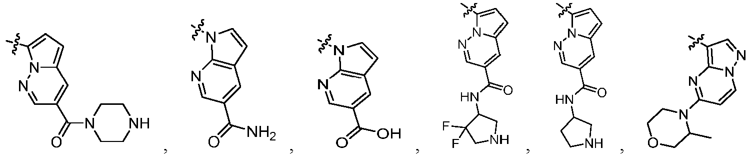 (preferably 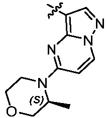 ), 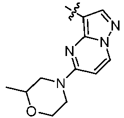 (preferably 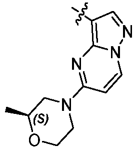 ), 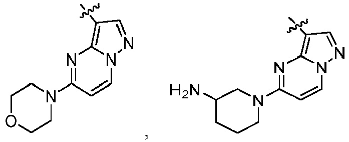 ( 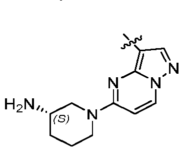 or 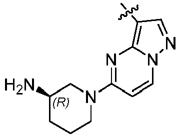 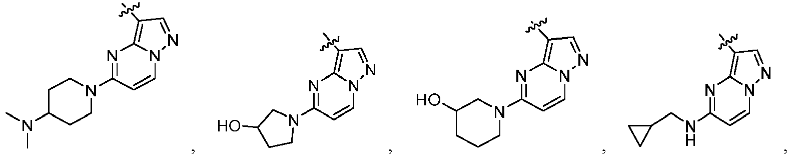 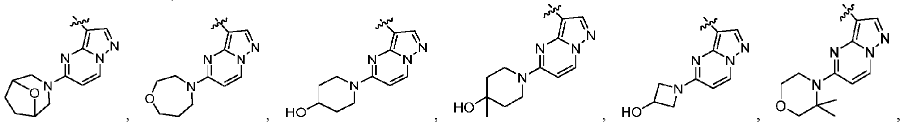 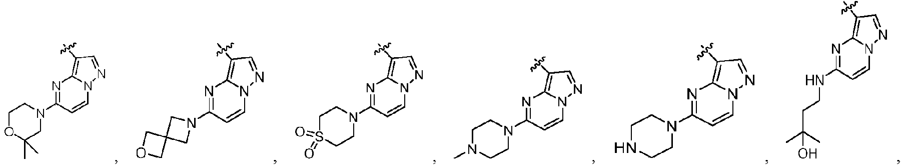 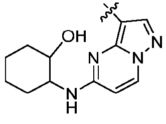 (preferably 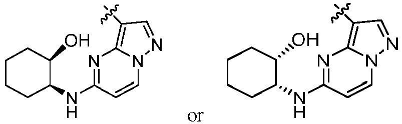 ), 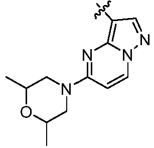 (preferably 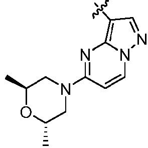 ), 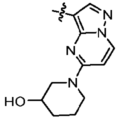 (preferably 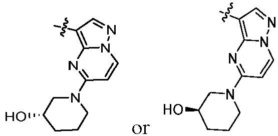 ), and 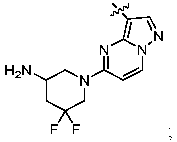
further preferably, B, as a whole, is selected from the group consisting of 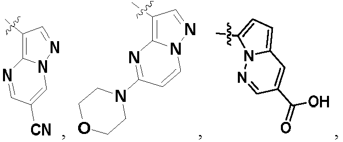 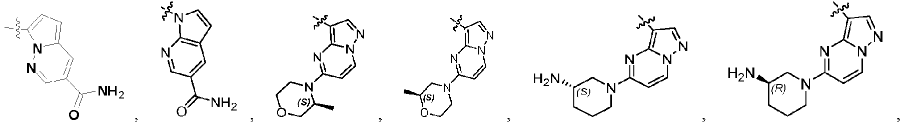 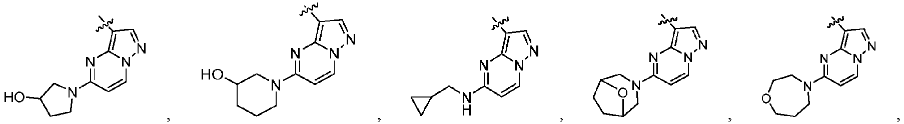 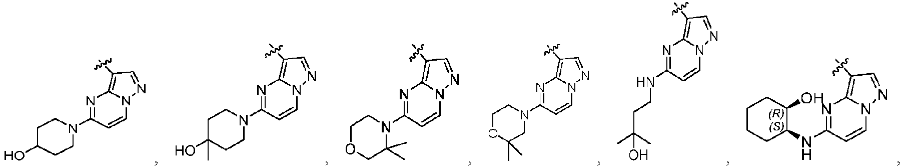 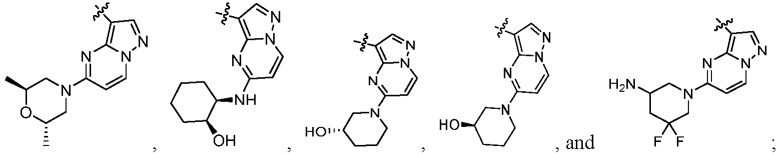
L¹ is selected from the group consisting of a direct bond, C1-C6 alkyl, 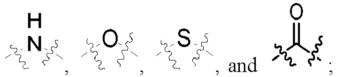 preferably, L¹ is selected from the group consisting of a direct bond, 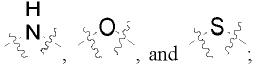 preferably, L¹ is selected from the group consisting of a direct bond, methylene, and 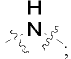 preferably, L¹ is selected from the group consisting of a direct bond, and 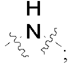 preferably, L¹ is a direct bond;
R¹ is selected from the group consisting of 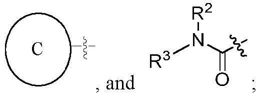
C is selected from 5-6 membered heteroaryl, and C is optionally substituted with at least one of substituents R^{d}, R^{e}, and R^{f}; preferably, C is selected from 5-6 membered heteroaryl containing 1-3 heteroatoms, and, C is optionally substituted with at least one of substituents R^{d}, R^{e}, and R^{f}; further preferably, C is selected from the group consisting of pyrazolyl, imidazolyl, thiazolyl, pyridinyl, pyrrolyl, oxazolyl, furanyl, 1,2,3-triazolyl, and 1,2,4-triazolyl, and, C is optionally substituted with at least one of substituents R^{d}, R^{e}, and R^{f}; further preferably, C is selected from the group consisting of pyrazolyl, thiazolyl, pyridinyl, pyrrolyl, oxazolyl, furanyl, 1,2,3-triazolyl, 1,2,4-triazolyl, and, C is optionally substituted with at least one of substituents R^{d}, R^{e}, and R^{f};
R^{d}, R^{e}, R^{f} are each independently selected from the group consisting of H, C1-C6 alkyl, C1-C6 haloalkyl (such as difluoromethyl), C3-C7 cycloalkyl, 6-10 membered aryl, 5-6 membered heteroaryl, 4-7 membered saturated heterocyclyl, and 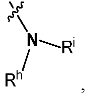 wherein, the C3-C7 cycloalkyl, 6-10 membered aryl, 5-6 membered heteroaryl, and 4-7 membered saturated heterocyclyl are each independently optionally substituted with C1-C6 alkyl, 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d}, R^{e}, R^{f} are each independently selected from the group consisting of H, C1-C6 alkyl, C1-C6 haloalkyl (such as difluoromethyl), C3-C7 cycloalkyl, 6-10 membered aryl, 5-6 membered heteroaryl, 4-7 membered saturated heterocyclyl, and 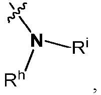 wherein, the C3-C7 cycloalkyl, 6-10 membered aryl, 5-6 membered heteroaryl, and 4-7 membered saturated heterocyclyl are each independently optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d} is selected from the group consisting of C1-C6 alkyl, C3-C6 cycloalkyl, phenyl, pyridinyl, morpholinyl, piperazinyl, and piperidyl, wherein, the C3-C6 cycloalkyl, phenyl, pyridinyl, morpholinyl, piperazinyl, and piperidyl are each independently optionally substituted with 1-2 substituents selected from the group consisting of C1-C6 alkyl, halogen, alkoxy (such as C1-C6 alkoxy), hydroxy, amino, hydroxy C1-C6 alkyl (such as methylol), amino C1-C6 alkyl, and formyl;
preferably, R^{d} is selected from the group consisting of C3-C6 cycloalkyl, phenyl, morpholinyl, and piperidyl, wherein, the C3-C6 cycloalkyl, phenyl, morpholinyl, and piperidyl are each independently optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
further preferably, R^{d} is selected from the group consisting of C1-C6 alkyl, 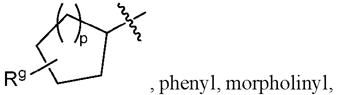 piperazinyl, and piperidyl, wherein, the 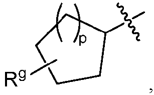 phenyl, morpholinyl, piperazinyl, and piperidyl are optionally substituted with C1-C6 alkyl (preferably methyl), further preferably, R^{d} is selected from the group consisting of 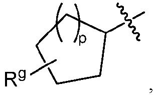 phenyl, morpholinyl, and piperidyl; p is selected from the group consisting of 0, 1, and 2; R^{g} is selected from the group consisting of H, amino, hydroxy, hydroxy C1-C6 alkyl, and formyl;
further preferably, R^{d} is selected from the group consisting of methyl, ethyl, isopropyl, 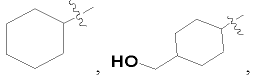 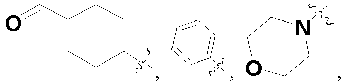 piperazinyl, piperidyl, 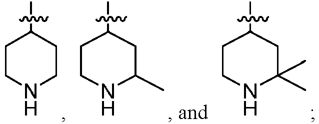
further preferably, R^{d} is selected from the group consisting of 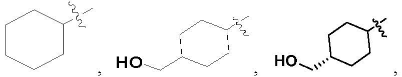 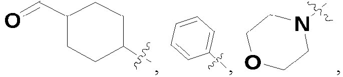 and piperazinyl;
further preferably, R^{d} is selected from the group consisting of 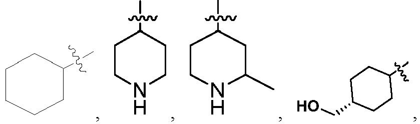 and 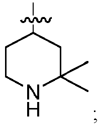
R^{h}, Rⁱ are each independently selected from the group consisting of H, C1-C6 alkyl, and C3-C6 cycloalkyl; preferably, R^{h}, Rⁱ are each independently selected from the group consisting of H, methyl, ethyl, isopropyl, and cyclopropyl;
preferably, R^{e} is selected from the group consisting of H, methyl, 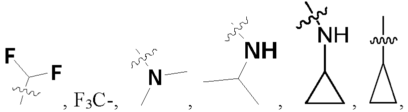 and -NHCH₃;
preferably, R^{e} is selected from the group consisting of H, methyl, 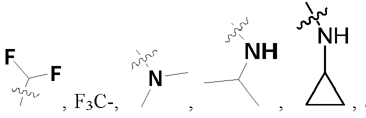 and 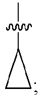
or, R^{e}, R^{f} and the carbon atoms to which they are attached, respectively, together form a substituted or unsubstituted 5-9 membered heterocycle, wherein, the substituent ais selected from the group consisting of C1-C6 alkyl (such as ethyl), alkoxy (such as C1-C6 alkoxy), C3-C6 cycloalkyl, amino, and hydroxy;
preferably, R^{e}, R^{f} and the carbon atoms to which they are attached, respectively, together form 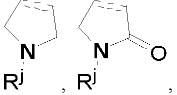 or 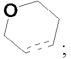
each R^{j} is independently selected from the group consisting of H, C1-C6 alkyl, alkoxy (such as C1-C6 alkoxy), C3-C6 cycloalkyl, amino, and hydroxy;
further preferably, R^{e}, R^{f} and the carbon atoms to which they are attached, respectively, together form 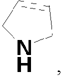 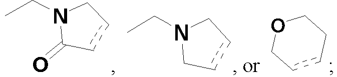
R², R³ are each independently selected from the group consisting of H, C1-C6 alkyl, and C3-C6 cycloalkyl; or, R², R³ and the N atom to which they are both attached together form 5-6 membered saturated heterocyclyl, preferably, the 5-6 membered saturated heterocyclyl contains 2-3 heteroatoms selected from the group consisting of N, S, and O atoms;
preferably, R², R³ are each independently selected from the group consisting of H, methyl, ethyl, propyl, and 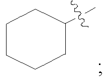
or preferably, R², R³ and the N atom to which they are both attached together form 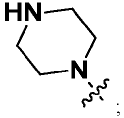
further preferably, R¹, as a whole, is selected from the group consisting of: 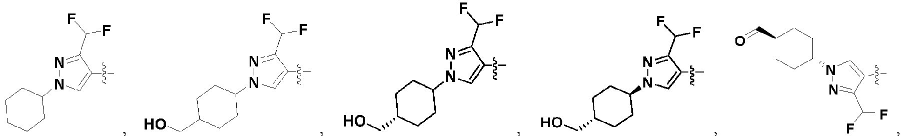 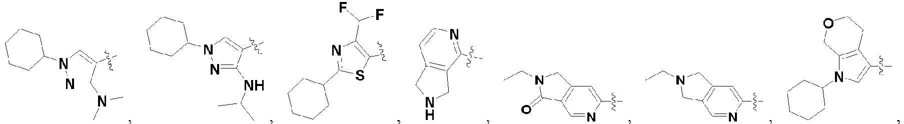 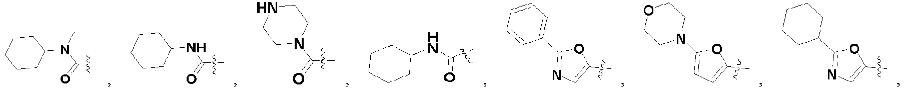 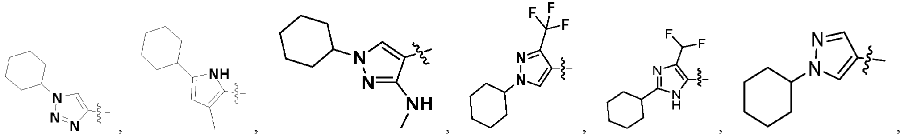 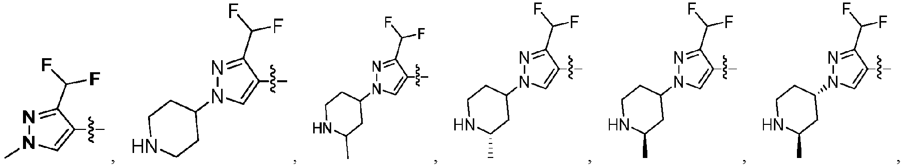 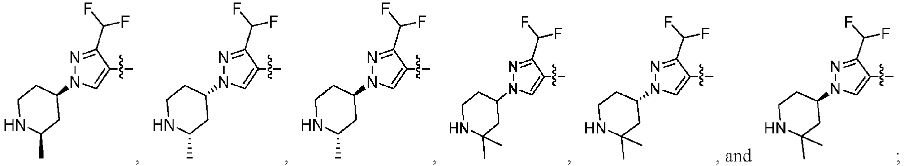
further preferably, R¹, as a whole, is selected from the group consisting of: 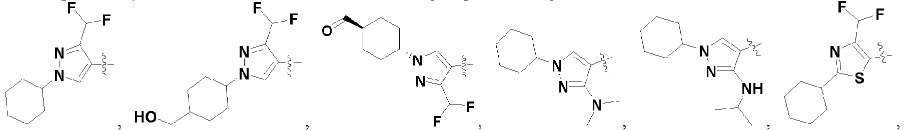 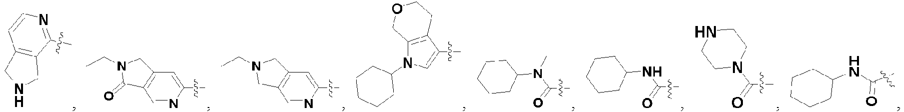 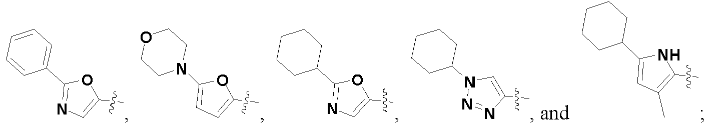
further preferably, R¹, as a whole, is selected from the group consisting of: 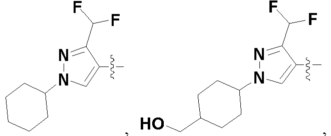 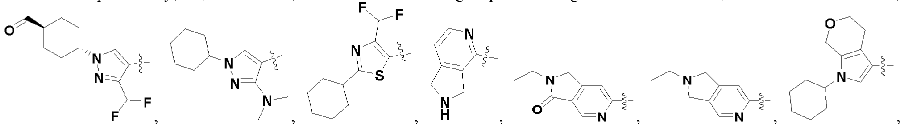 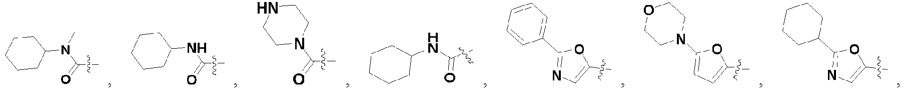 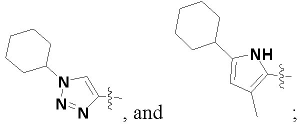
further preferably, R¹, as a whole, is selected from the group consisting of 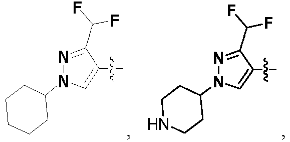 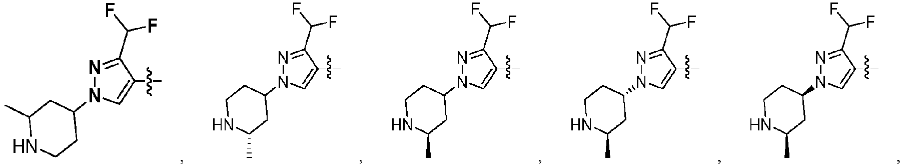 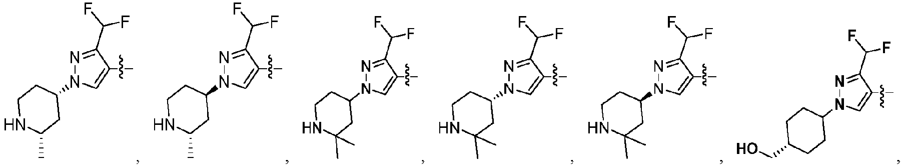 and

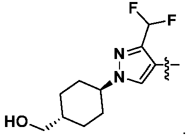

2. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to claim 1, wherein, the compound is represented by formula i, wherein: 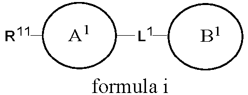
A¹ is selected from 5 membered heteroaromatic ring optionally substituted with substituent R^{k}; preferably, A¹ is selected from 5 membered heteroaromatic ring containing 2-3 heteroatoms, wherein at least two heteroatoms are N atoms; further preferably, A¹ is selected from the group consisting of 1,2,3-triazole ring, 1,2,4-triazole ring, pyrazole ring, imidazole ring, 1,3,4-thiadiazole ring, and 1,3,4-oxadiazole ring, further preferably, A¹ is selected from the group consisting of 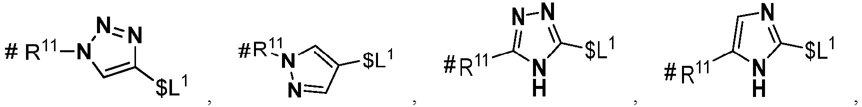 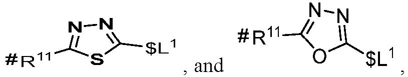 wherein #R¹¹ represents the attachment point to R¹¹, $L¹ represents the attachment point to L¹; further preferably, A¹ is selected from the group consisting of 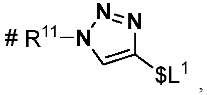 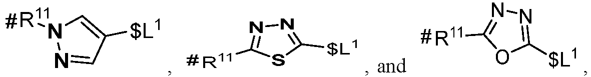 wherein #R¹¹ represents the attachment point to R¹¹, $L¹ represents the attachment point to L¹;
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl; preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl; further preferably, R^{k} is selected from the group consisting of isopropyl, cyclopropyl, dimethylamino, difluoromethyl, and 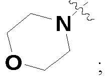
R^{p}, R^{q} are each independently selected from the group consisting of H, C1-C6 alkyl, C1-C6 alkoxy, and C1-C6 haloalkyl: preferably , R^{p}, R^{q} are each independently selected from the group consisting of h , and C1-C6 alkyl:
B¹ is selected from the group consisting of 6-10 membered heteroaryl, and 6-10 membered aryl, and B¹ is optionally substituted with at least one of substituent R^{a}, R^{b}, preferably, B¹ is substituted with at least one of substituent R^{a}, R^{b}, preferably, B¹ is selected from the group consisting of 9 membered heteroaryl, pyrinidyl, phenyl, and pyrimidinyl, and, B¹ is substituted with at least one of substituent R^{a}, R^{b}; preferably, the 9 membered heteroaryl contains 1-4 heteroatoms selected from the group consisting of N, O, and S atoms; preferably, B¹ is selected from the group consisting of 9 membered heteroaryl, pyridinyl, and phenyl, and, B¹ is substitued with at least one of substituents R^{a}, R^{b}, preferably, the 9 membered heteroaryl conatins 1-4 heteroatoms selected from the group consisting of N, O, and S atoms; further preferably, B¹ is selected from the group consisting of 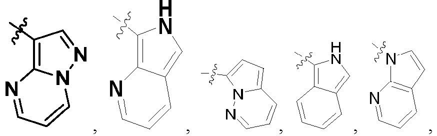 pyridinyl, phenyl, and pyrimidinyl, and, B¹ is substituted with at least one of substituents R^{a}, R^{b}; further preferably, B¹ is selected from the group consisting of 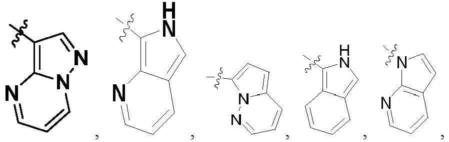 pyridinyl, and phenyl, and, B¹ is substituted with at least one of substituents R^{a}, R^{b};
further preferably, B¹ is selected from the group consisting of 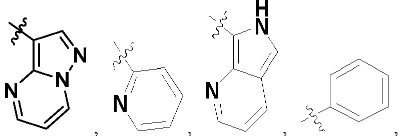 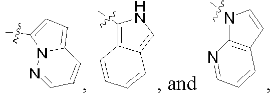 and, B¹ is substituted with substituents R^{a}, R^{b};
further preferably, B¹ is selected from the group consisting of 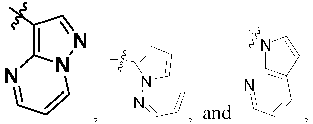 and, B¹ is substituted with substituents R^{a}, R^{b}; R^{a} is selected from the group consisting of H, halogen, cyano, carboxyl, 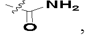 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, 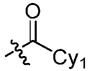 (wherein Cy₁ is 4-9 membered heterocyclyl optionally substituted with R_{cy1} and R_{cy2}), -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), and 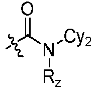 (wherein R_{z} is H or C1-C6 alkyl, Cy₂ is 5-7 membered heterocyclyl, C3-C7 cycloalkyl or C6-C10 aryl, wherein, the 5-7 membered heterocyclyl, C3-C7 cycloalkyl and C6-C10 aryl are optionally substituted with 1-3 substituents selected from the group consisting of halogen, C1-C6 alkyl, C1-C6 alkoxy, CN, and hydroxy), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl), wherein, the C4-C9 cycloalkyl, and 4-9 membered saturated heterocyclyl are optionally substituted with C1-C6 alkyl, halogen, hydroxy, cyano;
preferably, Cy₁ is 5-7 membered heterocyclyl, further preferably, Cy₁ is 6 membered heterocyclyl, further preferably, Cy₁ is morpholinyl, piperazinyl, thiomorpholinyl;
R_{cy1} and R_{cy2} are each independently selected from the group consisting of C1-C6 alkyl, C1-C6 alkoxy, C1-C6 haloalkyl, halogen, hydroxy, nitro, cyano, amino,-NH(C1-C6 alkyl),-N(C1-C6 alkyl)(C1-C6 alkyl), and oxo; preferably, R_{cy1} and R_{cy2} are each independently selected from the group consisting of C1-C6 alkyl, halogen, hydroxy, amino, and oxo, further preferably R_{cy1} and R_{cy2} are oxo;
preferably, Cy₂ is 5-7 membered heterocyclyl optionally substituted with 1-3 substituents selected from the group consisting of halogen, C1-C6 alkyl, C1-C6 alkoxy, CN, and hydroxy; further preferably, Cy₂ is selected from the group consisting of azetidinyl, pyrrolidinyl, imidazolidinyl, piperidyl, piperazinyl, and morpholinyl, wherein, the azetidinyl, pyrrolidinyl, imidazolidinyl, piperidyl, piperazinyl, and morpholinyl are optionally substituted with 1-3 substituents selected from the group consisting of halogen, and C1-C6 alkyl; further preferably, Cy₂ is selected from the group consisting of pyrrolidinyl, imidazolidinyl, and piperidyl, wherein, the pyrrolidinyl, imidazolidinyl, and piperidyl are optionally substituted with 1-3 substituents selected from the group consisting of F, Cl, Br, and methyl; further preferably, Cy₂ is pyrrolidinyl optionally substituted with 1-3 (such as 1 or 2 or 3) F;
preferably, R^{a} is selected from the group consisting of H, halogen, cyano, carboxyl, 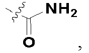 C1-C6 alkyl, C1-C6 haloalkyl (such as trifluoromethyl), C4-C9 cycloalkyl, C4-C9 cycloalkyl-C1-C6 alkyl, 4-9 membered saturated heterocyclyl, 4-9 membered saturated heterocyclyl-C1-C6 alkyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of hydroxy, C3-C6 cycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, halogen, hydroxy, hydroxy-C1-C6 alkyl-, cyano, oxo, - NH₂, -N(C1-C6 alkyl), and -N(C1-C6 alkyl)(C1-C6 alkyl);
preferably, R^{a} is selected from the group consisting of H, cyano, carboxyl, 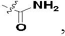 C1-C6 haloalkyl (such as trifluoromethyl), 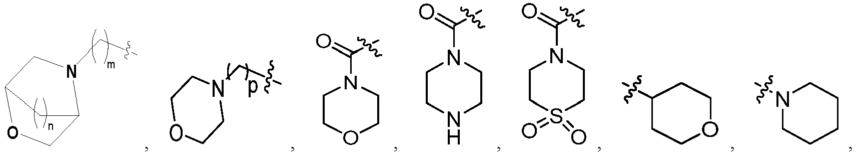 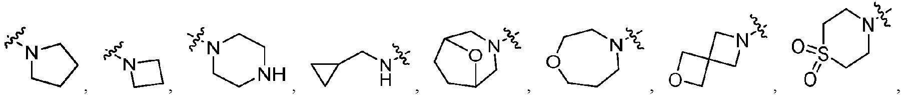 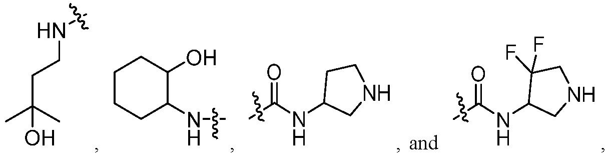 wherein, the 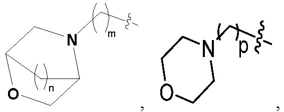 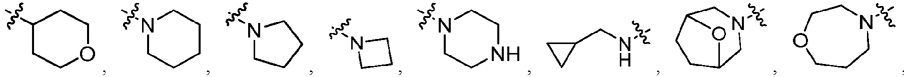 and 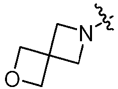 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl);
preferably, R^{a} is selected from the group consisting of H, cyano, carboxyl, 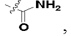 C1-C6 haloalkyl (such as trifluoromethyl), 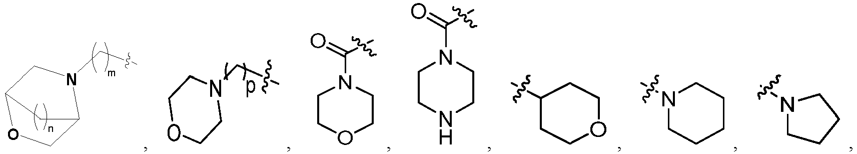 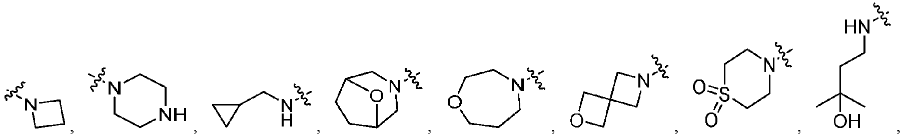 and 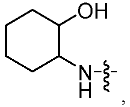 wherein, the 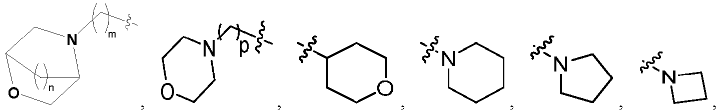 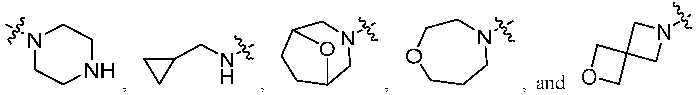 are optionally substituted with 1-3 substituents selected from the group consisting of C1-C6 alkyl, halogen, hydroxy, cyano, -NH₂, -N(C1-C6 alkyl), and - N(C1-C6 alkyl)(C1-C6 alkyl);
preferably, R^{a} is selected from the group consisting of H, cyano, 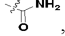 C1-C6 haloalkyl (such as trifluoromethyl), and 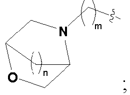 wherein, the 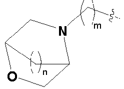 is optionally substituted with C1-C6 alkyl (preferably methyl), halogen (such as F, Cl, Br);
m is selected from the group consisting of 0, 1, 2, and 3; preferably, m is selected from the group consisting of 0, and 1;
n is selected from the group consisting of 0, 1, 2, and 3; preferably, n is selected from the group consisting of 0, and 1;
p is selected from the group consisting of 0, 1, 2, and 3; preferably, p is selected from the group consisting of 0, and 1;
further preferably, R^{a} is selected from the group consisting of H, cyano, carboxyl, 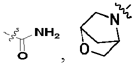 (preferably 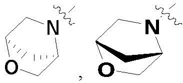 ), 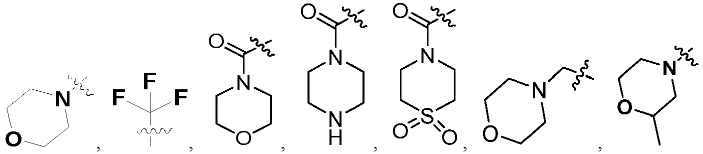 (preferably 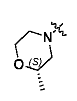 ), 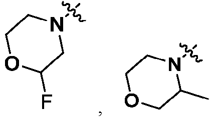 (preferably 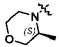 ), 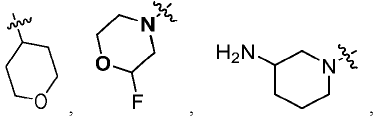 (preferably 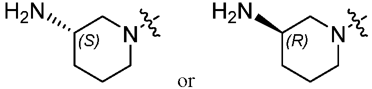 ), 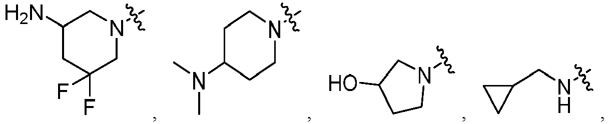 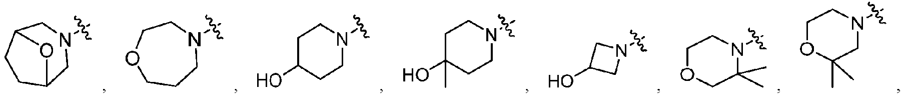 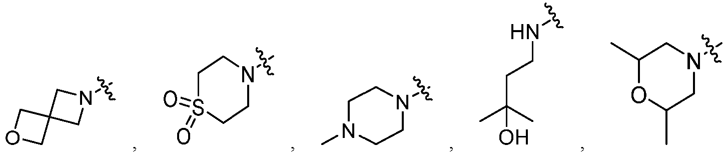 (preferably 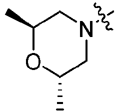 ), 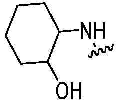 (preferably 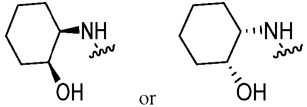 ), 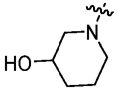 (preferably 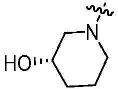 or 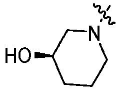 ), 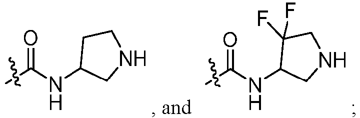
further preferably, R^{a} is selected from the group consisting of H, cyano, 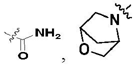 (preferably 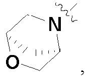 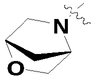 ), 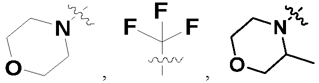 (preferably 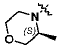 ), 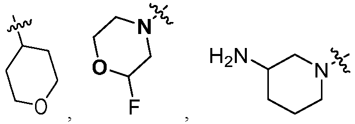 (preferably 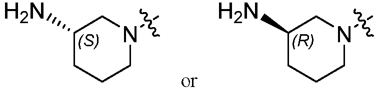 ), 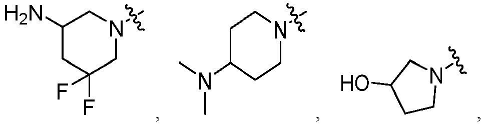 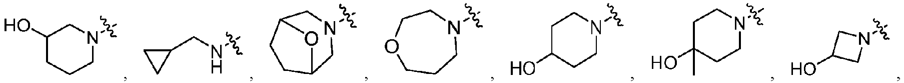 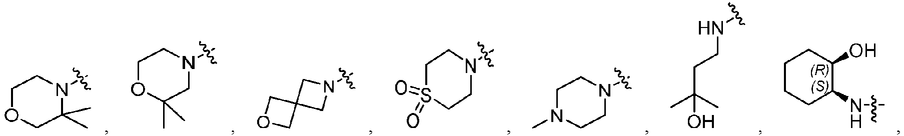 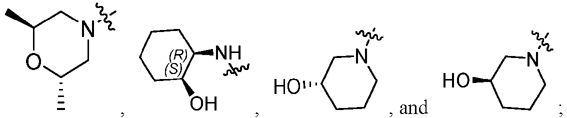 R^{b} is selected from the group consisting of H, halogen, amino, cyano, carboxyl, 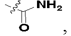 C1-C6 alkyl, C1-C6 haloalkyl (such as trifluoromethyl), and 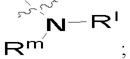 preferably, R^{b} is selected from the group consisting of H, cyano, 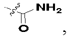 and 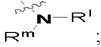 R^{l}, R^{m} are each independently selected from the group consisting of H, C1-C6 alkyl, and C3-C6 cycloalkyl; preferably, R^{l}, R^{m} are each independently selected from the group consisting of H, and C1-C6 alkyl;
or, R^{a}, R^{b} and the carbon atoms to which they are attached, respectively, together form a substituted or unsubstituted 5-membered heterocycle, wherein, the substituent is selected from C1-C6 alkyl (such as tert-butyl);
preferably, R^{a}, R^{b} and the carbon atoms to which they are attached, respectively, together form 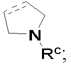
R^{c} is selected from the group consisting of H, and C1-C6 alkyl, preferably, R^{c} is selected from C1-C6 alkyl;
further preferably, B¹, as a whole, is selected from the group consisting of: 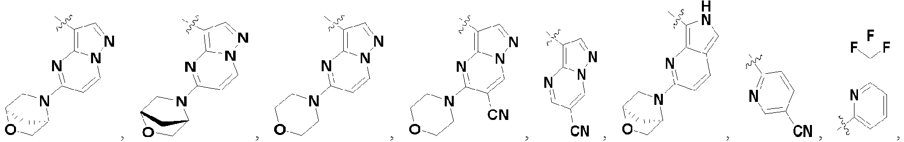 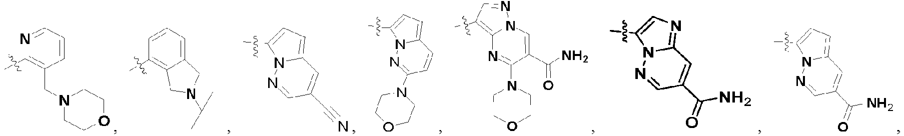 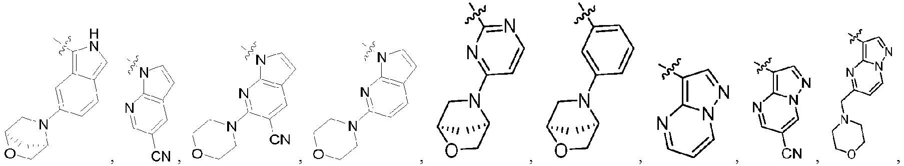 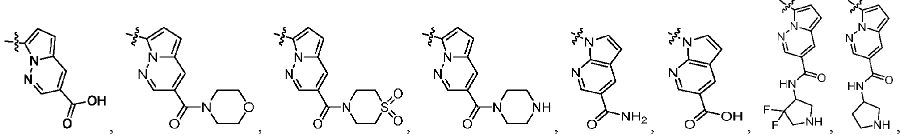 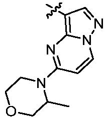 (preferably 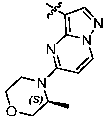 ), 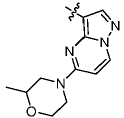 (preferably 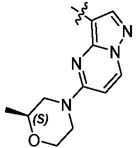 ), 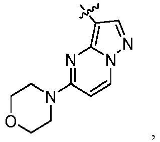 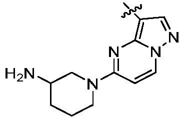 ( 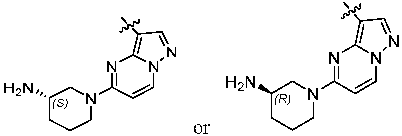 ), 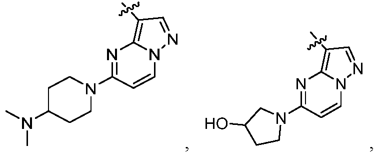 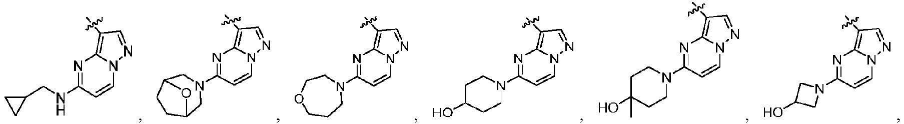 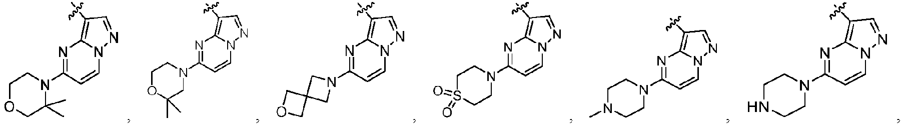 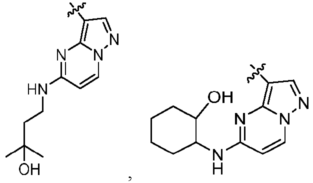 (preferably 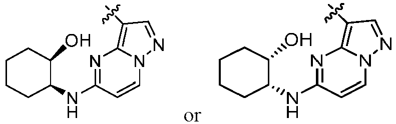 ), 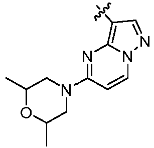 (preferably 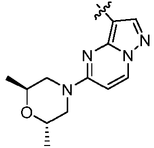 ), 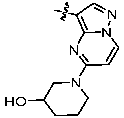 (preferably 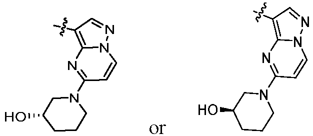 ), and 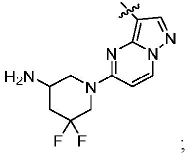
further preferably, B¹ as a whole, is selected from the group consisting of: 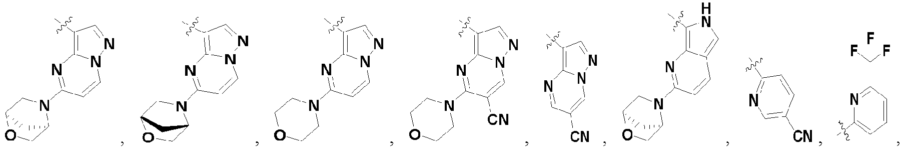 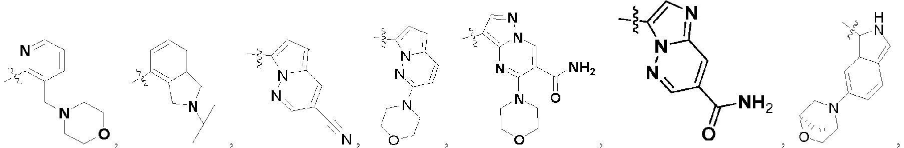 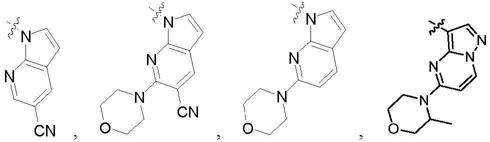 (preferably 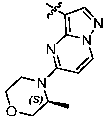 ), 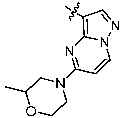 (preferably 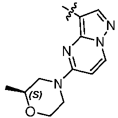 ), 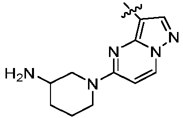 ( 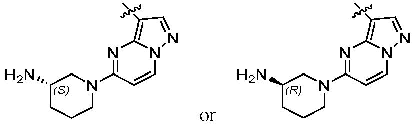 ), 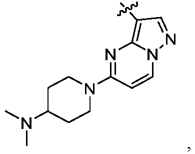 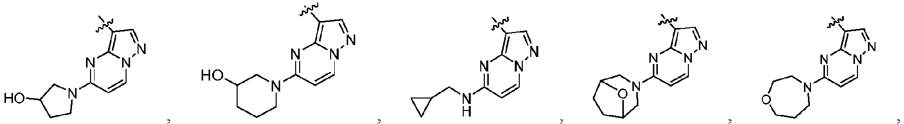 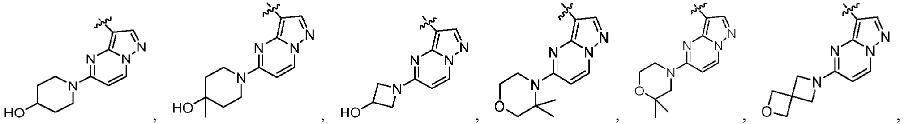 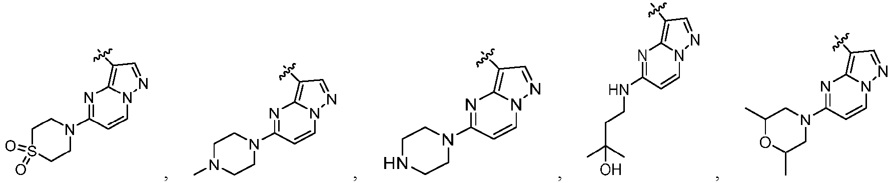 (preferably 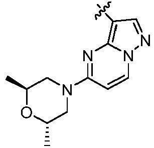 ), 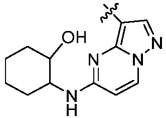 (preferably 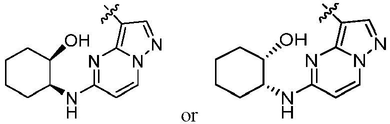 ), 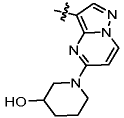 (preferably 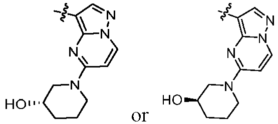 ), and 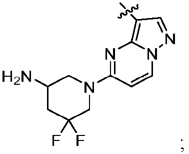 further preferably, B¹, as a whole, is selected from the group consisting of 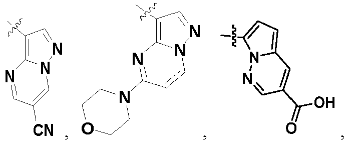 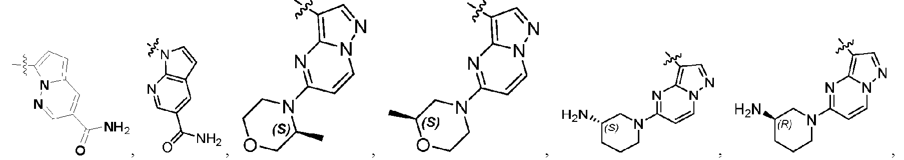 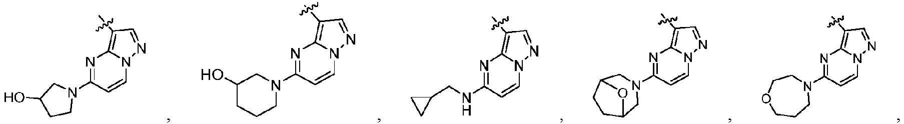 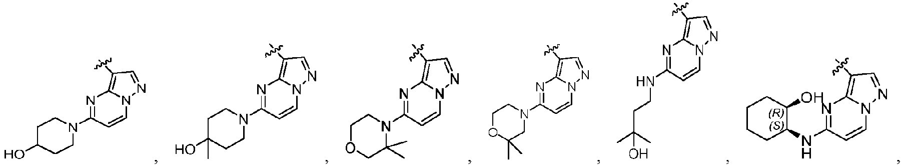 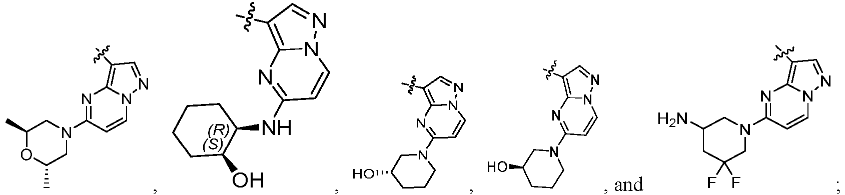
L¹ is selected from the group consisting of a direct bond, C1-C6 alkyl, 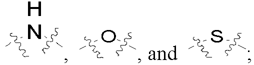 preferably, L¹ is selected from the group consisting of a direct bond, 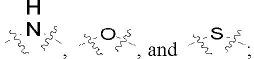 preferably, L¹ is selected from the group consisting of a direct bond, methylene, and 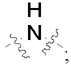 preferably, L¹ is selected from the group consisting of a direct bond, and 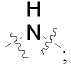
R¹¹ is selected from the group consisting of 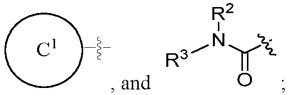
C¹ is selected from 5-6 membered heteroaryl, and C¹ is optionally substituted with at least one of substituents R^{d}, R^{e}, R^{f}; preferably, C¹ is selected from 5-6 membered heteroaryl containing 1-3 heteroatoms, and, C¹ is optionally substituted with at least one of substituents R^{d}, R^{e}, R^{f}; further preferably, C¹ is selected from the group consisting of pyrazolyl, imidazolyl, thiazolyl, pyridinyl, and pyrrolyl, and, C¹ is optionally substituted with at least one of substituents R^{d}, R^{e}, R^{f}; further preferably, C¹ is selected from the group consisting of pyrazolyl, thiazolyl, pyridinyl, and pyrrolyl, and, C¹ is optionally substituted with at least one of substituents R^{d}, R^{e}, R^{f};
R^{d}, R^{e}, R^{f} are each independently selected from the group consisting of H, C1-C6 alkyl, C1-C6 haloalkyl (such as difluoromethyl), C3-C7 cycloalkyl, 4-7 membered saturated heterocyclyl, and 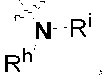 wherein, the C3-C7 cycloalkyl and 4-7 membered saturated heterocyclyl are optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
R^{d}, R^{e}, R^{f} are each independently selected from the group consisting of H, C1-C6 alkyl, C1-C6 haloalkyl (such as difluoromethyl), C3-C7 cycloalkyl, and 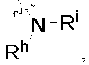 wherein, the C3-C7 cycloalkyl is optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d} is selected from C3-C6 cycloalkyl optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl; preferably, R^{d} is selected from 4-7 membered saturated heterocyclyl optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl; further preferably, R^{d} is selected from the group consisting of C1-C6 alkyl, and 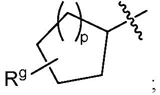
or further preferably, R^{d} is selected from the group consisting of pyrrolyl, piperidyl, imidazolidinyl, and piperazinyl, wherein, the pyrrolyl, piperidyl, imidazolidinyl, and piperazinyl are optionally substituted with 1-2 substituents selected from the group consisting of halogen, C1-C6 alkoxy, and C1-C6 alkyl (preferably methyl); further preferably, R^{d} is selected from 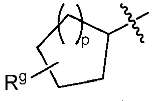 ; p is selected from the group consisting of 0, 1, and 2;
or further preferably, R^{d} is selected from the group consisting of pyrrolyl, and piperidyl, wherein, the pyrrolyl, and piperidyl are optionally substituted with 1-2 substituents selected from the group consisting of halogen, and C1-C6 alkyl (preferably methyl);
R^{g} is selected from the group consisting of H, amino, hydroxy, hydroxy C1-C6 alkyl, and formyl;
further preferably, R^{d} is selected from the group consisting of methyl, ethyl, isopropyl, 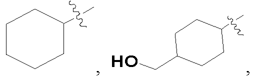 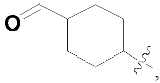 piperidyl, 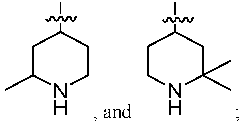 further preferably, R^{d} is selected from the group consisting of 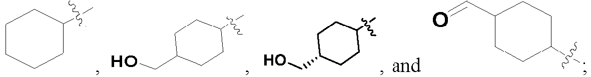 further preferably, R^{d} is selected from the group consisting of 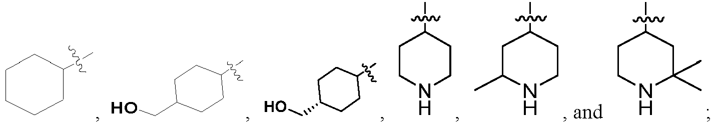
R^{h}, Rⁱ are each independently selected from the group consisting of H, C1-C6 alkyl, and C3-C6 cycloalkyl;
preferably, R^{h}, Rⁱ are each independently selected from the group consisting of H, methyl, ethyl, isopropyl, and cyclopropyl;
preferably, R^{e} is selected from the group consisting of H, methyl, 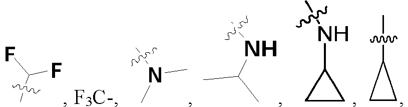 and -NHCH₃; preferably, R^{e} is selected from the group consisting of H, methyl, 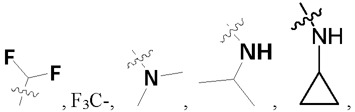 and 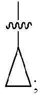
or, R^{e}, R^{f} and the carbon atoms to which they are attached, respectively, together form a substituted or unsubstituted 5-9 membered heterocycle, wherein, the substituent is selected from the group consisting of C1-C6 alkyl (such as ethyl), alkoxy (such as C1-C6 alkoxy), C3-C6 cycloalkyl, amino, and hydroxy,
preferably, R^{e}, R^{f} and the carbon atoms to which they are attached, respectively, together form 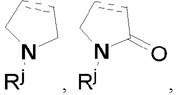 or 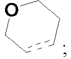
each R^{j} is independently selected from the group consisting of H, C1-C6 alkyl, alkoxy (such as C1-C6 alkoxy), C3-C6 cycloalkyl, amino, and hydroxy;
further preferably, R^{e}, R^{f} and the carbon atoms to which they are attached, respectively, together form 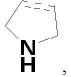 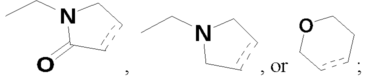
R², R³ are each independently selected from the group consisting of H, C1-C6 alkyl, and C3-C6 cycloalkyl; or, R², R³ and the N atom to which they are both attached together form 5-6 membered saturated heterocyclyl, preferably, the 5-6 membered saturated heterocyclyl contains 2-3 heteroatoms selected from the group consisting of N, S, and O atoms; preferably, R², R³ are each independently selected from the group consisting of H, methyl, ethyl, propyl, and 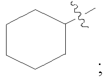 or preferably, R², R³ and the N atom to which they are both attached together form 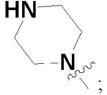
further preferably, R¹¹, as a whole, is selected from the group consisting of: 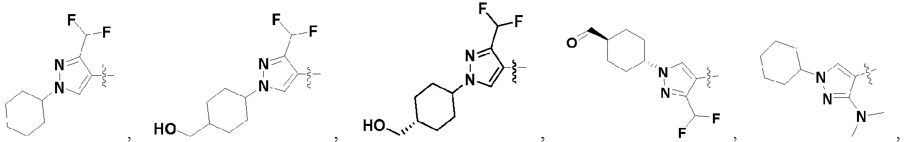 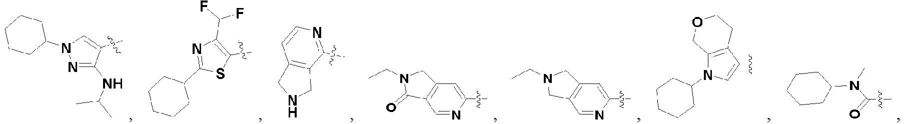 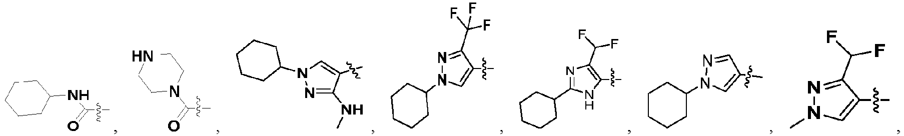 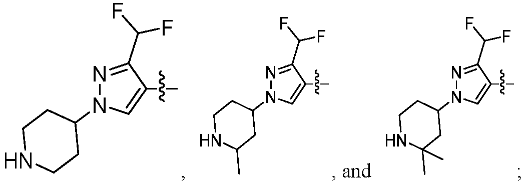
further preferably, R¹¹, as a whole, is selected from the group consisting of: 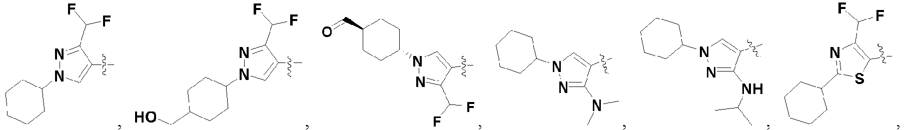 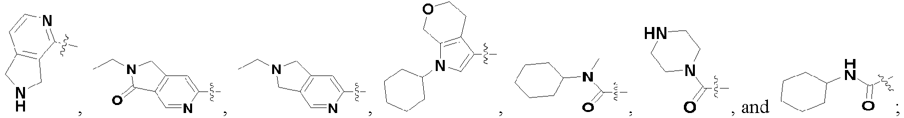
further preferably, R¹¹, as a whole, is 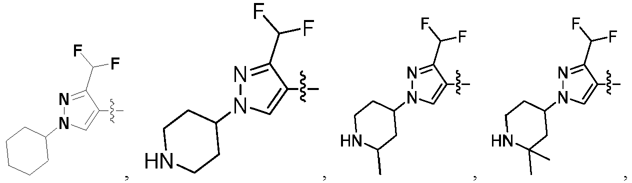
or

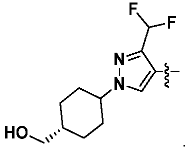

3. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to claim 2, wherein, the compound is represented by formula i-1, 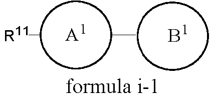 wherein: A¹, B¹, R¹¹ are as defined in claim 2.

4. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 2-3, wherein, the compound is represented by formula i-1-1, 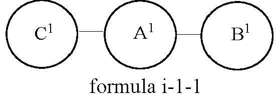 wherein: A¹, B¹, C¹ are as defined in claim 2.

5. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to claim 1, wherein, the compound is represented by formula ii, 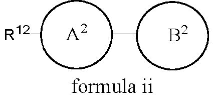
wherein: A² is selected from 6 membered heteroaromatic ring optionally substituted with substituent R^{k}; preferably, A² is selected from 6 membered heteroaromatic ring containing 1-2 heteroatoms, and, at least one heteroatom is N atom, and, the 6 membered heteroaromatic ring is optionally substituted with substituent R^{k}; further preferably, A² is selected from the group consisting of 2-pyridone, and pyridazine ring, wherein, the 2-pyridone is optionally substituted with substituent R^{k}; further preferably, A² is selected from the group consisting of 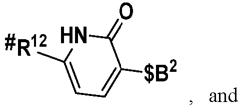 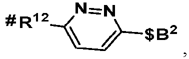 wherein, the 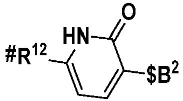 is optionally substituted with substituent R^{k}, wherein #R¹² represents the attachment point to R¹², $B² represents the attachment point to B²;
further preferably, A² is selected from the group consisting of 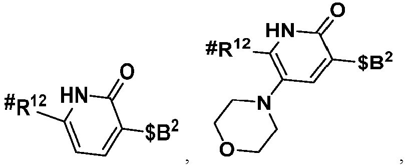 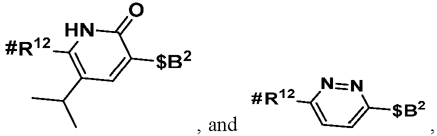 wherein #R¹² represents the attachment point to R¹², $B² represents the attachment point to B²;
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl; preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl;
R^{p}, R^{q} are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
further preferably, R^{k} is selected from the group consisting of isopropyl, cyclopropyl, dimethylamino, difluoromethyl, and 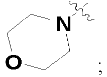
B² is selected from 6-10 membered heteroaryl, and B² is optionally substituted with at least one of substituents R^{a}, R^{b}; preferably, B² is substituted with at least one of substituents R^{a}, R^{b}; preferably, B² is selected from 9 membered heteroaryl, and B² is substituted with at least one of substituents R^{a}, R^{b}; preferably, the 9 membered heteroaryl contains 1-4 heteroatoms selected from the group consisting of N, O, and S atoms; further preferably, B² is selected from the group consisting of 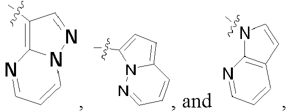 and B² is optionally substituted with at least one of substituents R^{a}, R^{b};
R^{a} is selected from the group consisting of H, halogen, amino, cyano, carboxyl, 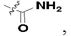 C1-C6 alkyl, and C1-C6 haloalkyl (such as trifluoromethyl); preferably, R^{a} is selected from the group consisting of H, cyano, 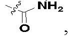 and C1-C6 haloalkyl (such as trifluoromethyl), further preferably, R^{a} is selected from the group consisting of H, cyano, 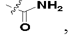 and 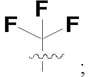
R^{b} is selected from the group consisting of H, halogen, amino, cyano, carboxyl, 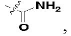 C1-C6 alkyl, C1-C6 haloalkyl (such as trifluoromethyl), and 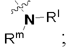 preferably, R^{b} is selected from the group consisting of H, cyano, and 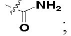
R^{l}, R^{m} are each independently selected from the group consisting of H, C1-C6 alkyl, and C3-C6 cycloalkyl; preferably, R^{l}, R^{m} are each independently selected from the group consisting of H, and C1-C6 alkyl;
further preferably, B², as a whole, is selected from the group consisting of 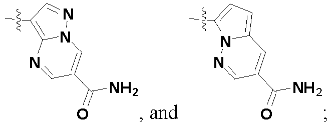
R¹² is selected from 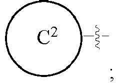
C² is selected from 5-6 membered heteroaryl, and C² is optionally substituted with at least one of substituents R^{d}, R^{e}, R^{f};
preferably, C² is selected from 5-6 membered heteroaryl containing 1-3 heteroatoms, and C² is optionally substituted with at least one of substituents R^{d}, R^{e}, R^{f}; further preferably, C² is selected from the group consisting of pyrrolyl, oxazolyl, furanyl, 1,2,3-triazolyl, and 1,2,4-triazolyl, and C² is optionally substituted with at least one of substituents R^{d}, R^{e}, R^{f};
R^{d}, R^{e}, R^{f} are each independently selected from the group consisting of H, C1-C6 alkyl, C1-C6 haloalkyl (such as difluoromethyl), C3-C7 cycloalkyl, 6-10 membered aryl, 5-6 membered heteroaryl, and 4-7 membered saturated heterocyclyl, wherein, the C3-C7 cycloalkyl, 6-10 membered aryl, 5-6 membered heteroaryl, and 4-7 membered saturated heterocyclyl are each independently optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl;
preferably, R^{d} is selected from the group consisting of C3-C6 cycloalkyl, phenyl, morpholinyl, and piperazinyl, wherein, the C3-C6 cycloalkyl, phenyl, morpholinyl, and piperazinyl are each independently optionally substituted with 1-2 substituents selected from the group consisting of halogen, alkoxy (such as C1-C6 alkoxy), hydroxy, amino, hydroxy C1-C6 alkyl (such as methylol), amino C1-C6 alkyl, and formyl; further preferably, R^{d} is selected from the group consisting of C3-C6 cycloalkyl, phenyl, morpholinyl, and piperazinyl; further preferably, R^{d} is selected from the group consisting of 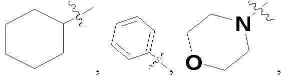 and piperazinyl;
preferably, R^{e} is selected from the group consisting of H, methyl, 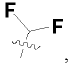 F₃C-, and 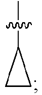
further preferably, R¹², as a whole, is selected from the group consisting of:

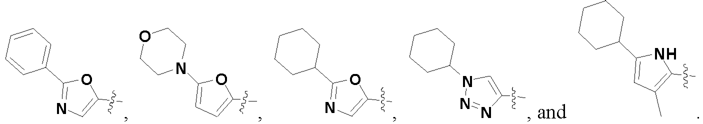

6. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to claim 1, wherein, the compound is represented by formula I, 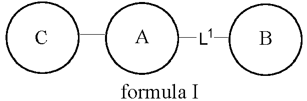
wherein: A is selected from 5-6 membered heteroaromatic ring optionally substituted with substituent R^{k}; preferably, A is selected from the group consisting of 5 membered heteroaromatic ring, and 6 membered heteroaromatic ring, the 5 membered heteroaromatic ring contains 2-3 heteroatoms, wherein at least two heteroatoms are N atoms, the 6 membered heteroaromatic ring contains 1-2 heteroatoms, wherein at least one heteroatom is N atom, and, the 5 membered heteroaromatic ring and 6 membered heteroaromatic ring are optionally substituted with substituent R^{k}; further preferably, A is selected from the group consisting of 1,2,3-triazole ring, 1,2,4-triazole ring, pyrazole ring, imidazole ring, 1,3,4-thiadiazole ring, 1,3,4-oxadiazole ring, 2-pyridone, and pyridazine ring, wherein, the 2-pyridone is optionally substituted with substituent R^{k}; further preferably, A is selected from the group consisting of 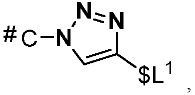 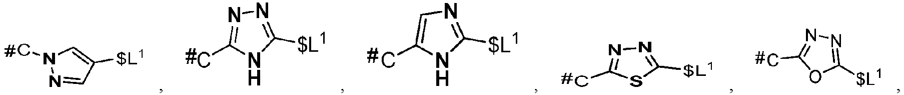 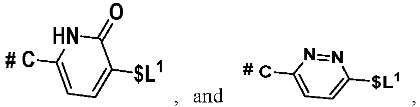 wherein, the 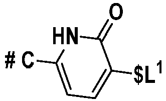 is optionally substituted with substituent R^{k}, wherein #C represents the attachment point to C, $L¹ represents the attachment point to L¹;
further preferably, A is selected from the group consisting of 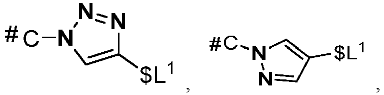 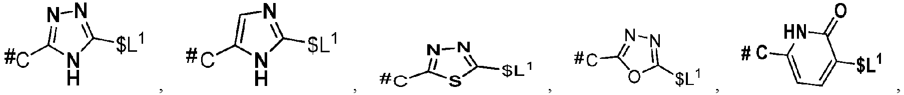 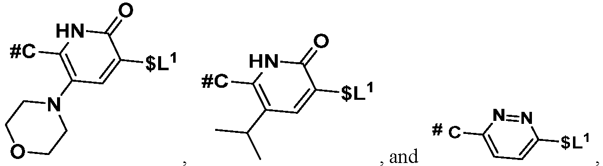 wherein #C represents the attachment point to C, $L¹ represents the attachment point to L¹;
further preferably, A is selected from the group consisting of 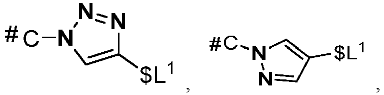 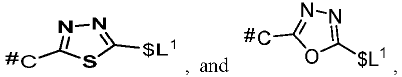 wherein #C represents the attachment point to C, $L¹ represents the attachment point to L¹;
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl; preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl;
R^{p}, R^{q} are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
further preferably, R^{k} is selected from the group consisting of isopropyl, cyclopropyl, dimethylamino, difluoromethyl, and 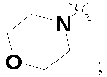
B is selected from any one of the groups (1)-(7): 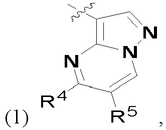 wherein: R⁴ is selected from the group consisting of H, C1-C6 alkyl, C4-C9 cycloalkyl, C4-C9 cycloalkyl-C1-C6 alkyl, 4-9 membered saturated heterocyclyl, 4-9 membered saturated heterocyclyl-C1-C6 alkyl, and - NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl);
preferably, R⁴ is selected from the group consisting of H, 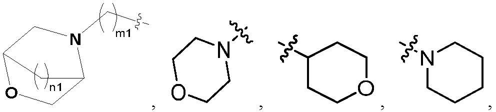 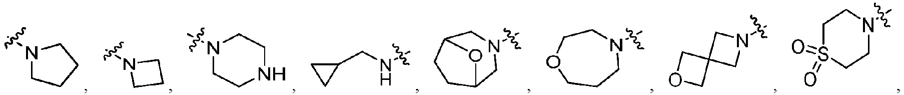 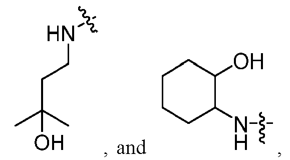 wherein, the 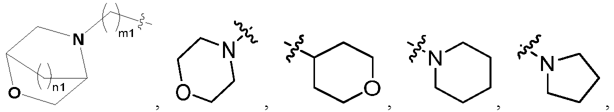 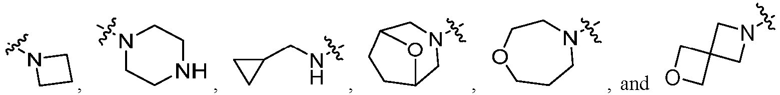 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl);
m1 is selected from the group consisting of 0, 1, 2, and 3; m1 is preferably 0; n1 is selected from the group consisting of 0, 1, 2, and 3; n1 is preferably 0, or 1;
further preferably, R⁴ is selected from the group consisting of H, 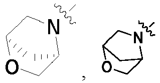 (preferably 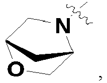 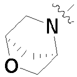 ), 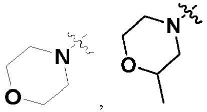 (preferably 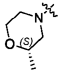 ), 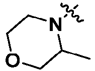 (preferably 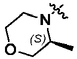 ), 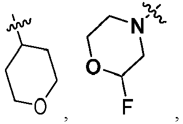 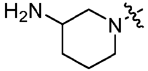 (preferably 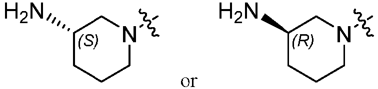 ), 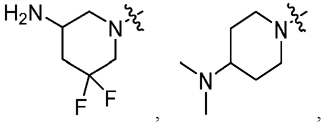 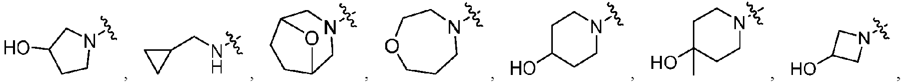 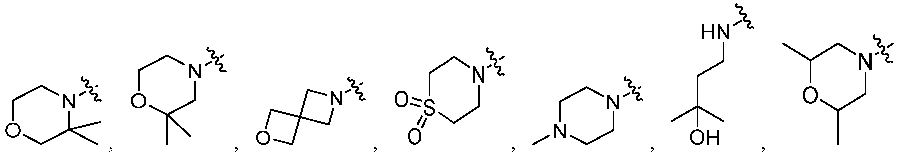 (preferably 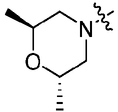 ), 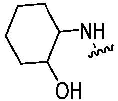 (preferably 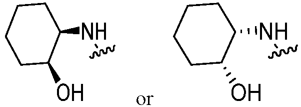 ), and 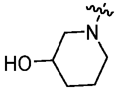 (preferably 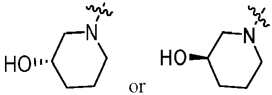 );
R⁵ is selected from the group consisting of H, cyano, C1-C6 alkyl, 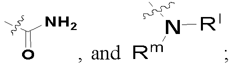 preferably, R⁵ is selected from the group consisting of H, cyano, 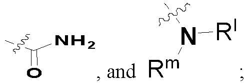
R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl;
preferably, R⁵ is selected from the group consisting of H, and cyano;
further preferably, 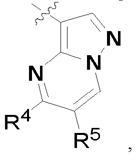 as a whole, is selected from the group consisting of 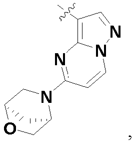 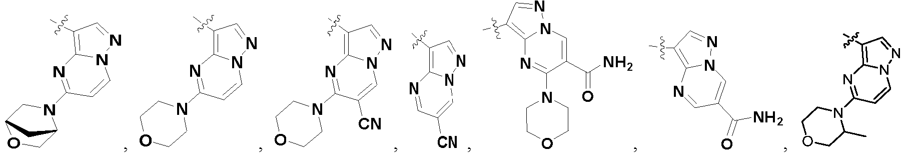 (preferably 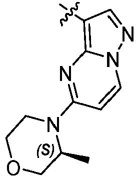 ), 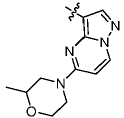 (preferably 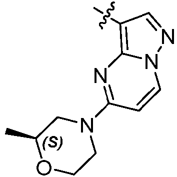 ), 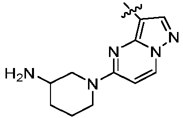 ( 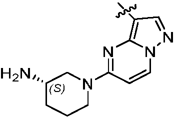 or 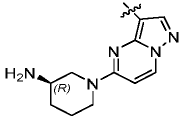 ), 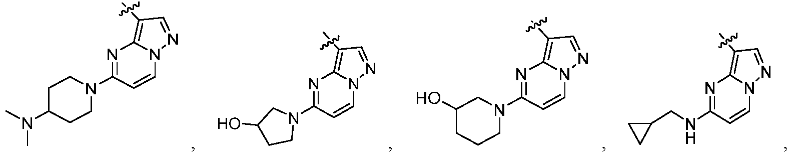 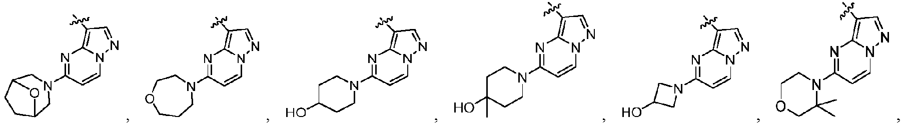 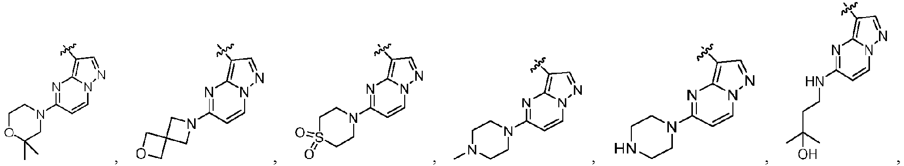 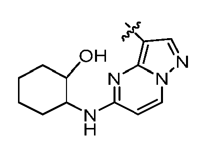 (preferably 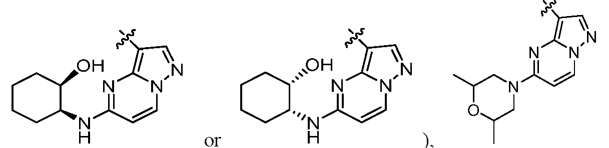 ) (preferably 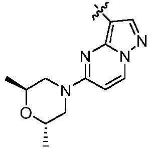 ), 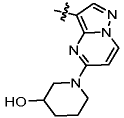 (preferably 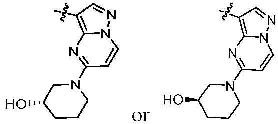 ), and 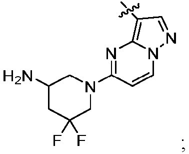
further preferably, 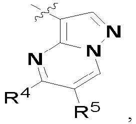 as a whole, is selected from the group consisting of 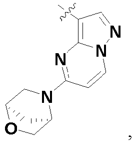 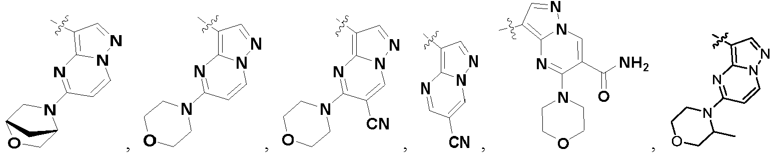 (preferably 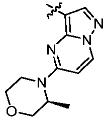 ), 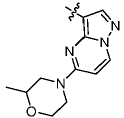 (preferably 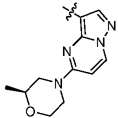 ), 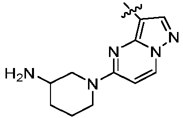 ( 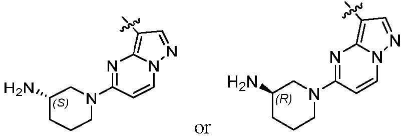 ), 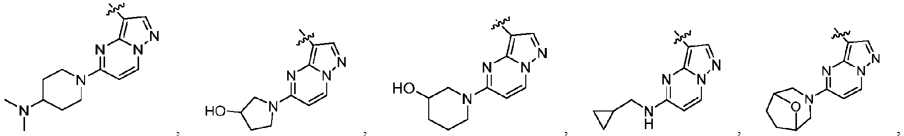 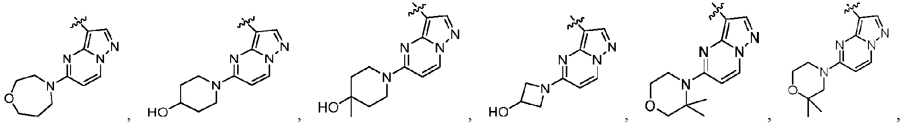 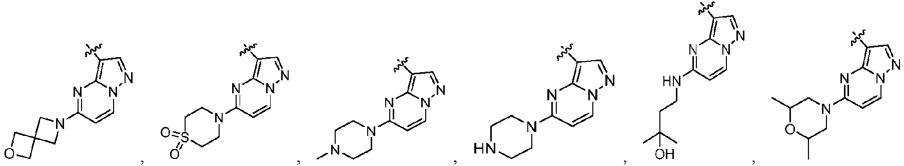 (preferably 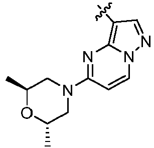 ) 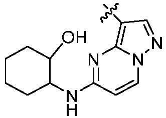 (preferably 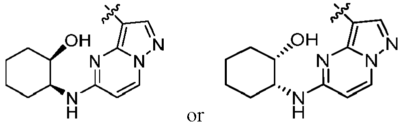 ), 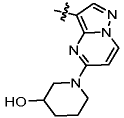 (preferably 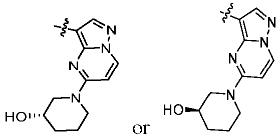 ), and 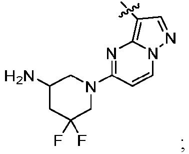 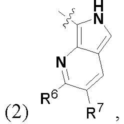
wherein: R⁶ is selected from the group consisting of H, C1-C6 alkyl, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R⁶ is selected from 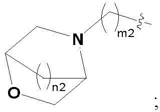 m2 is selected from the group consisting of 0, 1, 2, and 3; n2 is selected from the group consisting of 0, 1, 2, and 3; further preferably, R⁶ is 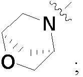
R⁷ is selected from the group consisting of H, and C1-C6 alkyl;
further preferably, 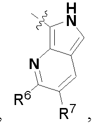 as a whole, is 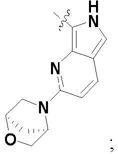 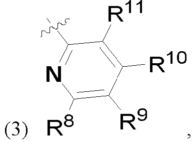
wherein: one of R⁸, R⁹, R¹⁰, and R¹¹ is selected from the group consisting of cyano, C1-C6 haloalkyl (such as trifluoromethyl), C4-C9 cycloalkyl, C4-C9 cycloalkyl-C1-C6 alkyl, 4-9 membered saturated heterocyclyl, and 4-9 membered saturated heterocyclyl-C1-C6 alkyl, and the rest are H; preferably, among R⁸, R⁹, R¹⁰, and R¹¹, R⁸, R⁹ or R¹¹ is selected from the group consisting of cyano, C1-C6 haloalkyl (such as trifluoromethyl), and 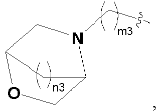 and the rest are H; m3 is selected from the group consisting of 0, 1, 2, and 3; n3 is selected from the group consisting of 0, 1, 2, and 3; further preferably, 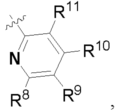 as a whole, is selected from the group consisting of 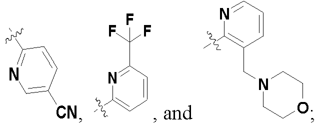 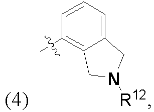
wherein: R¹² is selected from the group consisting of H, and C1-C6 alkyl; preferably, R¹² is selected from C1-C6 alkyl; 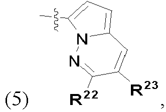
wherein: R²² is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl;
preferably, R²² is selected from the group consisting of H, and 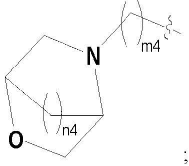 m4 is selected from the group consisting of 0, 1, 2, and 3; n4 is selected from the group consisting of 0, 1, 2, and 3; further preferably, R²² is selected from the group consisting of H, and 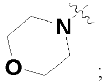
R²³ is selected from the group consisting of H, C1-C6 alkyl, cyano, carboxyl, 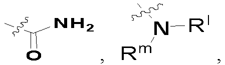 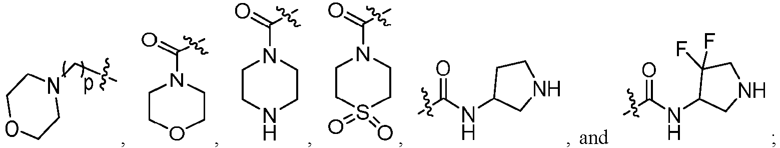 preferably, R²³ is selected from the group consisting of H, C1-C6 alkyl, cyano, 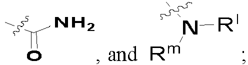 preferably, R²³ is selected from the group consisting of H, cyano, carboxyl, 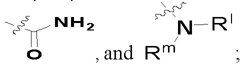 preferably, R²³ is selected from the group consisting of H, cyano, 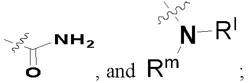
R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl;
further preferably, 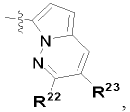 as a whole, is selected from the group consisting of 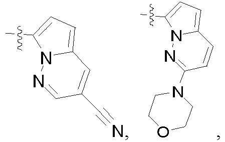 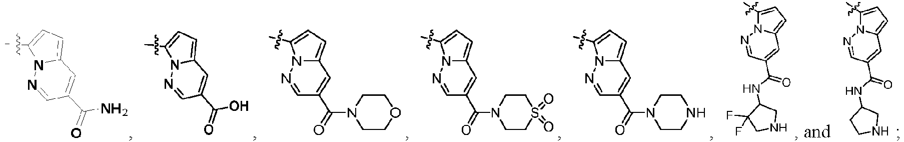
further preferably, 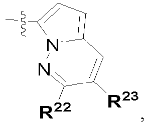 as a whole, is selected from the group consisting of 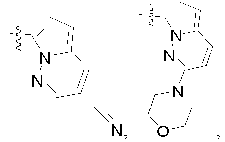 and 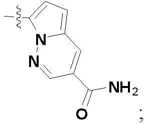
further preferably, 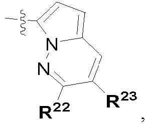 as a whole, is selected from the group consisting of 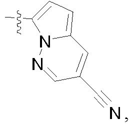 and 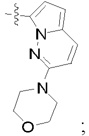 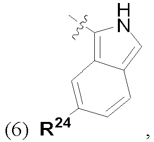
wherein: R²⁴ is selected from the group consisting of H, C1-C6 alkyl, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²⁴ is selected from 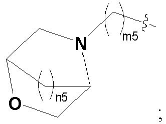 m5 is selected from the group consisting of 0, 1, 2, and 3; n5 is selected from the group consisting of 0, 1, 2, and 3; further preferably, R²⁴ is 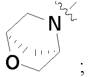 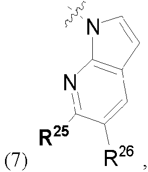
wherein: R²⁵ is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²⁵ is selected from the group consisting of H, 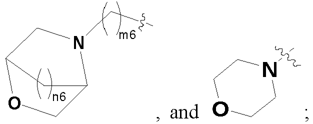 m6 is selected from the group consisting of 0, 1, 2, and 3; n6 is selected from the group consisting of 0, 1, 2, and 3; further preferably, R²⁵ is selected from the group consisting of H, and 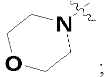 preferably, R²⁵ is H;
R²⁶ is selected from the group consisting of H, C1-C6 alkyl, cyano, carboxyl, and 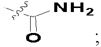 preferably, R²⁶ is selected from the group consisting of H, C1-C6 alkyl, cyano, and 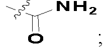
preferably, R²⁶ is selected from the group consisting of H, C1-C6 alkyl, and 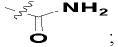
further preferably, 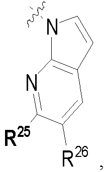 as a whole, is selected from the group consisting of 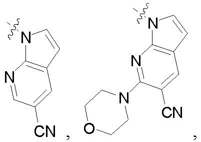 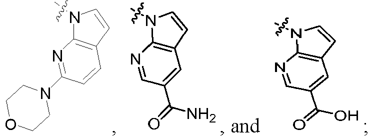
further preferably, 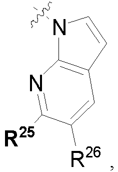 as a whole, is selected from the group consisting of 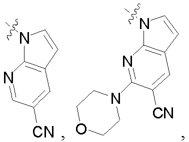 and 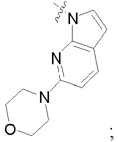
L¹ is selected from the group consisting of a direct bond, C1-C6 alkyl, 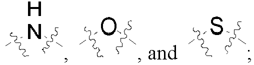 preferably, L¹ is selected from the group consisting of a direct bond, and 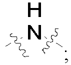 preferably, L¹ is a direct bond;
C is selected from any one of groups (1)-(10): (1) 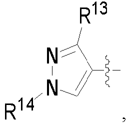 wherein: R¹³ is selected from the group consisting of C1-C6 haloalkyl (such as difluoromethyl), and 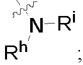 R^{h}, Rⁱ are each independently selected from the group consisting of H, and C1-C6 alkyl; preferably, R^{h}, Rⁱ are each independently selected from the group consisting of H, methyl, and isopropyl;
preferably, R¹³ is selected from the group consisting of 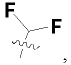 trifluoromethyl, 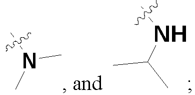 preferably, R¹³ is selected from the group consisting of 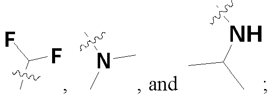 further preferably, R¹³ is selected from the group consisting of 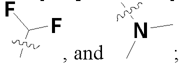
R¹⁴ is selected from the group consisting of C3-C6 cycloalkyl, and piperidyl, wherein, the C3-C6 cycloalkyl and piperidyl are optionally substituted with 1-2 substituents selected from the group consisting of hydroxy C1-C6 alkyl (such as methylol), formyl, and C1-C6 alkyl; preferably, R¹⁴ is selected from C3-C6 cycloalkyl optionally substituted with 1-2 substituents selected from the group consisting of hydroxy C1-C6 alkyl (such as methylol), and formyl;
preferably, R¹⁴ is selected from 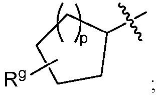 p is selected from the group consisting of 0, 1, and 2; R^{g} is selected from the group consisting of H, hydroxy C1-C6 alkyl, and formyl;
or, preferably, R¹⁴ is piperidyl optionally substituted with 1-2 substituents selected from C1-C6 alkyl (preferably methyl); further preferably, R¹⁴ is selected from the group consisting of 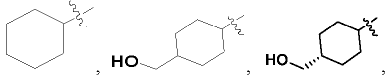 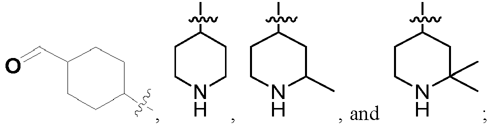 further preferably, 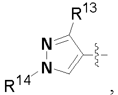 as a whole, is selected from the group consisting of 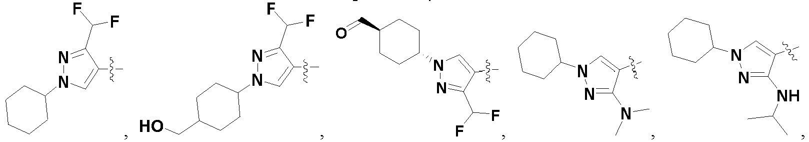 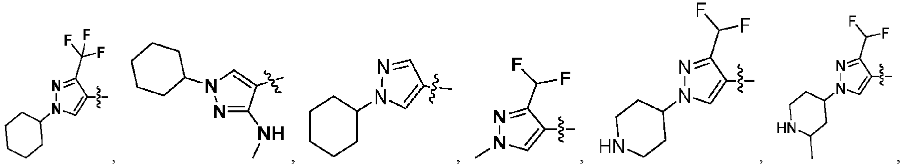 and 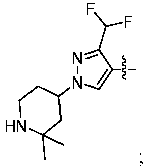
further preferably, 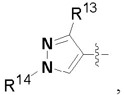 as a whole, is selected from the group consisting of 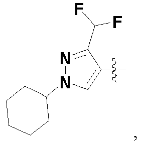 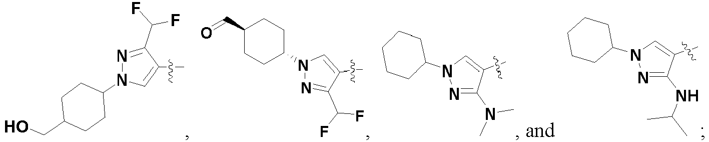
further preferably, 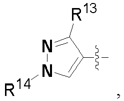 as a whole, is selected from the group consisting of 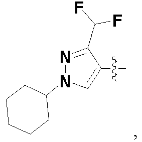 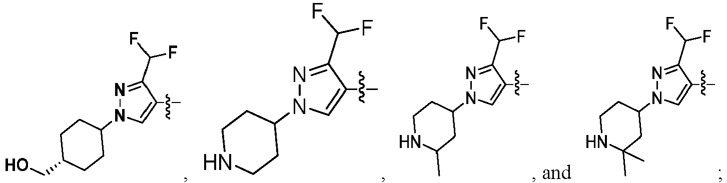
(2) 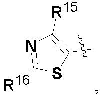 wherein: R¹⁵ is selected from C1-C6 haloalkyl; R¹⁶ is selected from C3-C6 cycloalkyl; further preferably, 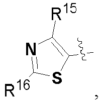 as a whole, is 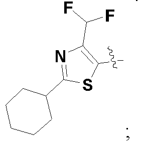
(3) 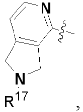 wherein: R¹⁷ is selected from the group consisting of H, and C1-C6 alkyl; preferably, 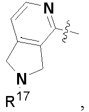 as a whole, is 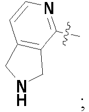
(4) 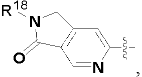 wherein: R¹⁸ is selected from the group consisting of H, and C1-C6 alkyl; preferably, R¹⁸ is selected from C1-C6 alkyl; further preferably, 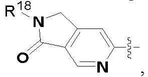 as a whole, is 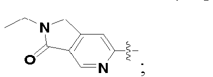
(5) 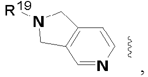 wherein: R¹⁹ is selected from the group consisting of H, and C1-C6 alkyl; preferably, R¹⁹ is selected from C1-C6 alkyl; further preferably, 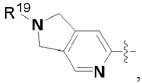 as a whole, is 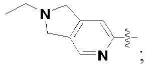
(6) 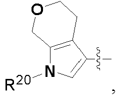 wherein: R²⁰ is selected from C3-C6 cycloalkyl; further preferably, 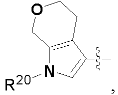 as a whole, is 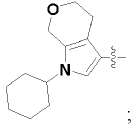
(7) 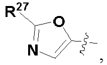 wherein: R²⁷ is selected from the group consisting of phenyl, and C3-C6 cycloalkyl; further preferably, 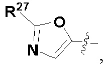 as a whole, is selected from the group consisting of 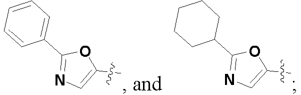
(8) 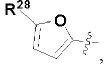 wherein: R²⁸ is selected from 6 membered saturated heterocyclyl; preferably, R²⁸ is morpholinyl; further preferably, 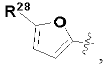 as a whole, is 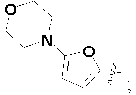
(9) 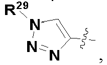 wherein: R²⁹ is selected from C3-C6 alkyl; further preferably, 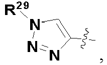 as a whole, is 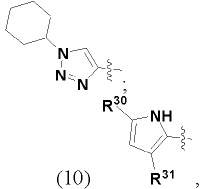 wherein: R³⁰ is selected from C3-C6 cycloalkyl; R³¹ is selected from C1-C6 alkyl; further preferably, 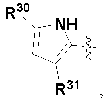 as a whole, is

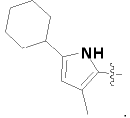

7. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to claim 6, wherein, the compound is represented by formula I-1, 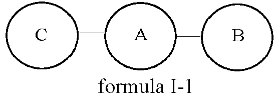 wherein, A, B, C are as defined in claim 6.

8. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 6-7, wherein, the compound is represented by formula I-1-1, 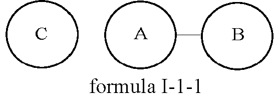
wherein: A is selected from 5-6 membered heteroaromatic ring optionally substituted with substituent R^{k}; preferably, A is selected from the group consisting of 5 membered heteroaromatic ring, and 6 membered heteroaromatic ring, the 5 membered heteroaromatic ring containing 2-3 heteroatoms, wherein at least two heteroatoms are N atoms, the 6 membered heteroaromatic ring containing 1-2 heteroatoms, wherein at least one heteroatom is N atom, and, the 5 membered heteroaromatic ring and 6 membered heteroaromatic ring are optionally substituted with substituent R^{k}; further preferably, A is selected from the group consisting of 1,2,3-triazole ring, 1,2,4-triazole ring, pyrazole ring, imidazole ring, 1,3,4-thiadiazole ring, 1,3,4-oxadiazole ring, 2-pyridone ring, and pyridazine ring, wherein, the 2-pyridone ring is optionally substituted with substituent R^{k};
further preferably, A is selected from the group consisting of 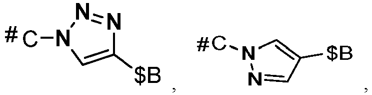 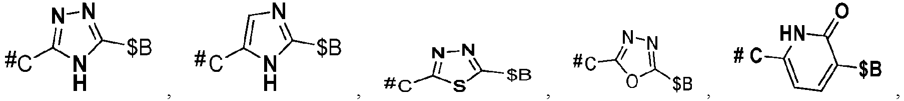 and 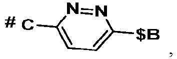 wherein, the 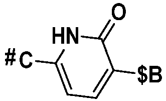 is optionally substituted with substituent R^{k}, wherein #C represents the attachment point to C, $B represents the attachment point to B;
further preferably, A is selected from the group consisting of 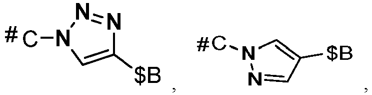 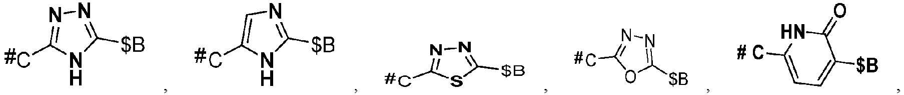 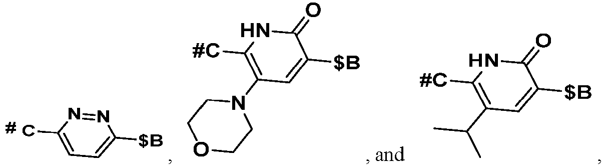 wherein #C represents the attachment point to C, $B represents the attachment point to B;
further preferably, A is selected from the group consisting of 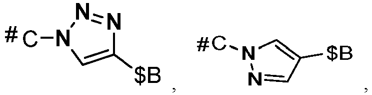 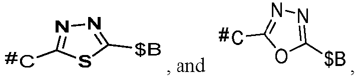 wherein #C represents the attachment point to C, $B represents the attachment point to B;
R^{k} is selected from the group consisting of C1-C6 alkyl, C3-C7 cycloalkyl, R^{p}R^{q}N-, C1-C6 haloalkyl, C1-C6 heteroalkyl (such as C1-C6 alkoxy), 4-9 membered heterocyclyl (such as 5-6 membered saturated heterocyclyl), 6-10 membered aryl, and 5-10 membered heteroaryl; preferably, R^{k} is selected from the group consisting of C1-C6 alkyl, C3-C6 cycloalkyl, R^{p}R^{q}N-, C1-C6 haloalkyl, and 4-9 membered saturated heterocyclyl (such as 5-6 membered saturated heterocyclyl); further preferably, R^{k} is selected from the group consisting of C1-C6 alkyl, C3-C6 cycloalkyl, R^{p}R^{q}N-, C1-C6 haloalkyl, and morpholinyl;
R^{p}, R^{q} are each independently selected from the group consisting of H, C1-C6 alkyl, C1-C6 alkoxy, and C1-C6 haloalkyl; preferably, R^{p}, R^{q} are each independently selected from the group consisting of H, and C1-C6 alkyl;
further preferably, R^{k} is selected from the group consisting of isopropyl, cyclopropyl, dimethylamino, difluoromethyl, and 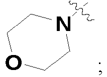
B is selected from any one of groups (1)-(7): 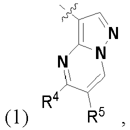 wherein: R⁴ is selected from the group consisting of H, C1-C6 alkyl, C4-C9 cycloalkyl, 4-9 membered saturated heterocyclyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl);
preferably, R⁴ is selected from the group consisting of H, 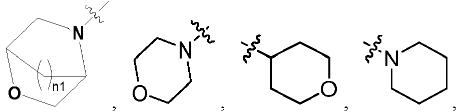 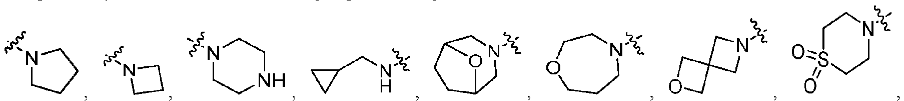 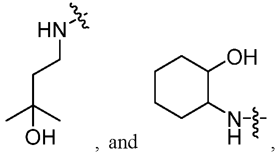 wherein, the 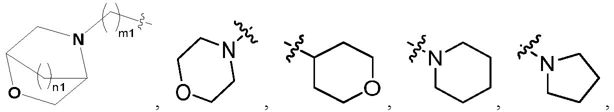 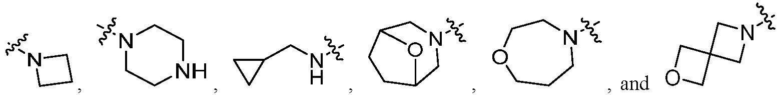 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl); n1 is selected from the group consisting of 0, 1, 2, and 3;
further preferably, R⁴ is selected from the group consisting of H, 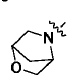 (preferably 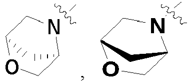 ), 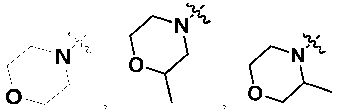 (preferably 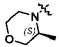 ), 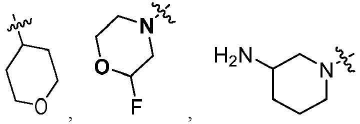 (preferably 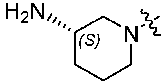 or 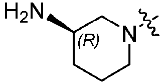 ), 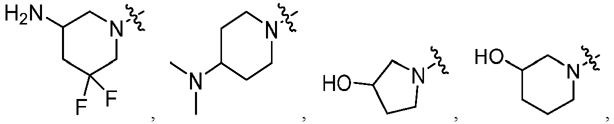 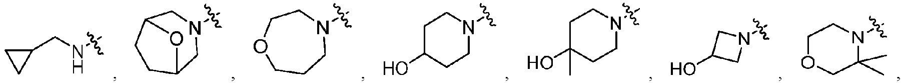 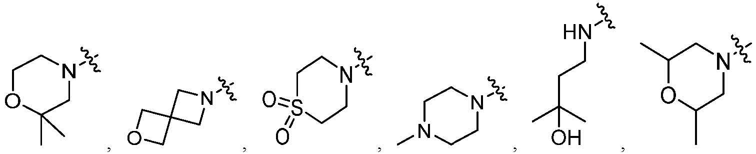 (preferably 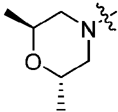 ), 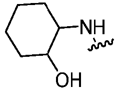 (preferably 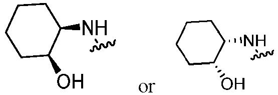 ), and 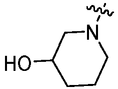 (preferably 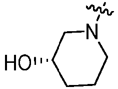 or 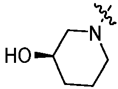 );
R⁵ is selected from the group consisting of H, cyano, C1-C6 alkyl, 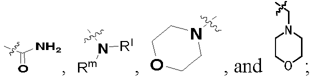 preferably, R⁵ is selected from the group consisting of H, cyano, C1-C6 alkyl, 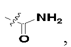 and 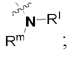 preferably, R⁵ is selected from the group consisting of H, cyano, 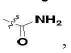 and 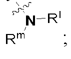 preferably, R⁵ is selected from the group consisting of H, cyano, and 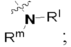
R^{l}, R^{m} are each independently selected from the group consisting of H, C1-C6 alkyl, and C3-C6 cycloalkyl, preferably, R^{l}, R^{m} are each independently selected from the group consisting of H, and C1-C6 alkyl;
further preferably, 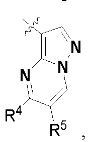 as a whole, is selected from the group consisting of 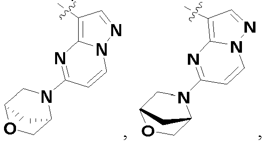 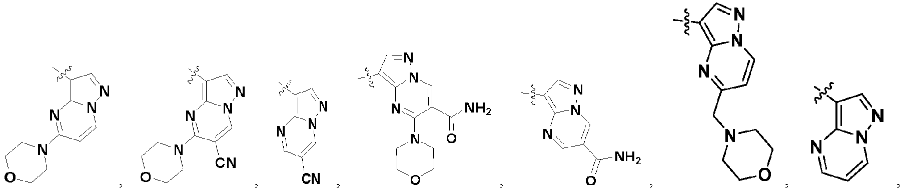 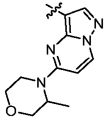 (preferably 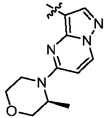 ), 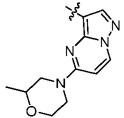 (preferably 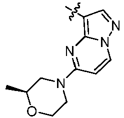 ), 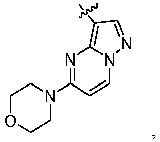 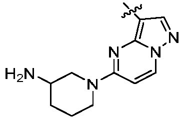 ( 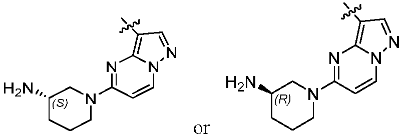 ), 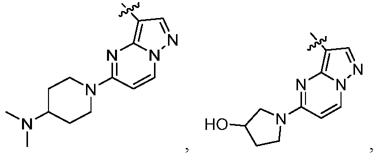 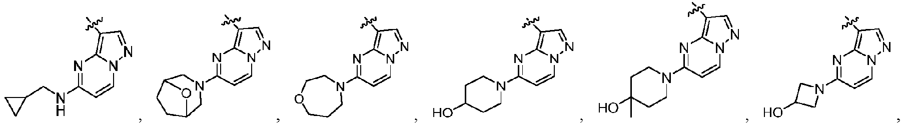 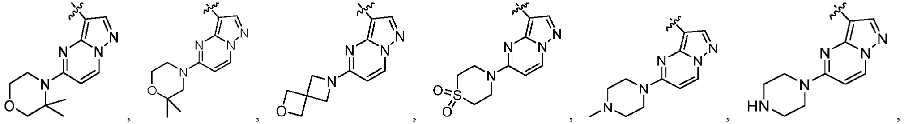 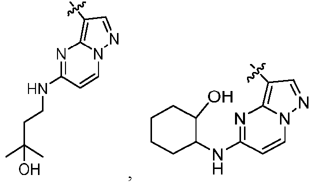 (preferably 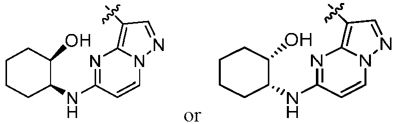 ), 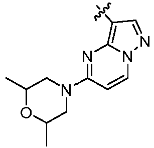 (preferably 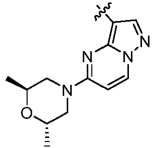 ), 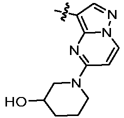 (preferably 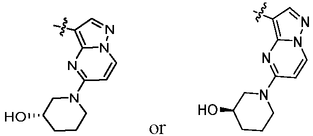 ), and 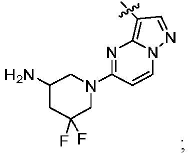
further preferably, 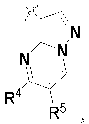 as a whole, is selected from the group consisting of 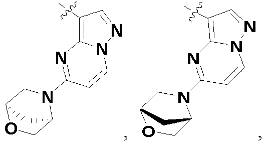 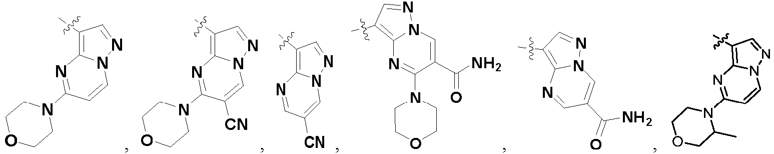 (preferably 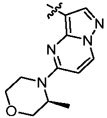 ), 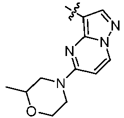 (preferably 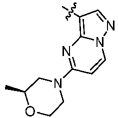 ), 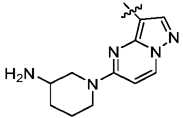 ( 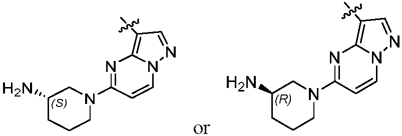 ), 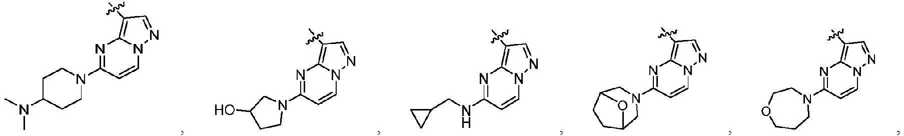 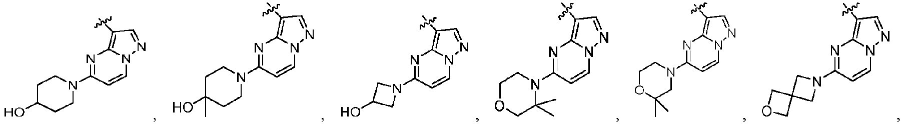 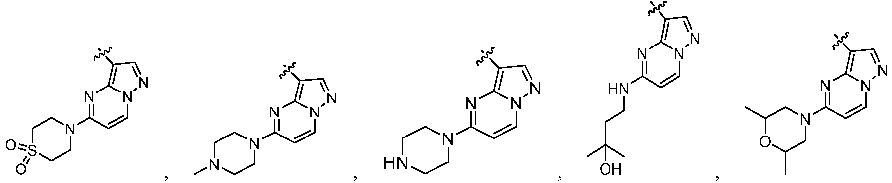 (preferably 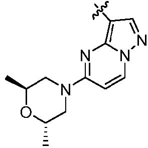 ), 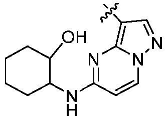 (preferably 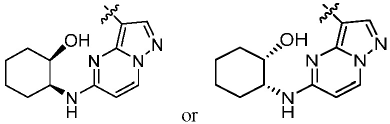 ), 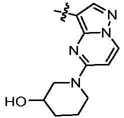 (preferably 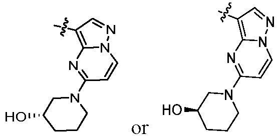 ), and 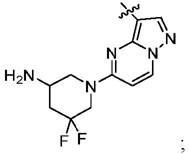
further preferably, 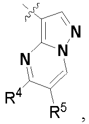 as a whole, is selected from the group consisting of 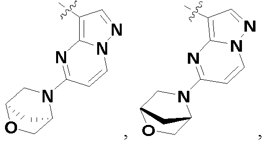 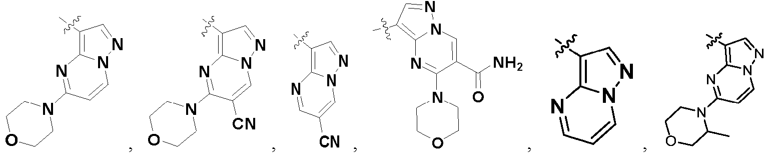 (preferably 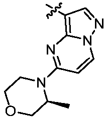 ), 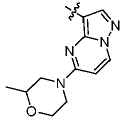 (preferably 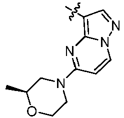 ), 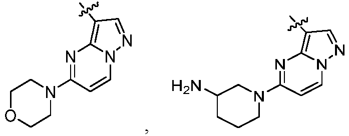 ( 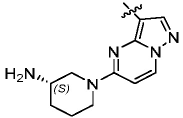 or 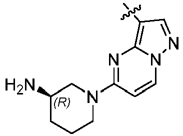 ), 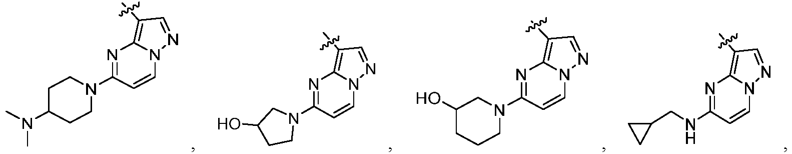 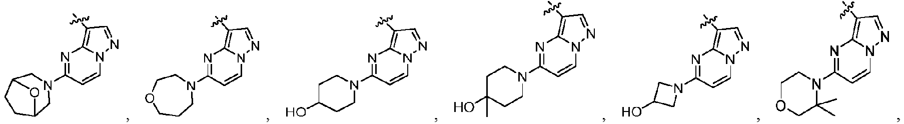 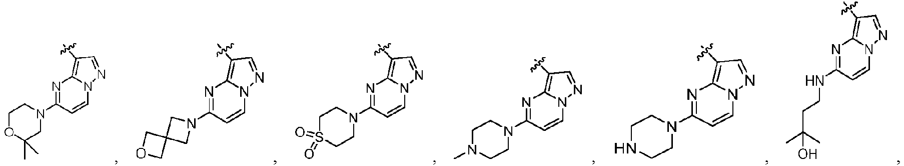 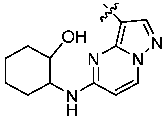 (preferably 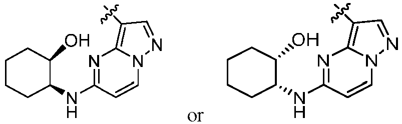 ), 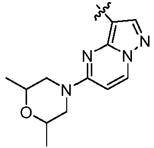 (preferably 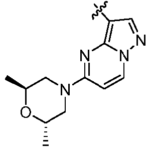 ), 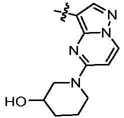 (preferably 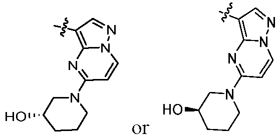 ), and 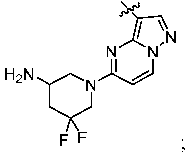
further preferably, 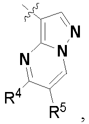 as a whole, is selected from the group consisting of 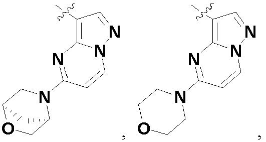 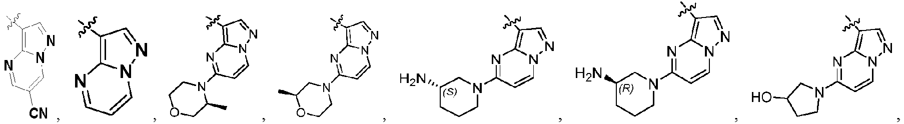 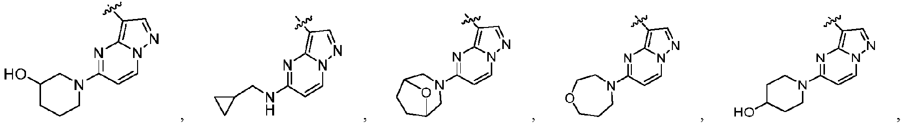 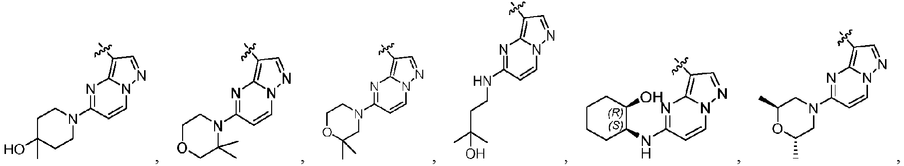 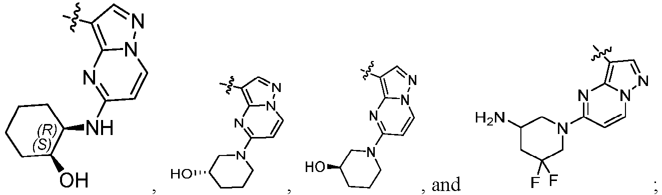
(2) 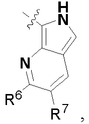 wherein: R⁶ is selected from the group consisting of H, C1-C6 alkyl, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R⁶ is selected from 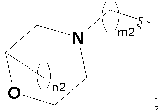 m2 is selected from the group consisting of 0, 1, 2, and 3; n2 is selected from the group consisting of 0, 1, 2, and 3; further preferably, R⁶ is 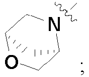
R⁷ is selected from the group consisting of H, and C1-C6 alkyl; further preferably, 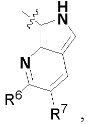 as a whole, is 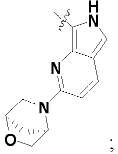
(3) 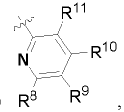 wherein: one of R⁸, R⁹, R¹⁰, and R¹¹ is selected from the group consisting of cyano, C1-C6 haloalkyl (such as trifluoromethyl), C4-C9 cycloalkyl, C4-C9 cycloalkyl-C1-C6 alkyl, 4-9 membered saturated heterocyclyl, and 4-9 membered saturated heterocyclyl-C1-C6 alkyl, and the rest are H;
preferably, among R⁸, R⁹, R¹⁰, and R¹¹, R⁸, R⁹ or R¹¹ is selected from the group consisting of cyano, C1-C6 haloalkyl (such as trifluoromethyl), and 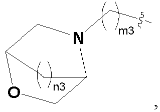 and the rest are H; m3 is selected from the group consisting of 0, 1, 2, and 3; n3 is selected from the group consisting of 0, 1, 2, and 3;
further preferably, 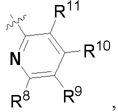 as a whole, is selected from the group consisting of 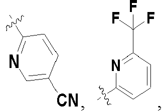 and 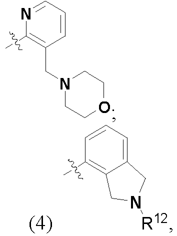 wherein: R¹² is selected from the group consisting of H, and C1-C6 alkyl; preferably, R¹² is selected from C1-C6 alkyl;
(5) 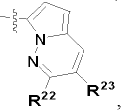 wherein: R²² is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²² is selected from the group consisting of H, and 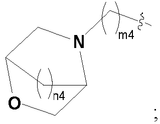 m4 is selected from the group consisting of 0, 1, 2, and 3; m4 is preferably 0; n4 is selected from the group consisting of 0, 1, 2, and 3; n4 is preferably 0, or 1; further preferably, R²² is selected from the group consisting of H, and 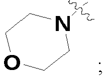
R²³ is selected from the group consisting of H, C1-C6 alkyl, cyano, 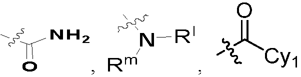 (wherein Cy₁ is 4-9 membered heterocyclyl optionally substituted with R_{cy1} and R_{cy2}), and 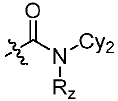 (wherein R_{z} is H or C1-C6 alkyl, Cy₂ is 5-7 membered heterocyclyl, C3-C7 cycloalkyl or C6-C10 aryl, wherein, the 5-7 membered heterocyclyl, C3-C7 cycloalkyl and C6-C10 aryl are optionally substituted with 1-3 substituents selected from the group consisting of halogen, C1-C6 alkyl, C1-C6 alkoxy, CN, and hydroxy);
preferably, Cy₁ is 5-7 membered heterocyclyl, further preferably, Cy₁ is 6 membered heterocyclyl, further preferably, Cy₁ is morpholinyl, piperazinyl, or thiomorpholinyl ;
R_{cy1} and R_{cy2} are each independently selected from the group consisting of C1-C6 alkyl, C1-C6 alkoxy, C1-C6 haloalkyl, halogen, hydroxy, nitro, cyano, amino, -NH(C1-C6 alkyl), -N(C1-C6 alkyl)(C1-C6 alkyl), and oxo; preferably, R_{cy1} and R_{cy2} are each independently selected from the group consisting of C1-C6 alkyl, halogen, hydroxy, amino, and oxo, further preferably R_{cy1} and R_{cy2} are oxo;
preferably, Cy₂ is 5-7 membered heterocyclyl optionally substituted with 1-3 substituents selected from the group consisting of halogen, C1-C6 alkyl, C1-C6 alkoxy, CN, and hydroxy; further preferably, Cy₂ is selected from the group consisting of azetidinyl, pyrrolidinyl, imidazolidinyl, piperidyl, piperazinyl, and morpholinyl, wherein, the azetidinyl, pyrrolidinyl, imidazolidinyl, piperidyl, piperazinyl, and morpholinyl are optionally substituted with 1-3 substituents selected from the group consisting of halogen, and C1-C6 alkyl, further preferably, Cy₂ is selected from the group consisting of pyrrolidinyl, imidazolidinyl, and piperidyl, wherein, the pyrrolidinyl, imidazolidinyl, and piperidyl are optionally substituted with 1-3 substituents selected from the group consisting of F, Cl, Br, and methyl;
preferably, R²³ is selected from the group consisting of H, C1-C6 alkyl, cyano, 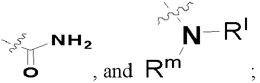
preferably, R²³ is selected from the group consisting of H, cyano, 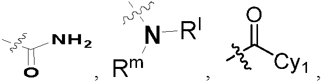 and 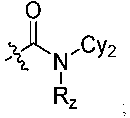 preferably, R²³ is selected from the group consisting of H, cyano, 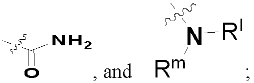
R^{l}, R^{m} are each independently selected from the group consisting of H, C1-C6 alkyl, and C3-C6 cycloalkyl; preferably, R^{l}, R^{m} are each independently selected from the group consisting of H, and C1-C6 alkyl,
further preferably, 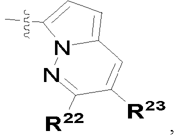 as a whole, is selected from the group consisting of 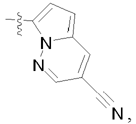 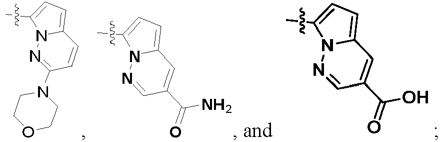
further preferably, 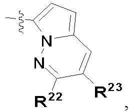 as a whole, is selected from the group consisting of 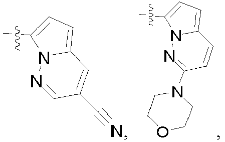 and 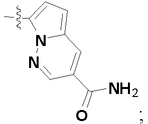
further preferably, 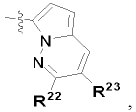 as a whole, is selected from the group consisting of 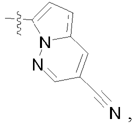 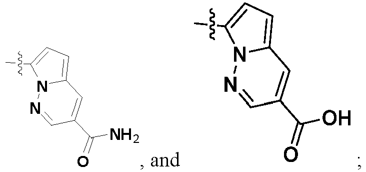
(6) 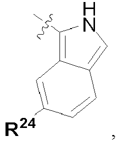 wherein: R²⁴ is selected from the group consisting of H, C1-C6 alkyl, C4-C9 cycloalkyl, C4-C9 cycloalkyl-C1-C6 alkyl, 4-9 membered saturated heterocyclyl, and 4-9 membered saturated heterocyclyl-C1-C6 alkyl;
preferably, R²⁴ is selected from 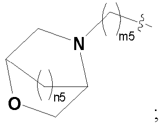 m5 is selected from the group consisting of 0, 1, 2, and 3; n5 is selected from the group consisting of 0, 1, 2, and 3; further preferably, R²⁴ is 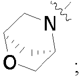 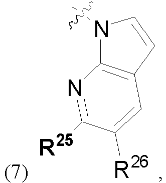
wherein: R²⁵ is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl; preferably, R²⁵ is selected from the group consisting of H, and 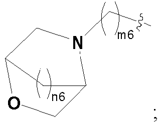 m6 is selected from the group consisting of 0, 1, 2, and 3; m6 is preferably 0; n6 is selected from the group consisting of 0, 1, 2, and 3; n6 is preferably 0, or 1;
further preferably, R²⁵ is selected from the group consisting of H, and 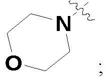
R²⁶ is selected from the group consisting of H, C1-C6 alkyl, cyano, carboxyl, and 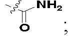 preferably, R²⁶ is selected from the group consisting of H, C1-C6 alkyl, cyano, and 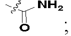 preferably, R²⁶ is selected from the group consisting of H, cyano, and 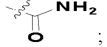
further preferably, 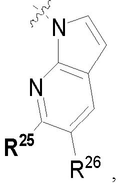 as a whole, is selected from the group consisting of 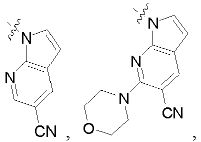 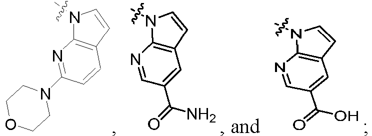
further preferably, 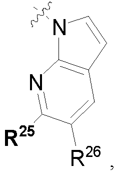 as a whole, is selected from the group consisting of 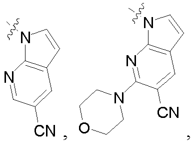 and 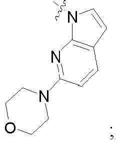
further preferably, 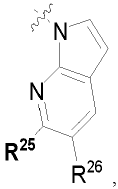 as a whole, is selected from the group consisting of 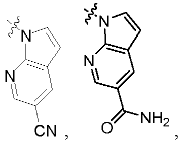 and 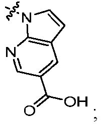
C is selected from any one of groups (1)-(11): 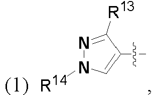 wherein: R¹³ is selected from the group consisting of C1-C6 haloalkyl (such as difluoromethyl), and 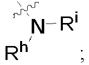
R^{h}, Rⁱ are each independently selected from the group consisting of H, and C1-C6 alkyl; preferably, R^{h}, Rⁱ are each independently selected from the group consisting of H, methyl, and isopropyl;
preferably, R¹³ is selected from the group consisting of 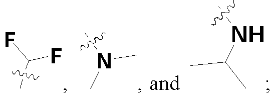 further preferably, R¹³ is selected from the group consisting of 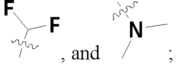
R¹⁴ is selected from C3-C6 cycloalkyl optionally substituted with 1-2 substituents selected from the group consisting of hydroxy C1-C6 alkyl (such as methylol), and formyl; or R¹⁴ is selected from the group consisting of phenyl, morpholinyl, piperazinyl, and piperidyl, wherein, the phenyl, morpholinyl, piperazinyl, and piperidyl are optionally substituted with 1-2 substituents selected from the group consisting of C1-C6 alkyl, halogen, alkoxy (such as C1-C6 alkoxy), hydroxy, amino, hydroxy C1-C6 alkyl (such as methylol), amino C1-C6 alkyl, and formyl, preferably, R¹⁴ is selected from 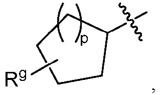 p is selected from the group consisting of 0, 1, and 2;
or preferably, R¹⁴ is piperidyl optionally substituted with 1-2 substituents selected from the group consisting of C1-C6 alkyl (preferably methyl), halogen, hydroxy, and amino;
R^{g} is selected from the group consisting of H, hydroxy C1-C6 alkyl, and formyl;
further preferably, R¹⁴ is selected from the group consisting of 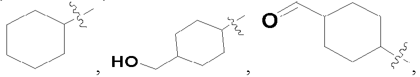 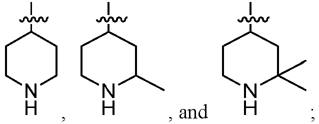
further preferably, R¹⁴ is selected from the group consisting of 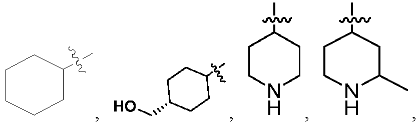 and 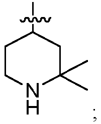
further preferably, 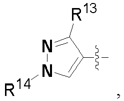 as a whole, is selected from the group consisting of 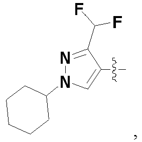 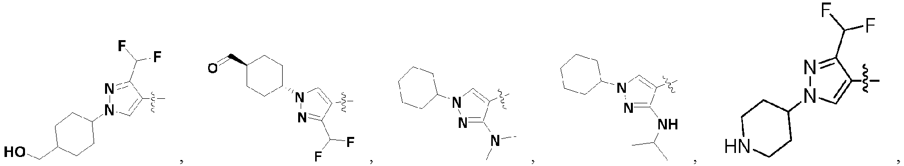 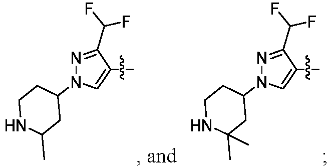
further preferably, 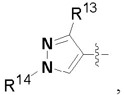 as a whole, is selected from the group consisting of 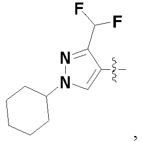 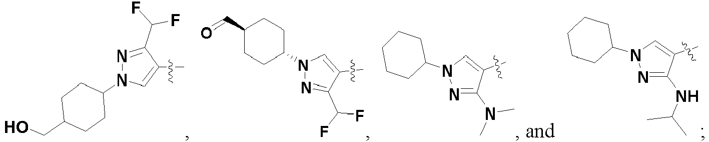
further preferably, 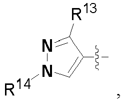 as a whole, is selected from the group consisting of 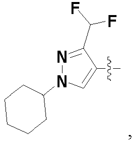 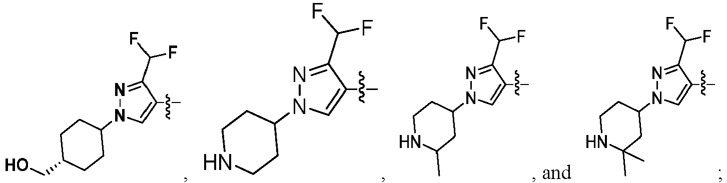 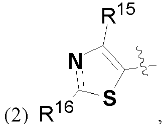
wherein: R¹⁵ is selected from C1-C6 haloalkyl; R¹⁶ is selected from C3-C6 cycloalkyl; further preferably, 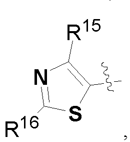 as a whole, is 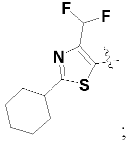
(3) 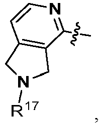 wherein: R¹⁷ is selected from the group consisting of H, and C1-C6 alkyl; preferably, 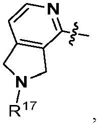 as a whole, is 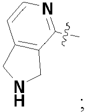
(4) 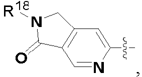 wherein: R¹⁸ is selected from the group consisting of H, and C1-C6 alkyl; preferably, R¹⁸ is selected from C1-C6 alkyl; further preferably, 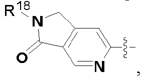 as a whole, is 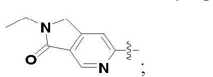
(5) 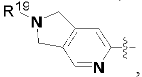 wherein: R¹⁹ is selected from the group consisting of H, and C1-C6 alkyl; preferably, R¹⁹ is selected from C1-C6 alkyl; further preferably, 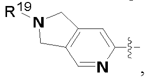 as a whole, is 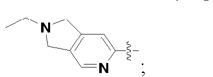
(6) 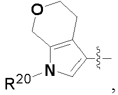 wherein: R²⁰ is selected from C3-C6 cycloalkyl; further preferably, 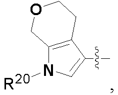 as a whole, is 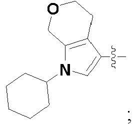
(7) 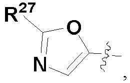 wherein: R²⁷ is selected from the group consisting of phenyl, and C3-C6 cycloalkyl; further preferably, 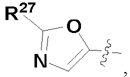 as a whole, is selected from the group consisting of 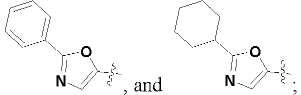
(8) 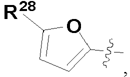 wherein: R²⁸ is selected from 6 membered saturated heterocyclyl; preferably, R²⁸ is morpholinyl; further preferably, 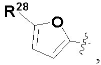 as a whole, is 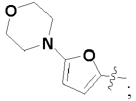
(9) 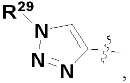 wherein: R²⁹ is selected from C3-C6 alkyl; further preferably, 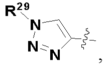 as a whole, is 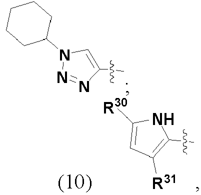 wherein: R³⁰ is selected from C3-C6 cycloalkyl; R³¹ is selected from C1-C6 alkyl;
further preferably, 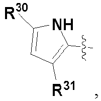 as a whole, is 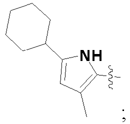
(11) 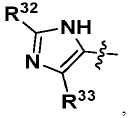 wherein: R³² is selected from the group consisting of phenyl, and C3-C6 cycloalkyl; R³³ is selected from the group consisting of C1-C6 alkyl, and C1-C6 haloalkyl (such as trifluoromethyl, difluoromethyl); further preferably, 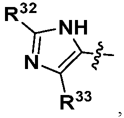 as a whole, is

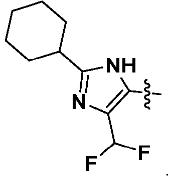

9. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to claim 1, wherein, the compound is represented by formula II, 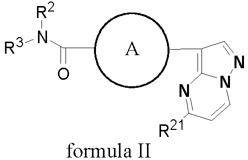 wherein:
A is selected from 5 membered heteroaromatic ring; preferably, A is selected from 5 membered heteroaromatic ring containing 2-3 heteroatoms, wherein at least two heteroatoms are N atoms; further preferably, A is selected from the group consisting of 1,2,3-triazole ring, and imidazole ring; further preferably, A is selected from the group consisting of 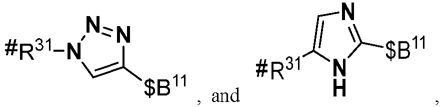 wherein #R³¹ represents the attachment point to carbonyl, $B¹¹ represents the attachment point to fused bicyclic ring;
R², R³ are each independently selected from the group consisting of H, C1-C6 alkyl, and C3-C6 cycloalkyl; or, R², R³ and the N atom to which they are both attached together form 6-membered saturated heterocyclyl, preferably, the 6 membered saturated heterocyclyl contains 2 heteroatoms, wherein, the heteroatoms are N atom; preferably, R², R³ are each independently selected from the group consisting of H, methyl, ethyl, isopropyl, and 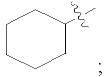
or preferably, R², R³ and the N atom to which they are both attached together form 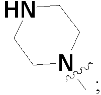 further preferably, 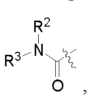 as a whole, is selected from the group consisting of 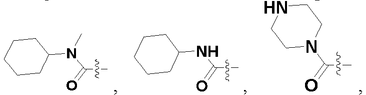 and 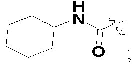
R²¹ is selected from the group consisting of C4-C9 cycloalkyl, C4-C9 cycloalkyl-C1-C6 alkyl, 4-9 membered saturated heterocyclyl, and 4-9 membered saturated heterocyclyl-C1-C6 alkyl;
preferably, R²¹ is selected from 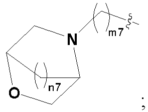 m7 is selected from the group consisting of 0, 1, 2, and 3; n7 is selected from the group consisting of 0, 1, 2, and 3;
preferably, R²¹ is selected from the group consisting of

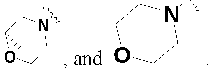

10. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 1-9, wherein, the compound is represented by formula III, 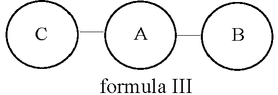 wherein:
A is selected from the group consisting of 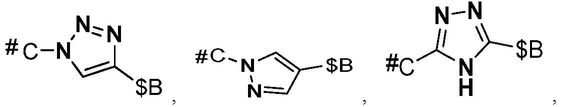 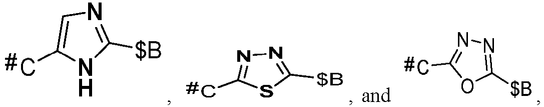 wherein #C represents the attachment point to C, $B represents the attachment point to B;
further preferably, A is selected from the group consisting of 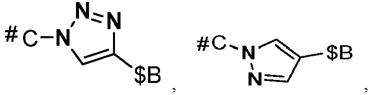 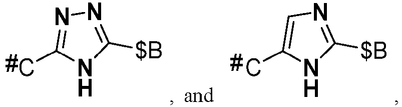 wherein #C represents the attachment point to C, $B represents the attachment point to B;
further preferably, A is 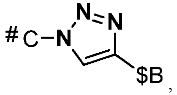 wherein #C represents the attachment point to C, $B represents the attachment point to B;
B is 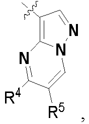 wherein: R⁴ is selected from the group consisting of H, C1-C6 alkyl, C4-C9 cycloalkyl, 4-9 membered saturated heterocyclyl, and -NR_{Na}R_{Nb} (wherein R_{Na} and R_{Nb} are each independently selected from the group consisting of H, and C1-C6 alkyl, wherein, the C1-C6 alkyl is optionally substituted with 1-3 substituents selected from the group consisting of halogen, hydroxy, C3-C6 cycloalkyl, C3-C6 halocycloalkyl, and 4-7 membered heterocyclyl), wherein, the C4-C9 cycloalkyl, and 4-9 membered saturated heterocyclyl are optionally substituted with 1-3 substituents selected from the group consisting of C1-C6 alkyl, C1-C6 alkoxy, C1-C6 haloalkyl, C1-C6 haloalkoxy, halogen, hydroxy, hydroxy-C1-C6 alkyl-, cyano, oxo, -NH₂, -N(C1-C6 alkyl), and -N(C1-C6 alkyl)(C1-C6 alkyl);
preferably, R⁴ is selected from the group consisting of H, 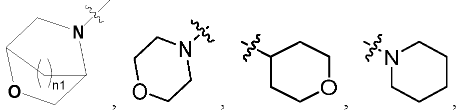 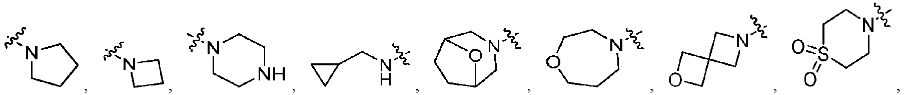 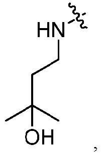 and 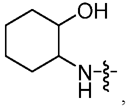 wherein, the 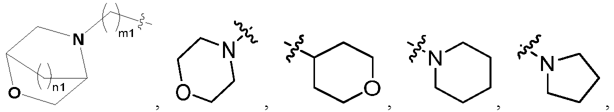 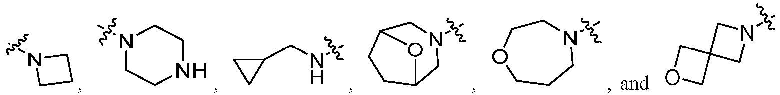 are optionally substituted with 1-3 substituents selected from the group consisting of C1-C6 alkyl, halogen, hydroxy, cyano, -NH₂, -N(C1-C6 alkyl), and -N(C1-C6 alkyl)(C1-C6 alkyl);
n1 is selected from the group consisting of 0, 1, 2, and 3; preferably n1 is 0;
further preferably, R⁴ is selected from the group consisting of H, 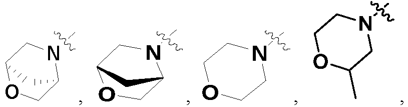 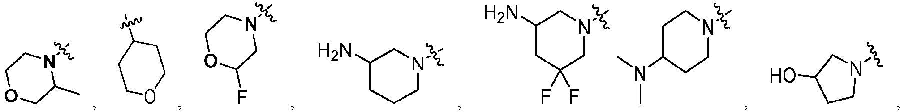 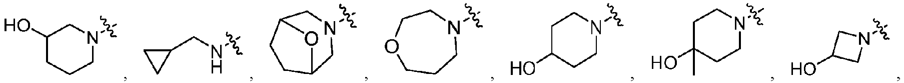 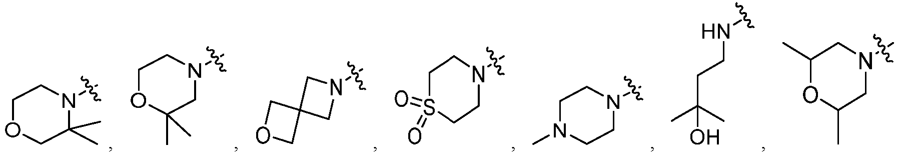 (preferably 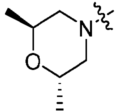 ), 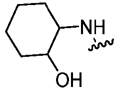 (preferably 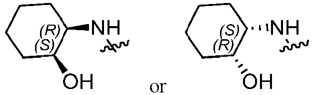 ), and 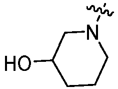 (preferably 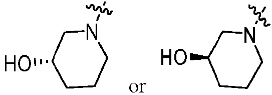 );
further preferably, R⁴ is selected from the group consisting of H, 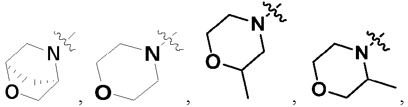 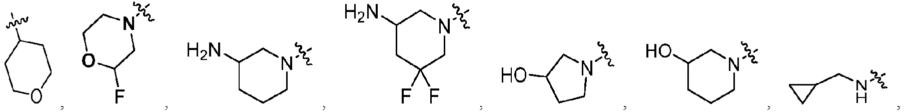 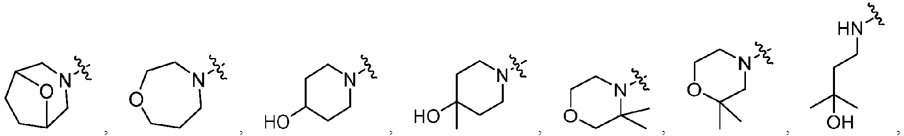 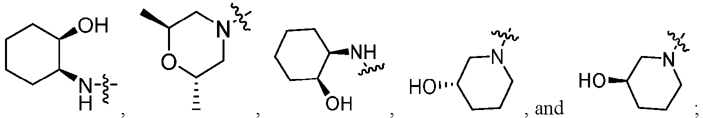
R⁵ is selected from the group consisting of H, cyano, C1-C6 alkyl, 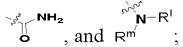
preferably, R⁵ is selected from the group consisting of H, and cyano;
preferably, 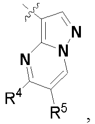 as a whole, is selected from the group consisting of 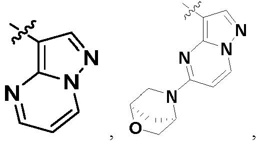 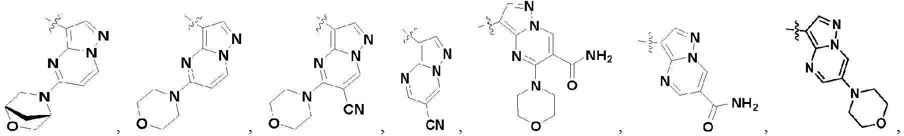 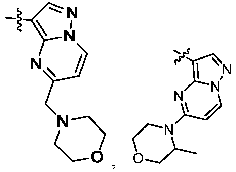 (preferably 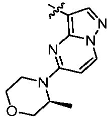 ), 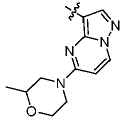 (preferably 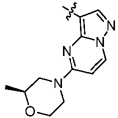 ), 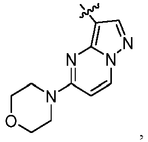 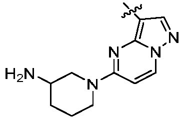 ( 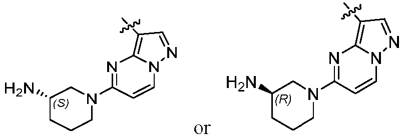 ), 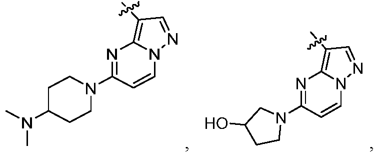 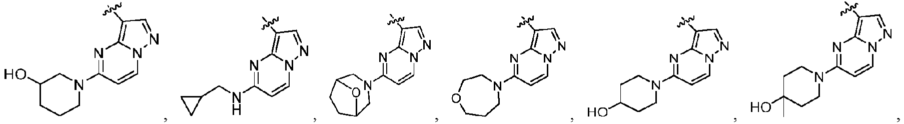 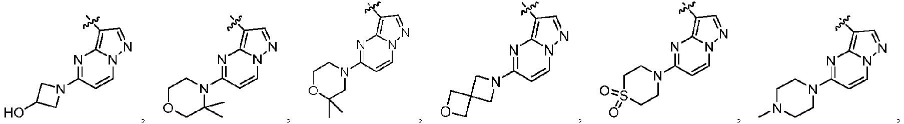 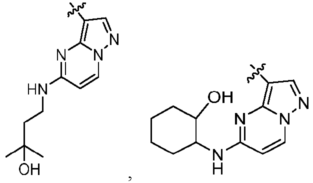 (preferably 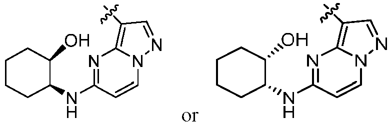 ), 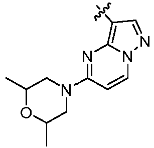 (preferably 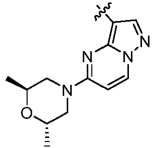 ), 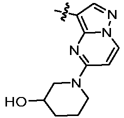 (preferably 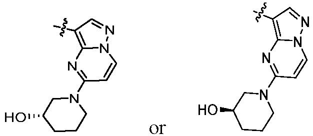 ), and 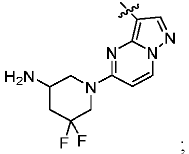
further preferably, 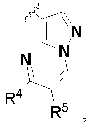 as a whole, is selected from the group consisting of 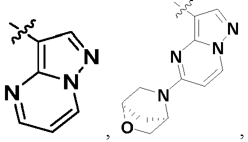 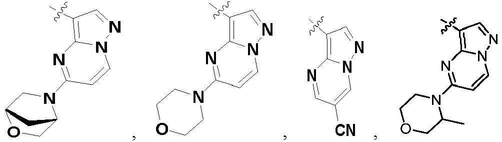 (preferably 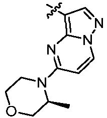 ), 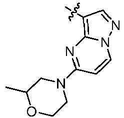 (preferably 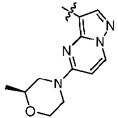 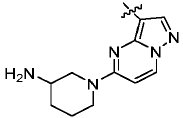 ( 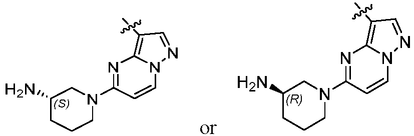 ), 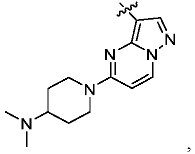 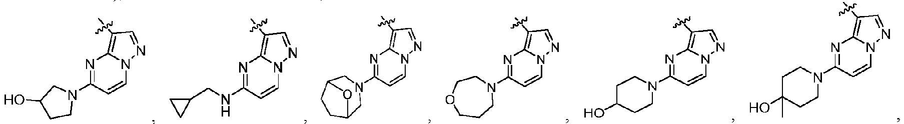 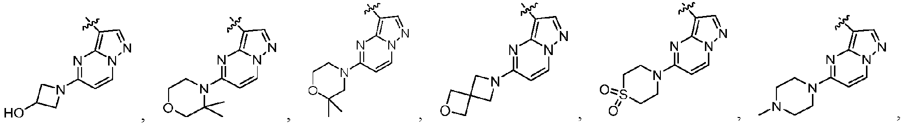 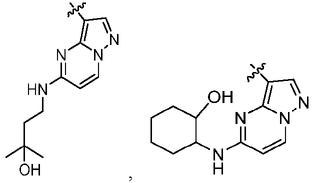 (preferably 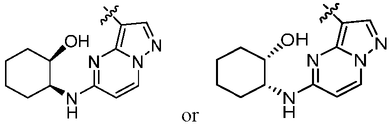 ), 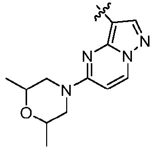 (preferably 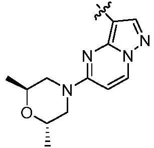 ), 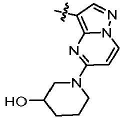 (preferably 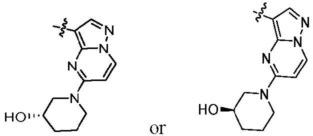 ), and 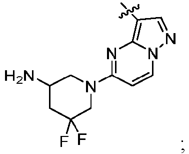
further preferably, 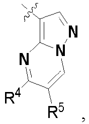 as a whole, is selected from the group consisting of 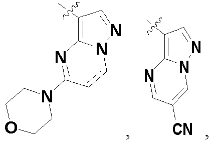 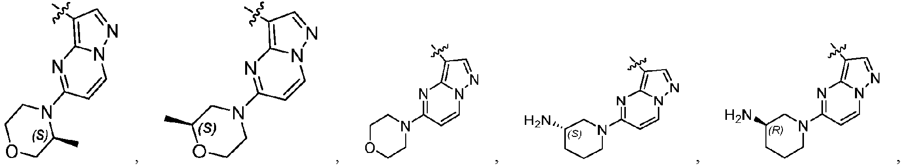 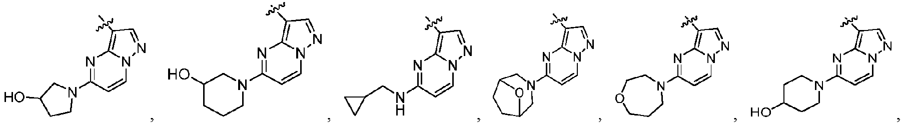 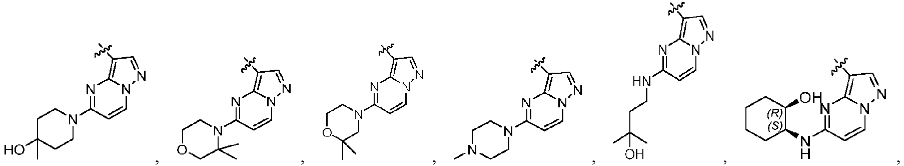 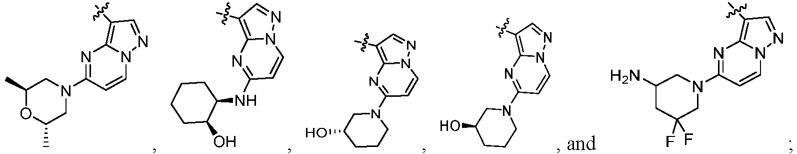
C is 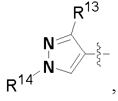 wherein: R¹³ is selected from the group consisting of C1-C6 haloalkyl (such as difluoromethyl or trifluoromethyl), and 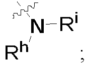
R^{h}, Rⁱ are each independently selected from the group consisting of H, and C1-C6 alkyl; preferably, R^{h}, Rⁱ are each independently selected from the group consisting of H, methyl, and isopropyl;
preferably, R¹³ is selected from C1-C6 haloalkyl (such as difluoromethyl); preferably, R¹³ is selected from the group consisting of 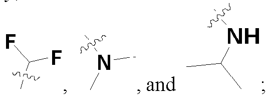 further preferably, R¹³ is selected from the group consisting of 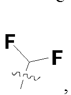 and 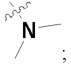
R¹⁴ is selected from the group consisting of C3-C6 cycloalkyl, piperidyl, piperazinyl, and morpholinyl, wherein, the C3-C6 cycloalkyl, piperidyl, piperazinyl, and morpholinyl are optionally substituted with 1-2 substituents selected from the group consisting of hydroxy C1-C6 alkyl (such as methylol), formyl, and C1-C6 alkyl (preferably methyl); preferably, R¹⁴ is selected from the group consisting of 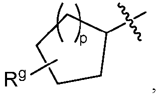 and piperidyl, wherein, the piperidyl is optionally substituted with 1-2 substituents selected from C1-C6 alkyl (preferably methyl);
p is selected from the group consisting of 0, 1, and 2; preferably, p is 2;
R^{g} is selected from the group consisting of H, hydroxy C1-C6 alkyl, and formyl; preferably, R^{g} is H;
further preferably, R¹⁴ is selected from the group consisting of 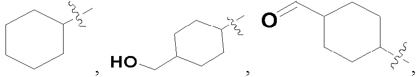 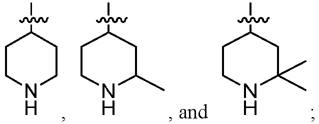
further preferably, R¹⁴ is selected from the group consisting of 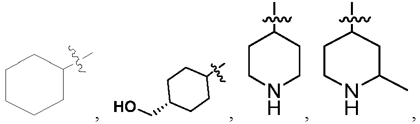 and 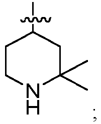
further preferably, 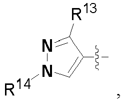 as a whole, is selected from the group consisting of 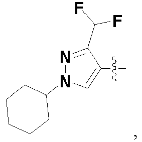 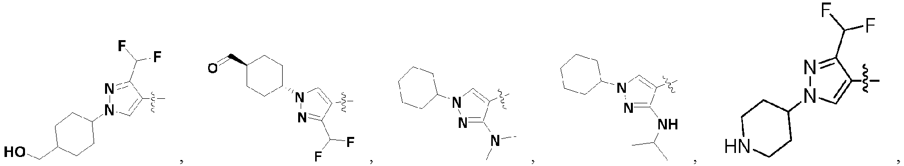 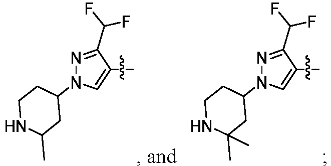
further preferably, 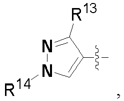 as a whole, is selected from the group consisting of

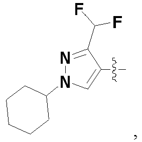

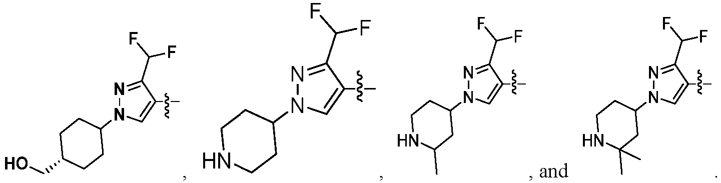

11. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 1-9, wherein, the compound is represented by formula IV, 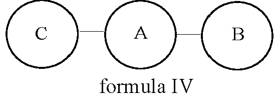 wherein:
A is selected from the group consisting of 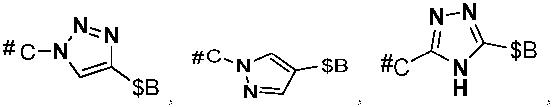 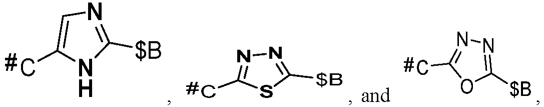 wherein #C represents the attachment point to C, $B represents the attachment point to B;
further preferably, A is selected from the group consisting of 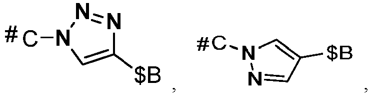 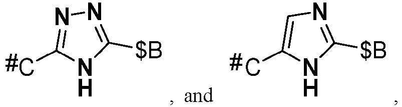 wherein #C represents the attachment point to C, $B represents the attachment point to B;
further preferably, A is 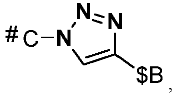 wherein #C represents the attachment point to C, $B represents the attachment point to B;
B is 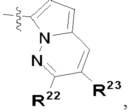 wherein: R²² is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl,
preferably, R²² is selected from the group consisting of H, and 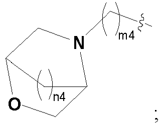 m4 is selected from the group consisting of 0, 1, 2, and 3; preferably, m4 is 0; n4 is selected from the group consisting of 0, 1, 2, and 3; preferably, n4 is selected from the group consisting of 0, and 1; further preferably, R²² is selected from the group consisting of H, and 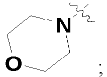
R²³ is selected from the group consisting of H, C1-C6 alkyl, cyano, carboxyl, 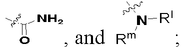 preferably, R²³ is selected from the group consisting of H, carboxyl, and 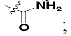
R^{l}, R^{m} are each independently selected from the group consisting of H, C1-C6 alkyl, and C3-C6 cycloalkyl; preferably, R^{l}, R^{m} are each independently selected from the group consisting of H, and C1-C6 alkyl;
further preferably, 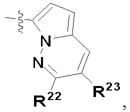 as a whole, is selected from the group consisting of 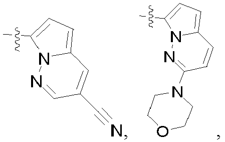 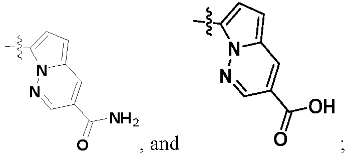
C is 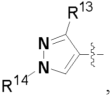 wherein: R¹³ is selected from the group consisting of C1-C6 haloalkyl (such as difluoromethyl or
trifluoromethyl), and 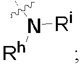
R^{h}, Rⁱ are each independently selected from the group consisting of H, and C1-C6 alkyl; preferably, R^{h}, Rⁱ are each independently selected from the group consisting of H, methyl, and isopropyl;
preferably, R¹³ is selected from C1-C6 haloalkyl (such as difluoromethyl); preferably, R¹³ is selected from the group consisting of 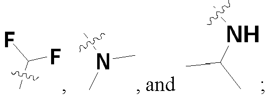 further preferably, R¹³ is selected from the group consisting of 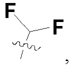 and 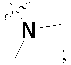
R¹⁴ is selected from the group consisting of C3-C6 cycloalkyl, piperidyl, piperazinyl, and morpholinyl, wherein, the C3-C6 cycloalkyl, piperidyl, piperazinyl, and morpholinyl are optionally substituted with 1-2 substituents selected from the group consisting of hydroxy C1-C6 alkyl (such as methylol), formyl, and C1-C6 alkyl (preferably methyl); preferably, R¹⁴ is selected from the group consisting of 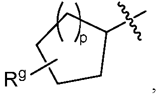 and piperidyl, wherein, the piperidyl is optionally substituted with 1-2 substituents selected from C1-C6 alkyl (preferably methyl);
p is selected from the group consisting of 0, 1, and 2; preferably, p is 2;
R^{g} is selected from the group consisting of H, hydroxy C1-C6 alkyl, and formyl; preferably, R^{g} is H;
further preferably, R¹⁴ is selected from the group consisting of 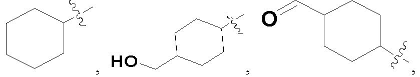 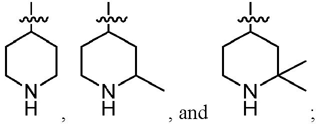
further preferably, R¹⁴ is selected from the group consisting of 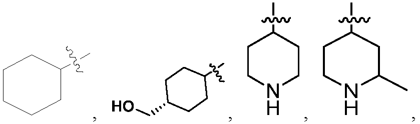 and 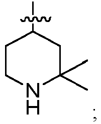
further preferably, 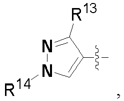 as a whole, is selected from the group consisting of 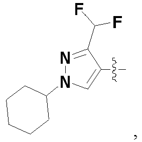 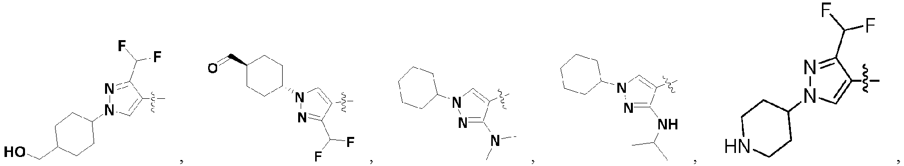 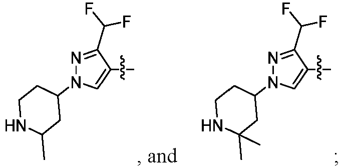
further preferably, 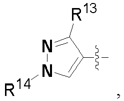 as a whole, is selected from the group consisting of

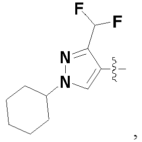

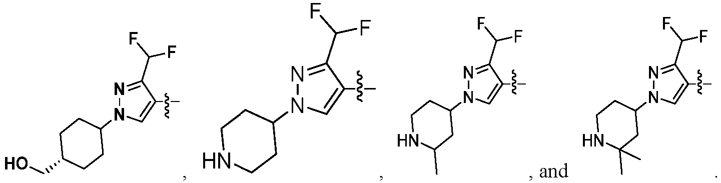

12. The compound, or a stereoisomer, a tautomer, a prodrug, a crystal form, a hydrate, an isotope-labeled compound (a deuteride), a metabolite, an ester, a pharmaceutically acceptable salt or a pharmaceutically acceptable solvate thereof according to anyone of claims 1-9, wherein, the compound is represented by formula V, 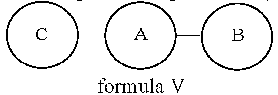 wherein:
A is selected from the group consisting of 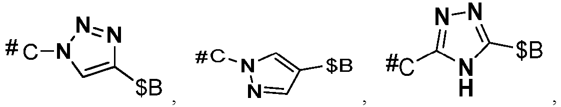 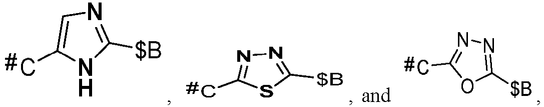 wherein #C represents the attachment point to C, $B represents the attachment point to B;
further preferably, A is selected from the group consisting of 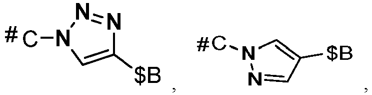 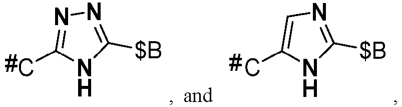 wherein #C represents the attachment point to C, $B represents the attachment point to B;
further preferably, A is 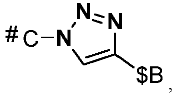 wherein #C represents the attachment point to C, $B represents the attachment point to B;
B is 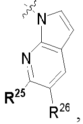 wherein: R²⁵ is selected from the group consisting of H, C4-C9 cycloalkyl, C4-C9 cycloalkyl-C1-C6 alkyl, 4-9 membered saturated heterocyclyl, and 4-9 membered saturated heterocyclyl-C1-C6 alkyl,
preferably, R²⁵ is selected from the group consisting of H, and 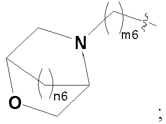 m6 is selected from the group consisting of 0, 1, 2, and 3; preferably, m6 is 0; n6 is selected from the group consisting of 0, 1, 2, and 3; preferably, n6 is 0, or 1;
further preferably, R²⁵ is selected from the group consisting of H, and 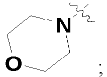
R²⁶ is selected from the group consisting of H, C1-C6 alkyl, cyano, carboxyl, and 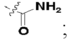 preferably, R²⁶ is selected from the group consisting of H, cyano, carboxyl, and 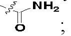
further preferably, 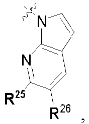 as a whole, is selected from the group consisting of 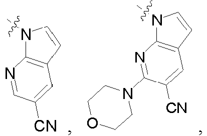 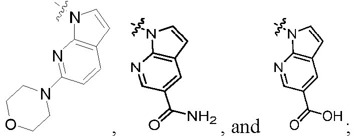
further preferably, 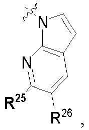 as a whole, is selected from the group consisting of 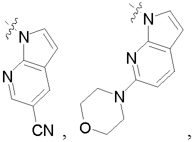 and 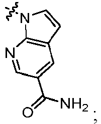
Cis 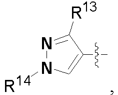 wherein:
R¹³ is selected from the group consisting of C1-C6 haloalkyl (such as difluoromethyl or trifluoromethyl), and 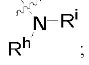 R^{h}, Rⁱ are each independently selected from the group consisting of H, and C1-C6 alkyl; preferably, R^{h}, Rⁱ are each independently selected from the group consisting of H, methyl, and isopropyl; preferably, R¹³ is selected from C1-C6 haloalkyl (such as difluoromethyl); preferably, R¹³ is selected from the group consisting of 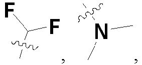 and 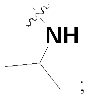
further preferably, R¹³ is selected from the group consisting of 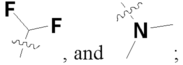
R¹⁴ is selected from the group consisting of C3-C6 cycloalkyl, piperidyl, piperazinyl, and morpholinyl, wherein, the C3-C6 cycloalkyl, piperidyl, piperazinyl, and morpholinyl are optionally substituted with 1-2 substituents selected from the group consisting of hydroxy C1-C6 alkyl (such as methylol), formyl, and C1-C6 alkyl (preferably methyl);
preferably, R¹⁴ is selected from the group consisting of 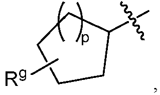 and piperidyl, wherein, the piperidyl is optionally substituted with 1-2 substituents selected from C1-C6 alkyl (preferably methyl); p is selected from the group consisting of 0, 1, and 2; preferably, p is 2; R^{g} is selected from the group consisting of H, hydroxy C1-C6 alkyl, and formyl; preferably, R^{g} is H;
further preferably, R¹⁴ is selected from the group consisting of 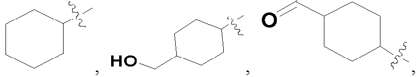 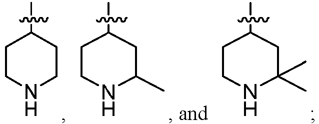
further preferably, R¹⁴ is selected from the group consisting of 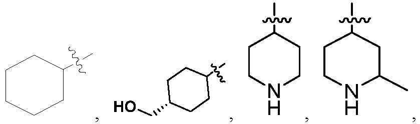 and 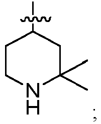
further preferably, 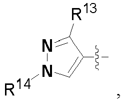 as a whole, is selected from the group consisting of 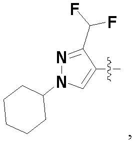 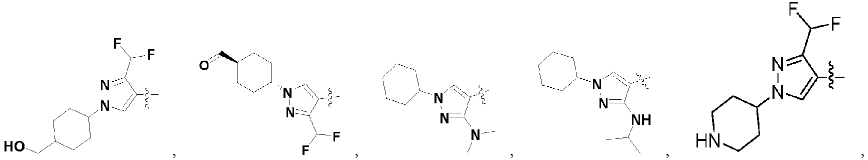 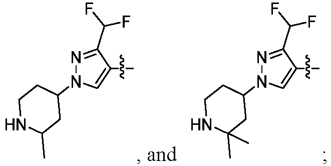
further preferably, 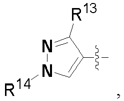 as a whole, is selected from the group consisting of

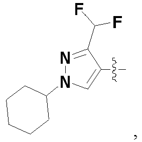

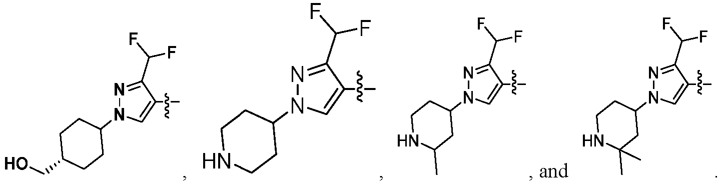

13. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 1-12, wherein, the compound is represented by formula VI-1, formula VI-2, formula VI-3, formula VII-1-1 or formula VII-1-2, 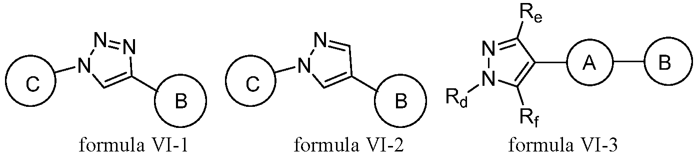 in formula VI-1, formula VI-2, and formula VI-3, A, B, C, R_{d}, Rₑ, R_{f} are as defined in anyone of claims 1-12; 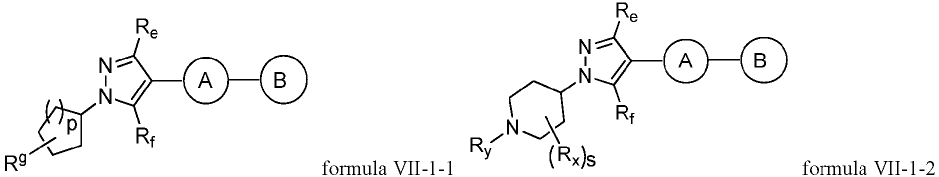
in formula VII-1-1, A, B, Rₑ, R_{f}, R^{g}, p are as defined in anyone of claims 1-12;
in formula VII-1-2, each Rₓ is independently selected from the group consisting of halogen, C1-C6 alkoxy, hydroxy, amino, C1-C6 alkyl, and cyano, s is selected from the group consisting of 0, 1, 2, and 3, or two Rₓ together with the C atom to which they are attached may form C1-C6 cycloalkyl, R_{y} is selected from the group consisting of H, and C1-C6 alkyl; A, B, Rₑ, R_{f} are as defined in anyone of claims 1-12.

14. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 1-13, wherein, the compound is represented by formula VII-2-1, formula VII-2-2, formula VII-3-1, formula VII-3-2 or formula VII-3-3, 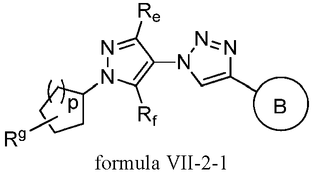 in formula VII-2-1, B, Rₑ, R_{f}, R^{g}, p are as defined in anyone of claims 1-13; 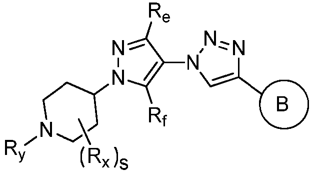 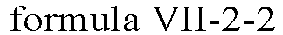 in formula VII-2-2, B, Rₑ, R_{f}, Rₓ, R_{y}, s are as defined in anyone of claims 1-13; 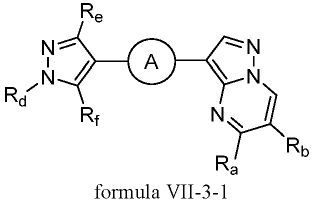 in formula VII-3-1, A, R_{d}, Rₑ, R_{f}, Rₐ, R_{b} are as defined in anyone of claims 1-13; 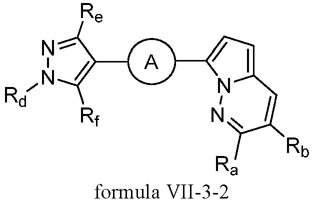 in formula VII-3-2, A, R_{d}, Rₑ, R_{f}, Rₐ, R_{b} are as defined in anyone of claims 1-13; 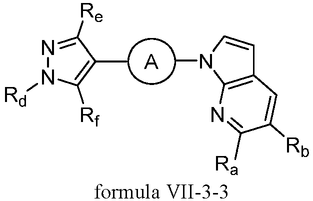 in formula VII-3-3, A, R_{d}, Rₑ, R_{f}, Rₐ, R_{b} are as defined in anyone of claims 1-13.

15. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 1-14, wherein, the compound is represented by formula VIII-1, formula VIII-2 or formula VIII-3, 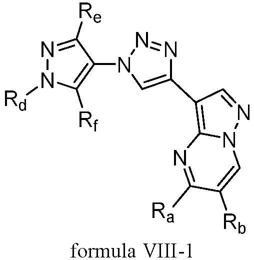 in formula VIII-1, R_{d}, Rₑ, R_{f}, Rₐ, R_{b} are as defined in anyone of claims 1-14; 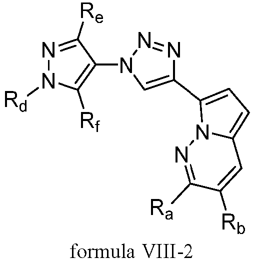 in formula VIII-2, R_{d}, Rₑ, R_{f}, Rₐ, R_{b} are as defined in anyone of claims 1-14; 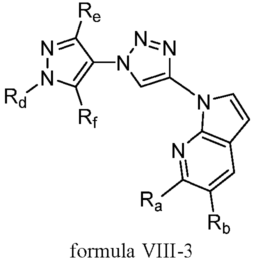 in formula VIII-3, Rₐ, Rₑ, R_{f}, Rₐ, R_{b} are as defined in anyone of claims 1-14.

16. The compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 1-15, wherein, the compound is selected from the group consisting of: 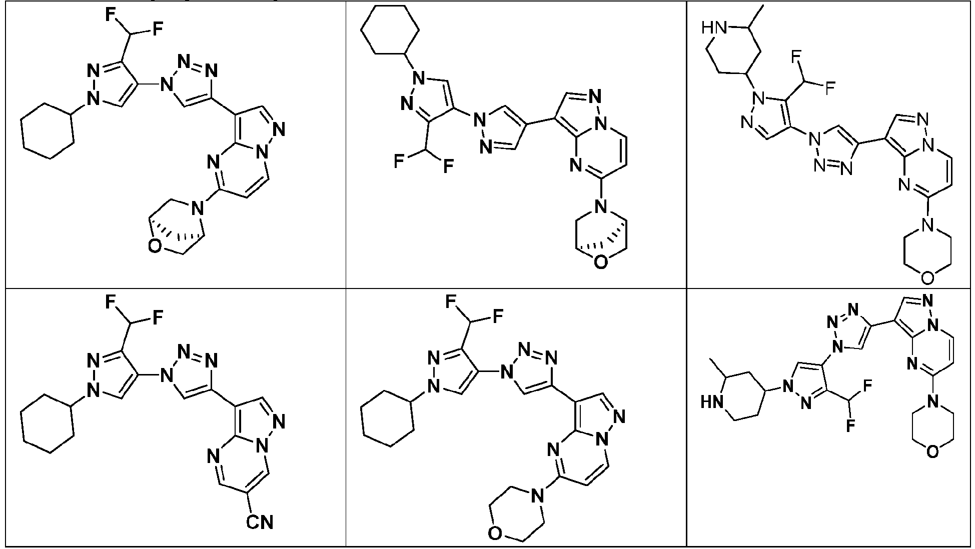 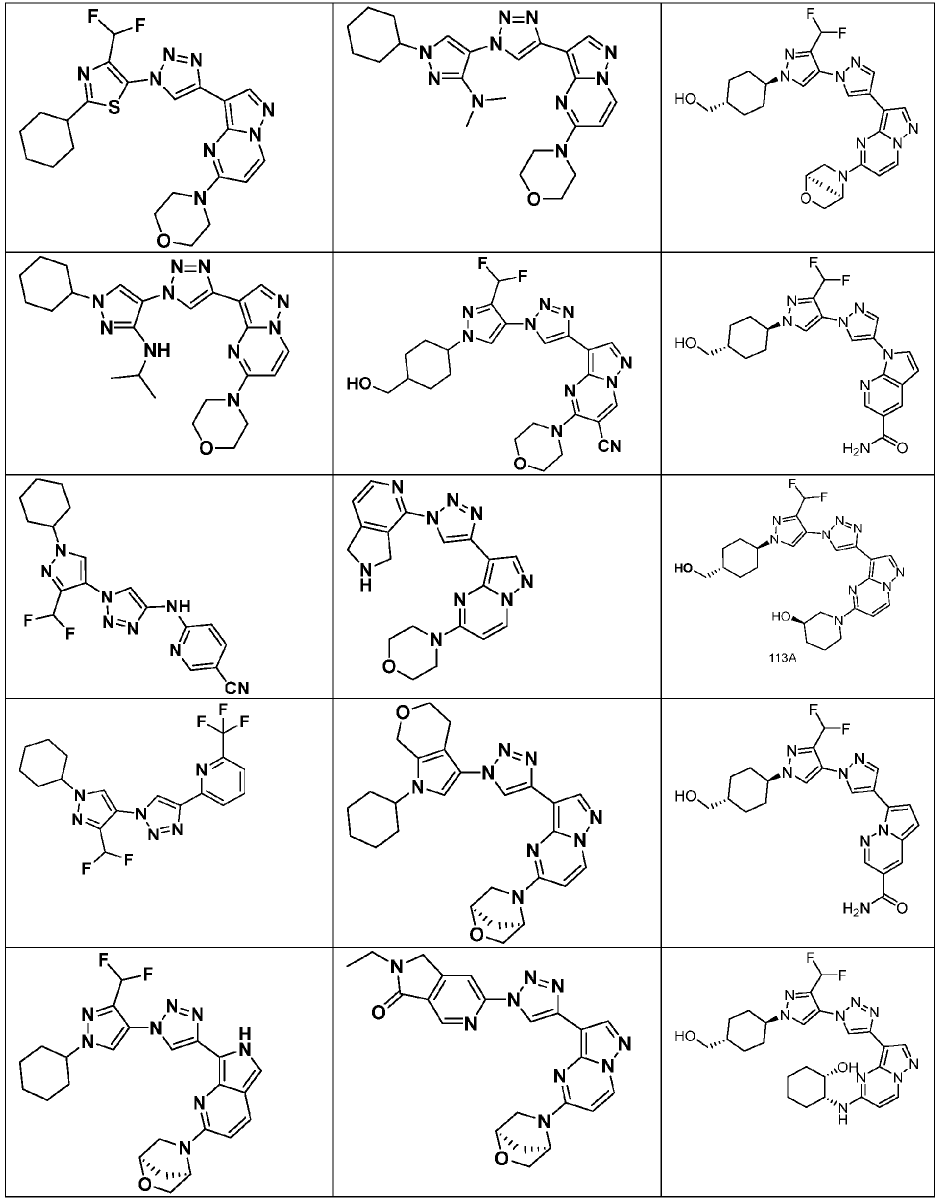 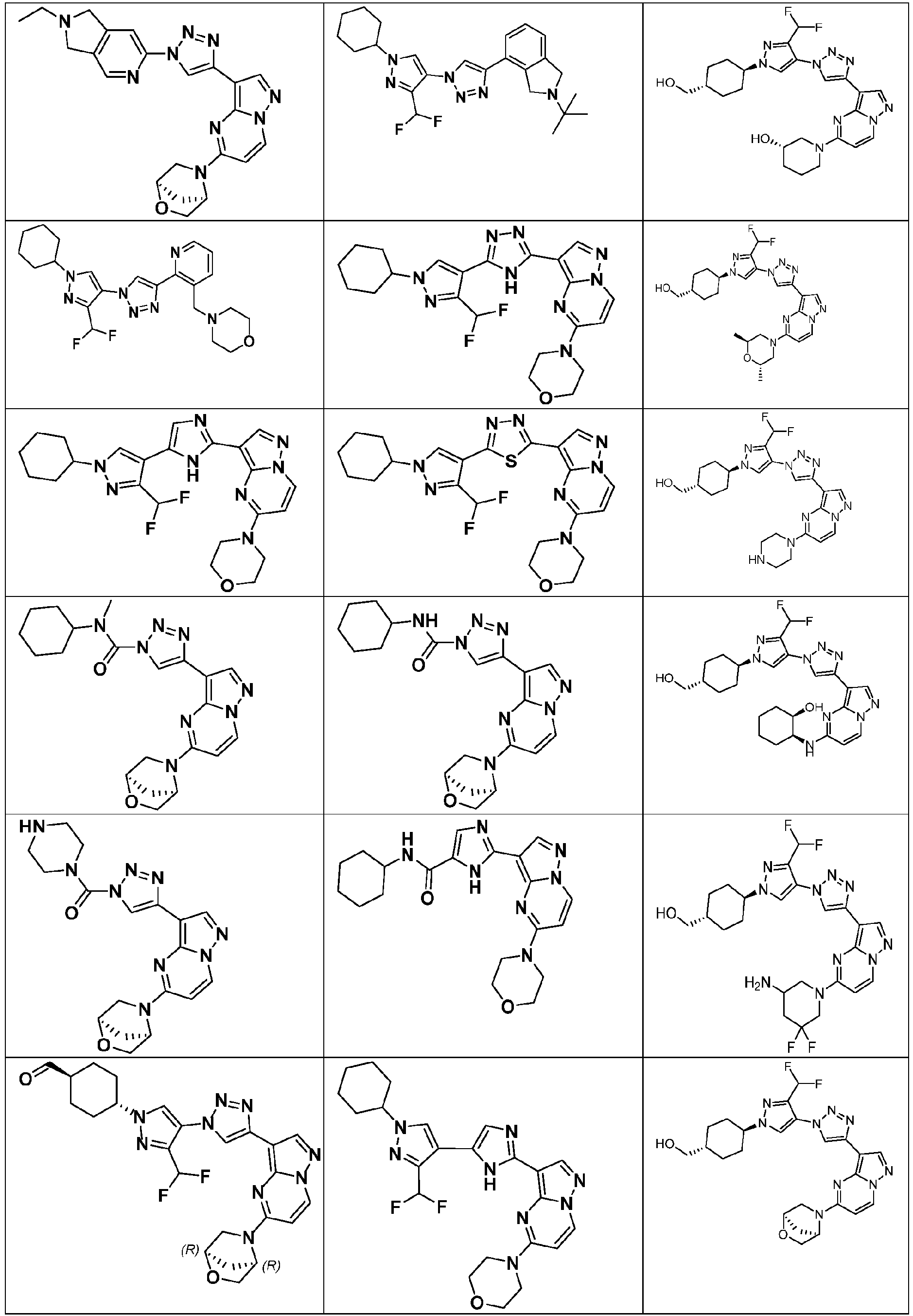 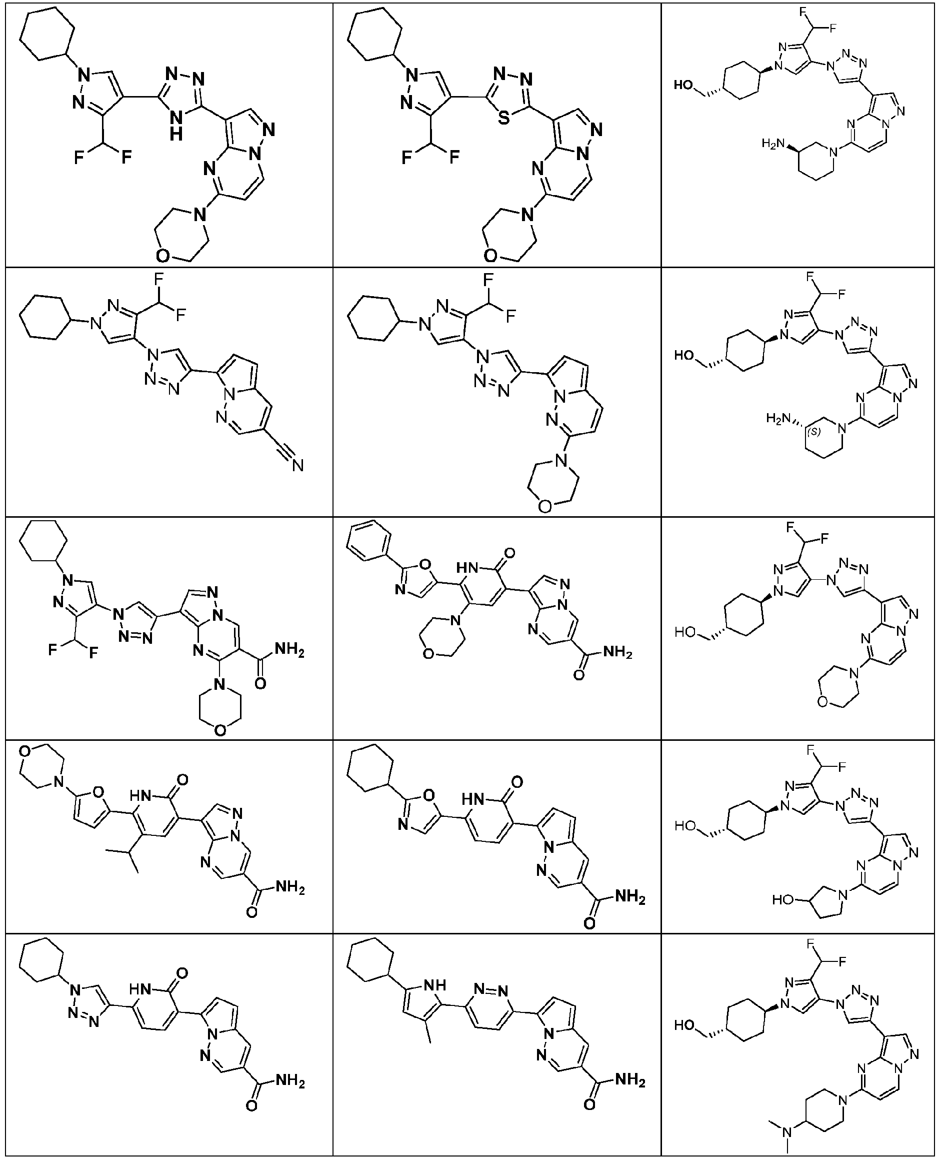 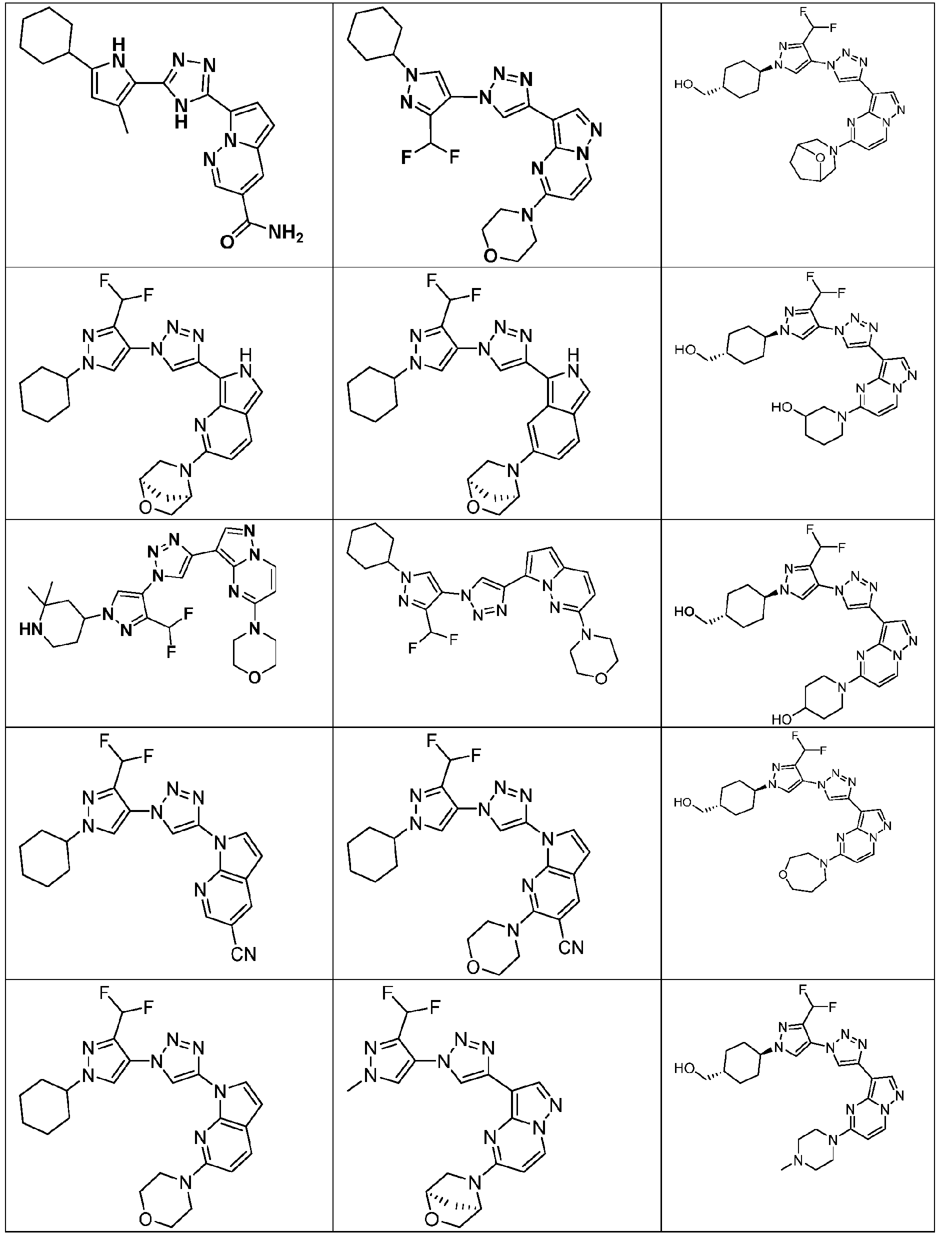 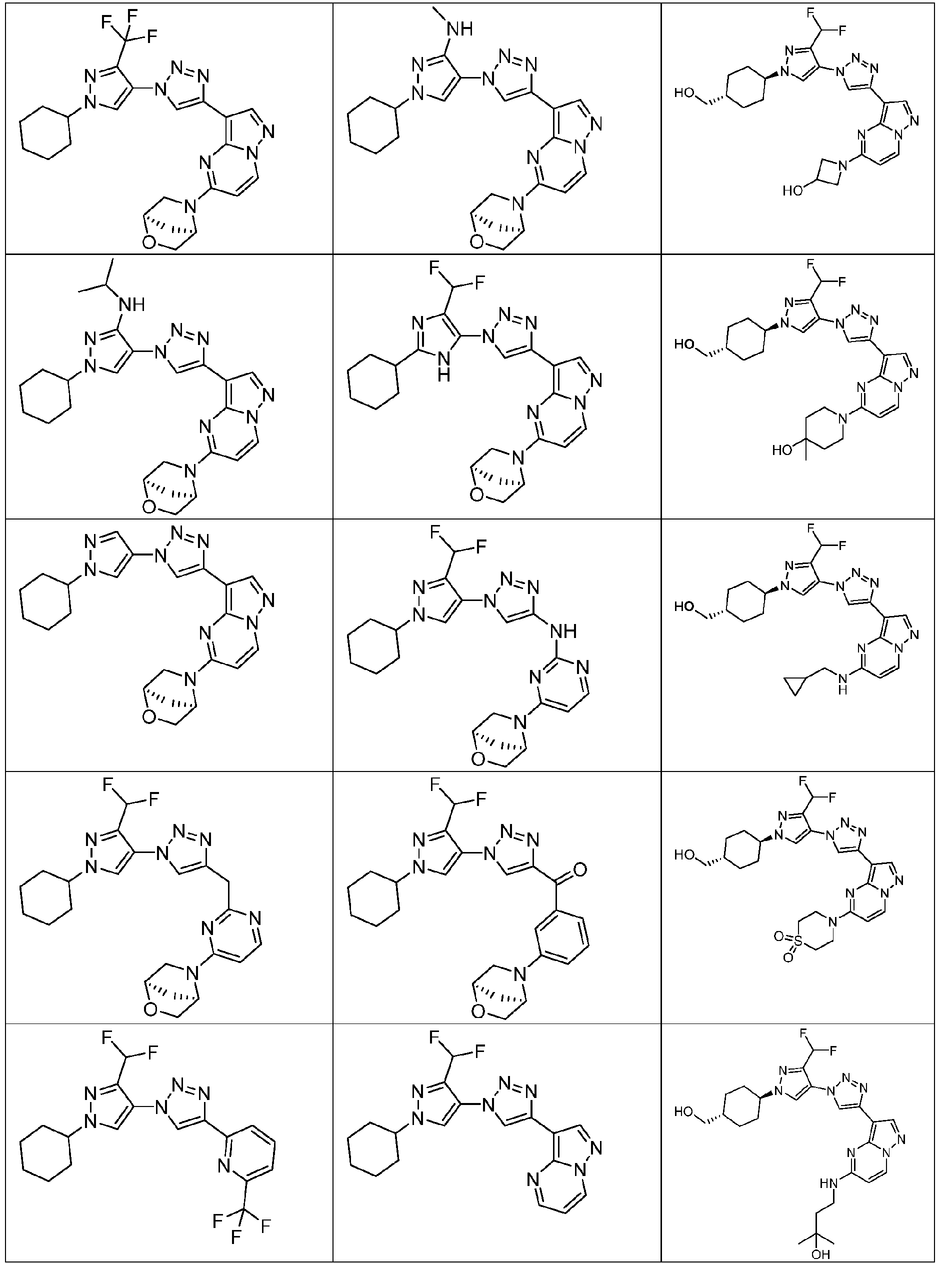 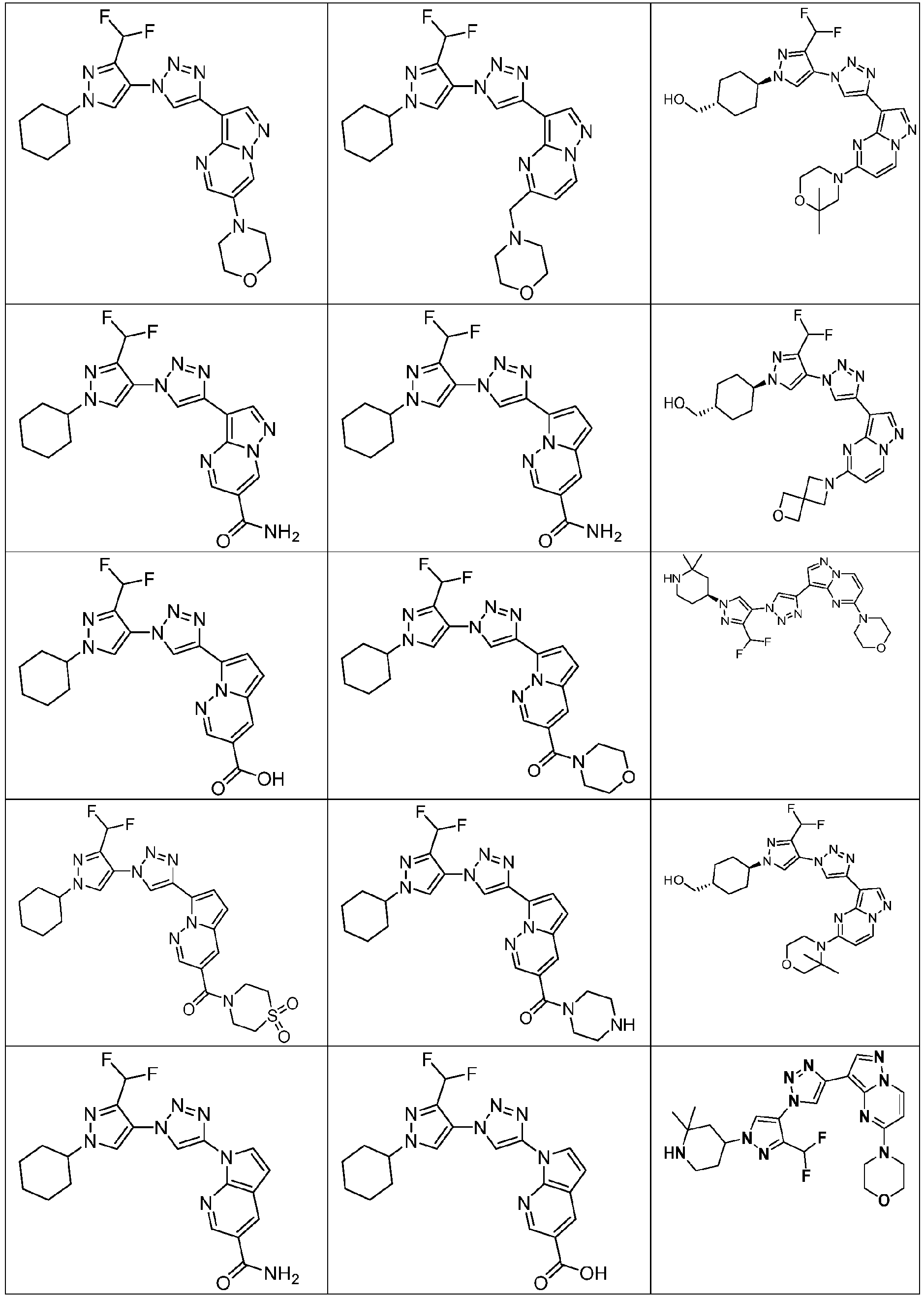 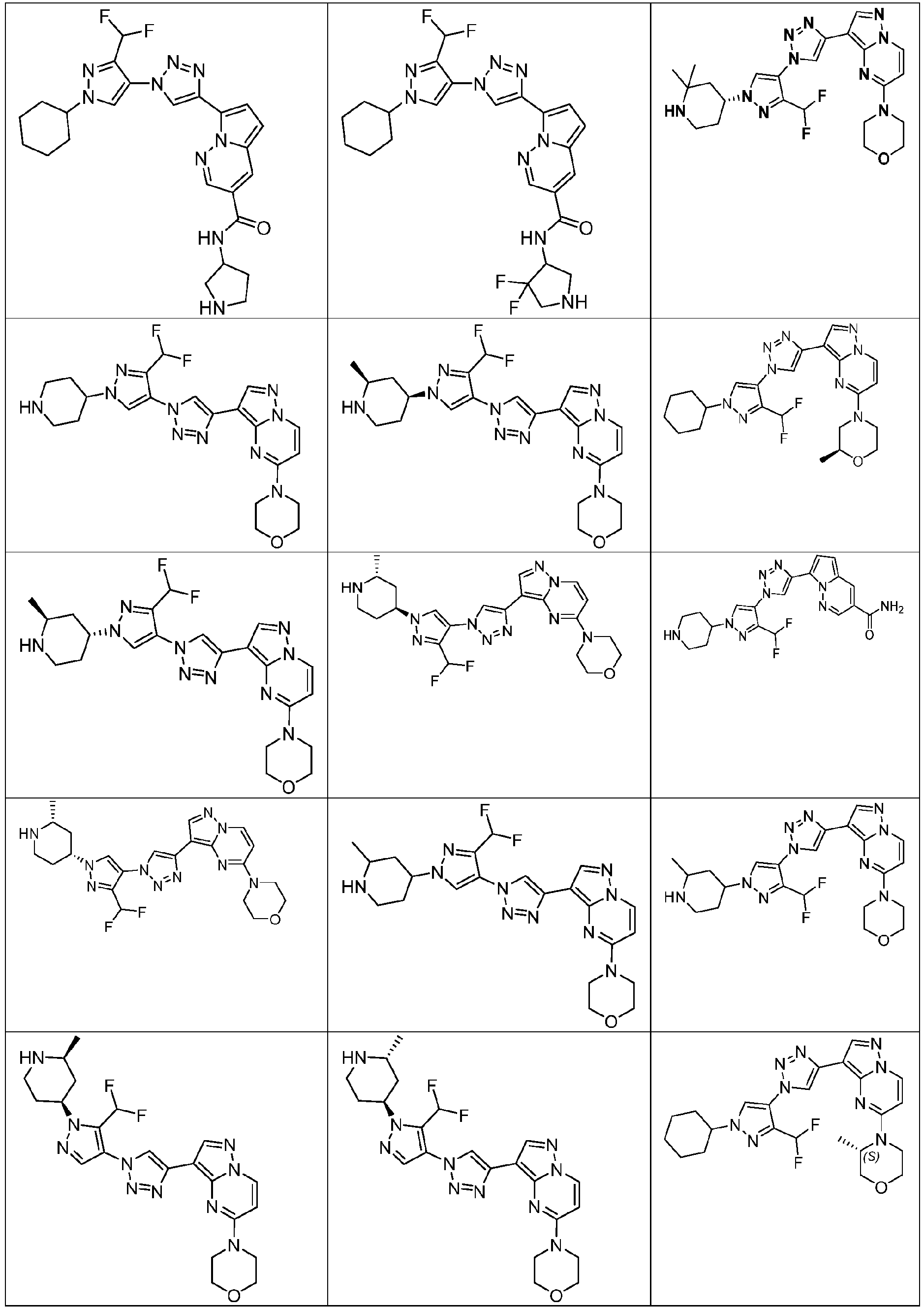 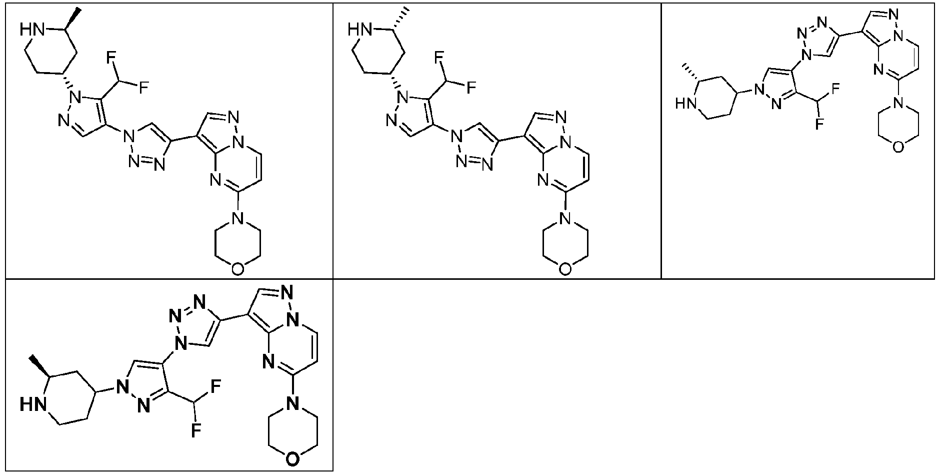 ; preferably, the compound is selected from the group consisting of:

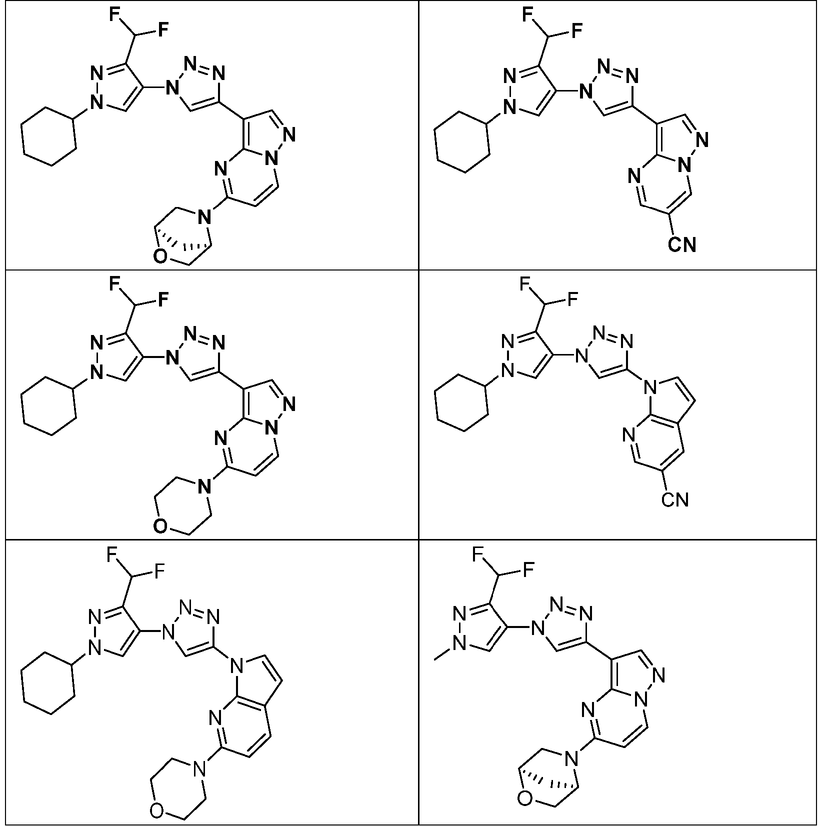

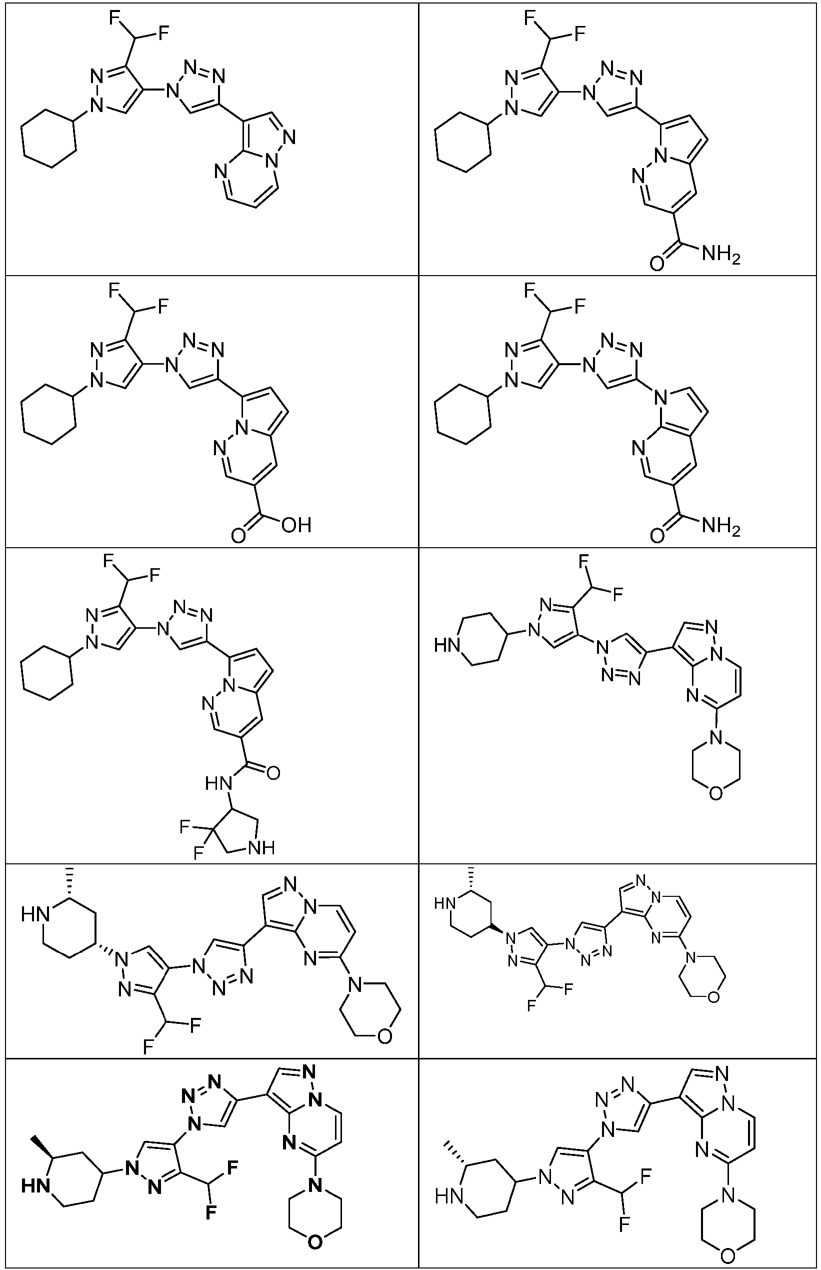

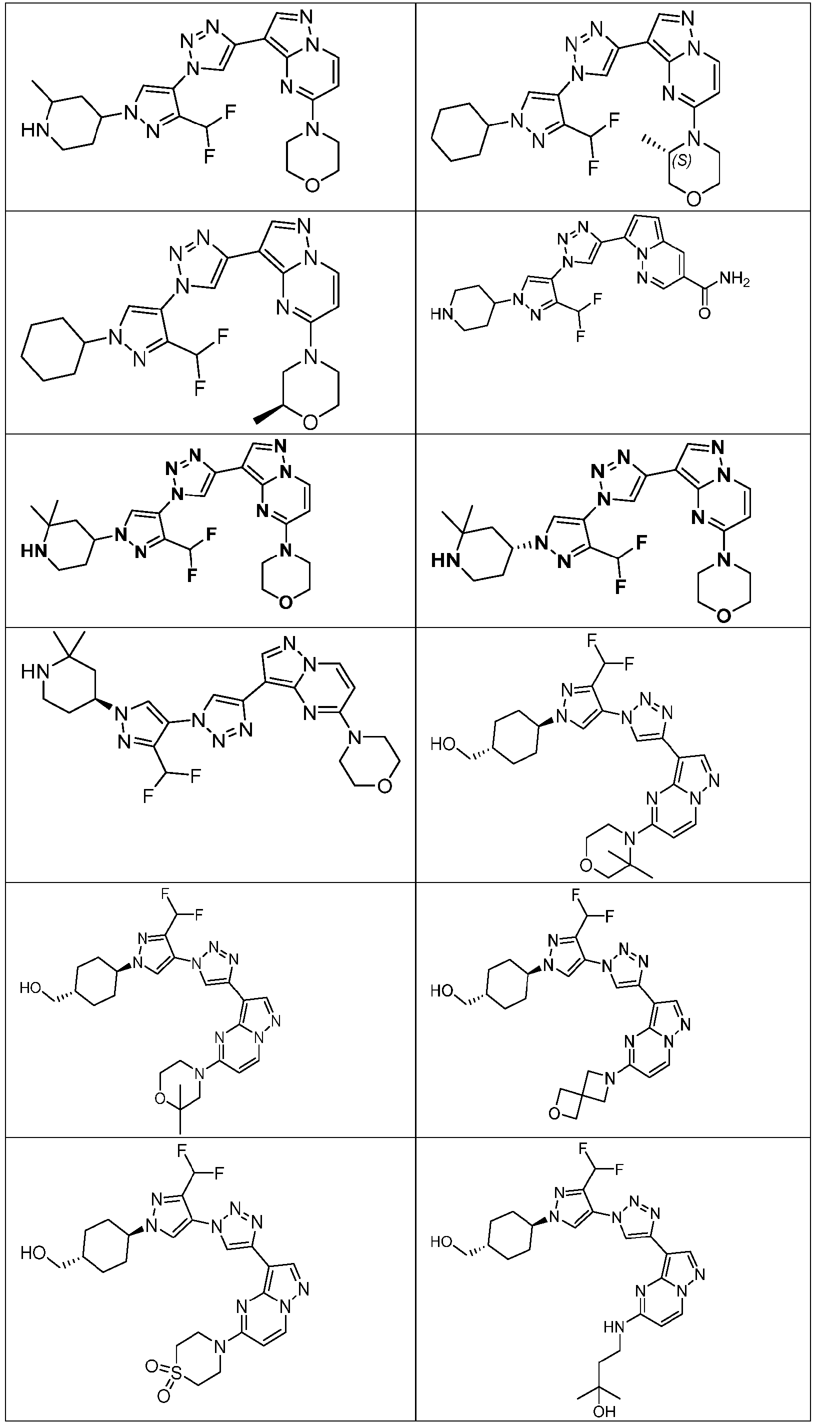

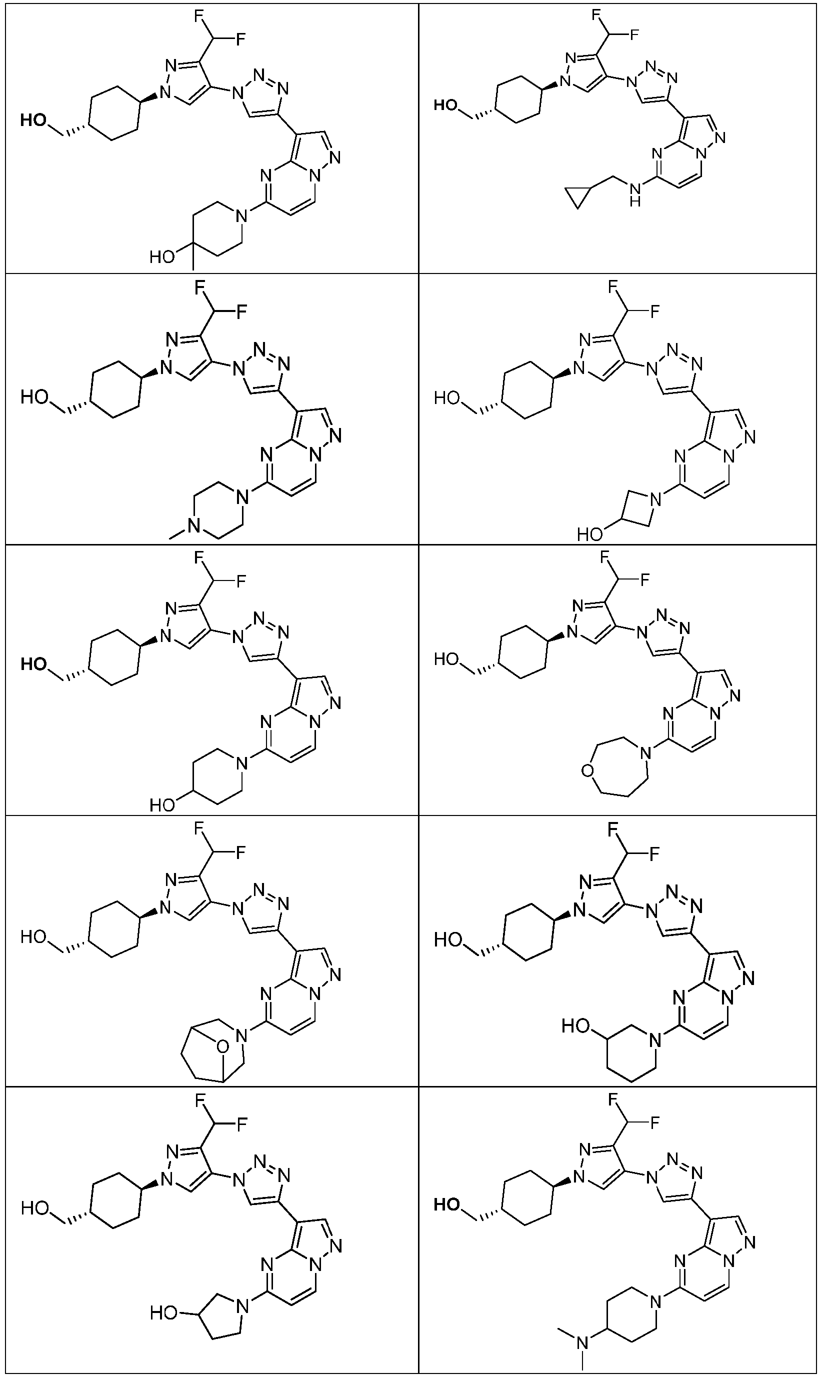

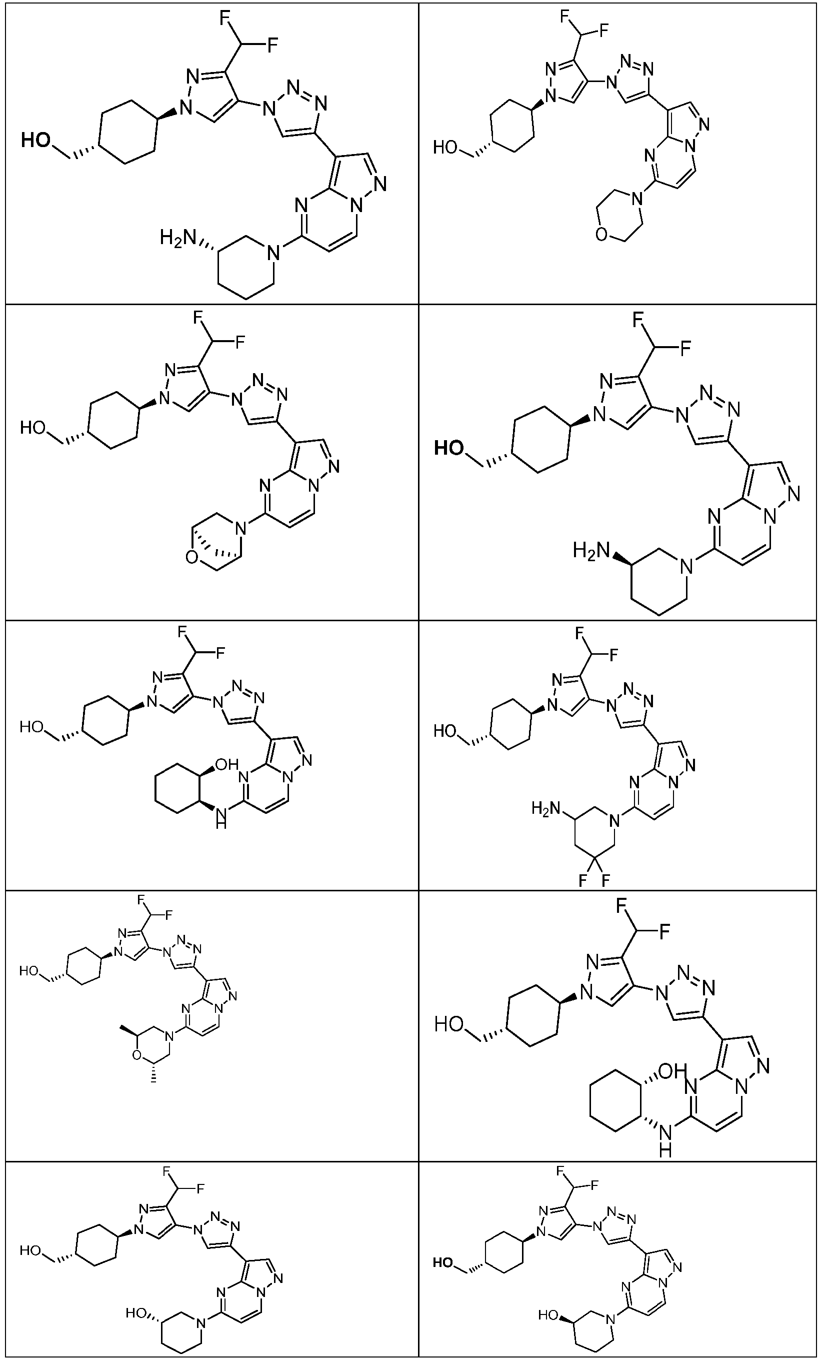

17. A method for preparing a compound of formula A according to anyone of claims 1-16, which is **characterized in that**:
in formula A, A is 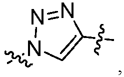 the method comprises: 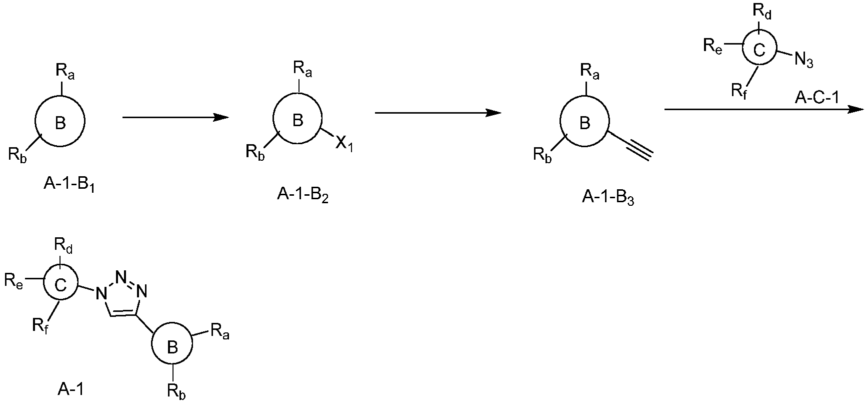
allowing compound A-1-B₁ to undergo a halogenation reaction to obtain compound A-1-B₂, allowing the compound A-1-B₂ to undergo a substitution reaction to obtain compound A-1-B₃, and allowing the compound A-1-B₃ to undergo a cyclization reaction with compound A-C-1 to obtain compound A-1;
wherein X₁ is halogen (such as Cl, Br, I), Rₐ, R_{b}, R_{d}, Rₑ, R_{f}, B, and C are as defined in anyone of claims 1-27;
preferably, the method for preparing the compound A-C-1 comprises: 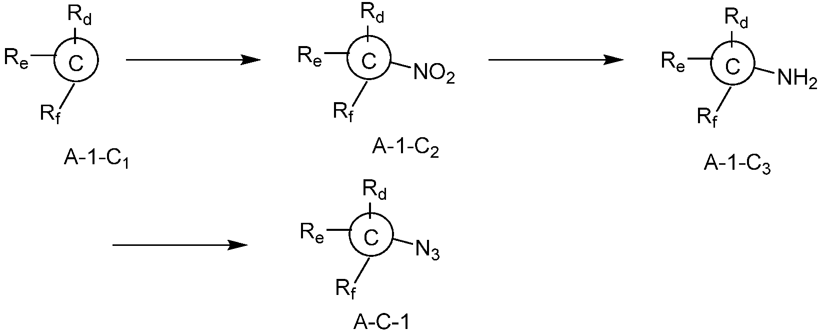
allowing compound A-1-C₁ to undergo a nitration reaction to obtain compound A-1-C₂, allowing the compound A-1-C₂ to undergo a reduction reaction to obtain compound A-1-C₃, and allowing the compound A-1-C₃ to undergo an azidation reaction to obtain the compound A-C-1, wherein R_{d}, Rₑ, R_{f} and C are as defined in anyone of claims 1-27.

18. A method for preparing a compound of formula A according to anyone of claims 1-16, wherein,
in formula A, A is 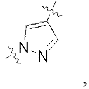 the method comprises: 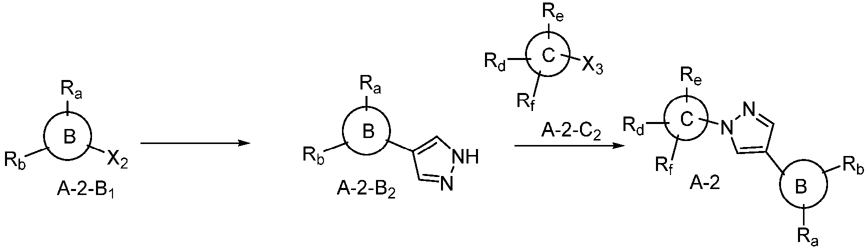
allowing compound A-2-B₁ to undergo a coupling reaction to obtain compound A-2-B₂, and allowing the compound A-2-B₂ and compound A-2-C₂ to undergo a coupling reaction to obtain compound A-2, wherein X₂ and X₃ are each independently halogen (such as Cl, Br, I), Rₐ, R_{b}, Rₐ, Rₑ, R_{f}, B, and C are as defined in anyone of claims 1-16;
preferably, the method for preparing the compound A-2-C₂ comprises: 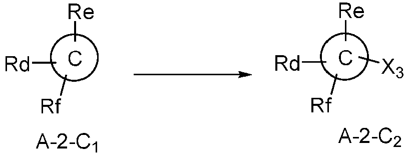
allowing the compound A-2-C₁ to undergo a halogenation reaction to obtain the compound A-2-C₂, wherein X₃ is halogen (such as Cl, Br, I), R_{d}, Rₑ, R_{f}, and C are as defined in anyone of claims 1-16;
or in formula A, A is 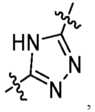
the method comprises: 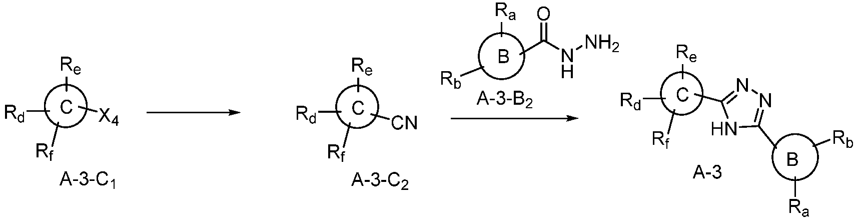
allowing compound A-3-C₁ to undergo a cyanation reaction to obtain compound A-3-C₂, and allowing the compound A-3-C₂ to undergo a cyclization reaction with A-3-B₂ to obtain A-3;
wherein X₄ is halogen (such as Cl, Br, I), Rₐ, R_{b}, R_{d}, Rₑ, R_{f}, B, and C are as defined in anyone of claims 1-16;
preferably, the method for preparing the compound A-3-B₂ comprises: 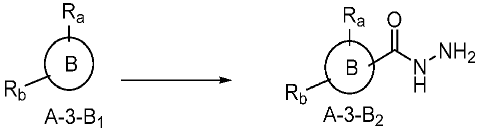
allowing compound A-3-B₁ to undergo an acylation reaction to obtain the compound A-3-B₂, wherein Rₐ, R_{b}, B are as defined in anyone of claims 1-16.

19. A pharmaceutical composition, comprising a compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 1-16, and optionally a pharmaceutically acceptable excipient.

20. A PROTAC molecule, comprising: a warhead moiety, a ligase binding moiety and a connecting chain, wherein, the warhead moiety is a compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 1-16, the connecting chain is covalently linked to the warhead moiety and the ligase binding moiety respectively ;
preferably, the ligase binding moiety is an E3 ubiquitin ligase binding moiety.

21. A compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 1-16, or a pharmaceutical composition according to claim 19, or a PROTAC molecule according to claim 20 in the preparation of a medicament for the treatment and/or prevention of IRAK4 kinase-related diseases;
preferably, the IRAK4 kinase-related disease is cancer or autoimmune disease;
preferably, the autoimmune disease is selected from rheumatoid arthritis, osteoarthritis, chronic obstructive pulmonary disease, systemic lupus erythematosus, psoriasis, ulcerative colitis, irritable bowel syndrome, or any combination thereof;
preferably, the cancer is selected from B-cell chronic lymphocytic leukemia, acute lymphocytic leukemia, non Hodgkin lymphoma, Hodgkin lymphoma, acute myeloid leukemia, diffuse large B-cell lymphoma, multiple myeloma, Fahrenheit macroglobulinemia, or any combination thereof.

22. A method for the treatment and/or prevention of IRAK4 kinase-related diseases, comprising administering to a subject an effective amount of a compound, or an enantiomer, a diastereoisomer, a racemate, a tautomer, a stereoisomer, a geometric isomer, a nitrogen oxide, a metabolite or a pharmaceutically acceptable salt, an ester, a solvate, a hydrate, an isotope-labeled compound (preferably a deuteride), or a prodrug thereof according to anyone of claims 1-16, or a pharmaceutical composition according to claim 19, or a PROTAC molecule according to claim 20;
preferably, the IRAK4 kinase-related disease is cancer or autoimmune disease;
preferably, the autoimmune disease is selected from rheumatoid arthritis, osteoarthritis, chronic obstructive pulmonary disease, systemic lupus erythematosus, psoriasis, ulcerative colitis, irritable bowel syndrome, or any combination thereof;
preferably, the cancer is selected from B-cell chronic lymphocytic leukemia, acute lymphocytic leukemia, non Hodgkin lymphoma, Hodgkin lymphoma, acute myeloid leukemia, diffuse large B-cell lymphoma, multiple myeloma, Fahrenheit macroglobulinemia, or any combination thereof.
